# EUROPEAN PATENT APPLICATION

(11) **EP 4 595 973 A1**
(43) Date of publication of application: **06.08.2025**
(21) Application number: 23872590.7
(22) Date of filing: 29.09.2023
(51) Int. Cl.: A61K 38/12, A61P 27/02, A61P 27/06

(54) **CYCLIC PEPTIDE DERIVATIVE COMPOSITION FOR TREATING OR PREVENTING EYE DISEASE**

(30) Priority: 30.09.2022 JP 2022159075
(71) Applicant: DKS Co. Ltd., Kyoto-shi, Kyoto 600-8873 (JP)
(72) Inventor: MATSUMOTO, Kohei, Kyoto-shi, Kyoto 600-8873 (JP); ONISHI, Kaito, Kyoto-shi, Kyoto 600-8873 (JP); TERAGAMI, Kana, Kyoto-shi, Kyoto 600-8873 (JP); KARITA, Yuma, Kyoto-shi, Kyoto 600-8873 (JP); TANABE, Shichidai, Kyoto-shi, Kyoto 600-8873 (JP); YASUI, Kosuke, Kyoto-shi, Kyoto 600-8873 (JP); SHOSU, Takeshi, Kyoto-shi, Kyoto 600-8873 (JP)
(74) Representative: J A Kemp LLP
(86) International application number: PCT/JP2023/035591
(87) International publication number: WO 2024/071370

(57) **Abstract**

The present disclosure provides a cyclic peptide derivative composition for treating or preventing ophthalmic diseases.

The present disclosure relates to a composition comprising a compound for treating or preventing ophthalmic diseases or a pharmaceutically acceptable salt, solvate or prodrug thereof. More specifically, the composition of the present disclosure can treat or prevent diabetic retinopathy, glaucoma or age-related macular degeneration. The technology provided by the present disclosure can be used for a cyclic peptide derivative for modulating nervous system cell activity and a method for producing said cyclic peptide derivative.

## Description

### [Technical Field]

The present disclosure relates to cyclic peptide derivative compositions for treating or preventing ophthalmic diseases.

### [Background Art]

Various disorders of the eye are characterized by, cause, or result from choroidal neovascularization, retinal neovascularization or iris neovascularization or retinal edema. One of these disorders is macular degeneration. Age-related macular degeneration (AMD) is a disease that affects approximately 1 in 10 Americans over the age of 65. One type of AMD is "wet AMD", which accounts for only approximately 10% of cases of age-related macular degeneration, but results in roughly 90% of cases of legal blindness due to macular degeneration in the elderly. Another disorder of the eye is diabetic retinopathy, which can affect up to 80% of all patients with diabetes for 10 years or more, is the third leading cause of adult blindness, and accounts for approximately 7% of blindness in the United States. Other disorders include hypertensive retinopathy, central serous chorioretinopathy, cystoid macular edema, Coats' disease, and tumors of the eye and ocular adnexa, such as choroidal hemangioma, retinal pigment epithelial carcinoma, retinal vein occlusion, and intraocular lymphoma.

### [Summary of the Invention]

### [Solution to Problem]

The present disclosure provides cyclic peptide derivative compositions for treating or preventing ophthalmic diseases.

The present inventors have conducted intensive studies in an attempt and found that the compounds represented by the following formula and related structural formulas, or pharmaceutical acceptable salts thereof (hereinafter sometimes referred to as "the compound(s) of the disclosure") have significant proliferation activity, which resulted in the completion of the technical matters of the present disclosure. Accordingly, the present disclosure is as follows:
(Item 1)
   A composition for treating or preventing an ophthalmic disease, comprising a compound represented by the following Formula (1) 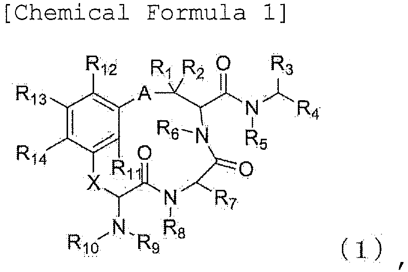 or a pharmaceutically acceptable salt, solvate or prodrug thereof:
   wherein
   R₁, R₂, R₅, R₆, R₇, R₈, R₉ and R₁₀ are each independently
   a hydrogen atom, or
   an optionally substituted hydrocarbon group, or R₇ and R₈, together with the carbon atom and nitrogen atom to which they are attached, form an optionally substituted heterocycloalkyl group,
   R₃ and R₄ are each independently
   a hydrogen atom, an optionally substituted hydrocarbon group, a carboxyl group,
   an optionally substituted alkoxycarbonyl group, or
   an optionally substituted alkoxycarbonyloxy group,
   R₁₁, R₁₂, R₁₃, and R₁₄ are each independently
   a hydrogen atom, an optionally substituted hydrocarbon group, a hydroxy group,
   an optionally substituted alkoxy group, or
   an optionally substituted alkoxycarbonyloxy group,
   X is CH₂ or CO, and
   A is O, NH or S, wherein the NH is optionally substituted.
(Item 2)
   The composition of any one of the preceding items, wherein the ophthalmic disease comprises a retinal disease.
(Item 3)
   The composition of any one of the preceding items, wherein the ophthalmic disease comprises at least one of diabetic retinopathy, glaucoma or age-related macular degeneration.
(Item 4)
   The composition of any one of the preceding items, wherein R₁ and R₂ are each independently a hydrogen atom, or a C₁₋₆ alkyl group.
(Item 5)
   The composition of any one of the preceding items, wherein R₁ and R₂ are each independently a hydrogen atom, a methyl group or an ethyl group.
(Item 6)
   The composition of any one of the preceding items, wherein R₃ and R₄ are each independently a hydrogen atom, a C₁₋₆ alkyl group substituted with a carboxyl group, or a carboxyl group.
(Item 7)
   The composition of any one of the preceding items, wherein R₃ and R₄ are each independently a hydrogen atom, a carboxymethyl group, a carboxyethyl group, a carboxypropyl group or a carboxyl group.
(Item 8)
   The composition of any one of the preceding items, wherein R₅ is a hydrogen atom, or a C₁₋₆ alkyl group.
(Item 9)
   The composition of any one of the preceding items, wherein R₅ is a hydrogen atom.
(Item 10)
   The composition of any one of the preceding items, wherein R₆ is a hydrogen atom, or a C₁₋₆ alkyl group.
(Item 11)
   The composition of any one of the preceding items, wherein R₆ is a hydrogen atom.
(Item 12)
   The composition of any one of the preceding items, wherein R₇ is a hydrogen atom, a C₁₋₆ alkyl group, a hydroxy C₁₋₆ alkyl group, a carbamoyl C₁₋₆ alkyl group, a C₆₋₁₀ aryl C₁₋₆ alkyl group, a hydroxy C₆₋₁₀ aryl C₁₋₆ alkyl group, a C₅₋₁₀ heteroaryl C₁₋₆ alkyl group, a carboxy C₁₋₆ alkyl group, an amino C₁₋₆ alkyl group, a thio C₁₋₆ alkyl group, a C₁₋₆ alkylthio C₁₋₆ alkyl group, or an amidinoamino C₁₋₆ alkyl group.
(Item 13)
   The composition of any one of the preceding items, wherein R₇ is a hydrogen atom, a methyl group, an isopropyl group, an isobutyl group, a sec-butyl group, a benzyl group, a hydroxymethyl group, a 1-hydroxyethyl group, a carboxymethyl group, a carboxyethyl group, a 4-hydroxybenzyl group, a 4-aminobutyl group, a thiomethyl group, a 2-methylthioethyl group, a carbamoylmethyl group, a carbamoylethyl group, an amidinoaminopropyl group, an indolylmethyl group or a 4-imidazolemethyl group.
(Item 14)
   The composition of any one of the preceding items, wherein R₈ is a hydrogen atom, or a C₁₋₆ alkyl group.
(Item 15)
   The composition of any one of the preceding items, wherein R₈ is a hydrogen atom.
(Item 16)
   The composition of any one of the preceding items, wherein R₇ and R₈, together with the carbon atom and nitrogen atom to which they are attached, form an optionally substituted heterocycloalkyl group.
(Item 17)
   The composition of any one of the preceding items, wherein R₇ and R₈, together with the carbon atom and nitrogen atom to which they are attached, form a C₅₋₁₀ heterocycloalkyl group.
(Item 18)
   The composition of any one of the preceding items, wherein R₉ and R₁₀ is a hydrogen atom, or a C₁₋₆ alkyl group.
(Item 19)
   The composition of any one of the preceding items, wherein R₉ and R₁₀ are each independently a hydrogen atom or a methyl group.
(Item 20)
   The composition of any one of the preceding items, wherein R₁₁, R₁₂, R₁₃, and R₁₄ are each independently a hydrogen atom, an alkoxy group or a hydroxy group.
(Item 21)
   The composition of any one of the preceding items, wherein R₁₂ is a hydrogen atom, or a hydroxy group.
(Item 22)
   The composition of any one of the preceding items, wherein R₁₁, R₁₂, R₁₃, and R₁₄ are each independently a hydrogen atom, or a hydroxy group.
(Item 23)
   The composition of any one of the preceding items, wherein X is CH₂ or CO.
(Item 24)
   The composition of any one of the preceding items, wherein A is O, NH substituted with a C₁₋₆ alkyl group, NH or S.
(Item 25)
   The composition of any one of the preceding items, wherein A is O, NH or S.
(Item A1)
   A composition for treating or preventing an ophthalmic disease, comprising a compound represented by the following Formula (A2) 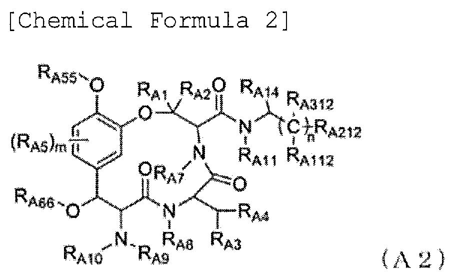 or a pharmaceutically acceptable salt, solvate or prodrug thereof:
   wherein m is 0 to 3, n≧1, R_{A1} to R_{A4}, R_{A7} to R_{A11}, R_{A312}, and R_{A112} are each independently a hydrogen atom, or a hydrocarbon group, R_{A14}, and R_{A212} are each independently hydrogen, a carboxyl group or a salt thereof, or an alkoxycarbonyl group, R_{A5} is a hydrocarbon group, a hydroxyl group, an alkoxy group, or an alkylcarbonyloxy group, and R_{A55}, and R_{A66} are each independently a hydrogen atom, a hydrocarbon group, or an alkylcarbonyloxy group.
(Item A2)
   The composition of any one of the preceding items, wherein the ophthalmic disease comprises a retinal disease.
(Item A3)
   The composition of any one of the preceding items, wherein the ophthalmic disease comprises at least one of diabetic retinopathy, glaucoma or age-related macular degeneration.

In the present disclosure, the above-described one or more characteristics intend that, in addition to the explicitly shown combination, further combinations may be provided. Further embodiment and advantage of the present disclosure can be recognized by those skilled in the art by reading and understanding the following detailed explanation as needed.

### [Effect of the Invention]

According to the present disclosure, there is provided a novel cyclic peptide derivative and composition that are useful for treating or preventing diabetic retinopathy, glaucoma or age-related macular degeneration, and the like.

### [Brief Description of Drawings]

[Fig. 1]

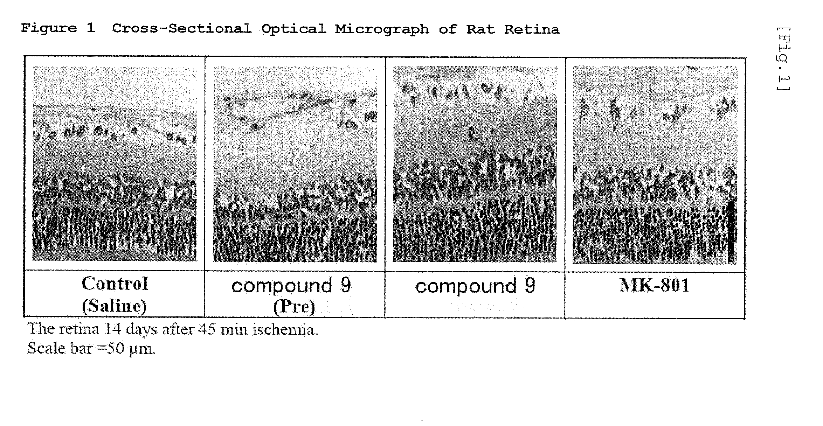

   Fig.1 shows cross-sectional optical micrograph of a rat retina.
[Fig. 2]

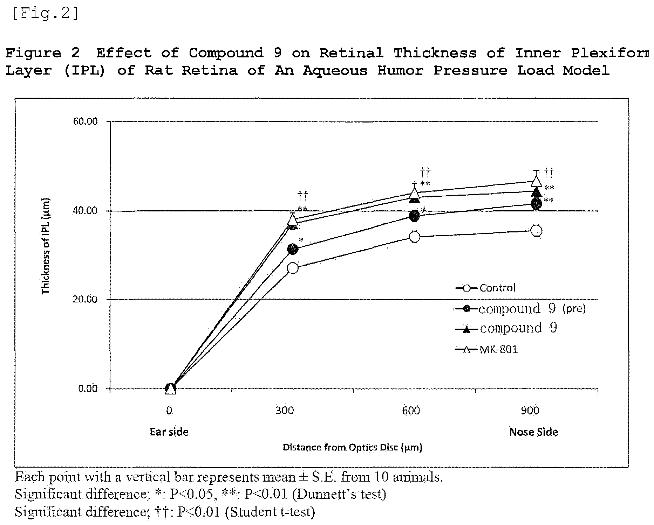

   Fig.2 shows the effect of the compound on the retinal thickness of the inner plexiform layer of the rat retina of an aqueous humor pressure load model.
[Fig. 3]

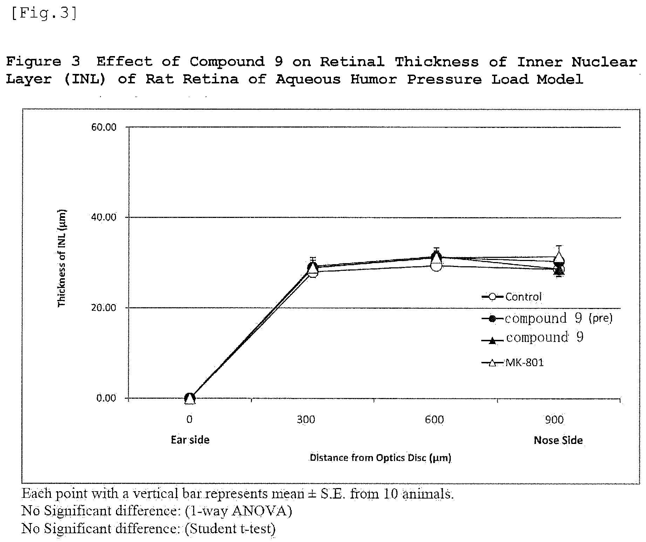

   Fig.3 shows the effect of the compound on the retinal thickness of the inner nuclear layer of the rat retina of an aqueous humor pressure load model.
[Fig. 4]

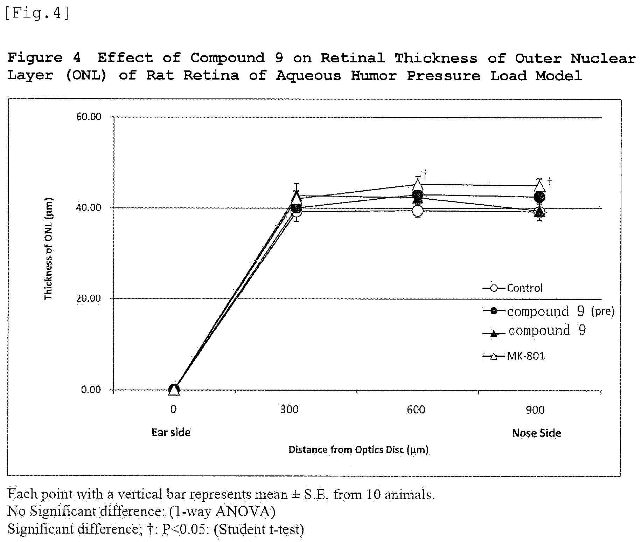

   Fig.4 shows the effect of the compound on the retinal thickness of the outer nuclear layer of the rat retina of an aqueous humor pressure load model.
[Fig. 5]

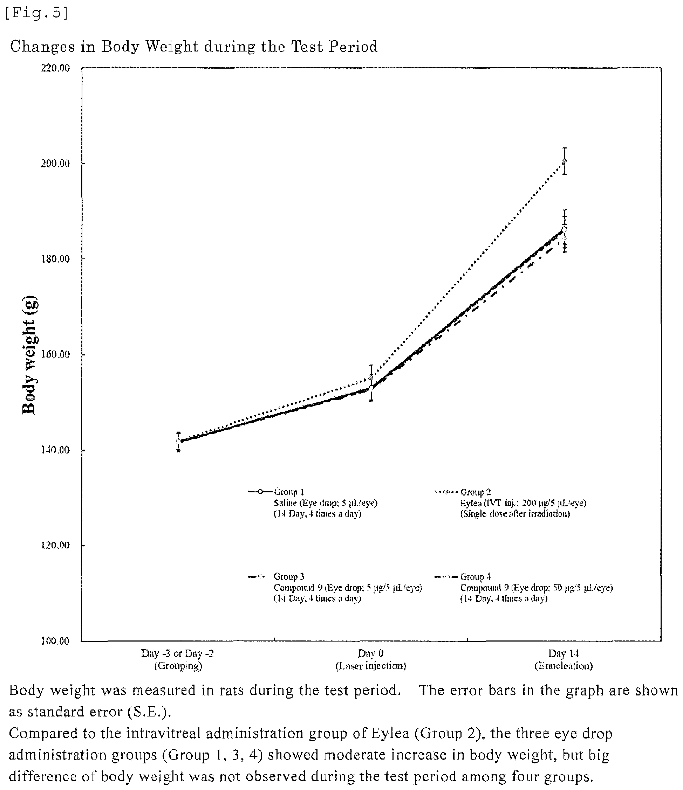

   Fig.5 shows the change in body weight after laser irradiation in a laser-induced choroidal neovascularization rat model.
[Fig. 6]

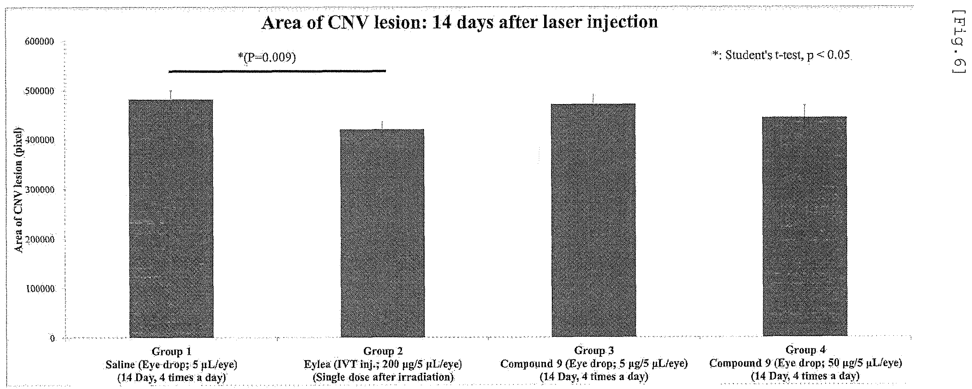

   Fig.6 shows the CNV area 14 days after laser irradiation in a laser-induced choroidal neovascularization rat model.

### [Description of Embodiments]

The present disclosure is described hereinafter in more detail.

Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present disclosure pertains. In case of a contradiction, the present specification (including the definitions) takes precedence.

### (Definition)

The terms and the general technology used in the present disclosure are first described.

Since the compound of the present disclosure may be present in the form of a hydrate and/or a solvate, hydrates and/or solvates of the compound of the present disclosure or a pharmaceutically acceptable salt thereof are also encompassed by the compounds of the present disclosure.

Since the compound of the present disclosure may have one, or optionally one or more, asymmetric carbon atom(s), and may result in geometrical isomerism or axial chirality, it may be present as several types of stereoisomers. In the present disclosure, these stereoisomers, and mixtures and racemates thereof are also encompassed by the compounds of the present disclosure. Therefore, the compounds described herein may contain one or more asymmetric centers and therefore may exist in various isomeric forms, such as enantiomers and/or diastereomers. For example, the compounds described herein may be in the form of individual enantiomers, diastereomers, or geometric isomers, or may be in the form of a mixture of stereoisomers (including racemic mixtures and mixtures enriched in one or more stereoisomers). The present disclosure additionally encompasses the compounds described herein as individual isomers substantially free of other isomers, and alternatively as mixtures of various isomers.

Further, a deuterated form in which any one or two or more ¹H of the compound of the present disclosure has been converted to ²H (D) is encompassed by the compound of the present disclosure.

The compound of the present disclosure or pharmaceutically acceptable salts thereof obtained as crystal may be present as crystalline polymorphism, and those in any crystalline forms are encompassed by the compounds of the present disclosure.

Next, the terms in the present specification are described below.

As used herein, the term "group" refers to a monovalent group, unless especially noted otherwise. Examples of a group that is not a monovalent group include alkylene group (divalent) and the like. The term "group" may also be abbreviated in the following description of substituents or the like.

As used herein, the number of substituents when a group is defined as "optionally substituted" or "substituted" is not particularly limited as long as it is substitutable and is one or more. The description for each group is also applicable when the substituent is a part of or a substituent on another substituent, unless specifically noted otherwise.

Substituent In the present disclosure include a hydrogen atom, a hydroxyl group, a carboxyl group, a sulfinic acid group, a sulfonic acid group, a phosphoric acid group, a guanidino group, a cyano group, a halogen atom (a fluorine atom, a chlorine atom, etc.), an alkyl group, an alkylthio group, a cycloalkylthio group, an alkenyl group, an alkynyl group, a cycloalkyl group, a cycloalkenyl group, a cycloalkylcarbonyl group, an alkylcarbonyloxy group, an alkylsulfinyl group, a cycloalkylsulfinyl group, an alkoxy group, a cycloalkoxy group, an alkoxycarbonyl group, a cycloalkyloxycarbonyl group, an alkylcarbonyl group, an aryl group, an arylcarbonyl group, an arylthio group, an aryloxycarbonyl group, a heteroaryl group, a heterocyclic group, an amino group, a cyclic amino group, an aminocarbonyl group, an aminosulfinyl group, an aminosulfonyl group, a heterocyclyloxycarbonyl group, heterocyclylsulfinyl group, a heterocyclylsulfonyl group, a heterocyclylcarbonyl group, an alkylsulfonyl group, a cycloalkylsulfonyl group, an arylsulfonyl group, an arylsulfinyl group, a heteroarylsulfonyl group, a heteroarylsulfinyl group, a triphenylphosphonium cation group. The above substituents may be further substituted with the above substituents.

As used herein, "maximum substitutable number" is the maximum number of substituents that a group can have. The number can vary for each group. For example, the number is 3 for a methyl group, 5 for an ethyl group, 7 for a benzyl group, and 11 for a naphthalenyl ethyl group.

For a group that is modified by "optionally substituted" or "substituted" herein, any portion of the group can be substituted. For example, "optionally substituted arylalkyl" and "substituted arylalkyl" can have the aryl moiety substituted, the alkyl moiety substituted, or both the aryl moiety and the alkyl moiety substituted.

As used herein, examples of substituents in the case of "optionally substituted" include substituent group α and substituent group β. Substituents in the case of "optionally substituted" may be selected from substituent group α and may be substituted with 1 to 5 of the same or different substituents. The type of atom within the substituent involved in the bonding is not particularly limited by the type of substituent, but when the atom to which the substituent bonds is an oxygen atom, a nitrogen atom, or a sulfur atom, the substituent is limited to the following substituents that bonds to a carbon atom.

Substituent group α includes
1) a halogen atom
2) a hydroxyl group
3) a carboxyl group
4) a cyano group
5) C₁₋₆ alkyl
6) C₂₋₆ alkenyl
7) C₂₋₆ alkynyl
8) C₁₋₆ alkoxy
9) C₁₋₆ alkylthio
10) C₁₋₆ alkylcarbonyl
11) C₁₋₆ alkylsulfonyl
(wherein each substituent from 5) to 11) is optionally substituent with 1 to 5 of the same or different substituents selected from substituent group β)
12) a C₃₋₁₀ cycloalkyl group
13) C₃₋₁₀ cycloalkyloxy
14) C₆₋₁₀ aryloxy
15) C₅₋₁₀ heteroaryloxy
16) C₄₋₁₀ non-aryl heterocyclyloxy
17) C₃₋₁₀ cycloalkylthio
18) C₆₋₁₀ arylthio
19) C₅₋₁₀ heteroarylthio
20) C₄₋₁₀ non-aryl heterocyclylthio
21) C₆₋₁₀ aryl
22) C₅₋₁₀ heteroaryl
23) C₄₋₁₀ non-aryl heterocycle
24) C₃₋₁₀ cycloalkylcarbonyl
25) C₆₋₁₀ arylcarbonyl
26) C₅₋₁₀ heteroarylcarbonyl
27) C₄₋₁₀ non-aryl heterocyclylcarbonyl
28) C₃₋₁₀ cycloalkylsulfonyl
29) C₆₋₁₀ arylsulfonyl
30) C₅₋₁₀ heteroarylsulfonyl
31) C₄₋₁₀ non-aryl heterocyclylsulfonyl
(wherein each substituent from 12) to 31) is optionally substituted with 1 to 5 of substituent group β or 5) C₁₋₆ alkyl above)
32) -NR^{10a}R^{11a}
33) -SO₂-NR^{10b}R^{11b}
34) -NR^{10c}-C(=O)R^{11c}
35) -NR^{10d}-C(=O)OR^{11d}
36) -NR^{12a}-C(=O)NR^{10e}R^{11e}
37) -NR¹⁰ⁱ-SO₂-R¹¹ⁱ
38) -NR^{12c}-SO₂-NR^{10j}R^{11j}
39) -C(=O)OR^{10k}
40) -C(=O)NR¹⁰¹R^{11k}
41) -C(=O)NR^{10m}OR¹¹¹
42) -C(=O)NR^{12d}-NR¹⁰ⁿR^{11m}
43) -C(=NR^{13a})R^{10s}
44) -C(=NR^{13c})NR^{10t}R^{11q}
45) -C(=NR^{13d})NR^{12f}-NR^{10u}R^{11r}
46) -NR^{17c}-C(=NR^{13k})R^{17d}
47) -NR^{12g}-C(=NR^{13e})-NR^{10v}R^{11s}
48) -NR¹⁴-C(=NR^{13f})-NR^{12h}-NR^{10w}R^{11t}
49) -OC(=O)R^{10x}
50) -OC(=O)OR^{10y}
51) -OC(=O)NR^{10z1}R^{11u}
52) -NR¹²ⁱ-NR^{10z2}R^{11v}
53) -NR^{10z3}OR^{11w}
54) a protecting group.

Substituent group β is a group consisting of
1) a halogen atom,
2) a hydroxyl group,
3) a carboxyl group,
4) a cyano group,
5) a C₃₋₁₀ cycloalkyl group,
6) C₁₋₆ alkoxy,
7) C₃₋₁₀ cycloalkyloxy,
8) C₁₋₆ alkylthio,
9) C₅₋₁₀ heteroarylthio,
10) C₆₋₁₀ aryl,
11) C₅₋₁₀ heteroaryl,
12) C₄₋₁₀ non-aryl heterocycle,
13) C₁₋₆ alkylcarbonyl,
14) C₃₋₁₀ cycloalkylcarbonyl,
15) C₆₋₁₀ arylcarbonyl,
16) C₅₋₁₀ heteroarylcarbonyl,
17) C₄₋₁₀ non-aryl heterocyclylcarbonyl,
18) -NR^{15a}R^{16a},
19) -SO₂-NR^{15b}R^{16b},
20) -NR^{15c}-C(=O)R^{16c}
21) -NR^{17a}-C(=O)NR^{15d}R^{16d}_{,}
22) -C(=O)NR^{15e}R^{16e},
23) -C(=NR^{13g})R^{15f},
24) -C(=NR^{13h})NR^{15g}R^{16f}
25) -NR^{16g}-C(=NR¹³ⁱ) R^{15h}
26) -NR^{17b}-C(=NR^{13j})-NR¹⁵ⁱR^{16h}
27) a protecting group
(wherein each substituent from 5) to 17) in substituent group β is optionally substituted with 1 to 5 substituents selected from the group consisting of a halogen atom, a hydroxyl group, a cyano group, a carboxyl group, and -NR^{18a}R^{18b}),
R13a, R^{13a2}, R^{13c}, R^{13c2}, R^{13d}, R13d2, R13e, R13f, R^{13 g}, R^{13g2}, R^{13h}, R^{13h2}, R¹³ⁱ, R^{13j}, R^{13k} are the same or different, each independently a hydrogen atom, a hydroxyl group, C₁₋₆ alkyl, C₁₋₆ alkoxy, or C₁₋₆ alkoxycarbonyl,
R^{10a}, R^{10b}, R^{10c}, R^{10d}, R^{10e}, R¹⁰ⁱ, R^{10j}, R^{10k}, R^{10l}, R^{10m}, R¹⁰ⁿ, R^{10s}, R^{10s2}, R^{10t}, R^{10t2}, R^{10u}, R^{10u2}, R^{10v}, R^{10w}, R^{10x}, R^{10y}, R^{10z1}, R^{10z2}, R^{10z3}, R^{11a}, R^{11b}, R^{11c}, R^{11d}, R^{11e}, R¹¹ⁱ, R^{11j}, R^{11k}, R^{11l}, R^{11m}, R^{11q}, R^{11q2}, R^{11r}, R^{11r2}, R^{11s}, R^{11t}, R^{11u}, R^{11v}, R^{11w}, R^{12a}, R^{12c}, R^{12d}, R12f, R^{12f2}, R^{12 g}, R^{12h}, R¹²ⁱ, R¹⁴, R^{15a}, R^{15b}, R^{15c}, R^{15d}, R^{15e}, R^{15f}, R^{15f2}, R^{15 g}, R^{15g2}, R^{15h}, R¹⁵ⁱ, R^{16a}, R^{16b}, R^{16c}, R^{16d}*,* R^{16e}, R^{16f}, R^{16f2}, R^{16 g}, R^{16h}, R^{17a}, R^{17b}, R^{17c}, R^{17d} are the same or different, each independently a hydrogen atom, C₁₋₆ alkyl (the C₁₋₆ alkyl is optionally substituted with 1 to 3 of the same or different substituents selected from a hydroxyl group, a cyano group, C₁₋₆ alkoxy and -NR^{18a}R^{18b}), or C₁₋₆ alkoxycarbonyl, and
R^{18a} and R^{18b} are the same or different, each independently a hydrogen atom or C₁₋₆ alkyl.

In an exemplary embodiment, any hydrogen atom of a hydroxyl group and an amino group in the substituent groups α and β may be substituted with a protecting group.

As used herein, "C₁₋₆" means that the number of carbon atoms is 1 to 6. The same applies to other numbers, for example, "C₁₋₄" means that the number of carbon atoms is 1 to 4, and "C₁₋₃" means that the number of carbon atoms is 1 to 3. A description with a limitation in the number of carbons herein is only a preferred numerical range, and it is intended so that groups with a substituent with a number of carbons other than the number of carbons specified in the present disclosure are also within the scope of the present disclosure.

As used herein, "hydrocarbon group" is also referred to as a "hydrocarbyl group", referring to a group generated by removing at least one hydrogen from "hydrocarbon" comprising at least one carbon and at least one hydrogen.

As used herein, "functional group" refers to any group conferring some type of functionality, encompassing a carboxyl group, nitrile group, carbonyl group, hydroxyl group, amino group, imino group, nitro group, halogen group, as well as alkyl group, and more broadly acid anhydrides and groups formed by a bond such as an ester bond, amide bond, or ether bond.

As used herein, "heteroatom" refers to atoms other than carbon atoms and hydrogen atoms such as oxygen atoms, nitrogen atoms, and sulfur atoms. A group comprising a heteroatom is also known as a hetero... group (e.g., heteroaryl group (means that an aryl group comprises at least a heteroatom), a heterocyclic... group (e.g., a heterocyclic group (means that a cyclic group (carbon ring group) comprises at least one heteroatom)) or the like.

As used herein, "halogen atom" is an atom belonging to the halogen group, referring to a fluorine atom, chlorine atom, bromine atom, iodine atom, or the like. The halogen atom is preferably a fluorine atom or chlorine atom. The halogen atom is more preferably a fluorine atom. A "halogen atom" is also referred to as "halogen" or "halo".

As used herein, "hydroxyl group" is a monovalent group of -OH. This group is also referred to as a "hydroxy group" or "hydroxy".

As used herein, "carboxyl group" is a monovalent group of -COOH. This group is also referred to as a "carboxy group", "carboxy", or "carboxyl".

As used herein, "amino" is a monovalent group of -NH₂. This group is also referred to as an "amino group".

As used herein, "thio" is a monovalent group of -SH. This group is also referred to as a "thio group".

As used herein, "cyano group" is a monovalent group of - CN.

As used herein, "alkyl" refers to a linear or branched saturated aliphatic hydrocarbon group. "C₁₋₁₂ alkyl" is an alkyl group with 1 to 12 carbon atoms. Examples thereof include, but are not limited to, C₁₋₆ alkyl, heptyl, iso-heptyl, octyl, iso-octyl, nonyl, iso-nonyl, decyl, iso-decyl, undecyl, iso-undecyl, dodecyl, iso-dodecyl, and the like. "C₁₋₆ alkyl" is an alkyl group with 1 to 6 carbon atoms, which is preferably "C₁₋₄ alkyl", more preferably "C₁₋₃ alkyl", and still more preferably "C₁₋₂ alkyl". Specific examples of "C₁₋₄ alkyl" include methyl, ethyl, propyl, isopropyl, n-butyl, isobutyl, tert-butyl, sec-butyl, and the like. Specific examples of "C₁₋₆ alkyl" include, but are not limited to, C₁₋₄ alkyl, n-pentyl, isopentyl, neopentyl, tert-pentyl, 1,2-dimethylpropyl, n-hexyl, and the like.

As used herein, "alkenyl" refers to a linear or branched unsaturated aliphatic hydrocarbon group comprising at least one carbon-carbon double bond. "C₂₋₁₂ alkenyl" is an alkenyl group with 2 to 12 carbon atoms. Examples thereof include, but are not limited to, heptenyl, isoheptenyl, octenyl, isooctenyl, nonenyl, isononenyl, decenyl, isodecenyl, undecenyl, isoundecenyl, dodecenyl, isododecenyl, and the like. "C₂₋₆ alkenyl" is an alkenyl group with 2 to 6 carbon atoms. Preferred examples thereof include "C₂₋₄ alkenyl". Specific examples of "C₂₋₆ alkenyl" include, but are not limited to, vinyl, 1-propenyl, 2-propenyl, 1-butenyl, 2-butenyl, 3-butenyl, 2-methyl-1-propenyl, 2-methyl-2-propenyl, and the like.

As used herein, "alkynyl" refers to a linear or branched unsaturated aliphatic hydrocarbon group comprising at least one carbon-carbon triple bond. "C₂₋₁₂ alkynyl" is an alkynyl group with 2 to 12 carbon atoms. Examples thereof include, but are not limited to, heptynyl, isoheptynyl, octynyl, isooctynyl, nonynyl, isononynyl, decynyl, isodecynyl, undecynyl, isoundecynyl, dodecynyl, isododecynyl, and the like. "C₂₋₆ alkynyl" is an alkynyl group with 2 to 6 carbon atoms. Preferred examples thereof include "C₂₋₄ alkynyl". Specific examples of "C₂₋₆ alkynyl" include, but are not limited to, ethynyl, 1-propynyl, 2-propynyl, 1-butynyl, 1-methyl-2-propynyl, 3-butynyl, 1-pentynyl, 1-hexynyl, and the like.

As used herein, "aryl" refers to a monovalent group of a monocyclic or bicyclic aromatic hydrocarbon ring. "C₆₋₁₀ aryl" refers to an aryl group with 6 to 10 carbon atoms. Examples of "aryl" include, but are not limited to, C₆ aryl, C₁₀ aryl, and the like. Specific examples of C₆ aryl include, but are not limited to, phenyl and the like. Specific examples of C₁₀ aryl include, but are not limited to, 1-naphthyl, 2-naphthyl, and the like.

An aryl group as a substituent or a portion thereof may be fused to an alicyclic group. For example, a phenyl group may be fused to a cyclohexane ring to form a 1,2,3,4-tetrahydronaphthalenyl group. In such a case, one of the possible carbon atoms on a benzene ring attaches to the backbone, or to a group near the backbone, or to its atom. An aryl group encompasses 5,6,7,8-tetrahydronaphthalen-1-yl and 5,6,7,8-tetrahydronaphthalen-2-yl.

As used herein, "arylalkyl" refers to alkyl substituted with at least one aryl. "C₆₋₁₀ aryl C₁₋₆ alkyl" refers to C₁₋₆ alkyl substituted with at least one C₆₋₁₀ aryl. Specific examples of C₆₋₁₀ aryl C₁₋₆ alkyl include, but are not limited to, benzyl (phenyl-CH₂-), phenethyl (phenyl-CH₂CH₂-), naphthalen-1-ylmethyl, naphthalen-2-ylmethyl, 2-(naphthalen-1-yl)ethyl, 2-(naphthalen-2-yl)ethyl, and the like.

As used herein, "(optionally substituted amino)-arylalkyl" refers to arylalkyl substituted with an optionally substituted amino group, wherein the alkyl group, the aryl group, or both is substituted with an amino group. An amino group of such an arylalkyl group may be unsubstituted, or substituted with 1, 2, or 3 substituents, such as optionally substituted alkyl (e.g., unsubstituted C₁₋₆ alkyl, C₃₋₆ cycloalkyl-C₁₋₆ alkyl, C₃₋₆ cycloalkylcarbonyl, or the like). Examples of (optionally substituted amino) -C₆₋₁₀ aryl C₁₋₆ alkyl include, but are not limited to, (di(alkyl)amino)benzyl, ((cycloalkylalkyl)amino)benzyl, ((cycloalkylcarbonyl)amino)benzyl, ((carbamoylalkyl)carbonylamino)benzyl, ((carboxyalkyl)carbonyl)aminobenzyl, (di(alkyl)amino)naphthalenylmethyl, ((cycloalkylalkyl)amino)naphthalenylmethyl, ((cycloalkylcarbonyl)amino)naphthalenylmethyl, ((carbamoylalkyl)carbonylamino)naphthalenylmethyl, ((carboxyalkyl)carbonyl)aminonaphthalenylmethyl, and the like.

As used herein, "hydroxyaryl" refers to aryl substituted with at least one hydroxy. "Hydroxy C₆₋₁₀ aryl" refers to C₆₋₁₀ aryl substituted with at least one hydroxy. Specific examples of hydroxy C₆₋₁₀ aryl include, but are not limited to, 2-hydroxyphenyl, 3-hydroxynaphthalene, and the like.

As used herein, "hydroxyarylalkyl" refers to alkyl substituted with at least one hydroxyaryl. "Hydroxy C₆₋₁₀ aryl C₁₋₆ alkyl" refers to C₁₋₆ alkyl substituted with at least one hydroxy C₆₋₁₀ aryl. Specific examples of hydroxy C₆₋₁₀ aryl C₁₋₆ alkyl include, but are not limited to, 2-hydroxybenzyl (2-hydroxyphenyl-CH₂-), 2-hydroxyphenethyl (2-hydroxyphenyl-CH₂CH₂-), 3-hydroxynaphthalen-1-ylmethyl, 3-hydroxynaphthalen-2-ylmethyl, 2-(3-hydroxynaphthalen-1-yl)ethyl, 2-(3-hydroxynaphthalen-2-yl)ethyl, and the like.

As used herein, the aryl moiety of "arylthio" has the same meaning as the aryl described above. Preferred examples of "C₆₋₁₀ arylthio" include "C₆ or C₁₀ arylthio". Specific examples of "C₆₋₁₀ arylthio" include, but are not limited to, phenylthio, 1-naphthylthio, 2-naphthylthio, and the like.

As used herein, "aryl sulfonyl" refers to sulfonyl substituted with the "aryl" described above. "C₆₋₁₀) aryl sulfonyl" is preferably "C₆ or C₁₀ aryl sulfonyl". Specific examples of "C₆₋₁₀ aryl sulfonyl" include, but are not limited to, phenylsulfonyl, 1-naphthylsulfonyl, 2-naphthylsulfonyl, and the like.

As used herein, "heteroaryl" refers to a monovalent group of a monocyclic or bicyclic aromatic heterocycle comprising the same or different heteroatoms selected from the group consisting of an oxygen atom, nitrogen atom, and sulfur atom. The number of heteroatoms may be any number up to the number of carbons in aryl, and for example, the number of heteroatoms is typically 1 to 4, and may be 1, 2, 3, or the like.

As used herein, "C₅₋₁₀ heteroaryl" refers to a monovalent group of a monocyclic or bicyclic aromatic heterocycle comprising 5 to 10 atoms comprising typically 1 to 4 same or different heteroatoms selected from the group consisting of an oxygen atom, nitrogen atom, and sulfur atom. Specific examples of "C₅₋₁₀ heteroaryl" include, but are not limited to, quinolyl, isoquinolyl, naphthyridinyl, quinoxalinyl, cinnolinyl, quinazolinyl, phthalazinyl, imidazopyridyl, imidazothiazolyl, imidazooxazolyl, benzothiazolyl, benzoxazolyl, benzoimidazolyl, indolyl, isoindolyl, indazolyl, pyrrolopyridyl, thienopyridyl, furopyridyl, benzothiadiazolyl, benzoxadiazolyl, pyridopyrimidinyl, benzofuryl, benzothienyl, benzo[1,3]dioxole, thienofuryl, chromenyl, chromanyl, coumarinyl, quinolonyl, and the like.

As used herein, "heteroarylalkyl" refers to alkyl substituted with at least one heteroaryl. "C₅₋₁₀ heteroaryl C₁₋₆ alkyl" refers to C₁₋₆ alkyl substituted with at least one C₅₋₁₀ heteroaryl. Specific examples of C₅₋₁₀ heteroaryl C₁₋₆ alkyl include, but are not limited to, pyridin-2-ylmethyl, pyridin-4-ylmethyl, 2-(quinolin-8-yl)ethyl, 2-(quinolin-5-yl)ethyl, 2-(quinoxalin-5-yl)ethyl, 2-(1H-indol-3-yl)ethyl, and the like.

As used herein, "cycloalkyl" refers to a non-aromatic saturated hydrocarbon ring group, including those that have a partially crosslinked structure, those that are partially spiro, those having 1, 2, or more carbonyl structures. "C₃₋₂₀ cycloalkyl" refers to monocyclic or bicyclic cycloalkyl with 3 to 20 carbon atoms. "C₃₋₆ cycloalkyl" refers to monocyclic cycloalkyl with 3 to 6 carbon atoms. Specific examples of C₃₋₆ cycloalkyl include, but are not limited to, cyclopropyl, cyclobutyl, cyclopentyl, cyclohexyl, and the like.

A cycloalkyl group can be fused to aryl and/or heteroaryl ring as a substituent or a portion thereof. For example, a cyclohexyl group can be fused to a benzene ring to form a 1,2,3,4-tetrahydronaphthalenyl group. In such a case, one of the possible carbon atoms on the cyclohexane ring attaches to the backbone, to a group near the backbone, or to its atom. A cycloalkyl group encompasses 1,2,3,4-tetrahydronaphthalen-1-yl, 1,2,3,4-tetrahydronaphthalen-2-yl, indan-1-yl, indan-2-yl, 5,6,7,8-tetrahydroquinolin-5-yl, and 5,6,7,8-tetrahydroquinolin-6-yl.

As used herein, "cycloalkylalkyl" refers to alkyl substituted with at least one cycloalkyl. "C₃₋₆ cycloalkyl C₁₋₆ alkyl" refers to C₁₋₆ alkyl substituted with at least one C₃₋₆ cycloalkyl. Specific examples of C₃₋₆ cycloalkyl C₁₋₆ alkyl include, but are not limited to, cyclopropylmethyl, cyclobutylmethyl, cyclopentylmethyl, cyclohexylmethyl, 2-cyclopropylethyl, 2-cyclobutylethyl, 2-cyclopentylethyl, 2-cyclohexylethyl, 3-cyclopropylpropyl, 3-cyclobutylpropyl, 3-cyclopentylpropyl, 3-cyclohexylpropyl, and the like.

As used herein, "heterocycloalkyl" refers to a non-aromatic saturated or partially unsaturated heterocycle comprised of 3 or more atoms, comprising 1, 2, or more same or different heteroatoms selected from the group consisting of an oxygen atom, nitrogen atom, and sulfur atom, including those that have a partially crosslinked structure and those that are partially spiro. "Heterocycloalkyl" encompasses "non-aryl heterocycle". Heterocycloalkyl can have a structure where a non-aromatic heterocycle is fused to an aryl ring and/or heteroaryl ring.

As used herein, "non-aryl heterocycle" refers to a monocyclic or bicyclic non-aromatic heterocycle comprised of 3 or more atoms, comprising 1, 2, or more same or different heteroatoms selected from the group consisting of an oxygen atom, nitrogen atom, and sulfur atom, including saturated non-aryl heterocycles, those that have a partially unsaturated attachment, those that have a partially crosslinked structure, and those that are partially spiro. A non-aryl heterocycle can form a fused ring with aryl or heteroaryl. For example, a non-aryl heterocycle fused to C₆₋₁₀ aryl or C₅₋₁₀ heteroaryl is also encompassed by a heterocycle. 1, 2, or more carbonyl, thiocarbonyl, sulfinyl, or sulfonyl can be comprised to constitute the non-aryl heterocycle. For example, lactam, thiolactam, lactone, thiolactone, cyclic imide, cyclic carbamate, cyclic thiocarbamate, and other cyclic groups are also encompassed by the non-aryl heterocycle. In this regard, an oxygen atom of carbonyl, sulfinyl, and sulfonyl and a sulfur atom of thiocarbonyl are not included in the number of members of the ring (ring size) or the number of heteroatoms constituting the ring.

As used herein, "C₄₋₁₀ non-aryl heterocycle" refers to a substituent with "C₄₋₁₀ non-aryl heterocycle" that is a monovalent group among the "non-aryl heterocycle" described above.

As used herein, the non-aryl heterocycle moiety of "non-aryl heterocyclyloxy" has the same meaning as the "non-aryl heterocycle" described above. Examples thereof include "C₄₋₁₀ non-aryl heterocyclyloxy", and "C₄₋₁₀ non-aryl heterocyclyloxy" is preferably "C₄₋₁₀ non-aryl heterocyclyloxy". Specific examples of "C₄₋₁₀ non-aryl heterocyclyloxy" include, but are not limited to, tetrahydrofuranyloxy, tetrahydropyranyloxy, azetidinyloxy, pyrrolidinyloxy, piperidinyloxy, and the like.

As used herein, the non-aryl heterocycle moiety of "non-aryl heterocyclylthio" has the same meaning as the "non-aryl heterocycle" described above. Examples thereof include "C₄₋₁₀ non-aryl heterocyclylthio", and "C₄₋₁₀ non-aryl heterocyclylthio" is preferably "C₄₋₆ non-aryl heterocyclylthio". Specific examples of "C₄₋₁₀ non-aryl heterocyclylthio" include, but are not limited to, tetrahydropyranylthio, piperidinylthio, and the like.

As used herein, "non-aryl heterocyclylcarbonyl" refers to a carbonyl group substituted with the "non-aryl heterocycle" described above. Examples thereof include "C₄₋₁₀ non-aryl heterocyclylcarbonyl", and "C₄₋₁₀ non-aryl heterocyclylcarbonyl" is preferably "C₄₋₆ non-aryl heterocyclylcarbonyl". Specific examples of "C₄₋₁₀ non-aryl heterocyclylcarbonyl" include, but are not limited to, azetidinylcarbonyl, pyrrolidinylcarbonyl, piperidinylcarbonyl, morpholinylcarbonyl, and the like.

As used herein, "non-aryl heterocyclylsulfonyl" refers to a sulfonyl group substituted with the "non-aryl heterocycle" described above. Examples thereof include "C₄₋₁₀ non-aryl heterocyclylsulfonyl", and "C₄₋₁₀ non-aryl heterocyclylsulfonyl" is preferably "C₄₋₆ non-aryl heterocyclylsulfonyl". Specific examples of "C₄₋₁₀ non-aryl heterocyclylsulfonyl" include, but are not limited to, azetidinylsulfonyl, pyrrolidinylsulfonyl, piperidinylsulfonyl, morpholinylsulfonyl, and the like.

As used herein, "C₅₋₁₀ heterocycloalkyl" refers to heterocycloalkyl comprised of 5 to 10 cyclic atoms, comprising 1, 2, or more same or different heteroatoms selected from the group consisting of an oxygen atom, nitrogen atom, and sulfur atom.

As used herein, "heterocycloalkylalkyl" refers to alkyl substituted with at least one heterocycloalkyl.

As used herein, "alkylcarbonyl" is a monovalent group of -C(=O)-alkyl. Preferred examples of alkylcarbonyl include C₁₋₆ alkylcarbonyl. Specific examples of C₁₋₆ alkylcarbonyl include, but are not limited to, acetyl (CH₃C(=O)-), n-propanoyl (CH₃CH₂C(=O)-) , n-butanoyl (CH₃CH₂CH₂C(=O)-), n-pentanoyl (CH₃(CH₂)₃C(=O)-), n-hexanoyl (CH₃(CH₂)₄C(=O)-), n-heptanoyl (CH₃(CH₂)₅C(=O)-), and the like.

As used herein, "alkoxy" is a monovalent group of -O-alkyl. Preferred examples of alkoxy include C₁₋₆ alkoxy (i.e., C₁₋₆ alkyl-O-), C₁₋₄ alkoxy (i.e., C₁₋₄ alkyl-O-), and the like. Specific examples of C₁₋₄ alkoxy include methoxy (CH₃O-), ethoxy (CH₃CH₂O-), n-propoxy (CH₃(CH₂)₂O-), isopropoxy ((CH₃)₂CHO-), n-butoxy (CH₃(CH₂)₃O-), isobutoxy ((CH₃)₂CHCH₂O-), tert-butoxy ((CH₃)₃CO-), sec-butoxy (CH₃CH₂CH(CH₃)O-), and the like. Specific examples of C₁₋₆ alkoxy include, but are not limited to, C₁₋₄ alkoxy, n-pentyloxy (CH₃(CH₂)₄O-), isopentyloxy ((CH₃)₂CHCH₂CH₂O-), neopentyloxy ((CH₃)₃CCH₂O-), tert-pentyloxy (CH₃CH₂C(CH₃)₂O-), 1,2-dimethylpropoxy (CH₃CH(CH₃)CH(CH₃)O-), and the like.

As used herein, "alkoxycarbonyl" is a monovalent group of -C(=O)-O-alkyl. Examples of alkoxycarbonyl include, but are not limited to, C₁₋₆ alkoxycarbonyl, preferably C₁₋₄ alkoxycarbonyl. Specific examples of C₁₋₄ alkoxycarbonyl include methoxycarbonyl, ethoxycarbonyl, n-propoxycarbonyl, isopropoxycarbonyl, n-butoxycarbonyl, sec-butoxycarbonyl, tert-butoxycarbonyl, isobutoxycarbonyl, and the like. Specific examples of C₁₋₆ alkoxycarbonyl include, but are not limited to, C₁₋₄ alkoxycarbonyl, n-pentyloxycarbonyl, isopentyloxycarbonyl, neopentyloxycarbonyl, tert-pentyloxycarbonyl, 1,2-dimethylpropyloxycarbonyl, n-hexyloxycarbonyl, and the like.

As used herein, "alkoxycarbonyloxy" is a monovalent group of -O-C(=O)-O-alkyl. Examples of alkoxycarbonyloxy include, but are not limited to, C₁₋₆ alkoxycarbonyloxy, preferably C₁₋₄ alkoxycarbonyloxy. Specific examples of C₁₋₄ alkoxycarbonyloxy include methoxycarbonyloxy, ethoxycarbonyloxy, n-propoxycarbonyloxy, isopropoxycarbonyloxy, n-butoxycarbonyloxy, sec-butoxycarbonyloxy, tert-butoxycarbonyloxy, isobutoxycarbonyloxy, and the like. Specific examples of C₁₋₆ alkoxycarbonyloxy include, but are not limited to, C₁₋₄ alkoxycarbonyloxy, n-pentyloxycarbonyloxy, isopentyloxycarbonyloxy, neopentyloxycarbonyloxy, tert-pentyloxycarbonyloxy, 1,2-dimethylpropyloxycarbonyloxy, n-hexyloxycarbonyloxy, and the like.

As used herein, "alkoxycarbonylamino" is a monovalent group of -NH-C(=O)-O-alkyl. Examples of alkoxycarbonylamino include, but are not limited to, C₁₋₆ alkoxycarbonylamino, preferably C₁₋₄ alkoxycarbonylamino. Specific examples of C₁₋₄ alkoxycarbonylamino include methoxycarbonylamino, ethoxycarbonylamino, n-propoxycarbonylamino, isopropoxycarbonylamino, n-butoxycarbonylamino, sec-butoxycarbonylamino, tert-butoxycarbonylamino, isobutoxycarbonylamino, and the like. Specific examples of C₁₋₆ alkoxycarbonylamino include, but are not limited to, C₁₋₄ alkoxycarbonylamino, n-pentyloxycarbonylamino, isopentyloxycarbonylamino, neopentyloxycarbonylamino, tert-pentyloxycarbonylamino, 1,2-dimethylpropyloxycarbonylamino, n-hexyloxycarbonylamino, and the like.

As used herein, "haloalkyl" is a monovalent group of halogenated alkyl, having one or more hydrogen on an alkyl group substituted with halogen. The term "perhaloalkyl" refers to haloalkyl with all hydrogen on the alkyl group substituted with halogen. For example, perfluoroethyl is -CF₂CF₃, and perchloro-n-propyl is -CCl₂CCl₂CCl₃. Examples of haloalkyl include C₁₋₆ haloalkyl, C₁₋₄ haloalkyl, C₁₋₃ haloalkyl, and the like. Specific examples of C₁₋₃ alkyl include, but are not limited to, fluoromethyl, chloromethyl, bromomethyl, difluoromethyl, dichloromethyl, dibromomethyl, trifluoromethyl, trichloromethyl, tribromomethyl, fluorochloromethyl, difluorochloromethyl, fluorodichloromethyl, fluoroethyl, chloroethyl, bromoethyl, trifluoroethyl, trichloroethyl, tribromoethyl, perfluoroethyl, perchloroethyl, perbromoethyl, perfluoropropyl, perchloropropyl, perbromopropyl, perfluoroisopropyl, perchloroisopropyl, perbromoisopropyl, and the like. Specific examples of C₁₋₄ alkyl include, but are not limited to, C₁₋₃ haloalkyl, perfluorobutyl, perchlorobutyl, perbromobutyl, perfluoroisobutyl, perfluoro-t-butyl, and the like. Specific examples of C₁₋₆ alkyl include, but are not limited to, C₁₋₄ haloalkyl, perfluoro-n-pentyl, perfluoroisopentyl, perfluoroneopentyl, perfluoro-tert-pentyl, perfluoro-1,2-dimethylpropyl, and the like.

As used herein, "haloalkoxy" as well as "haloalkyloxy" is a monovalent group of -O-haloalkyl with one or more hydrogen on the alkyl group substituted with halogen. The term "perhaloalkoxy" refers to haloalkoxy with all hydrogen on the alkyl group substituted with halogen. For example, perfluoroethoxy is -OCF₂CF₃, and perchloro-n-propoxy is - OCCl₂CCl₂CCl₃. Preferred examples of haloalkoxy include C₁₋₆ haloalkoxy, C₁₋₄ haloalkoxy, C₁₋₃ haloalkoxy, and the like. Specific examples of C₁₋₃ alkoxy include, but are not limited to, fluoromethoxy, chloromethoxy, bromomethoxy, difluoromethoxy, dichloromethoxy, dibromomethoxy, trifluoromethoxy, trichloromethoxy, tribromomethoxy, fluorochloromethoxy, difluorochloromethoxy, fluorodichloromethoxy, fluoroethoxy, chloroethoxy, bromoethoxy, trifluoroethoxy, trichloroethoxy, tribromoethoxy, perfluoroethoxy, perchloroethoxy, perbromoethoxy, perfluoropropoxy, perchloropropoxy, perbromopropoxy, perfluoroisopropoxy, perchloroisopropoxy, perbromoisopropoxy, and the like. Specific examples of C₁₋₄ alkoxy include, but are not limited to, C₁₋₃ haloalkoxy, perfluorobutoxy, perchlorobutoxy, perbromobutoxy, perfluoroisobutoxy, perfluoro-t-butoxy, and the like. Specific examples of C₁₋₆ alkoxy include, but are not limited to, C₁₋₄ haloalkoxy, perfluoro-n-pentyloxy, perfluoroisopentyloxy, perfluoroneopentyloxy, perfluoro-tert-pentyloxy, perfluoro-1,2-dimethylpropoxy, and the like.

As used herein, "alkylsulfonyl" refers to a sulfonyl group substituted with the "alkyl" described above. "C₁₋₆ alkylsulfonyl" is preferably "C₁₋₄ alkylsulfonyl". Specific examples of "C₁₋₆ alkylsulfonyl" include, but are not limited to, methylsulfonyl, propionylsulfonyl, butyrylsulfonyl, and the like.

As used herein, the alkyl moiety of "alkylthio" has the same meaning as the alkyl described above. Examples of "C₁₋₆ alkylthio" include "C₁₋₄ alkylthio", and preferred examples thereof include "C₁₋₃ alkylthio". Specific examples of "C₁₋₆ alkylthio" include, but are not limited to, methylthio, ethylthio, propylthio, butylthio, isopropylthio, isobutylthio, tert-butylthio, sec-butylthio, isopentylthio, neopentylthio, tert-pentylthio, 1,2-dimethylpropylthio, and the like.

As used herein, "arylcarbonyl" is a monovalent group of - C(=O)-aryl. Preferred examples of arylcarbonyl include C₆₋₁₀ arylcarbonyl. Specific examples of C₆₋₁₀ arylcarbonyl include, but are not limited to, benzoyl (i.e., phenyl-C(=O)-), 1-naphthylcarbonyl, 2-naphthylcarbonyl, and the like.

As used herein, the aryl moiety of "aryloxy" has the same meaning as the aryl described above. Preferred examples of "C₆₋₁₀ aryloxy" include "C₆ or C₁₀ aryloxy". Specific examples of "C₆₋₁₀ aryloxy group" include, but are not limited to, a phenoxy group, 1-naphthyloxy group, 2-naphthyloxy group, and the like.

As used herein, "heteroarylcarbonyl" is a monovalent group of -C(=O)-heteroaryl.

As used herein, "heteroarylcarbonyl group" refers to a carbonyl group substituted with the "heteroaryl" described above. Specific examples of "C₅₋₁₀ heteroarylcarbonyl group" include, but are not limited to, pyrazoylcarbonyl group, triazoylcarbonyl group, thiazoylcarbonyl group, thiadiazoylcarbonyl group, pyridylcarbonyl group, pyridazoylcarbonyl group, and the like.

As used herein, the heteroaryl moiety of the "heteroaryloxy group" has the same meaning as the "heteroaryl" described above. Specific examples of "C₅₋₁₀ heteroaryloxy group" include, but are not limited to, a pyrazoyloxy group, triazoyloxy group, thiazoyloxy group, thiadiazoyloxy group, pyridyloxy group, pyridazoyloxy group, and the like.

As used herein, the heteroaryl moiety of the "heteroarylthio group" has the same meaning as the "heteroaryl" described above. Specific examples of "C₅₋₁₀ heteroarylthio group" include, but are not limited to, a pyrazoylthio group, triazoylthio group, thiazoylthio group, thiadiazoylthio group, pyridylthio group, pyridazoylthio group, and the like.

As used herein, "optionally substituted carbonyl" group refers to a monovalent group of -C(=O)- (hydrogen or any group selected from a substituent group described herein). Examples of "optionally substituted carbonyl" group include, but are not limited to, formyl, and optionally substituted carbamoyl, alkylcarbonyl, alkoxycarbonyl, alkenylcarbonyl, alkenyloxycarbonyl, alkynylcarbonyl, alkynyloxycarbonyl, arylcarbonyl, aryloxycarbonyl, cycloalkylcarbonyl, cycloalkyloxycarbonyl, heteroarylcarbonyl, heteroaryloxycarbonyl, heterocycloalkylcarbonyl, heterocycloalkyloxycarbonyl, and the like. A carbonyl group substituted with hydrogen is a formyl group. A carbonyl group substituted with amino is a carbamoyl group.

As used herein, "optionally substituted oxy" group refers to a monovalent group of -O- (hydrogen or any group selected from a substituent group described herein). Examples of "optionally substituted oxy" group include, but are not limited to, hydroxy, and optionally substituted alkyloxy, alkenyloxy, alkynyloxy, aryloxy, heteroaryloxy, heterocycloalkyloxy, alkylcarbonyloxy, alkenylcarbonyloxy, alkynylcarbonyloxy, arylcarbonyloxy, heteroarylcarbonyloxy, heterocycloalkylcarbonyloxy, and the like. An oxy group substituted with hydrogen is a hydroxy group.

As used herein, "carbamoyl" is a monovalent group of - C(=O)-NH₂.

As used herein, "amidinoamino" is a monovalent group of - NH-C(=NH)-NH₂.

As used herein, the phrase "a substituent-substituted group" means that the group is substituted with at least one substituent. For example, "hydroxy-substituted C₁₋₆ alkyl" refers to C₁₋₆ alkyl that has at least one hydroxy substitution.

As used herein, "carbamoyl-substituted C₁₋₆ alkyl" is C₁₋₆ alkyl substituted with at least one -C(=O)-NH₂ group. Specific examples of "carbamoyl-substituted C₁₋₄ alkyl" include, but are not limited to, 2-amino-2-oxoethyl (i.e., H₂NC(=O)-CH₂- or carbamoylmethyl), 3-amino-3-oxopropyl (i.e., H₂NC(=O)-CH₂CH₂- or carbamoylethyl), 4-amino-4-oxobutyl (i.e., H₂NC(=O)-(CH₂)₃- or carbamoylpropyl), 5-amino-5-oxopentyl (i.e., H₂NC(=O)-(CH₂)₄- or carbamoylbutyl), and the like. Specific examples of "carbamoyl-substituted C₁₋₆ alkyl" include, but are not limited to, carbamoyl-substituted C₁₋₄ alkyl, 6-amino-6-oxohexyl (i.e., H₂NC(=O)-(CH₂)₃- or carbamoylpentyl), 7-amino-7-oxoheptyl (i.e., H₂NC(=O)-(CH₂)₆- or carbamoylhexyl), and the like.

As used herein, "thioalkyl" is alkyl substituted with at least one thio group. Specific examples of "thio C₁₋₆ alkyl" include, but are not limited to, thiomethyl, 2-thioethyl, 3-thiopropyl, 4-thiobutyl, and the like.

As used herein, "alkylthioalkyl" refers to alkyl substituted with at least one alkylthio. "C₁₋₆ alkylthio C₁₋₆ alkyl" refers to C₁₋₆ alkyl substituted with at least one C₁₋₆ alkylthio. Specific examples of C₁₋₆ alkylthio C₁₋₆ alkyl include, but are not limited to, methylthiomethyl, methylthioethyl, ethylthiomethyl, and the like.

As used herein, "aminoalkyl" is alkyl substituted with at least one amino group. Specific examples of "amino C₁₋₆ alkyl" include, but are not limited to, amimethyl, 2-aminoethyl, 3-aminopropyl, 4-aminobutyl, and the like.

As used herein, "amidinoaminoalkyl" or "guanidinoalkyl" is an alkyl substituted with at least one -NH-C(=NH)-NH₂ group, wherein the nitrogen atom of the amidinoamino group may be protected with a nitrogen protecting group (for example, a tert-butoxycarbonyl group). Examples of "amidinoamino C₁₋₆ alkyl" include, but are not limited to, "amidinoamino C₁₋₄ alkyl" and the like. Specific examples of "amidinoamino C₁₋₄ alkyl" include, but are not limited to, (amidinoamino)methyl, 2-(amidinoamino)ethyl, 3-(amidinoamino)propyl, 4-(amidinoamino)butyl, and the like. Specific examples of "amidinoamino C₁₋₆ alkyl" include, but are not limited to, amidinoamino-substituted C₁₋₄ alkyl, 5-(amidinoamino)pentyl, 6-(amidinoamino)hexyl, and the like. Examples of the amidinoamino group protected with a nitrogen protecting group include: 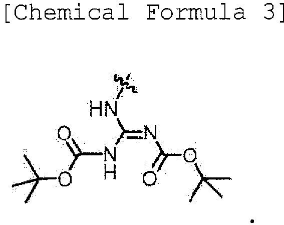 As used herein, "amidinoamino" and "guanidino" have the same meaning.

As used herein, "carboxyalkyl" is alkyl substituted with at least one -COOH group. Specific examples of "carboxy C₁₋₄ alkyl" include, but are not limited to, carboxymethyl, 2-carboxyethyl, 3-carboxypropyl, 4-carboxybutyl, and the like. Specific examples of "carboxy C₁₋₆ alkyl" include, but are not limited to, carboxy-substituted C₁₋₄ alkyl, 5-carboxypentyl, 6-carboxyhexyl, and the like.

A "protecting group" refers to a group of atoms that blocks, reduces, or prevents reactivity of a functional group when attached to a reactive functional group in a molecule. The compound of the present disclosure can have a substitution with a protecting group when appropriate or needed at any of R₁ to R₄ or any position of a substituent thereof or other substituents or the like. Compounds comprising such a protecting group are also within the scope of the present disclosure. Typically, a protecting group can be selectively removed during a synthesis process if desired. Examples of protecting groups are found in Greene and Wuts, Protective Groups in Organic Chemistry, 5th Edition, 2014, John Wiley & Sons, NY and Harrison et al., Compendium of Synthetic Organic Methods, Vol. 1 to 8, John Wiley & Sons, NY, or the like. Representative examples of nitrogen protecting groups include, but are not limited to, formyl, acetyl, trifluoroacetyl, benzyl, benzyloxycarbonyl ("CBZ"), tert-butoxycarbonyl ("Boc"), trimethylsilyl ("TMS"), 2-trimethylsilylethanesulfonyl ("TES"), trityl and substituted trityl group, allyloxycarbonyl, 9-fluorenylmethyloxycarbonyl ("FMOC"), nitro-veratryloxycarbonyl ("NVOC"), and the like. Representative examples of hydroxyl protecting groups include, but are not limited to, groups that acylate (esterify) or alkylate a hydroxyl group, such as benzyl and trityl ether, as well as alkyl ether, tetrahydropyranyl ether, trialkylsilyl ether (e.g., TMS, triethylsilyl, t-butyldimethylsilyl (TBDMS), and triisopropylsilyl (TIPS)), alkyldiarylsilyl ether (e.g., t-butyldiphenylsilyl (TBDPS)), triarylsilyl ether (e.g., triphenylsilyl), glycol ether (e.g., ethylene glycol ether, propylene glycol ether, and the like), and allyl ether.

An amino group of the compound of the present disclosure (e.g., amino group of the backbone, amino group as a substituent, amino group in a substituent of said compound, or the like) can be protected with a nitrogen protecting group or a group represented by "Protect". An amino group in a substituent listed in a substituent group can be further protected with a nitrogen protecting group or a group represented by "Protect". A protected substituent can also be used as a substituent.

A hydroxy group of the compound of the present disclosure (e.g., a hydroxy group as a substituent, a hydroxy group in a substituent of said compound, a hydroxy group in a substituent group described above, or the like) can also be protected with a protecting group of a hydroxy group. A hydroxy group in a substituent listed in a substituent group can be further protected with a hydroxyl protecting group described herein. A protected substituent can also be used as a substituent.

As used herein, the term "ophthalmic disease" or "ocular disease" is a term used in the art and refers to a disease related to the eye, including, but not limited to, glaucoma, age-related macular degeneration (AMD), ischemic retinopathy, optic neuropathy, diabetic retinopathy (DR), diabetic macular edema (DME), uveitis and senile cataract. The ocular disease may be a disease or abnormality associated with damages to the eyes, or more specifically to the retinal pigment epithelium (RPE) and photo cells, induced by oxidative stress and (or) hypoxia, including, but not limited to, glaucoma, AMD, ischemic retinopathy, optic neuropathy, DR and DME. The ocular disease may be a disease or abnormality associated with reduced ocular blood flow, including, but not limited to, glaucoma, ischemic retinopathy, DR and AMD

As used herein, the term "ocular disease", "ocular condition", "eye disease" and "eye condition" refer to eye diseases/conditions that can threaten vision, cause ocular discomfort, and signal systemic health problems.

As used herein, the present disclosure may be directed specifically to diseases or abnormalities associated with damages to the retinal pigment epithelium (RPE) and photo cells, and may be directed to diseases or abnormalities associated with reduced ocular blood flow.

As used herein, the term "retinal disease" is a term used in the art and refers to any disease, disorder or symptom related to the retina, including those caused by damage to the retina due to aging, disease or the like, and includes, but not limited to, glaucoma, retinitis pigmentosa, age-related macular degeneration, diabetic retinopathy, retinal detachment, diabetic maculopathy, hypertensive retinopathy, retinal vascular occlusion, retinal arteriosclerosis, retinal tear, retinal hole, macular hole, fundus hemorrhage, posterior vitreous detachment, pigmented paravenous retinochoroidal atrophy, gyrate atrophy of the retina and choroid, choroideremia, crystalline retinopathy, retinitis punctata albescens, cone dystrophy, central areolar choroidal dystrophy, Doyne's honeycomb retinal dystrophy, vitelliform macular dystrophy, cystoid macular edema, occult macular dystrophy, Stargardt disease, retinoschisis, central serous chorioretinopathy, spinocerebellar ataxia Type 7, familial exudative vitreoretinopathy, enhanced S-cone syndrome, angioid streaks, autosomal dominant optic atrophy, autosomal dominant drusen, acute zonal occult outer retinopathy, cancer associated retinopathy, light damage, or ischemic retinopathy.

As used herein, the term "glaucoma" refers to a disease with characteristic changes in the optic nerve and visual field, characterized by abnormalities in ocular function or structure, the optic nerve damage of which can usually be ameliorated or prevented by sufficient reduction of intraocular pressure (the Clinical Practice Guidelines for Glaucoma (4th Edition), edited by the Japan Glaucoma Society, Journal of Japanese Ophthalmological Society 122 (1), p.5-53 (2018.01) (hereinafter referred to as "guidelines for glaucoma (4th Edition)"), which can be classified into primary glaucoma, secondary glaucoma, and pediatric glaucoma. Primary glaucoma includes primary open-angle glaucoma (broad definition) and primary angle-closure glaucoma, and primary open-angle glaucoma (broad definition) includes primary open-angle glaucoma, normal tension glaucoma, and preperimetric glaucoma. Secondary glaucoma includes secondary open-angle glaucoma and secondary angle-closure glaucoma, and pediatric glaucoma includes primary congenital glaucoma, juvenile open-angle glaucoma, glaucoma associated with congenital ocular dysplasia, and glaucoma associated with congenital systemic disease, and the like.

As used herein, the term "diabetic retinopathy" refers to degeneration in the retina due to the micro vascular degeneration seen in diabetes. The blood vessels that supply oxygen to the retina of the eye are damaged due to long-term high levels of blood sugar (hyperglycemia). The disease generally develops slowly over a period of months, but over time, diabetic retinopathy can get worse and cause vision loss. Diabetic retinopathy usually affects both eyes. Diabetic retinopathy progresses from mild non-proliferative abnormalities, characterized by increased vascular permeability, to moderate and severe non-proliferative diabetic retinopathy (NPDR), characterized by vascular closure, and to proliferative diabetic retinopathy (PDR), characterized by the growth of new blood vessels on the retina and posterior surface of the vitreous. Macular edema, characterized by retinal thickening from leaky blood vessels, can develop at all stages of retinopathy. Furthermore, conditions such as pregnancy, puberty, blood glucose control, hypertension, and cataract surgery can accelerate these degenerations.

As used herein, the term "age-related macular degeneration" is a disease in which the macula in the retina of the eye degenerates with age. Age-related macular degeneration is classified into "exudative" type and "atrophic" type depending on the cause. The present disclosure is effective for both. In the "exudative" type, abnormal choroidal neovascular vessels are formed from the choroid, and progress to the retinal surface. The neovascular vessel is fragile, and therefore bleeding and accumulation of exudate are observed, causing functional disorders of the macula, resulting in deviation of vision, vision loss, etc. Ultimately, irreversible degeneration of the macula occurs, resulting in significant vision loss. In the "atrophic" type, the macula degenerates with age, and rapid loss of vision is observed depending on the extent of degeneration. Another characteristic of the atrophic type is that no choroidal neovascularization is observed.

As used herein, "modulation" of activity means to inhibit or promote activity, and "modulator" refers to an inhibitor or accelerator of activity. "Promoting activity" means that the activity (e.g., promotion of ocular blood flow and cell division) is increased by 1% or more, preferably 5% or more, more preferably 10% or more, still more preferably 20% or more, still more preferably by 30% or more, as compared to when an activity modulator is not used. "Inhibiting activity" means that the activity (e.g., promotion of ocular blood flow and cell division) is decreased by 1% or more, preferably 5% or more, more preferably 10% or more, still more preferably 20% or more, still more preferably by 30% or more, as compared to when an activity modulator is not used.

As used herein, "pharmaceutically acceptable salt" refers to an acid addition salt or base addition salt which is pharmaceutically acceptable for use. Specific examples of "pharmaceutically acceptable salts" include, but are not limited to, acid addition salts such as acetate, propionate, butyrate, formate, trifluoroacetate, maleate, fumarate, tartrate, citrate, stearate, succinate, ethylsuccinate, malonate, lactobionate, gluconate, glucoheptonate, benzoate, methanesulfonate, benzenesulfonate, para-toluenesulfonate (tosylate), laurylsulfate, malate, ascorbate, mandelate, saccharinate, xinafoate, pamoate, cinnamate, adipate, cysteine salt, N-acetyl cysteine salt, hydrochloride, hydrobromide, phosphate, sulfate, hydroiodide, nicotinate, oxalate, picrate, thiocyanate, undecanoate, acrylic acid polymer salt, and carboxyvinyl polymer; inorganic base addition salts such as lithium salt, sodium salt, potassium salt, and calcium salt; organic base addition salts such as morpholine and piperidine; amino acid addition salts such as aspartic acid and glutamic acid; and the like.

A suitable salt and pharmaceutically acceptable salt of a starting compound and a target compound are conventional nontoxic salts, which can be selected as appropriate by those skilled in the art, in addition to acid addition salts such as an organic acid salt (e.g., acetate, trifluoroacetate, maleate, fumarate, citrate, tartrate, methanesulfonate, benzenesulfonate, formate, para-toluenesulfonate, or the like) and inorganic acid salts (e.g., hydrochloride, hydrobromide, hydroiodide, sulfate, nitrate, phosphate, or the like), salts with amino acids (e.g., arginine, aspartic acid, glutamic acid, or the like), metal salts such as an alkali metal salt (e.g., sodium salt, potassium salt, or the like) and an alkaline earth metal salt (e.g., calcium salt, magnesium salt, or the like), ammonium salts, organic base salts (e.g., trimethylamine salt, triethylamine salt, pyridine salt, picoline salt, dicyclohexylamine salt, N,N'-dibenzylethylenediamine salt, or the like), or the like.

When it is desired to obtain a salt of the compound of the present disclosure, in a case where the compound of the present disclosure is obtained in the form of a salt, the compound may be purified as it is, and in a case where the compound of the present disclosure is obtained in the free form, the compound may be dissolved or suspended in an appropriate organic solvent, and an acid or a base may be added to form a salt by a common method.

The compound of the present disclosure and pharmaceutically acceptable salt thereof may also exist in the form of adducts with water or various solvents, and these adducts are also encompassed by the present disclosure.

The present disclosure includes the compound of the present disclosure or a pharmaceutically acceptable salt thereof. Solvates such as hydrates or ethanol solvates thereof are also included. Further, the present disclosure includes all tautomers, all stereoisomers present, and all forms of crystalline forms the compound of the present disclosure.

The phrase "compound or an enantiomer thereof, or a salt thereof, or a solvate thereof" refers to a compound, an enantiomer of the compound, a salt of the compound, a salt of the enantiomer, a solvate of the compound, a solvate of the enantiomer, a solvate of the salt of the compound, or a solvate of the salt of the enantiomer.

The compound described herein may have one or two or more asymmetric centers and thus may exist in a variety of isomeric forms, such as enantiomers and/or diastereomers. For example, the compound described herein can be in the form of individual enantiomers, diastereomers, or geometric isomers, or in the form of a mixture of stereoisomers (including a racemic mixture and a mixture enriched in one or two or more stereoisomers). Isomers may be isolated from a mixture by methods known to those skilled in the art (including chiral high pressure liquid chromatography (HPLC) and chiral salt formation and crystallization). Alternatively, preferred isomers can be prepared by asymmetric synthesis. See, for example, Jacques et al., Enantiomers, Racemates and Resolutions (Wiley Interscience, New York, 1981), Wilen et al., Tetrahedron 33:2725 (1977); Eliel, E.L. Stereochemistry of Carbon Compounds (McGraw-Hill, NY, 1962), and Wilen, S.H. Tables of Resolving Agents and Optical Resolutions p. 268 (E.L. Eliel, Ed., Univ. of Notre Dame Press, Notre Dame, IN 1972). The present disclosure additionally encompasses the compound described herein as an individual isomer that is substantially free of other isomers and alternatively as a mixture of various isomers.

Among the compounds of the present disclosure, optical isomers based on an optically active center, atropisomers based on axial or planar chirality caused by restriction of intramolecular rotation, other stereoisomers, tautomers, geometric isomers, and the like can exist, but these isomers, and all possible isomers and mixtures thereof are included within the scope of the present disclosure.

In particular, optical isomers and atropisomers can be obtained as racemates, or as optically active forms when optically active starting materials or intermediates are used, respectively. If necessary, at appropriate stages of the production method described below, the racemates of the corresponding raw materials, intermediates or final products can be physically or chemically divided into their optical antipodes by known separation methods such as methods using optically active columns and fractional crystallization methods. Specifically, for example, in a diastereomer method, two diastereomers are formed from a racemate by a reaction using an optical resolution agent. Since these different diastereomers generally have different physical properties, the diastereomers can be resolved by known methods such as fractional crystallization.

The phrase "pharmaceutically acceptable" refers to a compound, material, composition, and/or dosage form that is suitable for use in contact with human or animal tissue without excessive toxicity, stimulation, allergic reaction, other problems, or complications, with a reasonable risk-reward ratio, within the scope of a sound medical judgment, upon use herein.

As used herein, the phrase "pharmaceutically acceptable carrier" refers to a pharmaceutically acceptable material, composition, or vehicle such as a liquid or solid filler, diluent, excipient, solvent, or capsule material. Each carrier must be "acceptable" in terms of being compatible with other ingredients of a formulation and unharmful to a patient. Some examples of materials that can act as a pharmaceutically acceptable carrier include the following: (1) saccharide such as lactose, glucose, and sucrose; (2) starch such as corn starch and potato starch; (3) cellulose and derivatives thereof such as sodium carboxymethylcellulose, ethyl cellulose, and cellulose acetate; (4) powder tragacanth; (5) malt; (6) gelatin; (7) talc; (8) excipients such as cocoa butter and suppository wax; (9) oil such as peanut oil, cottonseed oil, safflower oil, sesame oil, olive oil, corn oil, and soybean oil; (10) glycol such as propylene glycol; (11) polyol such as glycerin, sorbitol, mannitol, and polyethylene glycol; (12) esters such as ethyl oleate and ethyl laurate; (13) agar; (14) buffer such as magnesium hydroxide and aluminum hydroxide; (15) alginic acid; (16) pyrogen-free water; (17) isotonic saline; (18) Ringer's solution; (19) ethyl alcohol; (20) phosphate buffer; and (21) other nontoxic compatible substances that are used in pharmaceutical formulations.

Furthermore, the scope of the present disclosure encompasses prodrugs of the compound of the present disclosure. In the present disclosure, a prodrug refers to a derivative that yields the compound of the present disclosure by acid hydrolysis or enzymatic degradation in the body. For example, if the compound of the present disclosure has a hydroxyl group, amino group, or carboxyl group, these groups can be modified by a conventional method to manufacture a prodrug.

Examples of compounds having a carboxy group include compounds whose carboxyl group is modified to be an alkoxycarbonyl group, alkylthiocarbonyl group, or alkylaminocarbonyl group.

Examples of compounds having an amino group include compounds whose amino group is substituted with an alkanoyl group to be an alkanoylamino group, compounds substituted with an alkoxycarbonyl group to be an alkoxycarbonylamino group, compounds modified to have an alkanoyloxymethylamino group, and compounds modified to have hydroxylamine.

Examples for compounds having a hydroxyl group include compounds whose hydroxyl group is substituted with an alkanoyl group to be an alkanoyloxy group, phosphate ester, or alkanoyloxymethyloxy group.

Examples of the alkyl moiety of a group used for preparing a prodrug thereof include the alkyl group. The alkyl group is optionally substituted with, for example, an alkoxy group or the like. Preferred examples thereof include the following.

Examples of a compound whose carboxyl group is modified to be an alkoxycarbonyl group include C₁₋₁₂ alkoxycarbonyl, C₄ alkoxycarbonyl, C₆ alkoxycarbonyl, C₈ alkoxycarbonyl, C₁₀ alkoxycarbonyl, and C₁₂ alkoxycarbonyl, specifically, alkoxycarbonyl such as methoxycarbonyl, ethoxycarbonyl, propyloxycarbonyl, isopropyloxycarbonyl, n-butyloxycarbonyl, isobutyloxycarbonyl, tert-butyloxycarbonyl, sec-butyloxycarbonyl, n-pentyloxycarbonyl, isopentyloxycarbonyl, neopentyloxycarbonyl, tert-pentyloxycarbonyl, 1,2-dimethylpropyloxycarbonyl, n-hexyloxycarbonyl, heptoxycarbonyl, isoheptoxycarbonyl, octoxycarbonyl, isooctoxycarbonyl, nonyloxycarbonyl, isononyloxycarbonyl, decyloxycarbonyl, isodecyloxycarbonyl, undecyloxycarbonyl, isoundecyloxycarbonyl, dodecyloxycarbonyl, or isododecyloxycarbonyl, an alkoxy group such as C₁₋₁₂ alkoxy C₁₋₁₂ alkoxycarbonyl, C₁₋₁₂ alkoxyethoxycarbonyl, and specifically, methoxymethoxycarbonyl, ethoxymethoxycarbonyl, 2-methoxyethoxycarbonyl, 2-methoxyethoxymethoxycarbonyl, or pivaloyloxymethoxycarbonyl, or alkoxycarbonyl substituted with C₁₋₁₂ alkyl PEG, C₄ alkyl PEG, C₆ alkyl PEG, C₈ alkyl PEG, C₁₀ alkyl PEG, or C₁₂ alkyl PEG. Here, PEG refers to polyethylene glycol, and alkyl may be linear or branched.

As used herein, "or" is used when "at least one or more" of the listed matters in the sentence can be employed. When explicitly described herein as "within the range of two values", the range also includes the two values themselves.

Reference literatures such as scientific literatures, patents, and patent applications cited herein are incorporated herein by reference to the same extent that the entirety of each document is specifically described.

### (Preferred embodiments)

The preferred embodiments of the present disclosure are described hereinafter. It is understood that the embodiments provided hereinafter are provided for the better understanding of the present disclosure, so that the scope of the present disclosure should not be limited by the following descriptions. Thus, it is apparent that those skilled in the art can refer to the descriptions herein to make appropriate modifications within the scope of the present disclosure. It is also understood that the following embodiments of the present disclosure can be used individually or as a combination.

### (Medicament for ophthalmic disease)

The present disclosure provides a medicament or composition or the like, for treating or preventing an ophthalmic disease, comprising a compound described herein or a pharmaceutically acceptable salt, solvate or prodrug thereof.

In one aspect, the compound utilized in the present disclosure can be exemplified as a compound represented by the following formula 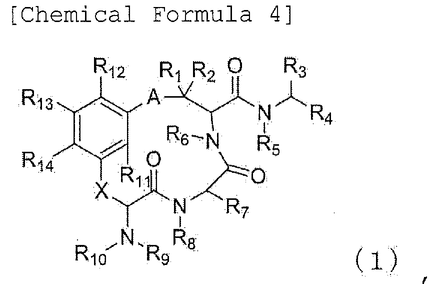 or a pharmaceutically acceptable salt, solvate or prodrug thereof:
wherein
R₁, R₂, R₅, R₆, R₇, R₈, R₉ and R₁₀ are each independently
a hydrogen atom, or
an optionally substituted hydrocarbon group, or
R₇ and R₈, together with the carbon atom and nitrogen atom to which they are attached, form an optionally substituted aryl group, an optionally substituted heteroaryl group, an optionally substituted cycloalkyl group, or an optionally substituted heterocycloalkyl group,
R₃ and R₄ are each independently
a hydrogen atom, an optionally substituted hydrocarbon group, a carboxyl group,
an optionally substituted alkoxycarbonyl group, or an optionally substituted alkoxycarbonyloxy group,
R₁₁, R₁₂, R₁₃, and R₁₄ are each independently
a hydrogen atom, an optionally substituted hydrocarbon group, a hydroxy group,
an optionally substituted alkoxy group, or
an optionally substituted alkoxycarbonyloxy group,
X is
CH₂ or CO, and
A is O, NH or S, wherein the NH is optionally substituted.

In one embodiment, R₁, R₂, R₈, R₆, R₇, R₈, R₉ and R₁₀ are each independently
a hydrogen atom, or
an optionally substituted hydrocarbon group, or
R₇ and R₈, together with the carbon atom and nitrogen atom to which they are attached, form an optionally substituted heterocycloalkyl group,
R₃ and R₄ are each independently
a hydrogen atom, an optionally substituted hydrocarbon group, a carboxyl group,
an optionally substituted alkoxycarbonyl group, or an optionally substituted alkoxycarbonyloxy group,
R₁₁, R₁₂, R₁₃, and R₁₄ are each independently
a hydrogen atom, a hydroxy group,
an optionally substituted alkoxy group, or
an optionally substituted alkoxycarbonyloxy group,
X is
CH₂ or CO, and
A is O, NH or S, wherein the NH is optionally substituted.

In one embodiment, R₁, R₂, R₅, R₆, R₇, R₈, R₉ and R₁₀ are each independently
a hydrogen atom, or
an optionally substituted alkyl group, or
R₇ and R₈, together with the carbon atom and nitrogen atom to which they are attached, form an optionally substituted heterocycloalkyl group,
R₃ and R₄ are each independently
a hydrogen atom, an optionally substituted alkyl group, a carboxyl group,
an optionally substituted alkoxycarbonyl group, or an optionally substituted alkoxycarbonyloxy group,
R₁₁, R₁₂, R₁₃, and R₁₄ are each independently
a hydrogen atom, a hydroxy group,
an optionally substituted alkoxy group, or
an optionally substituted alkoxycarbonyloxy group,
X is
CH₂ or CO, and
A is O, NH or S, wherein the NH is optionally substituted.

In one embodiment, R₁, R₂, R₅, R₆, R₇, R₈, R₉ and R₁₀ are each independently
a hydrogen atom, or
an alkyl group substituted with one to the maximum substitutable number of the same or different substituents selected from the group consisting of hydrogen, alkyl, alkylcarbonyl, arylalkylcarbonyl, hydroxy, alkoxy, alkoxycarbonyl, alkoxycarbonylamino, cycloalkyl, carboxy, amino, guanidino, alkoxycarbonyl-substituted guanidino, carbamoyl, and heterocycloalkyl, or
R₇ and R₈, together with the carbon atom and nitrogen atom to which they are attached, form a heterocycloalkyl group,
R₃ and R₄ are each independently
a hydrogen atom,
an alkyl group substituted with one to the maximum substitutable number of the same or different substituents selected from the group consisting of hydrogen, alkyl, alkylcarbonyl, arylalkylcarbonyl, hydroxy, alkoxy, alkoxycarbonyl, alkoxycarbonylamino, cycloalkyl, carboxy, amino, guanidino, alkoxycarbonyl-substituted guanidino, carbamoyl, and heterocycloalkyl, a carboxyl group, or an alkoxycarbonyl group substituted with one to the maximum substitutable number of the same or different substituents selected from the group consisting of alkyl, alkylcarbonyl, arylalkylcarbonyl, hydroxy, alkoxy, alkoxycarbonyl, alkoxycarbonylamino, cycloalkyl, carboxy, amino, guanidino, alkoxycarbonyl-substituted guanidino, carbamoyl, and heterocycloalkyl,
R₁₁, R₁₂, R₁₃, and R₁₄ are each independently
a hydrogen atom, a hydroxy group, or
an alkoxy group substituted with one to the maximum substitutable number of the same or different substituents selected from the group consisting of alkyl, alkylcarbonyl, arylalkylcarbonyl, hydroxy, alkoxy, and heterocycloalkyl,
X is CH₂ or CO, and
A is O, NH or S, wherein the NH may be optionally substituted with one to the maximum substitutable number of the same or different substituents selected from the group consisting of alkyl, alkylcarbonyl, arylalkylcarbonyl, hydroxy, alkoxy, alkoxycarbonyl, cycloalkyl, carboxy and heterocycloalkyl.

In one embodiment, R₁ and R₂ are each independently a hydrogen atom, or a C₁₋₆ alkyl group, R₃ and R₄ are each independently a hydrogen atom, a C₁₋₆ alkyl group substituted with a carboxyl group, or a carboxyl group, R₅ is a hydrogen atom, or a C₁₋₆ alkyl group, R₆ is a hydrogen atom, or a C₁₋₆ alkyl group, R₇ is a hydrogen atom, a C₁₋₆ alkyl group, a hydroxy C₁₋₆ alkyl group, a carbamoyl C₁₋₆ alkyl group, a C₆₋₁₀ aryl C₁₋₆ alkyl group, a hydroxy C₆₋₁₀ aryl C₁₋₆ alkyl group, a C₅₋₁₀ heteroaryl C₁₋₆ alkyl group, a carboxy C₁₋₆ alkyl group, an amino C₁₋₆ alkyl group, a thio C₁₋₆ alkyl group, a C₁₋₆ alkylthio C₁₋₆ alkyl group, or an amidinoamino C₁₋₆ alkyl group, R₈ is a hydrogen atom, or a C₁₋₆ alkyl group, or R₇ and R₈, together with the carbon atom and nitrogen atom to which they are attached, optionally form an optionally substituted heterocycloalkyl group, R₉ and R₁₀ are a hydrogen atom, or a C₁₋₆ alkyl group, R₁₁, R₁₂, R₁₃, and R₁₄ are each independently a hydrogen atom, an alkoxy group or a hydroxy group, X is CH₂ or CO, and A is O, NH substituted with a C₁₋₆ alkyl group, NH or S.

In one embodiment, R₁ and R₂ are each independently a hydrogen atom, a methyl group or an ethyl group, R₃ and R₄ are each independently a hydrogen atom, a carboxymethyl group, a carboxyethyl group, a carboxypropyl group or a carboxyl group, R₅ is a hydrogen atom, R₆ is a hydrogen atom, R₇ is a hydrogen atom, a methyl group, an isopropyl group, an isobutyl group, a sec-butyl group, a benzyl group, a hydroxymethyl group, a 1-hydroxyethyl group, a carboxymethyl group, a carboxyethyl group, a 4-hydroxybenzyl group, an aminoethyl group, a 4-aminobutyl group, a thiomethyl group, a 2-methylthioethyl group, a carbamoylmethyl group, a carbamoylethyl group, an amidinoaminopropyl group, an indolylmethyl group or a 4-imidazolemethyl group, R₈ is a hydrogen atom, or R₇ and R₈, together with the carbon atom and nitrogen atom to which they are attached, optionally form a C₅₋₁₀ heterocycloalkyl group, R₉ and R₁₀ are a hydrogen atom or a methyl group, R₁₁, R₁₃, and R₁₄ are each independently a hydrogen atom, R₁₂ is a hydrogen atom, a methoxy group or a hydroxy group, X is CH₂ or CO, and A is O, NH or S.

In one embodiment, R₁ and R₂ are each independently a hydrogen atom, or a C₁₋₆ alkyl group.

In one embodiment, R₁ and R₂ are each independently a hydrogen atom, a methyl group or an ethyl group.

In one embodiment, R₃ and R₄ are each independently a hydrogen atom, a C₁₋₆ alkyl group substituted with a carboxyl group, or a carboxyl group.

In one embodiment, R₃ and R₄ are each independently a hydrogen atom, a carboxymethyl group, a carboxyethyl group, a carboxypropyl group or a carboxyl group.

In one embodiment, R₅ is a hydrogen atom, or a C₁₋₆ alkyl group.

In one embodiment, R₅ is a hydrogen atom.

In one embodiment, R₆ is a hydrogen atom, or a C₁₋₆ alkyl group.

In one embodiment, R₆ is a hydrogen atom.

In one embodiment, R₇ is a hydrogen atom, a C₁₋₆ alkyl group, a hydroxy C₁₋₆ alkyl group, a carbamoyl C₁₋₆ alkyl group, a C₆₋₁₀ aryl C₁₋₆ alkyl group, a hydroxy C₆₋₁₀ aryl C₁₋₆ alkyl group, a C₅₋₁₀ heteroaryl C₁₋₆ alkyl group, a carboxy C₁₋₆ alkyl group, an amino C₁₋₆ alkyl group, a thio C₁₋₆ alkyl group, a C₁₋₆ alkylthio C₁₋₆ alkyl group, or an amidinoamino C₁₋₆ alkyl group.

In one embodiment, R₇ is a hydrogen atom, a methyl group, an isopropyl group, an isobutyl group, a sec-butyl group, a benzyl group, a hydroxymethyl group, a 1-hydroxyethyl group, a carboxymethyl group, a carboxyethyl group, a 4-hydroxybenzyl group, an aminoethyl group, a 4-aminobutyl group, a thiomethyl group, a 2-methylthioethyl group, a carbamoylmethyl group, a carbamoylethyl group, an amidinoaminopropyl group, an indolylmethyl group or a 4-imidazolemethyl group.

In one embodiment, R₈ is a hydrogen atom, or a C₁₋₆ alkyl group.

In one embodiment, R₈ is a hydrogen atom.

In one embodiment, R₇ and R₈, together with the carbon atom and nitrogen atom to which they are attached, form an optionally substituted heterocycloalkyl group.

In one embodiment, R₇ and R₈, together with the carbon atom and nitrogen atom to which they are attached, form a C₅₋₁₀ heterocycloalkyl group.

In one embodiment, R₉ and R₁₀ are a hydrogen atom, or a C₁₋₆ alkyl group.

In one embodiment, R₉ and R₁₀ are each independently a hydrogen atom or a methyl group.

In one embodiment, R₁₁, R₁₂, R₁₃, and R₁₄ are each independently a hydrogen atom, an alkoxy group or a hydroxy group.

In one embodiment, R₁₂ is a hydrogen atom, a methoxy group or a hydroxy group.

In one embodiment, R₁₁, R₁₂, R₁₃, and R₁₄ are each independently a hydrogen atom, a methoxy group, or a hydroxy group.

In one embodiment, X is CH₂ or CO.

In one embodiment, A is O, NH substituted with a C₁₋₆ alkyl group, NH or S.

In one embodiment, A is O, NH or S.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a hydrogen atom, R₃ and R₄ are a carboxyl group or a carboxyethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a hydrogen atom or a methyl group, R₃ and R₄ are a carboxyl group or a carboxyethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a hydrogen atom, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, R₁ and R₂ are a hydrogen atom, R₃ and R₄ are a carboxyl group or a carboxyethyl group, R₅ is a hydrogen atom, R₆ is a hydrogen atom, R₇ is a hydrogen atom, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, R₁ and R₂ are a hydrogen atom, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxymethyl group, R₅ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxymethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a hydrogen atom, R₃ and R₄ are a carboxyl group or a carboxymethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a hydrogen atom or a methyl group, R₃ and R₄ are a carboxyl group or a carboxymethyl group, R₅ is a hydrogen atom, R₆ is a hydrogen atom, R₇ is a hydrogen atom, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a hydrogen atom, R₈ is a hydrogen atom, R₉ and R₁₀ are a hydrogen atom, R₁₁, R₁₂, R₁₃, and R₁₄ are a hydrogen atom, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom or a methyl group, R₁₁, R₁₂, R₁₃, and R₁₄ are a hydrogen atom, X is CH₂, and A is O.

In one embodiment, R₁ and R₂ are a methyl group or an ethyl group, R₃ and R₄ are a carboxyl group or a carboxymethyl group, R₅ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, R₁ and R₂ are a methyl group, R₃ and R₄ are a carboxyl group or a carboxyethyl group, R₈ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group, R₃ and R₄ are a carboxyl group or a carboxymethyl group, R₈ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a hydrogen atom or a carboxypropyl group, R₅ is a hydrogen atom, R₆ is a hydrogen atom, R₇ is an isopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group, R₃ and R₄ are a carboxyl group or a carboxyethyl group, R₈ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₈ is a hydrogen atom, R₆ is a hydrogen atom, R₇ is a benzyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a carboxyethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₈ is a hydrogen atom, R₆ is a hydrogen atom, R₇ is a carboxymethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₈ is a hydrogen atom, R₆ is a hydrogen atom, R₇ is a hydroxymethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a 1-hydroxyethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a 4-hydroxybenzyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a 4-aminobutyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₈ is a hydrogen atom, R₆ is a hydrogen atom, R₇ is an amidinoaminopropyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a carbamoylethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is a carbamoylmethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₈ is a hydrogen atom, R₆ is a hydrogen atom, R₇ is a 4-imidazolemethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an indolylmethyl group, R₈ is a hydrogen atom, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ and R₈, together with the carbon atom and nitrogen atom to which they are attached, form a pyrrolidine ring, R₉ and R₁₀ are a hydrogen atom or a methyl group, R₁₁, R₁₃, and R₁₄ are a hydrogen atom, R₁₂ is a hydroxy group, X is CH₂, and A is O.

In one embodiment, R₁ and R₂ are a methyl group or an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a methoxy group, X is CH₂, and A is O.

In one embodiment, R₁ and R₂ are an ethyl group, R₃ and R₄ are a carboxyl group or a carboxyethyl group, R₅ is a hydrogen atom, R₆ is a hydrogen atom, R₇ is an isopropyl group, R₈ is a hydrogen atom, R₉ and R₁₀ are a hydrogen atom, R₁₁, R₁₃, and R₁₄ are a hydrogen atom, R₁₂ is a hydroxy group, X is CH₂, and A is O.

In one embodiment, X in the above embodiments is CO.

In one embodiment, A in the above embodiments is NH.

In one embodiment, A in the above embodiments is S.

In another aspect, the present disclosure provides a composition for treating or preventing an ophthalmic disease, comprising the compound utilized in the present disclosure, i.e., a compound represented by the following formula 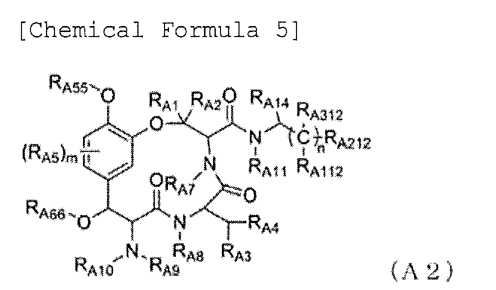 or a pharmaceutically acceptable salt, solvate or prodrug thereof:
wherein m is 0 to 3, n≧1, R_{A1} to R_{A4}, R_{A7} to R_{A11}, R_{A312}, and R_{A112} are each independently a hydrogen atom, or a hydrocarbon group, R_{A14}, and R_{A212} are each independently hydrogen, a carboxyl group or a salt thereof, or an alkoxycarbonyl group, R_{A5} is a hydrocarbon group, a hydroxyl group, an alkoxy group, or an alkylcarbonyloxy group, and R_{A55}, and R_{A66} are each independently a hydrogen atom, a hydrocarbon group, or an alkylcarbonyloxy group.

The above-mentioned cyclic peptide derivative is preferably a cyclic peptide derivative wherein, in the general Formula (1), R₁, R₂, R₃, and R₄ are each independently an alkyl group, n=2 to 4, R₅, and R₆ are hydrogen atoms, and R₇, and R₈ are carboxyl groups, or the like.

The present disclosure may be a compound having a substituent represented by any of the following:

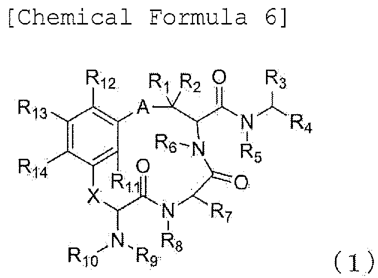

**[Table A-1]**

| Definition Table | | | | | | | | | | | | | | | | |
|---|---|---|---|---|---|---|---|---|---|---|---|---|---|---|---|---|
| Substi tuent | X | A | R1 | R2 | R3 | R4 | R5 | R6 | R7 | R8 | R9 | R10 | R11 | R12 | R13 | R14 |
| 1 | CH₂ | O | H | H | H | H | H | H | H | H | H | H | H | H | H | H |
| 2 | CO | NH | methyl | methyl | carboxy methyl | carboxy methyl | | | heterocyclo alkyl formed together with R₈ | heterocyclo alkyl formed together with R₇ | methyl | methyl | | hydroxy | | |
| 3 | | S | ethyl | ethyl | carboxy ethyl | carboxy ethyl | | | methyl | | | | | methoxy | | |
| 4 | | | | | carboxy propyl | carboxy propyl | | | isopropyl | | | | | | | |
| 5 | | | | | carboxyl | carboxyl | | | isobutyl | | | | | | | |
| 6 | | | | | | | | | sec-butyl | | | | | | | |
| 7 | | | | | | | | | benzyl | | | | | | | |
| 8 | | | | | | | | | hydroxy methyl | | | | | | | |
| 9 | | | | | | | | | 1-hydroxy ethyl | | | | | | | |
| 10 | | | | | | | | | carboxy methyl | | | | | | | |
| 11 | | | | | | | | | carboxy ethyl | | | | | | | |

**[Table A-2]**

| | | | | | | | | | | | | | | | | |
|---|---|---|---|---|---|---|---|---|---|---|---|---|---|---|---|---|
| 12 | | | | | | | | | 4-hydroxy benzyl | | | | | | | |
| 13 | | | | | | | | | 4-aminobutyl | | | | | | | |
| 14 | | | | | | | | | thiomethyl | | | | | | | |
| 15 | | | | | | | | | 2-methyl thioethyl | | | | | | | |
| 16 | | | | | | | | | carbamoyl methyl | | | | | | | |
| 17 | | | | | | | | | carbamoyl ethyl | | | | | | | |
| 18 | | | | | | | | | amidino aminopropyl | | | | | | | |
| 19 | | | | | | | | | indolyl methyl | | | | | | | |
| 20 | | | | | | | | | 4-imidazole methyl | | | | | | | |

In the above table, No. 19 indolylmethyl indicates independently in each instance 2-indolylmethyl or 3-indolylmethyl.

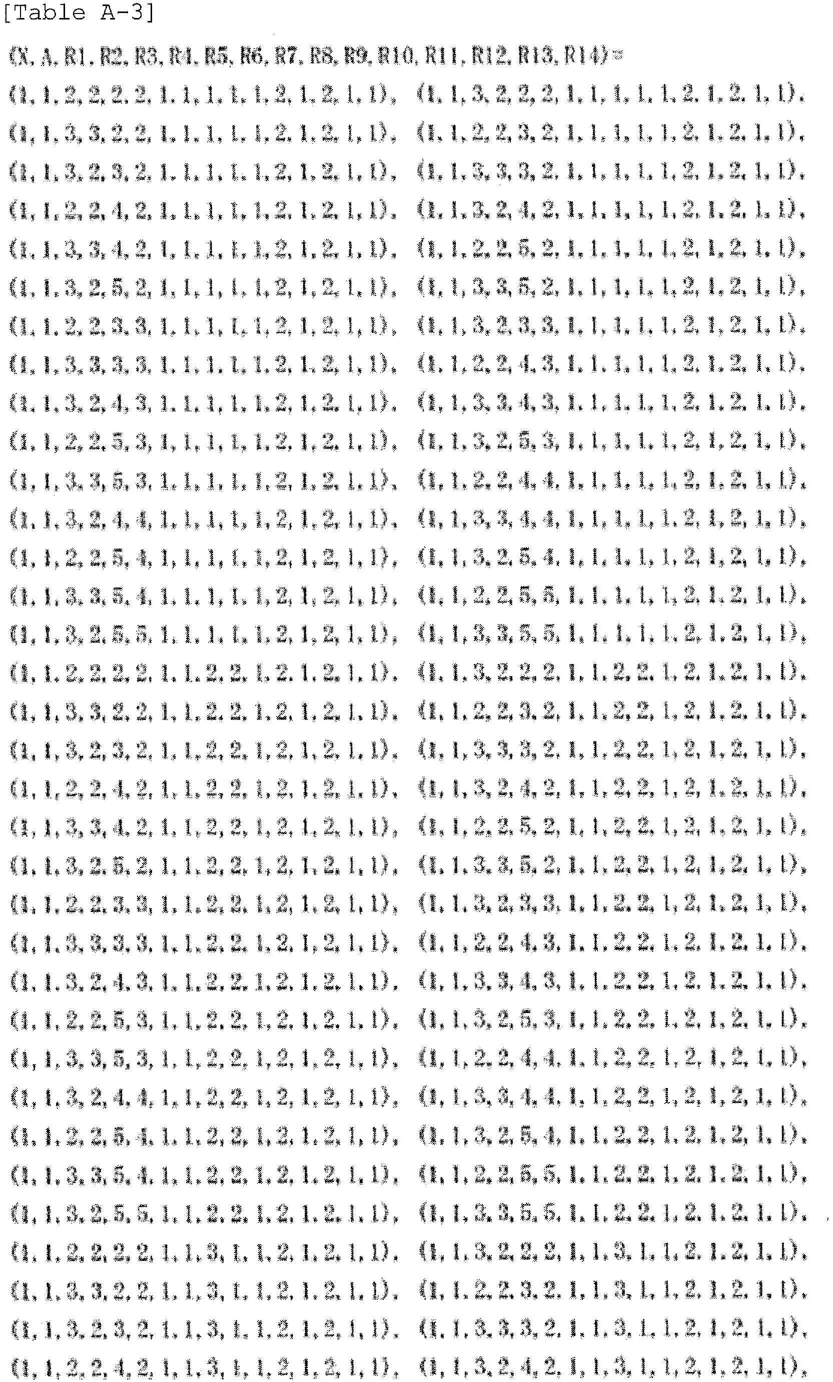

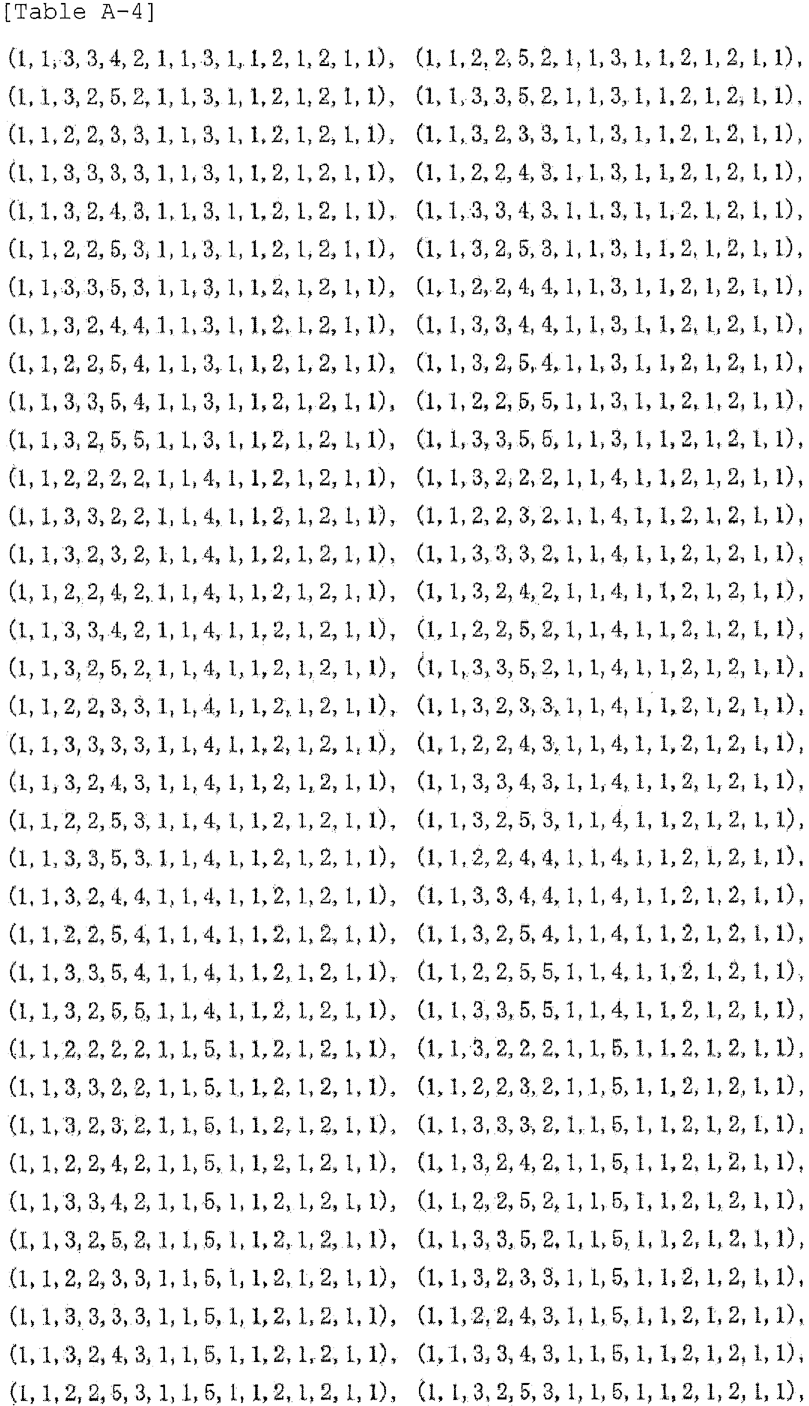

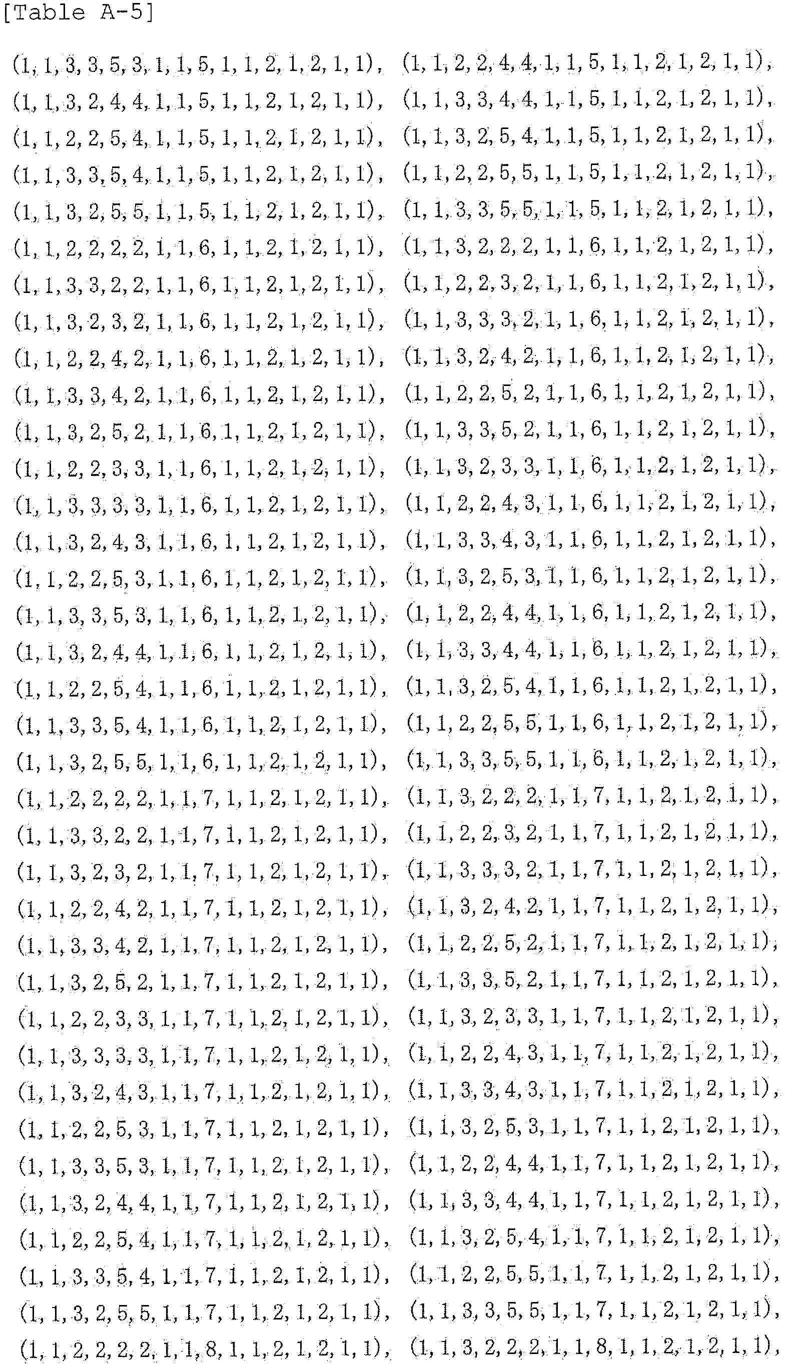

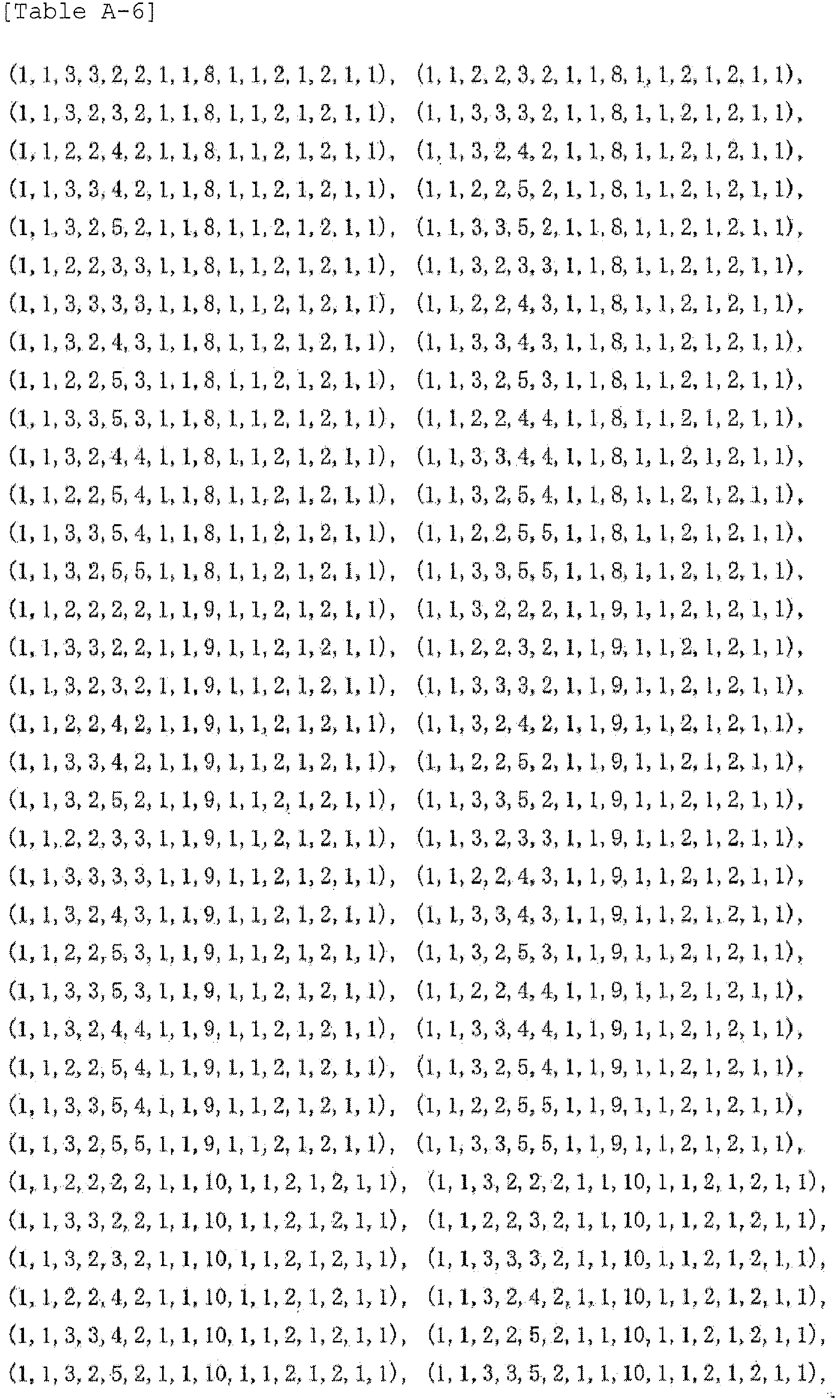

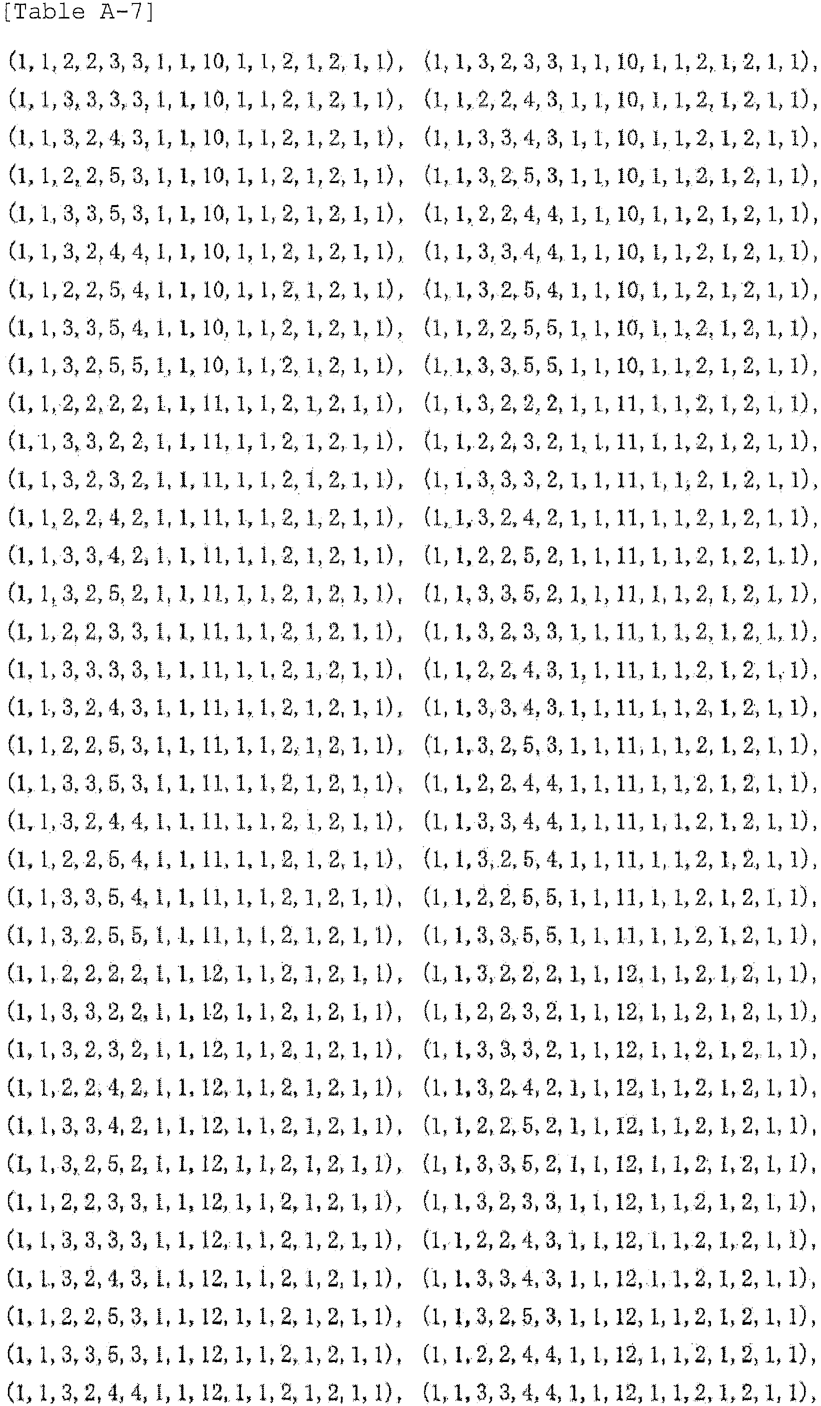

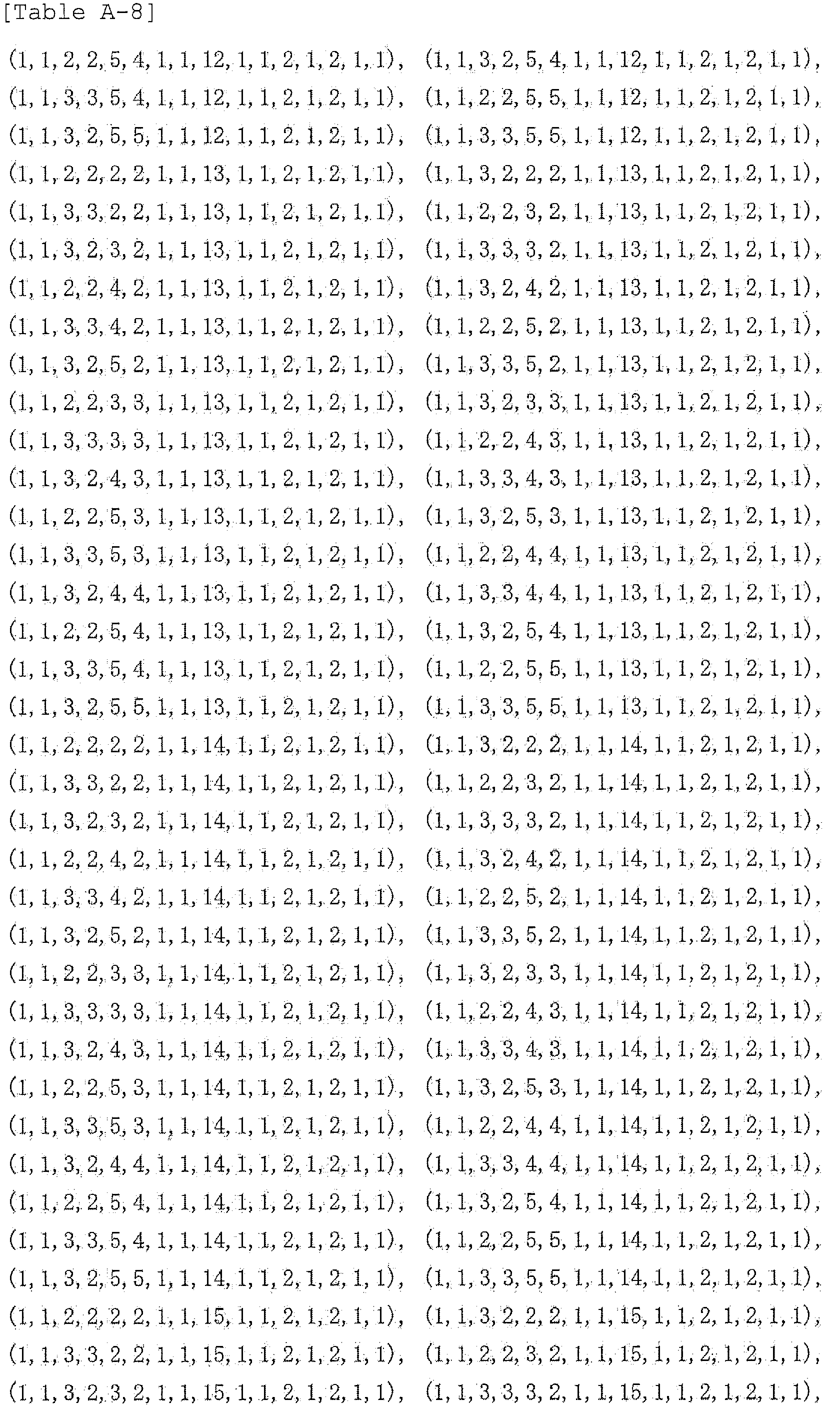

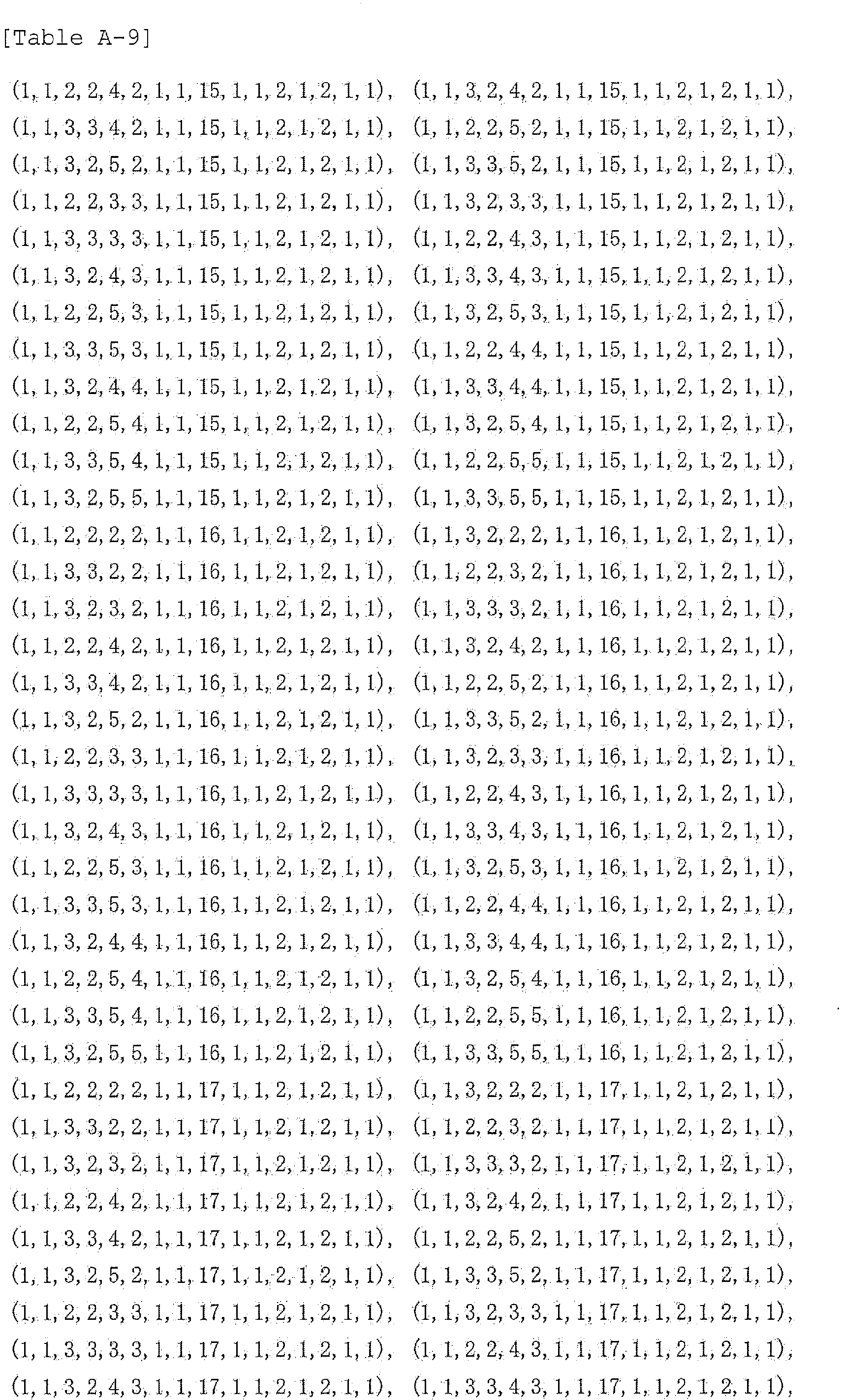

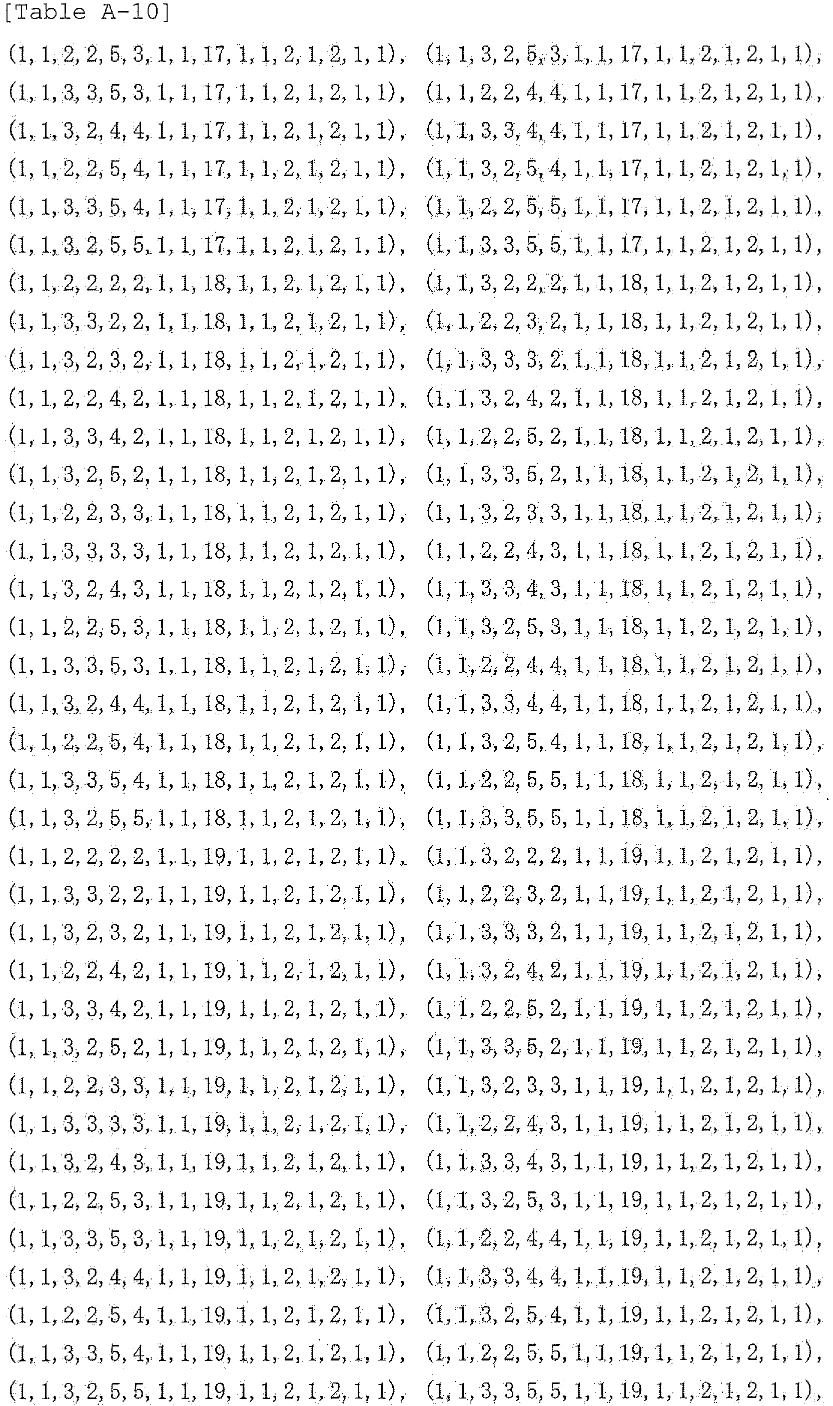

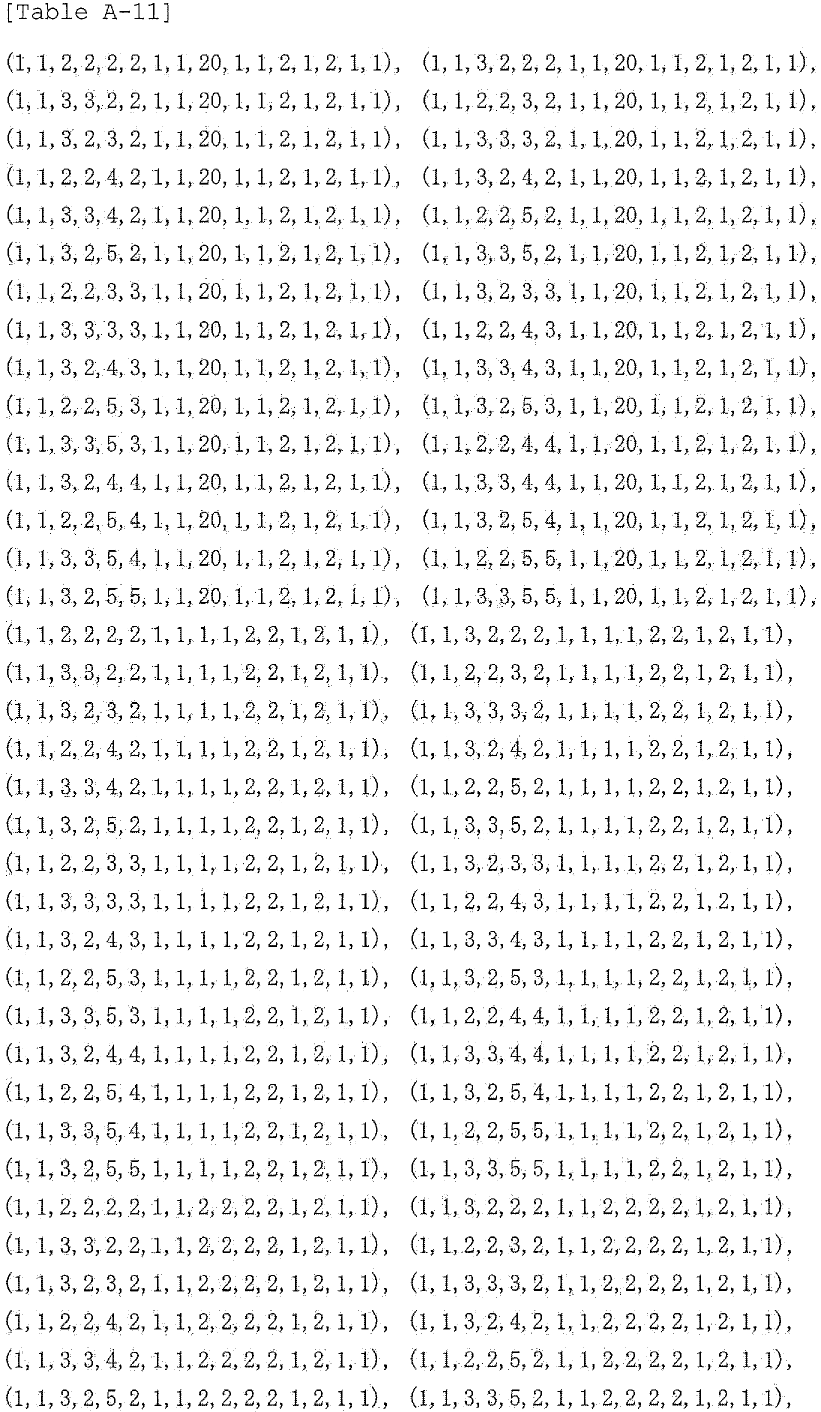

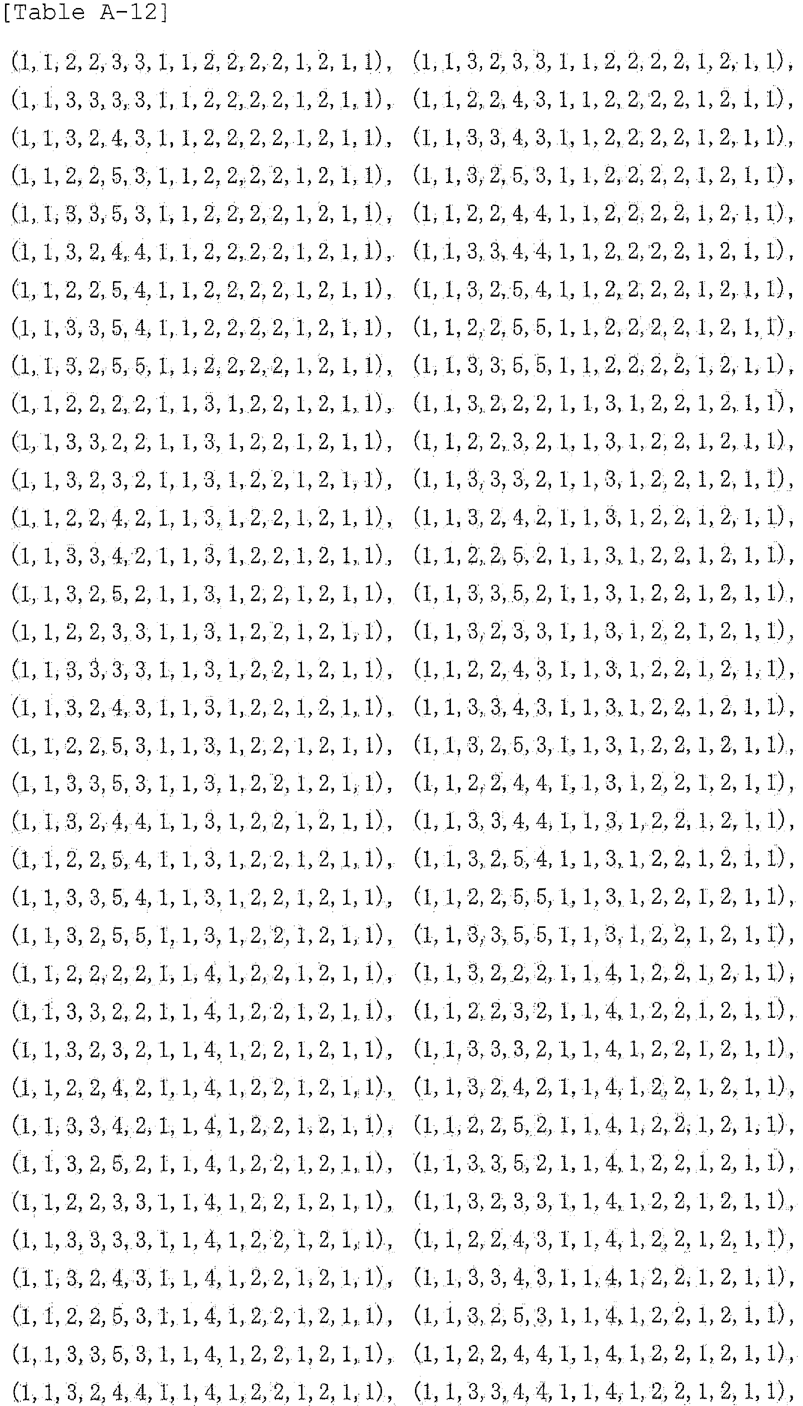

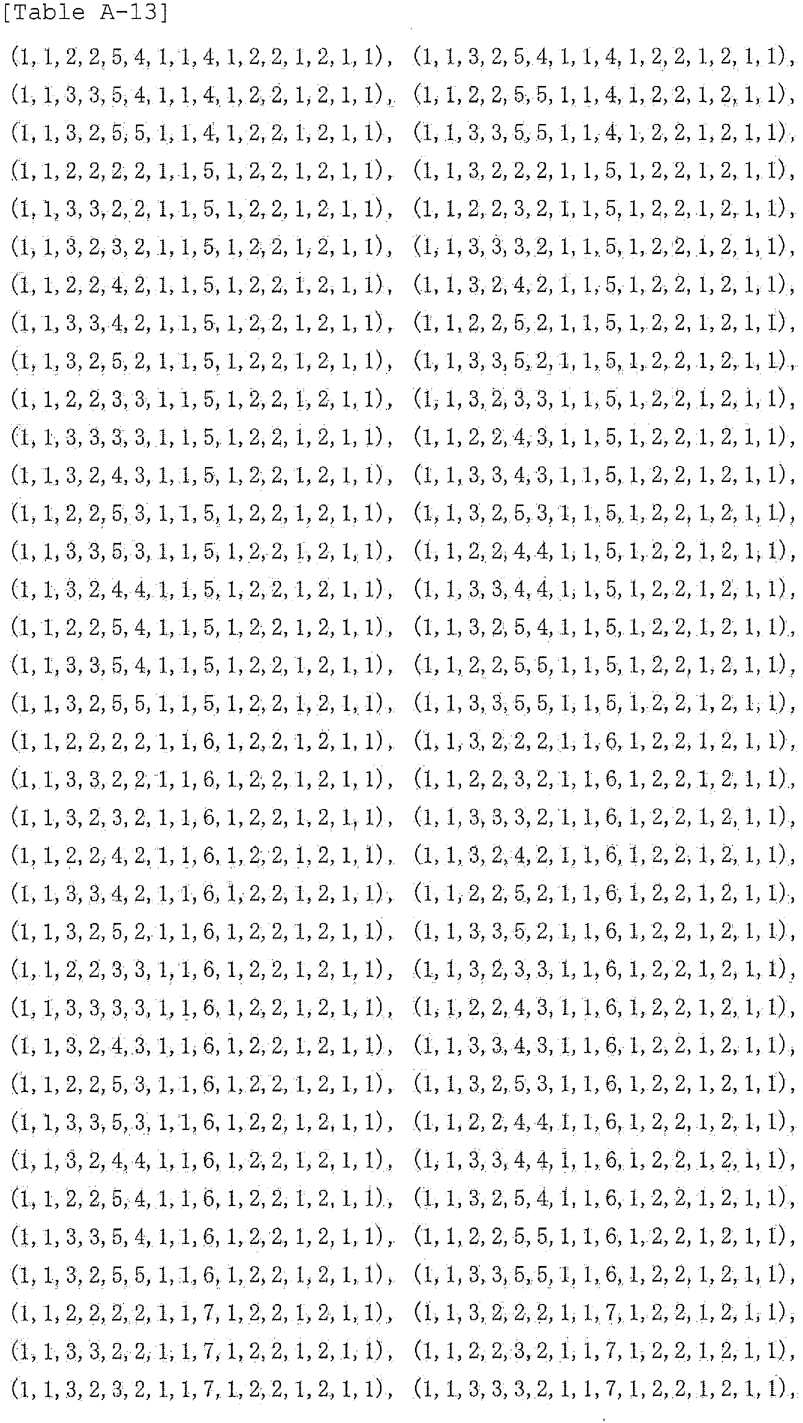

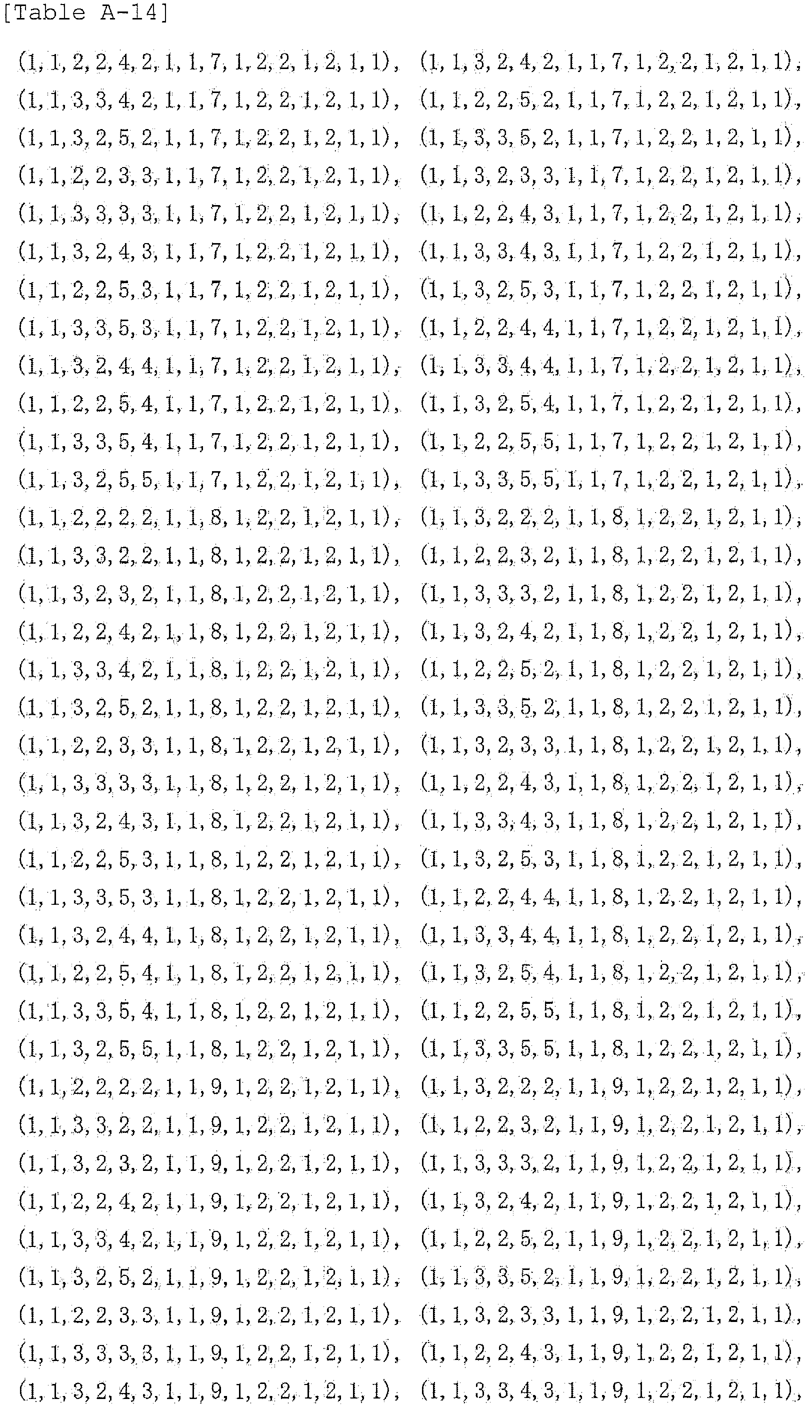

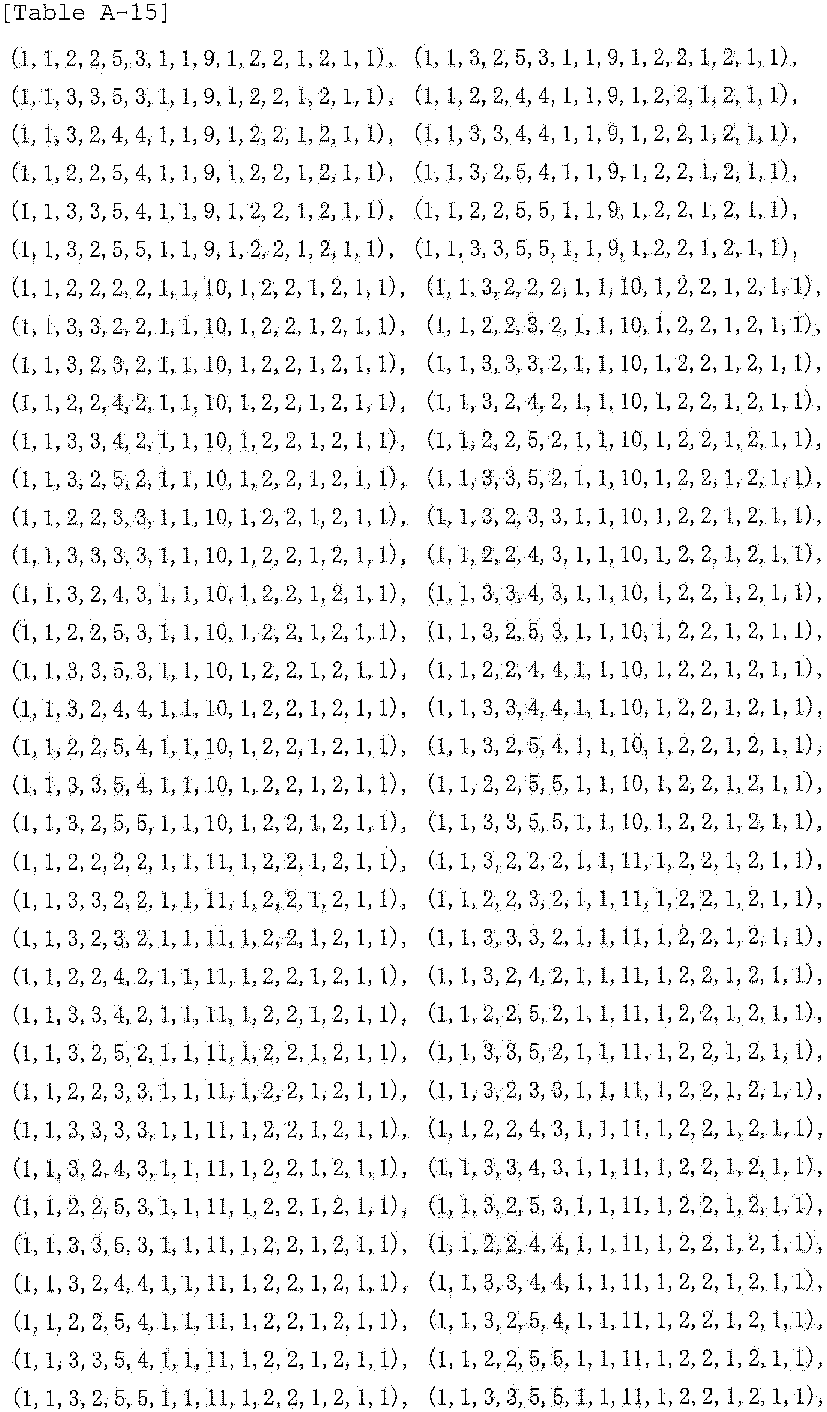

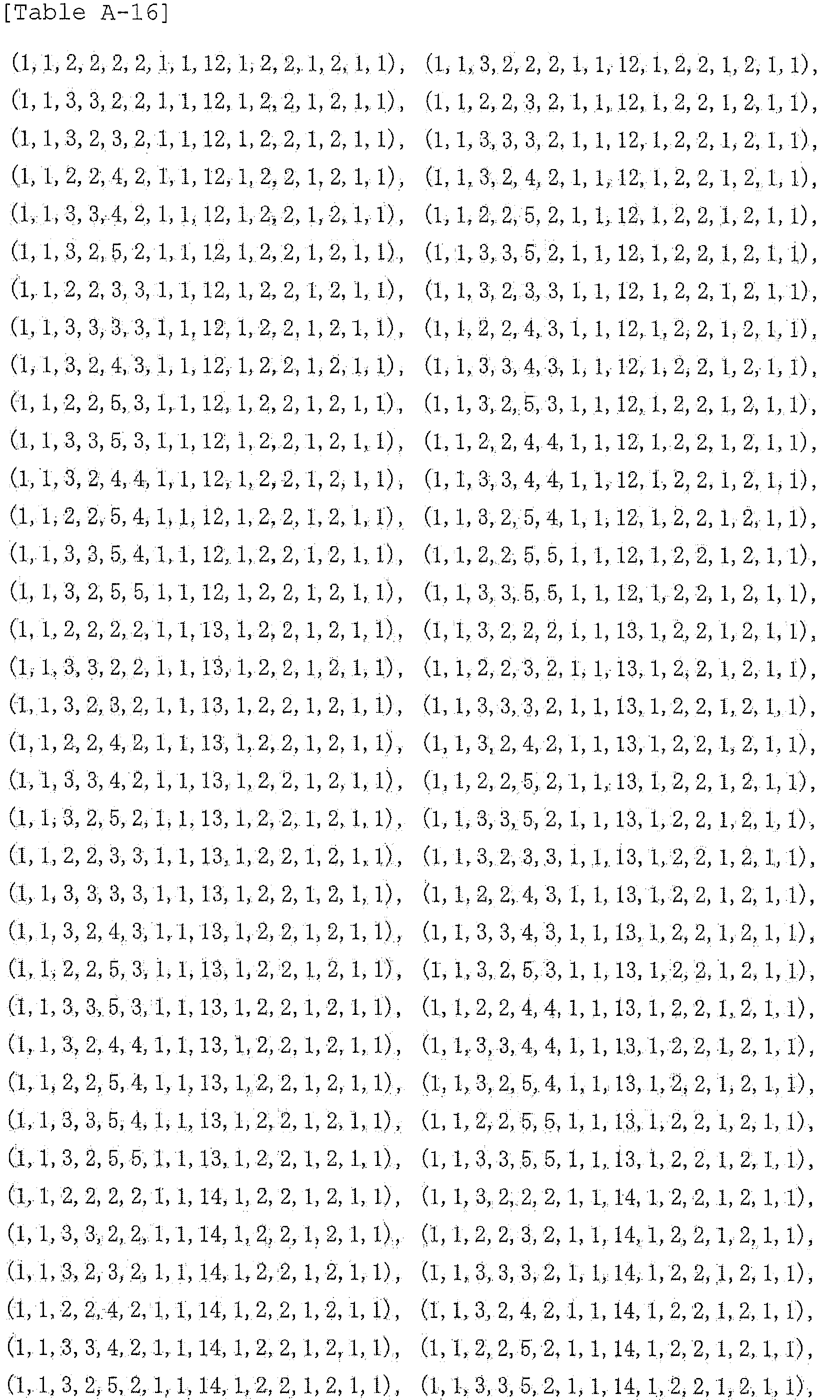

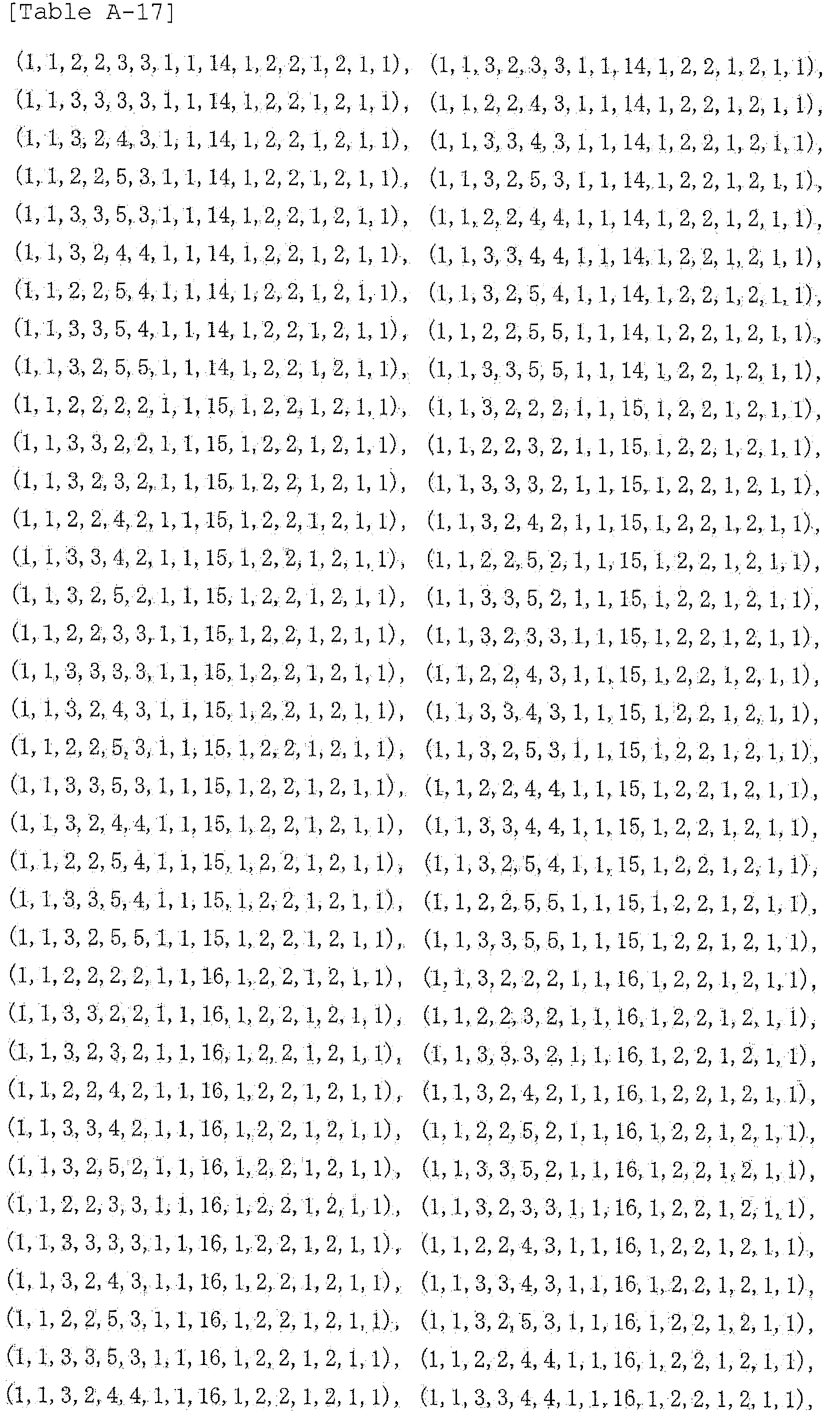

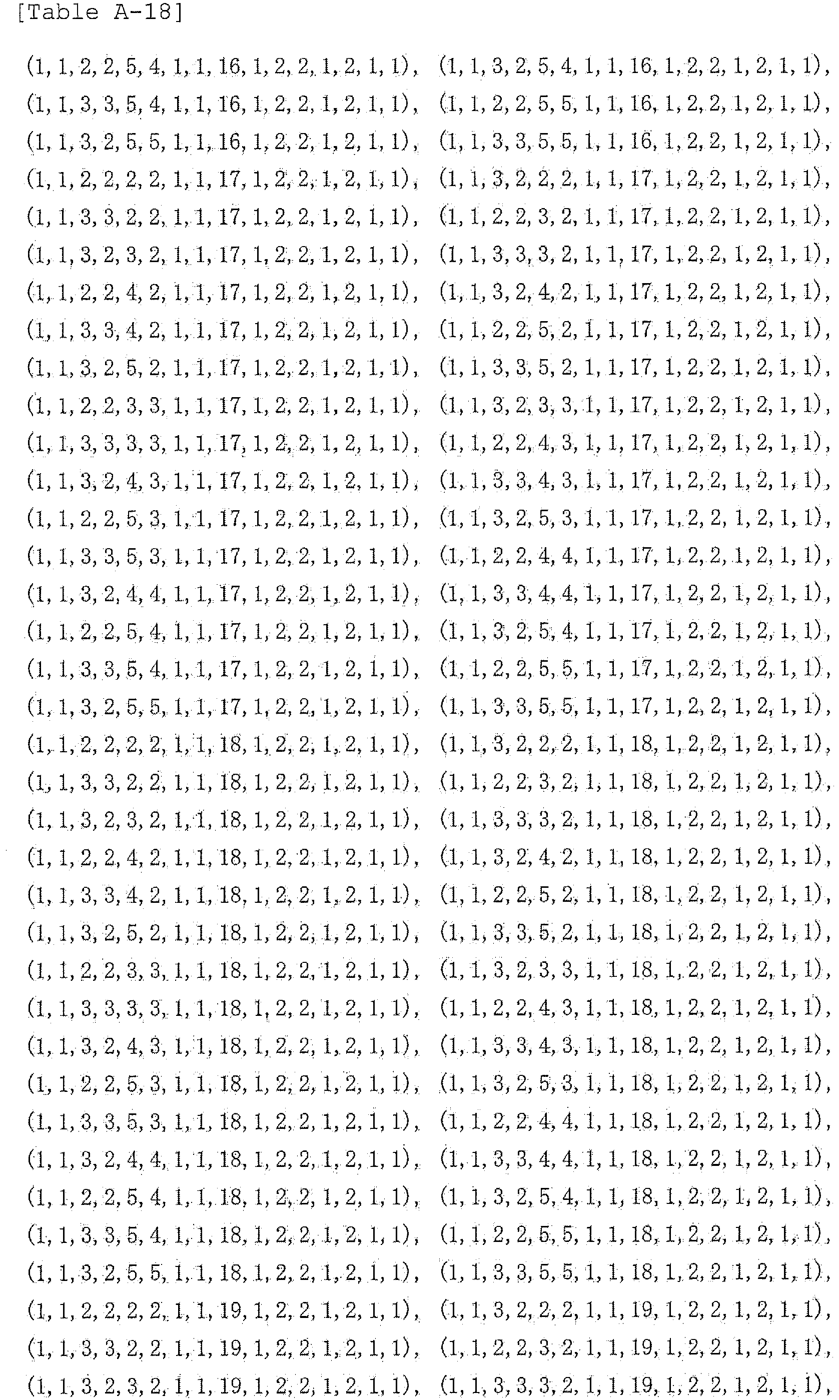

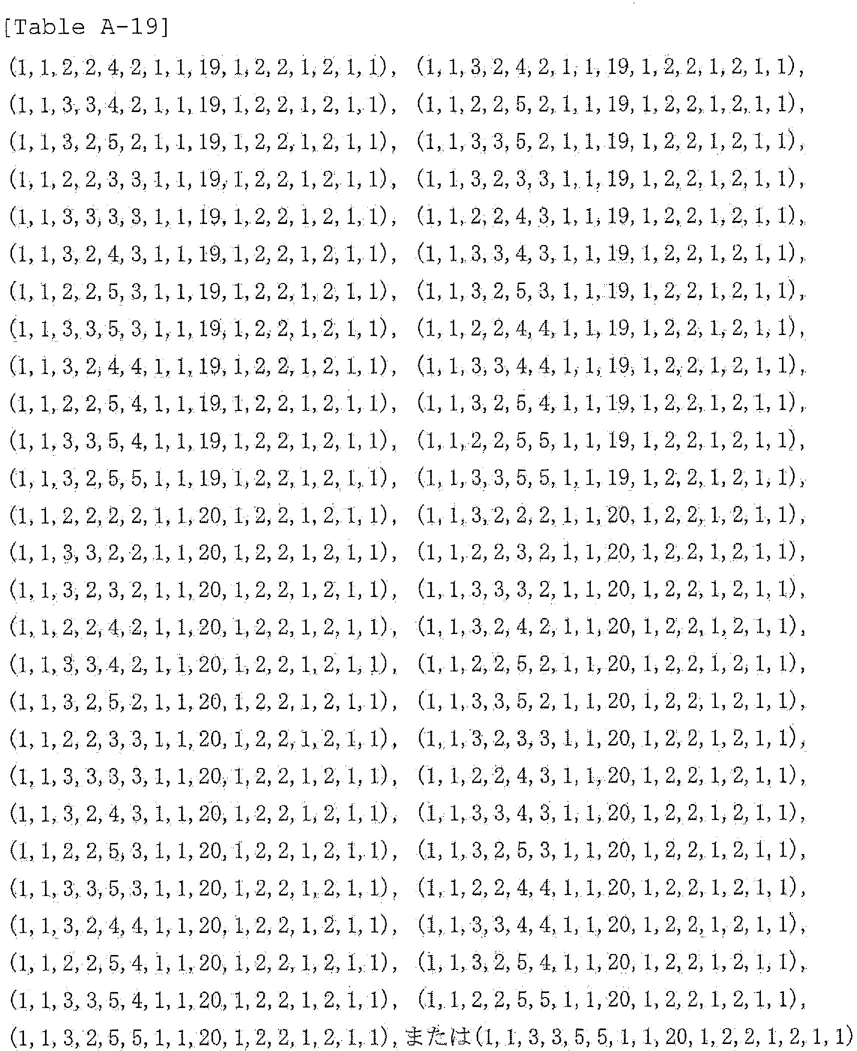

Here, each number of X, A, R1, R2, R3, R4, R5, R6, R7, R8, R9, R10, R11, R12, R13, and R14 is the corresponding substituent shown in the definition table above.

The present disclosure may be a compound having any combination of the substituents shown in the definition table above.

In one embodiment, the compounds disclosed herein are shown in the following tables.

**[Table 2-1]**

| | | |
|---|---|---|
| **Compound 1** | **Compound 2** | **Compound 3** |
| 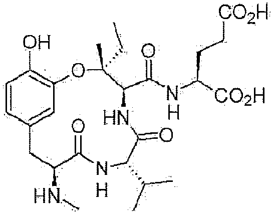 | 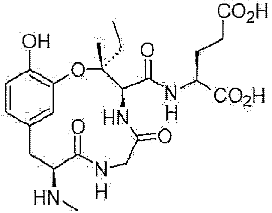 | 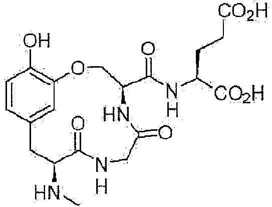 |
| **Compound 4** | **Compound 5** | **Compound 6** |
| 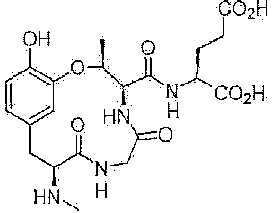 | 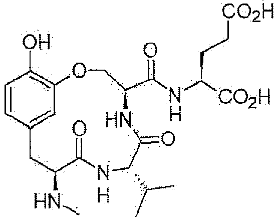 | 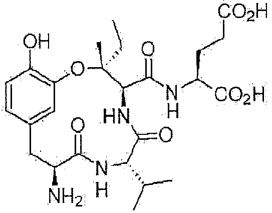 |
| **Compound 7** | **Compound 8** | **Compound 9** |
| 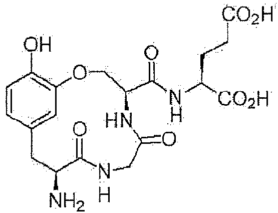 | 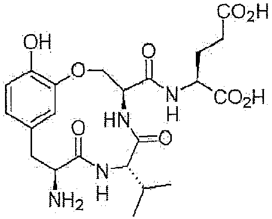 | 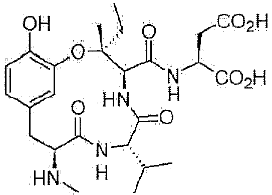 |
| **Compound 10** | **Compound 11** | **Compound 12** |
| 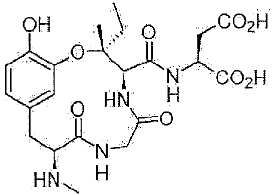 | 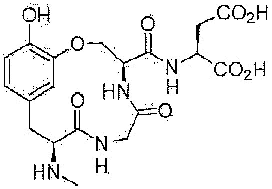 | 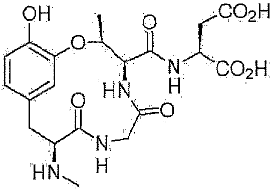 |

**[Table 2-2]**

| | | |
|---|---|---|
| **Compound 13** | **Compound 14** | **Compound 15** |
| 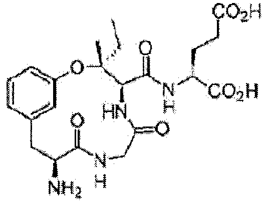 | 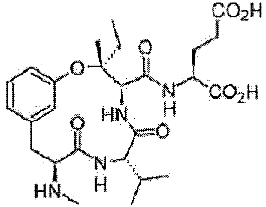 | 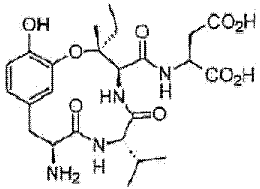 |
| **Compound 16** | **Compound 17** | **Compound 18** |
| 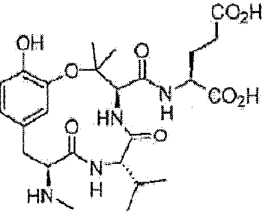 | 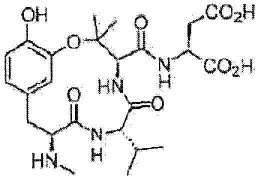 | 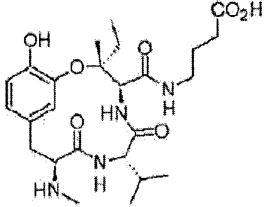 |
| **Compound 19** | **Compound 20** | **Compound 21** |
| 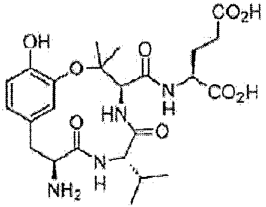 | 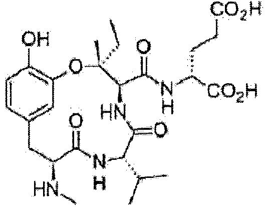 | 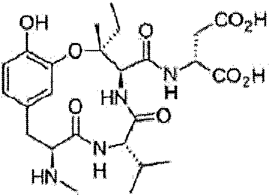 |
| **Compound 22** | **Compound 23** | **Compound 24** |
| 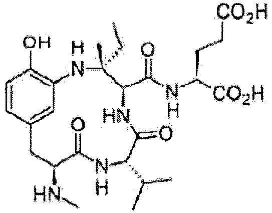 | 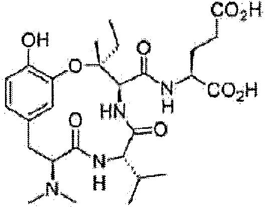 | 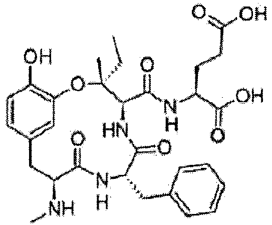 |
| **Compound 25** | **Compound 26** | **Compound 27** |
| 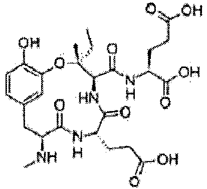 | 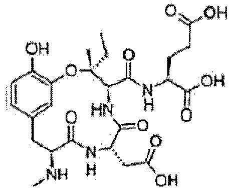 | 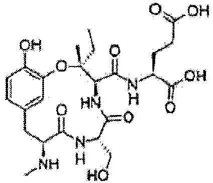 |

**[Table 2-3]**

| | | |
|---|---|---|
| **Compound 28** | **Compound 29** | **Compound 30** |
| 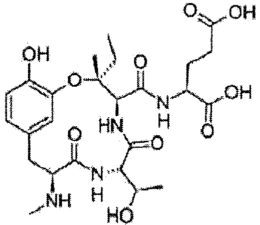 | 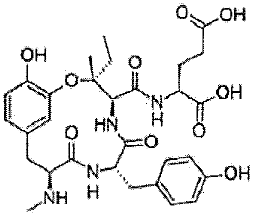 | 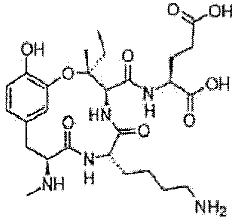 |
| **Compound 31** | **Compound 32** | **Compound 33** |
| 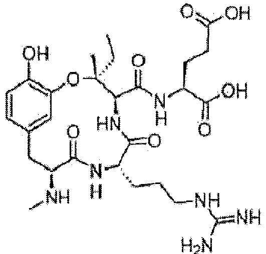 | 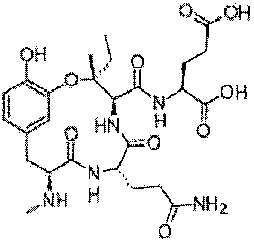 | 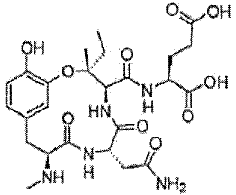 |
| **Compound 34** | **Compound 35** | **Compound 36** |
| 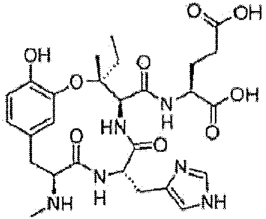 | 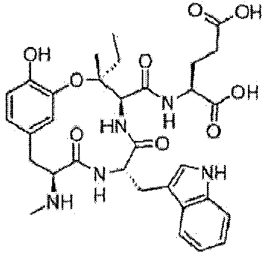 | 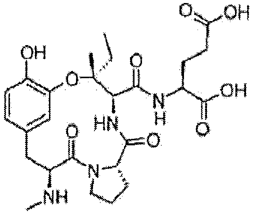 |
| **Compound 37** | **Compound 38** | **Compound 35A** |
| 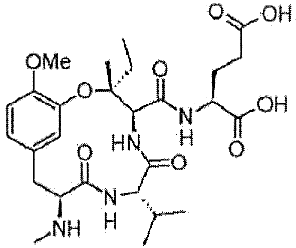 | 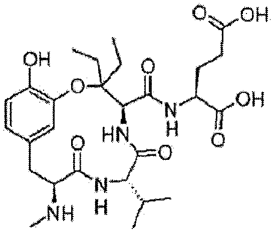 | 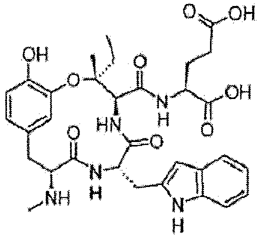 |

**[Table 2-4]**

| | |
|---|---|
| **Compound 9A** | **Compound 9B** |
| 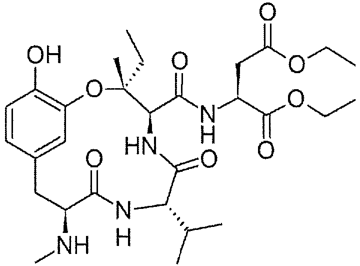 | 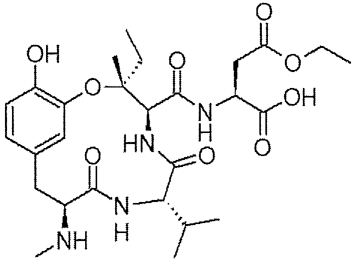 |
| **Compound 9C** | **Compound 9D** |
| 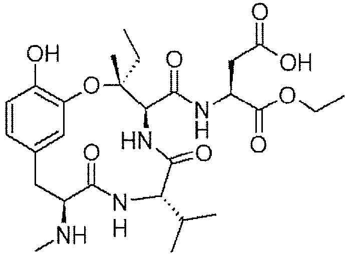 | 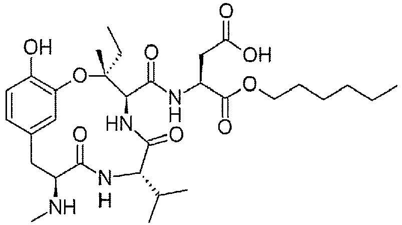 |
| **Compound 9E** | **Compound 9F** |
| 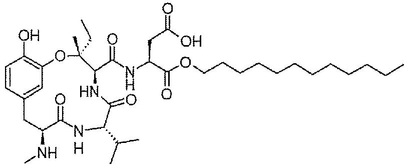 | 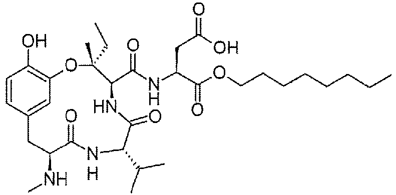 |
| **Compound 9G** | **Compound 39** |
| 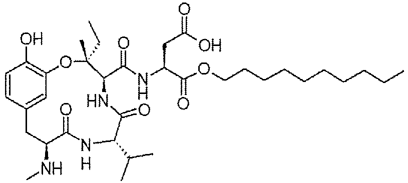 | 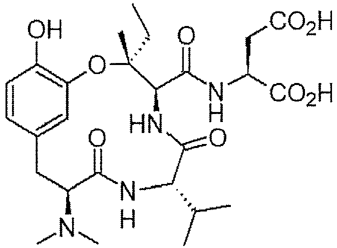 |

[Table 2-5] **Compound 40 Compound 41 Compound 42 Compound 43 Compound 44 Compound 45 Compound 47 Compound 48**

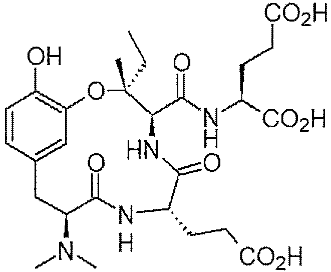

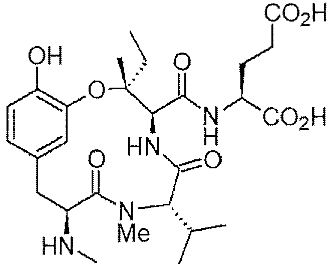

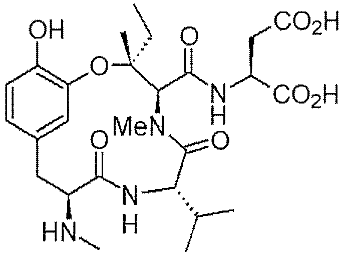

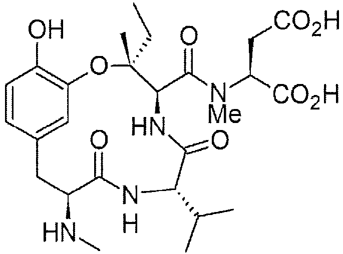

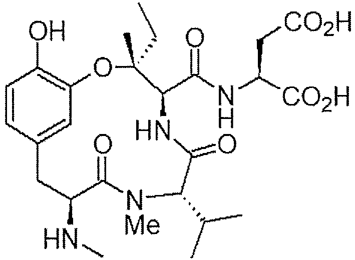

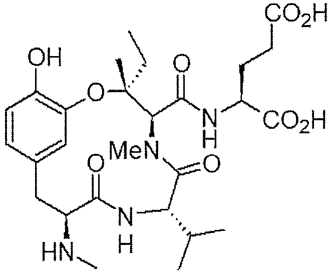

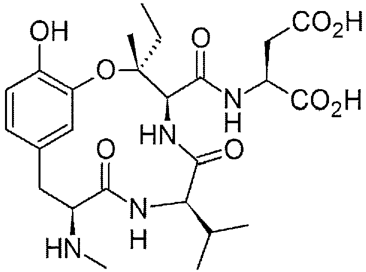

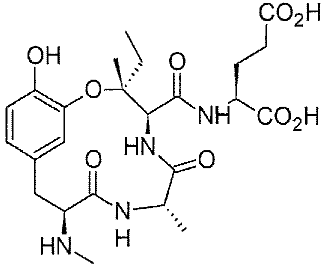

**[Table 2-6]**

| | |
|---|---|
| **Compound 49** | **Compound 50** |
| 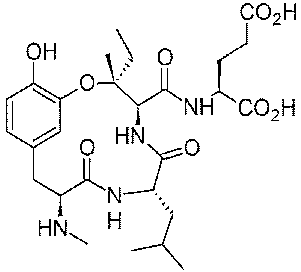 | 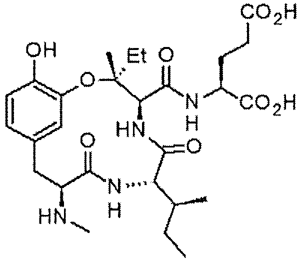 |

**[Table 2-7]**

| | | |
|---|---|---|
| **Compound 51** | **Compound 52** | **Compound 53** |
| 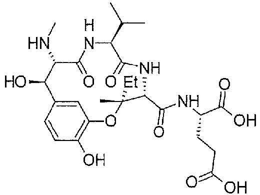 | 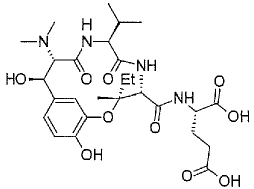 | 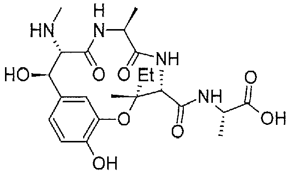 |

Further, examples of the compounds of the present disclosure include the following.

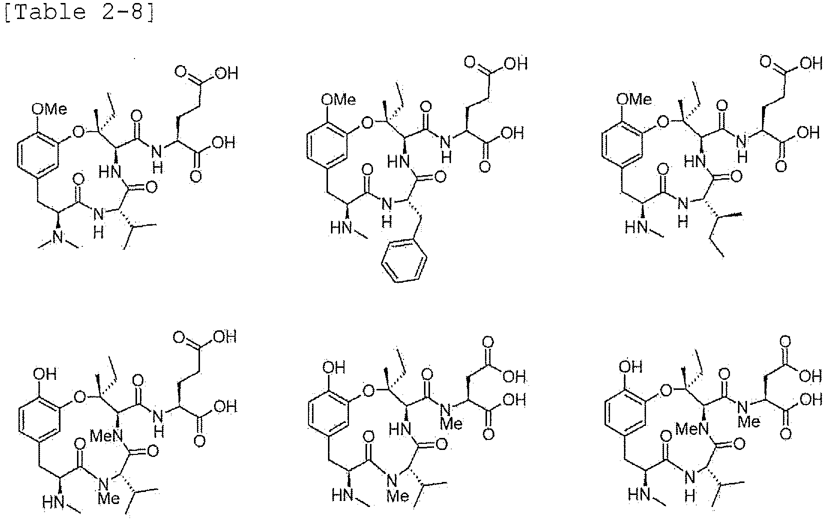

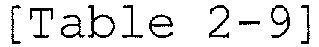

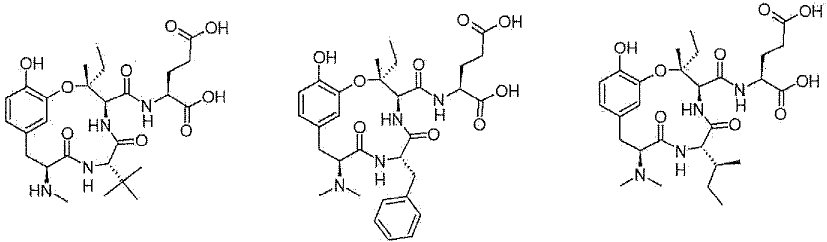

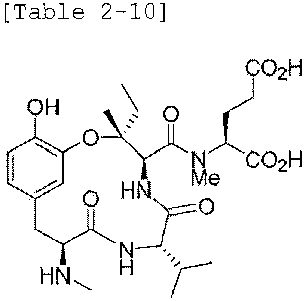

The present disclosure preferably uses a cyclic peptide derivative or a salt thereof as an active ingredient.

The present disclosure also preferably treats or prevents an ophthalmic disease. The ophthalmic disease is preferably a retinal disease, more preferably diabetic retinopathy, glaucoma, or age-related macular degeneration.

Without wishing to be bound by theory, in the present disclosure, when a control substance, a test substance and a positive control are administered to an anterior chamber aqueous humor pressure load rat model, the effects were observed on the general condition and retinal thickness (inner plexiform layer, inner nuclear layer, outer nuclear layer) in the model of retinal damage induced by ischemia caused by pressure overload, so it is understood that the compounds, medicaments or compositions disclosed herein are effective in any ophthalmic disease directly or indirectly related to these. It is also understood that these include many retinal diseases. In addition, from the contents of the present disclosure, it is believed that the compounds, medicaments or compositions disclosed herein are effective for diseases or abnormalities associated with damage to the retinal pigment epithelium (RPE) and photo cells, or for diseases or abnormalities associated with reduced ocular blood flow. (Pharmaceutical and therapeutic method, and the like)

### General description

In one embodiment, the compound of the present disclosure can be formulated into a formulation, a pharmaceutical, or a pharmaceutical composition by oral administration or parenteral administration, directly or using a suitable dosage form, and administered. Specific examples of such dosage forms include, but are not limited to, tablets, capsules, powdered agents, granules, liquid agents, suspension, injection agents, patch-on agents, poultice, and the like. These formulations can be manufactured by a known method using an additive that is commonly used as a pharmaceutical additive.

As these additives, an excipient, disintegrant, binding agent, fluidizer, lubricant, coating agent, solubilizing agent, solubilizing promotor, thickener, dispersant, stabilizer, sweetener, flavoring agent, or the like can be used depending on the objective. Specific examples of these additives include, but are not limited to, lactose, mannitol, crystalline cellulose, low-substituted hydroxypropyl cellulose, corn starch, partially pregelatinized starch, carmellose calcium, croscarmellose sodium, hydroxypropyl cellulose, hydroxypropyl methylcellulose, polyvinyl alcohol, magnesium stearate, sodium stearyl fumarate, polyethylene glycol, propylene glycol, titanium oxide, talc, and the like.

In one embodiment, the compounds of the present disclosure are compounds capable of treating or preventing an ophthalmic disease.

In one embodiment, the compounds of the present disclosure are compounds capable of treating or preventing a retinal disease.

In one embodiment, the compounds of the present disclosure are compounds capable of treating or preventing diabetic retinopathy, glaucoma or age-related macular degeneration.

In one embodiment, the retinal disease targeted by the present disclosure may be a disease in which degeneration, damage, or cell death of cells constituting the retina occurs, or a disease caused by degeneration, damage, or cell death of cells constituting the retina, and examples of such diseases include glaucoma, retinitis pigmentosa, age-related macular degeneration, diabetic retinopathy, retinal detachment, diabetic maculopathy, hypertensive retinopathy, retinal vascular occlusion (retinal artery occlusion; retinal vein occlusion such as central retinal vein occlusion, branch retinal vein occlusion, etc.), retinal arteriosclerosis, retinal tear, retinal hole, macular hole, fundus hemorrhage, posterior vitreous detachment, pigmented paravenous retinochoroidal atrophy, gyrate atrophy of the retina and choroid, choroideremia, crystalline retinopathy, retinitis punctata albescens, cone dystrophy, central areolar choroidal dystrophy, Doyne's honeycomb retinal dystrophy, vitelliform macular dystrophy, cystoid macular edema, occult macular dystrophy, Stargardt disease, retinoschisis, central serous chorioretinopathy (central retinopathy), spinocerebellar degeneration type 7, familial exudative vitreoretinopathy, enhanced S-cone syndrome, angioid streaks, autosomal dominant optic atrophy, autosomal dominant drusen, familial drusen, acute zonal occult outer retinopathy, cancer associated retinopathy, light damage, and ischemic retinopathy and the like. Among them, glaucoma, age-related macular degeneration, and diabetic retinopathy are suitable target diseases.

The present disclosure also targets diseases in which any of the cells constituting the retina are damaged, or diseases caused by damage to any of the cells constituting the retina. Examples of the cells constituting the retina include retinal ganglion cells, amacrine cells, horizontal cells, Muller glial cells, bipolar cells, retinal photoreceptor cells (cones, rods), and retinal pigment epithelial cells. In particular, diseases in which damage to retinal ganglion cells or retinal pigment epithelial cells is observed or which are caused by damage to these cells are suitable diseases.

The present disclosure also targets diseases in which any of the layers constituting the retina, i.e., the internal limiting membrane, nerve fiber layer, ganglion cell layer, inner plexiform membrane, inner nuclear layer, outer plexiform layer, outer nuclear layer, outer limiting membrane, photoreceptor cell layer, and retinal pigment epithelium layer, are damaged, or diseases caused by damage to any of these layers. In particular, diseases in which damage to the ganglion cell layer, inner nuclear layer, or outer nuclear layer are suitable target diseases.

The retinal disease may be one type, or two or more types.

Suitable patients for the present disclosure are patients with the above-mentioned retinal diseases.

Age-related macular degeneration and diabetic retinopathy are diseases caused by intraocular neovascularization, and vision is reduced due to damage to the macular. Diabetic retinopathy is thought to be caused by hyperglycemia-induced inflammation of vascular endothelial cells in retinal tissue, which then triggers neovascularization.

The compounds of the present disclosure inhibit the thinning of the retina (outer nuclear layer), and therefore prevent, ameliorate, or treat retinal diseases.

The timing of dosing of the compound of the present disclosure and therapeutic agents thereof is not limited. The compound and therapeutic agent can be administered concurrently or at staggered times to a subject being administered therewith. In addition, a combined agent of the compound of the present disclosure and therapeutic agent thereof may be used. The dosage of the therapeutic agent can be appropriately selected based on the clinically used dose. The ratio of the compound of the present disclosure and therapeutic agent thereof can be appropriately selected depending on the subject of administration, route of administration, target disease, disorder, symptom, combination, or the like.

In one embodiment of the present disclosure, the compound of the present disclosure can be combined and administered concurrently or at different times upon use of pharmaceutical compositions. Such a pharmaceutical composition is also within the scope of the present disclosure.

Such a medicament, formulation, and pharmaceutical composition can be manufactured by mixing the compound of the present disclosure and/or additional agent (e.g., antimicrobial drug, antiviral agent (e.g., ribavirin, amantadine, or the like), sedative (e.g., ketamine, midazolam, etc.), or the like) with any appropriate component, together or separately as a combined agent or separate agents, by using any technology known in the art, and can be formulated using any known technology in the art as an appropriate formulation such as a tablet, capsule, powder, granule, liquid agent, suspension, injection, patch, or poultice. If the compound of the present disclosure and/or additional agent (e.g., antimicrobial drug, antiviral agent (e.g., ribavirin, amantadine, or the like), sedative (e.g., ketamine, midazolam, etc.), or the like) are prepared as separate agents, they can be provided as a kit of two agents. One component can be provided as a single agent, with an instruction (package insert or the like) instructing to combine another component (for the compound of the present disclosure, additional agent, and for an additional agent (e.g., antimicrobial drug, antiviral agent (e.g., ribavirin, amantadine, or the like), sedative (e.g., ketamine, midazolam, etc.), or the like), the compound of the present disclosure) at the same time or different times for administration.

In a specific embodiment, concomitant administration of the compound of the disclosure and one or more additional therapeutic agent (s) (i.e., one or more additional chemotherapeutic agent(s)) provides improved potency compared to administration of each of the compound of the disclosure (e.g., compound of Formula (1), (A2) or (2)) and one or more additional therapeutic agent(s) individually. In such a specific embodiment, concomitant administration provides a synergistic effect, wherein the synergistic effect refers the sum of the effects of each individual administration of the compound of the disclosure and one or more additional

For use in the method of the present disclosure, the active compound itself can be given, or 0.1 to 99.5% (more preferably 0.5 to 90%) of active ingredient can be given as a pharmaceutical composition in combination with a pharmaceutically acceptable carrier.

The dosage of the compound of the present disclosure is appropriately selected depending on the subject targeted for administration, route of administration, disease, age, and body weight, and symptom of patient. For example, the dosage is 0.01 mg as the lower limit and 10,000 mg as the upper limit per day for adults for oral administration. This amount can be administered once daily, or divided into several doses.

The timing of dosing of the compound of the present disclosure and therapeutic agents thereof is not limited. The compound and therapeutic agent can be administered concurrently or sequentially to a subject being administered therewith. In addition, a combined agent of the compound of the present disclosure and therapeutic agent thereof may be used. The dosage of the therapeutic agent can be appropriately selected based on the clinically used dose. The ratio of the compound of the present disclosure and therapeutic agent thereof can be appropriately selected depending on the subject of administration, route of administration, target disease, disorder or symptom, the subject's age or body weight, or combination, or the like.

In one embodiment of the present disclosure, the compound of the present disclosure can be combined and administered concurrently or at different times upon use of pharmaceutical compositions. Such a pharmaceutical composition is also within the scope of the present disclosure.

If the compound of the present disclosure is used as an active ingredient of a medicament, the compound is intended for use in not just humans, but also animals other than humans (cat, dog, cow, horse, bat, fox, mongoose, raccoon, and the like).

### (Preventive or therapeutic method)

The present disclosure also provides a method of preventing or treating ophthalmic diseases, comprising administering, to a subject in need thereof, the compound of the present disclosure or a pharmaceutically acceptable salt thereof, or a pharmaceutical composition comprising the same. In one embodiment, a method of preventing or treating ophthalmic diseases comprises administering, to a subject in need thereof, a therapeutically effective amount of the compound of the present disclosure or a pharmaceutically acceptable salt thereof, or a solvate thereof, or a pharmaceutical composition comprising the same.

### (Use for prevention or treatment)

One embodiment of the present disclosure provides use of the compound of the present disclosure or a pharmaceutically acceptable salt thereof, for the manufacture of a medicament for preventing or treating ophthalmic diseases.

The present disclosure also provides the compound of the present disclosure or a pharmaceutically acceptable salt thereof, for use in preventing or treating ophthalmic diseases.

### (Synthesis Example)

For example, the present disclosure can be obtained by a binding reaction with four compound fragments (A, B, C and D), but is not limited thereto. As the compound to be used, a commercially available compound may be used, or a synthesized compound may be used.

The binding reaction generally refers to a general technique that can be used in organic synthetic chemistry, and examples thereof include a cyclization reaction, an addition reaction, a ring-opening addition reaction, and a dehydration condensation reaction. In addition to the binding reaction, a protection reaction, a deprotection reaction, an oxidation reaction, a reduction reaction, a hydrogen addition reaction, and the like can be used. Reaction conditions such as a reaction temperature and a reaction time can be appropriately set.

In each reaction, the functional group contained in the compound to be used may be protected with a protecting group or the like.

The order of the reaction is not particularly limited, and a cyclization reaction may be performed after four compounds are bonded, or a cyclization reaction may be performed at the stage of bonding three compounds, and a fourth compound may be bonded to the resulting cyclized compound.

As the compound used in each reaction, a purified compound may be used, or the reaction product in the previous stage may be used as it is.

### 1. Production method of cyclic peptide derivatives

In production method of cyclic peptide derivatives of the present disclosure, a cyclic peptide derivative represented by the following general Formula (2) is produced.

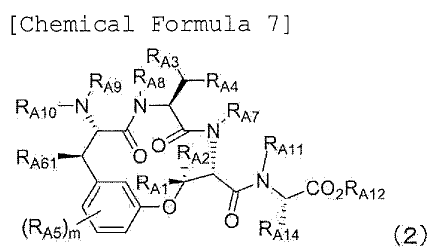

Wherein, in Formula (2),
R_{A1} represents a hydrogen atom or a hydrocarbon group,
R_{A2} represents a hydrogen atom or a hydrocarbon group,
R_{A3} represents a hydrogen atom or a hydrocarbon group,
R_{A4} represents a hydrogen atom or a hydrocarbon group,
R_{A5} represents -O-R_{A51} (R_{A51} is a hydrogen atom or a protecting group),
R_{A61} represents -O-R_{A6} (R_{A6} represents a hydrogen atom, a hydrocarbon group or a protecting group),
R_{A7} represents a hydrogen atom, a hydrocarbon group or a protecting group,
R_{A8} represents a hydrogen atom, a hydrocarbon group or a protecting group,
R_{A9} represents a hydrogen atom, a hydrocarbon group or a protecting group,
R_{A10} represents a hydrogen atom, a hydrocarbon group or a protecting group,
R_{A11} represents a hydrogen atom, a hydrocarbon group or a protecting group,
R_{A12} represents a hydrogen atom or a protecting group,
R_{A14} represents -(CH₂)ₙ-H or -(CH₂)ₙ-COOR_{A13} (R_{A13} represents a hydrogen atom or a protecting group, and n represents a number of 1 or more),
provided that
at least one of R_{A51}, R_{A6}, R_{A12}, and R_{A13} is other than a hydrogen atom, and
m is 1.

In particular, the production method of the present disclosure comprises a step of subjecting a product obtained by an oxidation reaction of compound represented by the following general Formula (2') to a condensation reaction with a compound having both a carboxy group and an amino group, or a salt or ester of the compound (hereinafter, this step is abbreviated as "Step A").

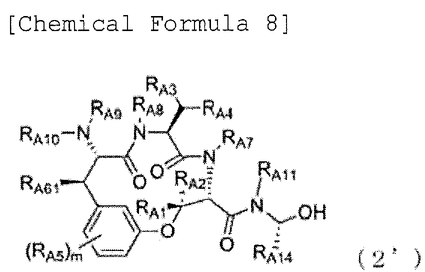

wherein R_{A1}, R_{A2}, R_{A3}, R_{A4}, R_{A5}, R_{A61}, R_{A7}, R_{A8}, R_{A9}, R_{A10} and m in Formula (2') are defined as R_{A1}, R_{A2}, R_{A3}, R_{A4}, R_{A5}, R_{A61}, R_{A7}, R_{A8}, R_{A9}, R_{A10} and m in Formula (2), respectively. In particular, R_{A1}, R_{A2}, R_{A3}, R_{A4}, R_{A5}, R_{A61}, R_{A7}, R_{A8}, R_{A9}, R_{A10} and m in Formula (2') are identical to R_{A1}, R_{A2}, R_{A3}, R_{A4}, R_{A5}, R_{A61}, R_{A7}, R_{A8}, R_{A9}, R_{A10} and m in Formula (2), respectively.

As used herein, the hydrocarbon group may be any of an alkyl group, an alkenyl group, and an alkynyl group. The number of carbon atoms in the hydrocarbon group is not particularly limited, and is, for example, 1 to 10, preferably 1 to 5, more preferably 1 to 4, and particularly preferably 1 to 3. Specific examples of the hydrocarbon group include a methyl group, an ethyl group, a vinyl group, an acetynyl group, a propyl group, an isopropyl group, and a propenyl group. The hydrocarbon group may be linear or branched.

As used herein, the protecting group is other than the above-mentioned hydrocarbon group, and examples thereof include aromatic groups; heterocyclic groups; oxygen-containing functional groups having an alkoxyalkyl group, a carbonyl group, an ester, etc.; and groups having a silicon atom, such as a silyl group.

When the protecting group is an aromatic group, examples thereof include a phenyl group, a benzyl group, an oxybenzyl group (-O-CH₂-Ph), a 2-nitrobenzenesulfonyl group (a nosyl group) and the like. When the protecting group is an oxygen-containing functional group, examples thereof include a tert-butoxycarbonyl group (Boc group). When the protecting group is a group having a silicon atom, examples thereof include a tert-butyldimethylsilyl group (-Si(t-Bu)(CH₃)₂), a tert-butyldiphenylsilyl group (-Si(t-Bu)Ph₂) and the like.

In Formulas (2) and (2'), when R_{A1} is a hydrocarbon group, the group is preferably an alkyl group having 1 to 5 carbon atoms, and R_{A1} is more preferably a hydrogen atom or an alkyl group having 1 to 3 carbon atoms, still more preferably a hydrogen atom, a methyl group or an ethyl group, particularly preferably a methyl group.

In Formulas (2) and (2'), when R_{A1} is a hydrocarbon group, the group is preferably an alkyl group having 1 to 5 carbon atoms, an alkenyl group having 2 to 5 carbon atoms, or an alkynyl group having 2 to 5 carbon atoms, and R_{A2} is more preferably a hydrogen atom, an alkyl group having 1 to 3 carbon atoms, an alkenyl group having 2 to 3 carbon atoms, or an alkynyl group having 2 to 3 carbon atoms, particularly preferably an acetynyl group (-C≡C). In particular, in Formula (2), when R_{A2} is an acetynyl group, the steric hindrance is smaller than that of an ethyl group, etc., and therefore the condensation reaction described below is more likely to proceed.

In Formulas (2) and (2'), when R_{A3} is a hydrocarbon group, the group is preferably an alkyl group having 1 to 5 carbon atoms, and R_{A3} is more preferably a hydrogen atom or an alkyl group having 1 to 3 carbon atoms, still more preferably a hydrogen atom, a methyl group or an ethyl group, particularly preferably a methyl group.

In Formulas (2) and (2'), when R_{A4} is a hydrocarbon group, R_{A3} is preferably a hydrogen atom or an alkyl group having 1 to 5 carbon atoms, more preferably a hydrogen atom or an alkyl group having 1 to 3 carbon atoms, still more preferably a hydrogen atom, a methyl group or an ethyl group, particularly preferably a methyl group.

In Formulas (2) and (2'), R_{A5} is -O-R_{A51} (R_{A51} represents a hydrogen atom or a protecting group), R_{A51} is preferably a protecting group, more preferably an aromatic group, particularly preferably an oxybenzyl group (-O-CH₂-Ph).

In Formulas (2) and (2'), R_{A61} is -O-R_{A6} (R_{A6} represents a hydrogen atom, a hydrocarbon group or a protecting group), R_{A6} is preferably a hydrocarbon group or a protecting group. When R_{A6} is a hydrocarbon group, it is preferably an allyl group, and when R_{A9} is a protecting group, examples thereof include a tert-butyldimethylsilyl group (-Si(t-Bu) (CH₃)₂), a tert-butyldiphenylsilyl group (-Si(t-Bu)Ph₂), a benzyl group, methoxymethyl group (MOM) and the like. R_{A6} is preferably a tert-butyldimethylsilyl group.

In Formulas (2) and (2'), R_{A7} is preferably a hydrogen atom, an alkyl group having 1 to 5 carbon atoms, more preferably a hydrogen atom or an alkyl group having 1 to 3 carbon atoms, still more preferably a hydrogen atom, a methyl group or an ethyl group, particularly preferably a hydrogen atom.

In Formulas (2) and (2'), R_{A8} is preferably a hydrogen atom or an alkyl group having 1 to 5 carbon atoms, more preferably a hydrogen atom or an alkyl group having 1 to 3 carbon atoms, still more preferably a hydrogen atom, a methyl group or an ethyl group, particularly preferably a hydrogen atom.

In Formulas (2) and (2'), R_{A9} is preferably a hydrogen atom or an alkyl group having 1 to 5 carbon atoms, more preferably a hydrogen atom or an alkyl group having 1 to 3 carbon atoms, still more preferably a hydrogen atom, a methyl group or an ethyl group, particularly preferably a methyl group.

In Formulas (2) and (2'), R_{A10} is preferably a protecting group mentioned above, particularly preferably a 2-nitrobenzenesulfonyl group (a nosyl group).

In Formula (2), R_{A11} is preferably a hydrogen atom or an alkyl group having 1 to 5 carbon atoms, more preferably a hydrogen atom or an alkyl group having 1 to 3 carbon atoms, still more preferably a hydrogen atom, a methyl group or an ethyl group, particularly preferably a hydrogen atom.

In Formula (2), R_{A12} is preferably a protecting group, more preferably an aromatic group, particularly preferably a benzyl group.

In Formula (2), R_{A14} is preferably -(CH₂)ₙ-H or -(CH₂)ₙ-COOR_{A13} (n is 1 to 4), more preferably -(CH₂)₂-COOR_{A13}. R_{A13} is preferably a protecting group, more preferably an aromatic group, particularly preferably a benzyl group.

In Formula (2) and Formula (2'), the binding position of R_{A5} is not particularly limited, and can be, for example, a bonding position represented by the following Formula (2").

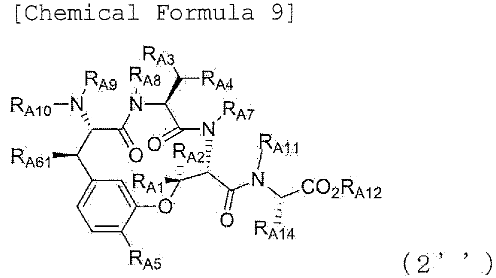

wherein R_{A1} to R_{A5}, R_{A7} to R_{A12} and R_{A14} in Formula (2") are defined as R_{A1} to R_{A5}, R_{A7} to R_{A12} and R_{A14} in Formula (2), respectively, and R_{A61} is defined as R_{A61} in Formula (2).

Step A comprises an oxidation reaction of the compound represented by Formula (2'). Specifically, the oxidation reaction is a reaction for oxidizing an alcohol to a carboxylic acid, whereby the hydroxyl group indicated by the arrow in the compound represented by Formula (2') is converted to a carboxyl group.

In Step A, the type of oxidation reaction is not particularly limited, and for example, a wide variety of known oxidation reactions of alcohol compounds can be used. The oxidation reaction may be, for example, a two-step reaction in which an alcohol is oxidized to an aldehyde, and then the aldehyde is oxidized to a carboxylic acid. When the oxidation reaction is carried out in two steps in this way, it is easy to prevent the occurrence of an unintended oxidation reaction of a functional group.

In Step A, the oxidation reaction preferably comprises Dess-Martin oxidation and Pinnick oxidation. This makes it easier to suppress racemization and allows oxidation to occur under mild reaction conditions. For example, the hydroxyl group is oxidized to an aldehyde by Dess-Martin oxidation, and then the aldehyde is oxidized to a carboxylic acid by Pinnick oxidation.

In Step A, when the oxidation reaction is carried out, the oxidizing agent is preferably used in an amount of 1 to 5 mol per 1 mol of the compound represented by Formula (2'). The type of the oxidizing agent is not particularly limited, and a wide variety of known oxidizing agents used in oxidation reactions can be used.

In the Dess-Martin oxidation, 1,1,1-triacetoxy-1,1-dihydro-1,2-benziodoxol-3(18)-one (DMP) is preferably used as an oxidizing agent. In the case of the Dess-Martin oxidation, the oxidizing agent is preferably used in an amount of 1 to 5 mol, more preferably 1 to 3 mol, per 1 mol of the compound represented by Formula (2').

In the Pinnick oxidation, sodium hypochlorite (NaClO₂) is preferably used as an oxidizing agent. In the case of the Pinnick oxidation, the oxidizing agent is preferably used in an amount of 1 to 5 mol, more preferably 2 to 5 mol, per 1 mol of the compound represented by Formula (2').

Other oxidizing agents can also be used in the oxidation reaction, for example, an oxidizing agent that combines a nitroxyl radical species such as 2,2,6,6-tetramethylpiperidine 1-oxyl with iodosobenzene diacetate can be exemplified.

In Step A, a solvent may be used as necessary in the oxidation reaction. Examples of the solvent in the oxidation reaction include chlorine-containing compounds such as dichloromethane, dichloroethane and the like, acetonitrile, and tert-butanol.

In Step A, the reaction temperature of the oxidation reaction is not particularly limited, and, for example, the reaction can be carried out at -20 to 60°C, preferably 0 to 30°C.

In Step A, after the oxidation reaction, the product obtained by the oxidation reaction (the carboxylic acid compound) is subjected to a condensation reaction with a compound having both a carboxy group and an amino group, or a salt or ester of the compound. Hereinafter, the compound having both a carboxy group and an amino group, or a salt or ester of the compound will be abbreviated as "Compound C".

Among Compounds C, the compounds having both a carboxy group and an amino group are not particularly limited, and examples thereof include amino acids. Preferred are glutamic acid and aspartic acid.

In particular, in Step A, it is preferable to subject the product (the carboxylic acid compound) obtained in the above-mentioned oxidation reaction to a condensation reaction with a glutamic acid ester or an aspartic acid ester. When a glutamic acid ester is used in the condensation reaction, the resulting cyclic peptide derivative by the general Formula (2) is a compound in which, in Formula (2), R_{A14} is -(CH₂)₂-COOR_{A13}. When an aspartic acid ester is used in the condensation reaction, the resulting cyclic peptide derivative represented by the general Formula (2) is compound in which, in Formula (2), R_{A14} is -(CH₂)-COOR_{A13}. In any case, R_{A13} is preferably a protecting group, more preferably an aromatic group, and particularly preferably a benzyl group.

In the condensation reaction of Step A, Compound C is preferably used in an amount of 1 to 5 mol, more preferably 2 to 5 mol, particularly preferably 3 to 4 mol, per 1 mol of the carboxylic acid compound obtained by the oxidation reaction.

For example, in Step A, when the product (the carboxylic acid compound) obtained by the above-mentioned oxidation reaction is subjected to a condensation reaction with a glutamic acid ester, the resulting cyclic peptide derivative represented by Formula (2) is represented by the following general Formula (2A).

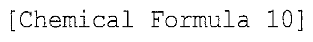

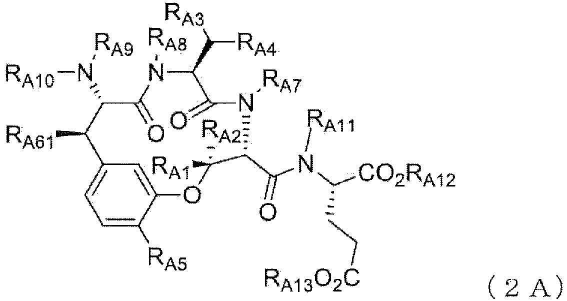

wherein R_{A1} to R_{A5}, R_{A61}, R_{A7} to R_{A13} and m in Formula (2A) are defined as R_{A1} to R_{A5}, R_{A61}, R_{A7} to R_{A13} and m in Formula (2), respectively.

In Step A, the method of the condensation reaction is not particularly limited, and for example, known condensation reaction conditions can be widely adopted. In order to easily suppress racemization and promote oxidation under mild reaction conditions, a condensing agent can also be used in the condensation reaction.

The type of condensing agent is not particularly limited, and for example, a wide variety of known condensing agents can be used. In particular, a phosphorus condensing agent is preferably used as a condensing agent for the condensation reaction of Step A. Examples of such condensing agents include 3-(diethoxyphosphoryloxy)-3H-benzo[d][1,2,3]triazin-4-one (depbt), 1-ethyl-3-(3-dimethylaminopropyl)carbodiimide hydrochloride, 4-(4,6-dimethoxy-1,3,5-triazin-2-yl)-4-methylmorpholinium chloride, 1-[bis(dimethylamino)methylene]-1H-1,2,3-triazolo[4,5-b]pyridinium 3-oxide hexafluorophosphate, 1-[bis(dimethylamino)methylene]-1H-benzotriazolium 3-oxide hexafluorophosphate, n-[1-(cyano-2-ethoxy-2-oxoethylideneaminooxy)dimethylamino(morpholino)]uronium hexafluorophosphate and the like. The phosphorus condensing agent is preferably a phosphoryl azide compound, particularly preferably DEPBT.

When a condensing agent is used, the amount to be used is not particularly limited. For example, the amount of the condensing agent to be used is preferably 1 to 3 mol, more preferably 1.2 to 2.5 mol, per 1 mol of the carboxylic acid compound obtained by the oxidation reaction.

A solvent can be used as necessary in the condensation reaction in Step A. The solvent is not particularly limited, and examples thereof include aliphatic hydrocarbons such as hexane, heptane and the like; alicyclic hydrocarbons such as cyclohexane and the like; aromatic hydrocarbons such as benzene, toluene and xylene and the like; chlorinated hydrocarbons such as dichloromethane, chloroform, 1,2-dichloroethane and the like; alcohols such as methanol, ethanol, isopropyl alcohol and t-butanol and the like; amide solvents such as N,N-dimethylacrylamide and the like; and the like.

The condensation reaction may be carried out in the presence of a basic catalyst such as diisopropylethylamine, dimethylaminopyridine and the like.

In Step A, the reaction temperature for the oxidation reaction is not particularly limited, and, for example, the reaction can be carried out at -20 to 60°C, preferably 0 to 30°C.

In Step A, the cyclic peptide derivative represented by Formula (2) can be produced in high yield by the oxidation reaction and subsequent condensation reaction. Conventionally, there has been no known method for stereoselectively reacting a cyclic peptide derivative formed by the linkage by condensation of a macrolactam structure containing a structure derived from a non-natural amino acid such as a β-hydroxydopa unit and a β-hydroxyisoleucine unit, and an amino acid derivative such as a glutamic acid derivative. In contrast, the present disclosure employs Step A using the compound represented by Formula (2'), thereby making it possible to produce the cyclic peptide derivative represented by Formula (2). Therefore, the cyclic peptide derivative represented by Formula (2) has a structure containing a β-hydroxydopa unit and a β-hydroxyisoleucine unit.

It is generally known that in biosynthesis, cyclic peptide derivatives are produced by binding all peptides and then cyclizing them. In contrast, the cyclic peptide derivative of the present disclosure is produced by binding Compound C (e.g., a glutamic acid ester) to a compound having a cyclized structure. In other words, the cyclic peptide derivative of the present disclosure is produced by binding Compound C at the last step, which has the advantage that cyclic peptide derivatives to which various amino acids and the like are bonded can be obtained depending on the type of Compound C. The route of constructing the ring structure in advance and then introducing the side chain (Compound C), as in the present disclosure, also has the advantage that it enables the simplification of the process for the desorption of the protecting group and gives the desired product in high yield.

### 2. Production method of cyclic peptide compound

The production method of the cyclic peptide compound of the present disclosure comprises a step of subjecting the cyclic peptide derivative obtained by the above-mentioned production method of the cyclic peptide derivative to a hydrogenation reduction reaction to obtain a cyclic peptide compound. That is, the production method of the cyclic peptide compound of the present disclosure comprises a step of subjecting the cyclic peptide derivative to a hydrogenation reduction reaction after the condensation reaction in Step A in the production method of the cyclic peptide derivative.

In the production method of the cyclic peptide compound, the method of hydrogenation reduction reaction is not particularly limited, and for example, known hydrogenation reduction reaction conditions can be widely adopted. For example, the hydrogenation reduction reaction can be carried out by using hydrogen in the presence of a catalyst. For example, a known catalyst used in hydrogenation reduction reaction can be used as a catalyst, and specific examples thereof include palladium on carbon. In the hydrogenation reduction reaction, a solvent can be used as necessary. Examples of the solvent include lower alcohols such methanol, ethanol and the like.

When the cyclic peptide derivative represented by Formula (2) obtained in the condensation reaction in Step A has a protecting group such as a tert-butoxycarbonyl group (Boc group), a tert-butyldimethylsilyl group (TBS group), a 2-nitrobenzenesulfonyl group (a nosyl group) and the like, the protecting group can may be deprotected in advance before carrying out the hydrogenation reduction reaction. The deprotection method is not particularly limited, and for example, a wide variety of known deprotection methods may be used. For example, deprotection of TBS group and deprotection of a nosyl group may be carried out in this order.

When the cyclic peptide derivative represented by Formula (2) has an alkynyl group (for example, R_{A2} of Formula (2)), the alkynyl group can be converted to an alkyl group by the above-mentioned hydrogenation reduction reaction. For example, when an acetynyl group is present in the cyclic peptide derivative represented by Formula (2), it can be converted to an ethyl group by the above-mentioned hydrogenation reduction reaction.

In one embodiment of the production method of the cyclic peptide compound of the present disclosure, for example, the cyclic peptide derivative represented by Formula (2) obtained by the condensation reaction in Step A is deprotected and then subjected to a hydrogenation reduction reaction. As a result, for example, a compound represented by the following Formula (10), and specifically, a compound represented by the following Formula (A) can be produced. 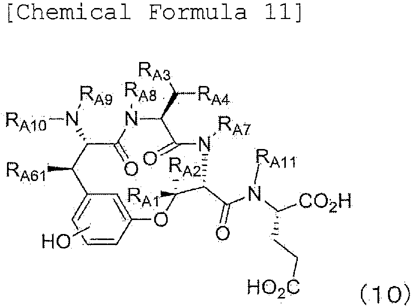 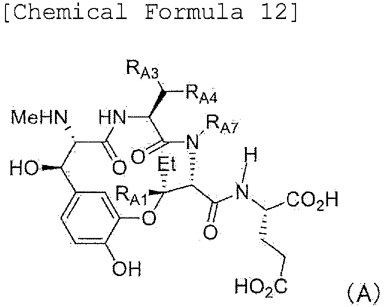
wherein R_{A1}, R_{A2}, R_{A3}, R_{A4}, R_{A61}, R_{A7}, R_{A8}, R_{A9}, R_{A10} and R_{A11} in Formula (10) are defined as R_{A1}, R_{A2}, R_{A3}, R_{A4}, R_{A61}, R_{A7}, R_{A8}, R_{A9}, R_{A10} and R_{A11} in Formula (2), respectively. In this case, R_{A61} is preferably a hydroxyl group.
wherein R_{A1}, R_{A3} and R_{A4} in Formula (A) are defined as R_{A1}, R_{A3} and R_{A4} in Formula (2). Specifically, the compound represented by Formula (A) is a compound in which, in Formula (2), R_{A5} is a hydroxyl group (m is 1), R_{A12} is a hydrogen atom, and R_{A14} is - (CH₂)₂-COOH. In Formula (A), R_{A2} is an ethyl group, R_{A61} is OH, R_{A7}, R_{A8}, R_{A9} and R_{A11} are hydrogen atoms, and R_{A10} is a methyl group, but is not limited thereto.

By the hydrogenation reduction reaction, when the cyclic peptide derivative represented by Formula (2) has an alkynyl group, the alkynyl group is converted to an alkyl group, and, for example, the ester moiety derived from Compound C is converted to a carboxylic acid. When R_{A5} has a protecting group (for example, a group having an ether structure such as an oxybenzyl group and the like), R_{A5} can be converted to a hydroxyl group by the hydrogenation reduction reaction (see Formula (A)).

The cyclic peptide compound obtained by the hydrogenation reduction reaction is not particularly limited as long as it is a compound that can be produced by hydrogenation reduction of the compound represented by Formula (2), and preferably is a compound in which, in Formula (2), R_{A5} is a hydroxyl group, R_{A12} is a hydrogen atom, and R_{A13} in COOR_{A13} in R_{A14} is a hydrogen atom. In this case, R_{A2} is preferably an ethyl group. In the cyclic peptide compound, R_{A14} is more preferably -(CH₂)₂-COOH or -CH₂-COOH. Most preferably, the cyclic peptide compound obtained by the hydrogenation reduction reaction is a compound represented by the general Formula (A) in which R_{A1}, R_{A3} and R_{A4} are all methyl groups.

In the production method of the cyclic peptide compound of the present disclosure, the cyclic peptide derivative obtained by the above-mentioned production method of the cyclic peptide derivative is subjecting to a hydrogenation reduction reaction, so that the cyclic peptide compound can be obtained in high yield. Moreover, depending on the type of Compound C used in the production method of the cyclic peptide derivative, a cyclic peptide compound to which various amino acids are bonded can be obtained.

### 3. Production method of raw materials used in the present disclosure

Below, an example of production method of the compound represented by Formula (2') used in Step A in the production method of the cyclic peptide derivative of the present disclosure will be described. The production method of the compound represented by Formula (2') is not particularly limited, and for example, a wide variety of known production methods can be used adopted.

When R_{A6} is a protecting group, the production method of the compound represented by Formula (2') comprises, for example, a step of subjecting a cyclisation precursor represented by the following Formula (21a) to an intramolecular cyclization reaction to obtain a compound represented by the following Formula (22a) (hereinafter referred to as cyclization step).

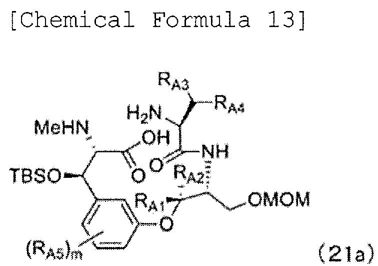

wherein R_{A1} to R_{A5} and m in Formula (21a) are defined as R_{A1} to R_{A5} and m in Formula (1), respectively. In Formula (21a), MOM represents a methoxymethyl group (hereinafter the same). TBS represents a tert-butyldimethylsilyl group.

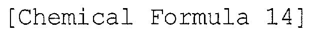

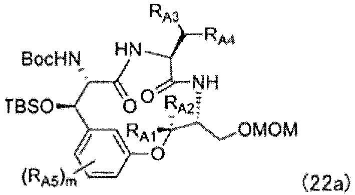

wherein R_{A1} to R_{A5} and m in Formula (22a) are defined as R_{A1} to R_{A5} and m in Formula (1).

In the cyclization step, by intramolecular cyclization reaction of the cyclisation precursor represented by Formula (21a), an amide bond is formed between the β-hydroxyisoleucine unit and the β-hydroxydopa unit to give the compound represented by Formula (22a).

Regarding the intramolecular cyclization reaction, it was known that the yield was only 10 to 20% in a conventional method (e.g., the method described in P. Li, C. D. Evans, M. M. Joullie, Org. Lett., 2005, 7, 5325) and a large amount of dimer of the cyclization precursor was by-produced. In addition, in other methods (P. Li, C. D. Evans, Y. Wu, B. Cao, E. Hamel, M. M. Joullie, J. Am. Chem. Soc., 2008, 130, 2351), the yield improved to 30 to 40%, but still did not exceed 40%.

In this respect, in the present disclosure, in the cyclization step, a dilute substrate solution is dropped into a solvent containing a binder to dilute the concentration of the substrate (i.e., the compound represented by Formula (21a)), suppressing the progress of intermolecular reactions while promoting the progress of intramolecular cyclization reactions, thereby enabling an intramolecular cyclization reaction with a higher yield than conventional methods. It is presumed that such intramolecular cyclization reactions suppress intermolecular reactions, thereby increasing the yield of the target cyclized compound (i.e., the compound represented by Formula (22a)).

In the cyclization step, the type of binder is not particularly limited, and a wide variety of binders used in intramolecular cyclization reactions can be used. Examples of the binder include 1-(3-dimethylaminopropyl)-3-ethylcarbodiimide hydrochloride, (benzotriazol-1-yloxy)tripyrrolidinophosphonium hexafluorophosphate, 3-(diethoxyphosphoryloxy)-1,2,3-benzotriazin-4(3H)-one (DEPBT).

The solvent used to dissolve the binder and the substrate in the cyclization step is not particularly limited, and is preferably a polar solvent such as chlorinated hydrocarbons such as dichloromethane, chloroform, 1,2-dichloroethane and the like; alcohols such as methanol, ethanol, isopropyl alcohol and t-butanol and the like; amide solvents such as N,N-dimethylacrylamide and the like; and the like. The solvent may be used alone or in combination of two or more kinds.

The concentration of the binder may be, for example, within the range of 1 mM to 15 mM based on the solvent used, and the concentration of the substrate may be within the range of 0.1 mM to 1.5 mM based on the solvent used.

The reaction temperature for the intramolecular cyclization reaction is not particularly limited, and, for example, the reaction can be carried out at -20 to 60°C, preferably 0 to 30°C. The reaction time can be appropriately set depending on the reaction temperature and the like, and for example, the substrate can be added dropwise over 6 to 24 hours, and then the reaction can be continued for 6 to 24 hours, but is not limited thereto. In particular, by carrying out the reaction over a long period at a low temperature, the intramolecular cyclization reaction is promoted, and the yield of the cyclized compound (the compound represented by Formula (22a)) can be improved by about 1.5 to 2.5 times compared to conventional cyclization reactions.

The compound represented by Formula (2) can be obtained by subjecting the compound represented by Formula (21a) to an intramolecular cyclization reaction, and then deprotecting the MOM of the resulting compound represented by Formula (22a). The deprotection method of the MOM is not particularly limited, and for example, the same conditions as those for known methods for deprotecting the MOM can be used. Before deprotecting the MOM, for example, the protecting group in the compound represented by Formula (22a) can also be replaced with another protecting group (for example, the Boc group substituted on the N atom can be replaced with a nosyl group). Furthermore, before deprotecting the MOM, the N atom to which the protecting group such as a nosyl group is bonded can also be methylated. For example, a wide variety of known methods can be used for the methylation, and an example of methylation is methyl p-nitrobenzenesulfonate.

The production method of the compound represented by Formula (21a) used in the cyclization step is not particularly limited, and for example, the compound represented by Formula (21a) can be obtained by a known reaction. As an example, the compound represented by Formula (21a) can be obtained through a step of reacting a compound represented by the following Formula (7a) and a compound represented by the following Formula (8a).

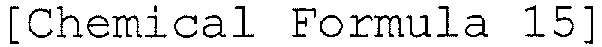

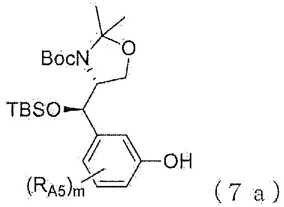

wherein R_{A5} and m in Formula (7a) are defined as R_{A5} and m in Formula (1). The compound represented by Formula (7a) is a β-hydroxydopa unit. 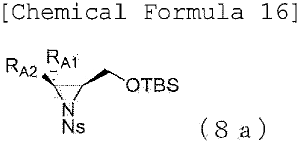 wherein, R_{A1} and R_{A2} in Formula (8a) are defined as R_{A1} and R_{A2} in Formula (2). The compound represented by Formula (8a) is an aziridine compound.

The reaction of the compound represented by Formula (7a) and the compound represented by Formula (8a) can be carried out under conditions similar to those of known ring-opening reactions. This ring-opening reaction can be carried out, for example, in the presence of 1,5,7-triazabicyclo[4.4.0]dec-5-ene (TBD).

In the case where R_{A2} in Formula (8a) is an acetynyl group, the nucleophilic reaction rate is extremely fast, and therefore the nucleophilic addition reaction between the β-hydroxydopa unit and the aziridine proceeds quickly, and the yield of the reaction product is also high. Therefore, the production method of the present disclosure has the advantage that aziridine that has not reacted with an amino acid can be used.

After the reaction of the compound represented by Formula (7a) and the compound represented by Formula (8a), a compound represented by the following Formula (13a) is synthesized via an introduction reaction of a protecting group (MOM). The reaction conditions for introducing the protecting group are not particularly limited, and can be the same as those of a known method. 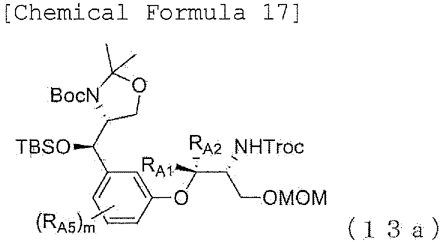 wherein R_{A1}, R_{A2}, R_{A5} and m in Formula (13a) are defined as R_{A1}, R_{A2}, R_{A5} and m in Formula (2).

Thereafter, the compound represented by Formula (13a) is subjected to a deprotection reaction and an esterification reaction to obtain a compound represented by the following Formula (18a). The conditions for the deprotection reaction and the esterification reaction are not particularly limited and may be the same as those of a known method. 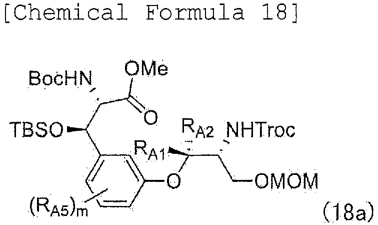 wherein R_{A1}, R_{A2}, R_{A5} and m in Formula (18a) are defined as R_{A1}, R_{A2}, R_{A5} and m in Formula (2) .

The obtained compound represented by Formula (18a) is deprotected at the Troc group (2,2,2-trichloroethoxycarbonyl group), and then condensed with, for example, a valine compound having a 9-fluorenylmethyloxycarbonyl group, followed by further deprotection to produce the compound represented by Formula (21a) (the cyclization precursor). In this condensation reaction, an appropriate condensing agent can be used.

When R_{A2} is an acetynyl group, it may be reduced as necessary. This reduction can be carried out in the hydrogenation reduction reaction step after the condensation reaction in Step A described above, or can be carried out at any time after the nucleophilic addition reaction with the aziridine. In this regard, when R_{A2} is an acetynyl group, the acetynyl group is relatively not bulky compared to an alkyl group, and therefore does not inhibit the reaction, so that the acetynyl group can be maintained without being reduced until the hydrogenation reduction reaction.

The production method of the compound represented by Formula (7a) is not particularly limited. For example, the compound can be produced by a reaction using a compound represented by the following Formula (1a) and a compound represented by the following Formula (2a) as starting materials. 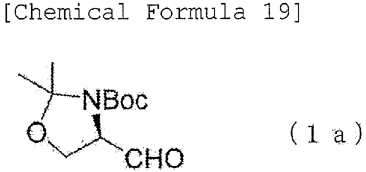 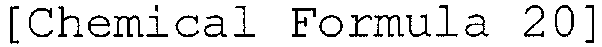 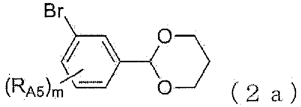 wherein R_{A5} and m in Formula (2a) are defined as R_{A5} Formula (2').

The compound represented by Formula (8a) can be obtained, for example, by a known production method, or can be obtained from a commercial product.

### (Pharmaceutical composition)

The composition and method of the present disclosure can be utilized for treating an individual in need thereof. In a specific embodiment, an individual is a mammal such as a human or non-human mammal. If administered to an animal such as a human, the composition or compound is preferably administered as a pharmaceutical composition preferably comprising, for example, the compound of the present disclosure and a pharmaceutically acceptable carrier. Pharmaceutically acceptable carriers are well known in the art. Examples thereof include aqueous solutions such as water and buffered saline, and other solvents and vehicles such as glycol, glycerol, oil such as olive oil, and organic esters for injection. In a preferred embodiment, an aqueous solution does not, or substantially does not, contain a pyrogen if such a pharmaceutical composition is for administration to a human, especially for administration through an invasive route (e.g., route such as injection or implantation that avoids transport or diffusion through the epithelial barrier). An excipient can be selected, for example, to achieve delayed release of an agent or to selectively target one or more cells, tissues, or organs. A pharmaceutical composition can be a unit dose, such as a tablet, capsule (including sprinkle capsule and gelatin capsule), granule, lyophilized form for reconstitution, powder, liquid agent, syrup, suppository, injection, or the like. A composition can also be in a transdermal delivery system such as a skin patch. A composition can also be a suitable liquid agent for topical administration, such as an eye drop.

A pharmaceutically acceptable carrier can comprise a physiologically acceptable agent, which has an effect of, for example, stabilizing, increasing the solubility, or increasing the absorption of a compound such as the compound of the present disclosure. Examples of such a physiologically acceptable agent include carbohydrates such as glucose, sucrose, and dextran, antioxidants such as ascorbic acid and glutathione, chelating agents, low molecular weight proteins, other stabilizers, excipients, and the like. Selection of a pharmaceutically acceptable carrier including physiologically acceptable carrier is dependent on, for example, the route of administration of a composition. A preparation or pharmaceutical composition can be a self-emulsifying drug delivery system or self-microemulsifying drug delivery system. A pharmaceutical composition (preparation) can also be a liposome or other polymer matrix, and a compound of the present disclosure can be incorporated therein. A liposome such as a liposome comprising a phospholipid or another lipid is a nontoxic, physiologically acceptable, and metabolizable carrier that is relatively easy to prepare and administer.

A pharmaceutical composition (preparation) can be administered to a subject through any of several routes of administration including, for example, oral administration (e.g., oral medicine, tablet, capsule (including sprinkle capsule and gelatin capsule), bolus, powder, granule, or paste agent for application on the tongue in an aqueous or nonaqueous liquid agent of suspension); absorption through an oral mucous membrane (e.g., sublingual administration); rectal, through the anus, or vaginal administration (e.g., as a pessary, cream, foam, or the like); parenteral administration (including intramuscular, intravenous, subcutaneous, and intraspinal cavity administration as, for example, sterilized liquid agent or suspension); intranasal administration; intraperitoneal administration; subcutaneous administration; transdermal administration (e.g., as a patch applied to the skin); topical administration (e.g., cream, ointment, or spray applied to the skin, and eye drop); and the like. A compound can also be formulated for inhalation. In a specific embodiment, a compound only needs to be dissolved or suspended in sterilized water. Details for suitable routes of administration and composition that are suited thereto can be found in, for example, U.S. Pat. Nos. 6,110,973, 5,731,000, 5,541,231, 5,427,798, 5,358,970, and 4,172,896, and patents cited therein.

A formulation can be provided in a convenient unit dosage form and prepared by any method that is well known in the pharmaceutical field. The amount of active ingredient that can form a unit dose in combination with a carrier varies depending on the host being treated or specific dosing mode. The amount of active ingredient that can form a unit dose in combination with a carrier is generally an amount of compound that results in a therapeutic effect. In general, such an amount is in the range of about 1 percent to about 99 percent active ingredient, preferably about 5 percent to about 70 percent active ingredient, and most preferably about 10 percent to about 30 percent active ingredient with respect to 100 percent.

A method of preparing such formulations or compositions comprises associating an active compound such as the compound of the present disclosure with a carrier and optionally one or more secondary ingredients. In general, a formulation is prepared by associating the compound of the present disclosure with a liquid carrier or a finely divided solid carrier or both in a uniform and dense manner and then optionally molding a product.

The formulation of the present disclosure that is suitable for oral administration can be in a form of a capsule (including sprinkle capsule and gelatin capsule), cachet, pill, tablet, lozenge (flavored base, generally using sucrose and acacia or tragacanth), lyophilized formulation, powder, granule, liquid agent or suspension in an aqueous or nonaqueous liquid, oil-in-water or water-in-oil liquid emulsion, elixir or syrup, pastille (using an inert base such as gelatin or glycerin, or sucrose and acacia), and/or mouthwash. Each of them contains a determined amount of the compound of the present disclosure as an active ingredient. The composition or compound can also be administered as a bolus, lozenge, or paste.

To prepare a solid dosage form for oral administration (capsule (including sprinkle capsule and gelatin capsule), tablet, pill, sugar-coated tablet, powder, granule, or the like), an active ingredient is mixed with one or more pharmaceutically acceptable carriers such as sodium citrate or calcium monohydrogen phosphate, and/or one of the following: (1) a filler or bulking agent such as starch, lactose, sucrose, glucose, mannitol, and/or silicic acid; (2) binding agent, such as carboxymethyl cellulose, alginate, gelatin, polyvinylpyrrolidone, sucrose, and/or acacia; (3) moisturizing agent such as glycerol; (4) disintegrant such as agar, calcium carbonate, potato starch or tapioca starch, alginic acid, specific silicate, and sodium carbonate; (5) dissolution retardant such as paraffin; (6) absorption promoting agent such as a quaternary ammonium compound; (7) humectant such as cetyl alcohol and glycerol monostearate; (8) absorbent such as kaolin or bentonite clay; (9) lubricant such as talc, calcium stearate, magnesium stearate, solid polyethylene glycol, sodium lauryl sulfate, or a mixture thereof; (10) complexing agent such as modified or unmodified cyclodextrin; and (11) colorant. For a capsule (including sprinkle capsule and gelatin capsule), tablet, and pill, a pharmaceutical composition can also comprise a buffer. A same type of solid composition can also use an excipient such as lactose or milk sugar, high molecular weight polyethylene glycol, and the like as a filler in a soft or hard filled gelatin capsule.

A tablet can be prepared by compressing or molding the active ingredient together with one or more optional secondary ingredients. A compressed tablet can be prepared by using a binding agent (e.g., gelatin or hydroxypropyl methylcellulose), lubricant, inert diluent, preservative, disintegrant (e.g., sodium carboxymethyl starch or crosslinked sodium carboxymethylcellulose), surface activator, or dispersant. A molded tablet can be prepared by molding a mixture of a powder compound moisturized with an inert liquid diluent in a suitable instrument.

A tablet or other solid dosage form of a pharmaceutical composition such as a sugar-coated tablet, capsule (including sprinkle capsule and gelatin capsule), pill, or granule can be optionally notched or prepared using a coating or shell such as an enteric coating or other coating that is known in pharmaceutical product formulation technologies. The tablet or solid dosage form can also be formulated to provide sustained release or controlled release of the active ingredient by using, for example, hydroxypropyl methylcellulose, other polymer matrix, liposome, and/or microsphere, which contains the active ingredient, at different ratios to provide a desired release profile. The tablet or solid dosage form can be sterilized, for example, by filtration through a bacteria retaining filter or by incorporating a sterilizing agent in a form of a sterilized solid composition that can be dissolved in sterilized water or another medium for sterilized injection immediately prior to use. These compositions can also be a composition, which optionally comprises an emulsifying agent and releases an active ingredient (s) only at, or preferentially at, a specific part of a digestive tract, optionally in a delayed manner. Examples of embedded composition that can be used include polymeric substances and wax. An active ingredient can be in a microcapsule form by using one or more types of excipients described above when suitable.

Examples of liquid dosage forms that are useful for oral administration include a pharmaceutically acceptable emulsion, lyophilized form for reconstitution, microemulsion, liquid agent, suspension, syrup, and elixir. In addition to the active ingredient, a liquid dosage form can comprise an inert diluent that is commonly used in the art such as water or another solvent, cyclodextrin or a derivative thereof, solubilizing agent or emulsifying agent such as ethyl alcohol, isopropyl alcohol, ethyl carbonate, ethyl acetate, benzyl alcohol, benzyl benzoate, propylene glycol, 1,3-butylene glycol, oil (especially, cottonseed oil, peanut oil, corn oil, germ oil, olive oil, castor oil, or sesame oil), glycerol, tetrahydrofuryl alcohol, polyethylene glycol, sorbitan fatty acid ester, a mixture thereof, or the like.

In addition to an inert diluent, an oral composition can also comprise an adjuvant such as a humectant, emulsifying agent, suspending agent, sweetener, flavoring agent, colorant, fragrance, preservative, and the like.

In addition to an active compound, a suspension can comprise a suspending agent such as ethoxylated isostearyl alcohol, polyoxyethylene sorbitol, sorbitan ester, microcrystalline cellulose, aluminum metahydroxide, bentonite, agar, tragacanth, mixture thereof, or the like.

A formulation of a pharmaceutical composition for rectal, vaginal, or urethral administration can be given as a suppository, which can be prepared by mixing one or more active compounds with one or more suitable non-stimulatory excipients or carriers, including for example, cocoa butter, polyethylene glycol, suppository wax, salicylate, or the like. The formulation is a solid at room temperature, but a liquid at body temperature, so that the formulation melts in the rectum or vaginal cavity and releases an active compound.

A formulation of a pharmaceutical composition for oral administration can be given as a mouthwash, oral spray, or oral ointment.

Instead of or in addition, the composition can be formulated for delivery via a catheter, stent, wire, or another intracavity device. Delivery via such a device can be particularly useful for delivery to the bladder, urethra, urinary tract, rectum, or intestine.

A formulation that is suitable for transvaginal administration includes a pessary, tampon, cream, gel, paste, foam, or spray formulation, which comprises a carrier that is known to be suitable in the art.

A dosage form for topical or transdermal administration includes powder, spray agent, ointment, paste, cream, lotion, gel, liquid agent, patch, and inhalant. An active compound can be mixed with a pharmaceutically acceptable carrier and any preservative, buffer, or aerosol agent that may be needed under sterilized conditions.

In addition to an active compound, an ointment, paste, cream, and gel can contain an excipient such as an animal or vegetable oil and fat, wax, paraffin, starch, tragacanth, cellulose derivative, polyethylene glycol, silicone, bentonite, silicic acid, talc, zinc oxide, mixture thereof, or the like.

In addition to an active compound, powder and spray can contain an excipient such as lactose, talc, silicic acid, aluminum hydroxide, calcium silicate, polyamide powder, mixture thereof, or the like. A spray can further contain conventional aerosol agent such as chlorofluorohydrocarbon or volatile unsubstituted hydrocarbon such as butane or propane.

A transdermal patch has given an advantage of providing controlled delivery of the compound of the present disclosure to the body. Such a dosage form can be prepared by dissolving or dispersing an active compound in a suitable medium. The flux of a compound crossing the skin can be increased by using an absorption enhancing agent. Such a flux rate can be controlled either by providing a rate controlling membrane or by dispersing a compound within a polymer matrix or gel.

An ophthalmic formulation, ophthalmic ointment, powder, liquid agent, and the like are also envisioned to be within the scope of the present disclosure. Exemplary ophthalmic formulations are described in US Patent Application Publication Nos. 2005/0080056, 2005/0059744, 2005/0031697, and 2005/004074, and U.S. Pat. No. 6,583,124 (content of which is incorporated herein by reference). When desirable, a liquid ophthalmic formulation has the same property as a lachrymal fluid, aqueous humor, or vitreous humor, or is compatible with such fluids. The preferred route of administration is administration to the affected part (e.g., topical administration such as eye drops or administration through an implant).

The present disclosure has been described while showing preferred embodiments to facilitate understanding. While the present disclosure is described hereinafter based on the Examples, the above descriptions and the following Examples are provided for the sole purpose of exemplification, not limitation of the present disclosure. Thus, the scope of the present disclosure is not limited to the embodiments and Examples that are specifically described herein and is limited only by the scope of claims.

### [Examples]

In the examples, for ease of description, the abbreviations shown above and the abbreviations shown below may be used.
Ac: acetyl
AcOH: acetic acid
aq.: aqueous solution
Arg: arginine
Asp: aspartic acid
BHT: dibutylhydroxytoluene
Bn: benzyl
Boc: tert-butoxycarbonyl
Boc2O: di-tert-butyl dicarbonate
Bzl: benzyl
Cbz: benzyloxycarbonyl
CPME: cyclopentyl methyl ether
DBU: diazabicycloundecene
DEPBT: 3-(diethoxyphosphoryloxy)-1,2,3-benzotriazin-4(3H)-one
DIAD: diisopropyl azodicarboxylate
DIPEA: N,N-diisopropylethylamine
DMAP: 4-dimethylaminopyridine
DMEAD: bis(2-methoxyethyl) azodicarboxylate
DME: 1,2-dimethoxyethane
DMF: N,N-dimethylformamide
DMP: 2,2-dimethoxypropane
DMT-MM: 4-(4,6-dimethoxy-1,3,5-triazin-2-yl)-4-methylmorpholinium chloride
DMT-MMT: 4-(4,6-dimethoxytriazin-2-yl)-4-methylmorpholine tetrafluoroborate
EDCI: 1-(3-dimethylaminopropyl)-3-ethylcarbodiimide hydrochloride
Et: ethyl
eq.: equivalent
Fmoc: 9-fluorenylmethyloxycarbonyl
Gln: glutamine
Glu: glutamic acid
HATU: 1-[bis(dimethylamino)methylene]-1H-1,2,3-triazolo[4,5-b]pyridinium 3-oxide hexafluorophosphate
HBTU: 1-[bis(dimethylamino)methylene]-1H-benzotriazolium 3-oxide hexafluorophosphate
HOAt: 1-hydroxy-7-azabenzotriazole
HOBt: 1-hydroxybenzotriazole
m-CPBA: metachloroperbenzoic acid
Me: methyl
MS: molecular sieve
MTBE: methyl tert-butyl ether
NMM: N-methylmorpholine
Ns: 2-nitrobenzenesulfonyl
PMB: paramethoxybenzyl
Ser: serin
Su: succinimide
TBAF: tetrabutylammonium fluoride
TBAI: tetrabutylammonium iodide
TBD: 1,5,7-triazabicyclo[4.4.0]dec-5-ene
TBS: tert-butyldimethylsilyl
t-Bu: tert-butyl
TFA: trifluoroacetic acid
THF: tetrahydrofuran
Thr: threonine
TEMPO: 2,2,6,6-tetramethylpiperidine-1-oxyl radical
Tr: trityl
Trt: trityl
Ts: tosyl
TsOH: paratoluenesulfonic acid
Tyr: tyrosine
Pbf: 2,2,4,6,7-pentamethyldihydrobenzofuran-5-sulfonyl
Ph: phenyl
PyBOP: (benzotriazol-1-yloxy)tripyrrolidinophosphonium hexafluorophosphate
PyBrop: bromo-tris-pyrrolidino-phosphonium hexafluorophosphate Val: valine

Fragment C and Fragment D groups were obtained from the following sources.

**[Table 2A]**

| **Fragment number** | **Product Name** | **Seller** |
|---|---|---|
| Fragment C-1 | N-[(9H-Fluoren-9-ylmethoxy)carbonyl]-L-valine | TCI |
| Fragment C-1' | N-(tert-Butoxycarbonyl)-L-valine | TCI |
| Fragment C-1'' | L-Valine Methyl Ester Hydrochloride | NACALAI |
| Fragment C-1d | D-Valine Methyl Ester Hydrochloride | TCI |
| Fragment C-3 | N-[(9H-Fluoren-9-ylmethoxy)carbonyl]glycine | TCI |
| Fragment C-3' | N-(tert-Butoxycarbonyl)glycine | TCI |
| Fragment C-6 | N-[(9H-Fluoren-9-ylmethoxy)carbonyl]-L-phenylalanine | TCI |
| Fragment C-7 | Fmoc-O-benzyl-L-serine | NACALAI |
| Fragment C-8 | Fmoc-O-benzyl-L-threonine | NACALAI |
| Fragment C-9 | 5-tert-Butyl N-[(9H-Fluoren-9-ylmethoxy)carbonyl]-L-glutamate | TCI |
| Fragment C-10 | N-[(9H-Fluoren-9-ylmethoxy)carbonyl]-N-methyl-L-valine | TCI |
| Fragment C-11 | Fmoc-O-benzyl-L-tyrosine | NACALAI |
| Fragment C-13 | Nα-FMOC-Nδ-trityl-L-glutamine | FUJIFILM Wako Pure Chemical |
| Fragment C-14 | Nα-[(9H-Fluoren-9-ylmethoxy)carbonyl]-Nω-(2,2,4,6,7-pentamethyldihydrobenzofuran-5-sulfonyl)-L-arginine | TCI |
| Fragment C-15 | L-alanine Methyl Ester Hydrochloride | TCI |
| Fragment C-16 | L-leucine Methyl Ester Hydrochloride | TCI |
| Fragment C-17 | L-Isoleucine Methyl Ester Hydrochloride | TCI |
| Fragment C-18 | L-Tryptophan Methyl Ester Hydrochloride | TCI |

**[Table 2B]**

| **Fragment number** | **Product Name** | **Seller** |
|---|---|---|
| Fragment D-1 | D-benzyl L-Glutamate Hydrochloride | TCI |
| Fragment D-2 | H-Asp(OBzl)-OBzl hydrochloride | FUJIFILM Wako Pure Chemical |
| Fragment D-2' | 1,4-Dibenzyl L-Aspartate p-Toluenesulfon | TCI |
| Fragment D-4 | H-g-Abu-Obzl,TosOH | FUJIFILM Wako Pure Chemical |
| Fragment D-5 | (R)-Dibenzyl 2-aminopentanedioate hydrochloride | FUJI FILM Wako Pure Chemical |
| Fragment D-6 | D-Aspartic acid dibenzyl ester p-toluenesulfonate | FUJIFILM Wako Pure Chemical |
| Fragment D-7 | Dibenzyl L-Aspartate Hydrochloride | TCI |

### (Example 1: Synthesis of Fragment A-2.)

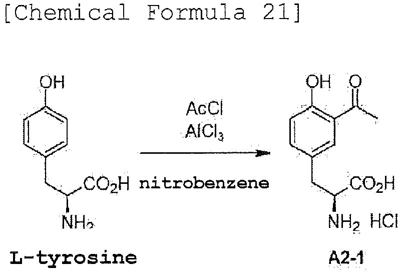

To a solution of L-tyrosine (1.0 eq., 6.51 g, 35.9 mmol) in nitrobenzene (130 mL) were added aluminium chloride (4.0 eq., 19.1 g, 143 mmol) and acetyl chloride (1.2 eq., 3.42 g, 43.6 mmol) under ice-cooling, and the mixture was stirred for 10 min while warming to room temperature. The reaction solution was warmed to 100°C, and stirred for 8 hr, and stirred for 16 hr while cooling to room temperature. The reaction solution was ice-cooled, and water (200 mL) was added, and the mixture was washed once with ethyl acetate (300 mL) by liquid separation, and the organic layer was extracted once with water (100 mL) by liquid separation. The aqueous layers were combined to give A2-1 as an aqueous solution (300 mL).

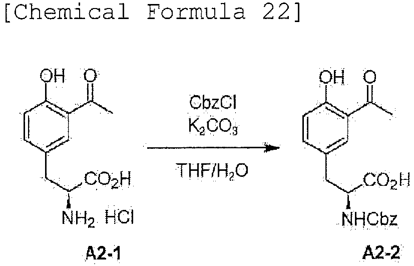

A2-1 (1.0 eq., aqueous solution 300 mL, calculated as 35.9 mmol) was adjusted to pH 9 by addition of potassium carbonate (7.5 eq., 36.9 g, 267 mmol) under ice-cooling, and THF (150 mL) and CbzCl (1.2 eq., 7.3 g, 42.8 mmol) were added, and the mixture was stirred at room temperature for 3 hr. The reaction solution was adjusted to pH 3 by addition of 2N aqueous hydrochloric acid solution (200 mL), and the THF was evaporated under reduced pressure. The concentrate was extracted three times with ethyl acetate (200 mL) by liquid separation, and the organic layers were combined, washed once with saturated aqueous sodium chloride solution (200 mL) by liquid separation, and dried over magnesium sulfate. The magnesium sulfate was removed by filtration, and the filtrate was concentrated under reduced pressure to give A2-2 (11.4 g) as a brown oil.

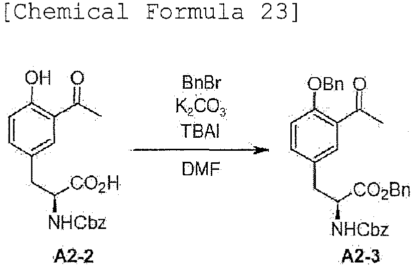

Under nitrogen atmosphere, to a solution of A2-2 (1.0 eq., 11.4 g, calculated as 35.9 mmol) in DMF (50 mL) were added potassium carbonate (3.0 eq., 14.9 g, 108 mmol), TBAI (0.10 eq., 1.33 g, 3.59 mmol) and BnBr (2.2 eq., 13.5 g, 79.1 mmol) under ice-cooling, and the mixture was stirred at room temperature for 3.5 hr. To the reaction solution was added water (100 mL), and the mixture was extracted twice with a mixture of hexane (40 mL)/ethyl acetate (80 mL) by liquid separation, and the organic layers were combined, and washed once with saturated aqueous sodium chloride solution (100 mL) by liquid separation, and dried over magnesium sulfate. The magnesium sulfate was removed by filtration, and the filtrate was concentrated under reduced pressure to give a crude product (23.2 g). The crude product was purified by flash silica gel column (normal phase silica gel 200 g, hexane/ethyl acetate = 80/20 - 50/50) to give A2-3 (9.56 g, three-step yield from L-tyrosine 50%) as a yellow viscous product. In the present specification, all mixtures are expressed as a mixture of solvent A (X mL)/solvent B (Y mL).

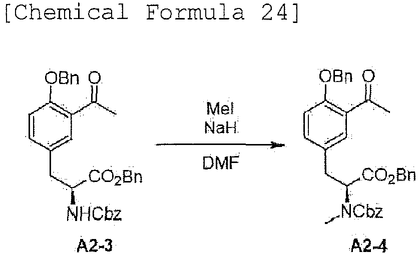

Under nitrogen atmosphere, to a solution of A2-3 (1.0 eq., 8.88 g, 16.5 mmol) in DMF (80 mL) were added sodium hydride (1.2 eq., 60%, dispersion in Paraffin Liquid, 796 mg, 19.9 mmol) and methyl iodide (3.0 eq., 7.07 g, 49.8 mmol) under ice-cooling, and the mixture was stirred under ice-cooling for 1.5 hr. The reaction solution was quenched by addition of methanol (12 mL), water (100 mL) was added, and the mixture was extracted twice with a mixture of hexane (50 mL)/ethyl acetate (100 mL) by liquid separation. The organic layers were combined, and washed once with saturated aqueous sodium chloride solution (100 mL) by liquid separation, and dried over magnesium sulfate. The magnesium sulfate was removed by filtration, and the filtrate was concentrated under reduced pressure to give a crude product (13.1 g). The crude product was purified by flash silica gel column (normal phase silica gel 50 g, hexane/ethyl acetate = 90/10 - 0/100) to give A2-4 (9.54 g) as an orange liquid.

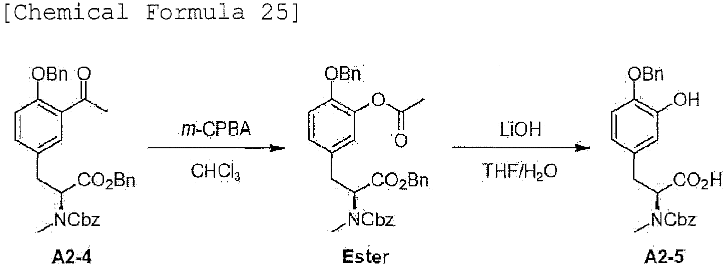

To a solution of A2-4 (1.0 eq., 10.1 g, calculated as 17.6 mmol) in chloroform (90 mL) was added m-CPBA (3.0 eq., 35% water content, 14.0 g, 52.7 mmol) at room temperature, and the mixture was stirred under reflux conditions for 5 hr. To the reaction solution were added water (50 mL) and saturated aqueous sodium hydrogencarbonate solution (100 mL) at room temperature, and the chloroform was evaporated under reduced pressure. The reaction solution was extracted three times with ethyl acetate (100 mL) by liquid separation, and the organic layers were combined, washed once with and saturated aqueous sodium chloride solution (100 mL) by liquid separation, and dried over magnesium sulfate. The magnesium sulfate was removed by filtration, and the filtrate was concentrated under reduced pressure to give Ester (10.6 g).

To a solution of Ester (1.0 eq., 10.6 g, calculated as 17.6 mmol) in a mixture of THF (40 mL)/water (40 mL) was added lithium hydroxide (4.0 eq., 1.68 g, 70.3 mmol) at room temperature, and the mixture was stirred at the same temperature for 16 hr. The reaction solution was washed twice with hexane (100 mL) by liquid separation, and the aqueous layer was adjusted to pH 2 by addition of 6N aqueous hydrochloric acid solution (11 mL). The aqueous layer was extracted three times with ethyl acetate (100 mL) by liquid separation, and the organic layers were combined, washed once with saturated aqueous sodium chloride solution (100 mL) by liquid separation, and dried over magnesium sulfate. The magnesium sulfate was removed by filtration, and the filtrate was concentrated under reduced pressure to give A2-5 (9.3 g) as an orange solid. 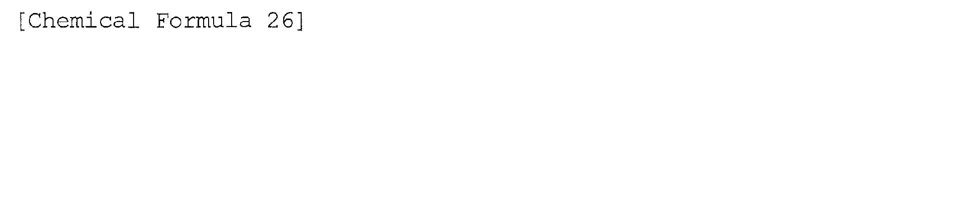 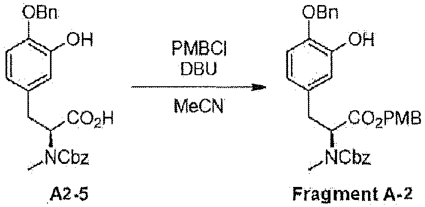 To a solution of A2-5 (1.0 eq., 9.3 g, calculated as 17.6 mmol) in acetonitrile (80 mL) were added DBU (1.1 eq., 2.96 g, 19.4 mmol) and PMBCl (1.1 eq., 3.00 g, 19.2 mmol) at room temperature, and the mixture was stirred at the external temperature of 60°C for 19 hr. The reaction solution was quenched by addition of acetic acid (3.0 eq., 3.15 g, 52.5 mmol), and concentrated under reduced pressure to give a crude product (16.7 g). The crude product was purified twice by flash silica gel column (1st run: normal phase silica gel 120 g, hexane/ethyl acetate = 75/25 - 50/50, 2nd run: normal phase silica gel 30 g, hexane/ethyl acetate = 75/25 - 67/23), and the obtained Fragment A-2 was dissolved in ethyl acetate (50 mL), and washed once with saturated aqueous sodium hydrogencarbonate solution (50 mL) by liquid separation. The aqueous layer was extracted twice with ethyl acetate (50 mL) by liquid separation, and the organic layers were combined, washed once with saturated aqueous sodium chloride solution (50 mL) by liquid separation, and dried over magnesium sulfate. The magnesium sulfate was removed by filtration, and the filtrate was concentrated under reduced pressure to give Fragment A-2 (3.54 g, three-step yield from A2-3 36%) as a yellow oil.

### (Example 2A: Synthesis of Fragment A-2')

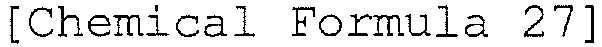

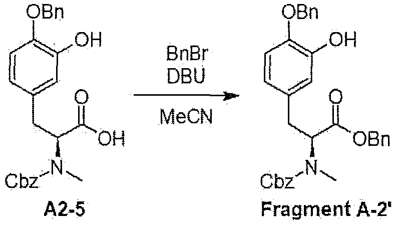

To a solution of A2-5 (1.0 eq., 3.4 g, calculated as 7.12 mmol) in acetonitrile (70 mL) were added DBU (1.5 eq., 1.6 mL, 10.7 mmol) and BnBr (1.2 eq., 1.0 mL, 8.54 mmol) at room temperature, and the mixture was stirred at room temperature for 16 hr. The mixture was quenched by addition of saturated aqueous ammonium chloride solution (30 mL), and washed three times with water (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (4.83 g). The crude product was purified by flash silica gel column (normal phase silica gel 53 g, hexane/ethyl acetate = 3/1 - 2/1) to give Fragment A-2' (1.87 g, three-step yield 50%) as a yellow viscous product.

### (Example 2AA: Synthesis of Fragment A-2")

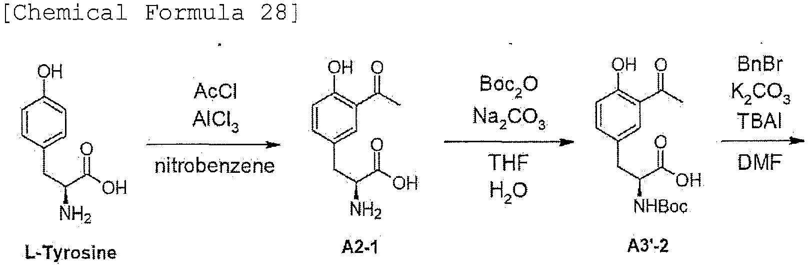

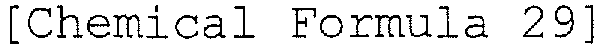

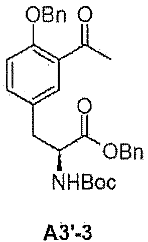

Synthesis of L-Tyrosine → A2-1 is as described in Example 1. Synthesis of A3'-3 from A2-1 via A3'-2 is as described in Example 2.

Synthesis of A2"-1 from A3'-3 is as follows.

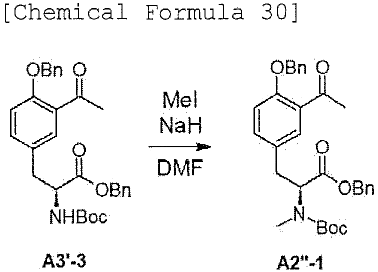

Under nitrogen atmosphere, to a solution of A3'-3 (1.0 eq., 18.3 g, 36.34 mmol) in DMF (150 mL) were added methyl iodide (3.0 eq., 6.8 mL, 109.23 mmol) and sodium hydride (60%, dispersion in Paraffin Liquid, 1.2 eq., 1.7 g, 43.45 mmol) at the external temperature of -20°C, and the mixture was stirred at the same temperature for 3 hr. 1N Aqueous hydrochloric acid solution (50 mL) was added at the external temperature of - 20°C, and then water (100 mL) was added. The mixture was extracted twice with a mixture of hexane (50 mL)/ethyl acetate (100 mL) by liquid separation, and the organic layers were combined, and washed once with saturated aqueous sodium chloride solution (150 mL) by liquid separation. The organic layer was dried over magnesium sulfate, the magnesium sulfate was removed by filtration, and the filtrate was concentrated under reduced pressure to give a crude product (20.2 g) as a yellow oil. The crude product was purified by flash silica gel column (normal phase silica gel 40 g, hexane/ethyl acetate = 9/1 - 3/1), and the obtained pale-yellow viscous product was washed with hexane to give A2"-1 (18.9 g, yield 101%).

Synthesis of A2"-2 from A2"-1 is as follows.

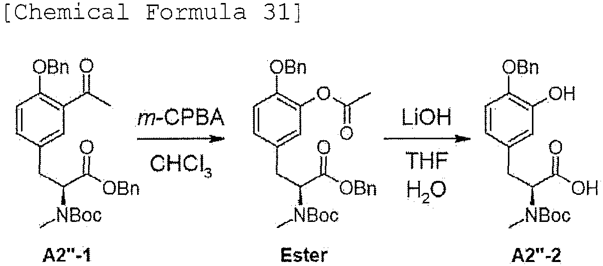

Under nitrogen atmosphere, to a solution of A2"-1 (1.0 eq., 18.9 g, 36.34 mmol) in chloroform (150 mL) was added m-CPBA (30% water content, 2.0 eq., 18.0 g, 73.01 mmol) at room temperature, and the mixture was stirred at the external temperature of 60°C for 4.5 hr. Additional m-CPBA (0.5 eq., 4.8 g, 19.47 mmol) was added at room temperature, and the mixture was stirred at the external temperature of 60°C for 1 hr, and stirred at the external temperature of 40°C for 15 hr. Under ice-cooling, 20% aqueous sodium sulfite solution (75 mL) and saturated aqueous sodium hydrogencarbonate solution (75 mL) were added. The organic layer of the reaction solution was recovered, and to the organic layer were added 20% aqueous sodium sulfite solution (75 mL), saturated aqueous sodium hydrogencarbonate solution (75 mL), saturated aqueous sodium chloride solution (250 mL) and ethyl acetate (400 mL), and the mixture was washed once by liquid separation. The aqueous layers were combined, and extracted once with ethyl acetate (100 mL) by liquid separation. The organic layers were combined, and dried over magnesium sulfate, the magnesium sulfate was removed by filtration, and the filtrate was concentrated under reduced pressure to give a crude product (20.6 g) as a brown viscous product. The crude product was purified by flash silica gel column (normal phase silica gel 40 g, hexane/ethyl acetate = 9/1 - 3/1) to give Ester (17.2 g, yield 89%) as a yellow viscous product.

To a solution of Ester (1.0 eq., 17.2 g, 32.17 mmol) in a mixture of THF (75 mL)/water (75 mL) was added lithium hydroxide (2.5 eq., 1.9 g, 80.25 mmol) at room temperature, and the mixture was stirred at room temperature for 3 hr. Additional lithium hydroxide (1.0 eq., 790 mg, 32.97 mmol) was added, the mixture was stirred for 1 hr, additional lithium hydroxide (0.5 eq., 392 mg, 16.37 mmol) was added again, and the mixture was stirred for 30 min. The reaction solution was washed twice with hexane (75 mL) by liquid separation, and the organic layers were combined, and extracted once with water (20 mL) by liquid separation. The aqueous layers were combined, adjusted to pH 1 by addition of 2N aqueous hydrochloric acid solution (65 mL) under ice-cooling, and extracted three times with ethyl acetate (100 mL) by liquid separation. The organic layers were combined, and dried over magnesium sulfate, the magnesium sulfate was removed by filtration, and the filtrate was concentrated under reduced pressure to give crude A2"-2 (14.3 g, crude yield 110%) as a brownish-red viscous product.

Synthesis of Fragment A-2" from A2"-2 is as follows.

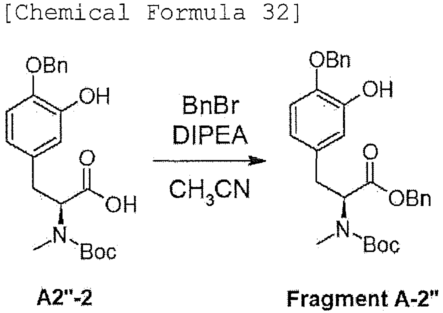

Under nitrogen atmosphere, to a solution of A2"-2 (1.0 eq., 14.2 g, 32.17 mmol) in acetonitrile (150 mL) were added DIPEA (1.2 eq, 6.6 mL, 38.81 mmol) and BnBr (1.2 eq., 3.8 mL, 38.66 mmol) at room temperature, and the mixture was stirred at room temperature for 2 hr. Additional DIPEA (0.2 eq, 1.1 mL, 6.47 mmol) and BnBr (0.2 eq., 650 µL, 6.61 mmol) were added, the mixture was stirred for 1 hr, and saturated aqueous ammonium chloride solution (75 mL) and water (75 mL) were added. The organic layer of the reaction solution was recovered, and the aqueous layer was extracted twice with ethyl acetate (100 mL) by liquid separation. The organic layers were combined, and washed twice with saturated aqueous sodium chloride solution (100 mL) by liquid separation. The organic layer was dried over magnesium sulfate, the magnesium sulfate was removed by filtration, and the filtrate was concentrated under reduced pressure to give a crude product (18.0 g) as a brown oil. The crude product was purified by flash silica gel column (normal phase silica gel 100 g, hexane/ethyl acetate = 5/1 - 0/1) to give Fragment A-2" (7.1 g, two-step yield 45%) as a pale-yellow viscous product.

### (Example 2: Synthesis of Fragment A-3)

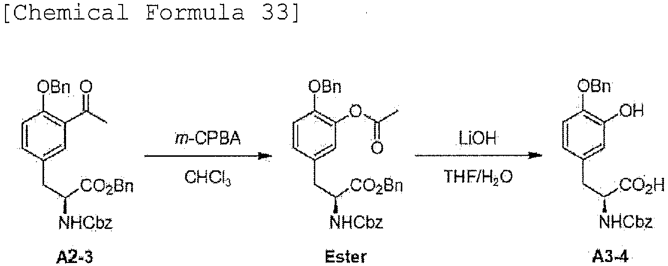

To a solution of A2-3 (1.0 eq., 13.1 g, 24.4 mmol) in chloroform (120 mL) was added m-CPBA (2.0 eq., 35% water content, 13.0 g, 48.9 mmol) under ice-cooling, and the mixture was stirred under reflux conditions for 18 hr. The reaction solution was quenched by addition of saturated aqueous sodium hydrogencarbonate solution (100 mL) at room temperature, and the chloroform was evaporated under reduced pressure. The reaction solution was extracted three times with ethyl acetate (200 mL) by liquid separation, and the organic layers were combined, washed once with saturated aqueous sodium chloride solution (200 mL) by liquid separation, and dried over magnesium sulfate. The magnesium sulfate was removed by filtration, and the filtrate was concentrated under reduced pressure to give Ester.

To a solution of Ester (1.0 eq., calculated as 24.4 mmol) in a mixture of THF (60 mL)/water (60 mL) was added lithium hydroxide (4.0 eq., 2.34 g, 97.7 mmol) at room temperature, and the mixture was stirred at the same temperature for 19 hr. To the reaction solution was added additional lithium hydroxide (2.0 eq., 1.17 g, 48.9 mmol) at room temperature, and the mixture was stirred at the same temperature for 4.5 hr. To the reaction solution was added water (100 mL), and the mixture was washed twice with hexane (100 mL) by liquid separation, and the aqueous layer was adjusted to pH 3 by addition of 6N aqueous hydrochloric acid solution. The aqueous layer was extracted twice with ethyl acetate (200 mL) by liquid separation, and the organic layers were combined, washed once with saturated aqueous sodium chloride solution (200 mL) by liquid separation, and dried over magnesium sulfate. The magnesium sulfate was removed by filtration, and the filtrate was concentrated under reduced pressure to give A3-4 (11.2 g) as a dark-brown oil. 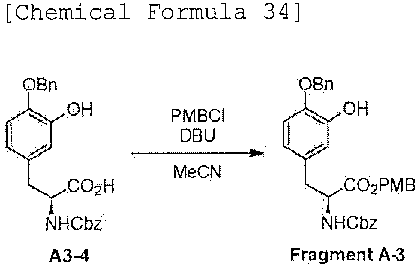 To a solution of A3-4 (1.0 eq., 11.2 g, calculated as 24.4 mmol) in acetonitrile (120 mL) were added DBU (1.1 eq., 4.09 g, 26.8 mmol) and PMBCl (1.1 eq., 4.20 g, 26.8 mmol) at room temperature, and the mixture was stirred at the external temperature of 60°C for 16 hr. The reaction solution was quenched by addition of acetic acid (3.0 eq., 4.41 g, 73.4 mmol), and concentrated under reduced pressure to give a crude product. The crude product was purified by flash silica gel column (normal phase silica gel 180 g, hexane/ethyl acetate = 75/25 - 50/50) to give Fragment A-3 (7.83 g, two-step yield from A2-3 59%) as an orange oil.

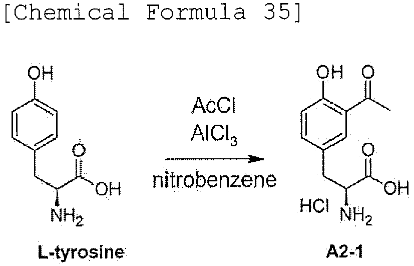

Under nitrogen atmosphere, to a solution of L-tyrosine (1.0 eq., 3.04 g, 16.78 mmol) in nitrobenzene (70 mL) were added aluminium chloride (4.1 eq., 9.08 g, 68.11 mmol) and acetyl chloride (1.3 eq., 1.5 mL, 21.12 mmol) under ice-cooling. The mixture was stirred under ice-cooling for 20 min, and then stirred at the external temperature of 100°C for 7 hr. The reaction solution was allowed to cool, and quenched by addition of ice-cooled 1N aqueous hydrochloric acid solution (100 mL). The mixture was washed three times with ethyl acetate (100 mL) by liquid separation to give A2-1 as an aqueous solution (100 mL).

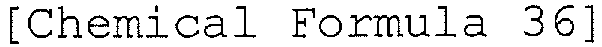

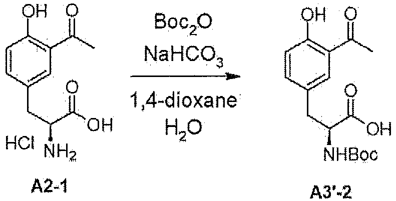

To A2-1 (1.0 eq., aqueous solution 100 mL, calculated as 16.78 mmol) were added water (100 mL) and 1,4-dioxane (100 mL), and the mixture was basified by addition of with sodium hydrogencarbonate (25.0 eq., 35.2 g, 419.05 mmol) under ice-cooling. Then, Boc₂O (1.2 eq., 4.6 mL, 20.02 mmol) was added under ice-cooling, and the mixture was stirred at room temperature for 4.5 hr. Additional sodium hydrogencarbonate (2.1 eq., 3.03 g, 36.07 mmol) and Boc₂O (1.0 eq., 4 mL, 17.41 mmol) were added, and the mixture was stirred for additional 17 hr. The reaction solution was concentrated under reduced pressure to evaporate the 1,4-dioxane, and adjusted to pH 1-2 by addition of 2N aqueous hydrochloric acid solution (about 300 mL). The mixture was extracted once with ethyl acetate (300 mL) by liquid separation, and the organic layer was washed once with 1N aqueous hydrochloric acid solution (100 mL) by liquid separation. The aqueous layers were combined, and extracted once with ethyl acetate (200 mL) by liquid separation. The organic layers were combined, and dried over magnesium sulfate, the magnesium sulfate was removed by filtration, and the filtrate was concentrated under reduced pressure to give A3'-2 (5.63 g, crude yield 104%) as a brown viscous product.

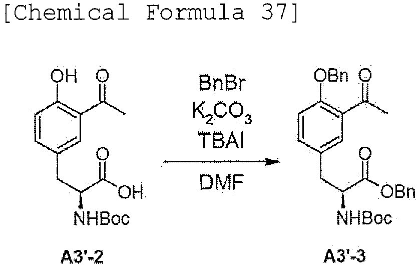

Under nitrogen atmosphere, to a solution of A3'-2 (1.0 eq., 5.63 g, calculated as 16.78 mmol) in DMF (80 mL) were added potassium carbonate (3.0 eq., 7.03 g, 50.85 mmol), TBAI (0.1 eq., 640 mg, 1.73 mmol) and BnBr (2.2 eq., 3.6 mL, 36.62 mmol) under ice-cooling, and the mixture was stirred at room temperature for 2 hr. To the reaction solution was added water (80 mL), and the mixture was extracted three times with a mixture of hexane (25 mL)/ethyl acetate (75 mL) by liquid separation. The organic layers were combined, and washed with saturated aqueous sodium chloride solution (200 mL) by liquid separation. The organic layer was dried over magnesium sulfate, the magnesium sulfate was removed by filtration, and the filtrate was concentrated under reduced pressure to give a crude product (9.84 g) as a brownish-red oil. The crude product was purified by flash silica gel column (normal phase silica gel 70 g, hexane/ethyl acetate = 5/1 - 1/1) to give A3'-3 (6.18 g, three-step yield 73%) as a pale-orange viscous product.

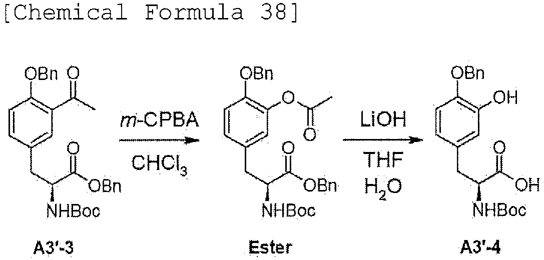

Under nitrogen atmosphere, to a solution of A3'-3 (1.0 eq., 19.32 g, 38.36 mmol) in chloroform (200 mL) was added m-CPBA (35% water content, 2.0 eq., 19.0 g, 77.07 mmol) under ice-cooling, and the mixture was stirred at the external temperature of 60°C for 6 hr. The reaction solution was concentrated under reduced pressure until the volume was reduced to about half, and saturated aqueous sodium hydrogencarbonate solution (200 mL) was added. The mixture was extracted once with ethyl acetate (200 mL) and twice with ethyl acetate (100 mL) by liquid separation, and the organic layers were combined, and washed with saturated aqueous sodium chloride solution (200 mL) by liquid separation. The organic layer was dried over magnesium sulfate, the magnesium sulfate was removed by filtration, and the filtrate was concentrated under reduced pressure to give Ester (24.9 g, crude yield 125%) as a pale-yellow solid.

To a solution of Ester (1.0 eq., 24.9 g, calculated as 38.36 mmol) in a mixture of THF (100 mL)/water (100 mL) was added lithium hydroxide (2.5 eq., 2.31 g, 96.43 mmol) under ice-cooling, and the mixture was stirred at room temperature for 3 hr. Then, additional lithium hydroxide (1.0 eq., 929 mg, 38.80 mmol) was added, and the mixture was stirred for additional 2 hr. Then, additional lithium hydroxide (1.5 eq., 1.39 g, 57.87 mmol) was added, and the mixture was stirred for 1 hr. The reaction solution was washed twice with hexane (100 mL) by liquid separation. The aqueous layer was adjusted to pH 2-3 by addition of 6N aqueous hydrochloric acid solution (30 mL) under ice-cooling, and extracted once with ethyl acetate (100 mL) by liquid separation. The aqueous layer was adjusted to pH 1-2 by addition of 6N aqueous hydrochloric acid solution (5 mL), and extracted twice with ethyl acetate (100 mL) by liquid separation. The organic layers were combined, and dried over magnesium sulfate, the magnesium sulfate was removed by filtration, and the filtrate was concentrated under reduced pressure to give a crude product (21.6 g) as a brown viscous product. The crude product was recrystallized from a mixture of hexane/ethyl acetate (4/1 - 1/1) to give A3'-4 (12.4 g, apparent yield 83%, containing about 30% m-CPBA) as a white solid.

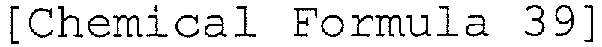

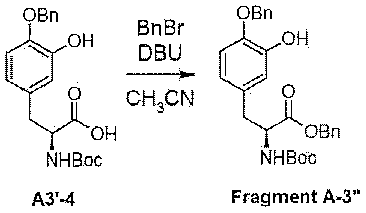

Under nitrogen atmosphere, to a solution of A3"-4 (1.0 eq., 3.03 g, containing about 30% m-CPBA, calculated as 7.83 mmol) in acetonitrile (40 mL) were added DBU (1.1 eq, 1.3 mL, 8.71 mmol) and BnBr (1.1 eq., 850 µL, 8.65 mmol) under ice-cooling, and the mixture was stirred at room temperature for 16 hr. The reaction solution was washed once with saturated aqueous ammonium chloride solution (40 mL) by liquid separation. The aqueous layer was extracted twice with ethyl acetate (40 mL) by liquid separation. The organic layers were combined, and washed once with water (100 mL) by liquid separation, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (3.7 g) as a brown oil. The crude product was purified by flash silica gel column (normal phase silica gel 50 g, hexane/ethyl acetate = 5/1 - 3/1) to give Fragment A-3" (1.8 g, yield 48%) as a pale-yellow viscous product.

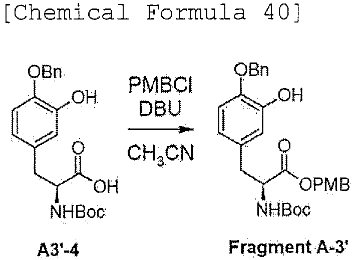

Under nitrogen atmosphere, to a solution of A3'-4 (1.0 eq., 4.42 g, containing about 30% of a hydrolyzate of A3'-3, calculated as 11.43 mmol) in acetonitrile (60 mL) were added DBU (1.1 eq, 1.9 mL, 12.73 mmol) and PMBCl (1.1 eq., 1.7 mL, 12.48 mmol) under ice-cooling, and the mixture was stirred at room temperature for 16 hr. The reaction solution was washed once with saturated aqueous ammonium chloride solution (60 mL) by liquid separation. The aqueous layer was extracted twice with ethyl acetate (60 mL) by liquid separation. The organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (6.17 g) as a brown oil. The crude product was purified by flash silica gel column (normal phase silica gel 70 g, hexane/ethyl acetate = 5/1 - 2/1) to give Fragment A-3' (3.47 g, containing about 30% of PMB ester of A3'-3, apparent yield 60%) as a pale-yellow viscous product.

### (Example 2A: Synthesis of Fragment A-13)

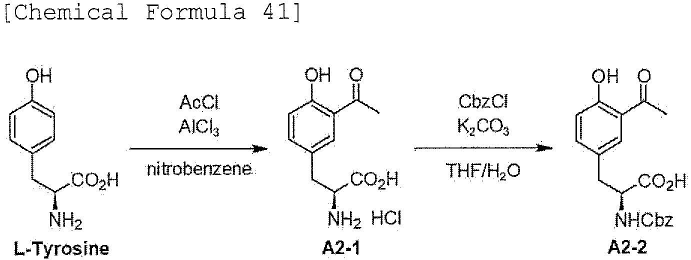

The two steps from L-Tyrosine to A2-2 were carried out as described in Example 2.

Synthesis of A13-1 from A2-2 is as follows.

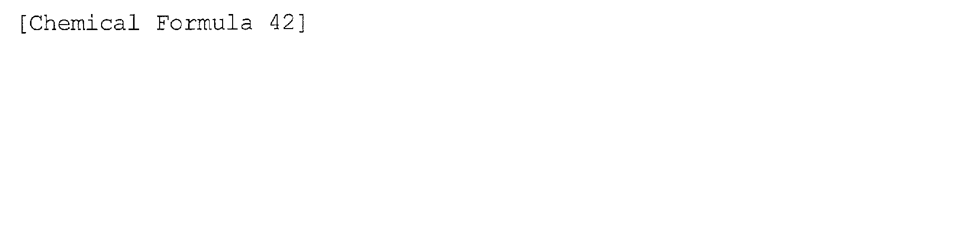

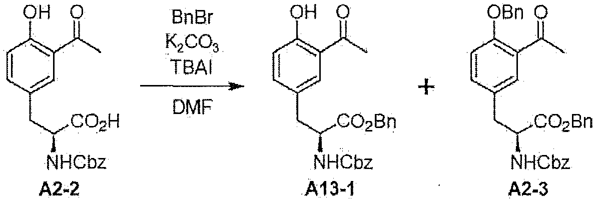

Under nitrogen atmosphere, to a solution of A2-2 (1.0 eq., 20.3 g, calculated as 55.2 mmol) in DMF (110 mL) were added potassium carbonate (3.0 eq., 22.9 g, 166 mmol), TBAI (0.10 eq., 2.04 g, 5.52 mmol) and BnBr (2.0 eq., 13.1 g, 110 mmol) under ice-cooling, and the mixture was stirred at room temperature for 2.5 hr. To the reaction solution was added water (200 mL), and the mixture was extracted twice with a mixture of hexane (50 mL)/ethyl acetate (150 mL) by liquid separation. The organic layers were combined, and washed once with water (100 mL) and once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (34.3 g) as a yellow liquid. The crude product was purified by flash silica gel column (normal phase silica gel 200 g, hexane/ethyl acetate = 80/20 - 50/50) to give a mixture (24.3 g) of A13-1 and A2-3 as a yellow liquid.

Synthesis of A13-2 from A13-1 is as follows.

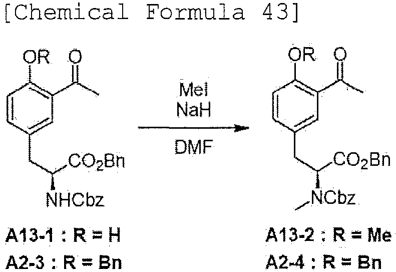

Under nitrogen atmosphere, to a solution of a mixture (1.0 eq., 24.3 g, calculated as 45.2 mmol) of A13-1 and A2-3 in DMF (90 mL) were added sodium hydride (1.2 eq., 2.17 g, 54.3 mmol) and methyl iodide (3.0 eq., 8.4 mL, 135 mmol) under ice-cooling, and the mixture was stirred under ice-cooling for 4 hr. The reaction solution was quenched by addition of 2N aqueous hydrochloric acid solution (20 mL), and water (200 mL) was added. The mixture was extracted once with a mixture of hexane (50 mL)/ethyl acetate (150 mL) and once with a mixture of hexane (40 mL)/ethyl acetate (120 mL) by liquid separation. The organic layers were combined, and washed once with water (100 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give a mixture (24.6 g) of A13-2 and A2-4.

Synthesis of A13-3 from A13-2 is as follows.

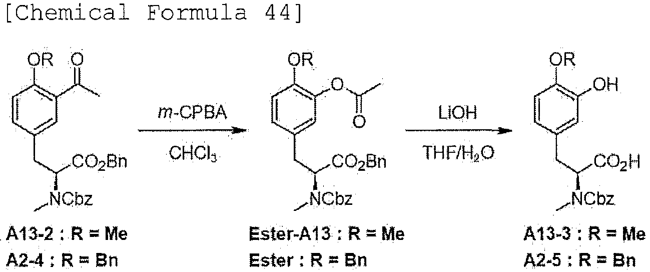

Under nitrogen atmosphere, to a solution of a mixture (1.0 eq., 24.6 g, calculated as 44.6 mmol) of A13-2 and A2-4 in chloroform (180 mL) was added m-CPBA (35% water content, 2.0 eq., 23.7 g, 89.3 mmol) at room temperature, and the mixture was stirred at the external temperature of 45°C for 17 hr. The reaction solution was ice-cooled, and quenched by addition of a mixture of 20% aqueous sodium sulfite solution (80 mL)/saturated aqueous sodium hydrogencarbonate solution (80 mL). The reaction solution was extracted once with ethyl acetate (500 mL) by liquid separation, and the organic layer was washed once with a mixture of 20% aqueous sodium sulfite solution (80 mL)/saturated aqueous sodium hydrogencarbonate solution (80 mL) and once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (26.2 g) as a brown oil. The crude product was purified by flash silica gel column (normal phase silica gel 220 g, hexane/ethyl acetate = 83/17 - 50/50) to give a mixture (22.2 g) of Ester-A13 and Ester as a yellow liquid.

To a solution of a mixture (1.0 eq., 22.2 g, calculated as 39.1 mmol) of Ester-A13 and Ester in a mixture of THF (80 mL)/water (80 mL) was added lithium hydroxide (4.0 eq., 3.74 g, 156 mmol) at room temperature, and the mixture was stirred at the same temperature for 3.5 hr. The reaction solution was washed three times with hexane (100 mL) by liquid separation, and the aqueous layer was adjusted to pH 1 by addition of 2N aqueous hydrochloric acid solution (72 mL). The aqueous layer was extracted three times with ethyl acetate (150 mL) by liquid separation, and the organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a mixture (17.9 g) of A13-3 and A2-5 as a brown viscous product.

Synthesis of Fragment A-13 from A13-3 is as follows.

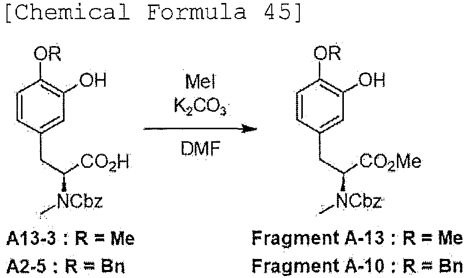

To a solution of a mixture (1.0 eq., 17.4 g, calculated as 39.1 mmol) of A13-3 and A2-5 in DMF (80 mL) were added potassium carbonate (3.1 eq., 16.6 g, 120 mmol) and methyl iodide (1.1 eq., 2.7 mL, 43.4 mmol) at room temperature, and the mixture was stirred at the same temperature for 1.5 hr. To the reaction solution was added water (200 mL), and the mixture was extracted once with a mixture of hexane (60 mL)/ethyl acetate (180 mL) and twice with a mixture of hexane (30 mL)/ethyl acetate (90 mL) by liquid separation, and the organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (17.9 g) as a brown viscous product. The crude product was purified twice by flash silica gel column (1st run: normal phase silica gel 200 g, hexane/ethyl acetate = 80/20 - 50/50, 2nd run: normal phase silica gel 25 g, hexane/ethyl acetate = 75/25 - 50/50) to give Fragment A-13 (2.42 g) as a brown viscous product and Fragment A-10 (11.8 g) as a pale-yellow viscous product.

### (Example 3: Synthesis of Fragment B-1)

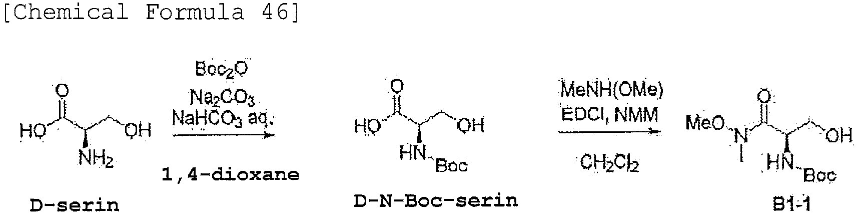

Under nitrogen atmosphere, to a solution of D-serin (1.0 eq., 25.0 g, 0.238 mol) in saturated aqueous sodium hydrogencarbonate solution (150 mL) were added water (150 mL) and sodium carbonate (1.0 eq., 25.2 g, 0.238 mol) at room temperature. A solution of Boc₂O (1.2 eq., 62.4 g, 0.286 mol) in 1,4-dioxane (125 mL) was added under ice-cooling, and the mixture was stirred at the same temperature for 0.5 hr, and then overnight at room temperature. The mixture was concentrated under reduced pressure to evaporate the 1,4-dioxane, and to the concentrated residue was added MTBE (200 mL), and the insoluble substance was removed by filtration. The aqueous layer of the filtrate was recovered, and the aqueous layer was washed once with MTBE (200 mL) by liquid separation, and adjusted to pH 2-3 by addition of conc. hydrochloric acid (about 25 mL) under ice-cooling. Sodium chloride (60 g) was added, and the mixture was extracted four times with ethyl acetate (200 mL) by liquid separation. To the aqueous layer were added conc. hydrochloric acid (5 mL) and sodium chloride (20 g), and the mixture was extracted four times with ethyl acetate (200 mL) by liquid separation. To the aqueous layer were added again sodium chloride and conc. hydrochloric acid, and the mixture was extracted six times with ethyl acetate (100 mL) and six times with ethyl acetate (50 mL)/THF (50 mL) by liquid separation. The organic layers were combined, and dried over sodium sulfate, the sodium sulfate was removed by filtration, and the filtrate was concentrated under reduced pressure to give D-N-Boc-serin (50.81 g, yield 92%) as a colorless oil.

Under nitrogen atmosphere, to a solution of D-N-Boc-serin (1.0 eq., 50.8 g, Net 44.9 g, 0.219 mol) in dichloromethane (300 mL) were added N,O-dimethylhydroxylamine hydrochloride (1.03 eq., 22.27 g, 0.228 mol), NMM (1.03 eq., 23.10 g, 0.228 mol) and EDCI (1.1 eq., 46.74 g, 0.244 mol) at the internal temperature of -10°C. This solution was stirred overnight at - 10°C to room temperature. The reaction solution was washed once with 1M aqueous hydrochloric acid solution by liquid separation under ice-cooling, and the aqueous layer was extracted once with dichloromethane (100 mL) by liquid separation, and the organic layers were combined, and washed once with saturated aqueous sodium hydrogencarbonate solution (100 mL) by liquid separation. The aqueous layer was again extracted once with dichloromethane (50 mL) by liquid separation, and the organic layers were combined, and dried over sodium sulfate. The sodium sulfate was removed by filtration, and the filtrate was concentrated under reduced pressure to give B1-1 (50.4 g, yield 93%) as a white solid.

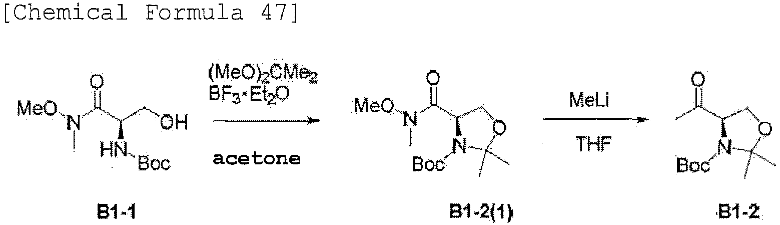

Under nitrogen atmosphere, to a solution of B1-1 (1.0 eq., 50.4 g, 0.203 mol) in acetone (327 mL) were added 2,2-dimethoxypropane (164 mL) and boron trifluoride diethyl ether complex (0.06 eq., 1.64 mL, 0.013 mol) at room temperature, and the mixture was stirred at the same temperature for 1 hr. To the reaction solution was added triethylamine (0.146 eq., 3.00 g, 29.65 mmol), and the mixture was concentrated under reduced pressure. To the concentrated residue was added THF (100 mL) and the mixture was concentrated again. These procedures were repeated three times to give B1-2(1) (60.4 g, quant.) as a pale-yellow oil.

Under nitrogen atmosphere, to a solution of B1-2(1) (1.0 eq., 60.4 g, calculated as 0.203 mol) in THF (570 mL) was added methyllithium (1.5M in Et₂O, 2.0 eq., 279 mL, 0.419 mmol) over 40 min at the internal temperature of -57 - -40°C, and the mixture was stirred at the same temperature for 2 hr. To the reaction solution was added saturated aqueous ammonium chloride solution (130 mL) at the same temperature, and the mixture was warmed to room temperature, and washed by liquid separation. The aqueous layer was extracted three times with ethyl acetate (200 mL) by liquid separation, and the organic layers were combined, and dried over sodium sulfate. The sodium sulfate was removed by filtration, and the filtrate was concentrated under reduced pressure to give a crude product (51.56 g) as a pale-yellow oil. The crude product was purified by flash silica gel column (normal phase silica gel 750 g, hexane/ethyl acetate = 10/1 - 4/1) to give B1-2 (34.8 g, yield 70%) as a colorless oil.

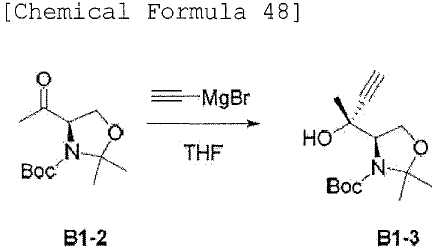

Under nitrogen atmosphere, to a solution of B1-2 (1.0 eq., 34.8 g, 0.143 mol) in THF (696 mL) was added ethynylmagnesium bromide (0.5M in THF, 2.5 eq., 715 mL, 0.358 mol) over 1.5 hr at room temperature, and the mixture was stirred at the same temperature for 2 hr. To the reaction solution was added saturated aqueous ammonium chloride solution (160 mL) at -55°C, and the mixture was warmed to -5°C, and concentrated under reduced pressure. To the concentrated residue was added water (300 mL), and the mixture was extracted once with ethyl acetate (300 mL) and once with ethyl acetate (150 mL) by liquid separation, and the organic layers were combined, washed once with saturated aqueous sodium chloride solution (150 mL) by liquid separation, and dried over sodium sulfate. The sodium sulfate was removed by filtration, and the filtrate was concentrated under reduced pressure to give a crude product (40.36 g) as a yellow solid. The crude product was purified by flash silica gel column (normal phase silica gel 370 g, chloroform/ethyl acetate = 100/0 - 20/1) to give B1-3 (diastereomer mixture, 33.9 g, yield 88%) as a white solid.

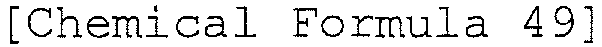

Under nitrogen atmosphere, to a solution of B1-3 (diastereomer mixture, 1.0 eq., 33.9 g, 0.126 mol) in THF (450 mL) was added conc. hydrochloric acid (12 eq., 130 mL, 1.56 mol) at room temperature, and the mixture was stirred at the same temperature for 4 hr. The reaction solution was concentrated under reduced pressure to give a crude product solution of B1-4(1).

Under nitrogen atmosphere, to the crude product solution of B1-4(1) were added THF (260 mL) and water (65 mL). To the solution were added sodium carbonate (5.0 eq., 66.70 g, 0.629 mol) and NsCl (1.0 eq., 27.90 g, 0.125 mol), and the mixture was stirred at room temperature for 17 hr. The reaction solution was washed with saturated aqueous sodium chloride solution (65 mL) by liquid separation, and the aqueous layer was extracted three times with ethyl acetate (260 mL) by liquid separation, and the organic layers were combined, and dried over sodium sulfate. The sodium sulfate was removed by filtration, and the filtrate was concentrated under reduced pressure to give a crude product (40.01 g) as a brown viscous product. The crude product was purified by flash silica gel column (normal phase silica gel 200 g, hexane/ethyl acetate = 1/1 - 3/7) to give B1-4 (diastereomer mixture, 38.05 g, containing 4.0wt% ethyl acetate, conversion yield 92%) as a pale-brown viscous product.

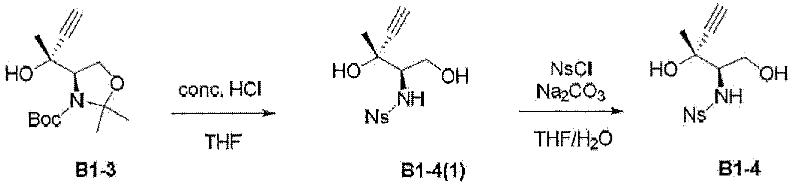

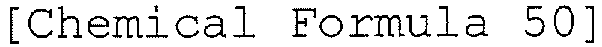

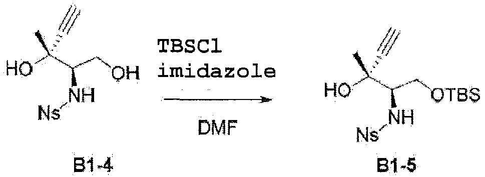

Under nitrogen atmosphere, to a solution of B1-4 (diastereomer mixture, 1.0 eq., 36.0 g, containing 4.0wt% ethyl acetate, 0.110 mol) in DMF (360 mL) were added imidazole (1.5 eq., 11.71 g, 0.172 mol) and TBSCl (1.2 eq., 20.74 g, 0.138 mol) at room temperature, and the mixture was stirred at the same temperature for 1 hr. To the reaction solution were added ethyl acetate (350 mL) and water (300 mL), and the mixture was extracted once by liquid separation, and the aqueous layer was extracted twice with ethyl acetate (100 mL) by liquid separation, and the organic layers were combined, and dried over sodium sulfate. The sodium sulfate was removed by filtration, and the filtrate was concentrated under reduced pressure to give a crude product as a brown viscous product. The crude product was purified by flash silica gel column (normal phase silica gel 540 g, hexane/ethyl acetate = 1/0 - 1/1) to give B1-5 (diastereomer mixture, 48.3 g, yield 98%) as a yellow viscous product.

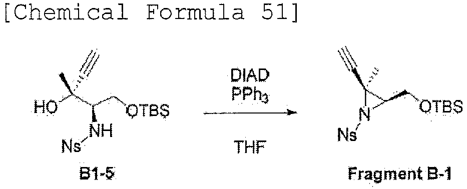

Under nitrogen atmosphere, to a solution of B1-5 (diastereomer mixture, 1.0 eq., 24.1 g, 0.056 mol) in THF (560 mL) were added triphenylphosphine (1.5 eq., 17.10 g, 0.085 mol) and DIAD (1.5 eq., 17.09 g, 0.084 mol) under ice-cooling, and the mixture was stirred at the same temperature for 2 hr. To the reaction solution were added ethyl acetate (650 mL), water (300 mL) and 5% aqueous sodium hydrogencarbonate solution (300 mL), and the mixture was extracted once by liquid separation, and the organic layer was washed once with 5% aqueous sodium hydrogencarbonate solution (300 mL) and once with saturated aqueous sodium chloride solution (300 mL) by liquid separation, and dried over sodium sulfate. The sodium sulfate was removed by filtration, and the filtrate was concentrated under reduced pressure to give a crude product as a yellow oil. The crude product was purified by flash silica gel column (normal phase silica gel 720 g, hexane/ethyl acetate = 10/1) to give Fragment B-1 (12.6 g, yield 54%) as a white solid, and also a mixture (4.09 g) containing 16% diastereomer.

To the diastereomer mixture (3.41 g) was added a mixture of hexane (8.5 mL)/diisopropyl ether (8.5 mL), and the mixture was dissolved by heating to the external temperature of 45°C. This solution was allowed to cool to the internal temperature of 11°C, and the precipitated solid was collected by filtration, and the solid was washed once with hexane (1.5 mL)/diisopropyl ether (1.5 mL) and once with hexane (3 mL) to give Fragment B-1 (2.41 g, containing 0.7 % diastereomer, yield 10%) as a white solid.

### (Example 4: Synthesis of Fragment B-2)

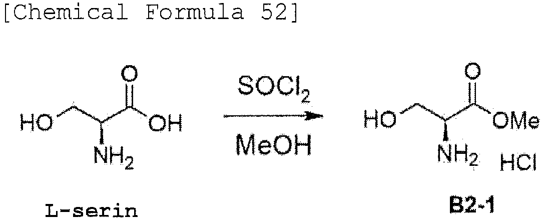

Under nitrogen atmosphere, to methanol (47 mL) was added dropwise thionyl chloride (1.5 eq., 5 mL, 69.35 mmol) under ice-cooling. L-serin (1.0 eq., 5.00 g, 47.66 mmol) was added at the same temperature, and the mixture was stirred under reflux conditions for 2.5 hr. The reaction solution was concentrated under reduced pressure, and the methanol was evaporated to give B2-1 (7.61 g, crude yield 104%) as a pale-yellow solid.

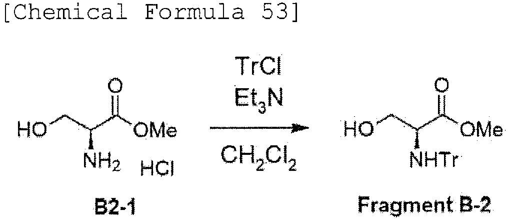

Under nitrogen atmosphere, to a solution of B2-1 (1.0 eq., 4.56 g, calculated as 29.69 mmol) in dichloromethane (30 mL) were added triethylamine (2.1 eq., 8.3 mL, 62.01 mmol) and TrCl (1.05 eq., 8.71 g, 31.23 mmol) under ice-cooling, and the mixture was stirred at room temperature for 3 hr. To the reaction solution was added dichloromethane (30 mL), and the mixture was washed once with water (60 mL) by liquid separation. The aqueous layer was extracted twice with dichloromethane (60 mL) by liquid separation. The organic layers were combined, and washed once with saturated aqueous sodium chloride solution (150 mL) by liquid separation, and the organic layer was dried over magnesium sulfate. The magnesium sulfate was removed by filtration, and the filtrate was concentrated under reduced pressure to give a crude product (10.99 g). To the crude product were added ethyl acetate (20 mL) and hexane (40 mL) under ice-cooling. The insoluble white solid was collected by filtration to give Fragment B-2 (7.67 g, two-step yield 71%).

### (Example 5: Synthesis of Fragment B-3)

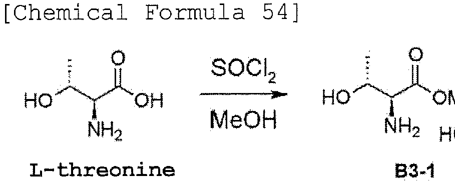

Under nitrogen atmosphere, to methanol (42 mL) was added dropwise thionyl chloride (1.5 eq., 4.5 mL, 62.03 mmol) under ice-cooling. L-threonine (1.0 eq., 5.01 g, 42.10 mmol) was added at the same temperature, and the mixture was stirred under reflux conditions for 5 hr. The reaction solution was concentrated under reduced pressure, and the methanol was evaporated to give B3-1 (8.29 g, crude yield 116%) as a pale-yellow solid.

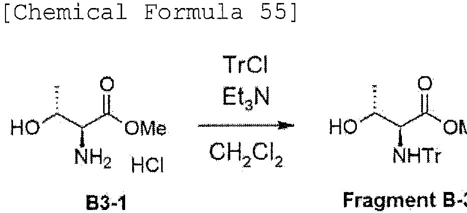

Under nitrogen atmosphere, to a solution of B3-1 (1.0 eq., 4.01 g, calculated as 23.66 mmol) in dichloromethane (48 mL) were added triethylamine (2.1 eq., 6.9 mL, 49.50 mmol) and TrCl (1.06 eq., 6.99 g, 25.08 mmol) under ice-cooling, and the mixture was stirred at room temperature for 5 hr. Additional triethylamine (1.1 eq., 3.6 mL, 25.83 mmol) and TrCl (0.53 eq., 3.50 g, 12.55 mmol) were added under ice-cooling, and the mixture was stirred at room temperature for additional 18 hr. The reaction solution was washed once with water (60 mL) by liquid separation. The aqueous layer was extracted twice with dichloromethane (60 mL) by liquid separation. The organic layers were combined, and washed with saturated aqueous sodium chloride solution (150 mL) by liquid separation, and the organic layer was dried over magnesium sulfate. The magnesium sulfate was removed by filtration, and the filtrate was concentrated under reduced pressure to give a crude product (13.4 g). The crude product was purified by flash silica gel column (normal phase silica gel 70 g, hexane/ethyl acetate = 2/1 - 1/1) to give Fragment B-3 (7.27 g, two-step yield 82%) as a white solid.

### (Example 6: Synthesis of Fragment B-9)

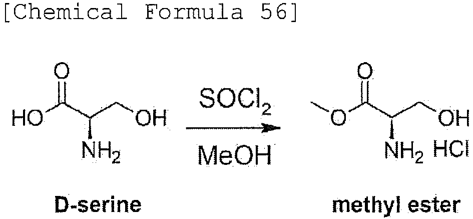

Under nitrogen atmosphere, to methanol (240 mL) was added thionyl chloride (1.45 eq., 25 mL, 345 mmol) under ice-cooling. D-serin (1.0 eq., 25 g, 238 mmol) was added at the same temperature, and the mixture was stirred at 70°C for 16.5 hr. The reaction solution was concentrated under reduced pressure to give a crude methyl ester (37 g).

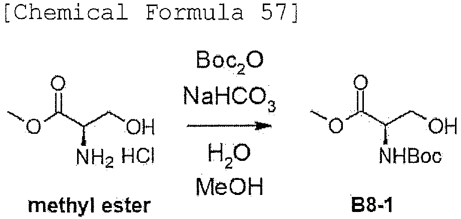

Under nitrogen atmosphere, to a solution of the methyl ester (1.0 eq., 37.0 g, calculated as 238 mmol) in a mixture of water (100 mL)/methanol (100 mL) were added sodium hydrogencarbonate (2.5 eq., 49.9 g, 595 mmol) and Boc₂O (1.05 eq., 65 mL, 250 mmol) under ice-cooling, and the mixture was stirred at room temperature for 5.5 hr. To the reaction solution was added water (200 mL), and the mixture was extracted twice with ethyl acetate (200 mL) by liquid separation. The organic layer was washed twice with water (100 mL) and once with saturated aqueous sodium chloride solution (100 mL) by liquid separation, and dried over magnesium sulfate, and concentrated under reduced pressure to give crude B8-1 (58.7 g).

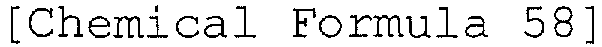

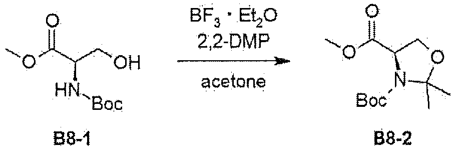

Under nitrogen atmosphere, to a solution of B8-1 (1.0 eq., 58.7 g, calculated as 238 mmol) in acetone (350 mL) were added 2,2-dimethoxypropane (268 mL) and boron trifluoride diethyl ether complex (0.05 eq., 1.5 mL, 11.9 mmol) at room temperature, and the mixture was stirred at the same temperature for 1 hr. To the reaction solution was added triethylamine (0.075 eq., 2.5 mL, 17.9 mmol), and the mixture was concentrated under reduced pressure to give crude B8-2 (68.0 g) as a pale-yellow oil.

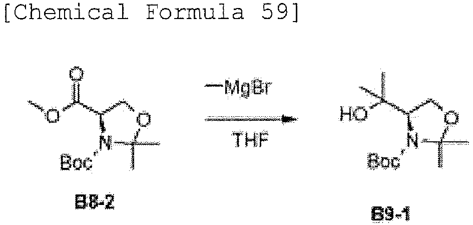

Under nitrogen atmosphere, to a solution of B8-2 (1.0 eq., 16.02 g, calculated as 61.0 mmol) in THF (300 mL) was added methylmagnesium bromide (12.4%, 3.0 eq., 176.06 g, 183 mmol) over 14 min under ice-cooling, and the mixture was stirred at the same temperature for 1 hr. To the reaction solution were added 10% aqueous ammonium chloride solution (200 mL), ethyl acetate (100 mL) and water (30 mL), and the mixture was extracted once by liquid separation, and the aqueous layer was extracted twice with ethyl acetate (50 mL) by liquid separation. The organic layers were combined, washed once with 5% aqueous sodium chloride solution (200 mL) by liquid separation, and dried over sodium sulfate, the sodium sulfate was removed by filtration, and the filtrate was concentrated under reduced pressure.

The crude product obtained from B8-2 (2.00 g) by the same procedure and the previous concentrated residue were combined, and purified by flash silica gel column (normal phase silica gel 100 g, hexane/ ethyl acetate = 80/20 - 20/80) to give B9-1 (17.22 g, containing 1.7wt% ethyl acetate, conversion yield 95%) as a pale-yellow oil.

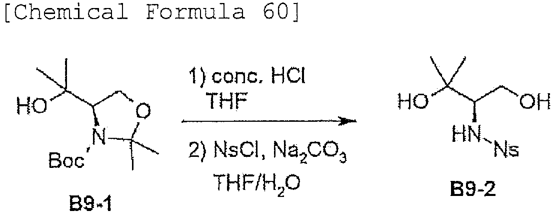

Under nitrogen atmosphere, to a solution of B9-1 (1.0 eq., 16.22 g, containing 1.7wt% ethyl acetate, 61.5 mmol) in THF (248 mL) was added conc. hydrochloric acid (12 eq., 62 mL, 744 mmol) at room temperature, and the mixture was stirred at the same temperature for 2 hr, and then at the external temperature of 50°C for 3 hr, and the reaction solution was concentrated to give a crude product (14.04 g) as a purple oil.

The crude product obtained from B9-1 (1.00 g) by the same procedure and the previous crude product were combined, and dissolved in THF (130 mL), and the solution was adjusted to pH 9 by addition of water (33 mL) and sodium carbonate (5.0 eq., 34.59 g, 326 mmol) at room temperature. NsCl (1.0 eq., 14.46 g, 65.3 mmol) was added, and the mixture was stirred at the same temperature for 16 hr, and additional NsCl (0.3 eq., 4.34 g, 19.6 mmol) was added, and the mixture was stirred at the same temperature for 30 min. To the reaction solution were added saturated aqueous sodium chloride solution (150 mL), ethyl acetate (50 mL) and water (180 mL), and the mixture was extracted once by liquid separation, and the aqueous layer was extracted three times with ethyl acetate (30 mL) by liquid separation. The organic layers were combined, and dried over sodium sulfate, the sodium sulfate was removed by filtration, and the filtrate was concentrated under reduced pressure. The concentrated residue was purified by flash silica gel column (normal phase silica gel 100 g, hexane/ethyl acetate = 50/50 - 0/100) to give B9-2 (19.70 g, containing impurities, apparent yield 99%) as a brown oil.

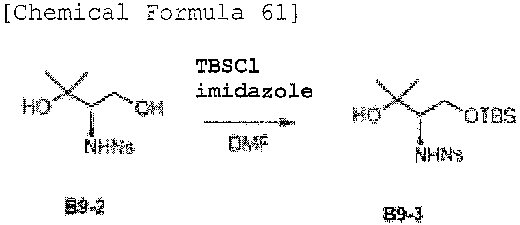

Under nitrogen atmosphere, to a solution of B9-2 (1.0 eq., 19.59 g, 64.4 mmol) in DMF (65 mL) were added TBSCl (1.2 eq., 11.64 g, 77.3 mmol) and imidazole (1.5 eq., 6.57 g, 96.6 mmol) at room temperature, and the mixture was stirred at the same temperature for 30 min.

The reaction solution obtained by treating B9-2 (100 mg) in the same manner and the previous reaction solution were combined, ethyl acetate (130 mL) and water (200 mL) were added, and the mixture was extracted once by liquid separation. The aqueous layer was extracted twice with ethyl acetate (30 mL) by liquid separation, and the organic layers were combined, and 5% aqueous sodium chloride solution (150 mL) and saturated aqueous sodium chloride solution (30 mL) were added, and the mixture was washed once by liquid separation, and dried over sodium sulfate. The sodium sulfate was removed by filtration, and the filtrate was concentrated under reduced pressure, and the concentrated residue was purified by flash silica gel column (normal phase silica gel 100 g, hexane/ethyl acetate = 90/10 - 64/36) to give B9-3 (22.09 g, containing 3.1wt% ethyl acetate, three-step conversion yield from B9-1 79%) as a white viscous product.

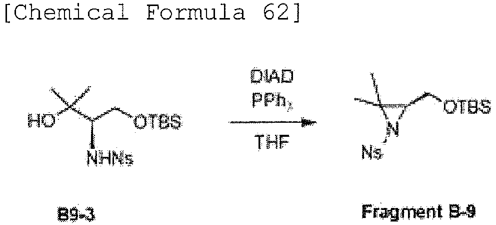

Under nitrogen atmosphere, to a solution of B9-3 (1.0 eq., 21.95 g, containing 3.1wt% ethyl acetate, 50.8 mmol) and triphenylphosphine (1.5 eq., 19.99 g, 76.2 mmol) in THF (508 mL) was added DIAD (90%, 1.5 eq., 17.12 g, 76.2 mmol) at room temperature, and the mixture was stirred at the same temperature for 3 hr. The reaction solution obtained by treating B9-3 (130 mg) in the same manner and the previous reaction solution were combined, and concentrated under reduced pressure, and the concentrated residue was purified twice by flash silica gel column (1st run: normal phase silica gel 480 g, hexane/ethyl acetate = 10/0 - 5/1, 2nd run: normal phase silica gel 100 g, hexane/ethyl acetate = 91/9 - 85/15). To the obtained pale-red solid was added hexane, and the mixture was subjected to ultrasonic irradiation, ice-cooled, and collected by filtration to give Fragment B-9 (15.07 g, yield 74%) as a white solid.

### (Example 6A: Synthesis of Fragment D-8)

Fragment D-8 was synthesized as follows.

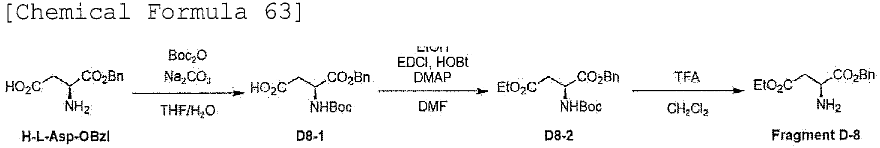

Synthesis of D8-1 from H-L-Asp-OBzl was carried out as follows.

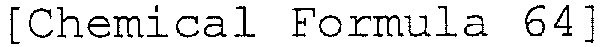

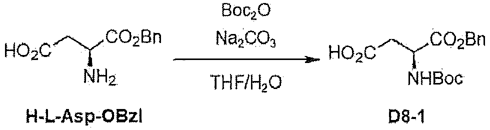

To a solution of H-L-Asp-OBzl (1.0 eq., 501 mg, 2.24 mmol) in a mixture of THF (3 mL)/water (6 mL) were added sodium carbonate (2.0 eq., 477 mg, 4.50 mmol) and Boc₂O (1.9 eq., 1.0 mL, 4.35 mmol) at room temperature, and the mixture was stirred at the same temperature for 5 hr. To the reaction solution was added ethyl acetate (50 mL), and the mixture was extracted twice with water (25 mL) by liquid separation. The aqueous layers were combined, adjusted to pH 7 by addition of 1N aqueous hydrochloric acid solution (0.5 mL), and concentrated under reduced pressure. The solid precipitated by concentration was collected by filtration, and the residue was washed with DMF. The filtrate and washing were combined, and concentrated under reduced pressure to give D8-1 (753.3 mg, quant.) as a cloudy oil.

Synthesis of D8-2 from D8-1 was carried out as follows.

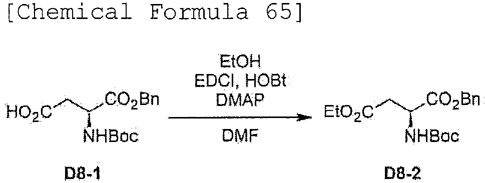

To a solution of D8-1 (1.0 eq., 753 mg, calculated as 2.24 mmol) in DMF (6 mL) were added HOBt•H₂O (1.2 eq., 413 mg, 2.69 mmol), EDCI (1.2 eq., 516 mg, 2.69 mmol), ethanol (2.0 eq., 0.26 mL, 4.45 mmol) and DMAP (1.0 eq., 274 mg, 2.24 mmol), and the mixture was stirred at the same temperature for 18 hr. To the reaction solution were added ethyl acetate (30 mL) and hexane (10 mL), and the mixture was washed twice with water (20 mL), twice with a mixture of water (10 mL)/saturated aqueous sodium hydrogencarbonate solution (10 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give D8-2 (511 mg, two-step yield 65%) as a pale-brown liquid.

Synthesis of Fragment D-8 from D8-2 was carried out as follows.

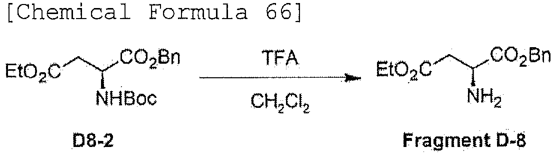

To a solution of D8-2 (1.0 eq., 472 mg, 1.34 mmol) in dichloromethane (2 mL) was added TFA (9.7 eq., 1.0 mL, 13.1 mmol) at room temperature, and the mixture was stirred at the same temperature for 2 hr. To the reaction solution was added ethyl acetate (20 mL), and the mixture was washed twice with water (10 mL), three time with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Fragment D-8 (229 mg, yield 68%) as a colorless, transparent liquid.

### (Example 6B: Synthesis of Fragment D-9)

Fragment D-9 was synthesized as follows.

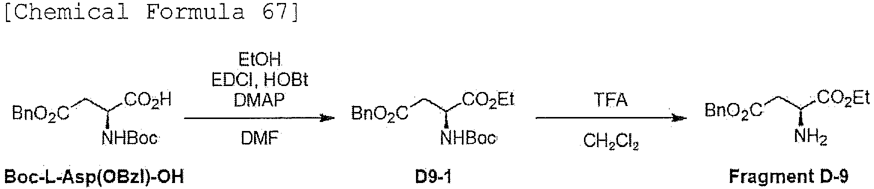

Synthesis of D9-1 from Boc-L-Asp(OBzl)-OH was carried out as follows.

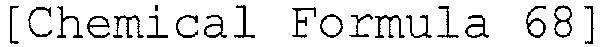

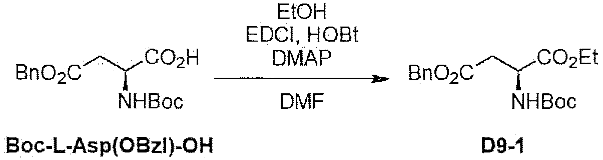

To a solution of Boc-L-Asp(OBzl)-OH (1.0 eq., 893 mg, 2.76 mmol) in DMF (8 mL) were added HOBt•H₂O (1.2 eq., 510 mg, 3.33 mmol), EDCI (1.2 eq., 639 mg, 3.34 mmol), ethanol (1.2 eq., 150 mg, 3.26 mmol) and DMAP (1.0 eq., 339 mg, 2.77 mmol) at room temperature, and the mixture was stirred at the same temperature for 18 hr. To the reaction solution were added ethyl acetate (30 mL) and hexane (10 mL), and the mixture was washed twice with water (20 mL), twice with a mixture of water (10 mL)/saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give D9-1 (881 mg, yield 91%) as a pale-yellow liquid.

Synthesis of Fragment D-9 from D9-1 was carried out as follows.

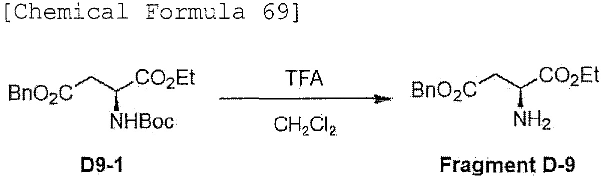

To a solution of D9-1 (1.0 eq., 864 mg, 2.46 mmol) in dichloromethane (3 mL) was added TFA (10 eq., 2.83 g, 24.8 mmol) at room temperature, and the mixture was stirred at the same temperature for 1.5 hr. The reaction solution was adjusted to pH 9 by addition of 1M aqueous sodium hydroxide solution (22 mL). The mixture was extracted once with ethyl acetate (30 mL) by liquid separation, and the organic layer was washed once with saturated aqueous sodium chloride solution (10 mL) by liquid separation. The organic layer was dried over magnesium sulfate, the magnesium sulfate was removed by filtration, and the filtrate was concentrated under reduced pressure to give Fragment D-9 (524 mg, yield 85%) as a pale-yellow liquid.

### (Example 6C: Synthesis of Fragment D-10)

Fragment D-10 was synthesized as follows.

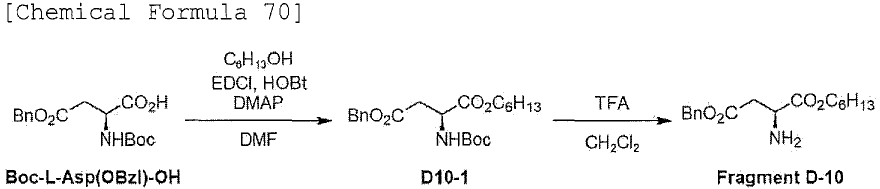

Synthesis of D10-1 from Boc-L-Asp(OBzl)-OH was carried out as follows.

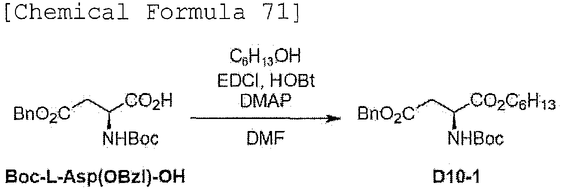

To a solution of Boc-L-Asp(OBzl)-OH (1.0 eq., 5.01 g, 15.5 mmol) in DMF (45 mL) were added HOBt•H₂O (1.2 eq., 2.85 g, 18.6 mmol), EDCI (1.2 eq., 3.57 g, 18.6 mmol), hexanol (1.2 eq., 1.89 g, 18.5 mmol) and DMAP (1.0 eq., 1.90 g, 15.6 mmol) at room temperature, and the mixture was stirred at the same temperature for 1 hr. To the reaction solution were added ethyl acetate (90 mL) and hexane (30 mL), and the mixture was washed twice with water (100 mL), twice with a mixture of water (50 mL)/saturated aqueous sodium hydrogencarbonate solution (50 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give D10-1 (6.08 mg, yield 96%) as a pale-yellow liquid.

Synthesis of Fragment D-10 from D10-1 was carried out as follows.

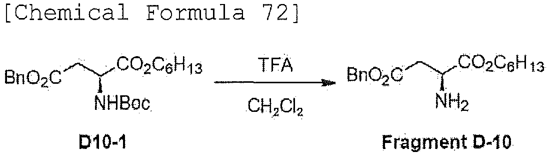

To a solution of D10-1 (1.0 eq., 6.07 g, 14.9 mmol) in dichloromethane (30 mL) was added TFA (10 eq., 17.0 g, 149 mmol) at room temperature, and the mixture was stirred at the same temperature for 1 hr. The reaction solution was adjusted to pH 9 by addition of 1M aqueous sodium hydroxide solution (140 mL). The mixture was extracted once with ethyl acetate (100 mL) by liquid separation, and the organic layer was washed once with water (50 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give Fragment D-10 (4.48 g, yield 98%) as a pale-yellow liquid.

### (Example 6D: Synthesis of Fragment D-11)

Fragment D-11 was synthesized as follows.

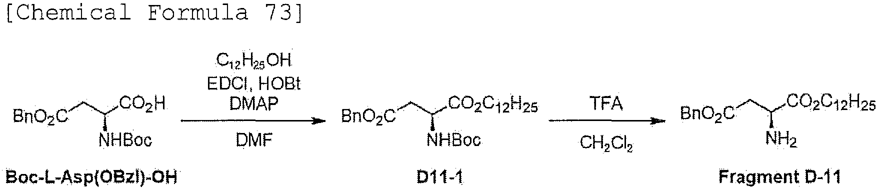

Synthesis of D11-1 from Boc-L-Asp(OBzl)-OH was carried out as follows.

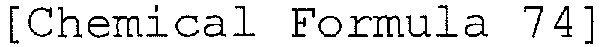

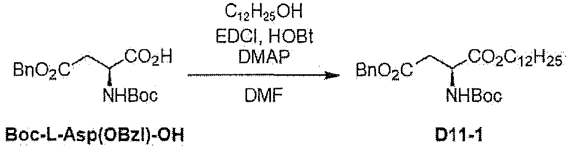

To a solution of Boc-L-Asp(OBzl)-OH (1.0 eq., 5.04 g, 15.6 mmol) in DMF (45 mL) were added HOBt·H₂O (1.2 eq., 2.87 g, 18.7 mmol), EDCI (1.2 eq., 3.59 g, 18.7 mmol), dodecanol (1.2 eq., 3.49 g, 18.7 mmol) and DMAP (1.0 eq., 1.91 g, 15.6 mmol) at room temperature, and the mixture was stirred at the same temperature for 2 hr. To the reaction solution were added ethyl acetate (90 mL) and hexane (30 mL), and the mixture was washed twice with water (100 mL), twice with a mixture of water (50 mL)/saturated aqueous sodium hydrogencarbonate solution (50 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give D11-1 (7.87 mg, crude yield 103%) as a pale-yellow liquid.

Synthesis of Fragment D-11 from D11-1 was carried out as follows.

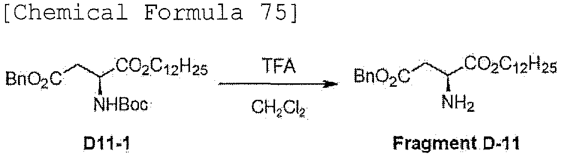

To a solution of D11-1 (1.0 eq., 7.87 g, calculated as 15.6 mmol) in dichloromethane (30 mL) was added TFA (8.0 eq., 14.2 g, 124 mmol) at room temperature, and the mixture was stirred at the same temperature for 1 hr. The reaction solution was adjusted to pH 9 by addition of 1M aqueous sodium hydroxide solution (113 mL). The mixture was extracted once with ethyl acetate (100 mL) by liquid separation, and the organic layer was washed twice with water (50 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give a crude product (6.56 g) as a pale-yellow liquid. The crude product was purified by flash silica gel column (normal phase silica gel 30 g, hexane/ethyl acetate = 75/25 - 25/75) to give Fragment D-11 (4.73 g, two-step yield from Boc-L-Asp(OBzl)-OH 77%) as a pale-yellow liquid.

### (Example 6E: Synthesis of Fragment D-12)

Fragment D-12 was synthesized as follows.

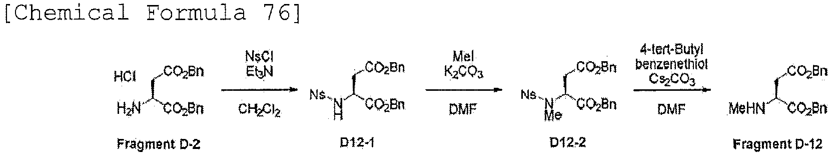

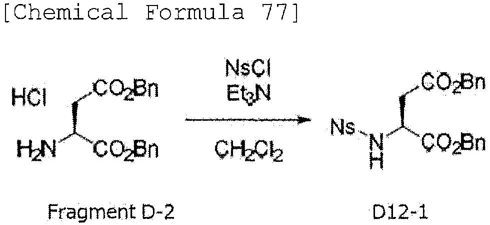

Under nitrogen stream, to a solution of Fragment D-2 (1.0 eq., 10.00 g, 28.58 mmol) in dichloromethane (100 mL) were added triethylamine (2.5 eq., 7.23 g, 71.44 mmol) and NsCl (1.2 eq., 7.60 g, 34.29 mmol) under ice-cooling, and the mixture was stirred at the same temperature for 0.5 hr, and then stirred for 17 hr while warming to room temperature. The reaction solution was washed once with water (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (14.78 g). This crude product was dissolved in ethyl acetate (100 mL), and the solution was washed once with 5% aqueous sodium chloride solution (50 mL) by liquid separation, and dried over sodium sulfate. The sodium sulfate was removed by filtration, and the filtrate was concentrated under reduced pressure to give D12-1 (14.55 g, containing 0.6wt% ethyl acetate, quant.) as a yellow solid.

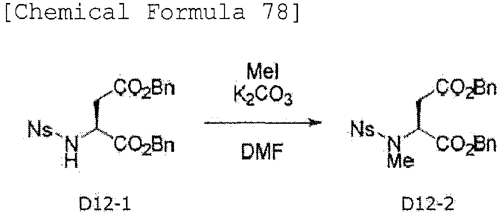

Under nitrogen atmosphere, to a solution of D12-1 (1.0 eq., 14.55 g, calculated as 28.58 mmol) in DMF (100 mL) were added potassium carbonate (2.0 eq., 7.90 g, 57.16 mmol) and methyl iodide (2.0 eq., 8.11 g, 57.14 mmol) under ice-cooling, and the mixture was stirred at the same temperature for 1 hr. To the reaction solution was added toluene (200 mL), and the mixture was washed once with water (200 mL) and once with 5% brine (200 mL) by liquid separation, and dried over sodium sulfate. The sodium sulfate was removed by filtration, and the filtrate was concentrated under reduced pressure to give D12-2 (14.78 g, DMF 0.4wt%, two-step conversion yield from Fragment D-2 99%) as a pale-brown viscous product.

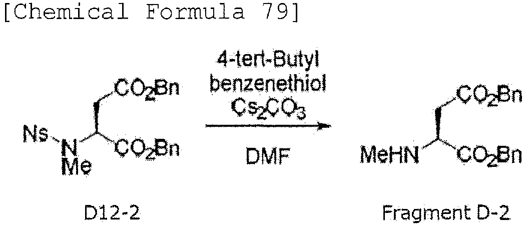

Under nitrogen atmosphere, to a solution of D12-2 (1.0 eq., 14.78 g, 28.29 mmol) in DMF (140 mL) were added cesium carbonate (1.5 eq., 13.84 g, 42.48 mmol) and 4-tert-butylbenzenethiol (1.5 eq., 7.06 g, 42.46 mmol) at room temperature, and the mixture was stirred at the same temperature for 2 hr. To the reaction solution was added toluene (300 mL), and the mixture was washed once with water (300 mL), once with 5% aqueous potassium carbonate solution (300 mL) and once with 5% aqueous sodium chloride solution (300 mL) by liquid separation, and dried over sodium sulfate. The sodium sulfate was removed by filtration, and the filtrate was concentrated under reduced pressure. The obtained crude product (19.66 g) was purified by flash silica gel column (normal phase silica gel 100 g, hexane/ethyl acetate = 90/10 - 0/100) to give Fragment D-12 (7.52 g, yield 81%) as an orange oil.

### (Example 6F: Synthesis of Fragment D-13)

Fragment D-13 was synthesized as follows.

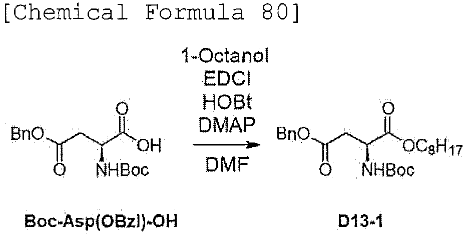

Under nitrogen atmosphere, to a solution of Boc-L-Asp(OBzl)-OH (1.0 eq., 5.00 g, 15.5 mmol) in DMF (45 mL) were added HOBt·H₂O (1.2 eq., 2.85 g, 18.6 mmol), EDCI (1.2 eq., 3.57 g, 18.6 mmol), 1-octanol (1.2 eq., 3.0 mL, 19.1 mmol) and DMAP (1.0 eq., 1.89 g, 15.5 mmol) at room temperature, and the mixture was stirred at the same temperature for 1 hr. To the reaction solution were added hexane (25 mL) and ethyl acetate (75 mL), and the mixture was washed twice with water (50 mL), twice with a mixture of saturated aqueous sodium hydrogencarbonate solution (25 mL)/water (25 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give D13-1 (7.09 g) as a pale-yellow liquid.

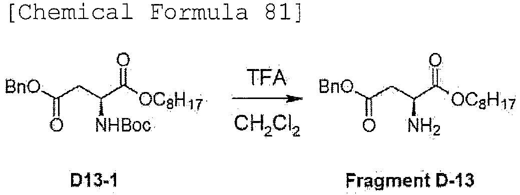

Under nitrogen atmosphere, to a solution of D13-1 (1.0 eq., 7.09 g, calculated as 15.5 mmol) in dichloromethane (30 mL) was added TFA (10 eq., 12.3 mL, 161 mmol) at room temperature, and the mixture was stirred at room temperature for 1 hr. To the reaction solution was added 1M aqueous sodium hydroxide solution (145 mL), and the mixture was extracted once with ethyl acetate (100 mL) by liquid separation. The organic layer was washed twice with water (50 mL) and once with saturated aqueous sodium chloride solution (25 mL) by liquid separation. The organic layer was dried over magnesium sulfate, the magnesium sulfate was removed by filtration, and the filtrate was concentrated under reduced pressure to give a crude product (5.62 g) as a pale-yellow liquid. The crude product was purified by flash silica gel column (normal phase silica gel 41 g, hexane/ethyl acetate = 3/1 - 1/1) to give Fragment D-13 (4.20 g, two-step yield 81%) as a pale-yellow liquid.

### (Example 6F: Synthesis of Fragment D-14)

Fragment D-14 was synthesized as follows.

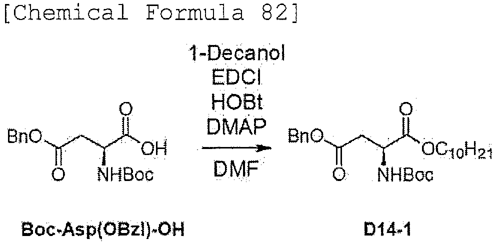

Under nitrogen atmosphere, to a solution of Boc-L-Asp(OBzl)-OH (1.0 eq., 5.00 g, 15.5 mmol) in DMF (45 mL) were added HOBt·H₂O (1.2 eq., 2.85 g, 18.6 mmol), EDCI (1.2 eq., 3.57 g, 18.6 mmol), 1-decanol (1.2 eq., 3.5 mL, 18.4 mmol) and DMAP (1.0 eq., 1.90 g, 15.5 mmol) at room temperature, and the mixture was stirred at the same temperature for 1 hr. To the reaction solution were added hexane (25 mL) and ethyl acetate (75 mL), and the mixture was washed twice with water (50 mL), twice with a mixture of saturated aqueous sodium hydrogencarbonate solution (25 mL)/water (25 mL) and once with saturated aqueous sodium chloride solution (25 mL) by liquid separation. The organic layer was dried over magnesium sulfate, the magnesium sulfate was removed by filtration, and the filtrate was concentrated under reduced pressure to give D14-1 (7.80 g) as a pale-yellow liquid.

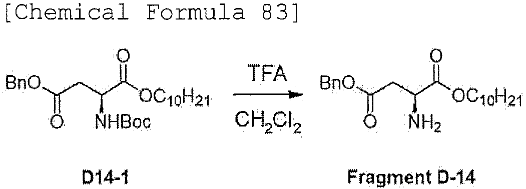

Under nitrogen atmosphere, to a solution of D14-1 (1.0 eq., 7.80 g, calculated as 15.5 mmol) in dichloromethane (30 mL) was added TFA (10 eq., 12.0 mL, 157 mmol) at room temperature, and the mixture was stirred at room temperature for 2 hr. To the reaction solution was added 1M aqueous sodium hydroxide solution (130 mL), and the mixture was extracted once with ethyl acetate (100 mL) by liquid separation. The organic layer was washed twice with water (50 mL) and once with saturated aqueous sodium chloride solution (25 mL) by liquid separation. The organic layer was dried over magnesium sulfate, the magnesium sulfate was removed by filtration, and the filtrate was concentrated under reduced pressure to give a crude product (6.61 g) as a pale-yellow liquid. The crude product was purified by flash silica gel column (normal phase silica gel 33 g, hexane/ethyl acetate = 3/1 - 1/1) to give Fragment D-14 (4.00 g, two-step yield 71%) as a pale-yellow liquid.

### (Example 7: Synthesis of Compound 1)

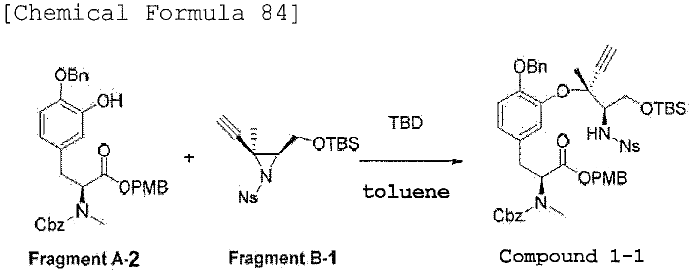

Under nitrogen atmosphere, to a solution of Fragment A-2 (1.05 eq., 5.81 g, containing 2.2wt% ethyl acetate, 10.23 mmol) and Fragment B-1 (1.0 eq., 4.00 g, 9.74 mmol) in toluene (111 mL) was added TBD (1.05 eq., 1.42 g, 10.20 mmol) under ice-cooling, and the mixture was stirred for 22 hr while warming to room temperature. To the reaction solution was added 5% aqueous citric acid solution (50 mL), and the mixture was washed once by liquid separation, and the organic layer was washed once with a mixture of 5% aqueous sodium hydrogencarbonate solution (50 mL)/5% aqueous sodium chloride solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (10.90 g) as a pale-brown viscous product. The crude product was purified by flash silica gel column (normal phase silica gel 100 g, hexane/ethyl acetate = 80/20 - 0/100) to give Compound 1-1 (8.49 g, containing Fragment A-2, PMBOH and ethyl acetate, apparent yield 90%) as a yellow viscous product.

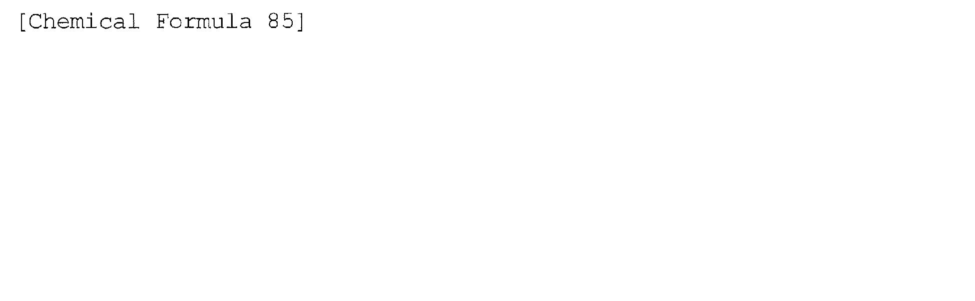

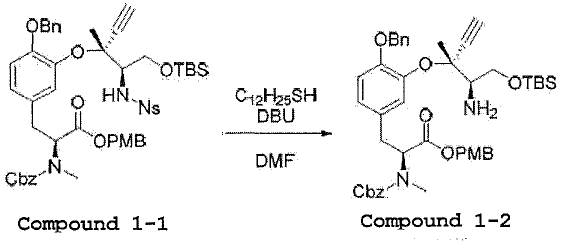

Under nitrogen atmosphere, to a solution of Compound 1-1 (1.0 eq., 2.97 g, containing Fragment A-2, PMBOH and ethyl acetate, calculated as 3.07 mmol) in DMF (15 mL) were added 1-dodecanethiol (6.0 eq., 3.73 g, 18.43 mmol) and DBU (6.0 eq., 2.81 g, 18.46 mmol) under ice-cooling, and the mixture was stirred at room temperature for 5 hr. To the reaction solution were added toluene (30 mL) and 5% aqueous citric acid solution (40 mL), and the mixture was extracted once by liquid separation, and the aqueous layer was extracted again with toluene (30 mL) by liquid separation. The organic layers were combined, washed once with a mixture of 5% aqueous sodium hydrogencarbonate solution (30 mL)/5% aqueous sodium chloride solution (30 mL) by liquid separation, and dried over sodium sulfate, the sodium sulfate was removed by filtration, and the filtrate was concentrated under reduced pressure to give a crude product (6.54 g) as a yellow oil.

The crude product (2.47 g) obtained from Compound 1-1 (1.03 g) by the same procedure and the previous crude product were combined, and purified by flash silica gel column (normal phase silica gel 100 g, hexane/ethyl acetate = 90/10 - 50/50) to give Compound 1-2 (2.32 g, containing PMBOH and ethyl acetate, apparent yield 71%) as a pale-yellow oil.

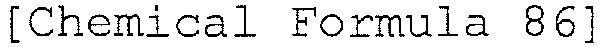

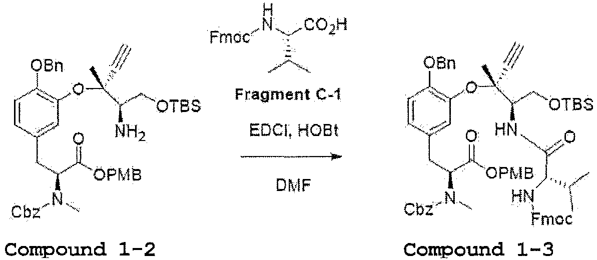

Under nitrogen atmosphere, to a solution of Compound 1-2 (1.0 eq., 2.32 g, containing 2.1wt% ethyl acetate, 2.06 mmol) in DMF (24 mL) were added N-Fmoc-L-valine (Fragment C-1, 2.0 eq., 1.39 g, 4.10 mmol), EDCI (2.0 eq., 0.79 g, 4.12 mmol) and HOBt·H₂O (2.0 eq., 0.63 g, 4.11 mmol), and the mixture was stirred at room temperature for 3.5 hr. To the reaction solution were added toluene (50 mL) and 5% aqueous sodium hydrogencarbonate solution (50 mL), and the mixture was extracted once by liquid separation, and the organic layer was washed once with 5% aqueous sodium chloride solution (50 mL) by liquid separation, and dried over sodium sulfate. The sodium sulfate was removed by filtration, and the filtrate was concentrated under reduced pressure to give a crude product (3.49 g) as a yellow viscous product. The crude product was purified by flash silica gel column (NH₂ silica gel 28 g, hexane/ethyl acetate = 90/10 - 50/50) to give Compound 1-3 (2.22 g, yield 98%) as a white amorphous.

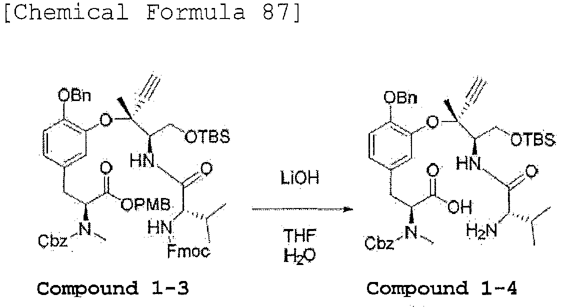

Under nitrogen atmosphere, to a solution of Compound 1-3 (1.0 eq., 2.22 g, 2.01 mmol) in a mixture of THF (20 mL)/water (10 mL) was added lithium hydroxide monohydrate (4.0 eq., 0.34 g, 8.10 mmol), and the mixture was stirred at room temperature for 3 hr. The reaction solution was ice-cooled, adjusted to pH 3 by addition of 5% aqueous citric acid solution (30 mL), and extracted once with ethyl acetate (30 mL) by liquid separation. The organic layer was washed once with 5% aqueous sodium chloride solution (30 mL) by liquid separation, and dried over sodium sulfate. The sodium sulfate was removed by filtration, and the filtrate was concentrated under reduced pressure to give a crude product (2.45 g) as a pale-yellow viscous product. The crude product was purified by flash silica gel column (normal phase silica gel 25 g, ethyl acetate/methanol = 100/0 - 90/10) to give Compound 1-4 (1.36 g, containing 4.4wt% ethyl acetate, conversion yield 85%) as a white amorphous.

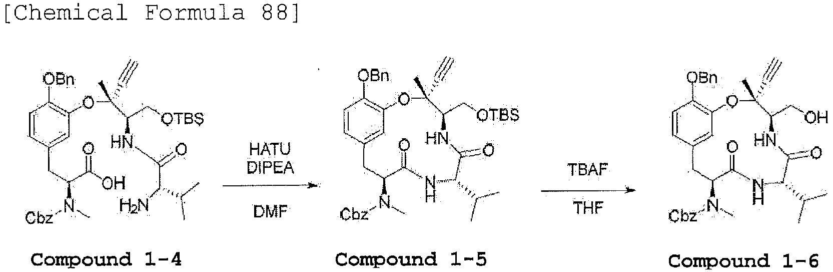

Under nitrogen atmosphere, to a solution of HATU (5.0 eq., 1.63 g, 4.29 mmol) in DMF (842 mL) was added DIPEA (10.0 eq., 1.11 g, 8.59 mmol) at room temperature. Then, a solution of Compound 1-4 (1.0 eq., 0.68 g, containing 4.4wt% ethyl acetate, 0.855 mmol) in DMF (8 mL) was added over 17 hr at the same temperature, and after the dropwise addition was completed, the mixture was stirred at room temperature for additional 1.5 hr. The reaction solution was concentrated under reduced pressure, to the concentrated residue were added ethyl acetate (15 mL) and 5% aqueous citric acid solution (15 mL), and the mixture was washed once by liquid separation. The organic layer was washed once with 5% aqueous sodium hydrogencarbonate solution (15 mL) and once with 5% aqueous sodium chloride solution (30 mL) by liquid separation, and dried over sodium sulfate. The sodium sulfate was removed by filtration, and the filtrate was concentrated under reduced pressure to give a mixture (1.05 g) of Compound 1-5 and Compound 1-6 as a pale-brown amorphous.

Under nitrogen atmosphere, to a solution of a mixture (1.0 eq., 0.95 g, calculated as 0.774 mmol) of Compound 1-5 and Compound 1-6 in THF (19 mL) was added TBAF (1.1M in THF, 8.0 eq., 5.63 mL, 6.193 mmol) under ice-cooling, and the mixture was stirred for 4 hr while warming to room temperature. The reaction solution was ice-cooled, 10% aqueous ammonium chloride solution (40 mL) was added, and the mixture was extracted once with ethyl acetate (40 mL) by liquid separation. The organic layer was washed once with 5% aqueous sodium chloride solution (40 mL) by liquid separation, and dried over sodium sulfate, the sodium sulfate was removed by filtration, and the filtrate was concentrated under reduced pressure to give a crude product (1.19 g) as a pale-brown viscous product.

The crude product (106 mg) obtained from a mixture (0.10 g, calculated as 0.081 mmol) of Compound 1-5 and Compound 1-6 by the same procedure and the previous crude product were combined, and purified by flash silica gel column (normal phase silica gel 10 g, hexane/ethyl acetate = 50/50) to give Compound 1-6 (0.60 g, an isomer mixture. containing 0.7wt% ethyl acetate and 36.4wt% TBAF, two-step conversion yield from Compound 1-4 70%) as a pale-orange solid.

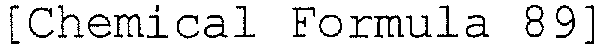

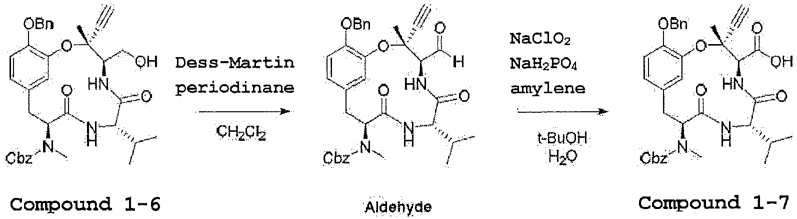

Under nitrogen atmosphere, to a solution of Compound 1-6 (1.0 eq., 0.66 g, an isomer mixture, containing 3.9wt% ethyl acetate and 38.9wt% TBAF, 0.60 mmol) in dichloromethane (13 mL) was added Dess-Martin periodinane (1.5 eq., 0.38 g, 0.90 mmol) under ice-cooling, and the mixture was stirred for 16 hr while warming to room temperature. To the reaction solution were added 5% aqueous sodium hydrogencarbonate solution (10 mL) and 5% aqueous sodium thiosulfate solution (10 mL), and the mixture was washed once by liquid separation. The organic layer was washed once with 5% aqueous sodium chloride solution (20 mL) by liquid separation, and dried over sodium sulfate, the sodium sulfate was removed by filtration, and the filtrate was concentrated under reduced pressure to give Aldehyde (0.392 g, containing an isomer) as a pale-orange amorphous.

Under nitrogen atmosphere, to a solution of Aldehyde (1.0 eq., 0.39 g, containing an isomer, calculated as 0.60 mmol) in a mixture of t-butyl alcohol (16 mL)/amylene (4 mL)/water (4 mL) were added sodium dihydrogen phosphate dihydrate (3.5 eq., 0.33 g, 2.12 mmol) and 80% sodium chlorite (4.5 eq., 0.30 g, 2.65 mmol) at room temperature, and the mixture was stirred at the same temperature for 1.5 hr. To the reaction solution were added ethyl acetate (20 mL) and water (20 mL), and the mixture was extracted once by liquid separation, and the organic layer was washed twice with water (20 mL) and once with 5% aqueous sodium chloride solution (20 mL) by liquid separation, and dried over sodium sulfate. The sodium sulfate was removed by filtration, and the filtrate was concentrated under reduced pressure to give Compound 1-7 (0.351 g, an isomer mixture, containing 12.5wt% TBAF, two-step conversion yield from Compound 1-6 80%) as a pale-orange solid.

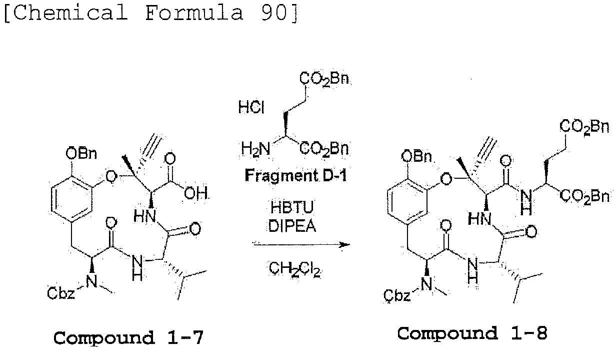

Under nitrogen atmosphere, to a solution of Compound 1-7 (1.0 eq., 130 mg, an isomer mixture, containing 24.8wt% TBAF, 0.15 mmol) and dibenzyl L-glutamate hydrochloride (Fragment D-1, 1.5 eq., 83 mg, 0.23 mmol) in dichloromethane (1.3 mL) were added DIPEA (3.0 eq., 78 µL, 0.46 mmol) and HBTU (1.5 eq., 87 mg, 0.23 mmol) under ice-cooling, and the mixture was stirred for 3.5 hr while warming to room temperature.

The reaction solution obtained by treating Compound 1-7 (100 mg) by the same procedure was combined with the previous reaction solution, and toluene (20 mL), 5% aqueous citric acid solution (20 mL) and 5% aqueous sodium chloride solution (10 mL) were added, and the mixture was washed once by liquid separation. The organic layer was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate, and the sodium sulfate was removed by filtration. The filtrate was concentrated under reduced pressure, and the crude product (290 mg) obtained as a pale-brown viscous product was purified by flash silica gel column (NH₂ silica gel 6.5 g, hexane/ethyl acetate = 50/50) to give Compound 1-8 (37 mg, yield 14%) as a yellow viscous product.

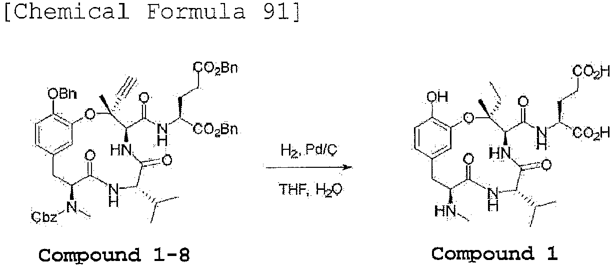

Under nitrogen atmosphere, to a solution of Compound 1-8 (1.0 eq., 37 mg, 0.039 mmol) in a mixture of THF (1.85 mL)/water (1.85 mL) was added 10% palladium-carbon (N.E. CHEMCAT NX type, 50% hydrous product, 11.1 mg) at room temperature, the system was replaced with hydrogen, and the mixture was stirred at the same temperature for 16 hr. The catalyst was removed by filtration through Celite, and the filtrate was concentrated under reduced pressure. To the concentrated residue was added a mixture of THF (1.85 mL)/water (1.85 mL), the system was replaced with nitrogen, and 10% palladium-carbon (N.E. CHEMCAT NX type, 50% hydrous product, 11.1 mg) was added at room temperature. The system was replaced with hydrogen, and the mixture was stirred at the same temperature for 3 hr. As the reaction proceeded, the target product precipitated as a solid, so TFA (2.0 eq., 6.0 µL, 0.078 mmol) was added to dissolve the target product. The catalyst was removed by filtration through Celite, and the filtrate was concentrated under reduced pressure to give a crude product (27 mg) as a pale-orange amorphous.

The crude product (15.5 mg) obtained from Compound 1-8 (21 mg) by the same procedure and the previous crude product were combined, purified twice by flash silica gel column (reverse-phase silica gel 60 g, 1st run: 0.05% aqueous TFA solution/acetonitrile = 99/1 - 90/10, 2nd run: 0.05% aqueous TFA solution/acetonitrile = 95/5), and freeze-dried to give a TFA salt of Compound 1 (18 mg, yield 45%, purity 97.1%) as a white solid.

¹H-NMR(400MHz,D₂O)δ6.94-6.87(m,3H),4.86(s,1H),4.37(dd,J=9.2,4.8Hz,1H),4.12(d,J = 100Hz,1H),4.05(dd,J=8.8,4.8Hz,1H),3.23(dd,J=13.2,4.8Hz,1H),2.92 (dd,J=13.2,8.8Hz,1H),2.72(s,3H),2.48-2.35(m,2H),2.22-2.14(m,1H),2.03-1.90(m,3H),1.77-1.63(m,1H),1.57(s,3H),1.04(t,J=7.2Hz,3H),0.85(d,J=6.4Hz,3H),0.7 8(d,J=6.4Hz,3H).

### (Example 8: Synthesis of Compound 9)

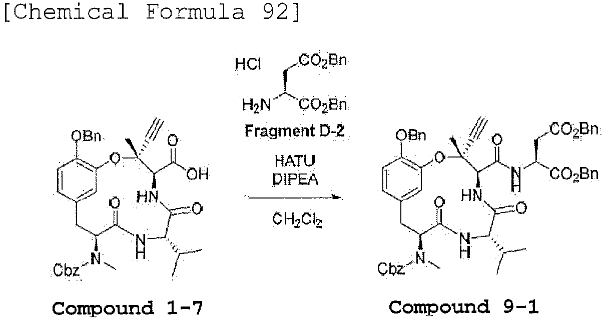

Under nitrogen atmosphere, to a solution of Compound 1-7 (1.0 eq., 264 mg, an isomer mixture, containing 12.5wt% TBAF, 0.36 mmol) and dibenzyl L-aspartate hydrochloride (Fragment D-2, 1.5 eq., 188 mg, 0.54 mmol) in dichloromethane (6.6 mL) were added DIPEA (1.5 eq., 91 µL, 0.54 mmol) and HATU (1.5 eq., 204 mg, 0.54 mmol) under ice-cooling, and the mixture was stirred at the same temperature for 6 hr. To the reaction solution was added 5% aqueous citric acid solution (10 mL), and the mixture was washed once by liquid separation, and the organic layer was washed once with a mixture of 5% aqueous sodium hydrogencarbonate solution (10 mL)/5% aqueous sodium chloride solution (10 mL) by liquid separation, and dried over sodium sulfate. The sodium sulfate was removed by filtration, and the filtrate was concentrated under reduced pressure. The crude product (496 mg) obtained as a pale-brown viscous product was purified twice by flash silica gel column (normal phase silica gel 10 g, 1st run: chloroform/ethyl acetate = 90/10 - 50/50, 2nd run: chloroform/ethyl acetate = 90/10 - 85/15) to give Compound 9-1 (129 mg) with low purity

Compound 9-1 with low purity was dissolved in toluene (10 mL), and the solution was washed five times by 5% aqueous sodium chloride solution (10 mL) by liquid separation, and dried over sodium sulfate. The sodium sulfate was removed by filtration, and the filtrate was concentrated under reduced pressure to give Compound 9-1 (93 mg, yield 28%) as a yellow amorphous.

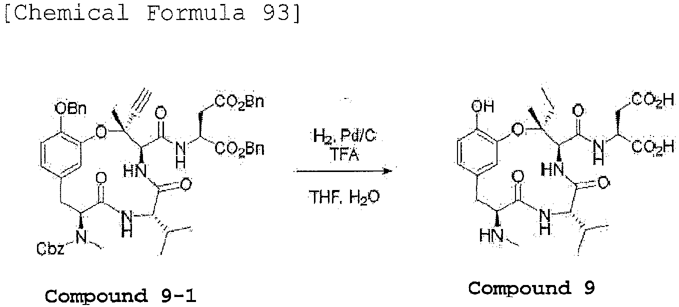

Under nitrogen atmosphere, to a solution of Compound 9-1 (1.0 eq., 50 mg, 0.053 mmol) in a mixture of THF (2.5 mL)/water (2.5 mL) were added 10% palladium-carbon (N.E. CHEMCAT NX type, 50% hydrous product, 2.5 mg) and TFA (2.0 eq., 8.2 µL, 0.107 mmol) at room temperature, the system was replaced with hydrogen, and the mixture was stirred at the same temperature for 23 hr. Additional 10% palladium-carbon (N.E. CHEMCAT NX type, 50% hydrous product, 2.5 mg) was added, and the mixture was stirred for 27 hr. Additional 10% palladium-carbon (N.E. CHEMCAT NX type, 50% hydrous product, 2.5 mg) was added again, and the mixture was stirred for additional 24 hr.

The reaction solution obtained by treating Compound 9-1 (10 mg) by the same procedure and the previous reaction solution were combined, the catalyst was removed by filtration through Celite, and the filtrate was concentrated under reduced pressure to give a crude product (58 mg) as an orange viscous product. The crude product was purified by flash silica gel column (reverse-phase silica gel 30 g, 0.05% aqueous TFA solution/acetonitrile = 95/5 - 90/10), and freeze-dried to give a TFA salt of Compound 9 (30.5 mg, yield 73%, purity 97.8%) as a white solid.

¹H-NMR(400MHz,D₂O)56.94-6.87(m,3H),4.66(dd,J=7.2,5.2Hz,1H),4.11(d,J = 100Hz,1H),4.05(dd,J=8.8,4.8Hz,1H),3.24(dd,J=13.6,4.8Hz,1H),2.96 -2.84(m,3H),2.72(s,3H),2.05-1.91(m,2H),1.78-1.69(m,1H),1.58(s,3H),1.05(t,J=7.2Hz,3H),0.86(d,J=7.2Hz,3H),0.7 8(d,J=6.8Hz,3H).

### (Example 9: Synthesis of Compound 2)

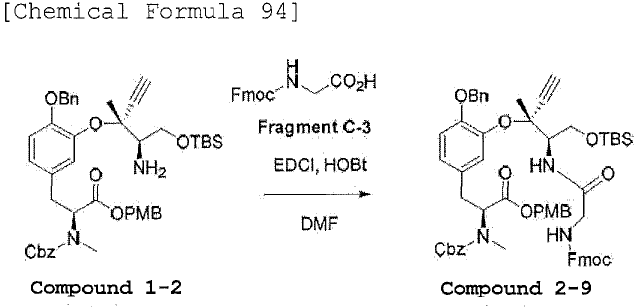

Under nitrogen atmosphere, to a solution of Compound 1-2 (1.0 eq., 2.32 g, containing PMBOH and ethyl acetate, calculated as 2.97 mmol) in DMF (35 mL) were added N-Fmoc-L-glycine (Fragment C-3, 2.0 eq., 1.77 g, 5.95 mmol), EDCI (2.0 eq., 1.14 g, 5.95 mmol) and HOBt·H₂O (2.0 eq., 0.91 g, 5.94 mmol), and the mixture was stirred at room temperature for 2 hr. To the reaction solution were added toluene (70 mL) and 5% aqueous sodium hydrogencarbonate solution (70 mL), and the mixture was extracted once by liquid separation, and the organic layer was washed once with 5% aqueous sodium chloride solution (70 mL) by liquid separation, and dried over sodium sulfate. The sodium sulfate was removed by filtration, and the filtrate was concentrated under reduced pressure to give a crude product (4.56 g) as a white solid. To the crude product was added ethyl acetate (45 mL), and the mixture was subjected to ultrasonic irradiation. The insoluble substance was removed by filtration, and the filtrate was concentrated under reduced pressure. The concentrated residue was dissolved in THF, and the solution was concentrated under reduced pressure to give Compound 2-9 (3.50 g, containing impurities, THF and DMF) as a white amorphous.

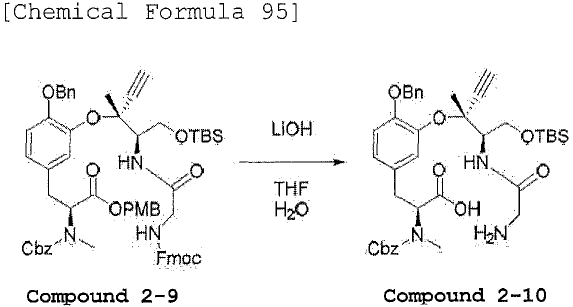

Under nitrogen atmosphere, to a solution of Compound 2-9 (1.0 eq., 3.50 g, 2.97 mmol) in a mixture of THF (28 mL)/water (14 mL) was added lithium hydroxide monohydrate (4.0 eq., 0.50 g, 11.92 mmol), and the mixture was stirred at room temperature for 3 hr. The reaction solution was ice-cooled, adjusted to pH 3 by addition of 5% aqueous citric acid solution (45 mL), and extracted once with ethyl acetate (45 mL) by liquid separation. The organic layer was washed once with 5% aqueous sodium chloride solution (45 mL) by liquid separation, and dried over sodium sulfate. The sodium sulfate was removed by filtration, and the filtrate was concentrated under reduced pressure to give a crude product (3.55 g) as a pale-yellow viscous product. The crude product was purified by flash silica gel column (normal phase silica gel 25 g, ethyl acetate/methanol = 100/0 - 70/30) to give Compound 2-10 (1.63 g, containing 7.3wt% ethyl acetate, four-step conversion yield from Fragment B-1 42%) as a pale-orange amorphous.

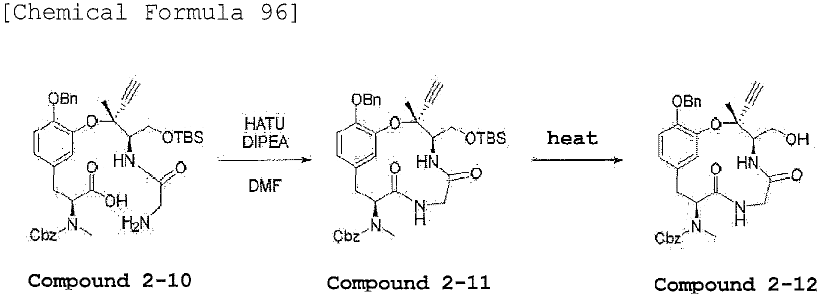

Under nitrogen atmosphere, to a solution of HATU (5.0 eq., 1.63 g, 4.29 mmol) in DMF (842 mL) was added DIPEA (10.0 eq., 1.11 g, 8.59 mmol) at room temperature. Then, a solution of Compound 2-10 (1.0 eq., 0.66 g, containing 7.3wt% ethyl acetate, 0.852 mmol) in DMF (8 mL) was added over 17 hr at the same temperature, and after the dropwise addition was completed, the mixture was stirred at room temperature for additional 1 hr. The reaction solution was concentrated under reduced pressure until the volume became several mL.

Under nitrogen atmosphere, and the concentrated residue was stirred at the external temperature of 50°C for 17 hr, and then at the external temperature of 70°C for 2 hr. To the reaction solution were added ethyl acetate (20 mL) and 5% aqueous citric acid solution (20 mL), and the mixture was extracted once by liquid separation, and the organic layer was washed once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give a crude product (0.98 g) as a pale-brown viscous product. The crude product was purified by flash silica gel column (normal phase silica gel 10 g, chloroform/ethyl acetate = 90/10 - 5/95) to give Compound 2-12 (0.168 g, containing an isomer and impurities, two-step apparent yield from Compound 2-10 34%) as an orange solid.

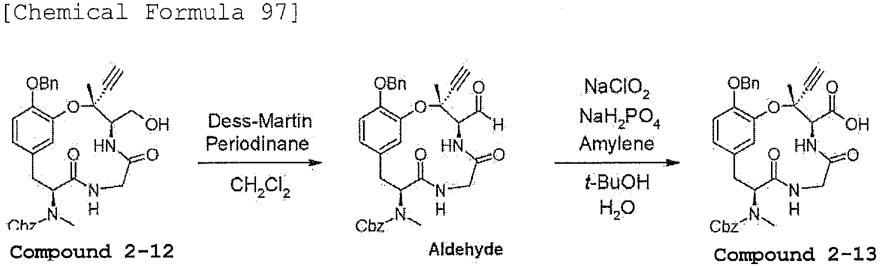

Under nitrogen atmosphere, to a solution of Compound 2-12 (1.0 eq., 165 mg, containing an isomer and impurities, calculated as 0.282 mmol) in dichloromethane (3.3 mL) was added Dess-Martin periodinane (1.5 eq., 179 mg, 0.422 mmol) under ice-cooling, and the mixture was stirred for 2 hr while warming to room temperature. To the reaction solution were added 5% aqueous sodium hydrogencarbonate solution (3 mL) and 5% aqueous sodium thiosulfate solution (3 mL), and the mixture was extracted once by liquid separation. The organic layer was washed once with 5% aqueous sodium chloride solution (3 mL) by liquid separation, and dried over sodium sulfate. The sodium sulfate was removed by filtration, and the filtrate was concentrated under reduced pressure to give Aldehyde (133 mg, containing an isomer, apparent yield 81%) as a pale-orange amorphous.

Under nitrogen atmosphere, to a solution of Aldehyde (1.0 eq., 131 mg, containing an isomer, calculated as 0.224 mmol) in a mixture of t-butyl alcohol (5.6 mL)/amylene (1.4 mL)/water (1.4 mL) were added sodium dihydrogen phosphate dihydrate (3.5 eq., 123 mg, 0.788 mmol) and 80% sodium chlorite (4.5 eq., 114 mg, 1.008 mmol) at room temperature, and the mixture was stirred at the same temperature for 1 hr. To the reaction solution were added ethyl acetate (15 mL) and water (15 mL), and the mixture was extracted once by liquid separation. The organic layer was washed once with 5% aqueous sodium chloride solution (15 mL) by liquid separation, and dried over sodium sulfate. The sodium sulfate was removed by filtration, and the filtrate was concentrated under reduced pressure to give Compound 2-13 (97 mg, containing an isomer, two-step conversion yield from Compound 2-12 58%) as a yellow solid.

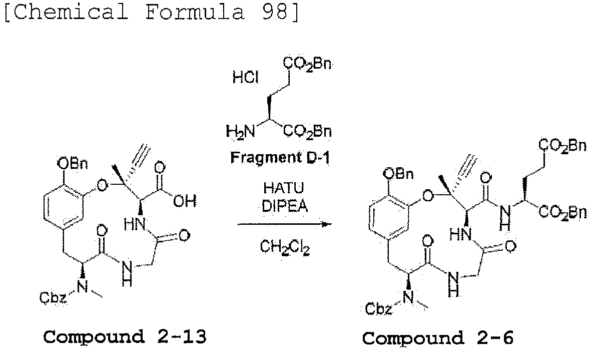

Under nitrogen atmosphere, to a solution of Compound 2-13 (1.0 eq., 79 mg, containing an isomer, 0.132 mmol) and dibenzyl L-glutamate hydrochloride (Fragment D-1, 1.5 eq., 72 mg, 0.198 mmol) in dichloromethane (2 mL) were added DIPEA (1.5 eq., 34 µL, 0.197 mmol) and HATU (1.5 eq., 75 mg, 0.200 mmol) under ice-cooling, and the mixture was stirred for 6 hr while warming to room temperature. To the reaction solution were added ethyl acetate (10 mL) and 5% aqueous citric acid solution (10 mL), and the mixture was extracted once by liquid separation. The organic layer was washed once with 5% aqueous sodium hydrogencarbonate solution (10 mL) and once with 5% aqueous sodium chloride solution (10 mL) by liquid separation, and dried over sodium sulfate. The sodium sulfate was removed by filtration, and the filtrate was concentrated under reduced pressure. The crude product (160 mg) obtained as a pale-brown viscous product was purified twice by flash silica gel column (1st and 2nd run: normal phase silica gel 10 g, chloroform/ethyl acetate = 90/10 - 60/40) to give Compound 2-6 (67 mg, containing Fragment D-1 and ethyl acetate, apparent yield 56%) as a yellow amorphous.

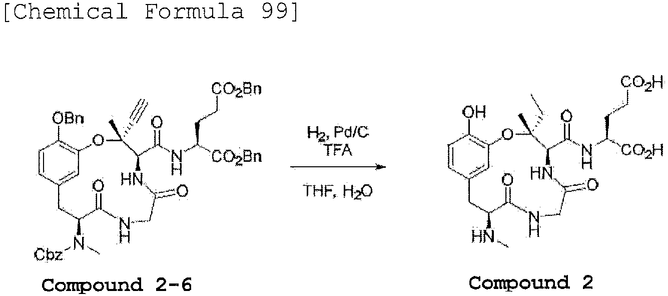

Under nitrogen atmosphere, to a solution of Compound 2-6 (1.0 eq., 64 mg, containing Fragment D-1 and ethyl acetate, calculated as 0.070 mmol) in a mixture of THF (3.2 mL)/water (3.2 mL) were added 10% palladium-carbon (N.E. CHEMCAT NX type, 50% hydrous product, 3.2 mg) and TFA (2.0 eq., 10.7 µL, 0.140 mmol) at room temperature, the system was replaced with hydrogen, and the mixture was stirred at the same temperature for 66 hr. Additional 10% palladium-carbon (N.E. CHEMCAT NX type, 50% hydrous product, 3.2 mg) was added, and the mixture was stirred for 21.5 hr. The catalyst was removed by filtration through Celite, and the filtrate was concentrated under reduced pressure, and then the reaction was carried out again. To the concentrated residue was added a mixture of THF (3.2 mL)/3.2 mL), and the system was replaced with nitrogen, 10% palladium-carbon (N.E. CHEMCAT NX type, 50% hydrous product, 5.8 mg) and TFA (2.0 eq., 10.7 µL, 0.140 mmol) were added at room temperature. The system was replaced with hydrogen, and the mixture was stirred at the same temperature for 23 hr. The catalyst was removed by filtration through Celite, and the filtrate was concentrated under reduced pressure to give a crude product (59 mg) as a pale-brown viscous product. The crude product was purified by flash silica gel column (reverse-phase silica gel 30 g, 0.05% aqueous TFA solution/acetonitrile = 95/5), and freeze-dried to give a TFA salt of Compound 2 (5.2 mg, yield 12%, purity 97.1%) as a white solid.

¹H-NMR(400MHz,D₂O)δ7.45(s,1H),7.05-7.03(m,1H),6.96(d,J=8.4Hz,1H),4.63(s,1H),4.45(dd,J=8.8,5.2Hz,1H ),4.26(d,J=16.8Hz,1H),3.98(dd,J=10.4,3.6Hz,1H),3.54(d,J=16.4Hz, 1H),3.25(dd,J=13.2,4.0Hz,1H),3.07-3.01(m,1H),2.72(s,3H),2.47(t,J=7.2Hz,2H),2.25-2.20(m,1H),2.01-1.97 (m,2H),1.76-1.70(m,1H),1.61(s,3H),0.87(t,J=7.2Hz, 3H).

### (Example 9A: Synthesis of Compound 9A)

The steps up to Compound 1-7 are similar to the synthesis method of Compound 1.

From Compound 1-7 to Compound 9A-1

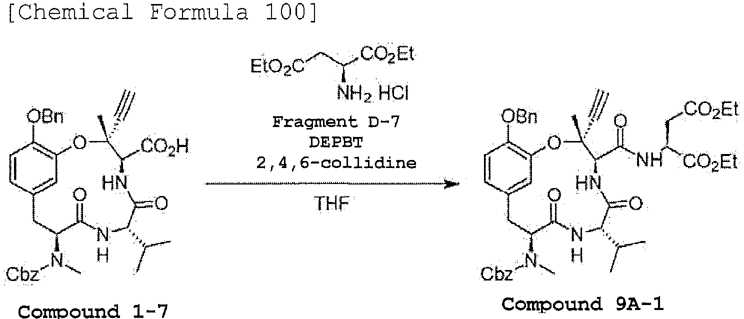

Under nitrogen atmosphere, to a solution of Compound 1-7 (1.0 eq., 82.2 mg, 0.128 mmol) and diethyl L-aspartate hydrochloride (Fragment D-7, 1.5 eq., 43.2 mg, 0.191 mmol) in THF (1.2 mL) were added DEPBT (1.5 eq., 57.5 mg, 0.192 mmol) and 2,4,6-collidine (3.0 eq., 46.7 mg, 0.383 mmol) under ice-cooling, and the mixture was stirred at the same temperature for 7 hr, and then stirred at room temperature for 15 hr. To the reaction solution was added ethyl acetate (10 mL), and the mixture was washed once with a mixture of water (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (162 mg) as a green oil. The crude product was purified by flash silica gel column (NH₂ silica gel 6 g, hexane/ethyl acetate = 75/25 - 50/50) to give Compound 9A-1 (48.5 mg, yield 47%) as a white amorphous.

Synthesis of Compound 9A from Compound 9A-1 was carried out as follows.

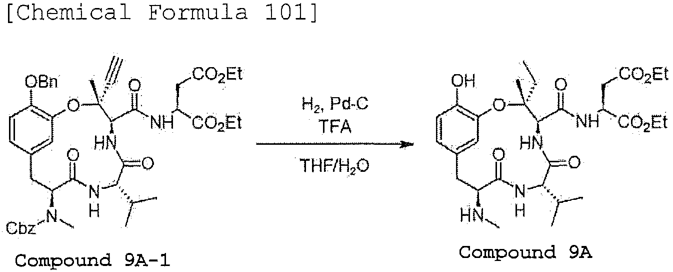

Under nitrogen atmosphere, to a solution of Compound 9A-1 (1.0 eq., 20 mg, 0.025 mmol) in a mixture of THF (800 µL)/water (40 µL) were added 10% palladium-carbon (N.E. CHEMCAT NX type, 50% hydrous product, 2.0 mg) and TFA (2.0 eq., 3.7 µL, 0.048 mmol) at room temperature. The system was replaced with hydrogen, and the mixture was stirred at the same temperature for 23 hr, and the catalyst was removed by filtration through Celite to give a filtrate.

The filtrate obtained from Compound 9A-1 (1.0 eq., 15 mg, 0.018 mmol) in the same manner was combined with the aforementioned filtrate were combined, and concentrated under reduced pressure to give a crude product. The obtained crude product (36.6 mg) was purified by flash silica gel column (reverse-phase silica gel 30 g, 0.05% aqueous TFA solution/acetonitrile = 95/5 - 75/25), and freeze-dried to give a TFA salt of Compound 9A (16.3 mg, yield 54%, purity 94.4%) as a white solid.

¹H-NMR(400MHz,D₂O)δ6.98-6.82(m,3H),4.24-4.09(m,5H),4.04(dd,J=8.8,4.4Hz,1H),3.22(dd,J=13.2,4.4Hz,1H),3.0 0-2.80(m,3H),2.71(s,3H),2.07-1.87(m,2H),1.79-1.62 (m,1H),1.57(s,3H),1.24(m,6H),1.05(t,J=7.2Hz,3H),0.85(d, J=6. 4Hz,3H),0.79(d,J=6.8Hz,3H).

### (Example 9B: Synthesis of Compound 9B)

The steps up to Compound 1-7 were carried out as described in Example 7.

Synthesis of Compound 9B-1 from Compound 1-7 was carried out as follows.

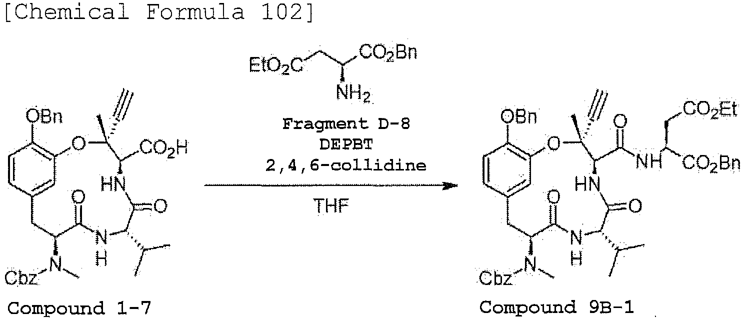

Under nitrogen atmosphere, to a solution of Compound 1-7 (1.0 eq., 78.0 mg, 0.122 mmol) and Fragment D-8 (1.5 eq., 47.2 mg, 0.188 mmol) in THF (1.2 mL) were added DEPBT (1.5 eq., 55.0 mg, 0.184 mmol) and 2,4,6-collidine (3.0 eq., 44.2 mg, 0.364 mmol) under ice-cooling, and the mixture was stirred at the same temperature for 6 hr, and then stirred at room temperature for 16 hr. To the reaction solution was added ethyl acetate (10 mL), and the mixture was washed once with a mixture of water (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (166 mg) as a green oil. The crude product was purified by flash silica gel column (NH₂ silica gel 6 g, hexane/ethyl acetate = 75/25 - 0/100) to give Compound 9B-1 (54.5 mg, yield 51%) as a white amorphous.

Synthesis of Compound 9B from Compound 9B-1 was carried out as follows.

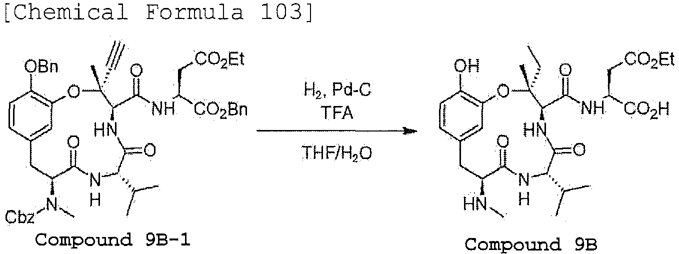

Under nitrogen atmosphere, to a solution of Compound 9B-1 (1.0 eq., 39 mg, 0.045 mmol) in a mixture of THF (1560 µL)/water (78 µL) were added 10% palladium-carbon (N.E. CHEMCAT NX type, 50% hydrous product, 3.9 mg) and TFA (5.0 eq., 17 µL, 0.223 mmol) at room temperature, the system was replaced with hydrogen, and the mixture was stirred at the same temperature for 4 hr. The catalyst was removed by filtration through Celite, and the filtrate was concentrated under reduced pressure to give a crude product (35 mg). The crude product (17 mg) obtained from Compound 9B-1 (15 mg, 0.017 mmol) by the same procedure and the aforementioned crude product were combined, purified by flash silica gel column (reverse-phase silica gel 30 g, 0.05% aqueous TFA solution/acetonitrile = 95/5 - 80/20), and freeze-dried to give a TFA salt of Compound 9B (24.3 mg, yield 58%, purity 95.2%) as a white solid.

¹H-NMR(400MHz,D₂O)δ6.97-6.86(m,3H),4.69(dd,J=7.2,5.2Hz,1H),4.18-4.10(m,3H),4.06(dd,J=9.2,4.8Hz,1H),3.24(dd,J=13.6,4.8Hz,1H),3.0 0-2.85(m,3H),2.73(s,3H),2.08-1.88(m,2H),1.79-1.65(m,1H),1.58(s,3H),1.24(t,J=7.2Hz,3H),1.05(t,J=7.2Hz,3H),0.8 6(d,J=6.8Hz,3H),0.79(d,J=6.8Hz,3H).

### (Example 9C: Synthesis of Compound 9C)

Synthesis of Compound 9C-1 from Compound 1-7 was carried out as follows.

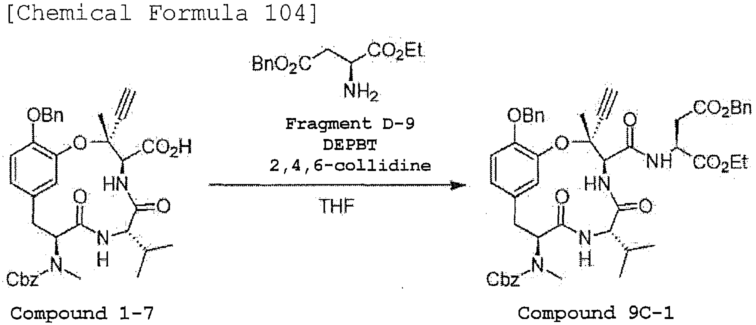

Under nitrogen atmosphere, to a solution of Compound 1-7 (1.0 eq., 59.7 mg, 93.0 µmol) and Fragment D-9 (1.5 eq., 34.6 mg, 138 µmol) in THF (1 mL) were added DEPBT (1.5 eq., 42.7 mg, 143 µmol) and 2,4,6-collidine (3.0 eq., 34.0 mg, 281 µmol) under ice-cooling, and the mixture was stirred at the same temperature for 5 hr, and then stirred at room temperature for 15.5 hr. To the reaction solution was added ethyl acetate (20 mL), and the mixture was washed once with a mixture of water (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (171 mg) as a green oil. The crude product was purified by flash silica gel column (NH₂ silica gel 6 g, hexane/ethyl acetate = 75/25 - 0/100) to give Compound 9C-1 (42.3 mg, yield 56%) as a white amorphous.

Synthesis of Compound 9C from Compound 9-1 was carried out as follows.

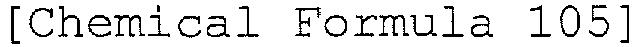

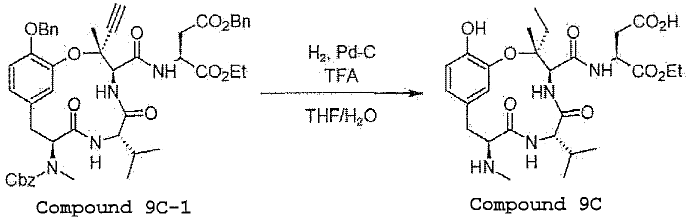

Under nitrogen atmosphere, to a solution of Compound 9C-1 (1.0 eq., 41 mg, 0.047 mmol) in a mixture of THF (1640 µL)/water (82 µL) were added 10% palladium-carbon (N.E. CHEMCAT NX type, 50% hydrous product, 4.1 mg) and TFA (2.0 eq., 7.2 µL, 0.094 mmol) at room temperature, the system was replaced with hydrogen, and the mixture was stirred at the same temperature for 6 hr. The catalyst was removed by filtration through Celite, and the filtrate was concentrated under reduced pressure to give a crude product (35 mg). The crude product was purified by flash silica gel column (reverse-phase silica gel 30 g, 0.05% aqueous TFA solution/acetonitrile = 95/5 - 80/20), and freeze-dried to give a TFA salt of Compound 9C (29.0 mg, yield 91%, purity 96.0%) as a white solid.

¹H-NMR(400MHz,D₂O)δ6.98-6.85(m,3H),4.24-4.17(m,2H),4.10(d,J=9.6Hz,1H),4.05(dd,J=9.2,4.8Hz,1H),3.23(dd,J =13.6,4.8Hz,1H),2.99-2.87(m,3H),2.71(s,3H),2.08-1.88(m,2H)1.81-1.68(m,1H),1.57(s,3H),1.24(t,J=7.2Hz,3H),1.05(t,J=7.2Hz,3H),0.8 5(d,J=6.8Hz,3H),0.78(d,J=6.4Hz,3H).

### (Example 9D: Synthesis of Compound 9D)

Compound 1-7 was synthesized as described in Example 7. Synthesis of Compound 9D-1 from Compound 1-7 was carried out as follows.

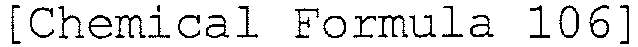

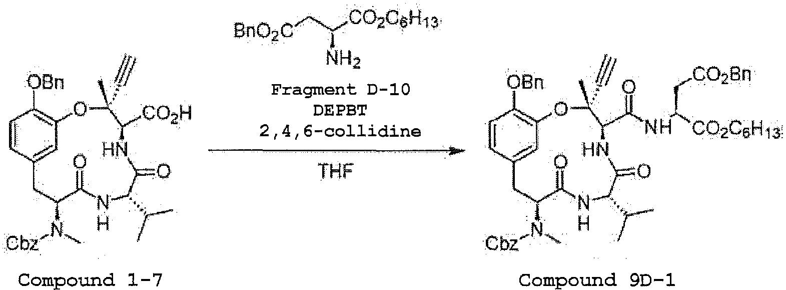

Under nitrogen atmosphere, to a solution of Compound 1-7 (1.0 eq., 69.0 mg, 108 µmol) and Fragment D-10 (1.5 eq., 50.1 mg, 163 µmol) in THF (1 mL) were added DEPBT (1.5 eq., 48.8 mg, 163 µmol) and 2,4,6-collidine (3.0 eq., 38.6 mg, 319 µmol) under ice-cooling, and the mixture was stirred at the same temperature for 6 hr, and then stirred at room temperature for 16 hr. To the reaction solution was added ethyl acetate (10 mL), and the mixture was washed once with a mixture of water (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (152 mg) as a green oil. The crude product was purified by flash silica gel column (NH₂ silica gel 6 g, hexane/ethyl acetate = 75/25 - 0/100) to give Compound 9D-1 (54.0 mg, yield 54%) as a white amorphous.

Synthesis of Compound 9D from Compound 9D-1 was carried out as follows.

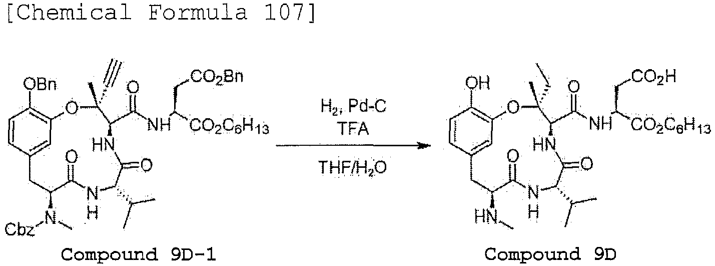

To a solution of Compound 9D-1 (1.0 eq., 38.0 mg, 0.041 mmol) in a mixture of THF (1600 µL)/water (80 µL) were added 10% palladium-carbon (N.E. CHEMCAT NX type, 50% hydrous product, 3.8 mg) and TFA (2.0 eq., 6.3 µL, 0.082 mmol) at room temperature. The system was replaced with hydrogen, the mixture was stirred at the same temperature for 19 hr, and the catalyst was removed by filtration through Celite to give a filtrate. The obtained crude product (32 mg) was purified by flash silica gel column (reverse-phase silica gel 30 g, 0.05% aqueous TFA solution/acetonitrile = 95/5 - 70/30), and freeze-dried to give a TFA salt of Compound 9D (14.9 mg, yield 50%, purity 95.6%) as a white solid.

¹H-NMR(400MHz,D₂O)δ6.96-6.86(m,3H),4.22-4.09(m,3H),4.04(dd,J=9.2,4.8Hz,1H),3.23(dd,J=13.6,4.8Hz,1H),2.9 5-2.88(m,3H),2.71(s,3H),2.08-1.88(m,2H),1.80-1.68(m,1H),1.67-1.53(m,5H),1.38-1.19(m,6H),1.04(t,J=7.2Hz,3H),0.90-0.80(m,6H),0.78(d,J=6.4Hz,3H).

### (Example 9E: Synthesis of Compound 9E)

Synthesis of Compound 9E was carried out.

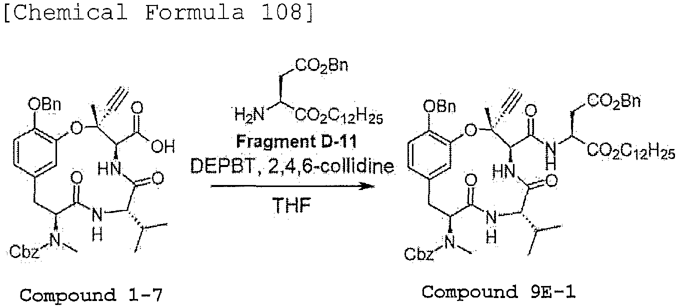

Under nitrogen atmosphere, to a solution of Compound 1-7 (1.0 eq., 90.3 mg, 141 µmol) and Fragment D-11 (1.4 eq, 78.5 mg, 200 µmol) in THF (1.4 mL) were added 2,4,6-collidine (3.0 eq., 55 µL, 418 µmol) and DEPBT (1.5 eq., 62.9 mg, 210 µmol) under ice-cooling, and the mixture was stirred under ice-cooling for 7 hr, and then stirred at room temperature for 16.5 hr. Fragment D-11 (0.5 eq., 27.1 mg, 69 µmol) was added at room temperature, and then DEPBT (0.5 eq., 22.5 mg, 75 µmol) was added under ice-cooling, and the mixture was stirred under ice-cooling for 2 hr, and then stirred at room temperature for 1.5 hr. To the reaction solution was added saturated aqueous ammonium chloride solution (1.5 mL), and the mixture was extracted once with ethyl acetate (8 mL) and once with ethyl acetate (4 mL) by liquid separation. The organic layers were combined, and washed twice with saturated aqueous sodium hydrogencarbonate solution (2 mL), once with water (2 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give a crude product (244 mg) as a deep-green viscous product. The crude product was purified by flash silica gel column (NH₂ silica gel 4.9 g, hexane/ethyl acetate = 3/1 - 1/1) to give Compound 9E-1 (71.3 mg, yield 50%) as a pale-yellow transparent viscous product.

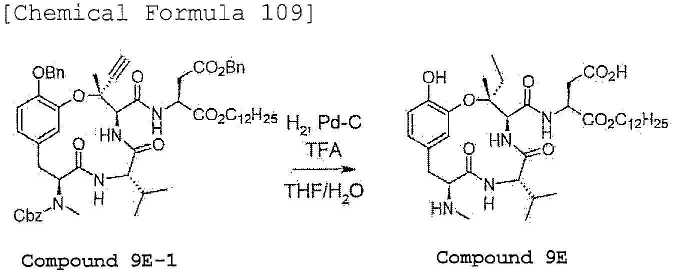

To a solution of Compound 9E-1 (1.0 eq., 53 mg, 0.052 mmol) in a mixture of THF (2200 µL)/water (110 µL) were added 10% palladium-carbon (N.E. CHEMCAT NX type, 50% hydrous product, 5.3 mg) and TFA (2.0 eq., 8.0 µL, 0.104 mmol) at room temperature. The system was replaced with hydrogen, the mixture was stirred at the same temperature for 23 hr, and the catalyst was removed by filtration through Celite to give a filtrate. The filtrate was concentrated under reduced pressure, and the obtained crude product (44 mg) was purified by flash silica gel column (reverse-phase silica gel 30 g, 0.05% aqueous TFA solution/acetonitrile = 95/5 - 50/50), and freeze-dried to give a TFA salt of Compound 9E (36.6 mg, yield 86%, purity 94.3%) as a white solid.

¹H-NMR(400MHz,DMSO-d6)δ9.12(s,1H),8.77(d,J=8.4Hz,1H),8.63(d,J=7.6Hz,1H),7.86(d,J=8 .8Hz,1H),6.80-6.72(m,3H),4.86(d,J=9.6Hz,1H),4.57(dd,J=13.2,7.2Hz,1H),4.23(dd, J=9.2,9.2Hz,1H),4.05-3.95(m,2H),3.89(brs,1H),3.05-2.95(m,1H),2.88(dd,J=14.4,6.0Hz,1H),2.73(dd,J=16.8,6.0Hz,1H),2. 60-2.52(m,1H),2.09-1.93(m,1H),1.87-1.74(m,1H),1.73-1.61(m,1H),1.58-1.46(m,2H),1.44-1.14(m,21H),1.00(t,J=7.2Hz,3H),0.92-0.73(m,9H)

### (Example 9F: Synthesis of Compound 9F)

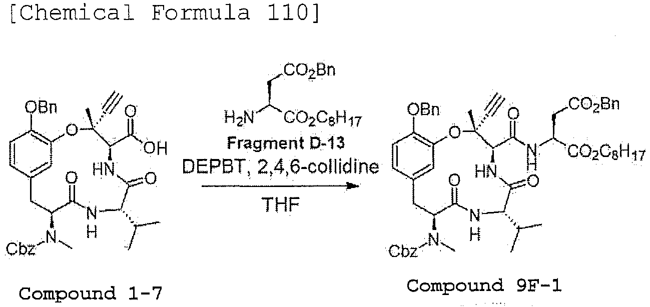

Under nitrogen atmosphere, to a solution of Compound 1-7 (1.0 eq., 93.3 mg, 141 µmol) and Fragment D-13 (1.6 eq, 75.6 mg, 225 µmol) in THF (1.45 mL) were added 2,4,6-collidine (3.0 eq., 57 µL, 433 µmol) and DEPBT (1.5 eq., 65.8 mg, 220 µmol) under ice-cooling, and the mixture was stirred under ice-cooling for 6 hr, and then stirred at room temperature for 16 hr. To the reaction solution was added saturated aqueous ammonium chloride solution (2.0 mL), and the mixture was extracted twice with ethyl acetate (6 mL) by liquid separation. The organic layers were combined, and washed twice with saturated aqueous sodium hydrogencarbonate solution (3 mL), twice with water (3 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give a crude product (238 mg) as a deep-green viscous product. The crude product was purified by flash silica gel column (NH₂ silica gel 5.2 g, hexane/ethyl acetate = 3/1 - 1/1) to give Compound 9F-1 (73.3 mg, yield 53%) as a white amorphous.

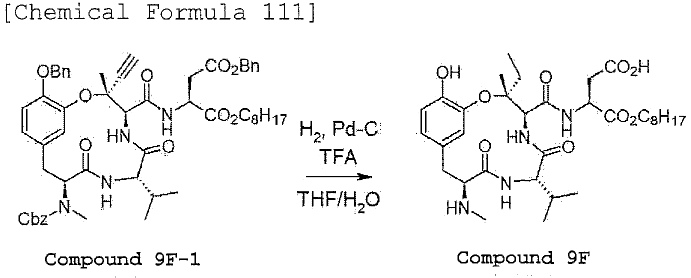

Under nitrogen atmosphere, to a solution of Compound 9F-1 (1.0 eq., 72 mg, 0.075 mmol) in a mixture of THF (2880 µL)/water (144 µL) were added 10% palladium-carbon (N.E. CHEMCAT NX type, 50% hydrous product, 7 mg) and TFA (2.0 eq., 25.5 µL, 0.15 mmol) at room temperature. The system was replaced with hydrogen, and the mixture was stirred at the same temperature for 7 hr. The catalyst was removed by filtration through Celite, and the filtrate was concentrated under reduced pressure to give a crude product (59 mg). The crude product was purified by flash silica gel column (reverse-phase silica gel 30 g, 0.05% aqueous TFA solution/acetonitrile = 95/5 - 55/45), and freeze-dried to give a TFA salt of Compound 9F (31.4 mg, yield 55%, purity 95.4%) as a white solid.

¹H-NMR(400MHz,DMSO-d6)δ9.11(s,1H),8.77(d,J=8.4Hz,1H),8.62(d,J=7.6Hz,1H),7.86(d,J=9 .6Hz,1H),6.80-6.75(m,3H),4.86(d,J=10.4Hz,1H),4.57(dd,J=13.6,7.2Hz,1H),4.23(dd ,J=9.2,9.2Hz,1H),4.07-3.85(m,3H),3.05-2.95(m,1H),2.89(dd,J=14.4,5.6Hz,1H),2.73(dd,J=16.8,6.0Hz,1H),2. 59-2.52(m,1H),2.10-1.93(m,1H),1.86-1.75(m,1H),1.74-1.61(m,1H),1.59-1.46(m,2H),1.37-1.16(m,13H),1.00(t,J=7.2Hz,3H),0.92-0.75(m,9H).

### (Example 9G: Synthesis of Compound 9G)

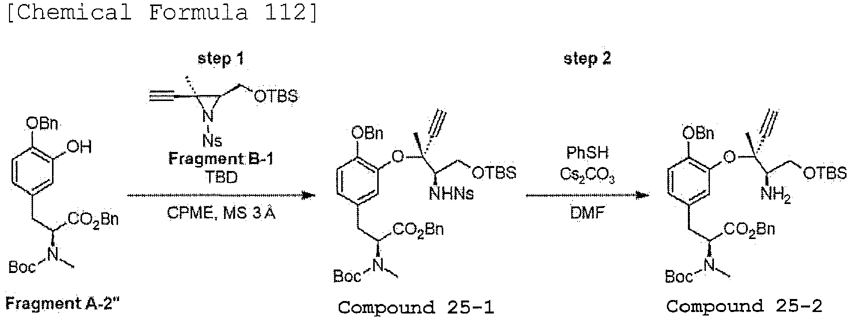

The two steps from Fragment A-2" to Compound 25-2 were as described in the following Example 29B.

Synthesis of Compound 9G-1 from Compound 25-2 was carried out as follows.

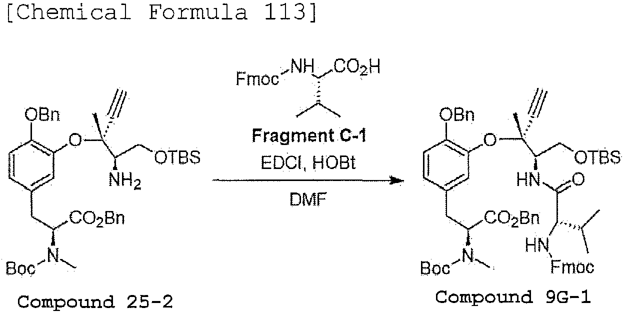

To a solution of Compound 25-2 (1.0 eq., 1.03 g, 1.44 mmol) in DMF (15 mL) were added HOBt·H₂O (1.2 eq., 265 mg, 1.73 mmol), EDCI (1.2 eq., 333 mg, 1.74 mmol) and N-Fmoc-L-valine (Fragment C-1, 1.2 eq., 587 mg, 1.73 mmol) at room temperature, and the mixture was stirred at room temperature for 5 hr. To the reaction solution was added a mixture of hexane (15 mL)/ethyl acetate (45 mL), and the mixture was washed twice with water (40 mL), twice with saturated aqueous sodium hydrogencarbonate solution (40 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give Compound 9G-1 (1.71 g, crude yield 115%) as a pale-brown amorphous.

Synthesis of Compound 9G-2 from Compound 9G-1 was carried out as follows.

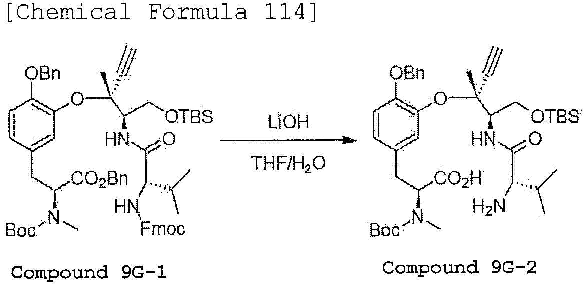

To a solution of Compound 9G-1 (1.0 eq., 1.71 g, calculated as 1.44 mmol) in a mixture of THF (10 mL)/water (5 mL) was added lithium hydroxide (6.0 eq., 207 mg, 8.62 mmol) at room temperature, and the mixture was stirred at room temperature for 4 hr. The reaction solution was adjusted to pH 7 by addition of 1N aqueous hydrochloric acid solution (5.0 mL). To the reaction solution was added water (10 mL), and the mixture was extracted three times with ethyl acetate (20 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (1.84 g) as a brown viscous product. The crude product was purified by flash silica gel column (normal phase silica gel 20 g, ethyl acetate/methanol = 100/0 - 40/60) to give Compound 9G-2 (1.06 g, two-step yield 102%) as a pale-brown amorphous.

Synthesis of Compound 9G-3 from Compound 9G-2 was carried out as follows.

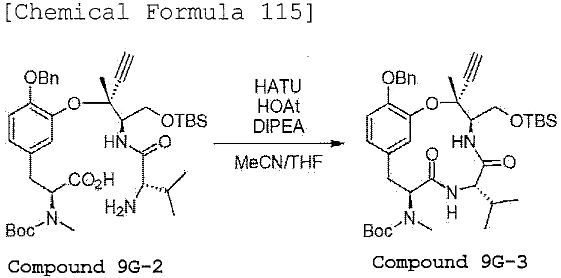

A solution of Compound 9G-2 (1.0 eq., 1.05 mg, calculated as 1.44 mmol) in a mixture of acetonitrile (14.5 mL)/THF (14.5 mL) was added dropwise to a solution of HATU (2.0 eq., 1.10 g, 2.88 mmol), HOAt (2.0 eq., 391 mg, 2.87 mmol) and DIPEA (2.0 eq., 0.49 mL, 2.89 mmol) in acetonitrile (718 mL) over 5 hr at about 100 µL/min at room temperature, and the mixture was stirred at room temperature for 15.5 hr. The reaction solution was concentrated under reduced pressure until the volume became about 250 mL, ethyl acetate (200 mL) was added, and the mixture was washed twice with a mixture of water (50 mL)/saturated aqueous ammonium chloride solution (100 mL), twice with saturated aqueous sodium hydrogencarbonate solution (100 mL) and once with saturated aqueous sodium chloride solution (50 mL) by liquid separation. The organic layer was dried over magnesium sulfate, the magnesium sulfate was removed by filtration, and the filtrate was concentrated under reduced pressure to give a crude product (1.92 g) as a red-brown viscous product. The crude product was purified by flash silica gel column (normal phase silica gel 25 g, hexane/ethyl acetate = 80/20 - 50/50) to give Compound 9G-3 (231 mg, three-step yield 23%) as a white amorphous.

Synthesis of Compound 9G-4 from Compound 9G-3 was carried out as follows.

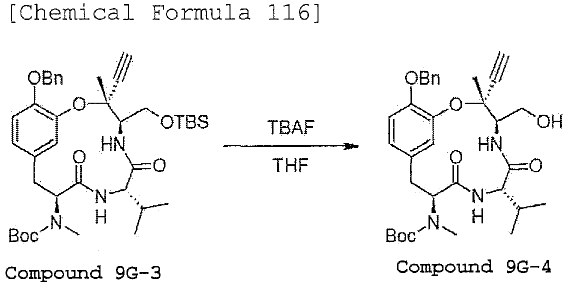

To a solution of Compound 9G-3 (1.0 eq., 230 mg, 0.324 mmol) in THF (3.2 mL) was added TBAF (1M in THF, 2.5 eq., 0.81 mL, 0.810 mmol) at room temperature, and the mixture was stirred at the same temperature for 2 hr. To the reaction solution was added ethyl acetate (30 mL), and the mixture was washed three time with saturated aqueous ammonium chloride solution (20 mL) and once with saturated aqueous sodium chloride solution (10 mL) by liquid separation, and dried over magnesium sulfate. The magnesium sulfate was removed by filtration, and the filtrate was concentrated under reduced pressure to give a crude product (214 mg) as a pale-yellow amorphous. The crude product was purified by flash silica gel column (normal phase silica gel 5 g, hexane/ethyl acetate = 50/50 - 0/100) to give Compound 9G-4 (191 mg, yield 99%) as a white amorphous.

Synthesis of Compound 9G-5 from Compound 9G-4 was carried out as follows.

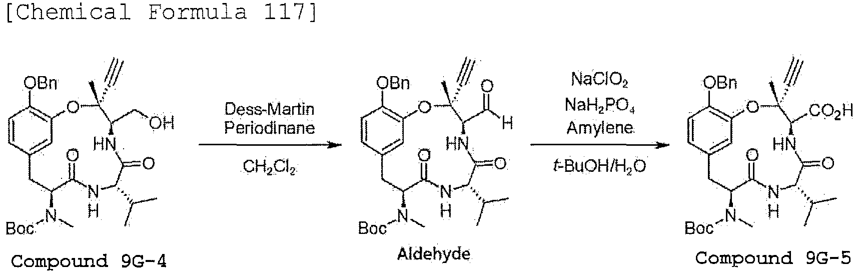

Under nitrogen atmosphere, to a solution of Compound 9G-4 (1.0 eq., 189 mg, 0.318 mmol) in dichloromethane (3 mL) was added Dess-Martin periodinane (1.5 eq., 203 mg, 0.477 mmol) at room temperature, and the mixture was stirred at the same temperature for 1 hr. The reaction solution was quenched by addition of a mixture of 10% aqueous sodium sulfite solution (10 mL)/saturated aqueous sodium hydrogencarbonate solution (10 mL), and extracted once with dichloromethane (20 mL) and twice with dichloromethane (10 mL) by liquid separation. The organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (187 mg, quant.) as a white amorphous.

To a solution of Aldehyde (1.0 eq., 186 mg, 0.314 mmol) in a mixture of amylene (1 mL)/t-butyl alcohol (4 mL) was added a solution of sodium dihydrogen phosphate dihydrate (3.4 eq., 167 mg, 1.07 mmol) and 80% sodium chlorite (4.5 eq., 160 mg, 1.42 mmol) in water (1 mL) at room temperature, and the mixture was stirred at the same temperature for 1 hr. To the reaction solution was added saturated aqueous ammonium chloride solution (10 mL), and the mixture was extracted once with ethyl acetate (20 mL) and twice with ethyl acetate (10 mL) by liquid separation. The organic layers were combined, washed once with saturated aqueous sodium chloride solution (20 mL) by liquid separation, and dried over magnesium sulfate. The magnesium sulfate was removed by filtration, and the filtrate was concentrated under reduced pressure to give a crude product (215 mg, yield 99%) as a white amorphous. The crude product was purified by flash silica gel column (normal phase silica gel 5 g, hexane/ethyl acetate = 33/67) to give Compound 9G-5 (178 mg, yield 93%) as a white amorphous.

Synthesis of Compound 9G-6 from Compound 9G-5 was carried out as follows.

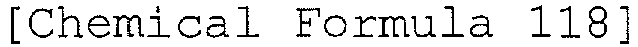

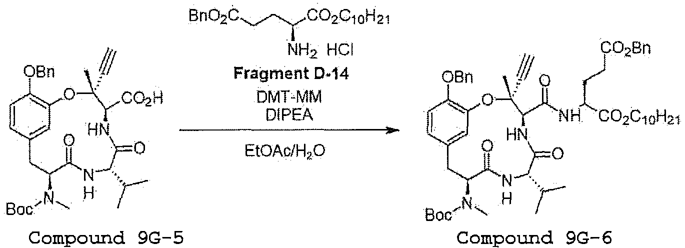

To a solution of Compound 9G-5 (1.0 eq., 177 mg, 0.291 mmol) in a mixture of ethyl acetate (1.8 mL)/water (540 µL) was added Fragment D-14 (1.5 eq., 164 mg, 0.435 mmol) at room temperature. DIPEA (2.4 eq., 0.12 mL, 0.706 mmol) was added under ice-cooling, the mixture was stirred for 10 min, DMT-MM (1.7 eq., 138 mg, 0.497 mmol) was added, and the mixture was stirred at the same temperature for 2.5 hr. To the reaction solution was added ethyl acetate (30 mL), and the mixture was washed once with water (15 mL), once with a mixture of water (10 mL)/saturated aqueous ammonium chloride solution (10 mL), once with water (10 mL)/saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (360 mg) as a yellow oil. The crude product was purified twice by flash silica gel column (1st run: NH₂ silica gel 6 g, hexane/ethyl acetate = 75/25 - 67/33, 2nd run: normal phase silica gel 4 g, hexane/ethyl acetate = 50/50) to give Compound 9G-6 (235 mg, yield 83%) as a white amorphous.

Synthesis of Compound 9G-7 from Compound 9G-6 was carried out as follows.

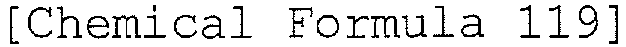

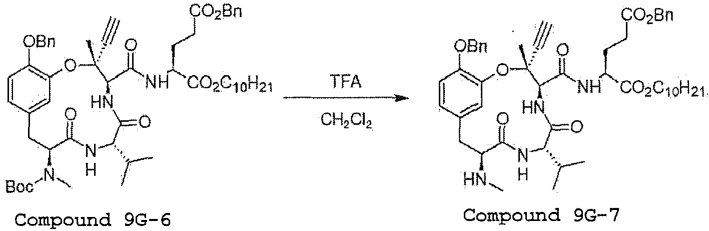

To a solution of Compound 9G-6 (1.0 eq., 235 mg, 0.243 mmol) in dichloromethane (2.4 mL) was added TFA (9.7 eq., 0.18 mL, 2.35 mmol) at room temperature, and the mixture was stirred at the same temperature for 1 hr. Then, additional TFA (9.7 eq., 0.18 mL, 2.35 mmol) was added, and the mixture was stirred at the same temperature for 7 hr. The reaction solution was ice-cooled, and adjusted to pH 8 by addition of saturated aqueous sodium hydrogencarbonate solution (15 mL). The reaction solution was extracted once with ethyl acetate (20 mL) and twice with ethyl acetate (10 mL) by liquid separation, and the organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give Compound 9G-7 (210 mg, yield 99%) as a pale-yellow amorphous.

Synthesis of Compound 9G from Compound 9G-7 was carried out as follows.

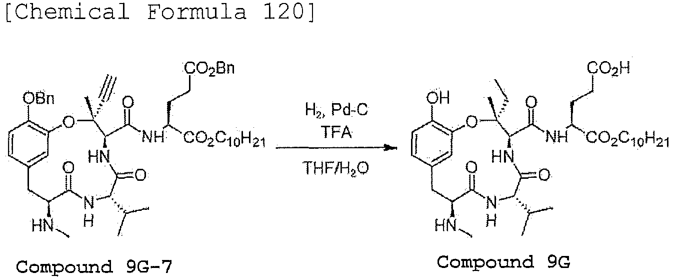

Under nitrogen atmosphere, to a solution of Compound 9G-7 (1.0 eq., 186 mg, 0.218 mmol) in a mixture of THF (7.4 mL)/water (370 µL) was added TFA (5.0 eq., 83 µL, 1.085 mmol) at room temperature, and the mixture was stirred at the same temperature for 1 hr. 10% Palladium-carbon (N.E. CHEMCAT NX type, 50% hydrous product, 18.6 mg) was added, the system was replaced with hydrogen, and the mixture was stirred at the same temperature for 6.5 hr. The catalyst was removed by filtration through Celite, and the filtrate was concentrated under reduced pressure to give a crude product (171 mg). The crude product (40 mg) obtained from Compound 9G-7 (40 mg) by the same procedure and the aforementioned crude product were combined, purified by flash silica gel column (reverse-phase silica gel 30 g, 0.05% aqueous TFA solution/acetonitrile = 95/5 - 55/45), and freeze-dried to give a TFA salt of Compound 9G (107 mg, yield 51%, purity 96.4%) as a white solid.

¹H-NMR(400MHz,DMSO-d6)δ9.11(s,1H),8.79(d,J=8.8Hz,1H),8.62(d,J=7.6Hz,1H),7.87(d,J = 100Hz,1H),6.80-6.75(m,3H),4.87(d,J = 100Hz,1H),4.57(dd,J=13.6,7.2Hz,1H),4.23(dd,J=9.2,9.2Hz,1H),4.05 -3.85(m,3H),3.05-2.95(m,1H),2.89(dd,J=14.4,5.6Hz,1H),2.73(dd,J=16.8,6.0Hz,1H),2. 60-2.50(m,1H),2.10-1.94(m,1H),1.87-1.61(m,2H),1.59-1.45(m,2H),1.36-1.15(m,17H),1.01(t,J=7.2Hz,3H),0.92-0.74(m,9H).

### (Example 10: Synthesis of Compound 10)

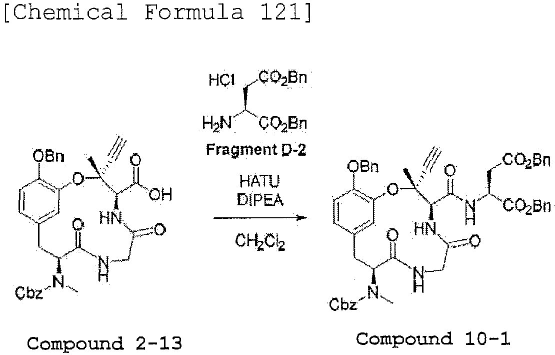

Under nitrogen atmosphere, to a solution of Compound 2-13 (1.0 eq., 95 mg, containing an isomer, 0.158 mmol) and dibenzyl L-aspartate hydrochloride (Fragment D-2, 1.5 eq., 83 mg, 0.237 mmol) in dichloromethane (2.4 mL) were added DIPEA (1.5 eq., 40 µL, 0.235 mmol) and HATU (1.5 eq., 90 mg, 0.237 mmol) under ice-cooling, and the mixture was stirred for 3 hr while warming to room temperature. To the reaction solution was added 5% aqueous citric acid solution (3 mL), and the mixture was washed once by liquid separation, and the organic layer was washed once with 5% aqueous sodium hydrogencarbonate solution (3 mL) and once with 5% aqueous sodium chloride solution (3 mL) by liquid separation, and dried over sodium sulfate. The sodium sulfate was removed by filtration, the filtrate was concentrated under reduced pressure, and the obtained crude product (197 mg) as a pale-brown viscous product was purified twice by flash silica gel column (1st run: normal phase silica gel 10 g, hexane/ethyl acetate = 40/60, 2nd run: NH₂ silica gel 6.5 g, hexane/ethyl acetate = 40/60 - 0/100) to give Compound 10-1 (54 mg, containing 5.0wt% hexane, conversion yield 36%) as a white solid.

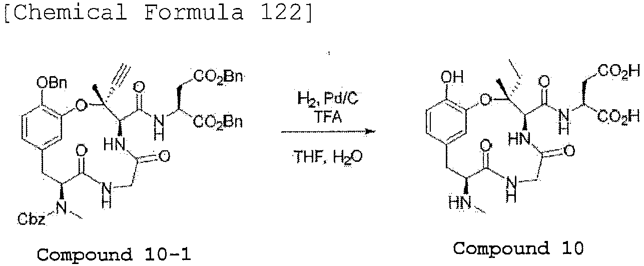

Under nitrogen atmosphere, to a solution of Compound 10-1 (1.0 eq., 52 mg, containing 5.0wt% hexane, 0.055 mmol) in a mixture of THF (2.5 mL)/water (2.5 mL) were added 10% palladium-carbon (N.E. CHEMCAT NX type, 50% hydrous product, 2.6 mg) and TFA (2.0 eq., 8.4 µL, 0.110 mmol) at room temperature, the system was replaced with hydrogen, and the mixture was stirred at the same temperature for 25 hr. Additional THF (2.5 mL) was added, and the mixture was stirred for additional 64 hr. Additional 10% palladium-carbon (N.E. CHEMCAT NX type, 50% hydrous product, 2.6 mg) was added, and the mixture was stirred for 24 hr. The catalyst was removed by filtration through Celite, and the filtrate was concentrated under reduced pressure to give a crude product (41 mg) as a pale-orange viscous product. The crude product was purified by flash silica gel column (reverse-phase silica gel 30 g, 0.05% aqueous TFA solution/acetonitrile = 95/5), and freeze-dried to give a TFA salt of Compound 10 (18.3 mg, yield 54%, purity 99.3%) as a white solid.

¹H-NMR(400MHz,D₂O)δ7.44(s,1H),7.05(dd,J=8.0,2.0Hz,1H),6.98(d,J=8.0 Hz,1H),4.65(s,1H),4.25(d,J=16.4Hz,1H),3.99(dd,J=10.4,4.0Hz,1H), 3.55(d,J=16.4Hz,1H),3.26(dd,J=13.2,4.0Hz,1H),3.08-2.99(m,1H),2.73(s,3H),2.03-1.97(m,1H),1.78-1.70(m,1H),1.63(s,3H),0.86(t,J=7.2Hz,3H).

### (Example 11: Synthesis of Compound 5)

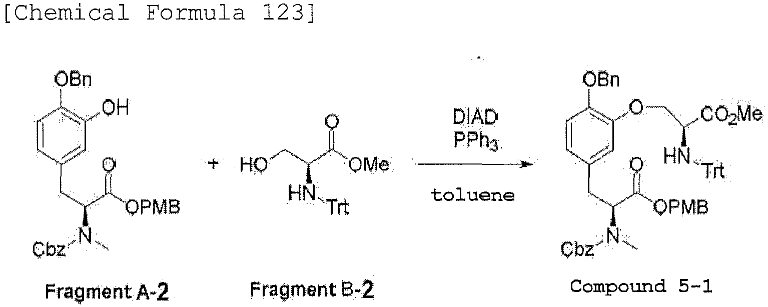

Under nitrogen atmosphere, to a solution of Fragment A-2 (1.0 eq., 2.81 g, 5.06 mmol) and Fragment B-2 (1.5 eq., 2.74 g, 7.58 mmol) in toluene (28 mL) were added triphenylphosphine (1.5 eq., 1.99 g, 7.59 mmol) and DIAD (1.5 eq., 1.63 mL, 7.58 mmol) at room temperature, and the mixture was stirred at the same temperature for 15 min, and then at the external temperature of 75°C for 2 hr. Triphenylphosphine (0.75 eq., 0.99 g, 3.77 mmol) and DIAD (1.5 eq., 0.81 mL, 3.77 mmol) were added, the mixture was stirred for additional 0.5 hr, and the reaction solution was allowed to cool, and concentrated under reduced pressure. The concentrated residue was purified by flash silica gel column (normal phase silica gel 50 g, hexane/ethyl acetate = 90/10 - 67/33) to give Compound 5-1 (5.95 g, containing impurities, apparent yield 130%) as a pale-yellow-brown oil.

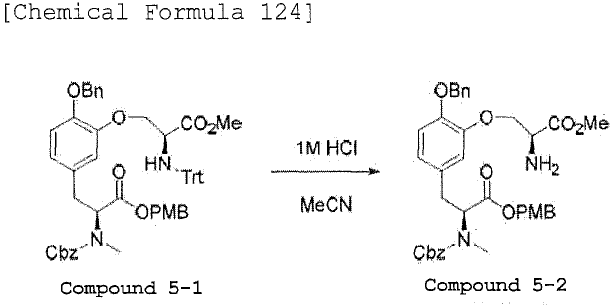

Under nitrogen atmosphere, to a solution of Compound 5-1 (1.0 eq., 5.35 g, containing impurities, calculated as 4.55 mmol) in acetonitrile (45 mL) was added 1M aqueous hydrochloric acid solution (1.1 eq., 5.00 mL, 5.00 mmol) under ice-cooling, and the mixture was stirred for 2 hr while warming to room temperature. The reaction solution was adjusted to pH 5-7 by addition of a few drops of saturated aqueous sodium hydrogencarbonate solution, and concentrated under reduced pressure. To the concentrated residue was added saturated aqueous sodium hydrogencarbonate solution (20 mL), and the mixture was extracted three times with ethyl acetate (25 mL) by liquid separation. The organic layers were combined, washed once with saturated aqueous sodium chloride solution (20 mL) by liquid separation, and dried over sodium sulfate. The sodium sulfate was removed by filtration, and the filtrate was concentrated under reduced pressure to give a crude product (5.42 g) as a pale-yellow-brown oil. The crude product was purified by flash silica gel column (normal phase silica gel 50 g, chloroform/acetonitrile = 90/10 - 80/20) to give Compound 5-2 (1.59 g, containing impurities, apparent yield 75%) as a pale-yellow oil.

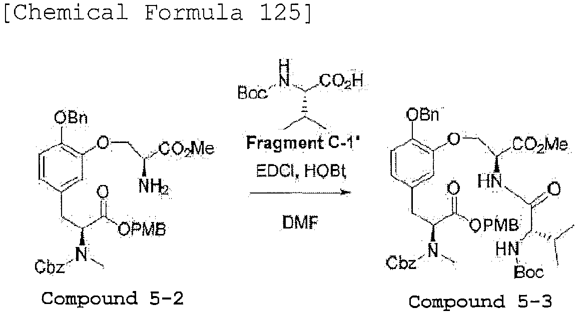

Under nitrogen atmosphere, to a solution of Compound 5-2 (1.0 eq., 0.80 g, containing impurities, calculated as 1.22 mmol) in DMF (12 mL) were added N-Boc-L-valine (Fragment C-1, 2.0 eq., 528 mg, 2.43 mmol), EDCI (2.0 eq., 466 mg, 2.43 mmol) and HOBt·H₂O (2.0 eq., 372 mg, 2.43 mmol), and the mixture was stirred at room temperature for 20 hr. To the reaction solution was added water (50 mL), and the mixture was extracted three times with ethyl acetate (25 mL) by liquid separation. The organic layers were combined, washed once with saturated aqueous sodium hydrogencarbonate solution (25 mL) and once with saturated aqueous sodium chloride solution (25 mL) by liquid separation, and dried over sodium sulfate. The sodium sulfate was removed by filtration, and the filtrate was concentrated under reduced pressure to give a crude product (1.50 g) as a pale-yellow oil. The crude product was purified twice by flash silica gel column (1st run: normal phase silica gel 50 g, hexane/ethyl acetate = 90/10 - 50/50, 2nd run: reverse-phase silica gel 120 g, 0.05% aqueous TFA solution/acetonitrile = 90/10 - 0/100) to give Compound 5-3 (0.74 g, three-step yield from Fragment A-2 38%) as a white amorphous.

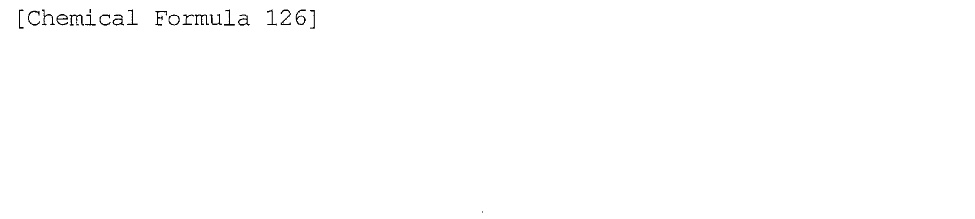

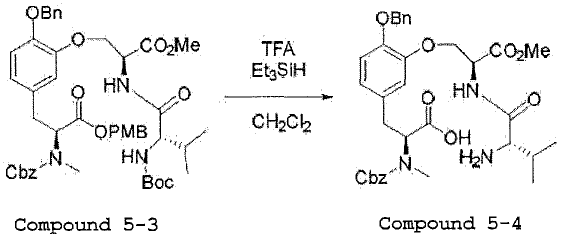

Under nitrogen atmosphere, to a solution of Compound 5-3 (1.0 eq., 0.74 g, 0.87 mmol) in dichloromethane (7.5 mL) were added triethylsilane (25 eq., 3.35 mL, 21.03 mmol) and TFA (3.75 mL) under ice-cooling, and the mixture was stirred at room temperature for 4 hr. The reaction solution was concentrated under reduced pressure. To the concentrated residue was added 0.05M hydrogen chloride/1,4-dioxane solution (20 mL), and the mixture was concentrated under reduced pressure. These procedures were repeated twice. To the concentrated residue was added 1,4-dioxane, and the mixture was concentrated under reduced pressure. These procedures were repeated three times to give Compound 5-4 (1.39 g) as a colorless oil.

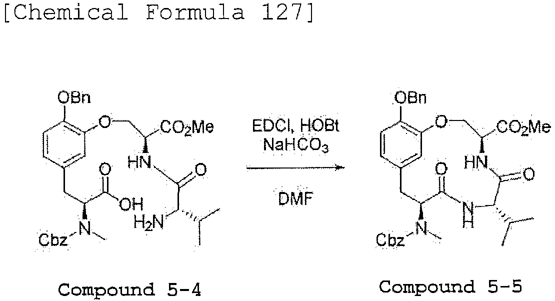

Under nitrogen atmosphere, to a solution of EDCI (5.0 eq., 830 mg, 4.33 mmol), HOBt·H₂O (5.0 eq., 663 mg, 7.33 mmol) and sodium hydrogencarbonate (10.0 eq., 726 mg, 8.64 mmol) in DMF (800 mL) was added a solution of Compound 5-4 (1.0 eq., 1.39 g, calculated as 0.865 mmol) in DMF (65 mL) over 2 hr at room temperature, and after the dropwise addition was completed, the mixture was stirred overnight at room temperature. The reaction solution was concentrated under reduced pressure, to the concentrated residue were added ethyl acetate (20 mL) and water (20 mL), and the mixture was extracted once by liquid separation. The organic layer was washed once with saturated aqueous sodium chloride solution (10 mL) by liquid separation, and dried over sodium sulfate. The sodium sulfate was removed by filtration, and the filtrate was concentrated under reduced pressure to give a crude product (0.71 g) as an ocher solid. The crude product was purified by flash silica gel column (normal phase silica gel 25 g, chloroform/ethyl acetate = 90/10 - 80/20) to give Compound 5-5 (226 mg, containing impurities, two-step apparent yield from Compound 5-3 42%) as a white solid.

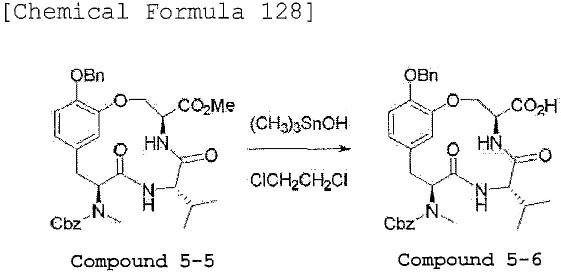

Under nitrogen atmosphere, to a solution of Compound 5-5 (1.0 eq., 138 mg, containing impurities, calculated as 0.22 mmol) in 1,2-dichloroethane (3.45 mL) was added tributyltin hydroxide (4.0 eq., 159 mg, 0.88 mmol), and the mixture was stirred at the external temperature of 80°C for 4 hr. The reaction solution was allowed to cool, ethyl acetate (20 mL) and 1M aqueous hydrochloric acid solution (20 mL) were added, and the mixture was extracted once by liquid separation. The organic layer was washed once with 10% aqueous sodium chloride solution (20 mL) by liquid separation, and dried over sodium sulfate, and the sodium sulfate was removed by filtration. The sodium sulfate was removed by filtration, and the filtrate was concentrated under reduced pressure to give a crude product (169 mg) as a white solid.

The crude product (20 mg) was obtained from Compound 5-5 (20 mg) by the same procedure. It was combined with the previous crude product, and purified by flash silica gel column (normal phase silica gel 10 g, ethyl acetate/methanol = 100/0 - 90/10) to give Compound 5-6 (147 mg, containing impurities, apparent yield 93%).

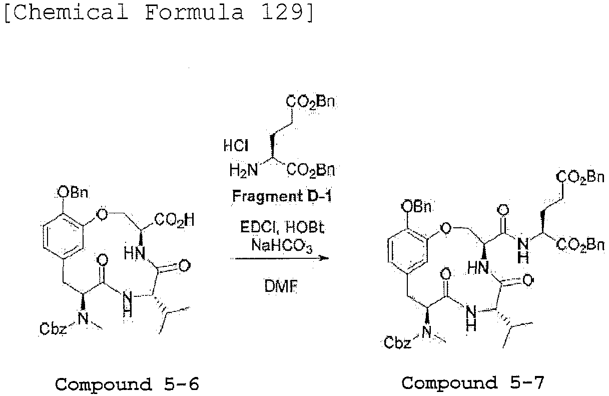

Under nitrogen atmosphere, to a solution of Compound 5-6 (1.0 eq., 73 mg, containing impurities, 0.12 mmol) and dibenzyl L-glutamate hydrochloride (Fragment D-1, 1.5 eq., 65 mg, 0.18 mmol) in DMF (0.73 mL) were added EDCI (1.5 eq., 34 mg, 0.18 mmol), HOBt·H₂O (1.5 eq., 27 mg, 0.18 mmol) and sodium hydrogencarbonate (3.0 eq., 30 mg, 0.36 mmol) at room temperature, and the mixture was stirred at the same temperature for 1.5 hr.

To the reaction solution was added toluene (3 mL), and the mixture was washed twice with 5% aqueous sodium hydrogencarbonate solution (3 mL) and once with 5% aqueous sodium chloride solution (3 mL) by liquid separation. The organic layer was dried over sodium sulfate, the sodium sulfate was removed by filtration, and the filtrate was concentrated under reduced pressure to give a crude product (115 mg) as a pale-yellow solid. The crude product was purified by flash silica gel column (NH₂ silica gel 6.5 g, hexane/ethyl acetate = 90/10 - 50/50) to give a white solid (52 mg). To this solid were added hexane (4 mL) and toluene (2 mL), and the mixture was subjected to ultrasonic irradiation, and stirred at room temperature for 15 min. This suspension was filtered to give Compound 5-7 (37 mg, yield 34%) as a white solid.

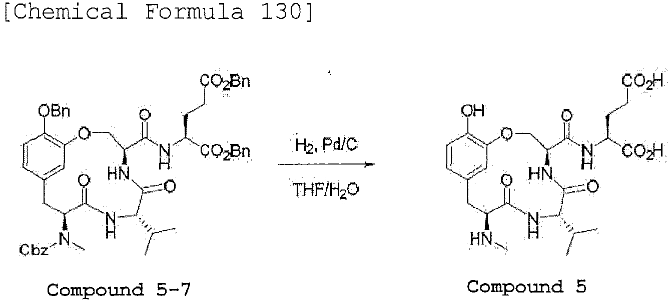

Under nitrogen atmosphere, to a solution of Compound 5-7 (1.0 eq., 35 mg, 0.038 mmol) in a mixture of THF (1.2 mL)/water (1.2 mL) was added 10% palladium-carbon (N.E. CHEMCAT NX type, 50% hydrous product, 8.8 mg) at room temperature, the system was replaced with hydrogen, and the mixture was stirred at the same temperature for 22 hr. The catalyst was removed by filtration through Celite, the solid was washed with 0.05% aqueous TFA solution, and the filtrate was concentrated under reduced pressure to give a crude product (20 mg) as a white solid. The crude product was purified twice by flash silica gel column (reverse-phase silica gel 60 g, 1st run: 0.05% aqueous TFA solution/acetonitrile = 99/1 - 90/10, 2nd run: 0.05% aqueous TFA solution/acetonitrile = 99/1 - 0/100), and freeze-dried to give a TFA salt of Compound 5 (14 mg, yield 59%, purity 99.7%) as a white solid.

¹H-NMR(400MHz,D₂O)δ6.89(d,J=8.0Hz,1H),6.81(dd,J=8.0,2.0Hz,1H),6.70 (d,J=2.0Hz,1H),4.65(dd,J=13.2,2.4Hz,1H),4.56(dd,J=13.2,6.0Hz,1H ),4.37(dd,J=8.8,4.8Hz,1H),4.11(d,J=10.8Hz,1H),3.95(dd,J=8.8,4.8 Hz,1H),3.21(dd,J=13.2,4.8Hz,1H),2.95(dd,J=13.2,8.8Hz,1H),2.70(s ,3H),2.45(t,J=7.2Hz,2H),2.24-2.15(m,1H),2.03-1.85(m,2H),0.84(t,J=6.8Hz,6H).

### (Example 12: Synthesis of Compound 3)

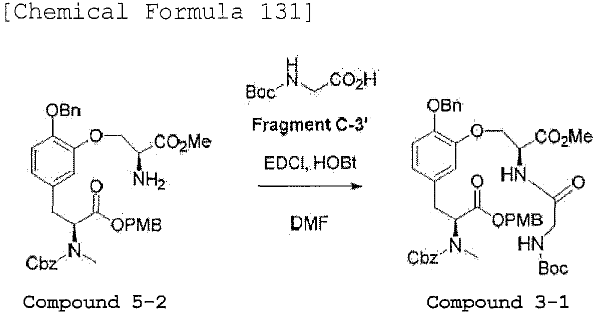

Under nitrogen atmosphere, to a solution of Compound 5-2 (1.0 eq., 0.79 g, containing impurities, calculated as 1.20 mmol) in DMF (12 mL) were added N-Boc-L-glycine (Fragment C-3, 2.0 eq., 0.42 g, 2.40 mmol), EDCI (2.0 eq., 0.46 mg, 2.40 mmol) and HOBt·H₂O (2.0 eq., 0.37 mg, 2.40 mmol), and the mixture was stirred at room temperature for 2 hr. To the reaction solution were added water (30 mL) and toluene (30 mL), and the mixture was extracted once by liquid separation. The organic layer was washed once with water (30 mL) and once with 10% aqueous sodium chloride solution (30 mL) by liquid separation, and dried over sodium sulfate. The sodium sulfate was removed by filtration, and the filtrate was concentrated under reduced pressure to give a crude product (1.175 g) as a yellow viscous product. The crude product was purified by flash silica gel column (NH₂ silica gel 14 g, hexane/ethyl acetate = 90/10 - 0/100) to give Compound 3-1 (0.87 g, containing 3.2wt% ethyl acetate, conversion yield 86%) as a pale-yellow viscous product.

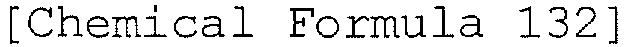

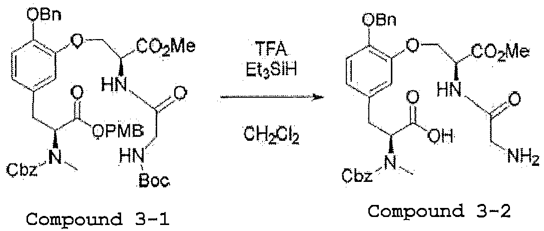

Under nitrogen atmosphere, to a solution of Compound 3-1 (1.0 eq., 0.87 g, containing 3.2wt% ethyl acetate, 1.03 mmol) in dichloromethane (8.7 mL) were added triethylsilane (25 eq., 3.01 g, 25.89 mmol) and TFA (4.35 mL) under ice-cooling, and the mixture was stirred at the same temperature for 2 hr. The reaction solution was concentrated under reduced pressure, to the concentrated residue was added 0.05M hydrogen chloride/1,4-dioxane solution (15 mL), and the mixture was concentrated again under reduced pressure to give Compound 3-2 (1.196 g, containing 1,4-dioxane) as a colorless viscous product.

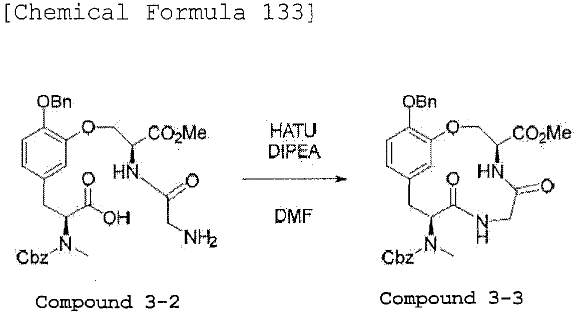

Under nitrogen atmosphere, to a solution of HATU (5.0 eq., 1.36 g, 3.58 mmol) and DIPEA (10.0 eq., 0.92 g, 7.12 mmol) in DMF (704 mL) was added a solution of Compound 3-2 (1.0 eq., 0.83 g, containing 1,4-dioxane, calculated as 0.72 mmol) in DMF (8 mL) over 18.5 hr at room temperature, and after the dropwise addition was completed, the mixture was stirred at room temperature for 3 hr. The reaction solution was concentrated under reduced pressure, to the concentrated residue were added ethyl acetate (15 mL) and 5% aqueous citric acid solution (15 mL), and the mixture was extracted once by liquid separation. The organic layer was washed once with 5% aqueous sodium hydrogencarbonate solution (15 mL) and once with 5% aqueous sodium chloride solution (15 mL) by liquid separation, and dried over sodium sulfate. The sodium sulfate was removed by filtration, and the filtrate was concentrated under reduced pressure to give a crude product (0.81 g) as a pale-brown amorphous. The crude product was purified by flash silica gel column (NH₂ silica gel 10 g, hexane/ethyl acetate = 50/50) to give Compound 3-3 (190 mg, yield 46%) as a white solid. This and Compound 3-3 (89 mg) obtained by the same procedure were combined, and purified by flash silica gel column (NH₂ silica gel 14 g, hexane/ethyl acetate = 50/50), and the fractions containing the target product were combined, and concentrated under reduced pressure. The concentrated residue was dissolved in 1,2-dichloroethane (3 mL), and the solution was concentrated under reduced pressure. These procedures were repeated twice to give Compound 3-3 (118 mg, containing 16.5wt% 1,2-dichloroethane, yield 35%) as a colorless viscous product.

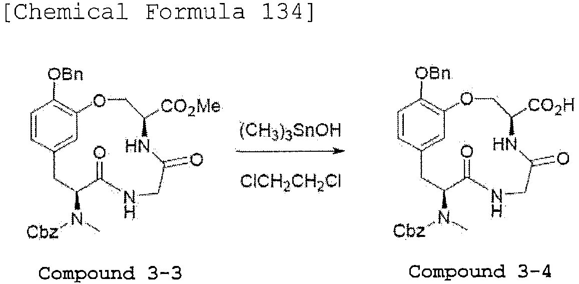

Under nitrogen atmosphere, to a solution of Compound 3-3 (1.0 eq., 118 mg, containing 16.5wt% 1,2-dichloroethane, 0.17 mmol) in 1,2-dichloroethane (2.7 mL) was added tributyltin hydroxide (4.0 eq., 124 mg, 0.69 mmol), and the mixture was stirred at the external temperature of 80°C for 3.5 hr. The reaction solution was allowed to cool, ethyl acetate (20 mL) and 1M aqueous hydrochloric acid solution (20 mL) were added, and the mixture was extracted once by liquid separation. The organic layer was washed once with 5% aqueous sodium chloride solution (20 mL) by liquid separation, and dried over sodium sulfate, and the sodium sulfate was removed by filtration. The sodium sulfate was removed by filtration, and the filtrate was concentrated under reduced pressure to give Compound 3-4 (104 mg, containing impurities, quant.) as a white solid.

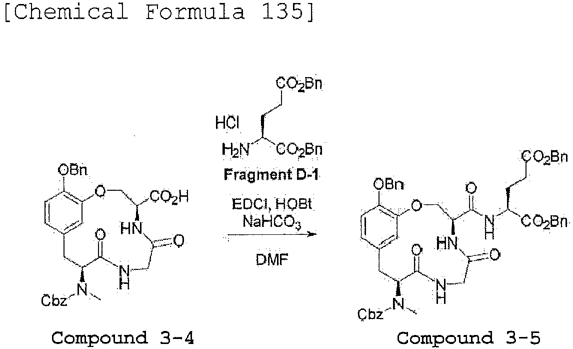

Under nitrogen atmosphere, to a solution of Compound 3-4 (1.0 eq., 50 mg, containing impurities, calculated as 0.082 mmol) and dibenzyl L-glutamate hydrochloride (Fragment D-1, 1.5 eq., 45 mg, 0.124 mmol) in DMF (0.5 mL) were added EDCI (1.5 eq., 24 mg, 0.125 mmol), HOBt·H₂O (1.5 eq., 19 mg, 0.124 mmol) and sodium hydrogencarbonate (1.5 eq., 10 mg, 0.119 mmol) at room temperature, and the mixture was stirred at the same temperature for 1.5 hr. To the reaction solution were added toluene (10 mL) and 5% aqueous citric acid solution (10 mL), and the mixture was extracted once by liquid separation, and the organic layer was washed once with 5% aqueous sodium hydrogencarbonate solution (10 mL) and once with 5% aqueous sodium chloride solution (10 mL) by liquid separation. The organic layer was dried over sodium sulfate, the sodium sulfate was removed by filtration, and the filtrate was concentrated under reduced pressure to give a crude product (82 mg) as a pale-yellow viscous product.

The crude product (60 mg) was obtained from Compound 3-4 (1.0 eq., 50 mg) by the same procedure. This was combined with the previous crude product, purified by flash silica gel column (NH₂ silica gel 6.5 g, hexane/ethyl acetate = 40/60) to give Compound 3-5 (69 mg, yield 48%) as a white amorphous.

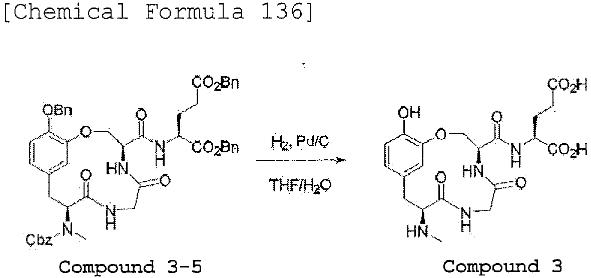

Under nitrogen atmosphere, to a solution of Compound 3-5 (1.0 eq., 67 mg, 0.077 mmol) in a mixture of THF (3.35 mL)/water (3.35 mL) was added at room temperature 10% palladium-carbon (N.E. CHEMCAT NX type, 50% hydrous product, 17 mg), the system was replaced with hydrogen, and the mixture was stirred at the same temperature for 19.5 hr. To the reaction solution was added TFA (2.0 eq., 12 µL, 0.157 mmol), the catalyst was removed by filtration through Celite, and the filtrate was concentrated under reduced pressure to give a crude product (45 mg). The crude product was purified by flash silica gel column (reverse-phase silica gel 60 g, 0.05% aqueous TFA solution/acetonitrile = 99/1 - 0/100), and freeze-dried to give a TFA salt of Compound 3 (34 mg, yield 76%, purity 99.9%) as a white solid.

¹H-NMR(400MHz,D₂O)δ7.00-6.85(m,3H),4.67(dd,J=13.6,5.6Hz,1H),4.53(dd,J=13.2,1.6Hz, 1H), 4. 42(dd,J=8.8,5.2Hz,1H),4.36(d,J=15.2Hz,1H),3.96(dd,J = 100,4.8Hz,1H),3.36-3.29(m,2H),2.98(dd,J=13.2,10.0Hz,1H),2.73(s,3H),2.50(t,J=7.2Hz, 2H),2.28-2.19(m,1H),2.08-1.90(m,1H).

### (Example 13: Synthesis of Compound 11)

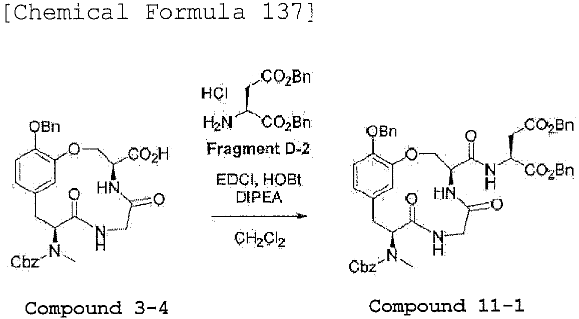

Under nitrogen atmosphere, to a solution of Compound 3-4 (1.0 eq., 366.6 mg, calculated as 0.408 mmol) in dichloromethane (3.7 mL) was added a solution of dibenzyl L-aspartate hydrochloride (Fragment D-2, 2.0 eq., 286 mg, 0.816 mmol) and DIPEA (1.9 eq., 135 µL, 0.775 mmol) in dichloromethane (2.5 mL) under ice-cooling. Then, HOBt·H₂O (2.0 eq., 125 mg, 0.816 mmol) and EDCI (2.0 eq., 156 mg, 0.816 mmol) were added, and the mixture was stirred at the same temperature for 3 hr.

The reaction solution obtained from Compound 3-4 (30 mg) under similar conditions and the previous reaction solution were combined, ethyl acetate (40 mL) and 1M aqueous hydrochloric acid solution (20 mL) were added, and the mixture was extracted once by liquid separation, and the aqueous layer was extracted three times with ethyl acetate (5 mL) by liquid separation. The organic layers were combined, washed once with 5% aqueous sodium chloride solution (20 mL) by liquid separation, and dried over sodium sulfate. The sodium sulfate was removed by filtration, and the filtrate was concentrated under reduced pressure to give a crude product (633.8 mg) as a yellow oil. The crude product was purified by flash silica gel column (NH₂ silica gel 80 g, hexane/ethyl acetate = 43/57 - 0/100). To the obtained white solid (294.0 mg) was added toluene, and the solid was pulverised by ultrasonic irradiation, and collected by filtration to give Compound 11-1 (234.3 mg, containing 1.9wt% ethyl acetate, two-step conversion yield from Compound 3-3 61%) as a white solid.

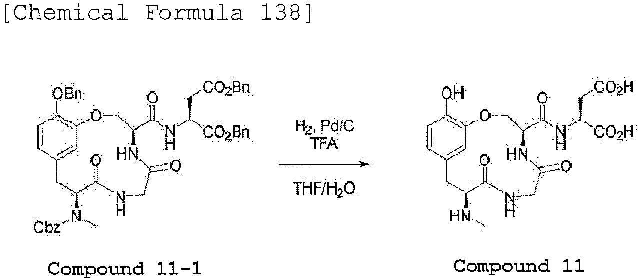

Under nitrogen atmosphere, to a solution of Compound 11-1 (1.0 eq., 234 mg, containing 1.9wt% ethyl acetate, 0.268 mmol) in a mixture of THF (10 mL)/water (10 mL) was added 10% palladium-carbon (N.E. CHEMCAT NX type, 50% hydrous product, 57 mg) at room temperature, the system was replaced with hydrogen, and the mixture was stirred at the same temperature for 2 hr. TFA (2.0 eq., 41 µL, 0.536 mmol) was added, the mixture was stirred at the same temperature for 1 hr, the catalyst was removed by filtration through Celite, and the filtrate was concentrated under reduced pressure to give a crude product (189.5 mg) as a pale-orange solid. To the obtained crude product was added ethyl acetate, the product was pulverised by ultrasonic irradiation, and the insoluble substance was collected by filtration, and dried under reduced pressure. The obtained solid was dissolved in water, the insoluble substance was removed by filtration, and the filtrate was concentrated to give a TFA salt of Compound 11 (150.9 mg, yield 99.6%, purity 99.4%) as a pale-orange solid.

¹H-NMR(400MHz,D₂O)δ6.89-6.76(m,3H),4.58(dd,J=13.2,5.2Hz,1H),4.40(d,J=13.2,lH),4.21(d,J= 15.2Hz,1H),3.86(dd,J=9.6,4.4Hz,1H),3.26(d,J=15.2Hz,1H),3.21(dd, J=13.2,4.4Hz,1H),2.95-2.82(m,3H),2.63(s,3H).

### (Example 14: Synthesis of Compound 6)

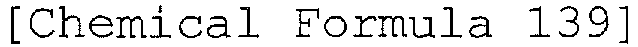

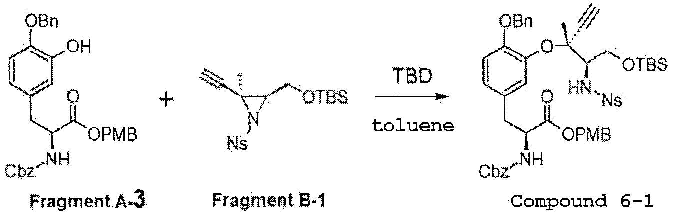

Under nitrogen atmosphere, to a solution of Fragment A-3 (1.0 eq., 3.10 g, 5.72 mmol) and Fragment B-1 (1.2 eq. 3.17 g, containing 20 mol% DIAD, 6.87 mmol) in toluene (57 mL) was added TBD (1.08 eq., 857 mg, 6.16 mmol) under ice-cooling, and the mixture was stirred at room temperature for 3 hr. The mixture was concentrated under reduced pressure to evaporate the toluene to give a crude product as a brownish-red amorphous. The crude product was purified by flash silica gel column (normal phase silica gel 70 g, hexane/ethyl acetate = 5/1 - 2/1) to give Compound 6-1 (3.84 g, yield 70%) as a pale-yellow amorphous.

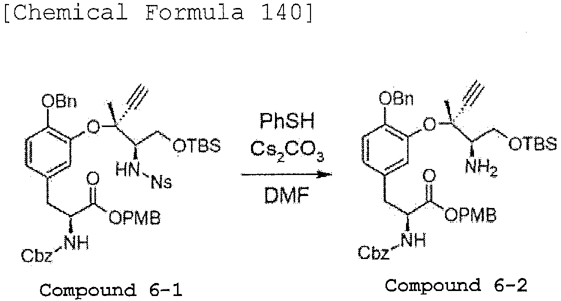

Under nitrogen atmosphere, to a solution of Compound 6-1 (1.0 eq., 1.41 g, 1.48 mmol) in DMF (20 mL) were added cesium carbonate (1.5 eq., 781 mg, 2.21 mmol) and thiophenol (1.5 eq., 230 µL, 2.25 mmol) at room temperature, and the mixture was stirred at room temperature for 3 hr. To the reaction solution were added saturated aqueous sodium hydrogencarbonate solution (10 mL), saturated aqueous sodium chloride solution (10 mL) and water (20 mL), and the mixture was extracted three times with a mixture of hexane (10 mL)/ethyl acetate (30 mL) by liquid separation. The organic layers were combined, washed once with saturated aqueous sodium chloride solution (60 mL) by liquid separation, and dried over magnesium sulfate. The magnesium sulfate was removed by filtration, and the filtrate was concentrated under reduced pressure to give a crude product (2.01 g) as a yellow oil. The crude product was purified by flash silica gel column (normal phase silica gel 20 g, hexane/ethyl acetate = 5/1 - 1/1) to give Compound 6-2 (1.02 g, yield 90%) as a pale-yellow viscous product.

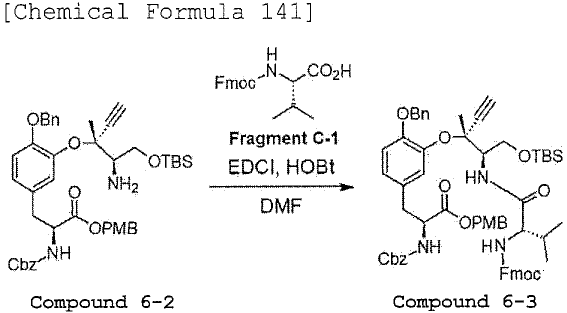

Under nitrogen atmosphere, to a solution of Compound 6-2 (1.0 eq., 4.44 g, 5.79 mmol) in DMF (58 mL) were added EDCI (2.0 eq. 2.23 g, 11.65 mmol), HOBt·H₂O (2.0 eq., 1.77 g, 11.59 mmol) and N-Fmoc-L-Val (2.0 eq., 3.97 g, 11.69 mmol) under ice-cooling, and the mixture was stirred at room temperature for 2 hr. To the reaction solution was added water (60 mL), and the mixture was extracted three times with a mixture of hexane (15 mL)/ethyl acetate (45 mL) by liquid separation. The organic layers were combined, washed twice with saturated aqueous sodium hydrogencarbonate solution (100 mL) by liquid separation, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (9.01 g). The crude product was purified by flash silica gel column (normal phase silica gel 70 g, hexane/ethyl acetate = 5/1 - 2/1) to give Compound 6-3 (5.69 g, yield 90%) as a white amorphous.

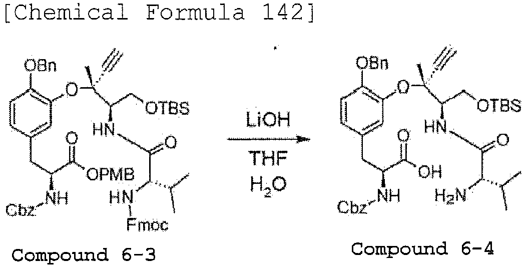

To a solution of Compound 6-3 (1.0 eq., 2.07 g, 1.90 mmol) in a mixture of THF (12 mL)/water (6 mL) was added lithium hydroxide (4.0 eq., 181 mg, 7.58 mmol) at room temperature, and the mixture was stirred at room temperature for 3.5 hr. The reaction solution was adjusted to pH 7 by addition of 1N aqueous hydrochloric acid solution (5 mL) under ice-cooling, and extracted three times with ethyl acetate (10 mL) by liquid separation. The organic layers were combined, and washed twice with saturated aqueous sodium chloride solution (30 mL) by liquid separation. The organic layer was dried over magnesium sulfate, the magnesium sulfate was removed by filtration, and the filtrate was concentrated under reduced pressure to give a crude product (2.21 g). The crude product was purified by flash silica gel column (normal phase silica gel 25 g, ethyl acetate/methanol = 20/1 - 5/1) to give Compound 6-4 (1.03 g, yield 73%) as a white amorphous.

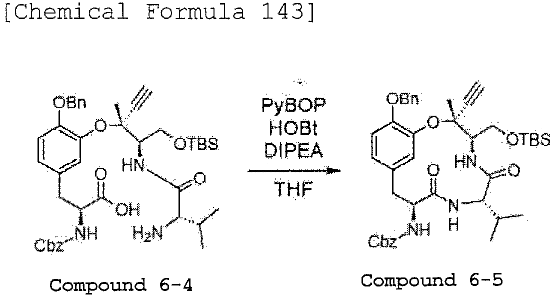

A solution of Compound 6-4 (1.0 eq., 2.80 g, 3.75 mmol) in THF (375 mL) was added dropwise to a solution of PyBOP (5.0 eq., 9.76 g, 18.76 mmol), HOBt·H₂O (5.0 eq., 2.87 g, 18.76 mmol) and DIPEA (5.0. eq, 3.2 mL, 18.82 mmol) in THF (1.5L) over 4 days at about 80 µL/min at room temperature. The reaction solution was concentrated under reduced pressure until the liquid volume was reduced to about one-tenth, and washed twice with saturated aqueous sodium hydrogencarbonate solution (100 mL) by liquid separation. The aqueous layers were combined, and extracted twice with ethyl acetate (100 mL) by liquid separation. The organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (16.80 g). The crude product was purified by flash silica gel column (normal phase silica gel 100 g, hexane/ethyl acetate = 3/1 - 1/1) to give Compound 6-5 (1.18 g, yield 43%) as a white amorphous.

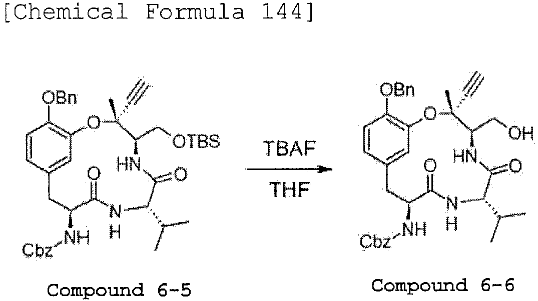

Under nitrogen atmosphere, to a solution of Compound 6-5 (1.0 eq., 619 mg, 850 µmol) in THF (9 mL) was added TBAF (1M in THF, 1.88 eq., 1.6 mL, 1.60 mmol) under ice-cooling, and the mixture was stirred at room temperature for 1 hr. To the reaction solution was added water (10 mL), and the mixture was extracted three times with ethyl acetate (10 mL) by liquid separation. The organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (677 mg). The crude product was purified by flash silica gel column (normal phase silica gel 7 g, hexane/ethyl acetate = 1/1 - 0/1) to give Compound 6-6 (495 mg, yield 95%) as a white solid.

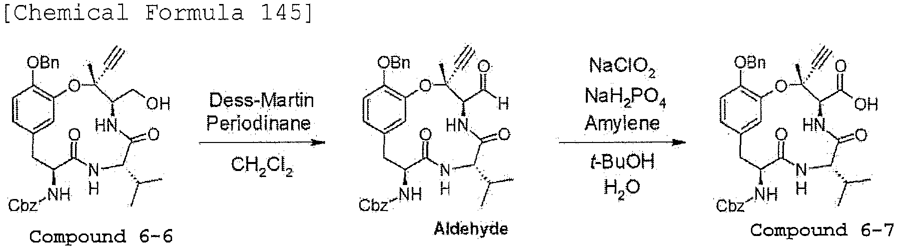

Under nitrogen atmosphere, to a solution of Compound 6-6 (1.0 eq., 495 mg, 807 µmol) in dichloromethane (8 mL) was added Dess-Martin periodinane (1.5 eq., 508 mg, 1.20 mmol) under ice-cooling, and the mixture was stirred at room temperature for 1 hr. To the reaction solution were added 10% aqueous sodium sulfite solution (10 mL) and saturated aqueous sodium hydrogencarbonate solution (10 mL), and the mixture was washed once by liquid separation. The aqueous layer was extracted twice with dichloromethane (10 mL) by liquid separation, and the organic layers were combined, and washed once with saturated aqueous sodium chloride solution (30 mL) by liquid separation. The organic layer was dried over magnesium sulfate, the magnesium sulfate was removed by filtration, and the filtrate was concentrated under reduced pressure to give Aldehyde (515 mg, crude yield 104%).

To a solution of Aldehyde (1.0 eq., 515 mg, calculated as 807 µmol) in a mixture of t-butyl alcohol (6 mL)/amylene (2 mL) was added a solution of sodium dihydrogen phosphate dihydrate (3.4 eq., 429 mg, 2.74 mmol) and 80% sodium chlorite (4.5 eq., 412 mg, 3.65 mmol) in water (2 mL) at room temperature, and the mixture was stirred at room temperature for 30 min. To the reaction solution was added saturated aqueous ammonium chloride solution (10 mL), and the mixture was extracted three times with ethyl acetate (10 mL) by liquid separation. The organic layers were combined, and dried over magnesium sulfate. The magnesium sulfate was removed by filtration, and the filtrate was concentrated under reduced pressure to give a crude product (526 mg). The crude product was purified by flash silica gel column (normal phase silica gel 10 g, chloroform/methanol = 100/1 - 30/1) to give Compound 6-7 (410 mg, two-step yield 83%) as a white amorphous.

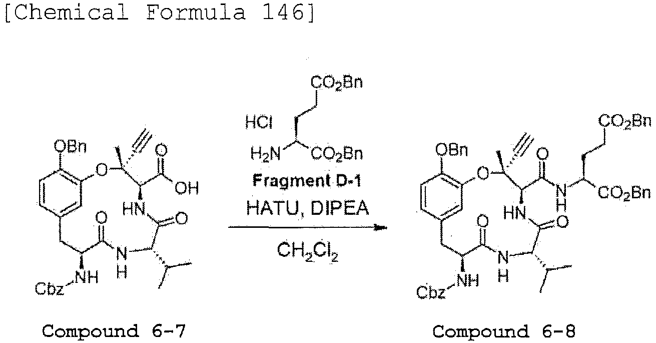

Under nitrogen atmosphere, to a solution of Compound 6-7 (1.0 eq., 254 mg, 404 µmol) and dibenzyl L-glutamate hydrochloride (Fragment D-1, 1.2 eq., 176 mg, 483 µmol) in dichloromethane (4 mL) were added DIPEA (1.5 eq., 100 µL, 588 µmol) and HATU (1.2 eq., 186 mg, 490 µmol) under ice-cooling, and the mixture was stirred under ice-cooling for 1 hr. To the reaction solution was added saturated aqueous ammonium chloride solution (4 mL), and the mixture was washed once by liquid separation. Then, the aqueous layer was extracted twice with dichloromethane (4 mL) by liquid separation. The organic layers were combined, washed once with saturated aqueous sodium hydrogencarbonate solution (20 mL) by liquid separation, and washed once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (463 mg). The crude product was purified twice by flash silica gel column (1st run: normal phase silica gel 7 g, hexane/ethyl acetate = 5/1 - 1/1, 2nd run: normal phase silica gel 10 g, hexane/ethyl acetate = 70/30 - 30/70) to give Compound 6-8 (40 mg, yield 10%) as a white amorphous.

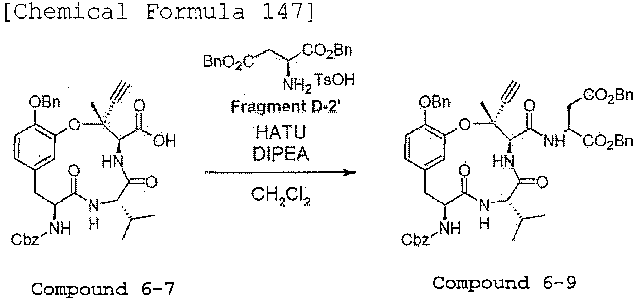

Under nitrogen atmosphere, to a solution of Compound 6-7 (1.0 eq., 369 mg, 589 µmol) and dibenzyl L-aspartate paratoluenesulfonate (Fragment D-2, 1.3 eq., 372 mg, 766 µmol) in dichloromethane (10 mL) were added DIPEA (2.3 eq., 230 µL, 1.35 mmol) and HATU (1.1 eq., 237 mg, 623 µmol) under ice-cooling, and the mixture was stirred under ice-cooling for 1 hr. To the reaction solution was added saturated aqueous ammonium chloride solution (10 mL), and the mixture was washed once by liquid separation. Then, the aqueous layer was extracted twice with dichloromethane (10 mL) by liquid separation. The organic layers were combined, washed once with saturated aqueous sodium hydrogencarbonate solution (20 mL) by liquid separation, and washed once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (761 mg). The crude product was purified twice by flash silica gel column (1st run: normal phase silica gel 13 g, hexane/ethyl acetate = 70/30 - 30/70, 2nd run: normal phase silica gel 7 g, hexane/ethyl acetate = 70/30 - 20/80) to give Compound 6-9 (45 mg, yield 8%) as a white amorphous.

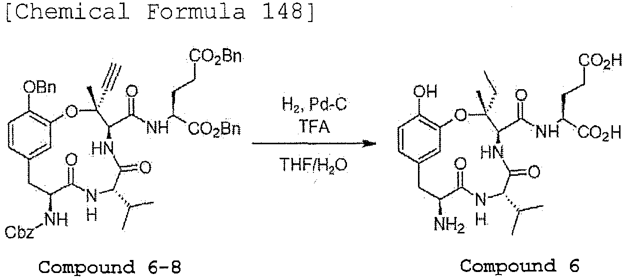

Under nitrogen atmosphere, to a solution of Compound 6-8 (1.0 eq., 29 mg, 0.031 mmol) in a mixture of THF (5.8 mL)/water (0.29 mL) were added 10% palladium-carbon (N.E. CHEMCAT NX type, 50% hydrous product, 2.9 mg) and TFA (2.0 eq., 11.6 µL, 0.152 mmol) at room temperature, the system was replaced with hydrogen, and the mixture was stirred at the same temperature for 16.5 hr. Additional 10% palladium-carbon (N.E. CHEMCAT NX type, 50% hydrous product, 0.6 mg) was added, and the mixture was stirred for additional 3 hr.

The reaction solution obtained by treating Compound 6-8 (1.0 eq., 5 mg, 5.34 µmol) in the same manner and the previous reaction solution were combined, the catalyst was removed by filtration through Celite, and the filtrate was concentrated under reduced pressure to give a crude product (25 mg). The crude product was purified by flash silica gel column (reverse-phase silica gel 30 g, 0.05% aqueous TFA solution/acetonitrile = 95/5 - 90/10), and freeze-dried to give a TFA salt of Compound 6 (20.3 mg, yield 86%, purity 99.6%) as a white solid.

¹H-NMR(400MHz,D₂O)δ6.99-6.83(m,3H),4.90(s,1H),4.39(dd,J=9.6,4.8Hz,1H),4.20(dd,J=8.4,4.8 Hz,1H),4.10(d,J=9.6Hz,1H),3.21(dd,J=13.6,4.8Hz,1H),2.92(dd,J=13 .6,8.4Hz,1H),2.51-2.35(m,2H),2.25-2.14(m,1H),2.16-1.91(m,3H),1.84-1.72(m,1H),1.53(s,3H),1.06(t,J=7.2Hz,3H),0.87(d,J=6.8Hz,3H),0.8 0(d,J=6.8Hz,3H).

### (Example 15: Synthesis of Compound 15)

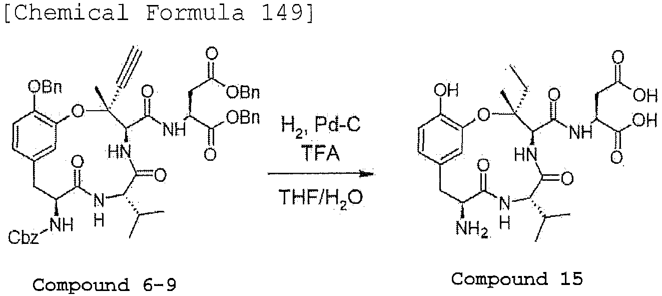

The reaction was carried out in the same manner as for Compound 6, and a TFA salt of Compound 15 (23.1 mg, yield 74%, purity 98.6%) was obtained from Compound 6-9.

¹H-NMR(400MHz,D₂O)δ6.92-6.71(m,3H),4.84(s,1H),4.63(dd,J=7.2,5.2Hz,1H),4.13(dd,J=8.0,4.8 Hz,1H),4.00(d,J = 100Hz,1H),3.13(dd,J=13.6,4.8Hz,1H),2.93-2.79(m,3H),1.95-1.82(m,2H),1.78-1.64(m,1H),1.45(s,3H),0.99(t,J=7.2Hz,3H),0.79(d,J=6.8Hz,3H),0.7 1(d,J=6.8Hz,3H).

### (Example 16: Synthesis of Compound 19)

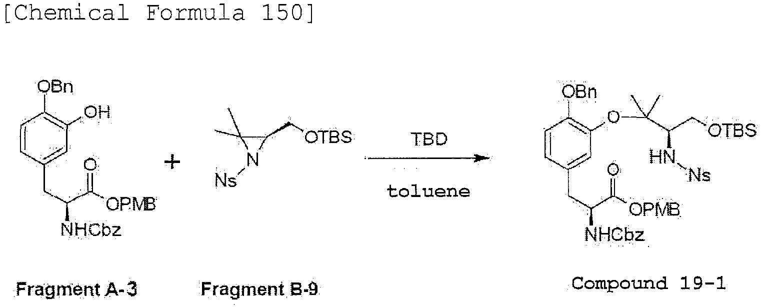

Under nitrogen atmosphere, to a solution of Fragment A-3 (1.0 eq., 514 mg, 0.949 mmol) and Fragment B-9 (1.12 eq., 425 mg, 1.06 mmol) in toluene (10 mL) was added TBD (1.10 eq., 145 mg, 1.04 mmol) under ice-cooling, and the mixture was stirred at room temperature for 1.5 hr. the mixture was warmed to 40°C, stirred for 1.5 hr, warmed to 60°C, and stirred for 16 hr. The reaction solution was concentrated under reduced pressure to give a crude product (1.21 g). The crude product was purified by flash silica gel column (normal phase silica gel 20 g, hexane/ethyl acetate = 5/1 - 1/1) to give Compound 19-1 (601 mg, quant.) as an orange amorphous.

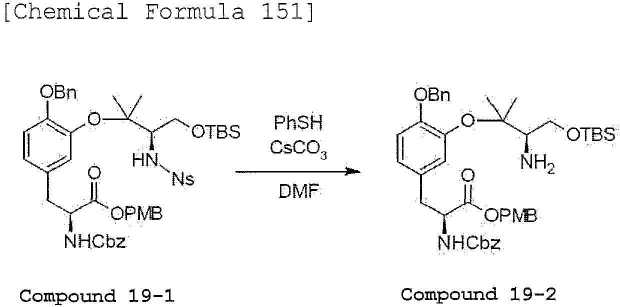

Under nitrogen atmosphere, to a solution of Compound 19-1 (1.0 eq., 601 mg, 638 µmol) in DMF (6 mL) were added cesium carbonate (1.51 eq., 339 mg, 960 µmol) and thiophenol (1.54 eq., 100 µl, 980 µmol) at room temperature, and the mixture was stirred at the same temperature for 2 hr. Saturated aqueous sodium hydrogencarbonate solution (6 mL) was added. The mixture was extracted three times with hexane (3.75 mL)/ethyl acetate (11.25 mL) by liquid separation. The organic layer was washed once with saturated aqueous sodium chloride solution (30 mL) by liquid separation, and dried over magnesium sulfate. The magnesium sulfate was removed by filtration, and the filtrate was concentrated under reduced pressure to give a crude product (814 mg) as a yellow oil. The crude product was purified by flash silica gel column (normal phase silica gel 10 g, hexane/ethyl acetate = 5/1 - 1/2) to give Compound 19-2 (392 mg, two-step yield 55%) as a pale-yellow liquid.

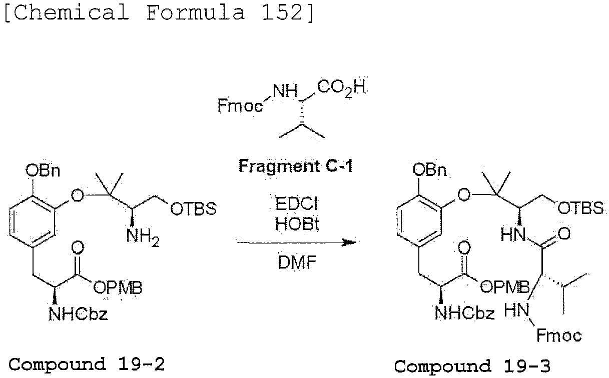

Under nitrogen atmosphere, to a solution of Compound 19-2 (1.0 eq., 392 mg, 518 µmol) in DMF (5 mL) were added N-Fmoc-L-valine (Fragment C-1, 1.2 eq., 211 mg, 620 µmol), EDCI (1.2 eq., 122 mg, 638 µmol) and HOBt·H₂O (1.2 eq., 97.5 mg, 637 µmol), and the mixture was stirred at room temperature for 2 hr. The mixture was extracted three times with hexane (2.5 mL)/ethyl acetate (7.5 mL) by liquid separation. The organic layers were combined, washed twice with saturated aqueous sodium hydrogencarbonate solution (20 mL) and once with saturated aqueous sodium chloride solution (20 mL) by liquid separation, and dried over magnesium sulfate. The magnesium sulfate was removed by filtration, and the filtrate was concentrated under reduced pressure to give Compound 19-3 (608 mg, quant.) as a white amorphous.

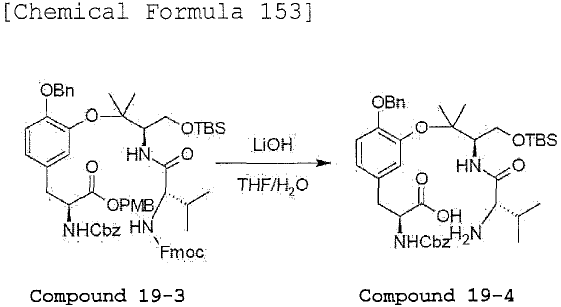

Under nitrogen atmosphere, to a solution of Compound 19-3 (1.0 eq., 608 mg, 518 µmol) in a mixture of THF (4 mL)/water (2 mL) was added lithium hydroxide (4.1 eq., 50.8 mg, 2.12 mmol) at room temperature, and the mixture was stirred at the same temperature for 4.5 hr. The mixture was adjusted to pH 7 by addition of 1N aqueous hydrochloric acid solution (about 1 mL) under ice-cooling. The mixture was diluted with water, and extracted three times with ethyl acetate (10 mL) by liquid separation, and the organic layer was dried over magnesium sulfate. The magnesium sulfate was removed by filtration, and the filtrate was concentrated under reduced pressure to give a crude product (528 mg). The crude product was purified by flash silica gel column (normal phase silica gel 10 g, ethyl acetate/methanol = 1/0 - 4/1) to give Compound 19-4 (155 mg, two-step yield 41%) as a white amorphous.

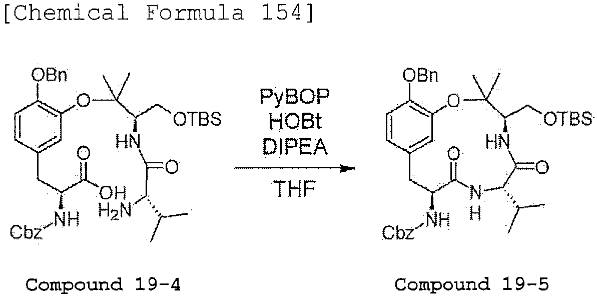

Under nitrogen atmosphere, to a solution of PyBOP (5 eq., 5.36 g, 10.3 mmol) and HOBt·H₂O (5 eq., 1.58 g, 10.3 mmol) in THF (1030 mL) was added DIPEA (5 eq., 1.8 mL, 10.3 mmol) at room temperature. Then, a solution of Compound 19-4 (1.0 eq., 1.52 g, 2.06 mmol) in THF (206 mL) was added over 50 hr at the same temperature, and after the dropwise addition was completed, the mixture was stirred for additional 62.5 hr at room temperature. The reaction solution was concentrated under reduced pressure at a bath temperature of 40°C to approximately 200 mL. The reaction solution was diluted with ethyl acetate (400 mL), and washed three time with saturated aqueous ammonium chloride solution (100 mL), three time with water (100 mL) and once with saturated aqueous sodium chloride solution (100 mL) by liquid separation, and the organic layer was dried over magnesium sulfate. The magnesium sulfate was removed by filtration, and the filtrate was concentrated under reduced pressure to give a crude product (7.84 g). The crude product was purified by flash silica gel column (normal phase silica gel 234 g, hexane/ethyl acetate = 2/1 - 1/3) to give Compound 19-5 (673 mg, yield 46%) as a white amorphous.

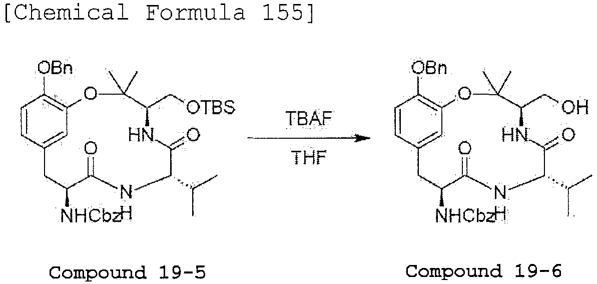

Under nitrogen atmosphere, to a solution of Compound 19-5 (1.0 eq., 673 mg, 0.94 mmol) in THF (9 mL) was added TBAF (1M in THF, 1.2 eq., 1.2 mL, 1.2 mmol) under ice-cooling. Then, the mixture was stirred at room temperature for 2 hr. The reaction solution was diluted with ethyl acetate (50 mL), and washed three times with water (30 mL) by liquid separation, and the organic layer was dried over magnesium sulfate. The magnesium sulfate was removed by filtration, and the filtrate was concentrated under reduced pressure to give a crude product (600 mg). To the crude product was added a mixture of hexane (7.3 mL)/ethyl acetate (0.73 mL), and the mixture was subjected to ultrasonic irradiation. Then, the precipitated white solid was collected by filtration to give Compound 19-6 (505 mg, yield 89%) as a white solid.

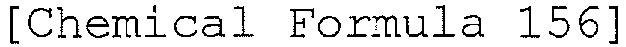

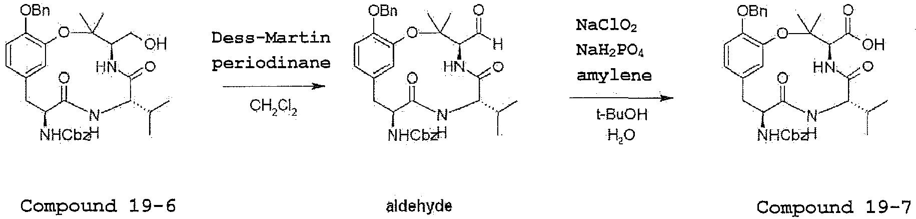

Under nitrogen atmosphere, to a solution of Compound 19-6 (1.0 eq., 505 mg, 0.84 mmol) in dichloromethane (8 mL) was added Dess-Martin periodinane (1.5 eq., 540 mg, 1.26 mmol) under ice-cooling, and the mixture was stirred at room temperature for 2 hr. The reaction solution was diluted with ethyl acetate (70 mL), and washed twice with a mixture of 10% aqueous sodium sulfite solution (12.5 mL)/saturated aqueous sodium hydrogencarbonate solution (12.5 mL) and twice with water (20 mL) by liquid separation, and the organic layer was dried over magnesium sulfate. The magnesium sulfate was removed by filtration, and the filtrate was concentrated under reduced pressure to give Aldehyde (482 mg, quant.) as a white solid.

Under nitrogen atmosphere, to a solution of Aldehyde (1.0 eq., 482 mg, calculated as 0.84 mmol) in a mixture of t-butyl alcohol (7.5 mL)/amylene (2.5 mL) was added dropwise a solution of sodium dihydrogen phosphate dihydrate (3.4 eq., 446 mg, 2.86 mmol) and 80% sodium sulfite (4.5 eq., 342 mg, 3.78 mmol) in water (2.5 mL) at room temperature, and the mixture was stirred at the same temperature for 2 hr. The reaction solution was diluted with ethyl acetate (50 mL), and washed once with saturated aqueous ammonium chloride solution (30 mL) and three times with water (30 mL) by liquid separation, and the organic layer was dried over magnesium sulfate. The magnesium sulfate was removed by filtration, and the filtrate was concentrated under reduced pressure to give a crude product (647 mg). The crude product was purified by flash silica gel column (normal phase silica gel 8.0 g, chloroform/methanol=30/1) to give Compound 19-7 (514 mg, yield 99%) as a white amorphous.

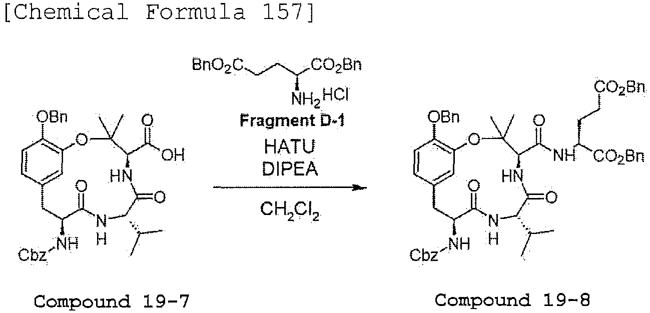

Under nitrogen atmosphere, to a solution of Compound 19-7 (1.0 eq., 251 mg, 0.40 mmol) and dibenzyl L-glutamate hydrochloride (Fragment D-1, 1.05 eq, 154 mg, 0.42 mmol) in dichloromethane (4 mL) were added DIPEA (1.05 eq., 70 µL, 0.42 mmol) and HATU (1.05 eq., 161 mg, 0.42 mmol) under ice-cooling, and the mixture was stirred under ice-cooling for 1 hr, and then stirred at room temperature for 19 hr. Additional DIPEA (0.2 eq., 14 µL, 0.08 mmol) and HATU (0.2 eq., 31.2 mg, 0.08 mmol) were added under ice-cooling, and the mixture was stirred for 2.5 hr. Then, to the reaction solution was added saturated aqueous ammonium chloride solution (5 mL). The mixture was diluted with ethyl acetate (30 mL), and washed once with saturated aqueous ammonium chloride solution (10 mL), twice with saturated aqueous sodium hydrogencarbonate solution (10 mL), twice with water (10 mL) and once with saturated aqueous sodium chloride solution (10 mL) by liquid separation, and the organic layer was dried over magnesium sulfate. The magnesium sulfate was removed by filtration, and the filtrate was concentrated under reduced pressure to give a crude product (396 mg). The crude product was purified twice by flash silica gel column (1st run: normal phase silica gel 6.4 g, hexane/ethyl acetate = 80/20 - 30/70, 2nd run: normal phase silica gel 3.9 g, hexane/ethyl acetate = 60/40 - 40/60) to give a crude product containing Compound 19-8 as a white amorphous. The crude product was dissolved in ethyl acetate (1 mL), and hexane (3 mL) was added dropwise, and the precipitated white solid was collected by filtration to give Compound 19-8 (20.5 mg, yield 5.5%).

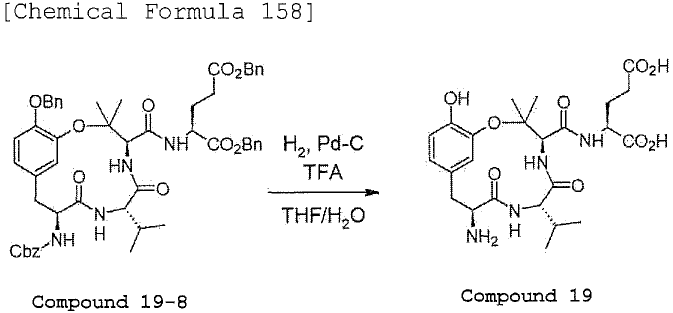

The reaction was carried out in the same manner as for Compound 6, and a TFA salt of Compound 19 (10.1 mg, yield 73%, purity 98.5%) was obtained from Compound 19-8.

¹H-NMR(400MHz,D₂O)δ6.85(s,2H)6.74(s,1H)4.84(s,1H)4.31(dd,J=9.6, 4.8Hz,1H),4.16(dd,J=7.2,4.8Hz,1H),4.02(d,J= 100Hz,1H),3.14(dd,J=14.0,4.8Hz,1H),2.84(dd,J=14.0,7.6Hz,1H),2.4 4-2.28(m,2H),2.19-2.07(m,1H),1.97-1.83(m,2H),1.48(s,3H),1.43(s,3H),0.80(d,J=6.8Hz,3H),0.72(d,J=6. 4Hz,3H).

### (Example 17: Synthesis of Compound 4)

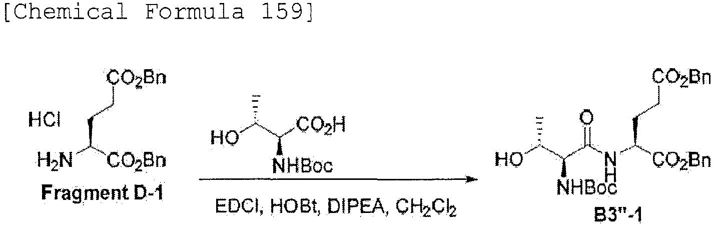

Under nitrogen atmosphere, to a solution of Fragment D-1 (2.61 g, 7.17 mmol, 1.0 eq.) and Boc-L-Thr-OH (1.73 g, 7.89 mmol, 1.1 eq.) in dichloromethane (30 mL) were added DIPEA (2.75 mL, 15.8 mmol, 2.2 eq.), HOBt·H₂O (1.32 g, 8.61 mmol, 1.2 eq.) and EDCI (1.65 g, 8.61 mmol, 1.2 eq.) under ice-cooling. The mixture was stirred at the same temperature for 10 min, and then at room temperature for 2 hr, additional EDCI (413 mg, 2.15 mmol, 0.3 eq.) was added, and the mixture was stirred at room temperature for additional 40 min. To the reaction solution was added water (20 mL), and the mixture was extracted once with ethyl acetate (90 mL) and three times with ethyl acetate (10 mL) by liquid separation. The organic layers were combined, washed once with 1M aqueous hydrochloric acid solution (30 mL), once with 5% aqueous sodium hydrogencarbonate solution (30 mL) and once with 5% aqueous sodium chloride solution (30 mL) by liquid separation, and dried over sodium sulfate. The sodium sulfate was removed by filtration, and the filtrate was concentrated to give B3"-1 (4.17 g, quant.) as a pale-orange oil.

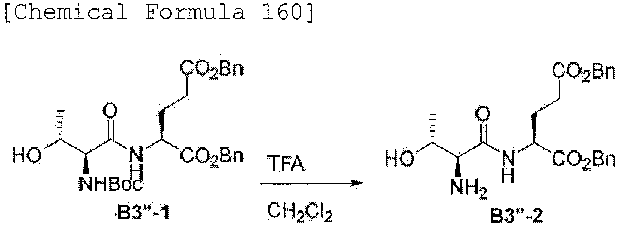

Under nitrogen atmosphere, to a solution of B3"-1 (4.17 g, calculated as 7.17 mmol, 1.0 eq.) in dichloromethane (15 mL) was added TFA (7.0 mL) at room temperature, the mixture was stirred at the same temperature for 3 hr, and the reaction solution was concentrated under reduced pressure. The obtained concentrated residue was subjected to azeotropic distillation with toluene (20 mL) three times to give B3-2 (4.61 g, quant.) as a pale-orange oil.

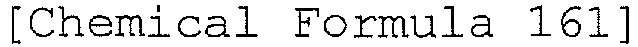

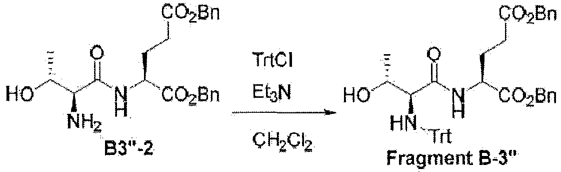

Under nitrogen atmosphere, to a solution of B3-2 (4.61 g, calculated as 7.17 mmol, 1.0 eq.) in dichloromethane (15 mL) were added triethylamine (4.0 mL, 28.7 mmol, 4.0 eq.) and TrCl (2.20 g, 7.89 mol, 1.1 eq.) under ice-cooling. The mixture was stirred at the same temperature for 1.5 hr, to the reaction solution was added 5% aqueous sodium hydrogencarbonate solution (20 mL), and the mixture was extracted once with ethyl acetate (50 mL) and three times with ethyl acetate (10 mL) by liquid separation. The organic layers were combined, washed once with 5% aqueous sodium chloride solution (30 mL) by liquid separation, and dried over sodium sulfate. The sodium sulfate was removed by filtration, and the filtrate was concentrated under reduced pressure to give a crude product. The crude product was purified by flash silica gel column (normal phase silica gel 80 g, hexane/ethyl acetate = 3/1 - 2/1) to give Fragment B-3" (4.20 g, Net: 4.10 g, containing ethyl acetate: 2.4wt%, conversion yield 85%, three steps from Fragment D-1) as a white viscous product.

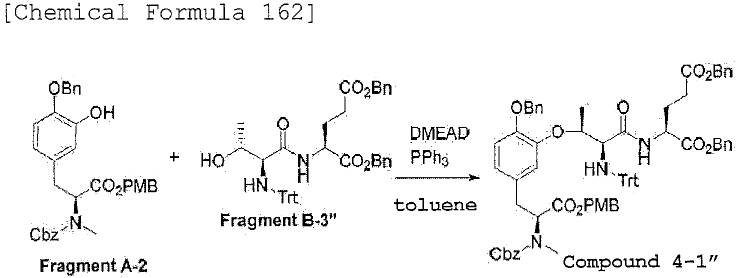

Under nitrogen atmosphere, to a solution of Fragment A-2 (1.0 eq., 1.10 g, containing 2.2wt% ethyl acetate, 1.94 mmol) and Fragment B-3" (2.5 eq., 3.34 g, containing 2.4wt% ethyl acetate, 4.86 mmol) in toluene (5 mL) was added triphenylphosphine (3.0 eq., 1.53 g, 5.83 mmol) at room temperature, and then DMEAD (3.0 eq., 1.37 g, 5.83 mmol) was added under ice-cooling, and the mixture was stirred at the same temperature for 30 min, and then at room temperature for 45 min. Additional triphenylphosphine (1.5 eq., 0.77 g, 2.91 mmol) and DMEAD (1.5 eq., 0.69 g, 2.91 mmol) were added at room temperature, the mixture was stirred at the same temperature for 45 min, and the reaction solution was concentrated under reduced pressure to give a crude product (9.49 g) as an orange oil. This crude product and the crude product (2.226 g) obtained from Fragment A-2 (204 mg) by the same procedure were combined, and purified by flash silica gel column (normal phase silica gel 117 g, hexane/ethyl acetate = 3/1 - 3/2) to give Compound 4-1" (1.81 g, containing impurities, apparent yield 65%) as a yellow oil.

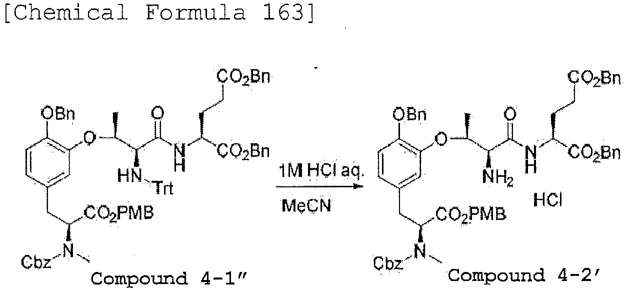

Under nitrogen atmosphere, to a solution of Compound 4-1" (1.0 eq., 1.70 g, calculated as 1.41 mmol) in acetonitrile (17 mL) was added 1M aqueous hydrochloric acid solution (1.1 eq., 1.55 mL, 1.55 mmol) at room temperature, the mixture was stirred at the same temperature for 45 min, and the reaction solution was concentrated under reduced pressure at a bath temperature of 30°C to give a crude product (2.23 g) as a yellow oil. This crude product and the crude product (89.2 mg) obtained from Compound 4-1" (100 mg) by the same procedure were combined, and purified by flash silica gel column (normal phase silica gel 50 g, hexane/ethyl acetate = 2/1 - 1/1→chloroform/methanol = 30/1 - 20/1) to give Compound 4-2' (0.85 g, containing 6.3wt% ethyl acetate, conversion yield 53%) as a yellow oil.

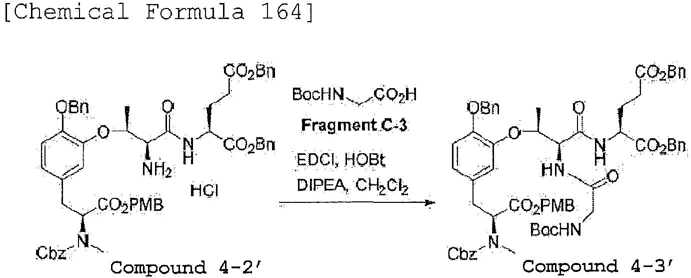

Under nitrogen atmosphere, to a solution of Compound 4-2' (1.0 eq., 0.85 g, containing 6.3wt% ethyl acetate, 0.80 mmol) and N-Boc-L-glycine (Fragment C-3, 1.5 eq., 0.21 g, 1.20 mmol) in dichloromethane (8 mL) were added DIPEA (3.0 eq., 0.417 mL, 2.39 mmol), HOBt·H₂O (1.5 eq., 0.18 g, 1.20 mmol) and EDCI (1.5 eq., 0.23 g, 1.20 mmol) under ice-cooling, and the mixture was stirred at room temperature for 19 hr. The reaction solution was concentrated under reduced pressure, to the concentrated residue were added 1M aqueous hydrochloric acid solution (15 mL) and water (10 mL), and the mixture was extracted once with ethyl acetate (30 mL) and three times with ethyl acetate (10 mL) by liquid separation. The organic layers were combined, washed once with 5% aqueous sodium hydrogencarbonate solution (10 mL) and once with 5% aqueous sodium chloride solution (10 mL) by liquid separation, and dried over sodium sulfate. The sodium sulfate was removed by filtration, and the filtrate was concentrated under reduced pressure to give a crude product (0.94 g) as a pale-yellow oil. The crude product was purified by flash silica gel column (NH₂ silica gel 20 g, hexane/ethyl acetate = 2/1 - 2/3) to give Compound 4-3' (0.8242 g, containing 2.4wt% ethyl acetate, conversion yield 90%) as a colorless oil.

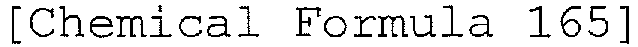

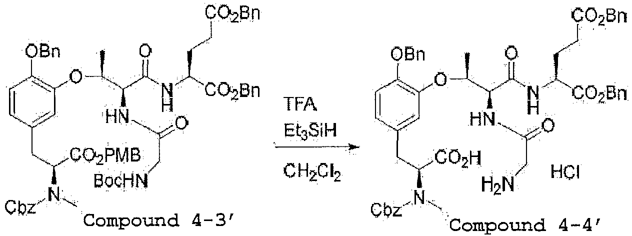

Under nitrogen atmosphere, to a solution of Compound 4-3' (1.0 eq., 0.82 g, containing 2.4wt% ethyl acetate, 0.712 mmol) in dichloromethane (6.3 mL) were added triethylsilane (25.6 eq., 2.91 mL, 18.3 mmol) and TFA (3.2 mL) under ice-cooling, the mixture was stirred at the same temperature for 20 min, and then at room temperature for 4 hr, and the reaction solution was concentrated under reduced pressure. To the obtained concentrated residue was added 0.05M hydrogen chloride/1,4-dioxane solution (20 mL), and the mixture was concentrated under reduced pressure. These procedures were repeated twice. 1,4-Dioxane (20 mL) was added, and the mixture was concentrated under reduced pressure. These procedures were repeated three times to give Compound 4-4' (1.35 g, containing 1,4-dioxane, quant.) as a pale-yellow oil.

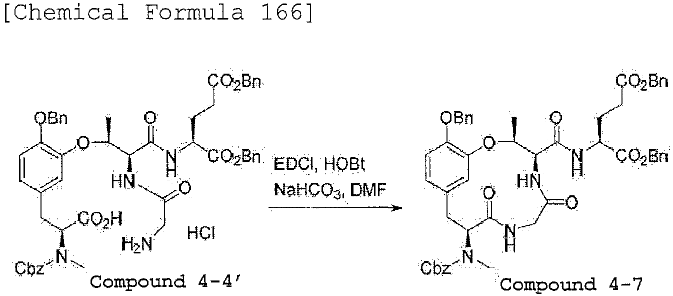

Under nitrogen atmosphere, to a solution of HOBt·H₂O (5.0 eq., 545 mg, 3.56 mmol) and sodium hydrogencarbonate (10.0 eq., 598 mg, 7.12 mmol) in DMF (712 mL) was added EDCI (5.0 eq., 682 mg, 3.56 mmol), and then a solution of Compound 4-4' (1.0 eq., 1.35 g, calculated as 0.712 mmol) in DMF (62 mL) was added dropwise over 2 hr at room temperature. The mixture was stirred at the same temperature for 18.5 hr, and the reaction solution was concentrated under reduced pressure at a bath temperature of 50°C. To the concentrated residue was added water (50 mL), and the mixture was extracted once with ethyl acetate (50 mL) and three times with ethyl acetate (10 mL) by liquid separation. The organic layers were combined, washed with 5% aqueous sodium chloride solution (20 mL) by liquid separation, and dried over sodium sulfate. The sodium sulfate was removed by filtration, and the filtrate was concentrated under reduced pressure to give a crude product (0.77 g) as a pale-orange oil. The crude product was purified by flash silica gel column (normal phase silica gel 25 g, hexane/ethyl acetate = 50/50 - 34/66) to give Compound 4-7 (150.7 mg, containing 4.6wt% ethyl acetate, conversion yield 23%) as a yellow oil.

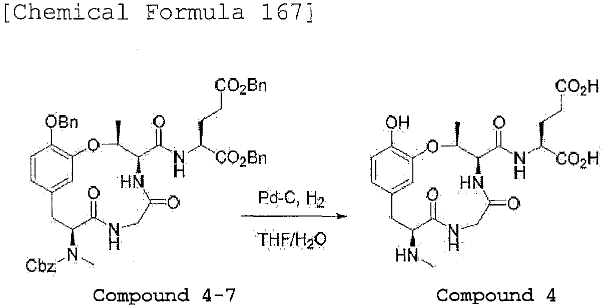

Under nitrogen atmosphere, to a solution of Compound 4-7 (1.0 eq., 51.9 mg, containing 4.6wt% ethyl acetate, 0.056 mmol) in a mixture of THF (2.0 mL)/water (2.0 mL) was added 10% palladium-carbon (N.E. CHEMCAT NX type, 50% hydrous product, 13.0 mg) at room temperature, the system was replaced with hydrogen, and the mixture was stirred at the same temperature for 4 hr and 15 min. The catalyst was removed by filtration through Celite, and the filtrate was concentrated under reduced pressure to give a crude product (36.5 mg) as a colorless amorphous. To the obtained crude product was added ethyl acetate (about 2 mL), the product was pulverised by ultrasonic irradiation, and the solid was collected by filtration, and washed with ethyl acetate. This solid was dissolved in water (10 mL), and the solution was concentrated under reduced pressure to give a colorless amorphous (29.1 mg). To this was added ethyl acetate, this was pulverised by ultrasonic irradiation, and the solid was collected by filtration, and dried under reduced pressure to give Compound 4 (23.9 mg, yield 89%, purity 95.1%) as a white solid.

¹H-NMR(400MHz,D₂O)δ6.94-6.82(m,3H),4.41(s,1H),4.30-4.20(m,2H),3.91(dd,J=9.2,4.4Hz,1H),3.34(d,J=16.0Hz,1H),3.18(dd, J=13.6,4.4Hz,1H),2.94(dd,J=13.6,9.2Hz,1H),2.63(s,3H),2.30(t,J=8 .0Hz,2H),2.13-2.02(m,1H),1.93-1.83(m,1H),1.49(d,J=6.8Hz,3H).

### (Example 18: Synthesis of Compound 12)

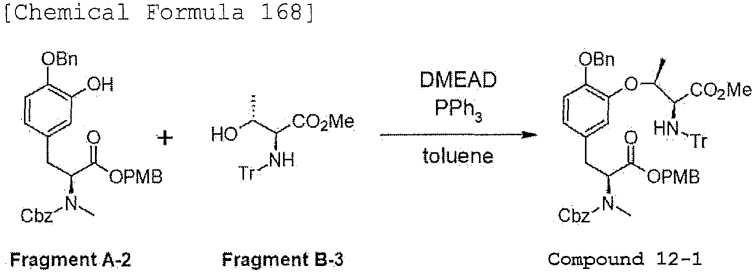

Under nitrogen atmosphere, to a solution of Fragment A-2 (1.0 eq., 1.10 g, containing 2.2wt% ethyl acetate, 1.94 mmol) and Fragment B-3 (1.5 eq., 1.15 g, containing 4.9wt% ethyl acetate, 2.92 mmol) in toluene (10 mL) were added triphenylphosphine (3.0 eq., 1.53 g, 5.83 mmol) and DMEAD (3.0 eq., 1.37 g, 5.83 mmol) at room temperature, and the mixture was stirred at the same temperature for 15 min, and then at the external temperature of 50°C for 2 hr. Additional Fragment B-3 (1.5 eq., 1.15 g, containing 4.9wt% ethyl acetate, 2.92 mmol), triphenylphosphine (0.75 eq., 383 mg, 1.46 mmol) and DMEAD (0.75 eq., 343 mg, 1.46 mmol) were added, the mixture was stirred at the same temperature for 15.5 hr, and additional triphenylphosphine (0.75 eq., 383 mg, 1.46 mmol) and DMEAD (0.75 eq., 343 mg, 1.46 mmol) was added again. Then, while stirring at the same temperature, Fragment B-3 (0.5 eq., 383 mg, containing 4.9wt% ethyl acetate, 0.97 mmol), triphenylphosphine (0.75 eq., 383 mg, 1.46 mmol) and DMEAD (0.75 eq., 343 mg, 1.46 mmol) were repeatedly added four times every 1.5 hr. Then, the mixture was stirred at the same temperature for 50 min, and then at room temperature for 15.5 hr, and the reaction solution was concentrated under reduced pressure to give a crude product (14.2 g) as an orange oil. The obtained crude product was purified by flash silica gel column (normal phase silica gel 120 g, hexane/ethyl acetate = 5/1 - 3/1) to give Compound 12-1 (1.61 g, containing impurities, apparent yield 91%) as a yellow oil.

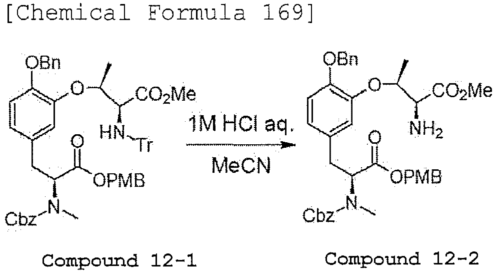

Under nitrogen atmosphere, to a solution of Compound 12-1 (1.0 eq., 1.61 g, calculated as 1.77 mmol) in acetonitrile (16 mL) was added 1M aqueous hydrochloric acid solution (1.5 eq., 2.65 mL, 2.65 mmol) under ice-cooling, the mixture was stirred at the same temperature for 1.5 hr, and additional 1M aqueous hydrochloric acid solution (0.5 eq., 0.88 mL, 0.88 mmol) was added. The mixture was stirred at room temperature for 15 min, 5% aqueous sodium hydrogencarbonate solution (50 mL) was added, and the mixture was extracted once with ethyl acetate (40 mL) and twice with ethyl acetate (15 mL) by liquid separation. The organic layers were combined, washed once with 5% aqueous sodium chloride solution (30 mL) by liquid separation, and dried over sodium sulfate. The sodium sulfate was removed by filtration, and the filtrate was concentrated under reduced pressure to give a crude product (1.49 g, quant.) as a yellow oil containing a white solid.

The crude product (254 mg) obtained from Compound 12-1 (257 mg) by the same procedure and the previous crude product were combined, and purified by flash silica gel column (normal phase silica gel 20 g, hexane/ethyl acetate = 2/1 - 1/1→chloroform/methanol=30/1) to give Compound 12-2 (958.1 mg, containing 3.5wt% ethyl acetate, conversion yield 67%, two steps from Fragment A-2) as a pale-yellow oil.

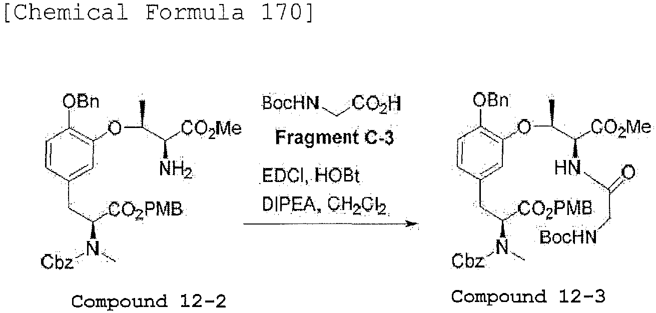

Under nitrogen atmosphere, to a solution of Compound 12-2 (1.0 eq., 958 mg, containing 3.5wt% ethyl acetate, 1.38 mmol) and N-Boc-L-glycine (Fragment C-3, 1.5 eq., 362 mg, 2.07 mmol) in dichloromethane (14 mL) were added DIPEA (2.0 eq., 0.48 mL, 2.76 mmol), HOBt·H₂O (1.5 eq., 317 mg, 2.07 mmol) and EDCI (1.5 eq., 396 mg, 2.07 mmol) under ice-cooling, and the mixture was stirred at room temperature for 17 hr. The reaction solution was concentrated under reduced pressure, to the concentrated residue were added 1M aqueous hydrochloric acid solution (15 mL) and water (10 mL), and the mixture was extracted once with ethyl acetate (35 mL) and twice with ethyl acetate (15 mL) by liquid separation. The organic layers were combined, washed once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give a crude product (1.18 g) as a pale-yellow oil. The crude product was purified twice by flash silica gel column (1st run: normal phase silica gel 25 g, hexane/ethyl acetate = 67/33 - 0/100→ethyl acetate/methanol = 50/50, 2nd run: NH₂ silica gel 25 g, hexane/ethyl acetate = 2/1 - 1/1) to give Compound 12-3 (1.13 g, containing 3.4wt% ethyl acetate, conversion yield 96%) as a colorless oil.

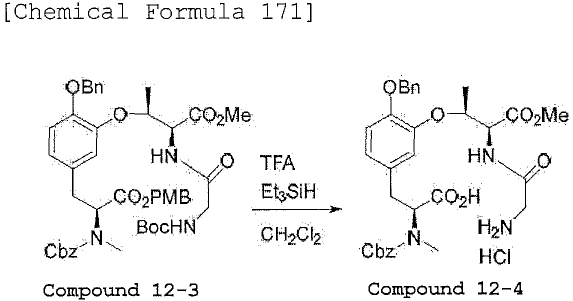

Under nitrogen atmosphere, to a solution of Compound 12-3 (1.0 eq., 1.13 g, containing 3.4wt% ethyl acetate, 1.31 mmol) in dichloromethane (11.8 mL) were added triethylsilane (25 eq., 5.23 mL, 32.8 mmol) and TFA (5.9 mL) under ice-cooling, the mixture was stirred at the same temperature for 10 min, and then at room temperature for 2.5 hr, and the reaction solution was concentrated under reduced pressure. To the obtained concentrated residue was added 0.05M hydrogen chloride/1,4-dioxane solution (37 mL), and the mixture was concentrated under reduced pressure. These procedures were repeated twice. 1,4-Dioxane (20 mL) was added, and the mixture was concentrated under reduced pressure. These procedures were repeated three times to give Compound 12-4 (3.02 g, containing 1,4-dioxane, quant.) as a pale-yellow oil.

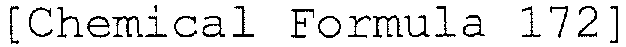

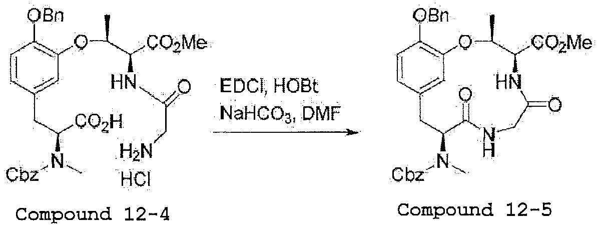

Under nitrogen atmosphere, to a solution of HOBt·H₂O (5.0 eq., 1.01 g, 6.57 mmol) and sodium hydrogencarbonate (10.0 eq., 1.10 g, 13.1 mmol) in DMF (1200 mL) was added EDCI (5.0 eq., 1.26 g, 6.57 mmol), and then a solution of Compound 12-4 (1.0 eq., 3.02 g, calculated as 1.31 mmol) in DMF (113 mL) was added dropwise over 2 hr at room temperature. The mixture was stirred at the same temperature for 21 hr, and the reaction solution was concentrated under reduced pressure at a bath temperature of 43°C. To the concentrated residue was added water (40 mL), and the mixture was extracted once with ethyl acetate (100 mL), and the aqueous layer was extracted three times with ethyl acetate (10 mL) by liquid separation. The organic layers were combined, washed once with 5% aqueous sodium chloride solution (50 mL) by liquid separation, and dried over sodium sulfate. The sodium sulfate was removed by filtration, and the filtrate was concentrated under reduced pressure to give a crude product (0.89 g) as a pale-orange oil. The crude product was purified by flash silica gel column (normal phase silica gel 25 g, hexane/ethyl acetate = 80/20 - 0/100) to give Compound 12-5 (107.3 mg, containing 1.6wt% ethyl acetate, conversion yield 14%) as a colorless oil.

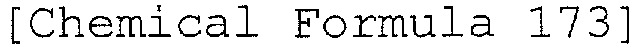

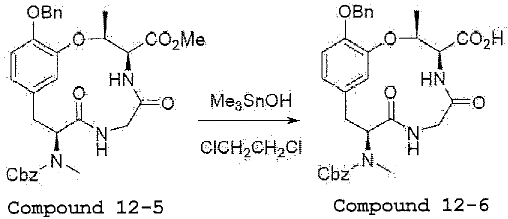

Under nitrogen atmosphere, to a solution of Compound 12-5 (1.0 eq., 107 mg, 0.18 mmol) in dichloroethane (3.4 mL) was added hydroxide trimethyltin (6.0 eq., 196 mg, 1.09 mmol) at room temperature, and the mixture was stirred at the external temperature of 80°C for 23 hr. After allowing to cool, 1M aqueous hydrochloric acid solution (20 mL) was added, and the mixture was extracted once with ethyl acetate (25 mL) and twice with ethyl acetate (10 mL) by liquid separation. The organic layers were combined, washed with 5% aqueous sodium chloride solution (20 mL) by liquid separation, and dried over sodium sulfate. The sodium sulfate was removed by filtration, and the filtrate was concentrated under reduced pressure to give Compound 12-6 (172.2 mg, containing impurities, quant.) as a colorless oil.

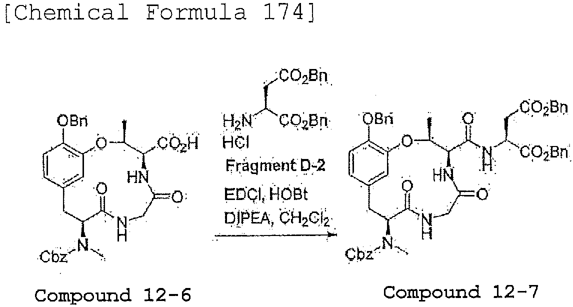

Under nitrogen atmosphere, to a solution of Compound 12-6 (1.0 eq., 154.7 mg, calculated as 0.17 mmol), dibenzyl L-aspartate hydrochloride (Fragment D-2, 2.0 eq., 119 mg, 0.34 mmol) and HOBt·H₂O (2.0 eq., 52 mg, 0.34 mmol) in dichloromethane (3.4 mL) were added DIPEA (2.0 eq., 59 µL, 0.34 mmol) and EDCI (2.0 eq., 65 mg, 0.34 mmol) under ice-cooling, and the mixture was stirred at the same temperature for 2 hr, and then at room temperature for 14.5 hr. To the reaction solution was added 1M aqueous hydrochloric acid solution (10 mL), and the mixture was extracted once with ethyl acetate (30 mL) and twice with ethyl acetate (10 mL) by liquid separation. The organic layers were combined, washed once with 5% aqueous sodium chloride solution (10 mL) by liquid separation, and dried over sodium sulfate. The sodium sulfate was removed by filtration, and the filtrate was concentrated under reduced pressure to give a crude product (239 mg) as a yellow oil.

The crude product (12 mg) obtained from Compound 12-6 (10 mg) by the same procedure and the previous crude product were combined, and purified twice by flash silica gel column (1st run: NH₂ silica gel 5 g, hexane/ethyl acetate = 2/3 - 1/3, 2nd run: NH₂ silica gel 20 g, hexane/ethyl acetate = 1/1 - 0/1) to give Compound 12-7 (70.2 mg, two-step yield from Compound 12-5 45%) as a colorless oil.

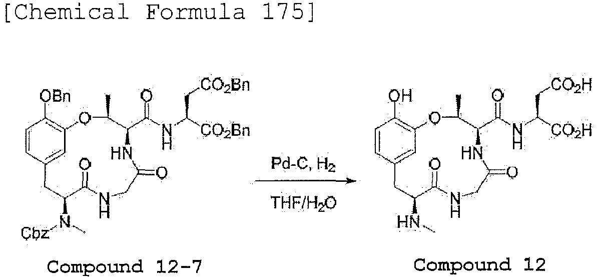

Under nitrogen atmosphere, to a solution of Compound 12-7 (1.0 eq., 68 mg, 0.078 mmol) in a mixture of THF (2.8 mL)/water (2.8 mL) was added 10% palladium-carbon (N.E. CHEMCAT NX type, 50% hydrous product, 17 mg) at room temperature, the system was replaced with hydrogen, and the mixture was stirred at the same temperature for 2 hr and 50 min. The catalyst was removed by filtration through Celite, and the filtrate was concentrated under reduced pressure to give a crude product (36.3 mg) as a pale-orange amorphous. To the obtained crude product was added ethyl acetate (about 2 mL), the product was pulverised by ultrasonic irradiation, and the solid was collected by filtration, and dried under reduced pressure to give Compound 12 (34.2 mg, yield 94%, purity 95.6%) as a pale-orange solid.

¹H-NMR(400MHz,D₂O)δ6.92-6.82(m,3H),4.50(dd,J=7.6,5.2Hz,1H),4.44(s,1H),4.25(d,J=15.6Hz, 1H),3.95(dd,J=8.8,4.8Hz,1H),3.34(d,J=15.6Hz,1H),3.20(dd,J=13.6, 4.8Hz,1H),2.97(dd,J=13.6,8.8Hz,1H),2.79(dd,J=16.4,5.2Hz,1H),2.7 1(dd,J=16.4,8.0Hz,1H),2.64(s,3H),1.51(d,J=6.8Hz,3H).

### (Example 19: Synthesis of Compound 7)

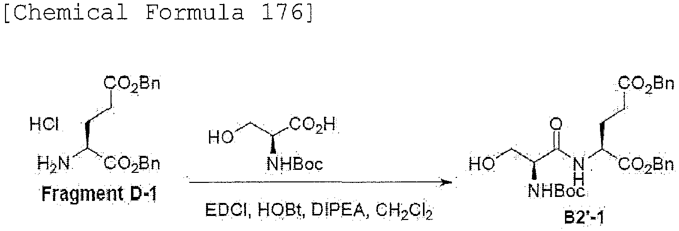

Under nitrogen atmosphere, to a solution of Fragment D-1 (2.61 g, 7.17 mmol, 1.0 eq.) and Boc-L-Ser-OH (1.62 g, 7.89 mmol, 1.1 eq.) in dichloromethane (30 mL) were added DIPEA (2.75 mL, 15.8 mmol, 2.2 eq.), HOBt·H₂O (1.32 g, 8.61 mmol, 1.2 eq.) and EDCI (1.65 g, 8.61 mmol, 1.2 eq.) under ice-cooling. The mixture was stirred at the same temperature for 15 min, and then at room temperature for 1 hr and 45 min, additional EDCI (413 mg, 2.15 mmol, 0.3 eq.) was added, and the mixture was stirred at room temperature for additional 45 min. To the reaction solution was added water (20 mL), and the mixture was extracted once with ethyl acetate (90 mL) and three times with ethyl acetate (10 mL) by liquid separation. The organic layers were combined, washed once with 1M aqueous hydrochloric acid solution (30 mL), once with 5% aqueous sodium hydrogencarbonate solution (30 mL) and once with 5% aqueous sodium chloride solution (30 mL) by liquid separation, and dried over sodium sulfate. The sodium sulfate was removed by filtration, and the filtrate was concentrated under reduced pressure to give B2'-1 (4.00 g, quant.) as a white solid.

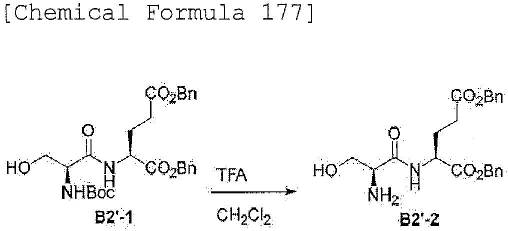

Under nitrogen atmosphere, to a solution of B2'-1 (4.00 g, calculated as 7.17 mmol, 1.0 eq.) in dichloromethane (15 mL) was added TFA (7.0 mL) at room temperature, the mixture was stirred at the same temperature for 1 hr and 20 min, and the reaction solution was concentrated under reduced pressure. The obtained concentrated residue was subjected to azeotropic distillation with toluene (20 mL) three times to give B2'-2 (4.65 g, quant.) as a pale-orange oil.

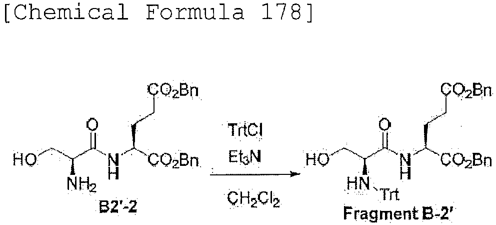

Under nitrogen atmosphere, to a solution of B2'-2 (4.65 g, calculated as 7.17 mmol, 1.0 eq.) in dichloromethane (15 mL) were added triethylamine (4.0 mL, 28.7 mmol, 4.0 eq.) and TrCl (2.20 g, 7.89 mmol, 1.1 eq.) under ice-cooling. The mixture was stirred at the same temperature for 40 min, to the reaction solution was added 5% aqueous sodium hydrogencarbonate solution (20 mL), and the mixture was extracted once with ethyl acetate (50 mL) and three times with ethyl acetate (10 mL) by liquid separation. The organic layers were combined, washed once with 5% aqueous sodium chloride solution (30 mL) by liquid separation, and dried over sodium sulfate. The sodium sulfate was removed by filtration, and the filtrate was concentrated under reduced pressure to give a crude product (5.23 g) as a pale-yellow viscous product. The crude product was purified by flash silica gel column (normal phase silica gel, 78 g, hexane/ethyl acetate = 3/1 - 3/2) to give Fragment B-2' (3.05 g, Net: 2.99 g, containing ethyl acetate: 2.0wt%, conversion yield 63%, three-step yield from Fragment D-1) as a white viscous product.

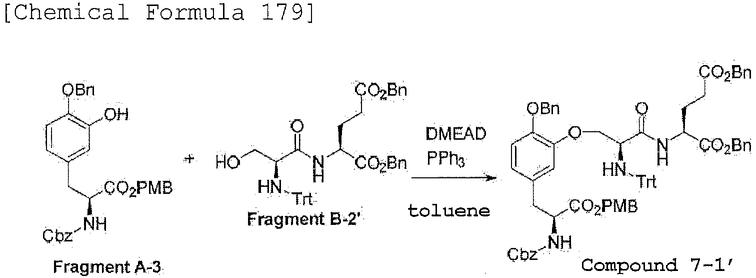

Under nitrogen atmosphere, to a solution of Fragment A-3 (1.0 eq., 1.99 g, 3.70 mmol) and Fragment B-2' (1.65 eq., 3.99 g, 6.11 mmol) in toluene (60 mL) were added triphenylphosphine (1.5 eq., 1.47 g, 5.55 mmol) and DMEAD (1.5 eq., 1.31 g, 5.55 mmol) at room temperature, and the mixture was stirred at the same temperature for 40 min. Additional triphenylphosphine (1.0 eq., 0.98 g, 3.74 mmol) and DMEAD (1.0 eq., 0.89 g, 3.80 mmol) were added, and the mixture was stirred at the same temperature for 1 hr and 20 min. Additional triphenylphosphine (0.5 eq., 0.48 g, 1.83 mmol) and DMEAD (0.5 eq., 0.44 g, 1.88 mmol) was added again, and the mixture was stirred at the same temperature for 40 min. Additional Fragment B-2' (0.20 eq., 0.49 g, 0.75 mmol) was added, and the mixture was stirred at the same temperature for 25 min. Additional Fragment B-2' (0.20 eq., 0.49 g, 0.75 mmol) was added again, and the mixture was stirred at the same temperature for 19 hr. Additional DMEAD (0.1 eq., 93 mg, 0.40 mmol) was added, and the mixture was stirred at the same temperature for 1 hr and 25 min. Additional triphenylphosphine (0.2 eq., 198 mg, 0.75 mmol) and DMEAD (0.2 eq., 177 mg, 0.76 mmol) were added, and the mixture was stirred at the same temperature for 45 min.

The reaction solution was concentrated under reduced pressure, and the concentrated residue was purified twice by flash silica gel column (normal phase silica gel 50 g, 1st run: hexane/ethyl acetate = 90/10 - 50/50, 2nd run: hexane/ethyl acetate = 75/25 - 50/50) to give Compound 7-1' (1.74 g, yield 40%) as a white solid.

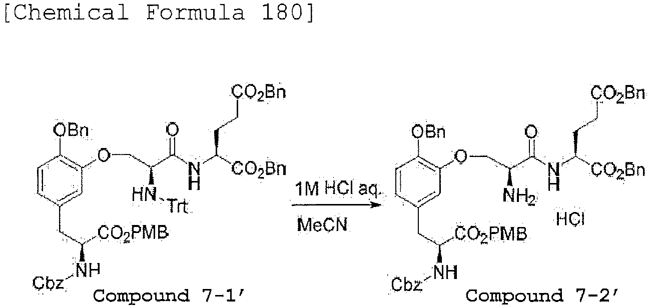

Under nitrogen atmosphere, to a solution of Compound 7-1' (1.0 eq., 1.74 g, 1.47 mmol) in acetonitrile (18 mL) was added 1M aqueous hydrochloric acid solution (1.1 eq., 1.62 mL, 1.62 mmol) at room temperature, and the mixture was stirred at the same temperature for 2 hr. The reaction solution was concentrated under reduced pressure, and the concentrated residue was purified by flash silica gel column (normal phase silica gel 50 g, chloroform/methanol = 100/0 - 90/10) to give Compound 7-2' (1.23 g, containing 1.4wt% methanol, conversion yield 33%, two-step yield from Fragment A-3) as a pale-yellow amorphous.

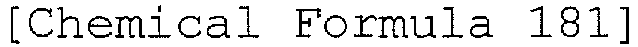

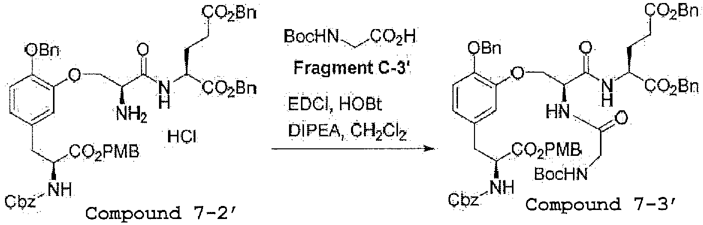

Under nitrogen atmosphere, to a solution of Compound 7-2' (1.0 eq., 0.61 g, 0.625 mmol) in dichloromethane (6 mL) were added DIPEA (3.0 eq., 0.327 mL, 1.88 mmol), N-Boc-L-glycine (Fragment C-3, 1.5 eq., 0.16 g, 0.938 mmol), HOBt•H₂O (1.5 eq., 0.14 g, 0.938 mmol) and EDCI (1.5 eq., 0.18 g, 0.938 mmol) under ice-cooling, and the mixture was stirred at room temperature for 2 hr. Additional EDCI (0.3 eq., 34 mg, 0.19 mmol) was added, and the mixture was stirred at the same temperature for 1 hr, and the reaction solution was concentrated under reduced pressure.

The reaction solution obtained by treating Compound 7-2' (71 mg) in the same manner and the previous concentrated residue were combined. To this mixture was added 1M aqueous hydrochloric acid solution (15 mL), and the mixture was extracted once with ethyl acetate (about 40 mL) and three times with ethyl acetate (15 mL) by liquid separation. The organic layers were combined, washed once with 5% aqueous sodium hydrogencarbonate solution (20 mL) and once with 5% aqueous sodium chloride solution (20 mL) by liquid separation, and dried over sodium sulfate. The sodium sulfate was removed by filtration, the filtrate was concentrated under reduced pressure, and the obtained concentrated residue was purified by flash silica gel column (NH₂ silica gel 15 g, hexane/ethyl acetate = 90/10 - 0/100) to give Compound 7-3' (706 mg, yield 86%) as a white solid.

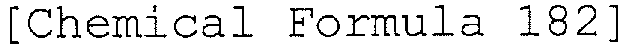

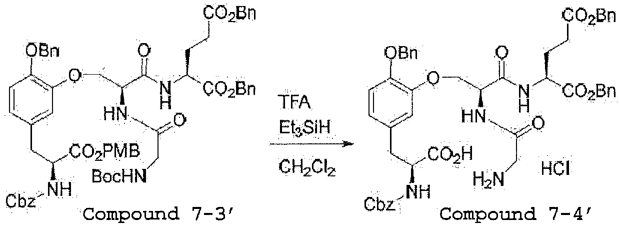

Under nitrogen atmosphere, to a solution of Compound 7-3' (1.0 eq., 0.683 g, 0.624 mmol) in dichloromethane (6.8 mL) were added triethylsilane (25 eq., 2.49 mL, 15.6 mmol) and TFA (3.4 mL) under ice-cooling, the mixture was stirred at room temperature for 50 min, and the reaction solution was concentrated under reduced pressure. To the obtained concentrated residue was added 0.05M hydrogen chloride/1,4-dioxane solution (20 mL), and the mixture was concentrated under reduced pressure. These procedures were repeated twice. 1,4-Dioxane (20 mL) was added, and the mixture was concentrated under reduced pressure. These procedures were repeated twice to give Compound 7-4' (700 mg, containing 1,4-dioxane, quant.) as a white solid.

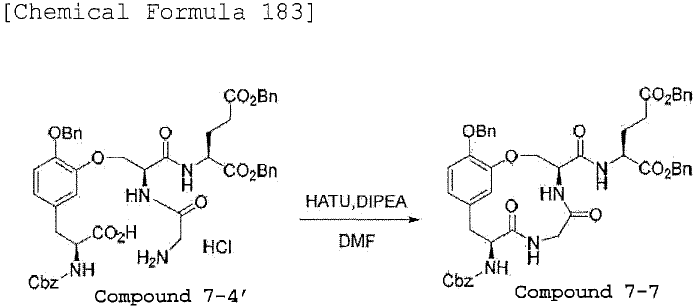

Under nitrogen atmosphere, to a solution of HATU (5.0 eq., 1.18 g, 3.10 mmol) in DMF (590 mL) was added DIPEA (10.0 eq., 1.08 mL, 6.20 mmol) at room temperature. Then, a solution of Compound 7-4' (1.0 eq., 0.700 g, calculated as 0.62 mmol) in DMF (14 mL) was added over 17.5 hr at the same temperature, and after the dropwise addition was completed, the mixture was stirred at room temperature for additional 2 hr and 50 min. The reaction solution was concentrated under reduced pressure at a bath temperature of 50°C, to the concentrated residue was added water (25 mL), and the mixture was extracted once with ethyl acetate (35 mL) and once with ethyl acetate (25 mL) by liquid separation. The organic layers were combined, washed once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give a crude product (866.4 mg) as a brown solid. The crude product was purified by flash silica gel column (NH₂ silica gel 22 g, chloroform/methanol = 100/0 - 97/3) to give a yellow solid. To this solid was added methanol, the solid was pulverised by ultrasonic irradiation, and collected by filtration to give White solid 1 (209 mg) and Filtrate 1.

Filtrate 1 was concentrated under reduced pressure, and the obtained solid was purified by flash silica gel column (reverse-phase silica gel 30 g, 0.1% aqueous TFA solution/0.05% TFA acetonitrile solution =60/40 - 20/80), and freeze-dried to give Compound 7-7 (106.9 mg, containing TFA).

White solid 1 was purified by flash silica gel column (reverse-phase silica gel 30 g, 0.1% TFA aqueous solution/0.05% TFA acetonitrile solution =60/40 - 20/80), and the obtained solid was collected by filtration, and washed with ethyl acetate (about 20 mL) to give Solid 2 and Filtrate 2. Solid 2 was dried under reduced pressure to give Compound 7-7 (109.0 mg, containing TFA) as a white solid.

The precipitated solid from Filtrate 2 was collected by filtration, and dried under reduced pressure to give Compound 7-7 (41.7 mg, containing TFA) as a white solid. Total yield 257.6 mg (apparent yield 47%, two-step yield from Compound 7-3')

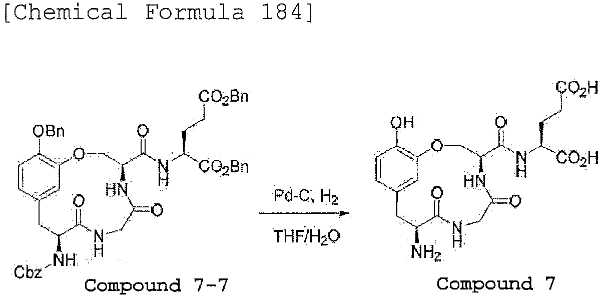

Under nitrogen atmosphere, to a solution of Compound 7-7 (1.0 eq., 77.5 mg, calculated as 0.080 mmol) in a mixture of THF (3.2 mL)/water (3.2 mL) was added 10% palladium-carbon (N.E. CHEMCAT NX type, 50% hydrous product, 19.4 mg) at room temperature, the system was replaced with hydrogen, and the mixture was stirred at the same temperature for 4 hr. The catalyst was removed by filtration through Celite, and the filtrate was concentrated under reduced pressure to give a crude product (43.9 mg) as a pale-yellow solid. To the obtained crude product was added ethyl acetate (about 2 mL), and the product was pulverised by ultrasonic irradiation, and the solid was collected by filtration, and dried under reduced pressure to give Compound 7 (40.6 mg, yield 90%, purity 99.1%) as a pale-yellow solid.

¹H-NMR (400MHz,D₂O)δ6.88-6.73(m,3H),4.58(dd,J=13.2,5.6Hz,1H),4.45(d,J=13.2Hz,1H),4.24(d, J=15.2Hz,1H),4.16(dd,J=8.4,4.8Hz,1H),3.99(dd,J=9.6,4.8Hz,1H),3. 26(d,J=15.2Hz,1H),3.17(dd,J=13.2,4.8Hz,1H),2.87(dd,J=13.2,9.2Hz ,1H),2.36-2.22(m,2H),2.11-2.00(m,1H),1.93-1.81(m,1H).

### (Example 20: Synthesis of Compound 8)

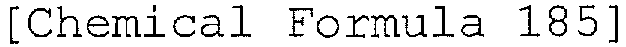

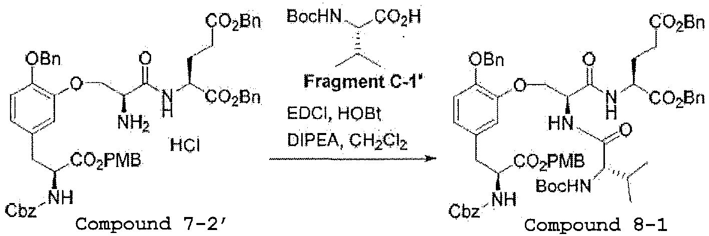

Under nitrogen atmosphere, to a solution of Compound 7-2' (1.0 eq., 620 mg, 0.636 mmol) and N-Boc-L-valine (Fragment C-1, 1.5 eq., 207 mg, 0.954 mmol) in dichloromethane (6.4 mL) were added DIPEA (3.0 eq., 332 µL, 1.91 mmol), HOBt·H₂O (1.5 eq., 146 mg, 0.954 mmol) and EDCI (1.5 eq., 183 mg, 0.954 mmol) under ice-cooling, and the mixture was stirred at room temperature for 2.5 hr. The reaction solution was concentrated under reduced pressure, to the concentrated residue were added 1M aqueous hydrochloric acid solution (10 mL) and water (6 mL), and the mixture was extracted once with ethyl acetate (20 mL) and three times with ethyl acetate (5 mL) by liquid separation. The organic layers were combined, washed once with 5% aqueous sodium hydrogencarbonate solution (10 mL) and once with 5% aqueous sodium chloride solution (10 mL) by liquid separation, and dried over sodium sulfate. The sodium sulfate was removed by filtration, and the filtrate was concentrated under reduced pressure to give a crude product (817 mg) as a white solid. The crude product was purified by flash silica gel column (NH₂ silica gel 20 g, hexane/ethyl acetate = 2/1 - 1/1) to give Compound 8-1 (636.8 mg, containing 1.7wt% ethyl acetate, conversion yield 87%) as a white solid.

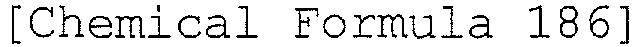

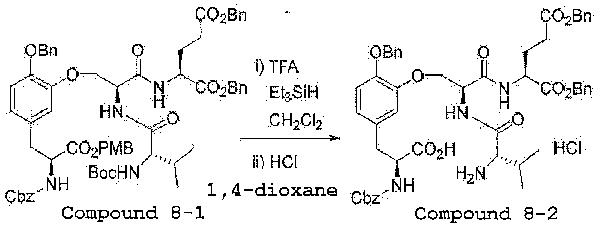

Under nitrogen atmosphere, to a solution of Compound 8-1 (1.0 eq., 636 mg, containing 1.7wt% ethyl acetate, 0.550 mmol) in dichloromethane (5.0 mL) were added triethylsilane (25 eq., 2.19 mL, 13.8 mmol) and TFA (2.5 mL) under ice-cooling, the mixture was stirred at the same temperature for 10 min, and then at room temperature for 1 hr and 45 min, and the reaction solution was concentrated under reduced pressure. To the obtained concentrated residue was added 0.05M hydrogen chloride/1,4-dioxane solution (20 mL), and the mixture was concentrated under reduced pressure. These procedures were repeated twice. 1,4-Dioxane (20 mL) was added, and the mixture was concentrated under reduced pressure. These procedures were repeated three times to give Compound 8-2 (1.4217 g, containing 1,4-dioxane, quant.) as a colorless oil.

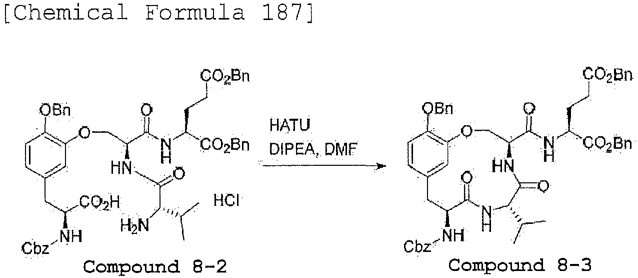

Under nitrogen atmosphere, to a solution of HATU (5.0 eq., 1.05 g, 2.75 mmol) in DMF (544 mL) was added DIPEA (10.0 eq., 958 µL, 5.50 mmol) at room temperature. Then, a solution of Compound 8-2 (1.0 eq., 1.41 g, calculated as 0.550 mmol) in DMF (6 mL) was added over 17.5 hr at the same temperature, and after the dropwise addition was completed, the mixture was stirred at room temperature for additional 2 hr. The reaction solution was concentrated under reduced pressure at a bath temperature of 50°C, to the concentrated residue were added ethyl acetate (35 mL) and water (15 mL), and the mixture was stirred at room temperature for 16 hr. The solid was collected by filtration, washed once with 5% aqueous sodium hydrogencarbonate solution (20 mL), once with water (20 mL) and once with ethyl acetate (20 mL), and the obtained solid was dried under reduced pressure to give a crude product (403.6 mg) as a pale-yellow solid. To the crude product was added THF (60 mL), and the product was dissolved by heating at a bath temperature of 60°C. The solution was filtered on hot, and the filtrate was concentrated to give Compound 8-3 (0.43 g, containing 5.0wt% BHT, conversion yield 83%) as a white solid.

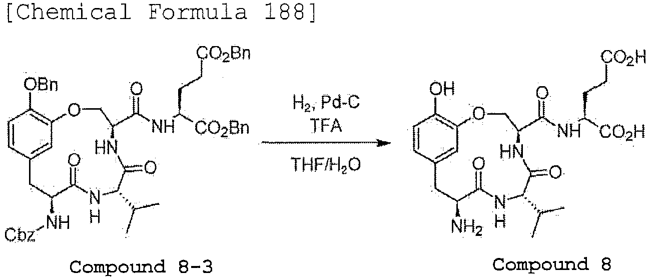

Under nitrogen atmosphere, to a solution of Compound 8-3 (1.0 eq., 211 mg, containing 5.0wt% BHT, 0.222 mmol) in a mixture of THF (9.0 mL)/water (9.0 mL) was added 10% palladium-carbon (N.E. CHEMCAT NX type, 50% hydrous product, 50 mg) at room temperature, the system was replaced with hydrogen, and the mixture was stirred at the same temperature for 1 hr and 40 min. Additional THF (9.0 mL) was added, and the mixture was stirred at the same temperature for 2 hr. TFA (2.0 eq., 34 µL, 0.444 mmol) was added, and the mixture was stirred again at the same temperature for 2 hr. The catalyst was removed by filtration through Celite, and the filtrate was concentrated under reduced pressure to give a crude product (151.7 mg) as a pale-red amorphous. To the obtained crude product was added ethyl acetate (about 2 mL), the product was pulverised by ultrasonic irradiation, and the solid was collected by filtration, and dried under reduced pressure to give a TFA salt of Compound 8 (129.6 mg, containing 1.8wt% ethyl acetate, conversion yield 94%, purity 98.6%) as a pale-yellow solid.

¹H-NMR(400MHz,DMSO-d6)δ9.09(brs,1H),8.51(d,J=9.6Hz,1H),8.13(d,J=7.6Hz,1H),8.10(d,J =7.6Hz,1H),6.75-6.65(m,2H),6.54(d,J=2.0Hz,1H),4.64-4.58(m,1H),4.48(dd,J=12.8,2.8Hz,1H),4.33-4.23(m,2H),4.16(t,J=9.6Hz,1H),3.88(dd,J=7.6,4.8Hz,1H),2.95(dd,J =13.6,4.4Hz,1H),2.78(dd,J=13.6,7.6Hz,1H),2.35-2.23(m,2H),2.06-1.77(m,3H),0.85(d,J=6.8Hz,3H),0.82(d,J=6.8Hz,3H).

### (Example 21: Synthesis of Compound 13)

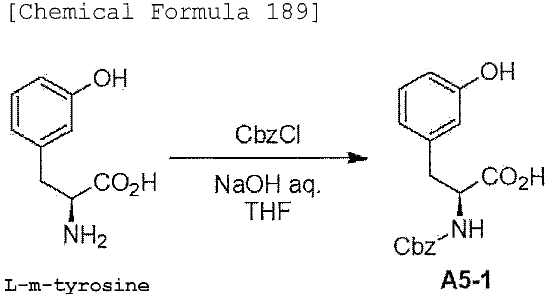

Under nitrogen atmosphere, to L-m-tyrosine (10.17 g, 56.1 mmol, 1.0 eq.) was added 1M aqueous sodium hydroxide solution (56 mL, 56 mmol, 1.0 eq.). Then, CbzCl (11.01 g, 64.6 mmol, 1.15 eq.) and 1M aqueous sodium hydroxide solution (65 mL, 65 mmol, 1.15 eq.) were added alternately over 6 min under ice-cooling. The mixture was stirred at the same temperature for 20 min, and then stirred at room temperature for 1 hr and 30 min, THF (50 mL) was added, and the mixture was stirred for 20 min. Additional 1M aqueous sodium hydroxide solution (28 mL, 28 mmol, 0.5 eq.) and CbzCl (1.92 g, 11.2 mmol, 0.2 eq.) were added at room temperature, and the mixture was stirred for 50 min. Additional CbzCl (0.96 g, 5.61 mmol, 0.1 eq.) was added again, and the mixture was stirred at the same temperature for 1 hr and 15 min. The reaction solution was ice-cooled, 5M aqueous sodium hydroxide solution (3.93 mL, 19.6 mmol, 0.35 eq.) and CbzCl (5.27 g, 30.9 mmol, 0.55 eq.) were added over 2 min, and the mixture was stirred at room temperature for 30 min. Then, 5M aqueous sodium hydroxide solution (11.2 mL, 56.1 mmol, 1.0 eq.) was added at room temperature, and the mixture was stirred for 35 min. Additional 5M aqueous sodium hydroxide solution (56.1 mL, 281 mmol,5.0 eq.) was added again, and the mixture was stirred for 1 hr. To the reaction solution was added hexane (200 mL), the mixture was washed once by liquid separation, and the aqueous layer was adjusted to pH 1 by addition of 6M aqueous hydrochloric acid solution (84 mL). The aqueous layer was extracted twice with ethyl acetate (20 mL) by liquid separation. The organic layers were combined, washed once with 5% aqueous sodium chloride solution (60 mL) by liquid separation, and dried over sodium sulfate. The sodium sulfate was removed by filtration, and the filtrate was concentrated to give A5-1 (21.84 g, Net: 17.91 g, containing ethyl acetate: 8.2wt%, BnOH: 9.1wt%, THF: 0.7wt%, quant.) as a white solid.

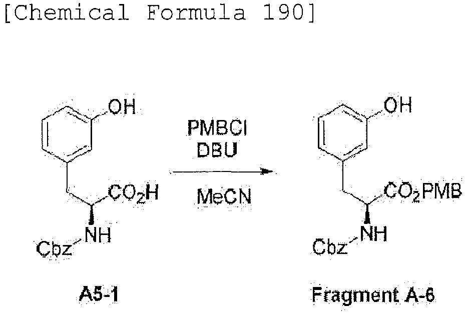

Under nitrogen atmosphere, to a solution of A5-1 (6.58 g, calculated as 16.9 mmol, 1.0 eq.) in acetonitrile (200 mL) were added DBU (2.78 mL, 18.6 mmol, 1.1 eq.) and PMBCl (2.53 mL, 18.6 mmol, 1.1 eq.) at room temperature, and the mixture was stirred at the external temperature of 60°C for 20 hr. Additional PMBCl (461 µL, 3.38 mmol, 0.2 eq.) and DBU (505 µL, 3.38 mmol, 0.2 eq.) were added, and the mixture was stirred at the same temperature for 1.5 hr. The reaction solution was allowed to cool, acetic acid (290 µL, 5.07 mmol, 0.3 eq.) was added, and the mixture was concentrated under reduced pressure to give a crude product. The crude product was purified twice by flash silica gel column (normal phase silica gel 100 g, 1st run: hexane/ethyl acetate = 75/25 - 0/100, 2nd run: hexane/ethyl acetate = 75/25 - 0/100) to give Fragment A-6 (5.61 g, Net: 5.25 g, ethyl acetate: 6.3wt%, conversion yield 71%, two-step yield from L-m-tyrosine) as a colorless oil.

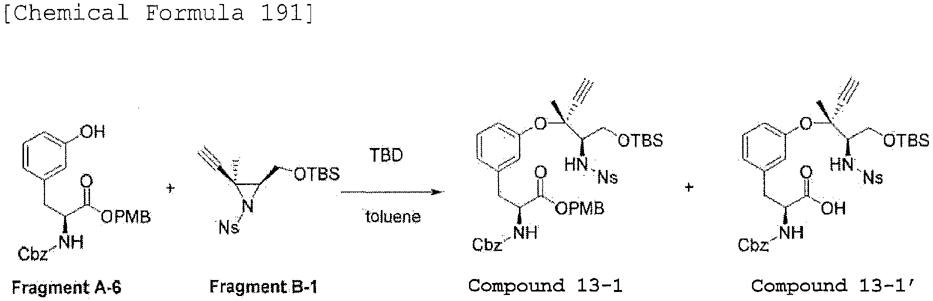

Under nitrogen atmosphere, to a solution of Fragment A-6 (1.20 eq., 1.83 g, containing 6.3wt% ethyl acetate, 3.95 mmol) and Fragment B-1 (1.0 eq., 1.35 g, 3.29 mmol) in toluene (36 mL) was added TBD (1.20 eq., 549 mg, 3.95 mmol) under ice-cooling, and the mixture was stirred at the external temperature of 50°C for 3 hr. The mixture was warmed to the external temperature of 60°C, and stirred for 25 hr, additional TBD (0.5 eq., 229 mg, 1.64 mmol) was added, and the mixture was stirred at the same temperature for additional 1.5 hr, and allowed to cool. To the reaction solution was added 5% aqueous citric acid solution (27 mL), and the mixture was extracted once with ethyl acetate (20 mL) and twice with ethyl acetate (10 mL) by liquid separation. The organic layers were combined, washed once with 5% aqueous sodium chloride solution (30 mL) by liquid separation, and dried over sodium sulfate. The sodium sulfate was removed by filtration, and the filtrate was concentrated under reduced pressure to give a crude product (3.90 g) as an orange oil. The crude product was purified by flash silica gel column (normal phase silica gel 100 g, hexane/ethyl acetate = 80/20 - 0/100→ethyl acetate/methanol=60/40). This was combined with Compound 13-1' (201 mg) obtained from Fragment B-1 (150 mg) to give a mixture of Compound 13-1 and Compound 13-1' (2.62 g, containing ethyl acetate and small amounts of impurities, Compound 13-1:Compound 13-1' = approximately 2:1, apparent yield Compound 13-1: 59%, Compound 13-1': 30%) as an orange oil.

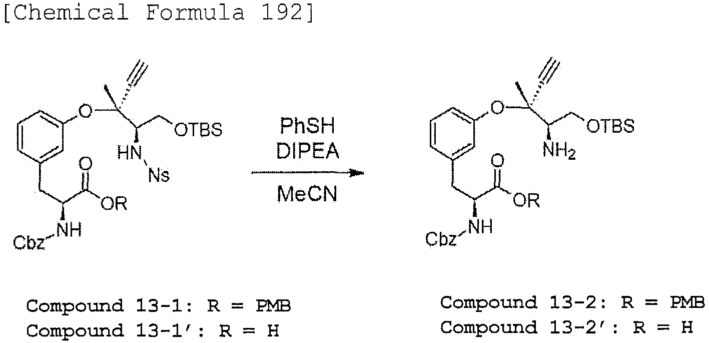

Under nitrogen atmosphere, to a solution of a mixture of Compound 13-1 and Compound 13-1' (2.33 g, calculated as Compound 13-1:Compound 13-1' = 2:1, total: 2.89 mmol) in acetonitrile (12 mL) were added thiophenol (3.0 eq., 883 µL, 8.66 mmol) and DIPEA (3.0 eq., 1.51 mL, 8.66 mmol) at room temperature, and the mixture was stirred at the same temperature for 30 min. Additional thiophenol (3.0 eq., 883 µL, 8.66 mmol) and DIPEA (3.0 eq., 1.51 mL, 8.66 mmol) were added, the mixture was stirred at the same temperature for additional 2 hr, and the reaction solution was concentrated under reduced pressure to give a crude product (4.71 g) as a brown oil. The crude product (261 mg) obtained from Compound 13-1 and Compound 13-1' (300 mg) in the same manner and the previous crude product were combined, and purified by flash silica gel column (normal phase silica gel 50 g, hexane/ethyl acetate = 80/20 - 0/100→ethyl acetate/methanol = 90/10 - 60/40) to give a mixture of Compound 13-2 and Compound 13-2' (1.78 g, containing impurities, Compound 13-2:Compound 13-2' = approximately 1:1, apparent yield Compound 13-2: 45%, Compound 13-2': 45%) as a brown oil.

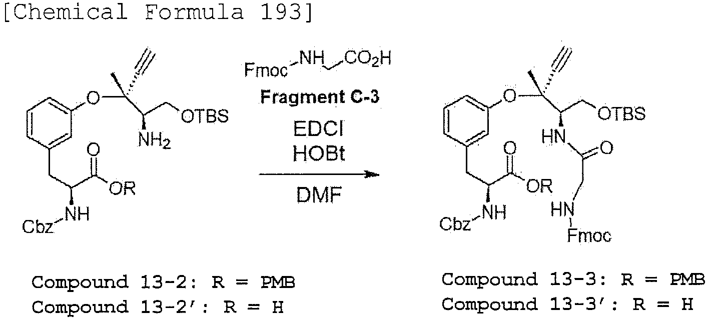

Under nitrogen atmosphere, to a solution of N-Fmoc-L-glycine (Fragment C-3, 2.0 eq., 780 mg, 2.62 mmol) in DMF (15 mL) were added EDCI (2.0 eq., 503 mg, 2.62 mmol) and HOBt·H₂O (2.0 eq., 402 mg, 2.62 mmol), and the mixture was stirred at room temperature for 1 hr. A mixture of Compound 13-2 and Compound 13-2' (1.0 eq., 790 mg, calculated as Compound 13-2:Compound 13-2' = 1:1, total: 1.31 mmol) was added, and the mixture was stirred at the same temperature for 2 hr.

The reaction solution obtained from a mixture (100 mg) of Compound 13-2 and Compound 13-2' under similar conditions and the previous reaction solution were combined, 5% aqueous sodium hydrogencarbonate solution (50 mL) was added, and the mixture was extracted once with ethyl acetate (50 mL) and once with ethyl acetate (20 mL) by liquid separation. The organic layers were combined, washed once with 5% aqueous citric acid solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (1.46 g) as a brown viscous product. The crude product was purified by flash silica gel column (normal phase silica gel 50 g, hexane/ethyl acetate = 80/20 - 0/100-ethyl acetate/methanol = 80/20) to give a mixture of Compound 13-3 and Compound 13-3' (1.28 g, containing impurities, Compound 13-3:Compound 13-3' = approximately 1:1, apparent yield Compound 13-3: 49%, Compound 13-3': 49%) as a pale-brown viscous product.

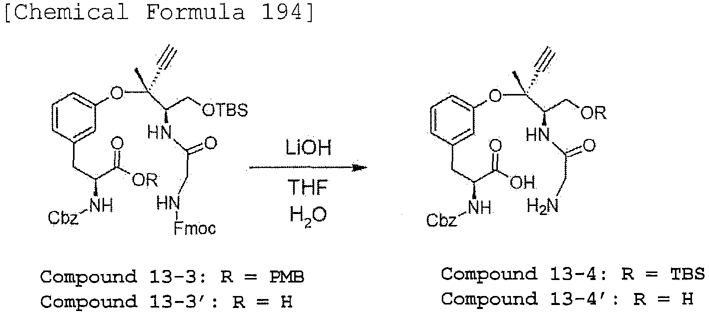

Under nitrogen atmosphere, to a solution of a mixture of Compound 13-3 and Compound 13-3' (1.0 eq., 1.28 g, calculated as Compound 13-3:Compound 13-3'=1:1, total: 1.45 mmol) in a mixture of THF (14 mL)/water (7 mL) was added lithium hydroxide monohydrate (4.0 eq., 244 mg, 5.82 mmol) at room temperature. The mixture was stirred at the same temperature for 2 hr, additional lithium hydroxide monohydrate (2.0 eq., 122 mg, 2.91 mmol) was added, and the mixture was stirred at the same temperature for 1 hr. The reaction solution was ice-cooled, and adjusted to pH 3 by addition of 5% aqueous citric acid solution (30 mL). The mixture was extracted once with ethyl acetate (30 mL) and twice with ethyl acetate (10 mL) by liquid separation, and the organic layers were combined, and washed once with 5% aqueous sodium chloride solution (30 mL) by liquid separation. The aqueous layers were combined, sodium chloride was added until the layer was saturated, and the mixture was extracted five times with THF (10 mL) by liquid separation. The organic layers were combined, and concentrated under reduced pressure to give a yellow oil (2.03 g) contain a solid. To this was added THF (100 mL), the insoluble substance was removed by filtration, and the filtrate was concentrated under reduced pressure to give a crude product (1.70 g) as an orange oil. The crude product was purified by flash silica gel column (normal phase silica gel 25 g, ethyl acetate/methanol = 100/0 - 5/95) to give a mixture of Compound 13-4 and Compound 13-4' (0.62 g, containing impurities, Compound 13-4:Compound 13-4' = approximately 1:2, apparent yield Compound 13-4: 27%, Compound 13-4': 55%) as a pale-orange viscous product.

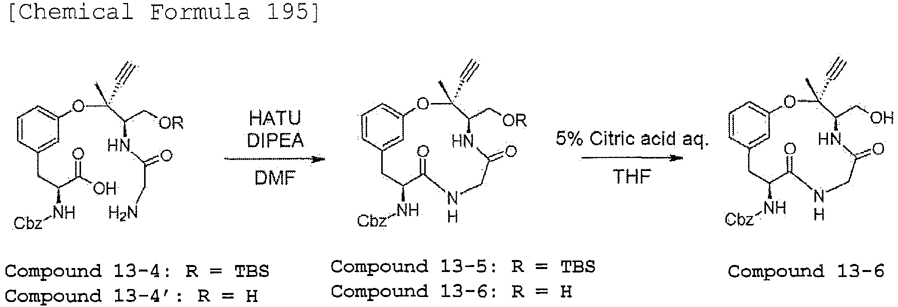

Under nitrogen atmosphere, to a solution of HATU (5.0 eq., 2.26 g, 5.94 mmol) in DMF (1190 mL) was added DIPEA (10.0 eq., 2.07 mL, 11.88 mmol) at room temperature. Then, a solution of a mixture of Compound 13-4 and Compound 13-4' (1.0 eq., 0.62 g, calculated as Compound 13-4:Compound 13-4'=1:2, total: 1.19 mmol) in DMF (15 mL) was added over 18 hr at the same temperature, and after the dropwise addition was completed, the mixture was stirred at room temperature for additional 1 hr. The reaction solution was concentrated under reduced pressure at a bath temperature of 50°C, to the concentrated residue was added 5% aqueous citric acid solution (30 mL), and the mixture was extracted once with ethyl acetate (50 mL) and four times with ethyl acetate (10 mL) by liquid separation. The organic layers were combined, and washed once with saturated aqueous sodium chloride solution (30 mL) by liquid separation, and dried over sodium sulfate. The sodium sulfate was removed by filtration, and the filtrate was concentrated under reduced pressure to give a mixture of Compound 13-5 and Compound 13-6 (2.32 g) as a brown solid.

Under nitrogen atmosphere, to a solution of a mixture (2.32 g) of Compound 13-5 and Compound 13-6 in THF (10 mL) was added 5% aqueous citric acid solution (10 mL) at room temperature, and the mixture was stirred at the same temperature for 2 hr. To the reaction solution were added ethyl acetate (30 mL) and saturated aqueous sodium chloride solution (40 mL), and the mixture was extracted once by liquid separation. The organic layer was dried over sodium sulfate, the sodium sulfate was removed by filtration, and the filtrate was concentrated under reduced pressure to give a crude product (2.70 g) as a brown solid. To the crude product was added ethyl acetate, the insoluble substance was removed by filtration, and the filtrate was concentrated under reduced pressure. The obtained concentrated residue was purified twice by flash silica gel column (1st run: normal phase silica gel 50 g, hexane/ethyl acetate = 20/80 - 0/100→ethyl acetate/methanol = 85/15, 2nd run: reverse-phase silica gel 60 g, water/acetonitrile = 90/10 - 0/100) to give Compound 13-6 (0.22 g, containing small amounts of impurities and an isomer, apparent yield 40%) as an orange viscous product.

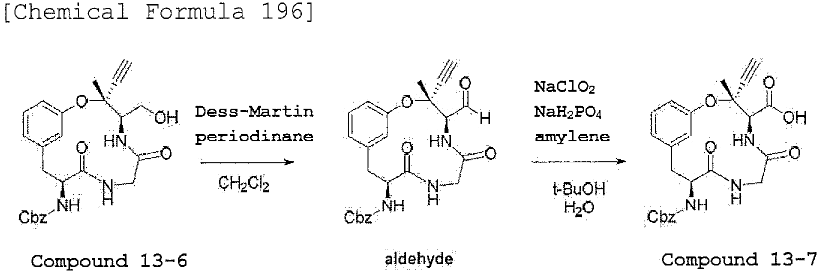

Under nitrogen atmosphere, to a solution of Compound 13-6 (1.0 eq., 0.22 g, an isomer mixture, 0.473 mmol) in dichloromethane (10 mL) was added Dess-Martin periodinane (1.5 eq., 0.30 g, 0.710 mmol) under ice-cooling, and the mixture was stirred at room temperature for 6 hr. The reaction solution was ice-cooled, 5% aqueous sodium hydrogencarbonate solution (10 mL) and 5% aqueous sodium thiosulfate solution (10 mL) were added, and the mixture was extracted once with ethyl acetate (30 mL) and twice with ethyl acetate (10 mL) by liquid separation. The organic layers were combined, washed once with saturated aqueous sodium chloride solution (30 mL) by liquid separation, and dried over sodium sulfate. The sodium sulfate was removed by filtration, and the filtrate was concentrated under reduced pressure to give Aldehyde (0.23 g, containing an isomer) as an orange oil.

Under nitrogen atmosphere, to a solution of Aldehyde (1.0 eq., 0.23 g, an isomer mixture, calculated as 0.473 mmol) in a mixture of t-butyl alcohol (13 mL)/amylene (3.2 mL)/water (3.2 mL) were added sodium dihydrogen phosphate dihydrate (3.5 eq., 258 mg, 1.66 mmol) and 80% sodium chlorite (4.5 eq., 241 mg, 2.13 mmol) at room temperature, and the mixture was stirred at the same temperature for 2.5 hr. To the reaction solution was added water (30 mL), and the mixture was extracted once with ethyl acetate (30 mL) and twice with ethyl acetate (10 mL) by liquid separation. Sodium chloride (10 g) was dissolved in the aqueous layer, and the mixture was extracted again three times with THF (10 mL) by liquid separation. The organic layers were combined, and dried over sodium sulfate. The sodium sulfate was removed by filtration, and the filtrate was concentrated under reduced pressure to give Compound 13-7 (0.30 g, an isomer mixture, containing impurities, quant.) as an orange oil.

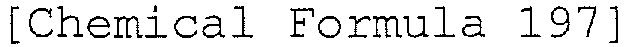

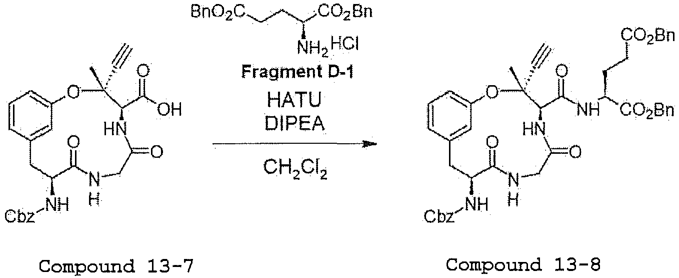

Under nitrogen atmosphere, to a solution of Compound 13-7 (1.0 eq., 0.30 g, an isomer mixture, calculated as 0.473 mmol) and dibenzyl L-glutamate hydrochloride (Fragment D-1, 1.5 eq., 258 mg, 0.710 mmol) in dichloromethane (9 mL) were added DIPEA (1.5 eq., 124 µL, 0.710 mmol) and HATU (1.5 eq., 270 mg, 0.710 mmol) under ice-cooling, and the mixture was stirred at the same temperature for 3.5 hr, and then at room temperature for 19.5 hr. To the reaction solution was added 5% aqueous citric acid solution (10 mL), and the mixture was extracted once with ethyl acetate (25 mL) and twice with ethyl acetate (5 mL) by liquid separation. The organic layers were combined, washed once with 5% aqueous sodium hydrogencarbonate solution (20 mL), once with 5% aqueous citric acid solution (20 mL) and once with 5% aqueous sodium chloride solution (20 mL) by liquid separation, and dried over sodium sulfate. The sodium sulfate was removed by filtration. The filtrate was concentrated under reduced pressure. The crude product (0.46 g) obtained as a brown viscous product was purified by flash column silica gel as follows to give an orange oil (80 mg).
1st run: normal phase silica gel 10 g, hexane/ethyl acetate = 60/40 - 0/100
2nd run: normal phase silica gel 25 g, hexane/ethyl acetate = 60/40 - 0/100
3rd run: normal phase silica gel 25 g, hexane/ethyl acetate = 60/40 - 45/55
4th run: normal phase silica gel 25 g, hexane/ethyl acetate = 50/50 - 47/53
5th run: reverse-phase silica gel 60 g, 0.05% TFA aqueous solution/0.05% TFA acetonitrile solution =40/60 - 1/99

To the obtained orange oil were added ethyl acetate (20 mL) and water (20 mL), and the mixture was extracted once by liquid separation. The organic layer was dried over sodium sulfate, the sodium sulfate was removed by filtration, and the filtrate was concentrated under reduced pressure to give Compound 13-8 (67 mg, yield 18%) as an orange oil.

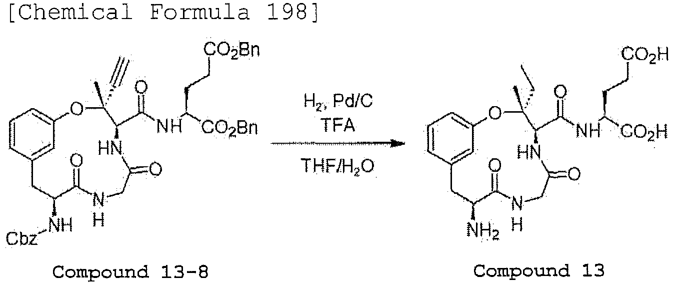

Under nitrogen atmosphere, to a solution of Compound 13-8 (1.0 eq., 63 mg, 0.08 mmol) in a mixture of THF (3.2 mL)/water (0.32 mL) were added TFA (2.0 eq., 12.2 µL, 0.16 mmol) and 10% palladium-carbon (N.E. CHEMCAT NX type, 50% hydrous product, 3.2 mg) at room temperature, the system was replaced with hydrogen, and the mixture was stirred at the same temperature for 24 hr. Additional 10% palladium-carbon (N.E. CHEMCAT NX type, 50% hydrous product, 3.2 mg) was added, and the mixture was stirred at the same temperature for additional 23 h. The catalyst was removed by filtration through Celite, and the filtrate was concentrated under reduced pressure to give a crude product (73 mg) as a pale-yellow viscous product. The crude product was purified by flash silica gel column (reverse-phase silica gel 60 g, 0.05% aqueous TFA solution/0.05% TFA acetonitrile solution = 95/5 - 79/21), and freeze-dried to give a TFA salt of Compound 13 (20.8 mg, yield 54%, purity 97.1%) as a white solid.

¹H-NMR(400MHz,D₂O)δ8.67-8.60(m,0.3H),7.66(d,J=7.6Hz,0.4H),7.29(t,J=8.0Hz,1H),7.15(s,1H) ,7.10-7.00(m,2H),6.82-6.68(m,0.4H),4.56(t,J=4.4Hz,1H),4.39(dd,J=8.0,4.0Hz,1H),4.21(d, J=15.6Hz,1H),4.01(dd,J=10.4,4.4Hz,1H),3.32(d,J=15.6Hz,1H),3.23 dd,J=13.2,4.4Hz,1H),2.95(dd,J=13.2,10.4Hz,1H),2.47-2.34(m,2H),2.21-2.10(m,1H),2.01-1.87(m,2H),1.73-1.62(m,1H),1.39(s,3H),0.90(t,J=7.6Hz,3H).

### (Example 22: Synthesis of Compound 14)

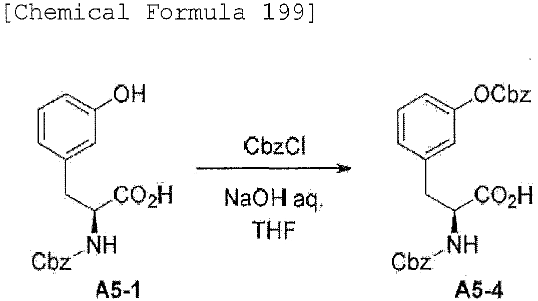

Under nitrogen atmosphere, to a solution of A5-1 (3.00 g, calculated as 7.71 mmol, 1.0 eq.) in THF (6 mL) was added 1M aqueous sodium hydroxide solution (16.3 mL, 16.3 mmol, 2.1 eq.). Then, CbzCl (1.20 mL, 8.51 mmol, 1.1 eq.) was added under ice-cooling, and the mixture was stirred at room temperature for 20 min. To the reaction solution were added water (10 mL) and hexane (30 mL), and the mixture was washed once by liquid separation. The aqueous layer was adjusted to pH 1 by addition of 2M aqueous hydrochloric acid solution (5 mL), and extracted once with ethyl acetate (30 mL) and twice with ethyl acetate (10 mL) by liquid separation. The organic layers were combined, washed once with 5% aqueous sodium chloride solution (20 mL) by liquid separation, and dried over sodium sulfate. The sodium sulfate was removed by filtration, and the filtrate was concentrated to give A5-4 (3.99 g, quant.) as a white solid.

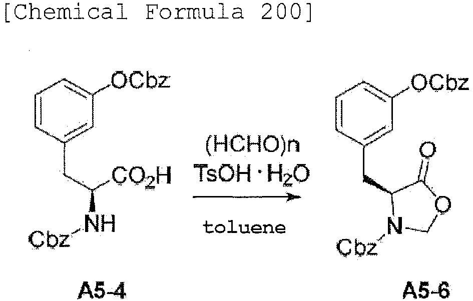

Under nitrogen atmosphere, to a solution of A5-4 (3.49 g, calculated as 6.77 mmol, 1.0 eq.) in toluene (100 mL) were added paraformaldehyde (1.75 g) and TsOH·H₂O (129 mg, 0.68 mmol, 0.1 eq.), and the mixture was stirred at the external temperature of 125°C for 1 hr. The reaction solution was allowed to cool, and 5% aqueous sodium hydrogencarbonate solution (20 mL) was added.

This was combined with the reaction solution obtained by treating A5-4 (0.5 g) in the same manner, and extracted once with ethyl acetate (60 mL) and twice with ethyl acetate (5 mL) by liquid separation. The organic layers were combined, washed once with 5% aqueous sodium chloride solution (30 mL) by liquid separation, and dried over sodium sulfate. The sodium sulfate was removed by filtration, the filtrate was concentrated, and the concentrated residue was purified by flash silica gel column (normal phase silica gel 100 g, hexane/ethyl acetate = 85/15 - 0/100) to give A5-6 (3.57 g, Net: 3.42 g, containing ethyl acetate: 4.3wt%, conversion yield 96%, three-step yield from L-m-tyrosine) as a colorless oil.

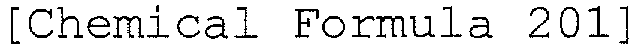

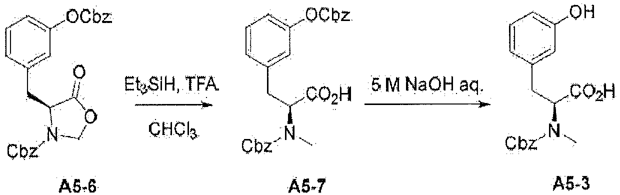

Under nitrogen atmosphere, to a solution of A5-6 (3.50 g, Net: 3.35 g, 7.26 mmol, 1.0 eq.) in chloroform (36 mL) was added triethylsilane (3.63 mL) at room temperature. TFA (36 mL) was added under ice-cooling, and the mixture was stirred at the same temperature for 5 min, and then at room temperature for 47.5 hr. The reaction solution was concentrated, and the concentrated residue (9.89 g) was diluted with THF (20 mL), 5M aqueous sodium hydroxide solution (20 mL) was added under ice-cooled, and the mixture was stirred at the same temperature for 30 min, and then at room temperature for 6 hr.

The reaction solution obtained by treating A5-6 (46 mg) in the same manner and the previous reaction solution were combined, and adjusted to pH 4 by addition of citric acid (4.7 g). Water (20 mL) was added, and the mixture was extracted once with ethyl acetate (25 mL) and twice with ethyl acetate (15 mL) by liquid separation. The organic layers were combined, washed once with 5% aqueous sodium chloride solution (30 mL) by liquid separation, and dried over sodium sulfate. The sodium sulfate was removed by filtration, and the filtrate was concentrated. To the obtained white solid was added toluene, and the solid was pulverised by ultrasonic irradiation, collected by filtration, and dried under reduced pressure to give A5-3 (2.00 g, containing impurities, apparent yield 83%, two-step yield) as a white solid.

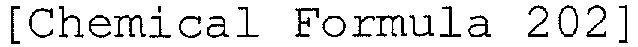

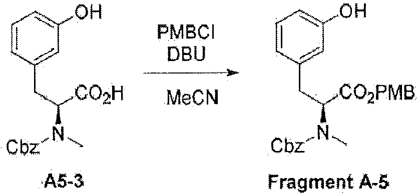

Under nitrogen atmosphere, to a solution of A5-3 (1.75 g, calculated as 5.31 mmol, 1.0 eq.) in acetonitrile (64 mL) were added DBU (872 µL, 5.85 mmol, 1.1 eq.) and PMBCl (796 µL, 5.85 mmol, 1.1 eq.) at room temperature, and the mixture was stirred at the external temperature of 60°C for 16 hr and 20 min. Additional PMBCl (145 µL, 1.06 mmol, 0.2 eq.) was added, the mixture was stirred at the same temperature for 3 hr and 30 min, and allowed to cool, and acetic acid (30 µL, 0.53 mmol, 0.1 eq.) was added.

The reaction solution obtained by treating A5-3 (0.25 g) in the same manner and the previous reaction solution were combined, and concentrated under reduced pressure. The concentrated residue was purified by flash silica gel column (normal phase silica gel 100 g, hexane/ethyl acetate = 80/20 - 30/70) to give Fragment A-5 (1.67 g, Net: 1.59 g, ethyl acetate: 4.6wt%, conversion yield 48%, three-step yield from A5-6) as a colorless oil.

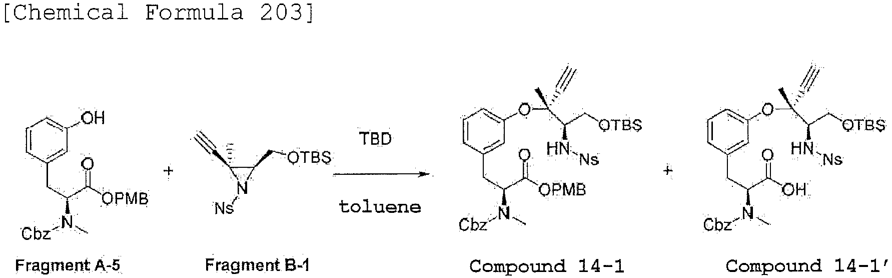

Under nitrogen atmosphere, to a solution of Fragment A-5 (1.05 eq., 1.49 g, containing 4.6wt% ethyl acetate, 3.16 mmol) and Fragment B-1 (1.0 eq., 1.24 g, 3.01 mmol) in toluene (33 mL) was added TBD (1.05 eq., 440 mg, 3.16 mmol) at room temperature, and the mixture was stirred at the external temperature of 50°C for 17 hr. Additional TBD (0.5 eq., 229 mg, 1.64 mmol) was added, and the mixture was stirred at the same temperature for additional 6 hr and 20 min, and allowed to cool.

The reaction solution obtained by treating Fragment B-1 (150 mg) in the same manner and the previous reaction solution were combined, 5% aqueous citric acid solution (25 mL) was added, and the mixture was extracted once with ethyl acetate (40 mL) and twice with ethyl acetate (10 mL) by liquid separation. The organic layers were combined, washed once with 5% aqueous sodium chloride solution (50 mL) by liquid separation, and dried over sodium sulfate. The sodium sulfate was removed by filtration, and the filtrate was concentrated under reduced pressure to give a crude product (3.86 g) as an orange oil. The crude product was purified by flash silica gel column (normal phase silica gel 50 g, hexane/ethyl acetate = 75/25 - 0/100→ethyl acetate/methanol=60/40) to give a mixture of Compound 14-1 and Compound 14-1' (2.64 g, containing ethyl acetate and small amounts of impurities, Compound 14-1:Compound 14-1' = approximately 2:1, apparent yield Compound 14-1: 64%, Compound 14-1': 32%) as a brown oil.

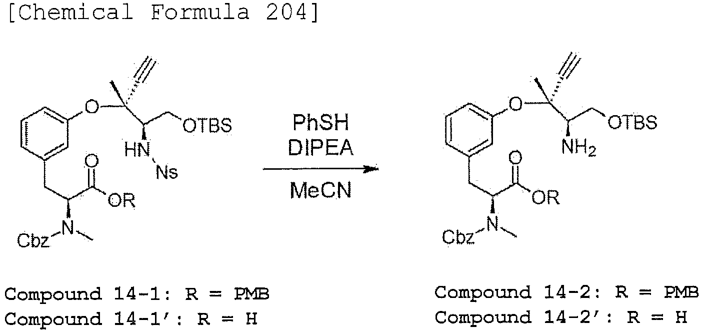

Under nitrogen atmosphere, to a solution of a mixture of Compound 14-1 and Compound 14-1' (2.64 g, calculated as Compound 14-1:Compound 14-1' = 2:1, total: 3.22mmol) in acetonitrile (14 mL) were added thiophenol (3.0 eq., 985 µL, 9.66 mmol) and DIPEA (3.0 eq., 1.68 mL, 9.66 mmol) at room temperature, and the mixture was stirred at the same temperature for 1 hr and 30 min. Additional thiophenol (3.0 eq., 985 µL, 9.66 mmol) and DIPEA (3.0 eq., 1.68 mL, 9.66 mmol) were added, the mixture was stirred at the same temperature for additional 3 hr, and the reaction solution was concentrated under reduced pressure to give a crude product (5.43 g) as a brown oil. The crude product was purified by flash silica gel column (normal phase silica gel 50 g, hexane/ethyl acetate = 80/20 - 0/100→ethyl acetate/methanol = 90/10 - 60/40) to give a mixture of Compound 14-2 and Compound 14-2' (2.02 g, containing small amounts of impurities, Compound 14-2:Compound 14-2' = approximately 2:1, apparent yield Compound 14-2: 65%, Compound 14-2': 33%) as an orange oil.

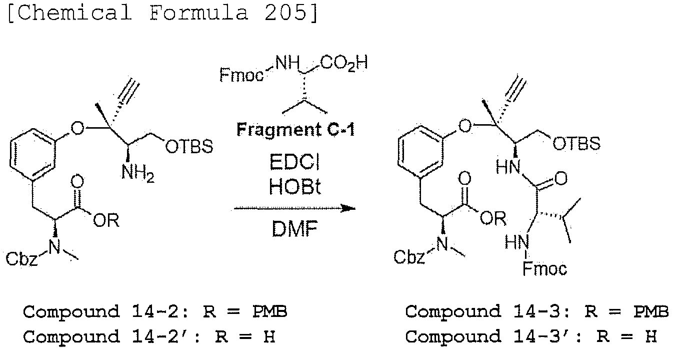

Under nitrogen atmosphere, N-Fmoc-L-valine (Fragment C-1, 2.0 eq., 1.08 g, 3.18 mmol), EDCI (2.0 eq., 610 mg, 3.18 mmol) and HOBt·H₂O (2.0 eq., 487 mg, 3.18 mmol) were dissolved in DMF (17 mL), and the solution was stirred at room temperature for 1 hr and 10 min. A mixture of Compound 14-2 and Compound 14-2' (1.0 eq., 1.01 g, calculated as Compound 14-2:Compound 14-2' = 2:1, total: 1.59 mmol) was added, and the mixture was stirred at the same temperature for 3 hr and 30 min. To the reaction solution was added 5% aqueous sodium hydrogencarbonate solution (50 mL), and the mixture was extracted once with ethyl acetate (100 mL) and once with ethyl acetate (10 mL) by liquid separation. The organic layers were combined, washed once with 5% aqueous citric acid solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (2.08 g) as an orange oil. The crude product was purified by flash silica gel column (normal phase silica gel 50 g, hexane/ethyl acetate = 80/20 - 0/100→ethyl acetate/methanol = 80/20) to give a mixture of Compound 14-3 and Compound 14-3' (1.71 g, containing impurities, Compound 14-3:Compound 14-3' = approximately 2:1, apparent yield quantitative) as a white viscous product.

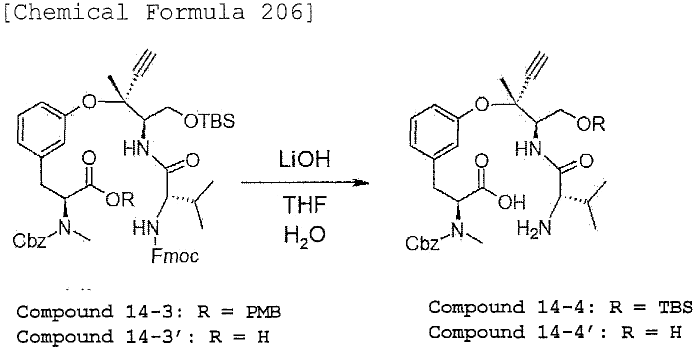

Under nitrogen atmosphere, to a solution of a mixture of Compound 14-3 and Compound 14-3' (1.0 eq., 1.71 g, calculated as 1.59 mmol) in a mixture of THF (15 mL)/water (7 mL) was added lithium hydroxide monohydrate (6.0 eq., 400 mg, 9.54 mmol) at room temperature, and the mixture was stirred at the same temperature for 21 hr. The reaction solution was ice-cooled, 5% aqueous citric acid solution (30 mL) and sodium chloride (10 g) were added, and the mixture was extracted once with ethyl acetate (30 mL), twice with ethyl acetate (10 mL) and twice with THF (10 mL) by liquid separation. The organic layers were combined, and dried over sodium sulfate. The sodium sulfate was removed by filtration, and the filtrate was concentrated under reduced pressure to give a crude product (1.72 g) as a yellow oil containing a solid. To this was added THF (100 mL), the insoluble substance was removed by filtration, and the filtrate was concentrated under reduced pressure to give a crude product (1.61 g) as a yellow oil. The crude product was purified by flash silica gel column (normal phase silica gel 25 g, hexane/ethyl acetate = 20/80 - 0/100→ethyl acetate/methanol=5/95) to give a mixture of Compound 14-4 and Compound 14-4' (0.74 g, containing impurities, Compound 14-4:Compound 14-4' = approximately 1:1, apparent yield Compound 14-4: 39%, Compound 14-4': 39%) as a white viscous product.

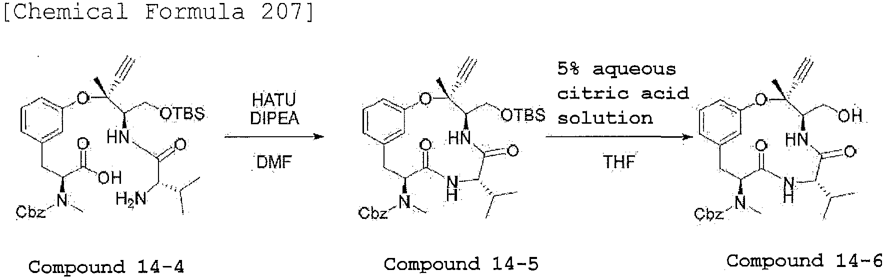

Under nitrogen atmosphere, to a solution of HATU (5.0 eq., 2.36 g, 6.20 mmol) in DMF (1225 mL) was added DIPEA (10.0 eq., 2.16 mL, 12.40 mmol) at room temperature. Then, a solution of a mixture of Compound 14-4 and Compound 14-4' (1.0 eq., 0.74 g, calculated as Compound 14-4:Compound 14-4'=1:1, total: 1.24 mmol) in DMF (15 mL) was added over 18 hr at the same temperature, and after the dropwise addition was completed, the mixture was stirred at room temperature for additional 4.5 hr. The reaction solution was concentrated under reduced pressure at a bath temperature of 50°C to give a mixture (3.80 g) of Compound 14-5 and Compound 14-6 as a brown solid.

Under nitrogen atmosphere, to a solution of a mixture (3.80 g) of Compound 14-5 and Compound 14-6 in THF (15 mL) was added 5% aqueous citric acid solution (10 mL) at room temperature, and the mixture was stirred at the same temperature for 45.5 hr. To the reaction solution was added saturated aqueous sodium chloride solution (40 mL), and the mixture was extracted once with ethyl acetate (50 mL) and twice with ethyl acetate (10 mL) by liquid separation. The organic layers were combined, and dried over sodium sulfate, the sodium sulfate was removed by filtration, and the filtrate was concentrated under reduced pressure to give a crude product (3.44 g) as a brown solid. To the crude product was added ethyl acetate, the insoluble substance was removed by filtration, and the filtrate was concentrated under reduced pressure. The concentrated residue was purified by flash silica gel column as follows to give Compound 14-6 (0.32 g, containing small amounts of impurities and an isomer, apparent yield 49%) as a pale-yellow viscous product.
1st run: normal phase silica gel 50 g, hexane/ethyl acetate = 65/35 - 0/100→ethyl acetate/methanol = 90/10
2nd run: reverse-phase silica gel 60 g, water/acetonitrile = 90/10 - 0/100
3rd run: reverse-phase silica gel 60 g, water/acetonitrile = 90/10 - 0/100

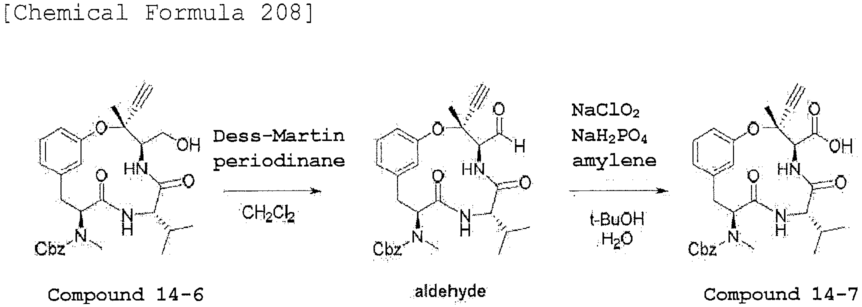

Under nitrogen atmosphere, to a solution of Compound 14-6 (1.0 eq., 0.32 g, an isomer mixture, 0.613 mmol) in dichloromethane (13 mL) was added Dess-Martin periodinane (1.5 eq., 0.39 g, 0.920 mmol) under ice-cooling, and the mixture was stirred at room temperature for 2 hr and 30 min. The reaction solution was ice-cooled, 5% aqueous sodium hydrogencarbonate solution (10 mL) and 5% aqueous sodium thiosulfate solution (10 mL) were added, and the mixture was extracted once with ethyl acetate (30 mL) and twice with ethyl acetate (10 mL) by liquid separation. The organic layers were combined, washed once with 5% aqueous sodium chloride solution (10 mL) by liquid separation, and dried over sodium sulfate. The sodium sulfate was removed by filtration, and the filtrate was concentrated under reduced pressure to give Aldehyde (0.32 g, containing an isomer) as a yellow oil.

Under nitrogen atmosphere, to a solution of Aldehyde (1.0 eq., 0.32 g, an isomer mixture, calculated as 0.613 mmol) in a mixture of t-butyl alcohol (17 mL)/amylene (4.1 mL)/water (4.1 mL) were added sodium dihydrogen phosphate dihydrate (3.5 eq., 335 mg, 2.15 mmol) and 80% sodium chlorite (4.5 eq., 312 mg, 2.76 mmol) at room temperature, and the mixture was stirred at the same temperature for 50 min. To the reaction solution was added saturated aqueous sodium chloride solution (30 mL), the mixture was extracted once with ethyl acetate (30 mL) and twice with ethyl acetate (10 mL) by liquid separation, and the organic layers were combined, and dried over sodium sulfate. The sodium sulfate was removed by filtration, and the filtrate was concentrated under reduced pressure to give Compound 14-7 (0.34 g, an isomer mixture, containing impurities, two-step apparent yield from Compound 14-6 quantitative) as a pale-yellow viscous product.

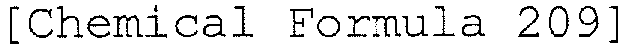

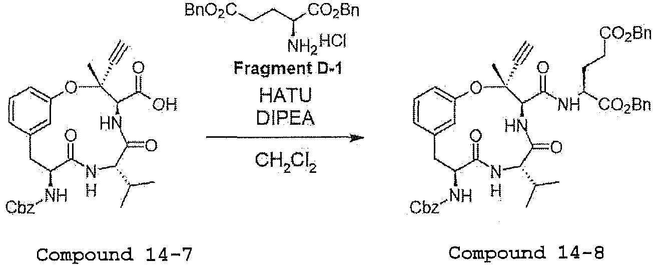

Under nitrogen atmosphere, to a solution of Compound 14-7 (1.0 eq., 0.34 g, an isomer mixture, calculated as 0.613 mmol) and dibenzyl L-glutamate hydrochloride (Fragment D-1, 1.5 eq., 335 mg, 0.920 mmol) in dichloromethane (13 mL) were added DIPEA (1.5 eq., 160 µL, 0.920 mmol) and HATU (1.5 eq., 350 mg, 0.920 mmol) under ice-cooling, and the mixture was stirred at the same temperature for 1 hr and 15 min, and then at room temperature for 4 hr and 15 min.

To the reaction solution was added 5% aqueous citric acid solution (15 mL), and the mixture was extracted once with ethyl acetate (30 mL) and twice with ethyl acetate (10 mL) by liquid separation. The organic layers were combined, washed once with 5% aqueous sodium hydrogencarbonate solution (30 mL), once with 5% aqueous citric acid solution (30 mL) and once with 5% aqueous sodium chloride solution (30 mL) by liquid separation, and dried over sodium sulfate. The sodium sulfate was removed by filtration, and the filtrate was concentrated under reduced pressure. The obtained crude product (0.60 g) as a red-brown oil was purified by flash silica gel column as follows to give Compound 14-8 (78 mg, containing 1.2wt% ethyl acetate, conversion yield 15%) as a colorless viscous product.
1st run: normal phase silica gel 25 g, chloroform/ethyl acetate = 90/10 - 60/40
2nd run: NH₂ silica gel 23 g, hexane/ethyl acetate = 50/50 - 80/20
3rd run: normal phase silica gel 25 g, chloroform/ethyl acetate = 85/15 - 50/50
4th run: normal phase silica gel 25 g, chloroform/ethyl acetate = 80/20 - 0/100
5th run: normal phase silica gel 25 g, chloroform/ethyl acetate = 75/25 - 70/30
6th run: normal phase silica gel 10 g, chloroform/ethyl acetate = 75/25 - 60/40
7th run: normal phase silica gel 10 g, chloroform/ethyl acetate = 78/22 - 40/60

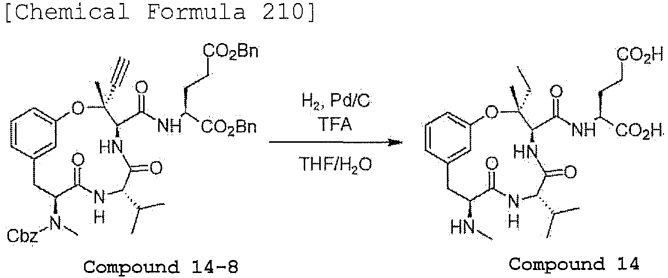

Under nitrogen atmosphere, to a solution of Compound 14-8 (1.0 eq., 78 mg, containing 1.2wt% ethyl acetate, 0.091 mmol) in a mixture of THF (3.9 mL)/water (0.39 mL) were added TFA (2.0 eq., 14.0 µL, 0.182 mmol) and 10% palladium-carbon (N.E. CHEMCAT NX type, 50% hydrous product, 3.9 mg) at room temperature, the system was replaced with hydrogen, and the mixture was stirred at the same temperature for 4 hr. Additional 10% palladium-carbon (N.E. CHEMCAT NX type, 50% hydrous product, 3.9 mg) was added, and the mixture was stirred at the same temperature for additional 3 hr. The catalyst was removed by filtration through Celite, and the filtrate was concentrated under reduced pressure to give a crude product (68 mg) as a pale-yellow viscous product. The crude product was purified by flash silica gel column (reverse-phase silica gel 60 g, 0.05% aqueous TFA solution/0.05% TFA acetonitrile solution = 95/5 - 73/27), and freeze-dried to give a TFA salt of Compound 14 (42.6 mg, yield 72%, purity 99.6%) as a white solid.

¹H-NMR(40OMHz,D₂O)58.55(d,J=7.2Hz,0.1H)8.41(d,J=8.0Hz,0.4H)7.24 t,J=8.0Hz,1H),7.06(dd,J=8.0,2.0Hz,1H),6.93(d,J=8.0Hz,1H),6.61(s ,1H),4.32(dd,J=9.2,5.2Hz,1H),4.10(d,J=10.4Hz,1H),3.87(dd,J=10.8 ,5.2Hz,1H),3.21(dd,J=12.4,5.2Hz,1H),2.84(t,J=12.4Hz,1H),2.64(s, 3H),2.43-2.25(m,2H),2.27-2.07(m,2H),1.95-1.68(m,3H),1.36(s,3H),1.00(t,J=7.6Hz,3H),0.77(d,J=6.8Hz,3H),0.6 9(d,J=6.4Hz,3H)

### (Example 23: Synthesis of Compound 16)

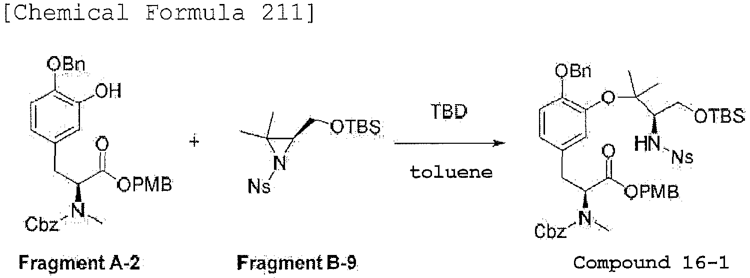

Under nitrogen atmosphere, to a solution of Fragment A-2 (1.05 eq., 1.00 g, containing 1.9wt% ethyl acetate and 0.3wt% acetic acid, 1.76 mmol) and Fragment B-9 (1.0 eq., 671 mg, 1.68 mmol) in toluene (18 mL) was added TBD (1.05 eq., 245 mg, 1.76 mmol) at room temperature, and the mixture was stirred at the external temperature of 50°C for 2 hr and 20 min. Additional TBD (0.3 eq., 70 mg, 0.503 mmol) was added, and the mixture was stirred at the same temperature for 15.5 hr. Additional TBD (0.2 eq., 47 mg, 0.335 mmol) was added again, and the mixture was stirred at the same temperature for additional 4 hr, and allowed to cool. To the reaction solution was added 5% aqueous citric acid solution (20 mL), and the mixture was extracted once with ethyl acetate (20 mL) and twice with ethyl acetate (5 mL) by liquid separation. The organic layers were combined, washed once with 5% aqueous sodium chloride solution (20 mL) by liquid separation, and dried over sodium sulfate. The sodium sulfate was removed by filtration, and the filtrate was concentrated under reduced pressure to give a crude product (1.928 g) as a yellow oil. The crude product was purified by flash silica gel column (normal phase silica gel 25 g, hexane/ethyl acetate = 80/20 - 0/100) to give a mixture of Compound 16-1 and a de-PMB product of Compound 16-1 (1.537 g, containing impurities, Compound 16-1:de-PMB product = approximately 2:1, apparent yield quantitative) as a colorless oil.

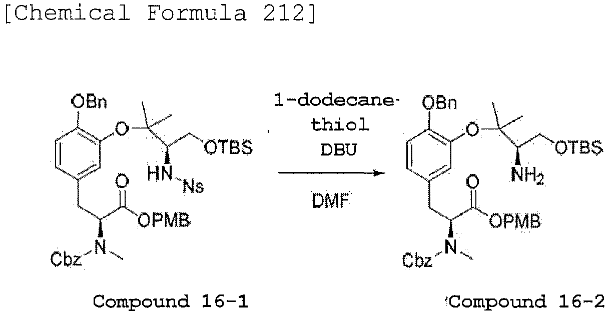

Under nitrogen atmosphere, to a solution of a mixture of Compound 16-1 and a de-PMB product of Compound 16-1 (1.0 eq., 1.53 g, calculated as 1.67 mmol) in DMF (7.65 mL) was added 1-dodecanethiol (6.0 eq., 2.38 mL, 10.0 mmol) at room temperature, and the mixture was ice-cooled. DBU (6.0 eq., 1.50 mL, 10.0 mmol) was added, and the mixture was stirred at room temperature for 1.5 hr. To the reaction solution were added ethyl acetate (40 mL), 5% aqueous citric acid solution (13.4 mL) and water (100 mL), and the mixture was extracted once by liquid separation. To the aqueous layer was added additional 5% aqueous citric acid solution (1 mL), and the aqueous layer was extracted three times with ethyl acetate (10 mL) by liquid separation. The organic layers were combined, washed once with 5% aqueous sodium hydrogencarbonate solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (3.43 g) as a yellow oil.

The crude product (505 mg) obtained from Compound 16-1 (219 mg) by the same procedure and the previous crude product were combined, and purified by flash silica gel column (normal phase silica gel 50 g, hexane/ethyl acetate = 70/30 - 0/100→ethyl acetate/methanol=60/40 - 20/80) to give a mixture of Compound 16-2 and a de-PMB product of Compound 16-2 (1.272 g, containing impurities, Compound 16-2:de-PMB product = approximately 2:1, Compound 16-2: apparent yield 61%, de-PMB product: apparent yield 30%) as a yellow oil.

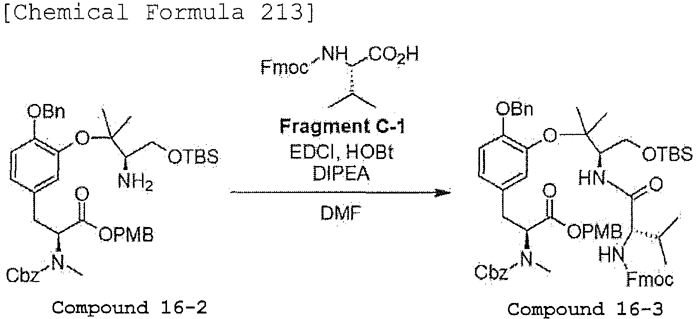

Under nitrogen atmosphere, N-Fmoc-L-valine (Fragment C-1, 2.0 eq., 1.18 g, 3.48 mmol), EDCI (2.0 eq., 667 mg, 3.48 mmol) and HOBt·H₂O (2.0 eq., 533 mg, 3.48 mmol) were dissolved in DMF (20 mL), and the solution was stirred at room temperature for 1 hr. A solution of a mixture of Compound 16-2 and a de-PMB product of Compound 16-2 (1.0 eq., 1.272 g, Compound 16-2:de-PMB product=2:1, calculated as 1.74 mmol) in DMF (2 mL) was added, and the mixture was stirred at the same temperature for 1 hr and 50 min, warmed to the external temperature of 40°C, stirred for 1 hr and 20 min, cooled to room temperature, and stirred for 50 min. A solution of N-Fmoc-L-valine (Fragment C-1, 1.0 eq., 591 mg, 1.74 mmol), EDCI (1.0 eq., 334 mg, 1.74 mmol) and HOBt·H₂O (1.0 eq., 267 mg, 1.74 mmol) in DMF (10 mL), which was separately stirred for 30 min, was added. The mixture was stirred at room temperature for 30 min, DIPEA (0.2 eq., 61 µL, 0.35 mmol) was added, and the mixture was stirred at the same temperature for 20 min. DIPEA (0.5 eq., 152 µL, 0.87 mmol) was added again, and the mixture was stirred at the same temperature for 40 min. To the reaction solution was added 5% aqueous sodium hydrogencarbonate solution (50 mL), and the mixture was extracted once with toluene (90 mL) and twice with ethyl acetate (15 mL) by liquid separation. The organic layers were combined, washed once with 5% aqueous citric acid solution (50 mL), once with 5% aqueous sodium hydrogencarbonate solution (50 mL) and once with 5% aqueous sodium chloride solution (50 mL) by liquid separation, and dried over sodium sulfate. The sodium sulfate was removed by filtration, and the filtrate was concentrated under reduced pressure to give Compound 16-3 (2.94 g, containing impurities, apparent yield quantitative) as a brown viscous product.

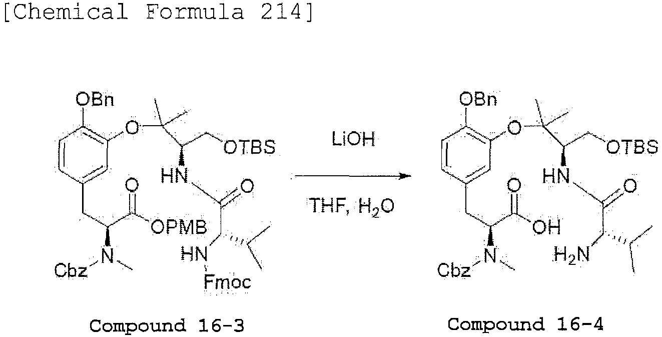

Under nitrogen atmosphere, to a solution of Compound 16-3 (1.0 eq., 2.75 g, calculated as 1.55 mmol) in a mixture of THF (24 mL)/water (12 mL) was added lithium hydroxide monohydrate (10 eq., 650 mg, 15.5 mmol) at room temperature. The mixture was stirred at the same temperature for 16.5 hr, and the reaction solution was ice-cooled, and adjusted to pH 3 by addition of 5% aqueous citric acid solution (43 mL). Sodium chloride (14 g) was dissolved therein, ethyl acetate (25 mL) and THF (10 mL) were added, and the mixture was extracted once by liquid separation. The aqueous layer was extracted three times with THF (15 mL) by liquid separation, and the organic layers were combined, washed once with saturated aqueous sodium chloride solution (50 mL) by liquid separation, and dried over sodium sulfate. The sodium sulfate was removed by filtration, and the filtrate was concentrated under reduced pressure to give a crude product (2.49 g) as a pale-orange oil.

The crude product (0.16 g) obtained from Compound 16-3 (0.32 g) under similar conditions and the previous crude product were combined, THF (50 mL) was added, and the insoluble substance was removed by filtration. The filtrate was concentrated under reduced pressure, to the concentrated residue was added ethyl acetate, the insoluble substance was removed by filtration again, and the filtrate was concentrated under reduced pressure to give a crude product (2.72 g) as an orange oil. The crude product was purified four times by flash silica gel column (normal phase silica gel 25 g, 1st run: hexane/ethyl acetate = 80/20 - 0/100→ethyl acetate/methanol = 30/70, 2nd run: ethyl acetate/methanol = 100/0 - 30/70, 3rd run: chloroform/methanol = 100/0 - 30/70, 4th run: chloroform/methanol = 100/0 - 50/50) to give Compound 16-4 (543 mg, containing 4.7wt% ethyl acetate, four-step conversion yield from Fragment B-9 36%) as a pale-brown viscous product, and a de-TBS product of Compound 16-4 (232 mg, containing 3.9wt% ethyl acetate, four-step conversion yield from Fragment B-9 18%) as a brown viscous product. 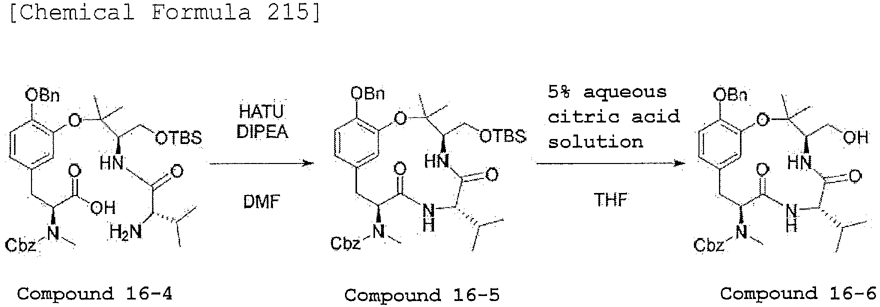 Under nitrogen atmosphere, to a solution of HATU (5.0 eq., 1.31 g, 3.44 mmol) in DMF (680 mL) was added DIPEA (10.0 eq., 1.20 mL, 6.89 mmol) at room temperature. Then, a solution of Compound 16-4 (1.0 eq., 542 mg, containing 4.7wt% ethyl acetate, 0.689 mmol) in DMF (10 mL) was added over 16.5 hr at the same temperature, and after the dropwise addition was completed, the mixture was stirred at room temperature for additional 1 hr. The reaction solution was concentrated under reduced pressure at a bath temperature of 50°C to give a mixture (5.749 g) of Compound 16-5 and Compound 16-6 as a brown oil.

Under nitrogen atmosphere, a mixture (5.749 g) of Compound 16-5 and Compound 16-6 was stirred for 3.5 hr at the external temperature of 70°C, and allowed to cool to room temperature. THF (15 mL) and 5% aqueous citric acid solution (10 mL) were added, and the mixture was stirred at the same temperature for 17 hr. To the reaction solution were added ethyl acetate (50 mL) and water (30 mL), and the mixture was extracted once by liquid separation. To the aqueous layer was added water (80 mL), and the mixture was extracted twice with ethyl acetate (20 mL) by liquid separation. The organic layers were combined, washed once with 5% aqueous sodium hydrogencarbonate solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (0.62 g) as a brown oil. The crude product was purified by flash silica gel column (normal phase silica gel 25 g, hexane/ethyl acetate = 60/40 - 20/80) to give Compound 16-6 (164 mg, containing 5.4wt% ethyl acetate, two-step conversion yield 36%) as a yellow viscous product.

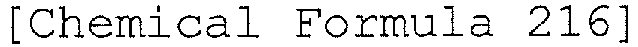

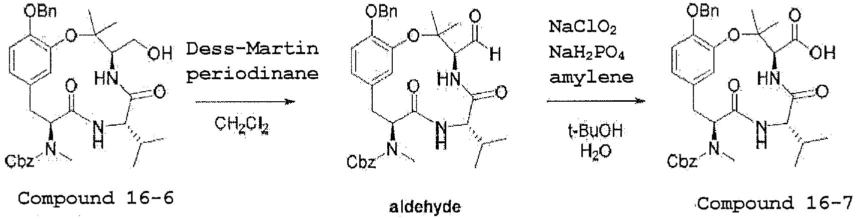

Under nitrogen atmosphere, to a solution of Compound 16-6 (1.0 eq., 225 mg, 0.349 mmol) in dichloromethane (7 mL) was added Dess-Martin periodinane (1.5 eq., 222 mg, 0.524 mmol) under ice-cooling, and the mixture was stirred at room temperature for 1.5 hr. The reaction solution was ice-cooled, 5% aqueous sodium hydrogencarbonate solution (6 mL) and 5% aqueous sodium thiosulfate solution (6 mL) were added, and the mixture was extracted once with ethyl acetate (30 mL) and twice with ethyl acetate (10 mL) by liquid separation. The organic layers were combined, washed once with 5% aqueous sodium chloride solution (20 mL) by liquid separation, and dried over sodium sulfate. The sodium sulfate was removed by filtration, and the filtrate was concentrated under reduced pressure to give Aldehyde (193 mg, yield 90%) as a yellow oil.

Under nitrogen atmosphere, to a solution of Aldehyde (1.0 eq., 191 mg, calculated as 0.349 mmol) in a mixture of t-butyl alcohol (10 mL)/amylene (2.4 mL)/water (2.4 mL) were added sodium dihydrogen phosphate dihydrate (3.5 eq., 191 mg, 1.22 mmol) and 80% sodium chlorite (4.5 eq., 178 mg, 1.57 mmol) at room temperature, and the mixture was stirred at the same temperature for 1 hr. To the reaction solution was added saturated aqueous sodium chloride solution (20 mL), the mixture was extracted once with ethyl acetate (20 mL) and twice with ethyl acetate (7 mL) by liquid separation, and the organic layers were combined, and dried over sodium sulfate. The sodium sulfate was removed by filtration, and the filtrate was concentrated under reduced pressure to give Compound 16-7 (201 mg, containing 2.5wt% ethyl acetate, two-step conversion yield 89%) as a pale-yellow viscous product.

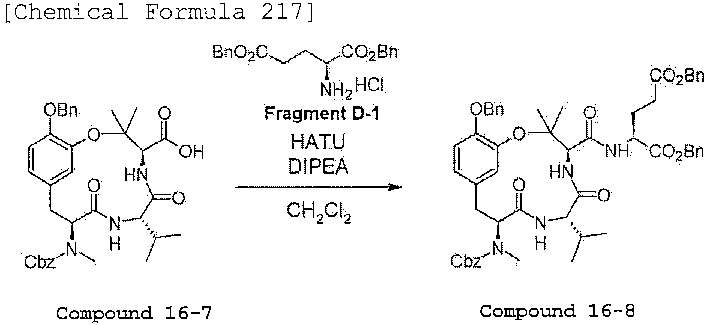

Under nitrogen atmosphere, to a solution of Compound 16-7 (1.0 eq., 98 mg, containing 2.5wt% ethyl acetate, 0.151 mmol) and dibenzyl L-glutamate hydrochloride (Fragment D-1, 1.5 eq., 82.5 mg, 0.227 mmol) in dichloromethane (3 mL) were added DIPEA (1.5 eq., 39.5 µL, 0.227 mmol) and HATU (1.5 eq., 86.3 mg, 0.227 mmol) under ice-cooling, and the mixture was stirred at the same temperature for 1 hr, and then at room temperature for 3 hr. Additional DIPEA (1.0 eq., 26.3 µL, 0.151 mmol) was added at room temperature, the mixture was stirred at the same temperature for 1.5 hr, and additional DIPEA (1.0 eq., 26.3 µL, 0.151 mmol) was added again. The mixture was stirred at the same temperature for 17 hr, 5% aqueous citric acid solution (15 mL) was added, and the mixture was extracted once with ethyl acetate (10 mL) and twice with ethyl acetate (7 mL) by liquid separation. The organic layers were combined, washed once with a mixture of 5% aqueous sodium hydrogencarbonate solution (15 mL)/5% aqueous sodium chloride solution (15 mL) by liquid separation, and dried over sodium sulfate, and the sodium sulfate was removed by filtration. The filtrate was concentrated under reduced pressure, and the crude product (183 mg) obtained as a yellow oil was purified twice by flash silica gel column (1st run: normal phase silica gel 10 g, chloroform/ethyl acetate = 60/40 - 10/90, 2nd run: normal phase silica gel 25 g, chloroform/ethyl acetate = 90/10 - 60/40) to give Compound 16-8 (28 mg, yield 20%) as a colorless viscous product.

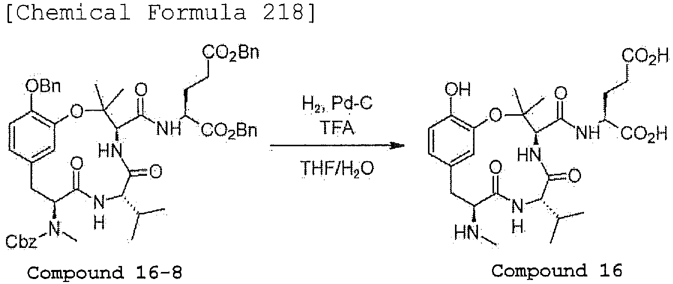

Under nitrogen atmosphere, to a solution of Compound 16-8 (1.0 eq., 27 mg, 0.029 mmol) in a mixture of THF (1.4 mL)/water (0.14 mL) were added TFA (2.0 eq., 4.4 µL, 0.057 mmol) and 10% palladium-carbon (N.E. CHEMCAT NX type, 50% hydrous product, 1.4 mg) at room temperature, the system was replaced with hydrogen, and the mixture was stirred at the same temperature for 2.5 hr. Additional 10% palladium-carbon (N.E. CHEMCAT NX type, 50% hydrous product, 2.7 mg) was added, and the mixture was stirred at the same temperature for additional 20.5 hr. The catalyst was removed by filtration through Celite, and the filtrate was concentrated under reduced pressure to give a crude product (37 mg) as a brown viscous product. The crude product was purified twice by flash silica gel column (reverse-phase silica gel 30 g, 1st run: 0.05% aqueous TFA solution/0.05% TFA acetonitrile solution = 95/5 - 70/30, 2nd run: 0.05% aqueous TFA solution/0.05% TFA acetonitrile solution = 99/1 - 70/30), and freeze-dried to give a TFA salt of Compound 16 (17.5 mg, yield 94%, purity 99.0%) as a white solid.

¹H-NMR(400MHz,D₂O)δ8.80(d,J=7.6Hz,0.3H),8.45(d,J=8.8Hz,0.1H),8.11( d,J=9.6Hz,0.8H),6.84(s,2H),6.76(s,1H),4.79(d,J=9.6Hz,1H),4.34 (d d,J=9.2,4.8Hz,1H),4.03(d,J = 100Hz,1H),4.00(dd,J=8.4,5.2Hz,1H),3.16(dd,J=13.6,5.2Hz,1H),2.85 (dd,J=13.6,8.4Hz,1H),2.64(s,3H),2.44-2.28(m,2H),2.20-2.08 (m,1H),1.96-1.81(m,2H),1.48(s,3H),1.47(s,3H),0.79(d,J=6.8Hz,3H),0.70(d,J=6. 8Hz,3H).

### (Example 24: Synthesis of Compound 17)

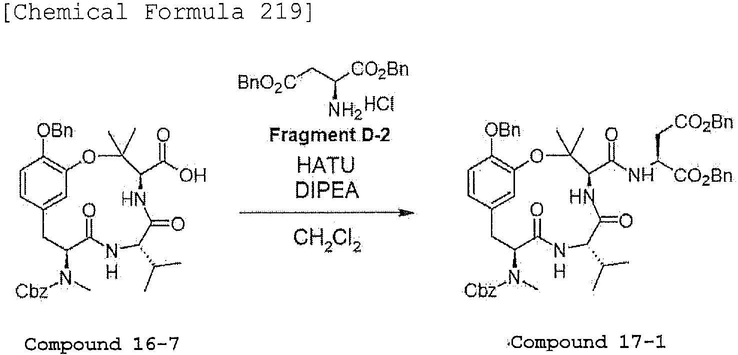

Under nitrogen atmosphere, to a solution of Compound 16-7 (1.0 eq., 100 mg, containing 2.5wt% ethyl acetate, 0.154 mmol) and dibenzyl L-aspartate hydrochloride (Fragment D-2, 1.5 eq., 81.0 mg, 0.232 mmol) in dichloromethane (3 mL) were added DIPEA (1.5 eq., 40.3 µL, 0.232 mmol) and HATU (1.5 eq., 88.0 mg, 0.232 mmol) under ice-cooling, and the mixture was stirred at the same temperature for 1 hr, and then at room temperature for 5 hr.

Additional DIPEA (2.0 eq., 53.7 µL, 0.308 mmol) was added at room temperature, the mixture was stirred at the same temperature for 17 hr, 5% aqueous citric acid solution (15 mL) was added, and the mixture was extracted once with ethyl acetate (15 mL) and twice with ethyl acetate (7 mL) by liquid separation. The organic layers were combined, washed once with 5% aqueous sodium hydrogencarbonate solution (15 mL) and once with 5% aqueous sodium chloride solution (15 mL) by liquid separation, and dried over sodium sulfate, and the sodium sulfate was removed by filtration. The filtrate was concentrated under reduced pressure, and the crude product (181 mg) obtained as a yellow oil was purified twice by flash silica gel column (1st run: normal phase silica gel 10 g, chloroform/ethyl acetate = 90/10 - 50/50, 2nd run: normal phase silica gel 25 g, chloroform/ethyl acetate = 90/10 - 67/33) to give Compound 17-1 (30 mg, yield 21%) as a colorless viscous product.

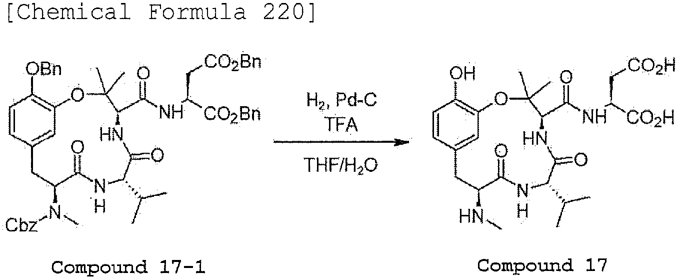

Under nitrogen atmosphere, to a solution of Compound 17-1 (1.0 eq., 27 mg, 0.029 mmol) in a mixture of THF (1.4 mL)/water (0.14 mL) were added TFA (2.0 eq., 4.5 µL, 0.058 mmol) and 10% palladium-carbon (N.E. CHEMCAT NX type, 50% hydrous product, 4.1 mg) at room temperature, the system was replaced with hydrogen, and the mixture was stirred at the same temperature for 19 hr. The catalyst was removed by filtration through Celite, and the filtrate was concentrated under reduced pressure to give a crude product (40 mg) as a brown viscous product. The crude product was purified by flash silica gel column (reverse-phase silica gel 30 g, 0.05% aqueous TFA solution/0.05% TFA acetonitrile solution = 99/1 - 70/30), and freeze-dried to give a TFA salt of Compound 17 (20.1 mg, quant., purity 99.6%) as a white solid.

¹H-NMR(400MHz,D₂O)δ6.84(s,2H),6.73(s,1H),4.79(s,1H),4.63(dd,J=7.6, 5.2Hz,1H),4.01(d,J=10.4Hz,1H),3.99(dd,J=8.0,5.2Hz,1H),3.15(dd,J =13.6,4.8Hz,1H),2.95-2.77(m,2H),2.63(s,3H),1.92-1.78(m,1H,1.48(s,3H),1.46(s,3H),0.78(d,J=6.4Hz,3H),0.69(d,J=6.8 Hz,3H).

### (Example 25: Synthesis of Compound 18)

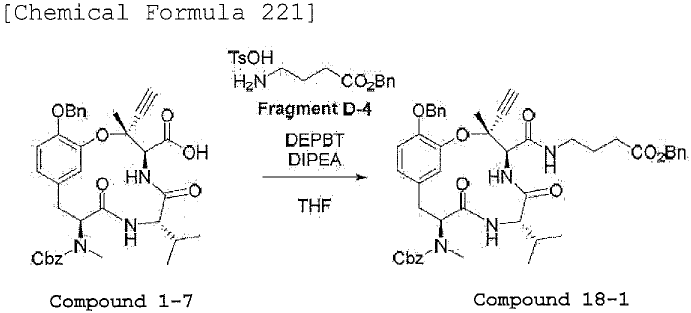

Under nitrogen atmosphere, to a solution of Compound 1-7 (1.0 eq., 66 mg, 0.103 mmol) and benzyl 4-aminobutyrate paratoluenesulfonate (Fragment D-4, 1.5 eq., 57 mg, 0.156 mmol) in THF (2.64 mL) were added DEPBT (1.5 eq., 46 mg, 0.154 mmol) and DIPEA (1.5 eq., 53 µL, 0.312 mmol) under ice-cooling, and the mixture was stirred at the same temperature for 1 hr, and then at room temperature for 17 hr. The reaction solution obtained from Compound 1-7 (5 mg) by the same procedure reaction and the above reaction solution were combined, ethyl acetate (10 mL) and 5% aqueous citric acid solution (10 mL) were added, and the mixture was extracted once by liquid separation. The organic layer was washed once with 5% aqueous sodium hydrogencarbonate solution (10 mL) and once with 5% aqueous sodium chloride solution (10 mL) by liquid separation, and dried over sodium sulfate. The sodium sulfate was removed by filtration, and the filtrate was concentrated under reduced pressure to give a crude product (89 mg) as a pale-brown viscous product. The crude product was purified by flash silica gel column (normal phase silica gel 100 g, hexane/ethyl acetate = 70/30 - 0/100) to give Compound 18-1 (44 mg, containing 1.8wt% ethyl acetate, conversion yield 48%) as a white solid.

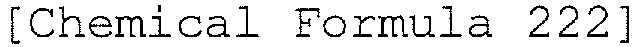

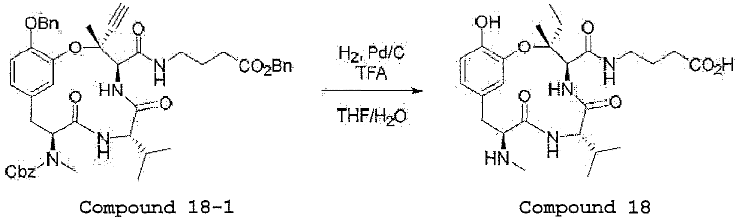

Under nitrogen atmosphere, to a solution of Compound 18-1 (1.0 eq., 44 mg, containing 1.8wt% ethyl acetate, 0.053 mmol) in a mixture of THF (2.2 mL)/water (0.11 mL) were added 10% palladium-carbon (N.E. CHEMCAT NX type, 50% hydrous product, 4.4 mg) and TFA (5.0 eq., 20 µL, 0.261 mmol) at room temperature, the system was replaced with hydrogen, and the mixture was stirred at the same temperature for 24 hr. To the reaction solution were added THF (6.6 mL), water (0.33 mL) and 10% palladium-carbon (N.E. CHEMCAT NX type, 50% hydrous product, 2.2 mg), and the mixture was stirred for 6 hr. 10% Palladium-carbon (N.E. CHEMCAT NX type, 50% hydrous product, 2.2 mg) was added again, and the mixture was stirred for 17 hr. The catalyst was removed by filtration through Celite, and the filtrate was concentrated under reduced pressure to give a crude product (43 mg). The crude product was purified by flash silica gel column (reverse-phase silica gel 60 g, 0.05% aqueous TFA solution/acetonitrile = 95/5 - 90/10), and freeze-dried to give a TFA salt of Compound 18 (16.9 mg, yield 51%, purity 95.0%) as a white solid.

H-NMR(400MHz,D₂O)86.90-6.75(m,3H),4.04(d,J = 100Hz,1H),3.97 (dd,J=9.2,4.8Hz,1H),3.21-3.12(m,3H),2.84(dd,J=13.2,9.2Hz,1H),2.65(s,3H),2.31(t,J=7.6Hz,2 H),2.00-1.82(m,2H),1.75-1.67(m,2H),1.64-1.55(m,1H),1.55(s,3H),0.96(t,J=7.6Hz,3H),0.78(d,J=6.8Hz,3H),0.7 0(d,J=6.4Hz,3H).

### (Example 26: Synthesis of Compound 20)

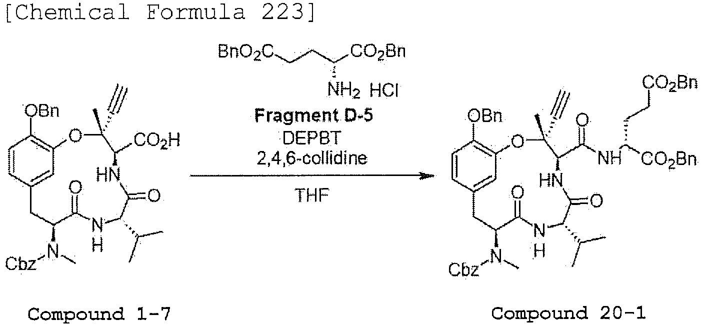

Under nitrogen atmosphere, to a solution of Compound 1-7 (1.0 eq., 123 mg, 0.191 mmol) in THF (1 mL) were added 2,4,6-collidine (1.5 eq., 35.0 mg, 0.288 mmol), DEPBT (1.5 eq., 86.6 mg, 0.289 mmol) and dibenzyl D-glutamate hydrochloride (Fragment D-5, 1.5 eq., 106 mg, 0.290 mmol) under ice-cooling, and the mixture was stirred at room temperature for 22 hr. To the reaction solution was added ethyl acetate (10 mL), and the mixture was washed twice with a mixture of water (5 mL)/saturated aqueous ammonium chloride solution (5 mL), twice with a mixture of water (5 mL)/saturated aqueous sodium hydrogencarbonate solution (5 mL) and once with saturated aqueous sodium chloride solution (10 mL) by liquid separation, and dried over magnesium sulfate. The magnesium sulfate was removed by filtration, and the filtrate was concentrated under reduced pressure to give a crude product (301 mg). The crude product was purified twice by flash silica gel column (1st run: NH₂ silica gel 8 g, hexane/ethyl acetate = 80/20 - 0/100, 2nd run: normal phase silica gel 4 g, hexane/ethyl acetate = 50/50) to give Compound 20-1 (28.1 mg, yield 15%) as a pale-white liquid.

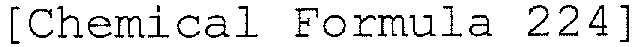

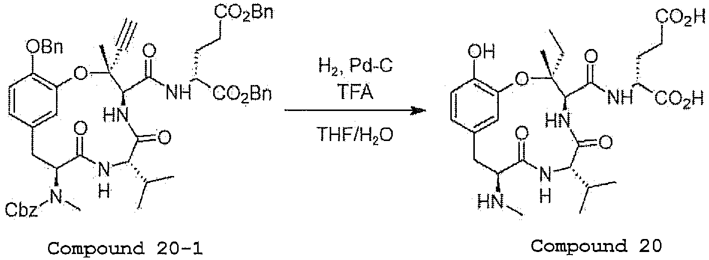

Under nitrogen atmosphere, to a solution of Compound 20-1 (1.0 eq., 28 mg, 0.029 mmol) in a mixture of THF (840 µL)/water (42 µL) were added 10% palladium-carbon (N.E. CHEMCAT NX type, 50% hydrous product, 2.5 mg) and TFA (5.0 eq., 11.3 µL, 0.148 mmol) at room temperature, the system was replaced with hydrogen, and the mixture was stirred at the same temperature for 6.5 hr. The catalyst was removed by filtration through Celite, and the filtrate was concentrated under reduced pressure to give a crude product (24.8 mg). The crude product was purified by flash silica gel column (reverse-phase silica gel 30 g, 0.05% aqueous TFA solution/acetonitrile = 95/5 - 90/10), and freeze-dried to give a TFA salt of Compound 20 (13.4 mg, yield 68%, purity 94.3%) as a white solid.

¹H-NMR(400MHz,D₂O)δ6.93-6.82(m,3H),4.88(s,1H),4.29(dd,J=9.2,5.2Hz,1H),4.09(d,J = 100Hz,1H),4.04(dd,J=8.8,5.2Hz,1H),3.23(dd,J=13.6,4.8Hz,1H),2.89 (dd,J=13.2,8.8Hz,1H),2.70(s,3H),2.51-2.46(m,2H),2.22-2.13(m,1H),2.05-1.87(m,3H),1.72-1.63(m,1H),1.55(s,3H),1.05(t,J=7.2Hz,3H),0.84(d,J=7.2Hz,3H),0.7 6 (d, J=6. 8Hz, 3H)

### (Example 27: Synthesis of Compound 21)

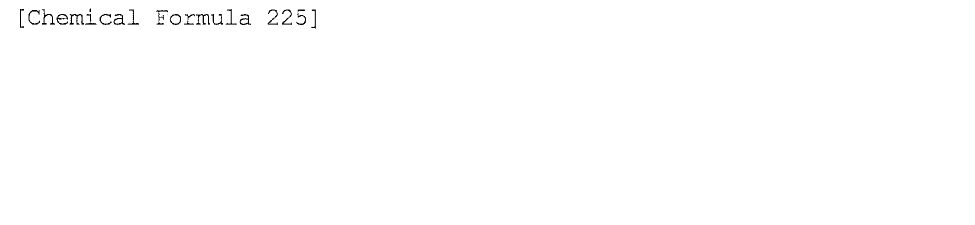

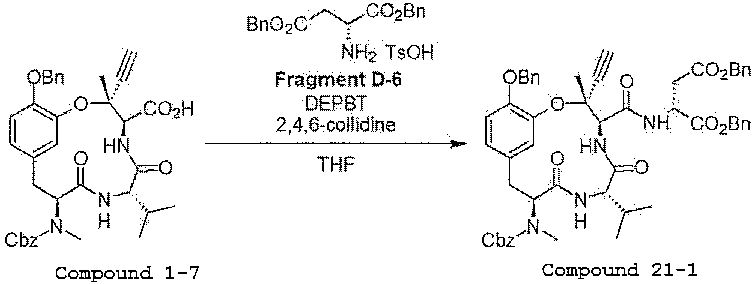

Under nitrogen atmosphere, to a solution of Compound 1-7 (1.0 eq., 207 mg, 0.322 mmol) in THF (3.2 mL) were added 2,4,6-collidine (2.8 eq., 110 mg, 0.911 mmol), DEPBT (1.5 eq., 145 mg, 0.483 mmol) and dibenzyl D-aspartate paratoluenesulfonate (Fragment D-6, 1.5 eq., 236 mg, 0.486 mmol) under ice-cooling, and the mixture was stirred at room temperature for 20 hr. To the reaction solution was added ethyl acetate (15 mL), and the mixture was washed twice with a mixture of water (5 mL)/saturated aqueous ammonium chloride solution (5 mL) and twice with a mixture of water (5 mL)/saturated aqueous sodium hydrogencarbonate solution (5 mL) by liquid separation. The organic layer was washed once with saturated aqueous sodium chloride solution (10 mL) by liquid separation, and dried over magnesium sulfate. The magnesium sulfate was removed by filtration, and the filtrate was concentrated under reduced pressure to give a crude product (438 mg). The crude product was purified twice by flash silica gel column (1st run: NH₂ silica gel 8 g, hexane/ethyl acetate = 75/25 - 0/100, 2nd run: normal phase silica gel 5 g, hexane/ethyl acetate = 67/23 - 50/50) to give Compound 21-1 (32.4 mg, yield 11%) as a pale-white liquid.

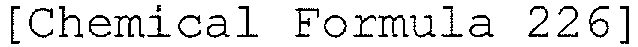

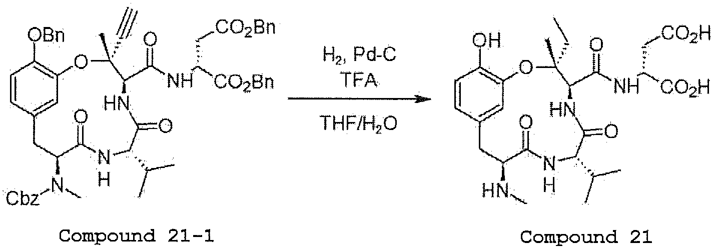

The reaction was carried out in the same manner as in synthesis of Compound 20, a TFA salt of Compound 21 (12.1 mg, yield 58%, purity 95.2%) was obtained from Compound 21-1.

¹H-NMR(400MHz,D₂O)δ6.92-6.82(m,3H),4.74-4.70(m,1H),4.09(d,J = 100Hz,1H),4.04(dd,J=8.8,4.8Hz,1H),3.22(dd,J=13.6,4.8Hz,1H),3.00 (dd,J=17.2,4.8Hz,1H),2.93-2.83(m,2H),2.70(s,3H),1.98-1.87(m,2H),1.72-1.63(m,1H),1.54(s,3H),1.02(t,J=7.2Hz,3H),0.84(d,J=6.4Hz,3H),0.7 6(d,J=6.4Hz,3H).

### (Example 28: Synthesis of Compound 23)

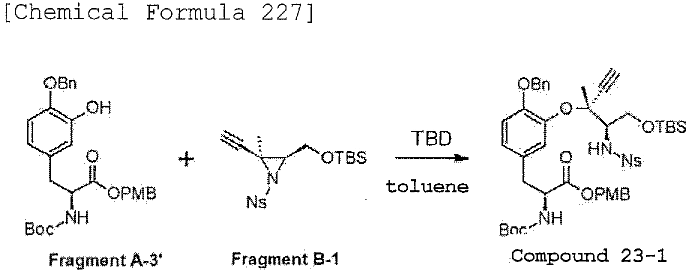

Under nitrogen atmosphere, to a solution of Fragment A-3' (1.0 eq., 3.46 g, containing about 30% PMB ester of A3'-4, calculated as 4.78 mmol) and Fragment B-1 (1.3 eq. 2.52 g, 6.14 mmol) in toluene (50 mL) was added TBD (1.3 eq., 853 mg, 6.13 mmol) under ice-cooling, and the mixture was stirred at room temperature for 16 hr. The toluene was evaporated by concentrating under reduced pressure to give a crude product as a brownish-red amorphous. The crude product was purified by flash silica gel column (normal phase silica gel 100 g, hexane/ethyl acetate = 5/1 - 1/1) to give Compound 23-1 (4.02 g, containing impurities, apparent yield 92%) as a yellow viscous product.

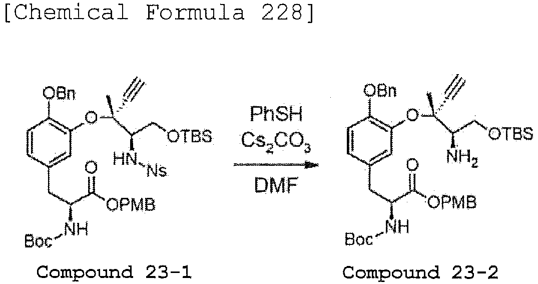

Under nitrogen atmosphere, to a solution of Compound 23-1 (1.0 eq., 4.02 g, 4.37 mmol) in DMF (40 mL) were added cesium carbonate (1.5 eq., 2.33 g, 6.61 mmol) and thiophenol (1.5 eq., 650 µL, 6.37 mmol) under ice-cooling, and the mixture was stirred at room temperature for 3 hr. To the reaction solution was added saturated aqueous sodium hydrogencarbonate solution (40 mL), and the mixture was extracted three times with a mixture of hexane (12.5 mL)/ethyl acetate (37.5 mL) by liquid separation. The organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product as a yellow oil. The crude product was purified by flash silica gel column (normal phase silica gel 70 g, hexane/ethyl acetate = 5/1 - 2/1) to give Compound 23-2 (2.70 g, yield 84%) as a pale-yellow viscous product.

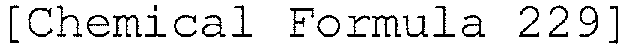

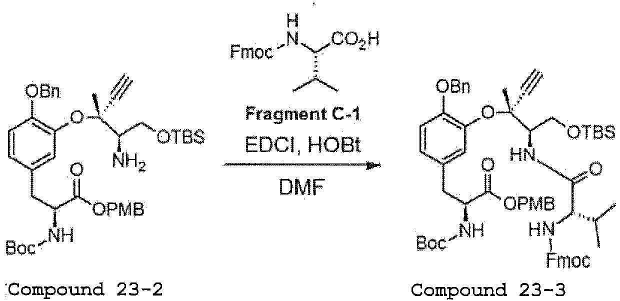

Under nitrogen atmosphere, to a solution of Compound 23-2 (1.0 eq., 2.70 g, 3.68 mmol) in DMF (40 mL) were added EDCI (1.2 eq., 867 mg, 4.52 mmol), HOBt•H₂O (1.2 eq., 679 mg, 4.43 mmol) and N-Fmoc-L-Val (1.2 eq., 1.52 g, 4.47 mmol) under ice-cooling, and the mixture was stirred at room temperature for 3 hr. To the reaction solution was added water (40 mL), and the mixture was extracted three times with a mixture of hexane (12.5 mL)/ethyl acetate (37.5 mL) by liquid separation. The organic layers were combined, and washed once with saturated aqueous sodium hydrogencarbonate solution (100 mL) and once with saturated aqueous sodium chloride solution (100 mL) by liquid separation. The organic layer was dried over magnesium sulfate, the magnesium sulfate was removed by filtration, and the filtrate was concentrated under reduced pressure to give Compound 23-3 (4.34 g, crude yield 112%) as a white amorphous.

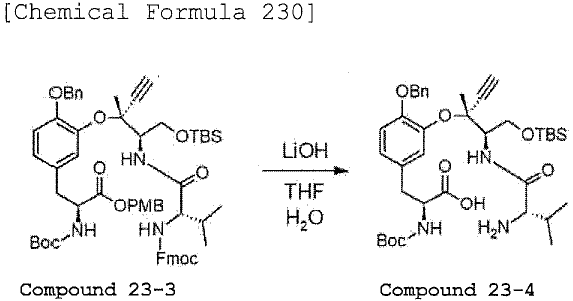

To a solution of Compound 23-3 (1.0 eq., 4.34 g, 3.68 mmol) in a mixture of THF (24 mL)/water (12 mL) was added lithium hydroxide (4.0 eq., 353 mg, 14.72 mmol) at room temperature, and the mixture was stirred at room temperature for 4 hr. The reaction solution was adjusted to pH 7 by addition of 2N aqueous hydrochloric acid solution under ice-cooling, and extracted three times with ethyl acetate (20 mL) by liquid separation. The organic layers were combined, and washed once with saturated aqueous sodium chloride solution (50 mL) by liquid separation. The organic layer was dried over magnesium sulfate, the magnesium sulfate was removed by filtration, and the filtrate was concentrated under reduced pressure to give a crude product (3.81 g). The crude product was purified by flash silica gel column (normal phase silica gel 70 g, ethyl acetate/methanol = 1/0 - 3/1) to give Compound 23-4 (1.84 g, two-step yield 70%) as a white amorphous.

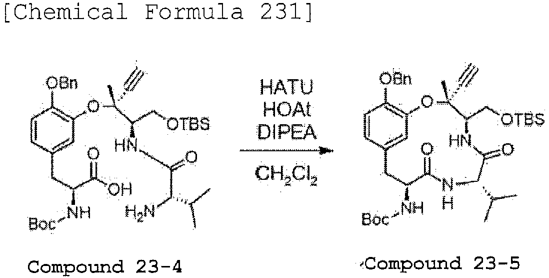

A solution of Compound 23-4 (1.0 eq., 796 mg, 1.12 mmol) in dichloromethane (110 mL) was added dropwise over 19 hr to a solution of HATU (5.0 eq., 2.12 g, 5.57 mmol), HOAt (5.0 eq., 2764 mg, 5.61 mmol) and DIPEA (5.0. eq, 950 µL, 5.59 mmol) in dichloromethane (560 mL) at about 100 µL/min at room temperature, and the mixture was stirred at room temperature for 23 hr. The reaction solution was concentrated under reduced pressure to evaporate the dichloromethane, ethyl acetate (30 mL) was added, and the mixture was washed twice with saturated aqueous ammonium chloride solution (30 mL), once with water (30 mL), twice with saturated aqueous sodium hydrogencarbonate solution (30 mL) and once with saturated aqueous sodium chloride solution (30 mL) by liquid separation. the aqueous layer of the saturated aqueous ammonium chloride solution and the aqueous layer of the saturated aqueous sodium hydrogencarbonate solution were each extracted once with ethyl acetate (20 mL) by liquid separation, and the organic layers were combined, and washed once with saturated aqueous sodium chloride solution (50 mL) by liquid separation. The organic layer was dried over magnesium sulfate, the magnesium sulfate was removed by filtration, and the filtrate was concentrated under reduced pressure to give a crude product (1.04 g) as a brown amorphous. The crude product was purified by flash silica gel column (normal phase silica gel 10 g, hexane/ethyl acetate = 3/1 - 1/1) to give Compound 23-5 (598 mg, yield 77%) as a white amorphous.

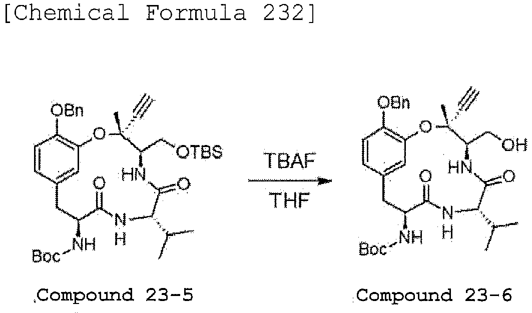

Under nitrogen atmosphere, to a solution of Compound 23-5 (1.0 eq., 598 mg, 861 µmol) in THF (10 mL) was added TBAF (1M in THF, 1.5 eq., 1.3 mL, 1.30 mmol) under ice-cooling, and the mixture was stirred at room temperature for 1.5 hr. To the reaction solution was added saturated aqueous ammonium chloride solution (10 mL), and the mixture was washed once by liquid separation. The aqueous layer was extracted twice with ethyl acetate (10 mL) by liquid separation, and the organic layers were combined, and washed once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (747 mg) as a white amorphous. The crude product was purified by flash silica gel column (normal phase silica gel 10 g, hexane/acetone =1/1 - 1/10) to give a white solid (482 mg). The obtained white solid was recrystallized from a mixture of hexane/ethyl acetate (1/1) to remove impurities to give Compound 23-6 (259 mg, yield 52%).

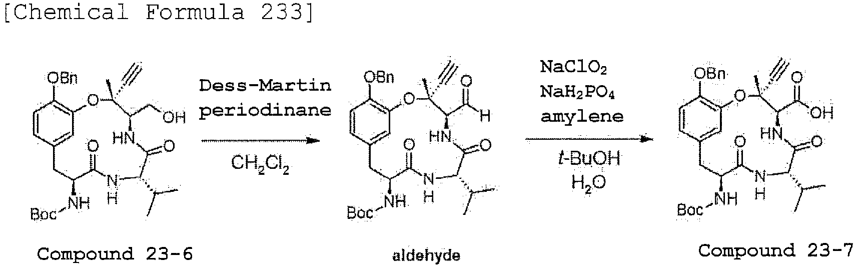

Under nitrogen atmosphere, to a solution of Compound 23-6 (1.0 eq., 256 mg, 442 µmol) in dichloromethane (5 mL) was added Dess-Martin periodinane (1.5 eq., 284 mg, 670 µmol) under ice-cooling, and the mixture was stirred at room temperature for 1 hr. To the reaction solution were added 10% aqueous sodium sulfite solution (5 mL) and saturated aqueous sodium hydrogencarbonate solution (5 mL), and the mixture was extracted three times with dichloromethane (10 mL) by liquid separation. The organic layers were combined, and washed once with saturated aqueous sodium chloride solution (30 mL) by liquid separation. The organic layer was dried over magnesium sulfate, the magnesium sulfate was removed by filtration, and the filtrate was concentrated under reduced pressure to give Aldehyde (267 mg, crude yield 104%).

To a solution of Aldehyde (1.0 eq., 267 mg, calculated as 442 µmol) in a mixture of t-butyl alcohol (3 mL)/amylene (1 mL) was added a solution of sodium dihydrogen phosphate dihydrate (3.5 eq., 238 mg, 1.53 mmol) and 80% sodium chlorite (4.6 eq., 228 mg, 2.02 mmol) in water (1 mL) at room temperature, and the mixture was stirred at room temperature for 30 min. To the reaction solution was added saturated aqueous ammonium chloride solution (5 mL), and the mixture was extracted three times with ethyl acetate (5 mL) by liquid separation. The organic layers were combined, and dried over magnesium sulfate. The magnesium sulfate was removed by filtration, and the filtrate was concentrated under reduced pressure to give a crude product (273 mg). The crude product was purified by flash silica gel column (normal phase silica gel 10 g, chloroform/methanol = 100/1 - 30/1) to give Compound 23-7 (218 mg, two-step yield 83%) as a white amorphous.

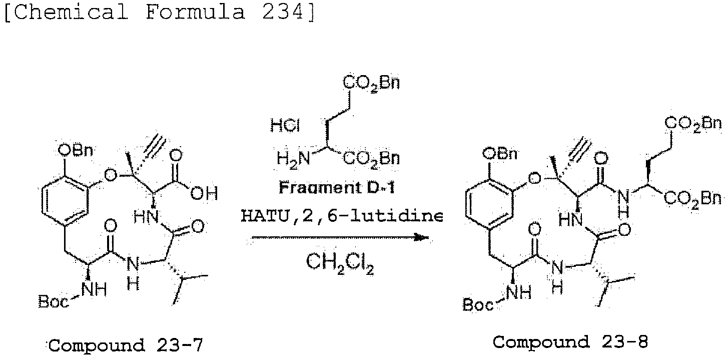

Under nitrogen atmosphere, to a solution of Compound 23-7 (1.0 eq., 211 mg, 356 µmol) in dichloromethane (4 mL) were added 2,6-lutidine (1.1 eq., 45 µL, 386 µmol), HATU (1.1 eq., 151 mg, 397 µmol) and benzyl L-glutamate hydrochloride (Fragment D-1, 1.1 eq., 144 mg, 395 µmol) under ice-cooling, and the mixture was stirred under ice-cooling for 2 hr. Additional 2,6-lutidine (0.24 eq., 10 µL, 86 µmol), HATU (0.5 eq., 70 mg, 184 µmol) and benzyl L-glutamate hydrochloride (Fragment D-1, 0.5 eq., 66 mg, 180 µmol) were added, and the mixture was stirred under ice-cooling for 2 hr, and then at room temperature for 2 hr. To the reaction solution was added saturated aqueous ammonium chloride solution (4 mL), and the mixture was washed once by liquid separation. Then, the aqueous layer was extracted twice with ethyl acetate (5 mL) by liquid separation. The organic layers were combined, and washed once with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (443 mg). The crude product was purified twice by flash silica gel column (1st run: normal phase silica gel 13 g, hexane/ethyl acetate = 70/30 - 30/70, 2nd run: normal phase silica gel 7 g, hexane/ethyl acetate = 3/1 - 1/3) to give Compound 23-8 (32 mg, yield 10%) as a white amorphous.

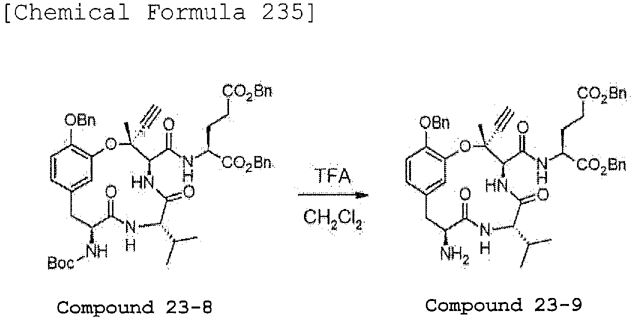

Under nitrogen atmosphere, to a solution of Compound 23-8 (1.0 eq., 28 mg, 31 µmol) in dichloromethane (500 µL) was added TFA (10.5 eq., 25 µL, 327 µmol) under ice-cooling, and the mixture was stirred at room temperature for 1 hr. Additional TFA (31.5 eq., 75 µL, 980 µmol) was added under ice-cooling, and the mixture was stirred at room temperature for 2 hr. To the reaction solution was added saturated aqueous sodium hydrogencarbonate solution (5 mL), and the mixture was extracted three times with ethyl acetate (5 mL) by liquid separation. The organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give Compound 23-9 (26 mg, crude yield 105%).

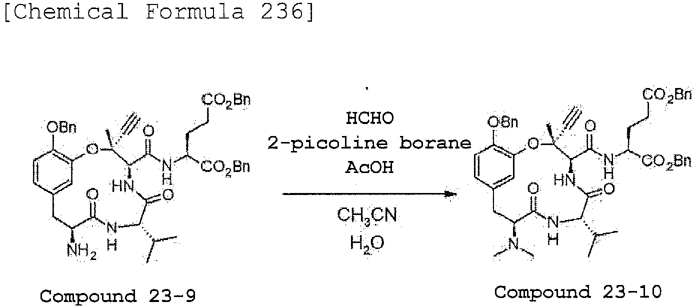

To a solution of Compound 23-9 (1.0 eq., 26 mg, calculated as 31 µmol) in a mixture of acetonitrile (600 µL)/water (300 µL) were added 37% formaldehyde aqueous solution (9.9 eq., 25 µL, 308 µmol) and acetic acid (2.8 eq., 5 µL, 87 µmol) at room temperature, and the mixture was stirred at room temperature for 30 min. Then, 2-picoline borane (3.0 eq., 10 mg, 94 µmol) was added under ice-cooling, and the mixture was stirred at room temperature for 1 hr. To the reaction solution was added saturated aqueous sodium hydrogencarbonate solution (5 mL), and the mixture was extracted three times with ethyl acetate (5 mL) by liquid separation. The organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (29 mg). The crude product was purified by flash silica gel column (normal phase silica gel 5 g, chloroform/methanol = 50/1 - 20/1) to give Compound 23-9 (13 mg, two-step yield 50%) as a white amorphous.

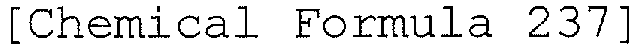

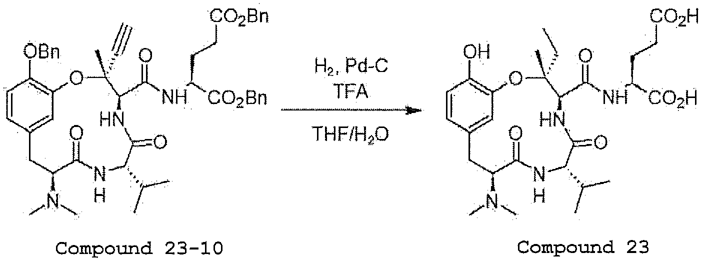

Under nitrogen atmosphere, to a solution of Compound 23-10 (1.0 eq., 12 mg, 0.014 mmol) in a mixture of THF (2.4 mL)/water (120 µL) were added 10% palladium-carbon (N.E. CHEMCAT NX type, 50% hydrous product, 1.2 mg) and TFA (5.0 eq., 5.5 µL, 0.072 mmol) at room temperature, the system was replaced with hydrogen, and the mixture was stirred at the same temperature for 16 hr. The catalyst was removed by filtration through Celite, to the filtrate was added 5% aqueous sodium hydrogencarbonate solution (2 mL), and the mixture was extracted twice with ethyl acetate (2 mL) by liquid separation. The organic layer was washed once with 5% aqueous sodium chloride solution (2 mL) by liquid separation, and dried over sodium sulfate. The sodium sulfate was removed by filtration, and the filtrate was concentrated under reduced pressure to recover Compound 23-10 (12 mg).

### (Re-reaction)

To a solution of the recovered Compound 23-10 (12 mg) in a mixture of THF (2.4 mL)/water (120 µL) were added 10% palladium-carbon (N.E. CHEMCAT NX type, 50% hydrous product, 1.2 mg) and TFA (5.0 eq., 5.5 µL, 0.072 mmol) under nitrogen atmosphere, and the system was replaced with hydrogen, and the mixture was stirred at room temperature for 74 hr while adding the catalyst. The time when the catalyst was added (representing the time elapsed since the re-reaction was started) and the amount added are shown below.

| Time (h) | Amount of catalyst added (mg) |
|---|---|
| 1.5 | 0.7 |
| 4.5 | 0.7 |
| 20 | 0.6 |
| 24.5 | 0.6 |
| 27 | 0.6 |
| 29 | 0.6 |
| 44 | 1.2 |
| 52.5 | 1.2 |
| 69 | 1.2 |
| 72 | 1.2 |

The catalyst was removed by filtration through Celite, the filtrate was concentrated under reduced pressure, and the residue was purified by flash silica gel column (reverse-phase silica gel 30 g, 0.05% aqueous TFA solution/acetonitrile = 95/5 - 90/10), and freeze-dried to give a TFA salt of Compound 23 (5.1 mg, yield 52%, purity 93.4%) as a white solid.

¹H-NMR(400MHz,D₂O)δ6.93-6.88(m,3H),4.35(dd,J=13.2,4.8Hz,1H),4.04(d,J = 100Hz,1H),3.93(dd,J=11.6,5.2Hz,1H),3.40(dd,J=12.4,5.2Hz,1H),2.9 7 (brs,6H),2.80(t,J=12.0Hz,1H),2.48-2.32(m,2H),2.21-2.13(m,1H),2.10-2.01(m,1H),1.98-1.89(m,1H),1.88-1.78(m,1H),1.66(s,3H),1.65-1.58(m,1H),1.04(t,J=7.2Hz,3H),0.81(d,J=6.8Hz,3H),0.72(d,J=6.4Hz ,3H).

### (Example 29: Synthesis of Compound 22)

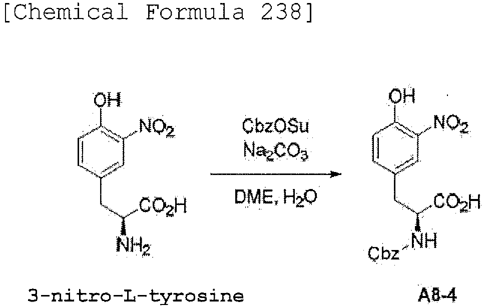

Under nitrogen atmosphere, to a solution of 3-nitro-L-tyrosine (1.0 eq., 5.11 g, 22.59 mmol) in a mixture of DME (200 mL)/water (220 mL) was added a solution of 10% aqueous sodium carbonate solution (1.1 eq., 26.34 g, 24.85 mmol) and CbzOSu (1.1 eq., 6.19 g, 24.84 mmol) in DME (20 mL) at room temperature, and the mixture was stirred at the same temperature for 2 hr. The DME was evaporated under reduced pressure, the residue was adjusted to pH 2-3 by addition of 1M aqueous hydrochloric acid solution (52 mL), and the precipitated solid was collected by filtration. The solid was washed with water, and dried under reduced pressure to give A8-4 (8.61 g, containing impurities, quant.) as an ocher solid.

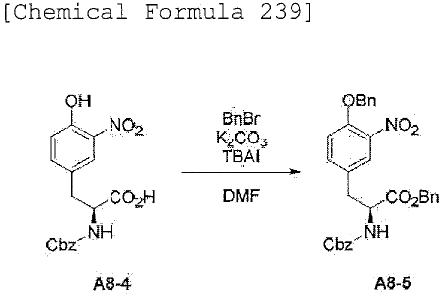

Under nitrogen atmosphere, to a solution of A8-4 (1.0 eq., 8.61 g, containing impurities, calculated as 22.59 mmol) in DMF (45 mL) were added potassium carbonate (2.2 eq., 6.87 g, 49.70 mmol), BnBr (2.2 eq., 8.50 g, 49.70 mmol) and TBAI (0.1 eq., 834 mg, 2.26 mmol) under ice-cooling, and the mixture was stirred at room temperature for 2 hr, and then at the external temperature of 30°C for 1.5 hr.

The reaction solution obtained by treating A8-4 (322 mg) and the previous reaction solution were combined, water (90 mL) was added, and the mixture was extracted once with ethyl acetate (80 mL) and twice with ethyl acetate (25 mL) by liquid separation. The organic layers were combined, washed once with 5% aqueous sodium chloride solution (50 mL) by liquid separation, and dried over sodium sulfate. The sodium sulfate was removed by filtration, and the filtrate was concentrated under reduced pressure, and the crude product (15.24 g) obtained as a brown oil was purified by flash silica gel column (normal phase silica gel 100 g, hexane/ethyl acetate = 80/20 - 50/50) to give A8-5 (12.33 g, containing 0.7wt% N-hydroxysuccinimide, two-step conversion yield from 3-nitro-L-tyrosine 97%) as a pale-orange solid.

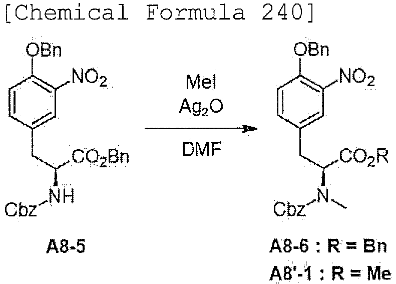

Under nitrogen atmosphere, to a solution of A8-5 (1.0 eq., 2.00 g, containing 0.7wt% N-hydroxysuccinimide, 3.67 mmol) in DMF (16 mL) were added silver(I) oxide (2.0 eq., 1.70 g, 7.34 mmol) and methyl iodide (2.4 eq., 1.24 g, 8.74 mmol), and the mixture was stirred for 25 min under microwave irradiation (80°C).

The reaction solution obtained by treating A8-5 (9.01 g) in the same manner and the previous reaction solution were combined, and the insoluble substance was removed by filtration through Celite. The residue was washed with ethyl acetate (250 mL), and the filtrate was washed once with 5% aqueous sodium thiosulfate solution (200 mL) and once with 5% aqueous sodium chloride solution (200 mL) by liquid separation, and dried over sodium sulfate. The sodium sulfate was removed by filtration, and the filtrate was concentrated under reduced pressure to give an orange viscous product. This orange viscous product was dissolved in toluene (100 mL), and the solution was washed once with water (100 mL) and once with 5% aqueous sodium chloride solution (200 mL) by liquid separation, and dried over sodium sulfate. The sodium sulfate was removed by filtration, and the filtrate was concentrated under reduced pressure to give a mixture of A8-6 and A8'-1 (4:1, 10.91 g, containing 1.3wt% toluene, conversion yield 97%) as an orange viscous product.

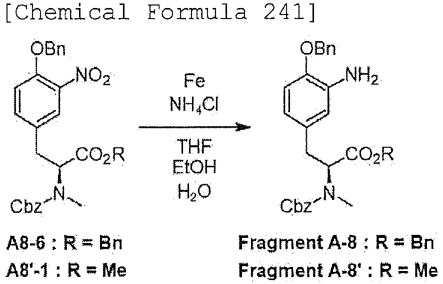

Under nitrogen atmosphere, to a solution of a mixture of A8-6 and A8'-1 (4:1, 1.0 eq., 10.91 g, containing 1.3wt% toluene, 19.42 mmol) in a mixture of THF (77 mL)/ethanol (77 mL)/water (16 mL) were added iron powder (5.0 eq., 5.42 g, 97.08 mmol) and ammonium chloride (2.5 eq., 2.60 g, 48.61 mmol), and the mixture was stirred at the external temperature of 75°C for 4.5 hr. The reaction solution was allowed to cool to room temperature, the insoluble substance was removed by filtration through Celite, and the residue was washed with ethyl acetate (200 mL) and water (200 mL). The organic layer of the filtrate was recovered, washed once with 5% aqueous sodium chloride solution (200 mL) by liquid separation, and dried over sodium sulfate. The sodium sulfate was removed by filtration, and the filtrate was concentrated under reduced pressure to give a mixture of Fragment A-8 and Fragment A-8' (4:1, 10.16 g, containing 0.3wt% ethyl acetate, conversion yield 99%) as a yellow-brown viscous product.

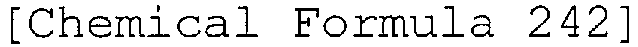

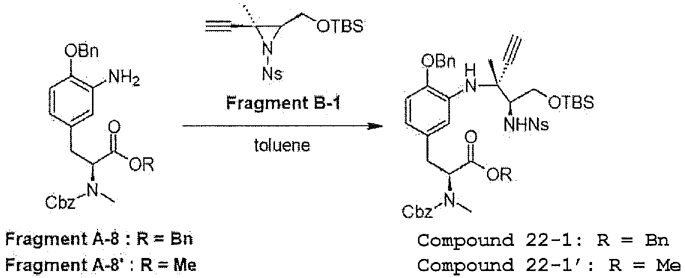

Under nitrogen atmosphere, a solution of a mixture of Fragment A-8 and Fragment A-8' (4:1, 2.0 eq., 5.15 g, containing 0.3wt% ethyl acetate, 9.79 mmol) and Fragment B-1 (1.0 eq., 2.01 g, 4.90 mmol) in toluene (49 mL) was stirred at the external temperature of 50°C for 19.5 hr. The reaction solution was concentrated under reduced pressure to give a mixture of Compound 22-1 and Compound 22-1' (7.45 g, quant.) as a pale-brown viscous product.

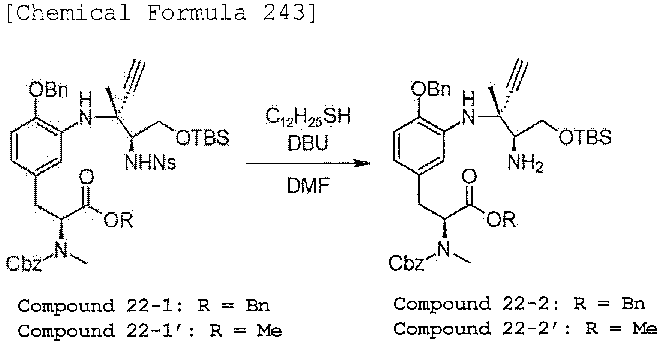

Under nitrogen atmosphere, to a solution of a mixture of Compound 22-1 and Compound 22-1' (1.0 eq., 7.45 g, calculated as 4.90 mmol) in DMF (37 mL) were added 1-dodecanethiol (9.0 eq., 8.93 g, 44.12 mmol) and DBU (9.0 eq., 6.71 g, 44.08 mmol) at room temperature, and the mixture was stirred at the same temperature for 3.5 hr. To the reaction solution was added toluene (80 mL), and the mixture was washed twice with 5% aqueous citric acid solution (80 mL) by liquid separation. To the organic layer was added ethyl acetate (80 mL), and the organic layer was washed once with a mixture of 5% aqueous sodium hydrogencarbonate solution (80 mL)/saturated aqueous sodium chloride solution (80 mL) and once with saturated aqueous sodium chloride solution (80 mL) by liquid separation, and dried over sodium sulfate. The sodium sulfate was removed by filtration, and the filtrate was concentrated under reduced pressure, and the crude product (16.37 g) obtained as a yellow oil was purified by flash silica gel column (normal phase silica gel 100 g, hexane/ethyl acetate = 90/10 - 50/50) to give a mixture of Compound 22-2 and Compound 22-2' (5.20 g, containing Fragment A-8 and ethyl acetate, quant.) as a paleorange viscous product.

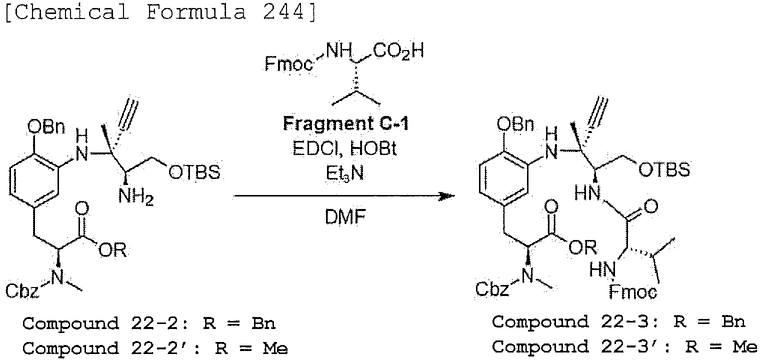

Under nitrogen atmosphere, to a solution of a mixture of Compound 22-2 and Compound 22-2' (1.0 eq., 4.68 g, containing Fragment A-8 and ethyl acetate, calculated as 4.41 mmol) in DMF (44 mL) were added N-Fmoc-L-valine (Fragment C-1, 1.1 eq., 1.65 g, 4.86 mmol), EDCI (1.1 eq., 0.93 g, 4.85 mmol), HOBt•H₂O (1.1 eq., 0.74 g, 4.83 mmol) and triethylamine (1.5 eq., 0.67 g, 6.62 mmol), and the mixture was stirred at room temperature for 2 hr. To the reaction solution was added toluene (90 mL), and the mixture was washed once with 5% aqueous citric acid solution (90 mL), once with 5% aqueous sodium hydrogencarbonate solution (50 mL) and once with 5% aqueous sodium chloride solution (50 mL) by liquid separation, and dried over sodium sulfate. The sodium sulfate was removed by filtration, and the filtrate was concentrated under reduced pressure to give a crude product (6.76 g) as a yellow viscous product. The crude product was purified twice by flash silica gel column (1st run: normal phase silica gel 100 g, hexane/ethyl acetate = 92/8 - 34/66, 2nd run: normal phase silica gel 50 g, hexane/ethyl acetate = 80/20) to give Compound 22-3 (2.50 g, containing 1.1wt% ethyl acetate, three-step conversion yield from Fragment B-1 47%) as a white amorphous.

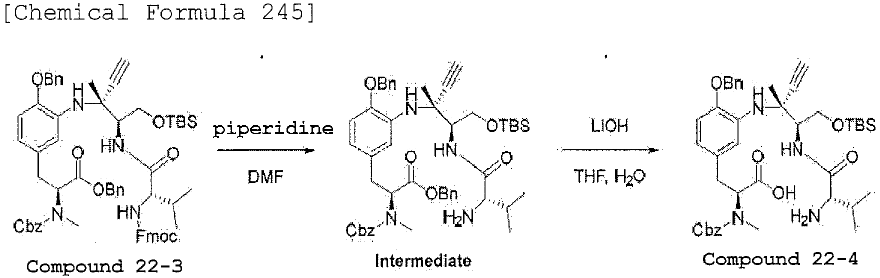

Under nitrogen atmosphere, to a solution of Compound 22-3 (1.0 eq., 2.50 g, containing 1.1wt% ethyl acetate, 2.31 mmol) in DMF (25 mL) was added piperidine (1.25 mL), and the mixture was stirred at room temperature for 1 hr. The reaction solution was ice-cooled, 5% aqueous citric acid solution (50 mL) was added, and the mixture was extracted once with toluene (50 mL) by liquid separation. The organic layer was washed once with 5% aqueous sodium hydrogencarbonate solution (50 mL) and once with 5% aqueous sodium chloride solution (50 mL) by liquid separation, and dried over sodium sulfate. The sodium sulfate was removed by filtration, the filtrate was concentrated under reduced pressure, and the obtained crude product (2.61 g) was purified by flash silica gel column (normal phase silica gel 25 g, hexane/ethyl acetate = 90/10 - 0/100) to give Intermediate (1.93 g, containing 4.0wt% ethyl acetate, conversion yield 95%) as a yellow viscous product.

Under nitrogen atmosphere, to a solution of Intermediate (1.0 eq., 1.93 g, containing 4.0wt% ethyl acetate, 2.18 mmol) in a mixture of THF (19 mL)/water (9.5 mL) was added lithium hydroxide monohydrate (2.0 eq., 0.18 g, 4.29 mmol), and the mixture was stirred at room temperature for 3 hr. The reaction solution was ice-cooled, 5% aqueous citric acid solution (30 mL) was added, and the mixture was extracted once with ethyl acetate (30 mL) by liquid separation. The organic layer was washed twice with 5% aqueous sodium chloride solution (30 mL) by liquid separation, and dried over sodium sulfate, the sodium sulfate was removed by filtration, and the filtrate was concentrated under reduced pressure to give a crude product (2.03 g) as a pale-yellow amorphous. The crude product was purified by flash silica gel column (normal phase silica gel 25 g, ethyl acetate/methanol = 100/0 - 80/20) to give a pale-yellow amorphous (1.78 g). This pale-yellow amorphous was dissolved in ethyl acetate (20 mL), and the solution was washed twice with 5% aqueous sodium chloride solution (20 mL) by liquid separation, and dried over sodium sulfate. The sodium sulfate was removed by filtration, and the filtrate was concentrated under reduced pressure to give Compound 22-4 (1.72 g, containing 4.2wt% ethyl acetate and 0.2wt% acetic acid, conversion yield 99%) as a crude pale-yellow amorphous product.

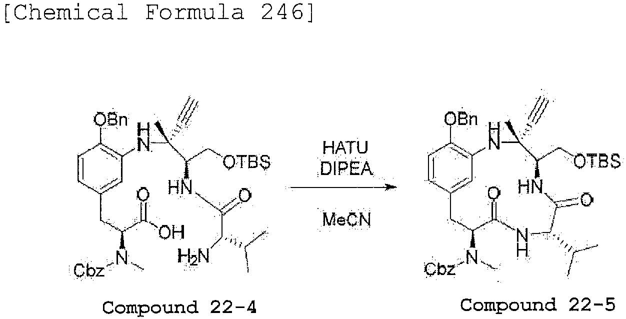

Under nitrogen atmosphere, to a solution of HATU (5.0 eq., 4.12 g, 10.84 mmol) in acetonitrile (2150 mL) was added DIPEA (10.0 eq., 2.80 g, 21.67 mmol) at room temperature. Then, a solution of Compound 22-4 (1.0 eq., 1.72 g, containing 4.2wt% ethyl acetate and 0.2wt% acetic acid, 2.17 mmol) in acetonitrile (11.5 mL)/THF (11.5 mL) was added over 44 hr at the same temperature, and after the dropwise addition was completed, the mixture was stirred at room temperature for additional 1 hr. The reaction solution was concentrated under reduced pressure, to the concentrated residue was added ethyl acetate (20 mL), and the insoluble substance was removed by filtration. The filtrate was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give a crude product (2.50 g) as a brown amorphous. The crude product was purified by flash silica gel column (normal phase silica gel 100 g, hexane/ethyl acetate = 80/20) to give Compound 22-5 (439 mg, containing 0.4wt% ethyl acetate, conversion yield 27%) as a white amorphous.

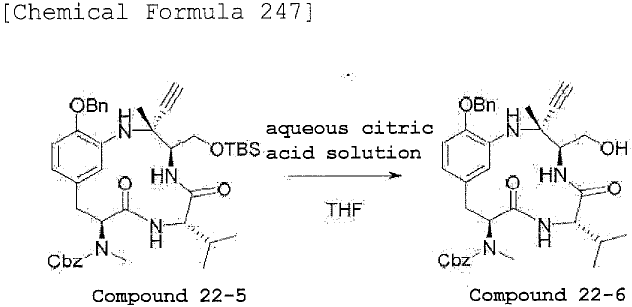

Under nitrogen atmosphere, to a solution of Compound 22-5 (1.0 eq., 364 mg, 0.488 mmol) in a mixture of THF (1.08 mL)/water (1.08 mL) was added citric acid (10.0 eq., 939 mg, 4.89 mmol), and the mixture was stirred at room temperature for 18.5 hr. To the reaction solution were added ethyl acetate (20 mL) and water (20 mL), the mixture was extracted once by liquid separation, and the organic layer was washed once with 5% aqueous sodium hydrogencarbonate solution (20 mL) and once with 5% aqueous sodium chloride solution (20 mL) by liquid separation. The organic layer was dried over sodium sulfate, the sodium sulfate was removed by filtration, and the filtrate was concentrated under reduced pressure to give a crude product (371 mg) as a pale-yellow viscous product. The crude product was purified by flash silica gel column (normal phase silica gel 10 g, hexane/ethyl acetate = 90/10 - 0/100) to give Compound 22-6 (285 mg, containing 3.0wt% ethyl acetate, conversion yield 90%) as a white solid.

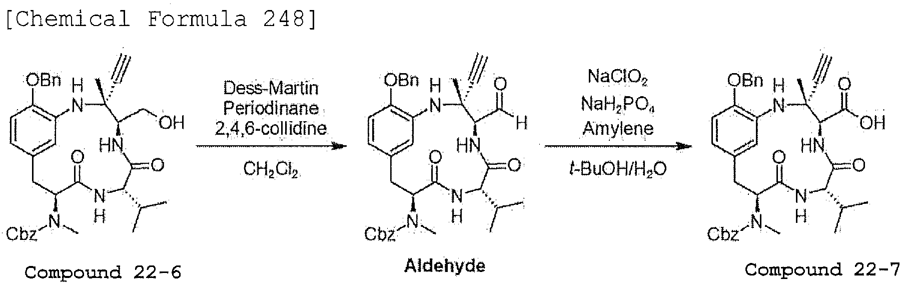

Under nitrogen atmosphere, to a solution of Compound 22-6 (1.0 eq., 235 mg, containing 3.0wt% ethyl acetate, 0.36 mmol) in dichloromethane (2.4 mL) were added 2,4,6-collidine (5.0 eq., 0.24 mL, 1.82 mmol) and Dess-Martin periodinane (1.5 eq., 231 mg, 0.54 mmol) under ice-cooling, and the mixture was stirred at the same temperature for 2 hr. To the reaction solution were added 5% aqueous sodium hydrogencarbonate solution (10 mL), 5% aqueous sodium thiosulfate solution (10 mL) and ethyl acetate (20 mL) at the same temperature, and the mixture was extracted once by liquid separation. The organic layer was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give Aldehyde (262 mg) as an orange amorphous.

Under nitrogen atmosphere, to a solution of Aldehyde (1.0 eq., 258 mg, calculated as 0.36 mmol) in a mixture of t-butyl alcohol (10 mL)/amylene (2.5 mL)/water (2.5 mL) were added sodium dihydrogen phosphate dihydrate (3.5 eq., 197 mg, 1.26 mmol) and 80% sodium chlorite (4.5 eq., 183 mg, 1.62 mmol) at room temperature, and the mixture was stirred at the same temperature for 0.5 hr.

The reaction solution obtained by treating Aldehyde (9.4 mg) in the same manner and the previous reaction solution were combined, ethyl acetate (30 mL) was added, and the mixture was washed twice with 5% aqueous sodium chloride solution (30 mL) by liquid separation, and dried over sodium sulfate. The sodium sulfate was removed by filtration, and the filtrate was concentrated under reduced pressure to give a crude product (303 mg) as a pale-brown amorphous. The crude product was purified twice by flash silica gel column (normal phase silica gel 10 g, 1st run: ethyl acetate/methanol = 100/0 - 70/30, 2nd run: hexane/ethyl acetate = 90/10 - 0/100→ethyl acetate/methanol = 100/0 - 70/30) to give Compound 22-7 (182 mg, containing 3.5wt% ethyl acetate, two-step conversion yield from Compound 22-6 75%).

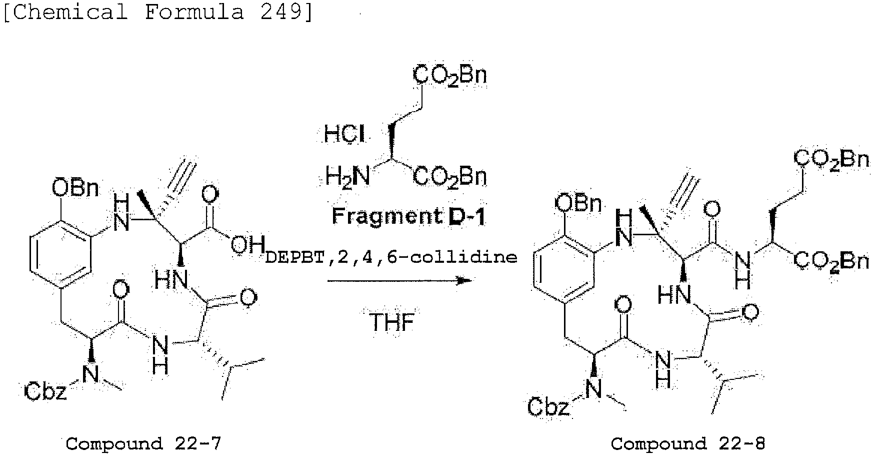

Under nitrogen atmosphere, to a solution of Compound 22-7 (1.0 eq., 156 mg, containing 3.5wt% ethyl acetate, 0.235 mmol) and dibenzyl L-glutamate hydrochloride (Fragment D-1, 1.5 eq., 128 mg, 0.352 mmol) in THF (1 mL) were added 2,4,6-collidine (3.0 eq., 93 µL, 0.706 mmol) and DEPBT (1.5 eq., 105 mg, 0.351 mmol) under ice-cooling, and the mixture was stirred at the same temperature for 5 hr, and then at room temperature for 21.5 hr. To the reaction solution was added ethyl acetate (3 mL), and the mixture was washed once with 5% aqueous citric acid solution (3 mL), once with 5% aqueous sodium hydrogencarbonate solution (3 mL) and once with 5% aqueous sodium chloride solution (3 mL) by liquid separation, and dried over sodium sulfate, and the sodium sulfate was removed by filtration. The filtrate was concentrated under reduced pressure, and the crude product (262 mg) obtained as a pale-brown viscous product was purified by flash silica gel column (normal phase silica gel 10 g, hexane/ethyl acetate = 65/35 - 50/50) to give Compound 22-8 (89.6 mg, containing 1.9wt% ethyl acetate, conversion yield 39%) as a yellow viscous product.

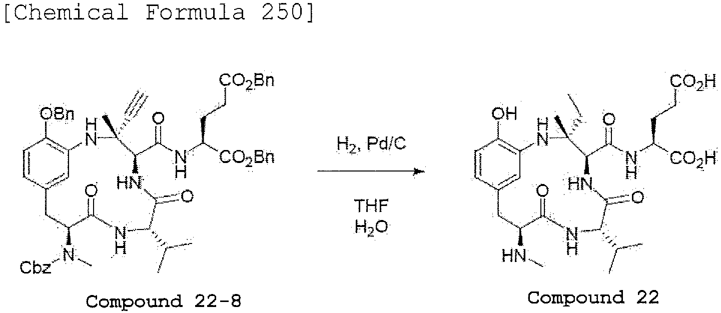

Under nitrogen atmosphere, to a solution of Compound 22-8 (1.0 eq., 20 mg, containing 1.9wt% ethyl acetate, 0.021 mmol) in a mixture of THF (1 mL)/water (50 µL) was added 10% palladium-carbon (N.E. CHEMCAT NX type, 50% hydrous product, 4 mg) at room temperature, the system was replaced with hydrogen, and the mixture was stirred at the same temperature for 22 hr. The catalyst was removed by filtration through Celite, and the filtrate was concentrated under reduced pressure to give a crude product. The crude product was purified by flash silica gel column (reverse-phase silica gel 30 g, 0.05% aqueous TFA solution/acetonitrile = 95/5 - 90/10), and freeze-dried to give a TFA salt of Compound 22 (12.4 mg, yield 77%, purity 98.8%) as a white solid.

¹H-NMR(400MHz,D₂O)δ7.22-7.21(m,2H),7.07-7.05(d,J=8.8Hz,1H),4.36(dd,J=9.2,4.8Hz,1H),4.05-4.02(m,2H),3.36(dd,J=12.8,4.8Hz,1H),2.87 (dd, J=12.0Hz,1H),2.71(s ,3H),2.48-2.34(m,2H),2.21-2.03(m,2H),2.00-1.76(m,3H),1.71(s,3H),1.06(t,J=7.2Hz,3H),0.80(d,J=6.8Hz,3H),0.7 2(d,J=6.8Hz,3H).

### (Example 29A: Synthesis of Compound 24)

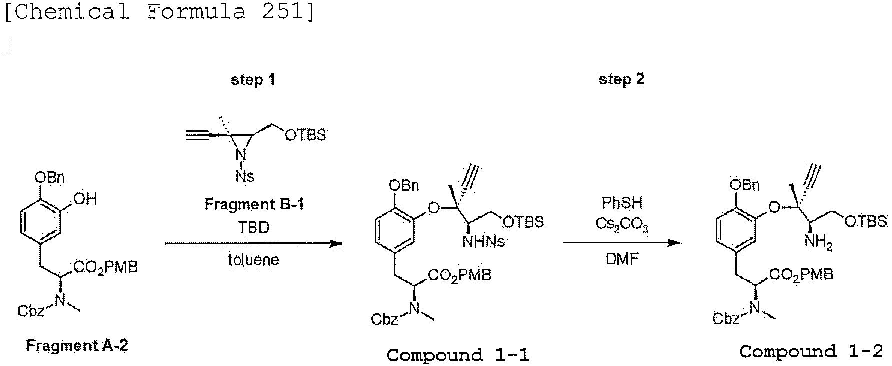

The two steps from Fragment A-2 to Compound 1-2 were carried out as described in Example 7.

Synthesis of Compound 24-1 from Compound 1-2 was carried out as follows.

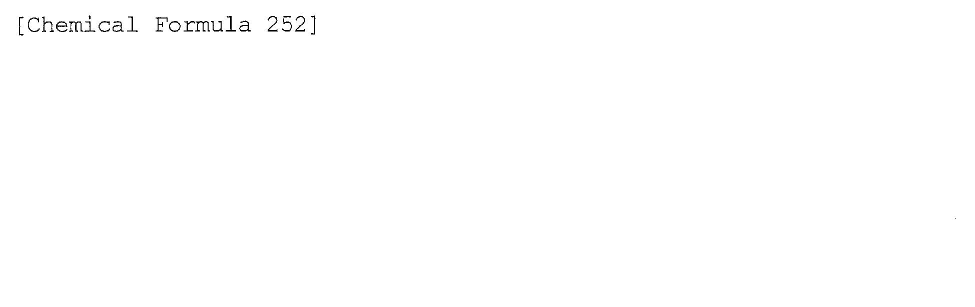

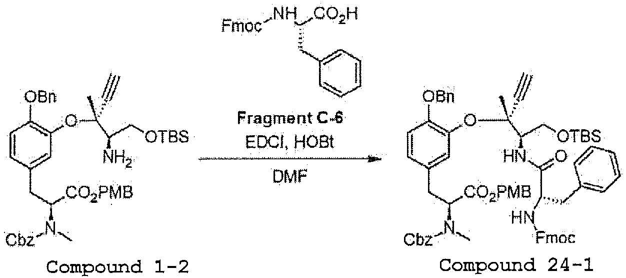

To a solution of Compound 1-2 (1.0 eq., 300 mg, 0.384 mmol) in DMF (5 mL) were added HOBt·H₂O (1.2 eq., 72.8 mg, 0.475 mmol), EDCI (1.2 eq., 88.5 mg, 0.462 mmol) and N-Fmoc-L-phenylalanine (Fragment C-6, 1.2 eq., 182 mg, 0.470 mmol) under ice-cooling, and the mixture was stirred at room temperature for 18 hr. To the reaction solution was added a mixture of hexane (5 mL)/ethyl acetate (15 mL), and the mixture was washed twice with water (10 mL), twice with saturated aqueous sodium hydrogencarbonate solution (10 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give Compound 24-1 (460 mg, crude yield 104%) as a white amorphous.

Synthesis of Compound 24-2 from Compound 24-1 was carried out as follows.

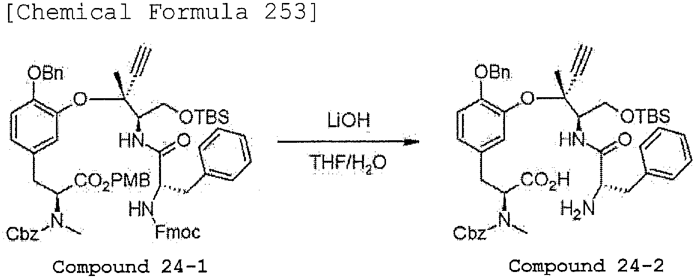

To a solution of Compound 24-1 (1.0 eq., 460 mg, calculated as 0.384 mmol) in a mixture of THF (3 mL)/water (1.5 mL) was added lithium hydroxide (6.0 eq., 55.5 mg, 2.32 mmol) under ice-cooling, and the mixture was stirred at room temperature for 4 hr. The reaction solution was adjusted to pH 7 by addition of 2N aqueous hydrochloric acid solution (0.7 mL) under ice-cooling, and water (10 mL) was added. The mixture was extracted three times with ethyl acetate (10 mL) by liquid separation, and the organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (474 mg) as a pale-yellow solid. The crude product was purified by flash silica gel column (normal phase silica gel 5 g, ethyl acetate/methanol = 100/0 - 50/50) to give Compound 24-2 (267 mg, two-step yield from Compound 1-2 86%) as a white amorphous.

Synthesis of Compound 24-3 from Compound 24-2 was carried out as follows.

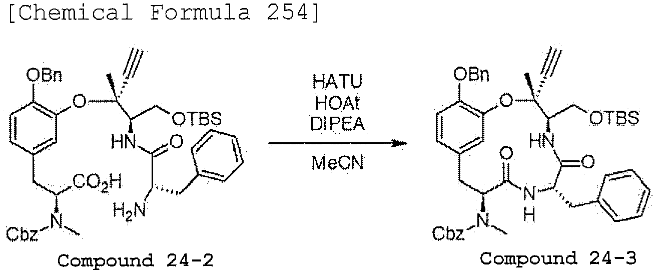

A solution of Compound 24-2 (1.0 eq., 257 mg, 0.318 mmol) in acetonitrile (32 mL) was added dropwise over 16 hr to a solution of HATU (5.0 eq., 604 mg, 1.59 mmol), HOAt (5.0 eq., 216 mg, 1.59 mmol) and DIPEA (10 eq., 410 mg, 24.9 mmol) in acetonitrile (160 mL) at about 33 µL/min at room temperature, and the mixture was stirred at room temperature for 8 hr. The reaction solution was washed once with a mixture of water (50 mL)/saturated aqueous sodium hydrogencarbonate solution (50 mL) and once with saturated aqueous ammonium chloride solution (50 mL) by liquid separation. The aqueous layers were combined, and extracted once with ethyl acetate (100 mL) by liquid separation, and the organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (963 mg) as a brown liquid. The crude product was purified by flash silica gel column (normal phase silica gel 6 g, hexane/ethyl acetate = 80/20 - 17/83) to give Compound 24-3 (109 mg, yield 43%) as a pale-yellow solid.

Synthesis of Compound 24-4 from Compound 24-3 was carried out as follows.

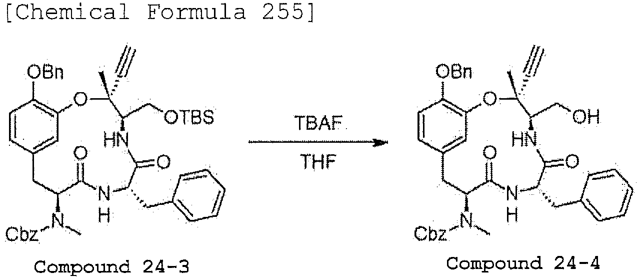

To a solution of Compound 24-3 (1.0 eq., 107 mg, 0.135 mmol) in THF (2 mL) was added TBAF (1M in THF, 2.5 eq., 0.34 mL, 0.340 mmol) at room temperature, and the mixture was stirred at the same temperature for 2 hr. To the reaction solution was added ethyl acetate (10 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (122 mg) as a pale-yellow amorphous. The crude product was purified by flash silica gel column (normal phase silica gel 5 g, hexane/ethyl acetate = 50/5 to 0/100) to give Compound 24-4 (70.7 mg, yield 77%) as a white amorphous.

Synthesis of Compound 24-5 from Compound 24-4 was carried out as follows.

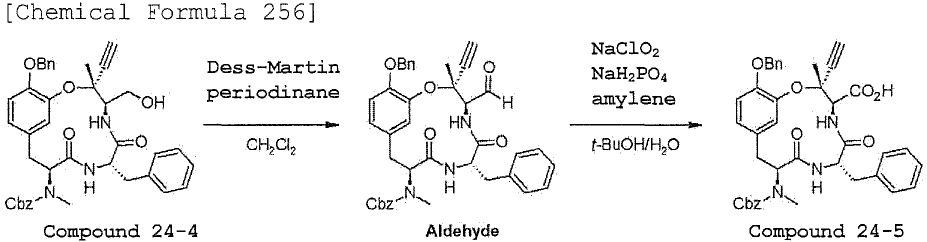

Under nitrogen atmosphere, to a solution of Compound 24-4 (1.0 eq., 68.8 mg, 0.102 mmol) in dichloromethane (11 mL) was added Dess-Martin periodinane (1.5 eq., 65.5 mg, 0.154 mmol) under ice-cooling, and the mixture was stirred at room temperature for 3 hr. To the reaction solution was added additional Dess-Martin periodinane (1.0 eq., 43.3 mg, 0.102 mmol) at the same temperature, and the mixture was stirred for 19 hr. The reaction solution was quenched by addition of saturated aqueous sodium hydrogencarbonate solution (10 mL), and extracted twice with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (77.3 mg, quant.) as a pale-yellow solid.

To a solution of Aldehyde (1.0 eq., 77.3 mg, calculated as 0.102 mmol) in a mixture of amylene (0.5 mL)/t-butyl alcohol (2 mL) was added a solution of sodium dihydrogen phosphate dihydrate (3.5 eq., 56.1 mg, 0.360 mmol) and 80% sodium chlorite (4.5 eq., 51.3 mg, 0.454 mmol) in water (0.5 mL) at room temperature, and the mixture was stirred at the same temperature for 1 hr. To the reaction solution was added saturated aqueous ammonium chloride solution (10 mL). The mixture was extracted twice with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (74.8 mg) as a pale-yellow solid. The crude product was purified by flash silica gel column (normal phase silica gel 5 g, hexane/ethyl acetate = 25/75 - 0/100) to give Compound 24-5 (49.5 mg, two-step yield from Compound 24-4 70%) as a white amorphous.

Synthesis of Compound 24-6 from Compound 24-5 was carried out as follows.

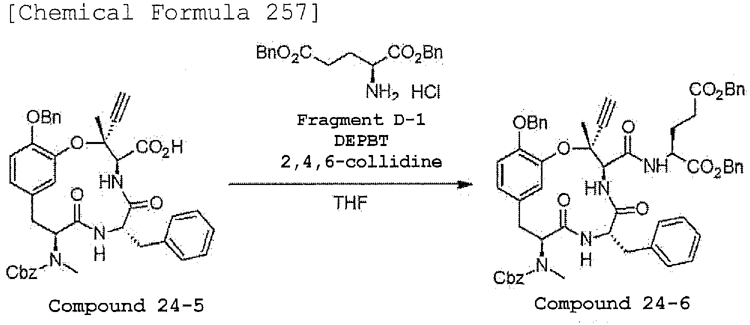

Under nitrogen atmosphere, to a solution of Compound 24-5 (1.0 eq., 49.0 mg, 71.0 µmol) and dibenzyl L-glutamate hydrochloride (Fragment D-1, 1.5 eq., 38.9 mg, 107 µmol) in THF (1 mL) were added DEPBT (1.5 eq., 31.8 mg, 107 µmol) and 2,4,6-collidine (3.0 eq., 25.8 mg, 213 µmol) under ice-cooling, and the mixture was stirred at the same temperature for 3 hr, and then at room temperature for 18 hr. To the reaction solution was added ethyl acetate (10 mL), and the mixture was washed once with a mixture of water (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (96.9 mg) as a brown oil. The crude product was purified twice by flash silica gel column (1st run: NH₂ silica gel 6 g, hexane/ethyl acetate = 75/25 - 50/50, 2nd run: normal phase silica gel 5 g, hexane/ethyl acetate = 67/23 - 50/50) to give Compound 24-6 (22.9 mg, yield 32%) as a white amorphous.

Synthesis of Compound 24 from Compound 24-6 was carried out as follows.

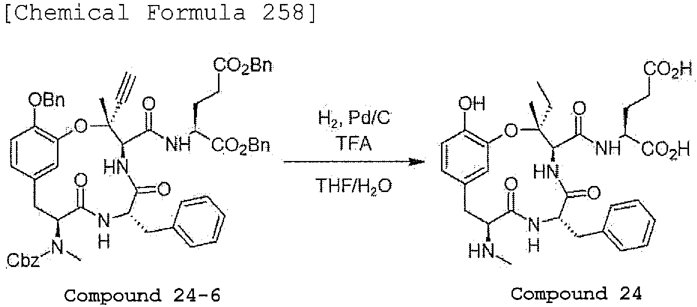

Under nitrogen atmosphere, to a solution of Compound 24-6 (1.0 eq., 23 mg, 0.023 mmol) in a mixture of THF (1400 µL)/water (70 µL) were added 10% palladium-carbon (N.E. CHEMCAT NX type, 50% hydrous product, 2.1 mg) and TFA (5.0 eq., 8.8 µL, 0.115 mmol) at room temperature, the system was replaced with hydrogen, and the mixture was stirred at the same temperature for 29.5 hr. During this period, 1.2 mg of 10% palladium-carbon was added after 20 hr and 26 hr. The catalyst was removed by filtration through Celite, and the filtrate was concentrated under reduced pressure to give a crude product. The crude product was purified by flash silica gel column (reverse-phase silica gel 30 g, 0.05% aqueous TFA solution/acetonitrile = 95/5 - 85/15), and freeze-dried to give a TFA salt of Compound 24 (8.7 mg, yield 53%, purity 99.8%) as a white solid.

¹H-NMR(400MHz,D₂O)δ7.38-7.15(m,5H)6.92(s,3H)4.74-4,71(m,1H),4.22(dd,J=8.0,6.0Hz,1H),3.87(dd,J=9.2,4.4Hz,1H),3.18 (dd, J=13.2,4.4Hz,1H),3.10 (dd, J=14.4,7.6Hz,1H),2.90(dd,J=13.6,9. 6Hz,1H),2.81(dd,J=13.6,8.8Hz,1H),2.48-2.27(m,5H),2.18-2.04(m,1H),2.03-1.87(m,2H),1.75-1.63(m,1H),1.54(s,3H),1.00(t,J=7.2Hz,3H).

### (Example 29B: Synthesis of Compound 25)

Compound 25 was synthesized.

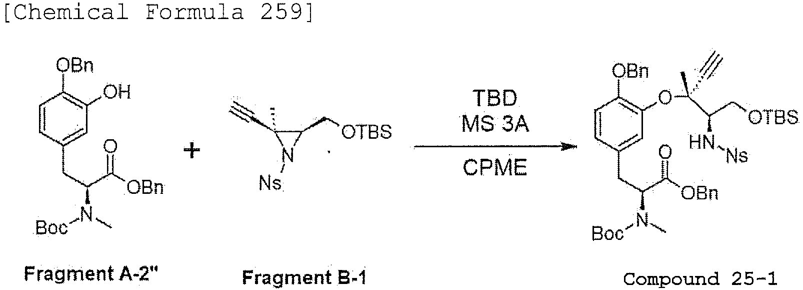

To a solution of Fragment A-2 (1.2 eq., 9.47 g, 16.0 mmol) in CPME (40 mL) was added MS3Å (3.0 g). TBD (1.1 eq., 2.46 g, 17.6 mmol) was added, and then a solution of Fragment B-1 (1.0 eq., 6.59 g, 16.0 mmol) in CPME (40 mL) was added dropwise thereto over 35 min under ice-cooling. The system was replaced with nitrogen, and the mixture was stirred at room temperature for 4 hr. Additional TBD (0.20 eq., 447 mg, 3.21 mmol) was added, and the mixture was stirred at the same temperature for 19 hr. The MS3Å was removed by filtration, and the filtrate was concentrated under reduced pressure to give a crude product (20.4 g) as a brown viscous product. The crude product was purified by flash silica gel column (NH₂ silica gel 200 g, hexane/ethyl acetate = 80/20 - 0/100) to give Compound 25-1 (13.1 g, yield 91%) as a yellow viscous product.

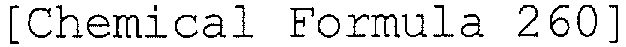

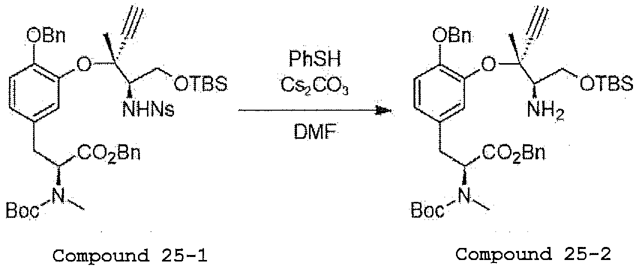

Under nitrogen atmosphere, to a solution of Compound 25-1 (1.0 eq., 2.56 g, 2.83 mmol) in DMF (28 mL) were added cesium carbonate (1.5 eq., 1.40 g, 4.29 mmol) and thiophenol (1.6 eq., 0.45 mL, 4.41 mmol) at room temperature, and the mixture was stirred at room temperature for 2.5 hr. To the reaction solution was added a mixture of hexane (40 mL)/ethyl acetate (120 mL), and the mixture was washed three time with water (100 mL) and once with saturated sodium chloride (50 mL) by liquid separation. The organic layer was dried over magnesium sulfate, the magnesium sulfate was removed by filtration, and the filtrate was concentrated under reduced pressure to give a crude product (3.08 g). The crude product was purified by flash silica gel column (normal phase silica gel 60 g, hexane/ethyl acetate = 75/25 - 0/100) to give Compound 25-2 (2.13 g, crude yield 105%) as a brown oil.

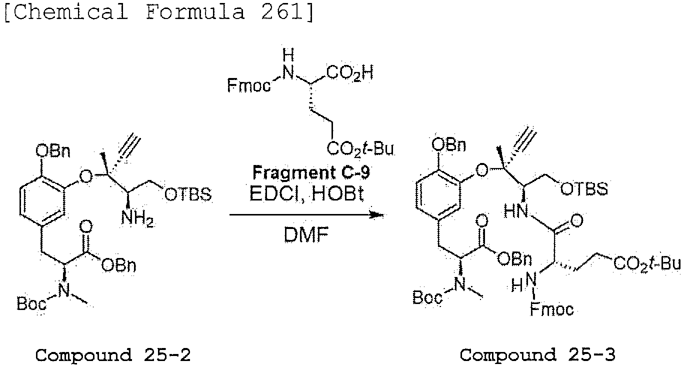

Under nitrogen atmosphere, to a solution of Compound 25-2 (1.0 eq., 727 mg, 1.01 mmol) and 5-t-Bu-N-Fmoc-L-glutamic acid (Fragment C-9: 1.2 eq., 519 mg, 1.22 mmol) in DMF (10 mL) were added HOBt·H₂O (1.2 eq., 187 mg, 1.22 mmol) and EDCI (1.2 eq., 232 mg, 1.21 mmol) at room temperature, and the mixture was stirred at the same temperature for 2.5 hr. To the reaction solution was added a mixture of hexane (4 mL)/ethyl acetate (12 mL), and the mixture was washed once with water (10 mL) by liquid separation. The aqueous layer was extracted once with a mixture of hexane (5 mL)/ethyl acetate (5 mL) by liquid separation, and the organic layers were combined, and washed twice with water (10 mL) and once with aqueous sodium chloride solution (5 mL) by liquid separation. The organic layer was dried over magnesium sulfate, the magnesium sulfate was removed by filtration, and the filtrate was concentrated under reduced pressure to give Compound 25-3 (1.22 g, crude yield 108%) as a white amorphous.

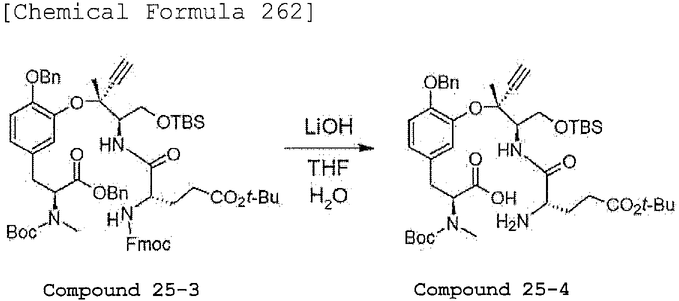

Under nitrogen atmosphere, to a solution of Compound 25-3 (1.0 eq., 1.22 g, calculated as 1.01 mmol) in a mixture of THF (7 mL)/water (3 mL) was added lithium hydroxide (4.0 eq., 95.3 mg, 4.00 mmol) at room temperature, and the mixture was stirred at the same temperature for 2.5 hr. 2N Aqueous hydrochloric acid solution (1.0 mL) was added under ice-cooling, and the mixture was extracted once with ethyl acetate (10 mL) and once with ethyl acetate (5 mL) by liquid separation. The organic layer was dried over magnesium sulfate, the magnesium sulfate was removed by filtration, and the filtrate was concentrated under reduced pressure to give a crude product as a brown liquid. The crude product was purified by flash silica gel column (normal phase silica gel 8.8 g, ethyl acetate/methanol = 1/0 - 3/1) to give Compound 25-4 (565 mg, two-step yield 69%) as a white amorphous.

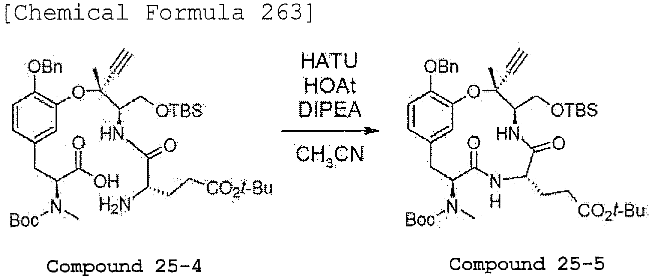

A solution of Compound 25-4 (1.0 eq., 565 mg, 696 µmol) in acetonitrile (18 mL)/THF (18 mL) was added dropwise over 5.5 hr to a solution of HATU (2.0 eq., 531 mg, 1.40 mmol), HOAt (2.0 eq., 190 mg, 1.39 mmol) and DIPEA (2.0 eq., 240 µL, 1.41 mmol) in acetonitrile (350 mL) at room temperature, and the mixture was stirred at room temperature for additional 18 hr. The reaction solution was concentrated under reduced pressure to about 20 mL, and washed once with saturated aqueous ammonium chloride solution (8 mL) by liquid separation. The aqueous layer was extracted once with ethyl acetate (5 mL) by liquid separation, and the organic layers were combined, and washed once with saturated aqueous ammonium chloride solution (8 mL) and once with saturated aqueous sodium chloride solution (8 mL) by liquid separation. The organic layer was dried over magnesium sulfate, the magnesium sulfate was removed by filtration, and the filtrate was concentrated under reduced pressure to give a crude product (868 mg) as an orange amorphous. The crude product was purified by flash silica gel column (normal phase silica gel 7.3 g, hexane/ethyl acetate = 3/1 - 2/1) to give Compound 25-5 (160 mg, 29%) as a white amorphous.

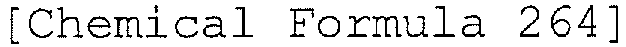

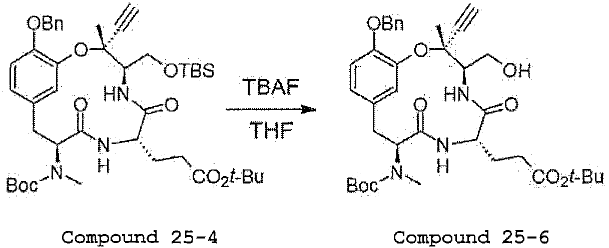

Under nitrogen atmosphere, to a solution of Compound 25-5 (1.0 eq., 160 mg, 201 µmol,) in THF (2 mL) was added TBAF (1M in THF, 1.5 eq., 300 µL, 300 µmol) at room temperature, and the mixture was stirred at room temperature for 1.5 hr. To the reaction solution was added saturated aqueous ammonium chloride solution (2 mL), and the mixture was extracted once with ethyl acetate (8 mL) and once with ethyl acetate (5 mL) by liquid separation. The organic layers were combined, and washed three time with saturated aqueous ammonium chloride solution (3 mL) and once with saturated aqueous sodium chloride solution (3 mL) by liquid separation. The organic layer was dried over magnesium sulfate, the magnesium sulfate was removed by filtration, and the filtrate was concentrated under reduced pressure to give a crude product (156 mg) as a pale-brown amorphous. The crude product was purified by flash silica gel column (normal phase silica gel 6.0 g, hexane/ethyl acetate = 3/2 to 0/1) to give Compound 25-6 (116 mg, 85%) as a white amorphous.

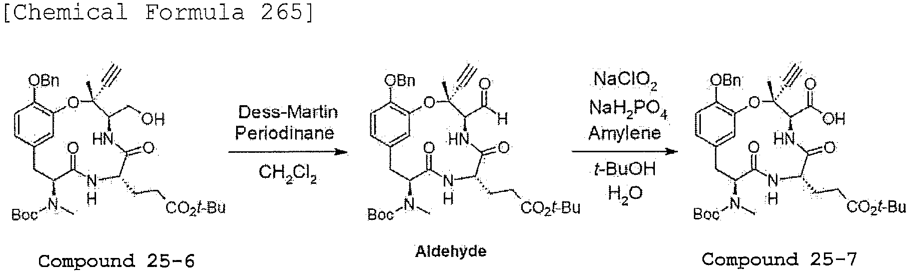

Under nitrogen atmosphere, to a solution of Compound 25-6 (1.0 eq., 116 mg, 171 µmol) in dichloromethane (1.7 mL) was added Dess-Martin periodinane (1.5 eq., 109 mg, 257 µmol) under ice-cooling, and the mixture was stirred at room temperature for 1.5 hr. To the reaction solution was added a mixture of 10% sodium hydrogen sulfite aqueous solution (1 mL)/saturated aqueous sodium hydrogencarbonate solution (1 mL), and the mixture was extracted once with ethyl acetate (10 mL) by liquid separation, and the organic layer was washed three time with a mixture of 10% sodium hydrogen sulfite aqueous solution (1 mL)/saturated aqueous sodium hydrogencarbonate solution (1 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give Aldehyde (121 mg) as a white amorphous.

To a solution of Aldehyde (1.0 eq., 121 mg, calculated as 171 µmol) in a mixture of t-butyl alcohol (0.9 mL)/amylene (0.4 mL) was added dropwise a solution of sodium dihydrogen phosphate dihydrate (3.3 eq., 88.8 mg, 569 µmol) and 80% sodium sulfite (3.6 eq., 69.6 mg, 616 µmol) in water (0.4 ml) at room temperature, and the mixture was stirred at the same temperature for 2.5 hr. To the reaction solution was added saturated aqueous ammonium chloride solution (2 mL), and the mixture was extracted once with ethyl acetate (7 mL) and once with ethyl acetate (2 mL) by liquid separation. The organic layers were combined, and dried over magnesium sulfate, the magnesium sulfate was removed by filtration, and the filtrate was concentrated under reduced pressure to give a crude product (135 mg) as a white amorphous. The crude product was slurrywashed with a mixture of hexane (1.8 mL)/ethyl acetate (0.2 mL) to give Compound 25-7 (122 mg, crude yield 103%, containing impurities) as a white amorphous.

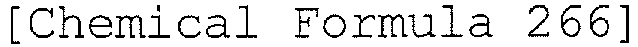

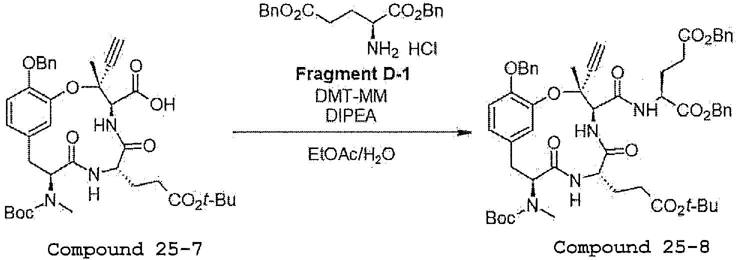

Under nitrogen atmosphere, to a solution of Compound 25-7 (1.0 eq., 122 mg, calculated as 171 µmol) and dibenzyl L-glutamate hydrochloride (Fragment D-1: 1.5 eq, 95.9 mg, 264 µmol) in a mixture of ethyl acetate (1.3 mL)/water (0.4 mL) were added DIPEA (2.5 eq., 73 µL, 429 µmol) and DMT-MM (1.7 eq., 82.1 mg, 297 µmol) under ice-cooling, and the mixture was stirred under ice-cooling for 2 hr. To the reaction solution was added ethyl acetate (7 mL), and the mixture was washed once with water (2 mL), once with saturated aqueous ammonium chloride solution (2 mL), once with saturated aqueous sodium hydrogencarbonate solution (2 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give a crude product (203 mg). The crude product was purified by flash silica gel column (NH₂ silica gel 5.0 g, hexane/ethyl acetate = 3/1 - 1/1) to give Compound 25-8 (128 mg, three-step yield 75%) as a white amorphous.

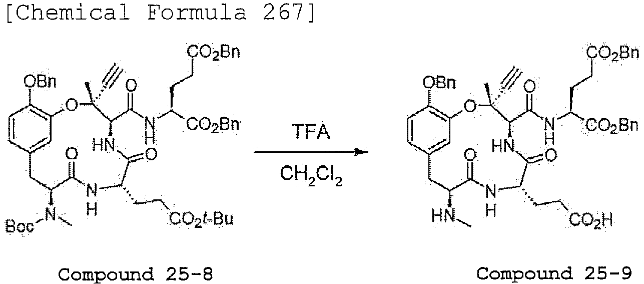

Under nitrogen atmosphere, to a solution of Compound 25-8 (1.0 eq., 128 mg, 128 µmol) in dichloromethane (1.2 mL) was added TFA (12 eq., 120 µL, 1.56 mmol) at room temperature, and the mixture was stirred at room temperature for 6 hr. Additional TFA (4.0 eq., 40 µL, 392 µmol) was added at room temperature, and the mixture was stirred at room temperature for 18 hr. To the reaction solution were added saturated aqueous sodium hydrogencarbonate solution (1.5 mL) and water (2 mL), and the mixture was extracted once with dichloromethane (5 mL) and twice with dichloromethane (3 mL) by liquid separation. The organic layer was dried over magnesium sulfate, the magnesium sulfate was removed by filtration, and the filtrate was concentrated under reduced pressure to give a crude product (107 mg) as a pale-brown amorphous. The crude product was dissolved in ethyl acetate (1 mL), hexane (0.5 mL) was added dropwise, and the precipitated white solid was collected by filtration to give Compound 25-9 (101 mg, yield 93%).

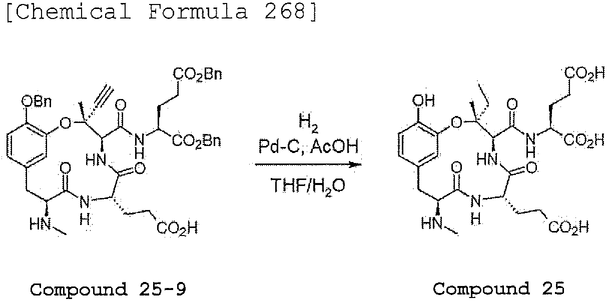

To a solution of Compound 25-9 (1.0 eq., 101 mg, 45.8 µmol) in a mixture of THF (2 mL)/water (1 mL) was added acetic acid (5.0 eq., 34 µL, 595 µmol) at room temperature, and the mixture was stirred at room temperature for 20 min. 10% Palladium-carbon (N.E. CHEMCAT NX type, 50% hydrous product, 10.6 mg) was added at room temperature, the system was replaced with hydrogen, and the mixture was stirred at the same temperature for 22 hr. The catalyst was removed by filtration through Celite, and the filtrate was concentrated under reduced pressure to give a crude product. The crude product was purified twice by flash silica gel column (reverse-phase silica gel 10 g, 1st run: 0.05% aqueous acetic acid solution/acetonitrile = 95/5 - 0/100, 2nd run: 0.05% aqueous acetic acid solution/acetonitrile = 95/5 - 92/5), and freeze-dried to give an acetate of Compound 25 (3.0 mg, yield 4.4%, purity 96.9%) as a white solid.

¹H-NMR(400MHz,D₂O)δ6.89-6.79(m,3H),4.37(dd,J=13,6.4Hz,1H),4.18(dd,J=8.8,4.8Hz,1H),3.87( dd,J=9.2,4.4Hz,1H),3.12(dd,J=13.4,4.6Hz,1H),2.83(dd,J=13.2,9.2H z,1H)2.60(s,3H),2.27(t,J=7.6Hz,2H),2.18-2.14(m,2H),2.11-1.99(m,1H),1.90-1.79(m,3H),1.76-1.68(m,1H,),1.67-1.57(m,1H),1.44(s,3H),0.87(t,J=7.2Hz,3H).

### (Example 29C: Synthesis of Compound 27)

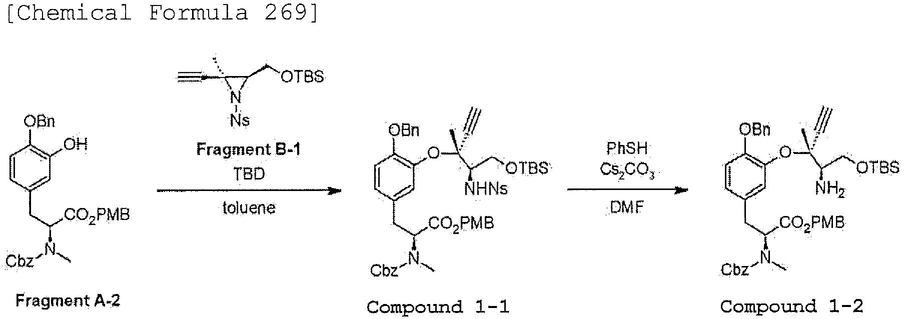

The two steps from Fragment A-2 to Compound 1-2 were carried out as described in Example 7.

Synthesis of Compound 27-1 from Compound 1-2 was carried out as follows.

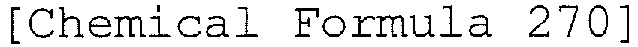

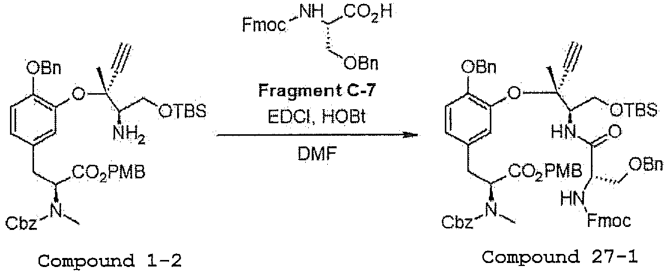

To a solution of Compound 1-2 (1.0 eq., 583 mg, 0.749 mmol) in DMF (8 mL) were added HOBt·H₂O (1.2 eq., 139 mg, 0.906 mmol), EDCI (1.2 eq., 174 mg, 0.906 mmol) and Fmoc-L-Ser(Bzl)-OH (Fragment C-7, 1.2 eq., 375 mg, 0.897 mmol) under ice-cooling, and the mixture was stirred at room temperature for 3 hr. To the reaction solution was added a mixture of hexane (10 mL)/ethyl acetate (30 mL), and the mixture was washed twice with water (20 mL), twice with a mixture of water (10 mL)/saturated aqueous sodium hydrogencarbonate solution (10 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give Compound 27-1 (898 mg, crude yield 102%) as a white amorphous.

Synthesis of Compound 27-2 from Compound 27-1 was carried out as follows.

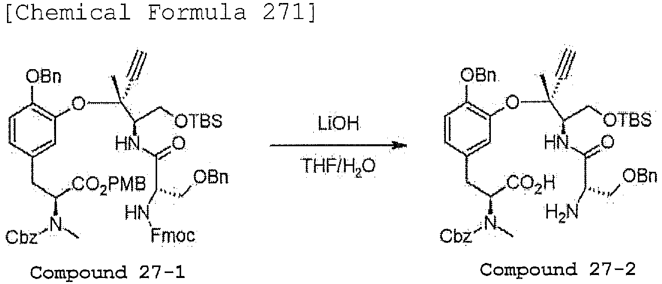

To a solution of Compound 27-1 (1.0 eq., 898 mg, calculated as 0.747 mmol) in a mixture of THF (8 mL)/water (4 mL) was added lithium hydroxide (6.1 eq., 110 mg, 4.57 mmol) under ice-cooling, and the mixture was stirred at room temperature for 3.5 hr. The reaction solution was adjusted to pH 7 by addition of 2N aqueous hydrochloric acid solution (1.3 mL). To the reaction solution was added ethyl acetate (20 mL), and the mixture was washed twice with water (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (1.13 g) as a yellow oil. The crude product was purified by flash silica gel column (normal phase silica gel 15 g, ethyl acetate/methanol = 100/0 - 50/50) to give Compound 27-2 (621 mg, two-step yield from Compound 1-2 99%) as a white amorphous.

Synthesis of Compound 27-3 from Compound 27-2 was carried out as follows.

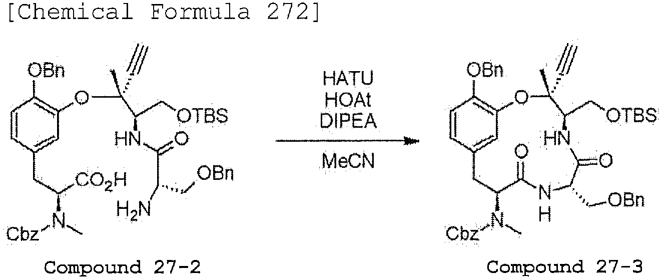

A solution of Compound 27-2 (1.0 eq., 605 mg, 0.722 mmol) in acetonitrile (72 mL) was added dropwise over 23 hr to a solution of HATU (5.0 eq., 1.37 mg, 3.61 mmol), HOAt (5.0 eq., 493 mg, 3.62 mmol) and DIPEA (9.8 eq., 912 mg, 7.06 mmol) in acetonitrile (360 mL) at about 53 µl/min at room temperature, and the mixture was stirred at room temperature for 17 hr. The reaction solution was washed once with a mixture of water (200 mL)/saturated aqueous sodium hydrogencarbonate solution (100 mL) and once with a mixture of water (50 mL)/saturated aqueous ammonium chloride solution (50 mL) by liquid separation. The aqueous layers were combined, and extracted once with ethyl acetate (100 mL) by liquid separation, and the organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (1.94 g) as a brown oil. The crude product was purified by flash silica gel column (normal phase silica gel 28 g, hexane/ethyl acetate = 80/20 - 25/75) to give Compound 27-3 (154 mg, yield 26%) as a white amorphous.

Synthesis of Compound 27-4 from Compound 27-3 was carried out as follows.

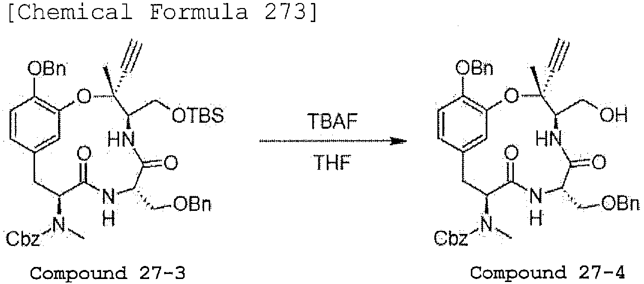

To a solution of Compound 27-3 (1.0 eq., 135 mg, 0.165 mmol) in THF (2 mL) was added TBAF (1M in THF, 1.5 eq., 0.25 mL, 0.250 mmol) at room temperature, and the mixture was stirred at the same temperature for 2 hr. To the reaction solution was added additional TBAF (1M in THF, 0.73 eq., 0.12 mL, 0.120 mmol) at room temperature, and the mixture was stirred at the same temperature for 3 hr. To the reaction solution was added ethyl acetate (10 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (122 mg) as a yellow amorphous. The crude product was purified by flash silica gel column (normal phase silica gel 4 g, hexane/ethyl acetate = 50/50 - 17/83) to give Compound 27-4 (89.9 mg, yield 77%) as a white amorphous.

Synthesis of Compound 27-5 from Compound 27-4 was carried out as follows.

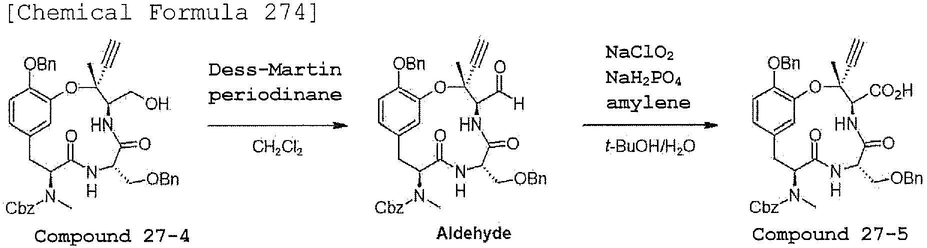

Under nitrogen atmosphere, to a solution of Compound 27-4 (1.0 eq., 89.9 mg, 0.127 mmol) in dichloromethane (1 mL) was added Dess-Martin periodinane (1.5 eq., 81.7 mg, 0.193 mmol) under ice-cooling, and the mixture was stirred at room temperature for 2 hr. To the reaction solution was added ethyl acetate (20 mL), and the mixture was washed once with a mixture of 10% aqueous sodium sulfite solution (9 mL)/saturated aqueous sodium hydrogencarbonate solution (9 mL) by liquid separation. The aqueous layer was extracted three times with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (87.7 mg, yield 98%) as a white amorphous.

Under nitrogen atmosphere, to a solution of Aldehyde (1.0 eq., 87.0 mg, 0.124 mmol) in a mixture of amylene (0.5 mL)/t-butyl alcohol (2 mL) was added a solution of sodium dihydrogen phosphate dihydrate (3.4 eq., 66.5 mg, 0.426 mmol) and 80% sodium chlorite (4.4 eq., 61.9 mg, 0.548 mmol) in water (0.5 mL) at room temperature, and the mixture was stirred at the same temperature for 17 hr. To the reaction solution was added saturated aqueous ammonium chloride solution (15 mL), and the mixture was extracted three times with ethyl acetate (15 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (93.2 mg) as a white amorphous. The crude product was purified by flash silica gel column (normal phase silica gel 5 g, hexane/ethyl acetate = 50/50 - 40/60) to give Compound 27-5 (81.4 mg, yield 91%) as a white amorphous.

Synthesis of Compound 27-6 from Compound 27-5 was carried out as follows.

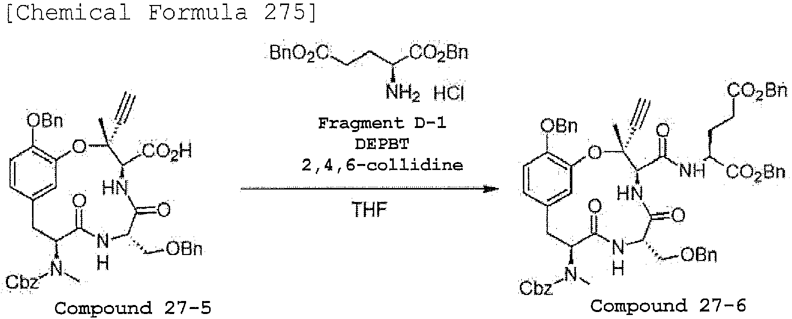

Under nitrogen atmosphere, to a solution of Compound 27-5 (1.0 eq., 79.4 mg, 110 µmol) and dibenzyl L-glutamate hydrochloride (Fragment D-1, 1.5 eq., 61.0 mg, 168 µmol) in THF (1 mL) were added DEPBT (1.5 eq., 50.2 mg, 168 µmol) and 2,4,6-collidine (3.0 eq., 40.5 mg, 334 µmol) under ice-cooling, and the mixture was stirred at the same temperature for 6.5 hr, and then at room temperature for 15.5 hr. To the reaction solution was added ethyl acetate (20 mL), and the mixture was washed once with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (151 mg) as a yellow oil. The crude product was purified twice by flash silica gel column (1st run: NH₂ silica gel 6 g, hexane/ethyl acetate = 75/25 - 50/50, 2nd run: normal phase silica gel 5 g, hexane/ethyl acetate = 67/23 - 50/50) to give Compound 27-6 (31.4 mg, yield 28%) as a white amorphous.

Synthesis of Compound 27 from Compound 27-6 was carried out as follows.

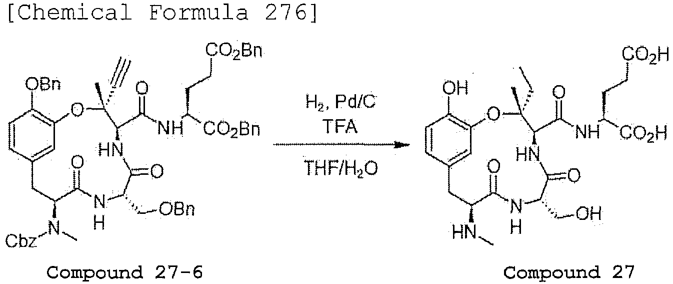

Under nitrogen atmosphere, to a solution of Compound 27-6 (1.0 eq., 20 mg, 0.0194 mmol) in a mixture of THF (2000 µL)/water (100 µL) were added 10% palladium-carbon (N.E. CHEMCAT NX type, 50% hydrous product, 2.0 mg) and TFA (2.0 eq., 3 µL, 0.0392 mmol) at room temperature, the system was replaced with hydrogen, and the mixture was stirred at the same temperature for 23 hr. The catalyst was removed by filtration through Celite, and the filtrate was concentrated under reduced pressure to give a crude product. The crude product obtained from Compound 27-6 (10 mg, 0.0097 mmol) by the same procedure and the aforementioned crude product were combined, and purified by flash silica gel column (reverse-phase silica gel 30 g, 0.05% aqueous TFA solution/acetonitrile = 95/5 - 90/10), and freeze-dried to give a TFA salt of Compound 27 (12.8 mg, yield 67%, purity 96.8%) as a white solid.

¹H-NMR(400MHz,D₂O)δ7.19(s,1H)6.99(dd,J=8.0,2.0Hz,1H)6.94(d,J=8.4 Hz,1H),4.73-4.70(m,1H),4.58-4.53(m,1H),4.50(dd,J=9.2,4.8Hz,1H),4.02(dd,J=9.6,4.0Hz,1H),3.83

3.70(m,2H),3.24(dd,J=13.2,4.0Hz,1H),3.01(dd,J=13.2,9.6Hz,1H),2. 73(s,3H),2.56-2.39(m,2H),2.30-2.18(m,1H),2.08-1.91(m,2H),1.80-1.67(m,1H),1.59(s,3H),0.93(t,J=7.2Hz,3H).

### (Example 29D: Synthesis of Compound 28)

Synthesis of Compound 28 was carried out.

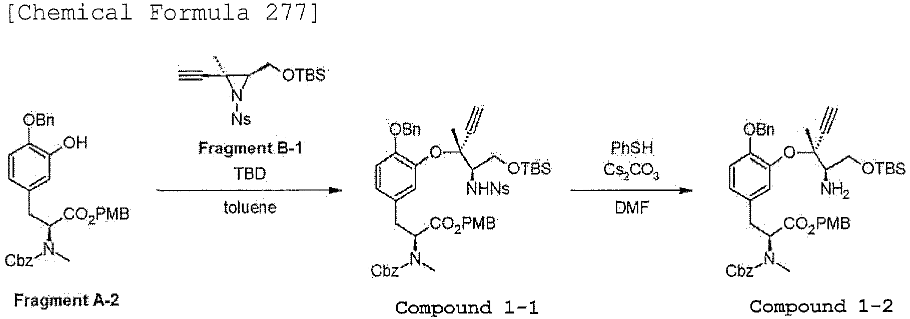

The two steps from Fragment A-2 to Compound 1-2 were carried out as described in Example 7.

Synthesis of Compound 28-1 from Compound 1-2 was carried out as follows.

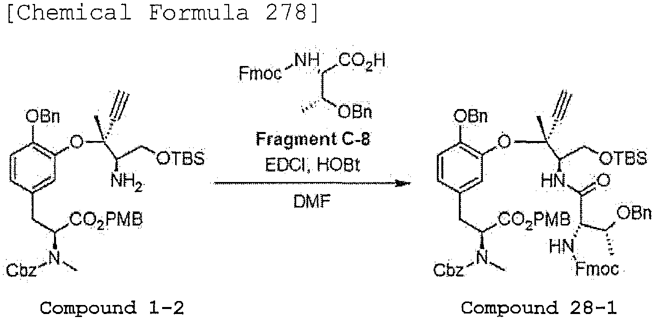

To a solution of Compound 1-2 (1.0 eq., 582 mg, 0.745 mmol) in DMF (8 mL) were added HOBt·H₂O (1.2 eq., 138 mg, 0.896 mmol), EDCI (1.2 eq., 172 mg, 0.899 mmol) and Fmoc-L-Thr(Bzl)-OH (Fragment C-8, 1.2 eq., 387 mg, 0.897 mmol) under ice-cooling, and the mixture was stirred at room temperature for 2 hr. To the reaction solution was added a mixture of hexane (5 mL)/ethyl acetate (15 mL), and the mixture was washed twice with water (10 mL), twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give crude Compound 28-1 (837 mg, crude yield 94%) as a white amorphous.

Synthesis of Compound 28-2 from Compound 28-1 was carried out as follows.

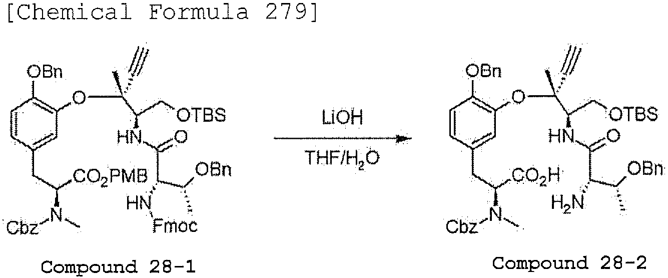

To a solution of Compound 28-1 (1.0 eq., 825 mg, 0.691 mmol) in a mixture of THF (7 mL)/water (3.5 mL) was added lithium hydroxide (6.0 eq., 100 mg, 4.18 mmol) at room temperature, and the mixture was stirred at room temperature for 2.5 hr. The reaction solution was adjusted to pH 7 by addition of 2N aqueous hydrochloric acid solution (1.2 mL), to the reaction solution was added ethyl acetate (20 mL), and the mixture was washed twice with water (10 mL) by liquid separation. The aqueous layers were combined, and extracted once with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (957 mg) as a yellow oil. The crude product was purified by flash silica gel column (normal phase silica gel 15 g, ethyl acetate/methanol = 100/0 - 50/50) to give Compound 28-2 (526 mg, yield 89%) as a white amorphous.

Synthesis of Compound 28-3 from Compound 28-2 was carried out as follows.

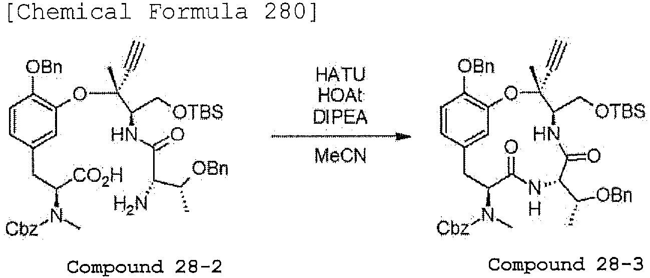

A solution of Compound 28-2 (1.0 eq., 523 mg, 0.614 mmol) in acetonitrile (62 mL) was added dropwise over 18 hr to a solution of HATU (5.0 eq., 1.17 mg, 3.07 mmol), HOAt (5.0 eq., 419 mg, 3.08 mmol) and DIPEA (9.8 eq., 760 mg, 5.88 mmol) in acetonitrile (310 mL) at about 57 µl/min at room temperature, and the mixture was stirred at room temperature for 3 hr. The reaction solution was washed once with a mixture of water (160 mL)/saturated aqueous sodium hydrogencarbonate solution (40 mL) and once with a mixture of water (80 mL)/saturated aqueous ammonium chloride solution (20 mL) by liquid separation. The aqueous layers were combined, and extracted once with ethyl acetate (100 mL) by liquid separation, and the organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (1.20 g) as a brown oil. The crude product was purified by flash silica gel column (normal phase silica gel 28 g, hexane/ethyl acetate = 80/20 - 67/23) to give Compound 28-3 (146 mg, yield 28%) as a white amorphous.

Synthesis of Compound 28-4 from Compound 28-3 was carried out as follows.

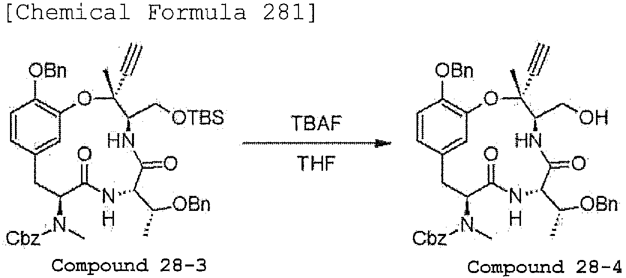

To a solution of Compound 28-3 (1.0 eq., 141 mg, 0.169 mmol) in THF (2 mL) was added TBAF (1M in THF, 2.5 eq., 0.42 mL, 0.420 mmol) under ice-cooling, and the mixture was stirred at room temperature for 2 hr. To the reaction solution was added ethyl acetate (10 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (106 mg) as a pale-yellow amorphous. The crude product was purified by flash silica gel column (normal phase silica gel 5 g, hexane/ethyl acetate = 50/50 - 17/83) to give Compound 28-4 (58.7 mg, yield 48%) as a white amorphous.

Synthesis of Compound 28-5 from Compound 28-4 was carried out as follows.

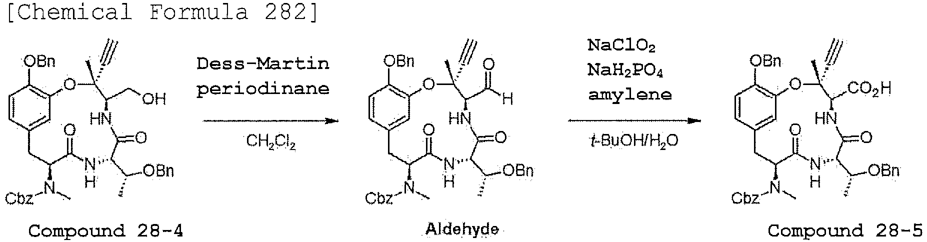

Under nitrogen atmosphere, to a solution of Compound 28-4 (1.0 eq., 55.9 mg, 77.7 µmol) in dichloromethane (1 mL) under was added Dess-Martin periodinane (1.5 eq., 49.9 mg, 118 µmol) ice-cooling, and the mixture was stirred at room temperature for 1.5 hr. To the reaction solution was added Dess-Martin periodinane (0.49 eq., 16.3 mg, 38.4 µmol) under ice-cooling, and the mixture was stirred at room temperature for 2.5 hr. To the reaction solution was added ethyl acetate (20 mL), and the mixture was washed once with a mixture of 10% aqueous sodium sulfite solution (9 mL)/saturated aqueous sodium hydrogencarbonate solution (9 mL) by liquid separation. The aqueous layer was extracted twice with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (57.9 mg, crude yield 104%) as a white amorphous.

To a solution of Aldehyde (1.0 eq., 57.1 mg, calculated as 77.7 µmol) in a mixture of amylene (0.5 mL)/t-butyl alcohol (2 mL) was added a solution of sodium dihydrogen phosphate dihydrate (3.4 eq., 41.4 mg, 265 µmol) and 80% sodium chlorite (3.8 eq., 33.2 mg, 294 µmol) in water (0.5 mL) at room temperature, and the mixture was stirred at the same temperature for 1 hr. To the reaction solution was added saturated aqueous ammonium chloride solution (15 mL), and the mixture was extracted three times with ethyl acetate (15 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (61.8 mg) as a white amorphous. The crude product was purified by flash silica gel column (normal phase silica gel 5 g, hexane/ethyl acetate = 33/67) to give Compound 28-5 (47.8 mg, two-step yield from Compound 28-4 84%) as a white amorphous.

Synthesis of Compound 28-6 from Compound 28-5 was carried out as follows.

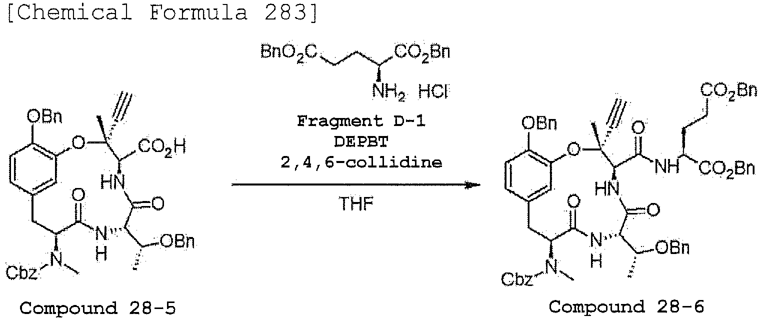

Under nitrogen atmosphere, to a solution of Compound 28-5 (1.0 eq., 47.4 mg, 64.6 µmol) and dibenzyl L-glutamate hydrochloride (Fragment D-1, 1.5 eq., 35.5 mg, 97.6 µmol) in THF (1 mL) were added DEPBT (1.5 eq., 29.2 mg, 97.6 µmol) and 2,4,6-collidine (2.9 eq., 23.0 mg, 190 µmol) under ice-cooling, and the mixture was stirred at the same temperature for 7 hr, and then at room temperature for 16 hr. To the reaction solution was added ethyl acetate (10 mL), and the mixture was washed once with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (90.3 mg) as a yellow oil. The crude product was purified by flash silica gel column (NH₂ silica gel 6 g, hexane/ethyl acetate = 75/25 - 50/50) to give Compound 28-6 (18.2 mg, yield 27%) as a white amorphous.

Synthesis of Compound 28 from Compound 28-6 was carried out as follows.

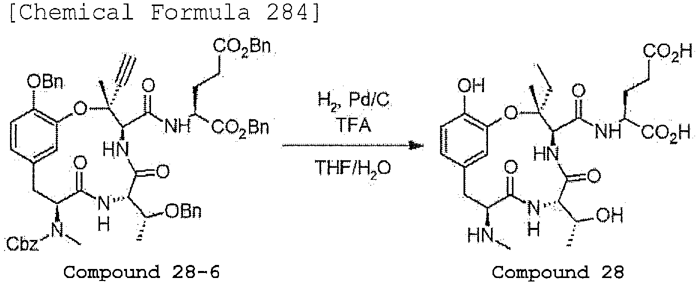

Under nitrogen atmosphere, to a solution of Compound 28-6 (1.0 eq., 18 mg, 0.017 mmol) in a mixture of THF (1800 µL)/water (90 µL) were added 10% palladium-carbon (N.E. CHEMCAT NX type, 50% hydrous product, 1.8 mg) and TFA (2.0 eq., 2.6 µL, 0.035 mmol) at room temperature, the system was replaced with hydrogen, and the mixture was stirred at the same temperature for 31.5 hr. During this period, 1.8 mg of 10% palladium-carbon was added after 23 hr and 29 hr. The catalyst was removed by filtration through Celite, and the filtrate was concentrated under reduced pressure to give a crude product. The crude product was purified by flash silica gel column (reverse-phase silica gel 30 g, 0.05% aqueous TFA solution/acetonitrile = 95/5 - 90/10), and freeze-dried to give a TFA salt of Compound 28 (6.6 mg, yield 57%, purity 98.3%) as a white solid.

¹H-NMR(400MHz,D₂O)57.04(s,1H),6.97(dd,J=8.4,2.0Hz,1H),6.93(d,J=8.4 Hz,1H),4.42(dd,J=9.2,5.2Hz,1H),4.38(d,J=6.8Hz,1H),4.12-4.02(m,2H),3.24(dd,J=13.6,4.4Hz,1H),2.99(dd,J=13.6,9.2Hz,1H),2. 74(s,3H),2.52-2.36(m,2H),2.26-2.13(m,1H),2.05-1.90(m,2H),1.79-1.66(m,1H),1.58(s,3H),1.11(d,J=6.4Hz,3H),0.97(t,J=7.2Hz,3H).

### (Example 29E: Synthesis of Compound 29)

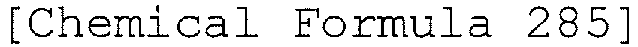

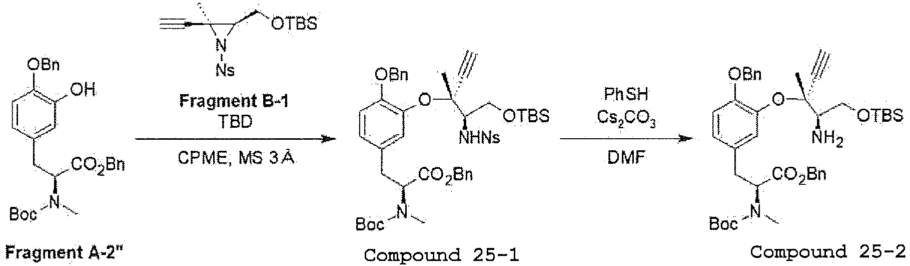

The two steps from Fragment A-2" → Compound 25-2 were carried out as described in Example 29B.

Synthesis of Compound 29-1 from Compound 25-2 was carried out as follows.

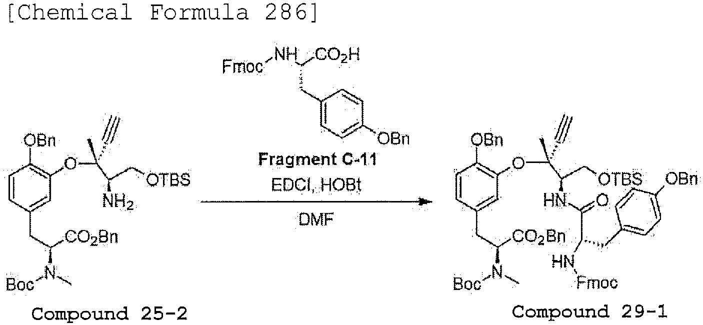

To a solution of Compound 25-2 (1.0 eq., 847 mg, 1.18 mmol) in DMF (12 mL) were added HOBt·H₂O (1.2 eq., 218 mg, 1.42 mmol), EDCI (1.2 eq., 272 mg, 1.42 mmol) and Fmoc-L-Tyr(Bzl)-OH (Fragment C-11,1.2 eq., 701 mg, 1.42 mmol) at room temperature, and the mixture was stirred at room temperature for 1 hr. To the reaction solution was added a mixture of hexane (15 mL)/ethyl acetate (45 mL), and the mixture was washed twice with water (40 mL), twice with saturated aqueous sodium hydrogencarbonate solution (40 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give crude Compound 29-1 (1.55 g, crude yield 110%) as a pale-brown amorphous.

Synthesis of Compound 29-2 from Compound 29-1 was carried out as follows.

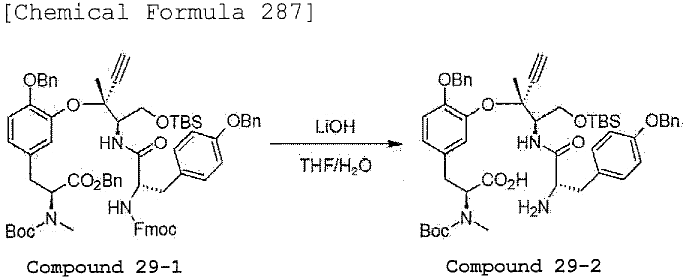

To a solution of Compound 29-1 (1.0 eq., 1.55 g, calculated as 1.18 mmol) in a mixture of THF (8 mL)/water (4 mL) was added lithium hydroxide (6.0 eq., 170 mg, 7.09 mmol) at room temperature, and the mixture was stirred at room temperature for 3.5 hr. The reaction solution was adjusted to pH 7 by addition of 1N aqueous hydrochloric acid solution (4.2 mL). To the reaction solution was added ethyl acetate (20 mL), and the mixture was washed once with water (10 mL) and once with saturated aqueous sodium chloride solution (10 mL) by liquid separation, and the white solid produced during the washing by liquid separation was removed by filtration. The filtrate was dried over magnesium sulfate, the magnesium sulfate was removed by filtration, and the filtrate was concentrated under reduced pressure to give a crude product (1.46 g) as a brown viscous product. The crude product was purified by flash silica gel column (normal phase silica gel 20 g, ethyl acetate/methanol = 100/0 - 40/60) to give Compound 29-2 (996 mg, two-step yield 96%) as a pale-brown amorphous.

Synthesis of Compound 29-3 from Compound 29-2 was carried out as follows.

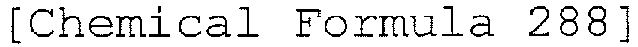

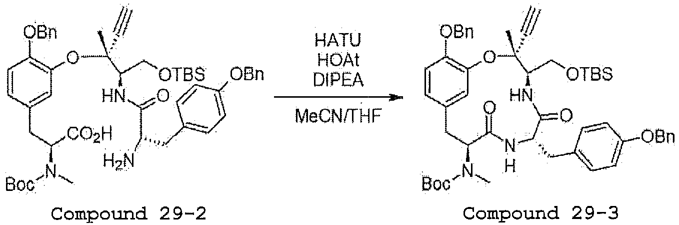

A solution of Compound 29-2 (1.0 eq., 937 mg, 1.06 mmol) in a mixture of acetonitrile (27 mL)/THF (27 mL) was added dropwise over 16 hr to a solution of HATU (2.0 eq., 809 mg, 2.13 mmol), HOAt (2.0 eq., 290 mg, 2.13 mmol) and DIPEA (2.0 eq., 360 µL, 2.12 mmol) in acetonitrile (532 mL) at about 56 µL/min at room temperature, and the mixture was stirred at room temperature for 2 hr. The reaction solution was concentrated under reduced pressure, to the concentrate was added ethyl acetate (50 mL), and the mixture was washed twice with a mixture of water (25 mL)/saturated aqueous ammonium chloride solution (25 mL), once with saturated aqueous sodium hydrogencarbonate solution (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (1.66 g) as a red-brown viscous product. The crude product was purified by flash silica gel column (normal phase silica gel 25 g, hexane/ethyl acetate = 80/20 - 50/50) to give Compound 29-3 (243 mg, yield 27%) as a white amorphous.

Synthesis of Compound 29-4 from Compound 29-3 was carried out as follows.

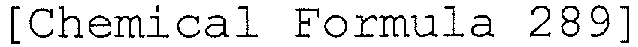

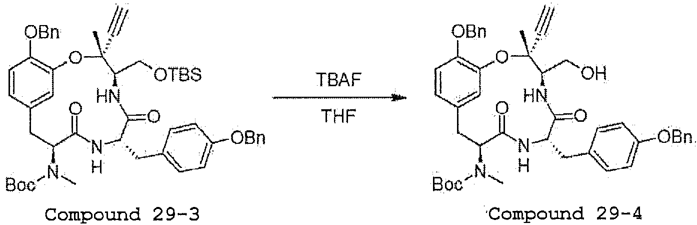

To a solution of Compound 29-3 (1.0 eq., 238 mg, 0.276 mmol) in THF (2.8 mL) was added TBAF (1M in THF, 2.0 eq., 552 µL, 0.552 mmol) at room temperature, and the mixture was stirred at the same temperature for 2 hr. To the reaction solution was added ethyl acetate (30 mL), and the mixture was washed twice with saturated aqueous ammonium chloride solution (20 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (226 mg) as a white amorphous. The crude product was purified by flash silica gel column (normal phase silica gel 5 g, hexane/ethyl acetate = 50/50 - 0/100) to give Compound 29-4 (201 mg, yield 97%) as a white amorphous.

Synthesis of Compound 29-5 from Compound 29-4 was carried out as follows.

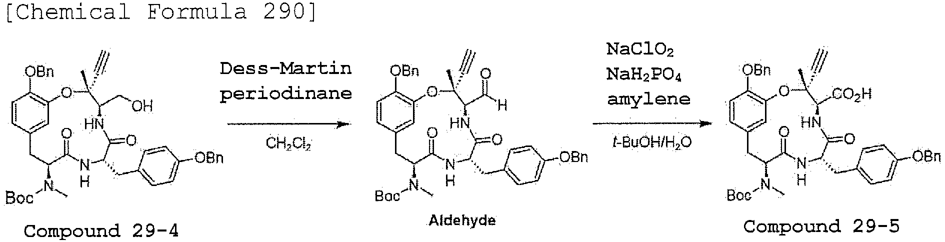

Under nitrogen atmosphere, to a solution of Compound 29-4 (1.0 eq., 196 mg, 0.262 mmol) in dichloromethane (2.6 mL) was added Dess-Martin periodinane (1.5 eq., 169 mg, 0.398 mmol) at room temperature, and the mixture was stirred at the same temperature for 1 hr. To the reaction solution was added a mixture of 10% aqueous sodium sulfite solution (5 mL)/saturated aqueous sodium hydrogencarbonate solution (5 mL), and the mixture was extracted once with ethyl acetate (20 mL) by liquid separation. The organic layer was washed once with a mixture of 10% aqueous sodium sulfite solution (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (199 mg) as a white amorphous.

To a solution of Aldehyde (1.0 eq., 197 mg, calculated as 0.262 mmol) in a mixture of amylene (1 mL)/t-butyl alcohol (4 mL) was added a solution of sodium dihydrogen phosphate dihydrate (3.4 eq., 141 mg, 0.904 mmol) and 80% sodium chlorite (4.6 eq., 135 mg, 1.20 mmol) in water (1 mL) at room temperature, and the mixture was stirred at the same temperature for 2.5 hr. To the reaction solution was added ethyl acetate (20 mL), and the mixture was washed once with a mixture of water (5 mL)/saturated aqueous ammonium chloride solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Compound 29-5 (197 mg, yield 99%) as a white solid.

Synthesis of Compound 29-6 from Compound 29-5 was carried out as follows.

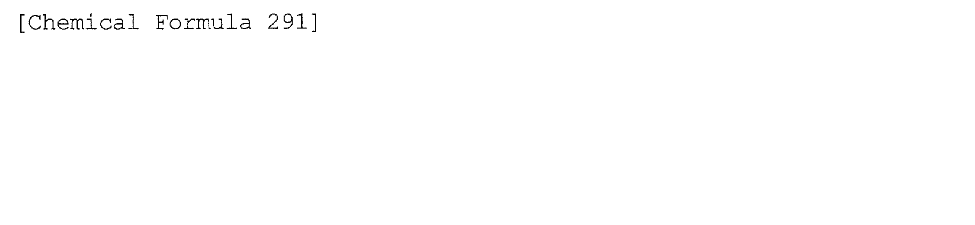

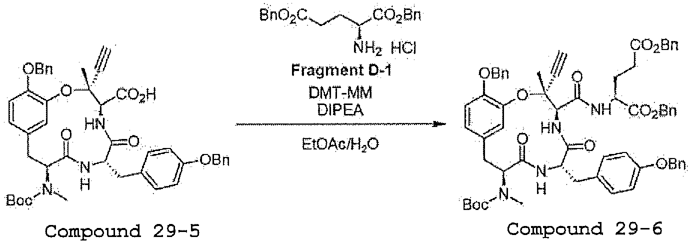

To a solution of Compound 29-5 (1.0 eq., 195 mg, 0.256 mmol) in a mixture of ethyl acetate (3 mL)/water (900 µL) was added dibenzyl L-glutamate hydrochloride (Fragment D-1, 1.5 eq., 141 mg, 0.387 mmol) at room temperature. DIPEA (2.4 eq., 103 µL, 0.606 mmol) was added under ice-cooling, the mixture was stirred for 5 min, DMT-MM (1.7 eq., 122 mg, 0.439 mmol) was added, and the mixture was stirred at the same temperature for 3 hr. To the reaction solution was added ethyl acetate (20 mL), and the mixture was washed twice with water (10 mL), twice with saturated aqueous ammonium chloride solution (10 mL), twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (316 mg) as a yellow amorphous. The crude product was purified by flash silica gel column (NH₂ silica gel 6 g, hexane/ethyl acetate = 88/12 - 50/50) to give Compound 29-6 (206 mg, yield 75%) as a white amorphous.

Synthesis of Compound 29-7 from Compound 29-6 was carried out as follows.

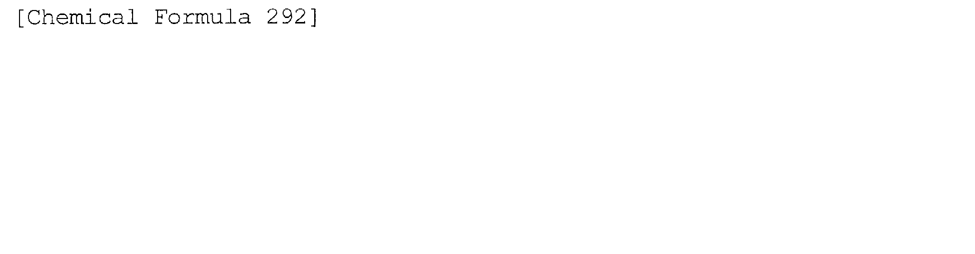

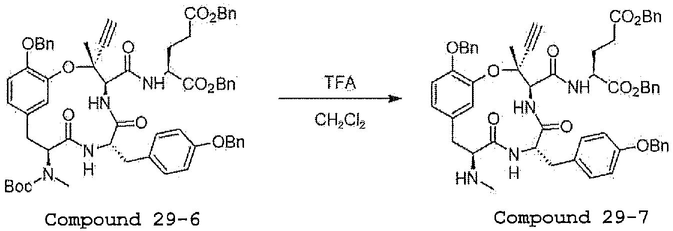

Under nitrogen atmosphere, to a solution of Compound 29-6 (1.0 eq., 204 mg, 0.190 mmol) in dichloromethane (2 mL) was added TFA (10 eq., 145 µL, 1.89 mmol) at room temperature, and the mixture was stirred at the same temperature for 18 hr. Then, additional TFA (10 eq., 145 µL, 1.89 mmol) was added, and the mixture was stirred at the same temperature for 6 hr. The mixture was adjusted to pH 8 by addition of saturated aqueous sodium hydrogencarbonate solution (10 mL) under ice-cooling. Ethyl acetate (20 mL) was added, and the mixture was washed twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give crude Compound 29-7 (182 mg, crude yield 98%) as a white amorphous.

Synthesis of Compound 29 from Compound 29-7 was carried out as follows.

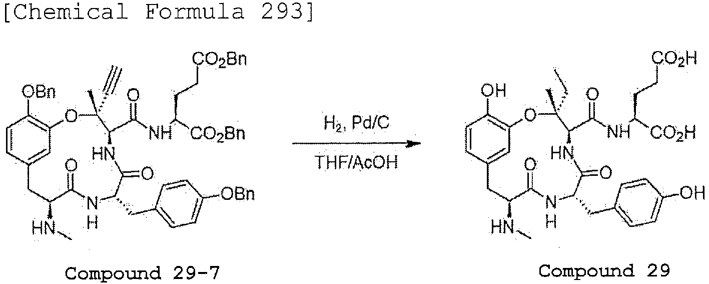

Under nitrogen atmosphere, to a solution of Compound 29-7 (1.0 eq., 182 mg, 0.187 mmol) in a mixture of THF (1 mL)/acetic acid (1 mL) was added 10% palladium-carbon (N.E. CHEMCAT NX type, 50% hydrous product, 18.1 mg) at room temperature, the system was replaced with hydrogen, and the mixture was stirred at the same temperature for 23 hr. The catalyst was removed by filtration through Celite, and the filtrate was concentrated under reduced pressure. To the concentrated residue was added acetone, and the precipitated white solid was collected by filtration. This solid was dissolved in water, the solution was filtered through 0.45 µm filter, and the filtrate was concentrated under reduced pressure to give a crude product (52.7 mg) as a gray solid. The crude product was purified twice by flash silica gel column (reverse-phase silica gel 10 g, 1st run: 0.05% aqueous acetic acid solution/acetonitrile = 95/5 - 70/30, 2nd run: 0.05% aqueous acetic acid solution/acetonitrile = 95/5 - 88/12), and freeze-dried to give an acetate of Compound 29 (10.3 mg, yield 9%, purity 98.3%) as a white solid.

¹H-NMR(400MHz,D₂O)37.77(d,J=9.6Hz,0.4H),6.86(d,J=8.1Hz,2H),6.78-6.68(m,3H),6.62(d,J=8.1Hz,2H),3.92(brt,J=7.0Hz,1H),3.78(dd,J=8. 6,4.6Hz,1H),3.04(dd,J=13.6,4.6Hz,1H),2.86(dd,J=13.8,8.1Hz,1H),2 .75(dd,J=13.6,8.6Hz,1H),2.58(dd,J=13.8,8.1Hz,1H),2.27-2.17(m,5H),1.94-1.86(m,1H),1.81-1.72(m,2H),1.59-1.51(m,1H),1.29(s,3H),0.877(t,J=7.2Hz,3H).

### (Example 29F: Synthesis of Compound 31)

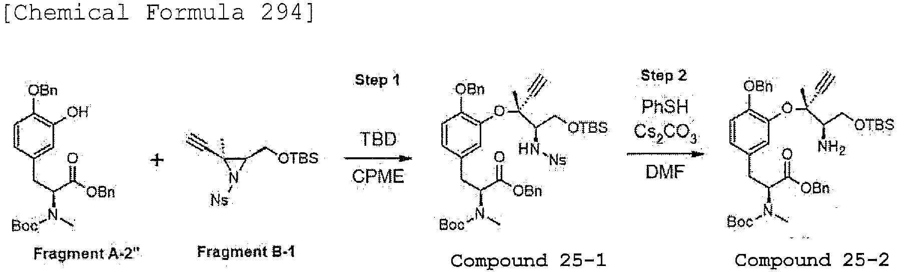

The experimental procedures up to Compound 25-2 were carried out as described in Example 29B.

Synthesis of Compound 31-1 from Compound 25-2 was carried out as follows.

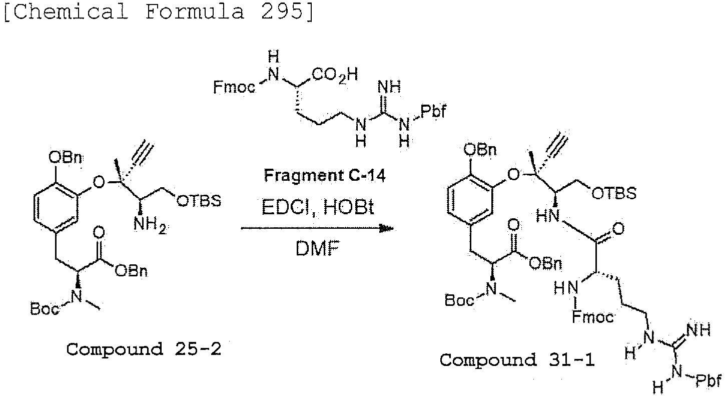

Under nitrogen atmosphere, to a solution of Compound 25-2 (1.0 eq., 1.00 g, 1.39 mmol) in DMF (15 mL) were added EDCI (1.2 eq., 321 mg, 1.68 mmol), HOBt·H₂O (1.2 eq., 256 mg, 1.67 mmol) and N-Fmoc-L-Arg(Pbf)-OH (Fragment C-14, 1.2 eq., 1.08 g, 1.67 mmol) under ice-cooling, and the mixture was stirred at room temperature for 4 hr. Water (40 mL) was added dropwise under ice-cooling, and the mixture was extracted three times with a mixture of hexane (5 mL)/ethyl acetate (15 mL) by liquid separation. The organic layers were combined, washed twice with saturated aqueous sodium hydrogencarbonate solution (30 mL) by liquid separation, and washed once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (2.18 g) as a pale-brown amorphous. The crude product was purified by flash silica gel column (normal phase silica gel 30 g, hexane/ethyl acetate = 50/50 - 0/100) to give Compound 31-1 (1.78 g, yield 95%) as a white amorphous.

Synthesis of Compound 31-2 from Compound 31-1 was carried out as follows.

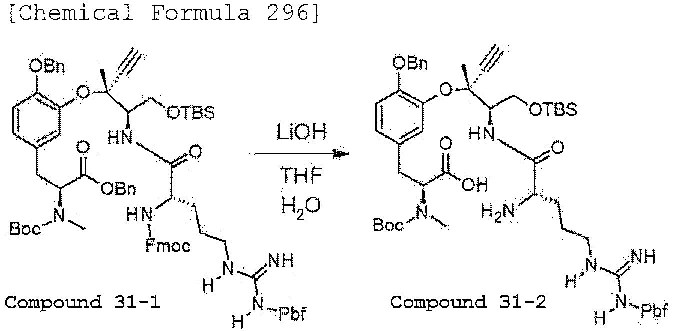

To a solution of Compound 31-1 (1.0 eq., 1.57 g, 1.17 mmol) in a mixture of THF (8 mL)/water (4 mL) was added lithium hydroxide (4.1 eq., 115 mg, 4.79 mmol) at room temperature, and the mixture was stirred at room temperature for 3.5 hr. The mixture was adjusted to pH 7 by addition of 1N aqueous hydrochloric acid solution (2.5 mL) under ice-cooling, and extracted three times with ethyl acetate (20 mL) by liquid separation. The organic layers were combined, and dried over magnesium sulfate, the magnesium sulfate was removed by filtration, and the filtrate was concentrated under reduced pressure to give a white solid. The obtained solid was washed with a mixture of hexane/ethyl acetate = 2/1, and then with water to give a crude product (1.36 g). The crude product was purified by flash silica gel column (normal phase silica gel 40 g, ethyl acetate/methanol=10/1 - 3/1) to give Compound 31-2 (775 mg, yield 64%) as a white solid.

Synthesis of Compound 31-3 from Compound 31-2 was carried out as follows.

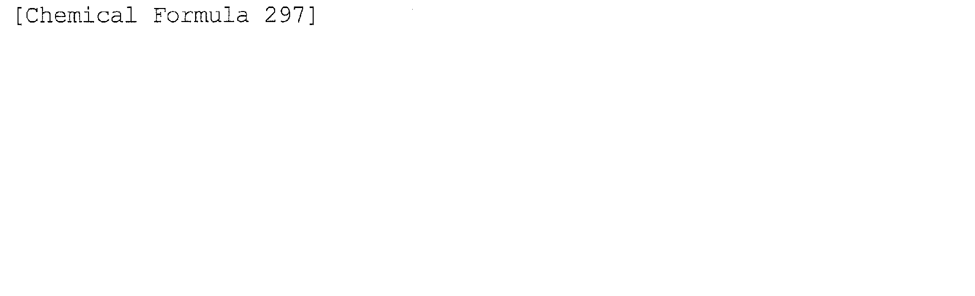

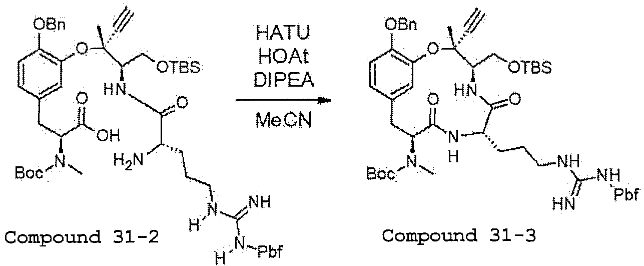

To a solution of Compound 31-2 (1.0 eq., 553 mg, 534 µmol) in DMF (3 mL) was added acetonitrile (27 mL), this solution was added dropwise over 17 hr to a solution of HATU (3.0 eq., 611 mg, 1.61 mmol), HOAt (3.1 eq., 222 mg, 1.63 mmol) and DIPEA (3.0.eq,275 µL, 1.62 mmol) in acetonitrile (270 mL) at about 30 µL/min at room temperature, and the mixture was stirred at room temperature for 3 hr. The reaction solution was concentrated under reduced pressure to evaporate the acetonitrile, ethyl acetate (30 mL) was added, and the mixture was washed twice with saturated aqueous sodium hydrogencarbonate solution (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (566 mg) as a brown amorphous. The crude product was purified by flash silica gel column (normal phase silica gel 30 g, hexane/ethyl acetate = 50/50 - 0/100) to give Compound 31-3 (237 mg, yield 44%) as a white solid.

Synthesis of Compound 31-4 from Compound 31-3 was carried out as follows.

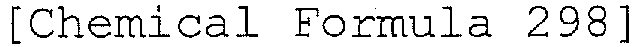

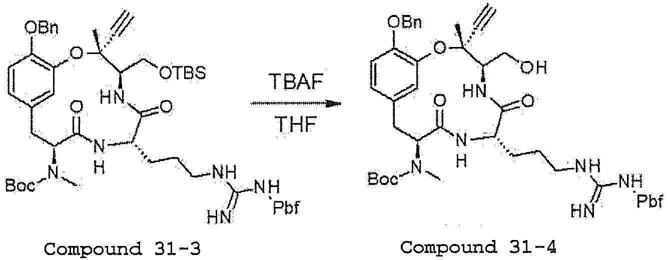

Under nitrogen atmosphere, to a solution of Compound 31-3 (1.0 eq., 316 mg, 311 µmol) in THF (5 mL) was added TBAF (1M in THF, 1.5 eq., 470 µL, 470 µmol) under ice-cooling, and the mixture was stirred at room temperature for 1 hr. Additional TBAF (1M in THF, 1.5 eq., 470 µL, 470 µmol) was added, and the mixture was stirred for 2 hr. Additional TBAF (1M in THF, 0.5 eq., 150 µL, 150 µmol) was added again, and the mixture was stirred for 1 hr. To the reaction solution was added saturated aqueous ammonium chloride solution (5 mL). The organic layer of the reaction solution was recovered, and the aqueous layer was extracted twice with ethyl acetate (5 mL) by liquid separation. The organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (433 mg) as a white amorphous. The crude product was purified by flash silica gel column (normal phase silica gel 5 g, chloroform/methanol = 100/0 - 85/15) to give Compound 31-4 (297 mg, containing impurities, apparent yield 106%) as a white amorphous.

Synthesis of Compound 31-5 from Compound 31-4 was carried out as follows.

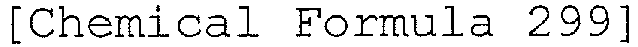

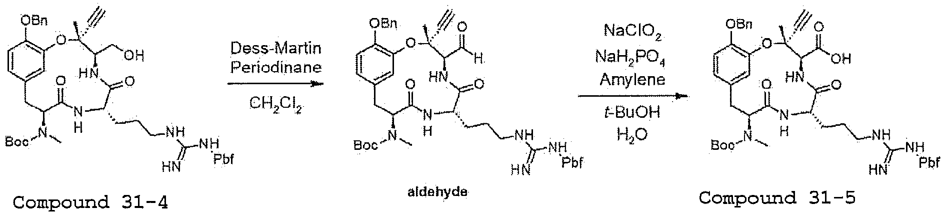

Under nitrogen atmosphere, to a solution of Compound 31-4 (1.0 eq., 297 mg, 311 µmol) in dichloromethane (3 mL) was added Dess-Martin periodinane (1.5 eq., 201 mg, 474 µmol) under ice-cooling, and the mixture was stirred at room temperature for 1 hr. To the reaction solution were added 20% aqueous sodium sulfite solution (5 mL) and saturated aqueous sodium hydrogencarbonate solution (5 mL), and the mixture was extracted three times with dichloromethane (5 mL) by liquid separation. The organic layers were combined, and washed once with saturated aqueous sodium chloride solution (20 mL) by liquid separation. The organic layer was dried over magnesium sulfate, the magnesium sulfate was removed by filtration, and the filtrate was concentrated under reduced pressure to give Aldehyde (297 mg, crude yield 106%).

To a solution of Aldehyde (1.0 eq., 297 mg, calculated as 311 µmol) in a mixture of t-butyl alcohol (3 mL)/amylene (1 mL) was added a solution of sodium dihydrogen phosphate dihydrate (3.6 eq., 172 mg, 1.11 mmol) and 80% sodium chlorite (4.6 eq., 161 mg, 1.42 mmol) in water (1 mL) at room temperature, and the mixture was stirred at room temperature for 30 min. To the reaction solution was added saturated aqueous ammonium chloride solution (5 mL), and the mixture was extracted three times with ethyl acetate (5 mL) by liquid separation. The organic layers were combined, and dried over magnesium sulfate, the magnesium sulfate was removed by filtration, and the filtrate was concentrated under reduced pressure to give a crude product (282 mg) as a pale-yellow amorphous. The crude product was purified by flash silica gel column (normal phase silica gel 5 g, chloroform/methanol = 100/0 - 75/25) to give Compound 31-5 (236 mg, two-step yield 83%) as a white amorphous.

Synthesis of Compound 31-6 from Compound 31-5 was carried out as follows.

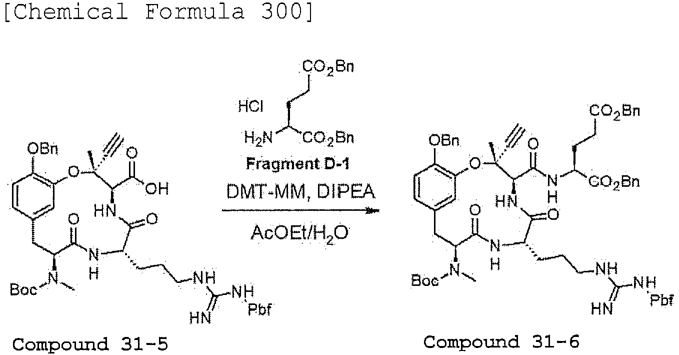

Under nitrogen atmosphere, to a solution of Compound 31-5 (1.0 eq., 232 mg, 253 µmol) in a mixture of ethyl acetate (1.4 mL)/water (0.6 mL) were added DIPEA (2.6 eq., 110 µL, 647 µmol), dibenzyl L-glutamate hydrochloride (Fragment D-1, 1.5 eq., 139 mg, 383 µmol) and DMT-MM (1.8 eq., 127 mg, 460 µmol) under ice-cooling, and the mixture was stirred under ice-cooling for 2 hr, and then at room temperature for 1 hr. The reaction solution was washed once with saturated aqueous sodium hydrogencarbonate solution (10 mL), once with water (10 mL), once with 1N aqueous hydrochloric acid solution (10 mL), once with water (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (299 mg) as a yellow-brown amorphous. The crude product was purified by flash silica gel column (normal phase silica gel 5 g, hexane/ethyl acetate = 50/50 - 0/100) to give Compound 31-6 (222 mg, yield 72%) as a yellow amorphous.

Synthesis of Compound 31-7 from Compound 31-6 was carried out as follows.

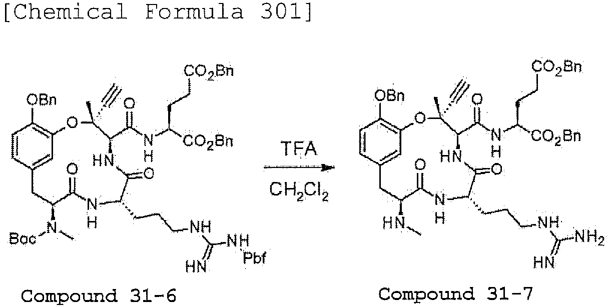

Under nitrogen atmosphere, to a solution of Compound 31-6 (1.0 eq., 193 mg, 157 µmol) in dichloromethane (2 mL) was added TFA (16.7 eq., 200 µL, 2.61 mmol) at room temperature, and the mixture was stirred at room temperature for 1 hr. Additional TFA (25.0 eq., 300 µL, 3.92 mmol) was added, and the mixture was stirred for additional 17 hr. To the reaction solution was added saturated aqueous sodium hydrogencarbonate solution (10 mL), the mixture was extracted three times with ethyl acetate (5 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (156 mg) as an orange-yellow amorphous. To the crude product was added ethyl acetate (2 mL), and the mixture was heated to 40°C to dissolve it. Hexane (2 mL) was added thereto, and the resulting pale-yellow solid was collected by filtration, and washed with a mixture of hexane/ethyl acetate = 2/1 to give Compound 31-7 (126 mg, yield 92%) as a pale-yellow solid.

Synthesis of Compound 31 from Compound 31-7 was carried out as follows.

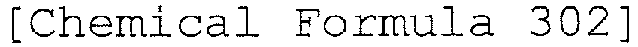

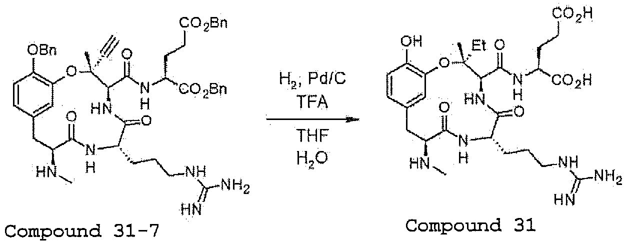

Under nitrogen atmosphere, to a solution of Compound 31-7 (1.0 eq., 120 mg, 137 µmol) in a mixture of THF (2.0 mL)/water (0.2 mL) was added TFA (9.5 eq., 100 µL, 1.31 mmol) at room temperature, and the mixture was stirred at room temperature for 2 hr. Then, 10% palladium-carbon (N.E. CHEMCAT NX type, 50% hydrous product, 12.5 mg) was added at room temperature, the system was replaced with hydrogen, and the mixture was stirred at room temperature for 20 hr. The catalyst was removed by filtration through Celite, and the filtrate was further filtered through 0.45 µm filter, and concentrated under reduced pressure to give a crude product (142 mg) as an orange amorphous. The crude product was purified three times by flash silica gel column (reverse-phase silica gel 6 g, 1st run: 0.05% aqueous acetic acid solution/acetonitrile =97/3 to 20/80, 2nd run: 0.05% aqueous acetic acid solution/acetonitrile =97/3, 3rd run: 0.05% aqueous acetic acid solution/acetonitrile =97/3), and freeze-dried to give an acetate of Compound 31 (5.8 mg, yield 7%, purity 94%) as a white solid.

¹H-NMR(400MHz,D₂O)δ6.80-6.71(m,3H),4.75(s,1H),4.36(t,J=7.6Hz,1H),4.03(dd,J=8.6,4.3Hz,1H ),3.89(dd,J=8.4,4.8Hz,1H),3.10(dd,J=13.54.3Hz,1H),3.03-2.92(m,2H),2.80(dd,J=13.5,8.6Hz,1H),2.58(s,3H),2.07(t,J=7.8Hz,2 H),1.97-1.81(m,2H),1.78-1.68(m,1H),1.66-1.56(m,2H),1.49-1.31 (m,5H),1.29-1.18(m,1H),0.91(t,J=7.2Hz, 3H).

### (Example 29H: Synthesis of Compound 32)

In this example, Synthesis of Compound 32 was carried out.

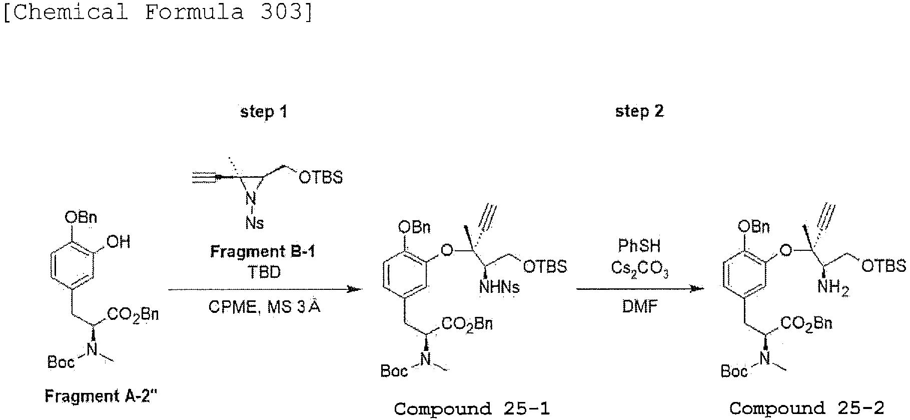

The two steps from Fragment A-2'' → Compound 25-2 were carried out as described in Example 29B.

Synthesis of Compound 32-1 from Compound 25-2 was carried out as follows.

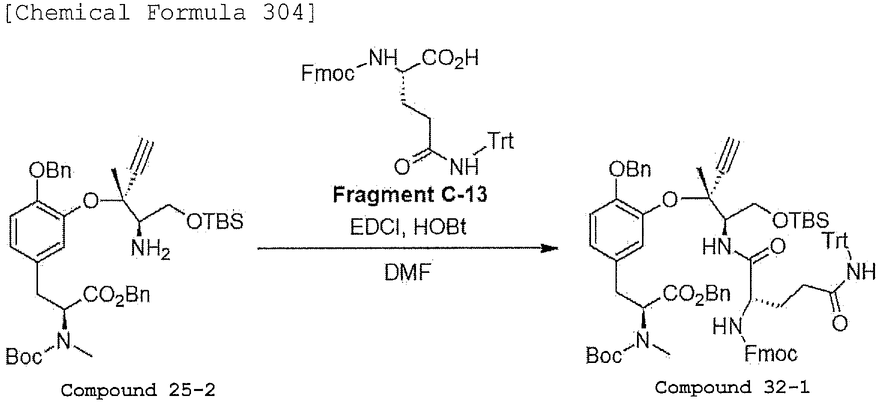

To a solution of Compound 25-2 (1.0 eq., 1.14 g, calculated as 1.59 mmol) in DMF (1.6 mL) were added HOBt•H₂O (1.2 eq., 294 mg, 1.92 mmol), EDCI (1.2 eq., 367 mg, 1.91 mmol) and Fmoc-L-Gln(Trt)-OH (Fragment C-13,1.2 eq., 1.17 g, 1.92 mmol) at room temperature, and the mixture was stirred at room temperature for 3 hr. To the reaction solution was added a mixture of hexane (15 mL)/ethyl acetate (45 mL), and the mixture was washed twice with water (40 mL), twice with saturated aqueous sodium hydrogencarbonate solution (40 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (2.42 g) as a white amorphous. The crude product was purified by flash silica gel column (normal phase silica gel 25 g, hexane/ethyl acetate = 75/25 - 60/40) to give Compound 32-1 (1.77 g, yield 85%) as a white amorphous.

Synthesis of Compound 32-2 from Compound 32-1 was carried out as follows.

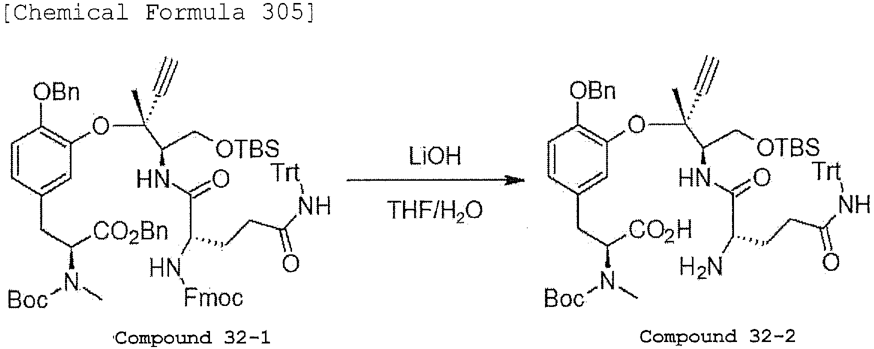

To a solution of Compound 32-1 (1.0 eq., 1.34 g, 1.34 mmol) in a mixture of THF (8 mL)/water (4 mL) was added lithium hydroxide (6.0 eq., 194 mg, 8.11 mmol) at room temperature, and the mixture was stirred at room temperature for 3.5 hr. The reaction solution was adjusted to pH 7 by addition of 1N aqueous hydrochloric acid solution (5.2 mL). To the reaction solution was added ethyl acetate (20 mL), and the mixture was washed twice with water (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (1.43 g) as a pale-yellow amorphous. The crude product was purified by flash silica gel column (normal phase silica gel 20 g, ethyl acetate/methanol = 100/0 - 40/60). Then, to the concentrate was added ethyl acetate, the precipitated white solid was removed by filtration, and the filtrate was concentrated under reduced pressure to give Compound 32-2 (979 mg, yield 73%) as a white amorphous.

Synthesis of Compound 32-3 from Compound 32-2 was carried out as follows.

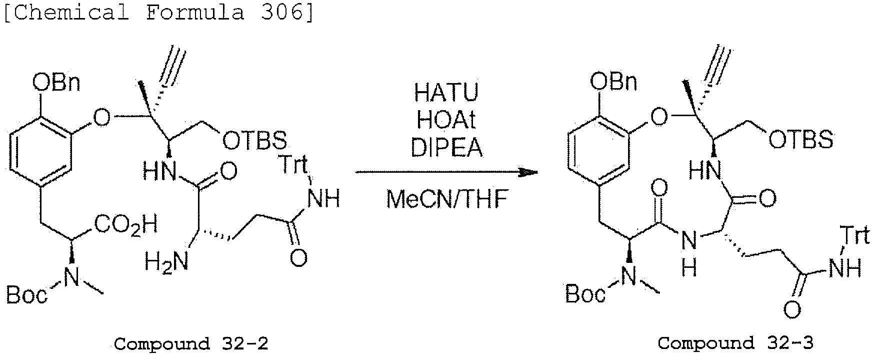

A solution of Compound 32-2 (1.0 eq., 976 mg, 0.978 mmol) in a mixture of acetonitrile (25 mL)/THF (25 mL) was added dropwise over 18 hr to a solution of HATU (2.0 eq., 745 mg, 1.96 mmol), HOAt (2.0 eq., 268 mg, 1.97 mmol) and DIPEA (2.0 eq., 332 µL, 1.95 mmol) in acetonitrile (490 mL) at about 46 µL/min at room temperature, and the mixture was stirred at room temperature for 2 hr. To the reaction solution was added saturated aqueous sodium hydrogencarbonate solution (30 mL), and the mixture was concentrated under reduced pressure to evaporate the organic solvent. To the concentrate was added ethyl acetate (50 mL), and the mixture was washed once with water (50 mL), once with saturated aqueous sodium hydrogencarbonate solution (30 mL), twice with saturated aqueous ammonium chloride solution (25 mL) and once with saturated aqueous sodium chloride solution (25 mL) by liquid separation. The organic layer was dried over magnesium sulfate, the magnesium sulfate was removed by filtration, and the filtrate was concentrated under reduced pressure to give a crude product (964 mg) as an orange amorphous. The crude product was purified by flash silica gel column (normal phase silica gel 10 g, hexane/ethyl acetate = 25/75 - 0/100) to give Compound 32-3 (172 mg, yield 18%) as a pale-yellow oil.

Synthesis of Compound 32-4 from Compound 32-3 was carried out as follows.

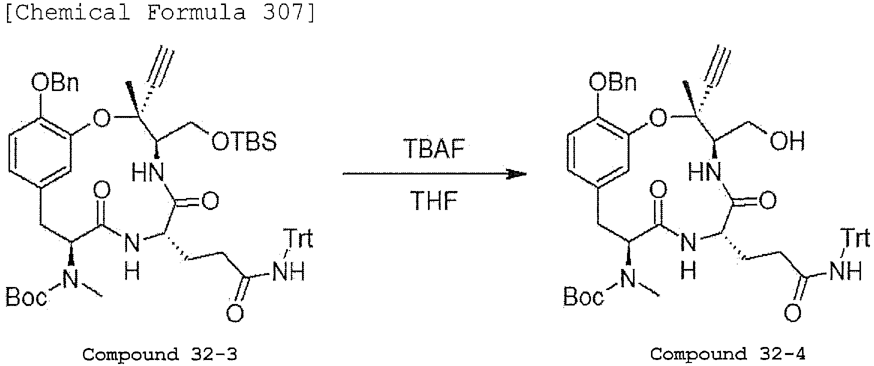

To a solution of Compound 32-3 (1.0 eq., 172 mg, 0.176 mmol) in THF (2 mL) was added TBAF (1M in THF, 2.0 eq., 351 µL, 0.351 mmol) at room temperature, and the mixture was stirred at the same temperature for 3 hr. The reaction solution was quenched by addition of saturated aqueous ammonium chloride solution (10 mL). Ethyl acetate (30 mL) was added, and the mixture was washed once with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (147 mg) as a pale-yellow amorphous. The crude product was purified by flash silica gel column (normal phase silica gel 10 g, hexane/ethyl acetate = 60/40 - 0/100) to give Compound 32-4 (92.3 mg, yield 61%) as a pale-yellow oil.

Synthesis of Compound 32-5 from Compound 32-4 was carried out as follows.

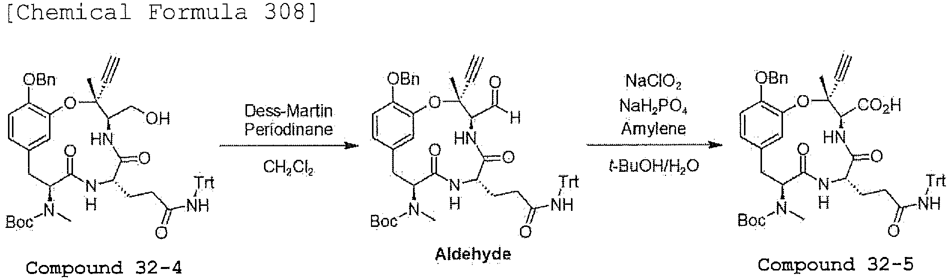

Under nitrogen atmosphere, to a solution of Compound 32-4 (1.0 eq., 92.3 mg, 0.107 mmol) in dichloromethane (1 mL) was added Dess-Martin periodinane (1.5 eq., 68.5 mg, 0.162 mmol) at room temperature, and the mixture was stirred at the same temperature for 3 hr. To the reaction solution was added a mixture of 10% aqueous sodium sulfite solution (5 mL)/saturated aqueous sodium hydrogencarbonate solution (5 mL), and the mixture was extracted once with ethyl acetate (20 mL) by liquid separation. The organic layer was washed once with a mixture of 10% aqueous sodium sulfite solution (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (80.7 mg, yield 88%) as a pale-yellow solid.

To a solution of Aldehyde (1.0 eq., 79.6 mg, 92.2 µmol) in a mixture of amylene (1 mL)/t-butyl alcohol (4 mL) was added a solution of sodium dihydrogen phosphate dihydrate (3.4 eq., 49.2 mg, 0.315 mmol) and 80% sodium chlorite (4.5 eq., 46.9 mg, 0.413 mmol) in water (1 mL) at room temperature, and the mixture was stirred at the same temperature for 3 hr. To the reaction solution was added ethyl acetate (20 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (88.2 mg) as a white amorphous. The crude product was purified by flash silica gel column (normal phase silica gel 5 g, hexane/ethyl acetate = 33/67 - 0/100) to give Compound 32-5 (71.0 mg, yield 88%) as a white amorphous.

Synthesis of Compound 32-6 from Compound 32-5 was carried out as follows.

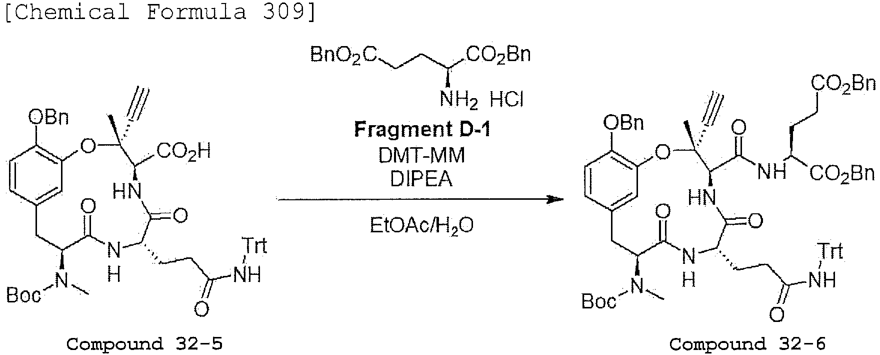

To a solution of Compound 32-5 (1.0 eq., 70.2 mg, 79.9 µmol) in a mixture of ethyl acetate (1 mL)/water (300 µL) was added dibenzyl L-glutamate hydrochloride (Fragment D-1, 1.5 eq., 44.4 mg, 0.122 mmol) at room temperature, and the mixture was ice-cooled. Then, DIPEA (2.4 eq., 32.6 µL, 0.192 mmol) was added, and the mixture was stirred for 10 min. DMT-MM (1.7 eq., 38.2 mg, 0.138 mmol) was added, and the mixture was stirred at the same temperature for 3 hr. To the reaction solution was added ethyl acetate (20 mL), and the mixture was washed once with water (10 mL), twice with saturated aqueous ammonium chloride solution (10 mL), twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (110 mg) as a yellow amorphous. The crude product was purified by flash silica gel column (normal phase silica gel 10 g, hexane/ethyl acetate = 88/12 - 31/69) to give Compound 32-6 (79.2 mg, yield 83%) as a yellow solid.

Synthesis of Compound 32-7 from Compound 32-6 was carried out as follows.

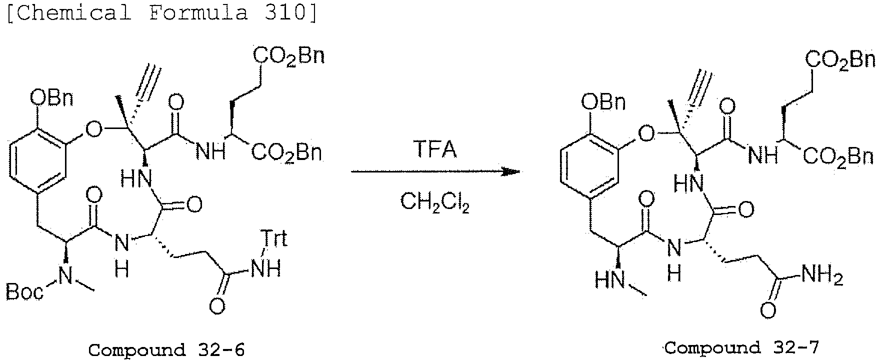

Under nitrogen atmosphere, to a solution of Compound 32-6 (1.0 eq., 79.2 mg, 66.6 µmol) in dichloromethane (1 mL) was added TFA (15 eq., 76.5 µL, 1.00 mmol) at room temperature, and the mixture was stirred at the same temperature for 7 hr. Then, additional TFA (15 eq., 76.5 µL, 1.00 mmol) was added, and the mixture was stirred at the same temperature for 17 hr. The reaction solution was quenched by addition of saturated aqueous sodium hydrogencarbonate solution (10 mL), and extracted once with ethyl acetate (20 mL) by liquid separation. The organic layer was washed once with water (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure. To the concentrated residue was added ethyl acetate, and the precipitated white solid was collected by filtration to give Compound 32-7 (34.6 mg, yield 36%).

Synthesis of Compound 32 from Compound 32-7 was carried out as follows.

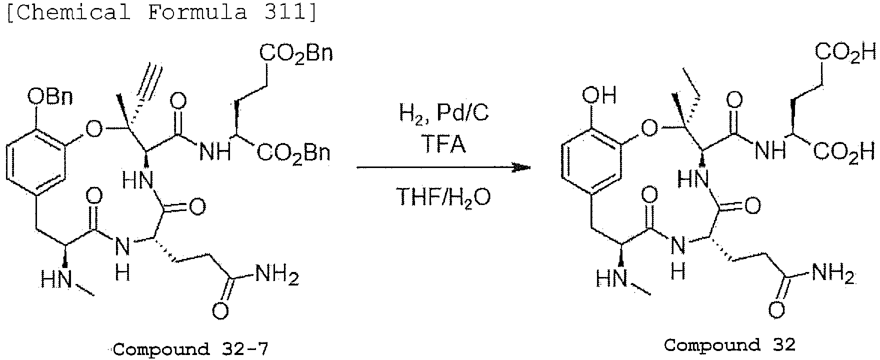

To a solution of Compound 32-7 (1.0 eq., 34.6 mg, 40.9 µmol) in a mixture of THF (1.38 mL)/water (69.2 µL) was added TFA (5.0 eq., 15.6 µL, 0.204 mmol) at room temperature, and the mixture was stirred at the same temperature for 1 hr. Then, the system was replaced with nitrogen, 10% palladium-carbon (N.E. CHEMCAT NX type, 50% hydrous product, 3.6 mg) was added, the system was replaced with hydrogen, and the mixture was stirred at the same temperature for 17 hr. The catalyst was removed by filtration through Celite. The filtrate was further filtered through 0.45 µm filter, and concentrated under reduced pressure to give a crude product (43.4 mg) as a pale-pink solid. The crude product was purified three times by flash silica gel column (1st run: reverse-phase silica gel 6 g, 0.05% aqueous acetic acid solution/acetonitrile = 95/5 - 90/10, 2nd run: reverse-phase silica gel 10 g, 0.05% aqueous acetic acid solution/acetonitrile = 99/1 - 95/5, 3rd run: reverse-phase silica gel 6 g, 0.05% aqueous acetic acid solution/acetonitrile =98/2) to give a mixture (7.3 mg) of an acetate of Compound 32 and impurities.

¹H-NMR(400MHz,D₂O)δ6.85-6.79(m,3H),4.36(t,J=7.5Hz,1H),4.14(dd,J=8.9,4.8Hz,1H),3.89(dd,J =9.0,4.6Hz,1H),3.13(dd,J=13.7,4.6Hz,1H),2.83(dd,J=13.7,9.0Hz,1H ),2.61(s,3H),2.24(t,J=7.6Hz,2H),1.43(s,3H),0.889(t,J=7.3Hz,3H).

### (Example 29I: Synthesis of Compound 35)

Synthesis of Compound 35-1 from Fragment A-10 + Fragment B-1 was carried out as follows.

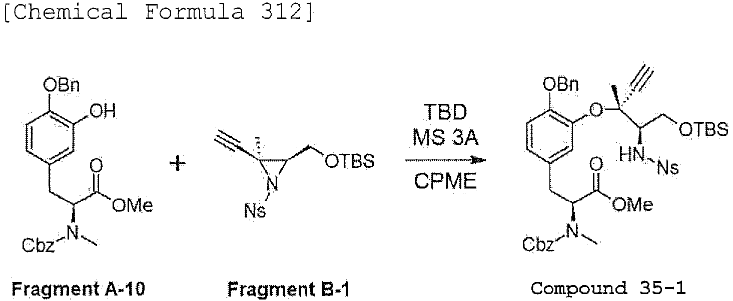

Under nitrogen atmosphere, to a solution of Fragment A-10 (1.0 eq., 7.61 g, 16.93 mmol) in CPME (85 mL) were added MS3A (3.8 g) at room temperature, and then TBD (1.1 eq., 2.49 g, 17.86 mmol) under ice-cooling. A solution of Fragment B-1 (1.2 eq.8.42 g, 20.51 mmol) in CPME (80 mL) was added dropwise at the same temperature, and the mixture was stirred at room temperature for 16 hr, and the CPME was evaporated by concentration under reduced pressure to give a crude product. The crude product was purified by flash silica gel column (normal phase silica gel 150 g, hexane/ethyl acetate = 3/1 - 1/1) to give Compound 35-1 (12.21 g, yield 84%) as a pale-yellow viscous product.

Synthesis of Compound 35-2 from Compound 35-1 was carried out as follows.

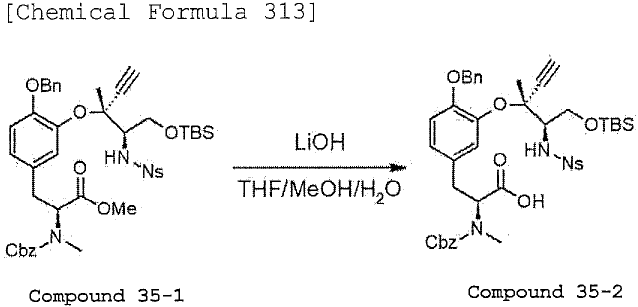

To a solution of Compound 35-1 (1.0 eq., 4.73 g, 5.50 mmol) in a mixture of THF (30 mL)/methanol (15 mL)/water (15 mL) was added lithium hydroxide (3.0 eq., 390 mg, 16.29 mmol) at room temperature, and the mixture was stirred at room temperature for 1.5 hr. The mixture was adjusted to pH about 5 by addition of 1N aqueous hydrochloric acid solution (15 mL), and extracted three times with ethyl acetate (30 mL) by liquid separation. The organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give crude Compound 35-2 (4.82 g, crude yield 104%) as a pale-yellow amorphous.

Synthesis of Compound 35-3 from Compound 35-2 was carried out as follows.

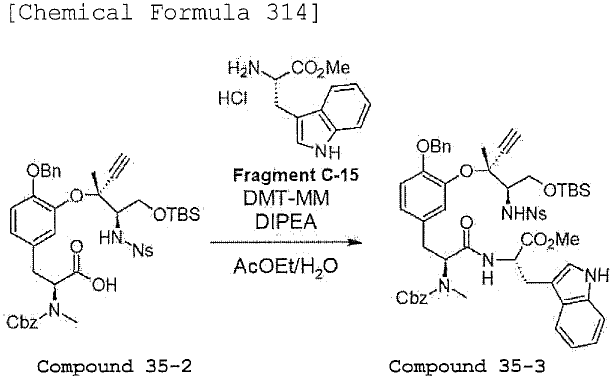

Under nitrogen atmosphere, to a solution of Compound 35-2 (1.0 eq., 1.21 g, 1.43 mmol) in a mixture of ethyl acetate (10 mL)/water (3 mL) were added L-tryptophan-OMe hydrochloride (1.5 eq., 551 mg, 2.16 mmol) at room temperature, and then DIPEA (1.7 eq., 410 µL, 2.41 mmol) and DMT-MM (1.7 eq., 678 mg, 2.45 mmol) under ice-cooling, and the mixture was stirred at room temperature for 2.5 hr. Ethyl acetate (20 mL) was added, and the mixture was washed once with 1N aqueous hydrochloric acid solution (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give crude Compound 35-3 (1.61 g, crude yield 107%) as a pale-yellow amorphous.

Synthesis of Compound 35-4 from Compound 35-3 was carried out as follows.

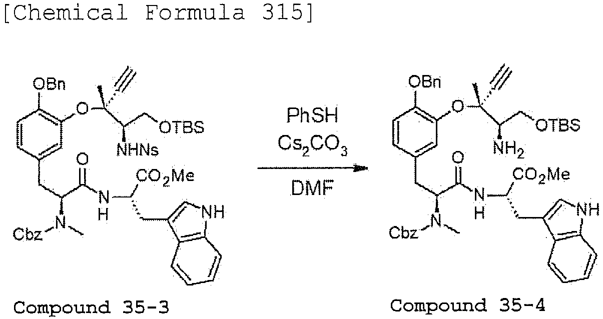

Under nitrogen atmosphere, to a solution of Compound 35-3 (1.0 eq., 1.61 g, calculated as 1.43 mmol) in DMF (15 mL) were added cesium carbonate (1.3 eq., 658 mg, 1.87 mmol) and thiophenol (1.3 eq., 190 µL, 1.86 mmol) at room temperature, and the mixture was stirred at room temperature for 2 hr. Water (15 mL) was added, and the mixture was extracted three times with a mixture of hexane (7.5 mL)/ethyl acetate (22.5 mL) by liquid separation. The organic layers were combined, and washed once with saturated aqueous sodium chloride solution (100 mL) by liquid separation. The organic layer was dried over magnesium sulfate, the magnesium sulfate was removed by filtration, and the filtrate was concentrated under reduced pressure to give a crude product (1.98 g) as a yellow oil. The crude product was purified by flash silica gel column (normal phase silica gel 30 g, hexane/ethyl acetate = 3/1 - 1/1) to give Compound 35-4 (1.15 g, yield 87%) as a white amorphous.

Synthesis of Compound 35-5 from Compound 35-4 was carried out as follows.

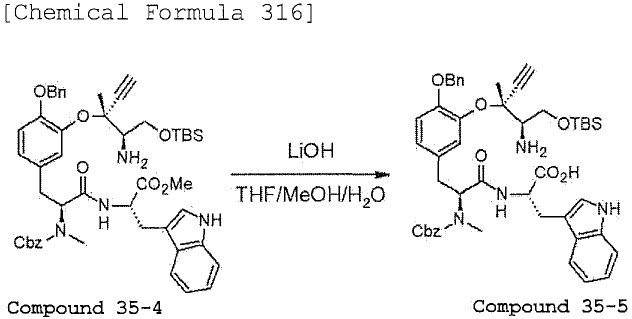

To a solution of Compound 35-4 (1.0 eq., 1.15 g, 1.34 mmol) in a mixture of THF (8 mL)/methanol (4 mL)/water (4 mL) was added lithium hydroxide (3.1 eq., 100 mg, 4.17 mmol) at room temperature, and the mixture was stirred at room temperature for 3 hr. The reaction solution was adjusted to pH 7 by addition of 1N aqueous hydrochloric acid solution (2.5 mL), and water (10 mL) was added. The organic solvent was evaporated by concentration under reduced pressure, and the residue was extracted three times with ethyl acetate (10 mL) by liquid separation. The organic layers were combined, and washed once with saturated aqueous sodium chloride solution (30 mL) by liquid separation. The organic layer was dried over magnesium sulfate, the magnesium sulfate was removed by filtration, and the filtrate was concentrated under reduced pressure to give crude Compound 35-5 (1.09 g, crude yield 96%) as a pale-yellow amorphous.

Synthesis of Compound 35-6 from Compound 35-5 was carried out as follows.

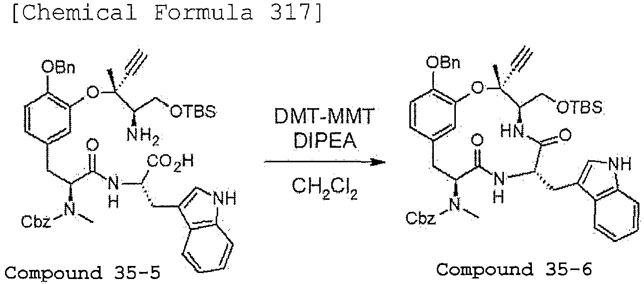

A solution of Compound 35-5 (1.0 eq., 1.09 g, 1.29 mmol) in dichloromethane (33 mL) was added dropwise over 4.5 hr to a solution of DMT-MMT (2.0 eq., 850 mg, 2.59 mmol) and DIPEA (2.0 eq,440 µL, 2.59 mmol) in dichloromethane (109 mL) at about 120 µL/min at room temperature, and the mixture was stirred at room temperature for 19 hr. The reaction solution was concentrated under reduced pressure to evaporate the dichloromethane, ethyl acetate (20 mL) was added, and the mixture was washed twice with 1N aqueous hydrochloric acid solution (20 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (1.24 g) as a gray amorphous. The crude product was purified by flash silica gel column (normal phase silica gel 30 g, hexane/ethyl acetate = 75/25 - 0/100) to give Compound 35-6 (237 mg, yield 22%) as a white solid.

Synthesis of Compound 35-7 from Compound 35-6 was carried out as follows.

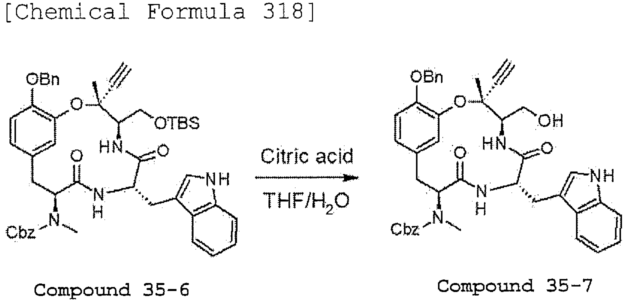

Under nitrogen atmosphere, to a solution of Compound 35-6 (1.0 eq., 237 mg, 286 µmol) in a mixture of THF (4 mL)/water (2 mL) was added citric acid (10.0 eq., 550 mg, 2.86 mmol) at room temperature, and the mixture was stirred at the external temperature of 40°C for 15 hr. The reaction was quenched by addition of saturated aqueous sodium hydrogencarbonate solution (10 mL), and extracted three times with ethyl acetate (10 mL) by liquid separation. The organic layers were combined, and washed once with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (227 mg) as a white amorphous. The crude product was purified by flash silica gel column (normal phase silica gel 5 g, hexane/acetonitrile = 50/50) to give Compound 35-7 (187 mg, 91%) as a white amorphous.

Synthesis of Compound 35-7 from Compound 35-6 was carried out as follows.

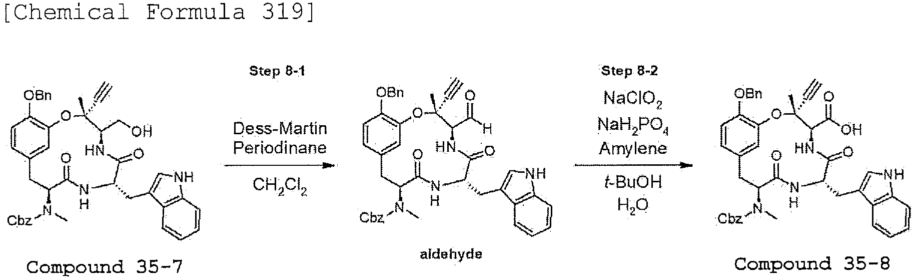

Under nitrogen atmosphere, to a solution of Compound 35-7 (1.0 eq., 187 mg, 261 µmol) in dichloromethane (3 mL) was added Dess-Martin periodinane (1.5 eq., 167 mg, 394 µmol) at room temperature, and the mixture was stirred at room temperature for 1 hr. 20% Aqueous sodium sulfite solution (10 mL) and saturated aqueous sodium hydrogencarbonate solution (10 mL) were added, and the mixture was extracted three times with dichloromethane (10 mL) by liquid separation. The organic layers were combined, and washed once with saturated aqueous sodium chloride solution (30 mL) by liquid separation, and this washing was again extracted twice with dichloromethane (10 mL). The organic layers were combined, and dried over magnesium sulfate, the magnesium sulfate was removed by filtration, and the filtrate was concentrated under reduced pressure to give crude Aldehyde (197 mg, crude yield 106%) as a brown amorphous.

To a solution of Aldehyde (1.0 eq., 197 mg, calculated as 261 µmol) in a mixture of t-butyl alcohol (1.8 mL)/amylene (0.6 mL) was added a solution of sodium dihydrogen phosphate dihydrate (3.5 eq., 144 mg, 923 µmol) and 80% sodium chlorite (4.5 eq., 134 mg, 1.18 mmol) in water (0.6 mL) at room temperature, and the mixture was stirred at room temperature for 30 min. Water (10 mL) was added, and the mixture was extracted three times with ethyl acetate (10 mL) by liquid separation. The organic layers were combined, and washed once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (201 mg) as a brown amorphous. The crude product was purified by flash silica gel column (normal phase silica gel 5 g, hexane/ethyl acetate = 50/50 - 0/100) to give Compound 35-8 (121 mg, 64%) as a pale-orange solid.

Synthesis of Compound 35-9 from Compound 35-8 was carried out as follows.

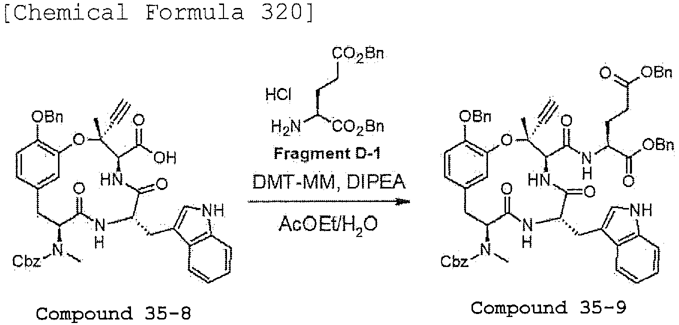

Under nitrogen atmosphere, to a solution of Compound 35-8 (1.0 eq., 121 mg, 166 µmol) in a mixture of ethyl acetate (1.4 mL)/water (0.6 mL) were added dibenzyl L-glutamate hydrochloride (Fragment D-1, 1.5 eq., 90 mg, 247 µmol) at room temperature, and then DIPEA (2.5 eq., 70 µL, 412 µmol) and DMT-MM (1.7 eq., 80 mg, 289 µmol) under ice-cooling, and the mixture was stirred under ice-cooling for 3 hr. Ethyl acetate (10 mL) was added, and the mixture was washed twice with 1N aqueous hydrochloric acid solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (170 mg) as an orange amorphous. The crude product was purified twice by flash silica gel column (1st run: normal phase silica gel 5 g, hexane/ethyl acetate = 75/25 - 25/75, 2nd run: NH₂ silica gel 10 g, hexane/ethyl acetate = 3/1 - 1/7) to give Compound 35-9 (29 mg, 17%) as a white amorphous.

Synthesis of Compound 35 from Compound 35-9 was carried out as follows.

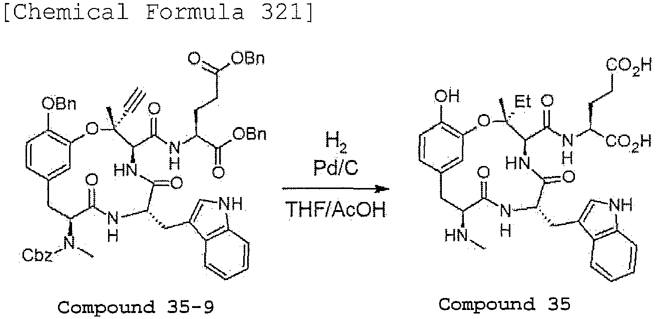

Under nitrogen atmosphere, to a solution of Compound 35-9 (1.0 eq., 29 mg, 27.9 µmol) in a mixture of THF (0.5 mL)/acetic acid (0.5 mL) was added 10% palladium-carbon (N.E. CHEMCAT NX type, 50% hydrous product, 4.3 mg) at room temperature, the system was replaced with hydrogen, and the mixture was stirred at room temperature for 20 hr. Additional 10% palladium-carbon (N.E. CHEMCAT NX type, 50% hydrous product, 4.9 mg) was added, the system was replaced with hydrogen, and the mixture was stirred at room temperature for 20 hr. The catalyst was removed by filtration through Celite, and the filtrate was concentrated under reduced pressure to give a gray solid. To this solid was added an appropriate amount of a mixture of acetonitrile /water=1/1, and the mixture was filtered through 0.45 µm filter to remove the black insoluble substance. The filtrate was concentrated under reduced pressure to give a crude product (18 mg) as a gray solid. The crude product was purified by flash silica gel column (reverse-phase silica gel 6 g, 0.05% aqueous acetic acid solution/acetonitrile = 99/1 - 10/90) to give a mixture (11.8 mg) of an acetate of Compound 35 and impurities as a white solid. [M+H]+=638.27

The mass spectrometry results indicate that the target product has been produced.

### (Example 29I2: Synthesis of Compound 37)

Synthesis of Compound 37-1 from Fragment A-13 and Fragment B-1 was carried out as follows.

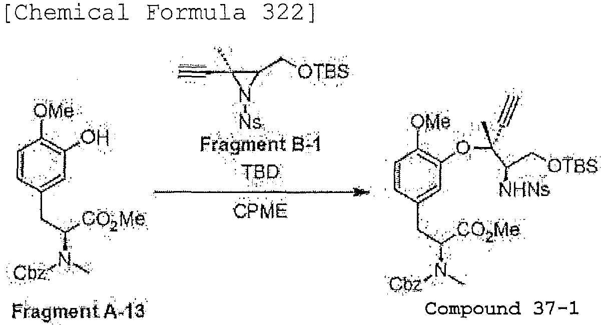

To a solution of Fragment A-13 (1.0 eq., 2.37 g, 6.35 mmol) in CPME (16 mL) was added TBD (1.05 eq., 929 mg, 6.68 mmol) under ice-cooling, and then a solution of Fragment B-1 (1.4 eq., 3.76 g, 9.16 mmol) in CPME (16 mL) was added dropwise thereto over 35 min, and the mixture was stirred at room temperature for 6 hr. The reaction solution was concentrated under reduced pressure to give a crude product (7.82 g) as a brown viscous product. The crude product was purified by flash silica gel column (normal phase silica gel 150 g, hexane/ethyl acetate = 67/33 - 40/60) to give Compound 37-1 (4.17 g, yield 84%) as a white amorphous.

Synthesis of Compound 37-2 from Compound 37-1 was carried out as follows.

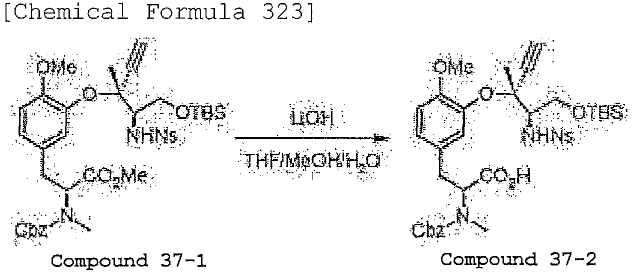

To a solution of Compound 37-1 (1.0 eq., 4.12 g, 5.25 mmol) in a mixture of THF (26 mL)/methanol (13 mL)/water (13 mL) was added lithium hydroxide (3.0 eq., 378 mg, 15.8 mmol) at room temperature, and the mixture was stirred at the same temperature for 1 hr. The reaction solution was adjusted to pH 7 by addition of 1N aqueous hydrochloric acid solution (9 mL), to the reaction solution was added ethyl acetate (40 mL), and the mixture was washed once with water (20 mL) by liquid separation. The aqueous layer was extracted once with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Compound 37-2 (4.35 g, quant.) as a pale-yellow amorphous.

Synthesis of Compound 37-3 from Compound 37-2 was carried out as follows.

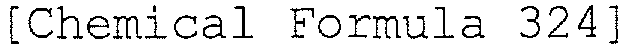

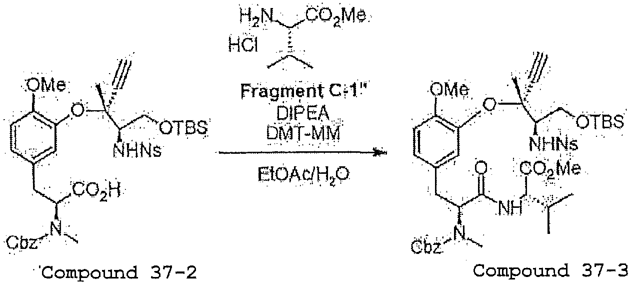

To a solution of Compound 37-2 (1.0 eq., 1.07 g, calculated as 1.39 mmol) in a mixture of ethyl acetate (14 mL)/water (4.2 mL) were added L-valine-OMe hydrochloride (1.5 eq., 350 mg, 2.09 mmol), DIPEA (1.7 eq., 402 µL, 2.36 mmol) and DMT-MM (1.7 eq., 654 mg, 2.36 mmol) at room temperature, and the mixture was stirred at the same temperature for 1 hr. To the reaction solution was added ethyl acetate (20 mL), and the mixture was washed once with water (10 mL), twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Compound 37-3 (1.21 g, crude yield 99%) as a white amorphous.

Synthesis of Compound 37-4 from Compound 37-3 was carried out as follows.

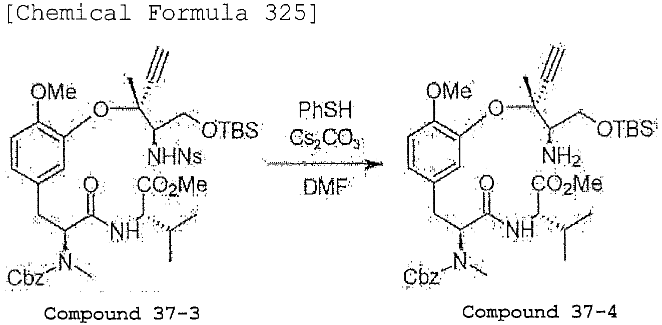

Under nitrogen atmosphere, to a solution of Compound 37-3 (1.0 eq., 1.22 g, 1.38 mmol) in DMF (14 mL) were added cesium carbonate (3.0 eq., 1.35 g, 4.13 mmol) and thiophenol (3.0 eq., 421 µL, 4.13 mmol) at room temperature, and the mixture was stirred at the same temperature for 1 hr. To the reaction solution was added a mixture of hexane (10 mL)/ethyl acetate (30 mL), and the mixture was washed three time with water (20 mL) and once with saturated sodium chloride (10 mL) by liquid separation. The organic layer was dried over magnesium sulfate, the magnesium sulfate was removed by filtration, and the filtrate was concentrated under reduced pressure to give a crude product (1.34 g) as a yellow oil. The crude product was purified by flash silica gel column (normal phase silica gel 20 g, hexane/ethyl acetate = 50/50 - 40/60) to give Compound 37-4 (833 mg, yield 87%) as a yellow oil.

Synthesis of Compound 37-5 from Compound 37-4 was carried out as follows.

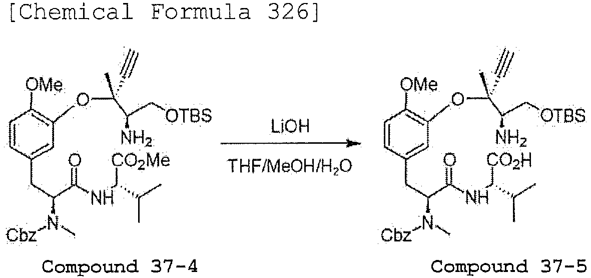

To a solution of Compound 37-4 (1.0 eq., 822 mg, 1.18 mmol) in a mixture of THF (6 mL)/methanol (3 mL)/water (3 mL) was added lithium hydroxide (3.0 eq., 85.0 mg, 3.55 mmol) at room temperature, and the mixture was stirred at the same temperature for 1 hr. The reaction solution was adjusted to pH 7 by addition of 1N aqueous hydrochloric acid solution (1.5 mL). To the reaction solution was added ethyl acetate (20 mL), and the mixture was washed once with water (10 mL) by liquid separation. The aqueous layer was extracted once with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Compound 37-5 (764 mg, crude yield 95%) as a pale-yellow amorphous.

Synthesis of Compound 37-6 from Compound 37-5 was carried out as follows.

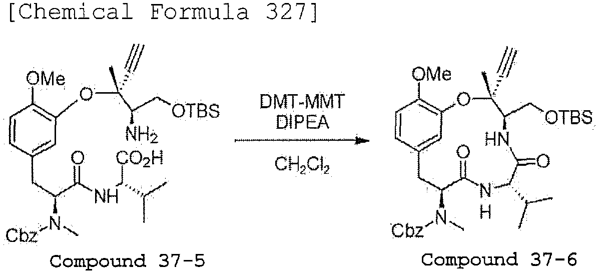

A solution of Compound 37-5 (1.0 eq., 699 mg, 1.02 mmol) in dichloromethane (42 mL) was added dropwise to a solution of DMT-MMT (2.0 eq., 671 mg, 2.04 mmol) and DIPEA (2.0 eq., 348 µL, 2.05 mmol) in dichloromethane (70 mL) at about 40 µL/min at room temperature, and the mixture was stirred at the same temperature for 19.5 hr. The reaction solution was washed three time with saturated aqueous ammonium chloride solution (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give a crude product. The crude product obtained from Compound 37-5 (50.3 mg, 73.5 µmol) by the same reaction was combined with the crude product previously obtained. Ethyl acetate was added, the precipitated white solid was removed by filtration, and the filtrate was concentrated under reduced pressure to give a crude product (789 mg) as a pale-yellow amorphous. The crude product was purified three times by flash silica gel column (1st run: normal phase silica gel 30 g, hexane/ethyl acetate = 80/20 - 59/41, 2nd run: NH₂ silica gel 5 g, hexane/ethyl acetate = 75/25 - 0/100, 3rd run: normal phase silica gel 5 g, hexane/ethyl acetate = 80/20 - 74/26) to give Compound 37-6 (234 mg, yield 32%) as a white amorphous.

Synthesis of Compound 37-7 from Compound 37-6 was carried out as follows.

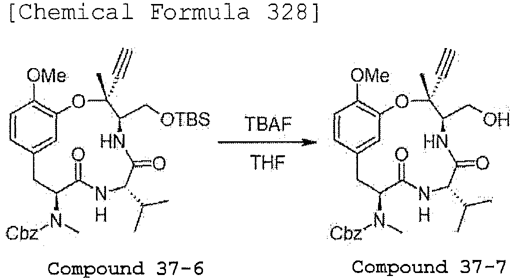

To a solution of Compound 37-6 (1.0 eq., 231 mg, 0.348 mmol) in THF (3.5 mL) was added TBAF (1M in THF, 2.0 eq., 695 µL, 0.695 mmol) at room temperature, and the mixture was stirred at the same temperature for 2 hr. To the reaction solution was added ethyl acetate (20 mL), and the mixture was washed three time with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (211 mg) as a white amorphous. The crude product was purified by flash silica gel column (normal phase silica gel 10 g, hexane/ethyl acetate = 50/50 - 0/100) to give Compound 37-7 (144 mg, yield 75%) as a white amorphous.

Synthesis of Compound 37-8 from Compound 37-7 was carried out as follows.

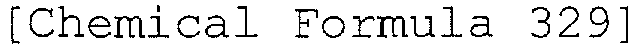

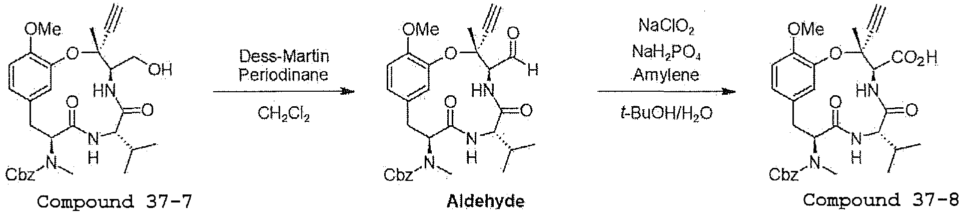

Under nitrogen atmosphere, to a solution of Compound 37-7 (1.0 eq., 139 mg, 0.252 mmol) in dichloromethane (2.5 mL) was added Dess-Martin periodinane (1.5 eq., 161 mg, 0.379 mmol) under ice-cooling, and the mixture was stirred at room temperature for 2 hr. Additional Dess-Martin periodinane (1.0 eq., 107 mg, 0.252 mmol) was added under ice-cooling, and the mixture was stirred at room temperature for 1.5 hr. To the reaction solution was added a mixture of 10% aqueous sodium sulfite solution (5 mL)/saturated aqueous sodium hydrogencarbonate solution (5 mL), and the mixture was extracted once with ethyl acetate (20 mL) by liquid separation. The organic layer was washed once with a mixture of 10% aqueous sodium sulfite solution (5 mL)/saturated aqueous sodium bicarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (132 mg, crude yield 95%) as a white amorphous.

To a solution of Aldehyde (1.0 eq., 131 mg, 0.238 mmol) in a mixture of amylene (1 mL)/t-butyl alcohol (4 mL) was added a solution of sodium dihydrogen phosphate dihydrate (3.4 eq., 127 mg, 0.816 mmol) and 80% sodium chlorite (4.5 eq., 121 mg, 1.07 mmol) in water (1 mL) at room temperature, and the mixture was stirred at the same temperature for 1 hr. To the reaction solution was added ethyl acetate (20 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Compound 37-8 (143 mg, crude yield 106%) as a white amorphous.

Synthesis of Compound 37-9 from Compound 37-8 was carried out as follows.

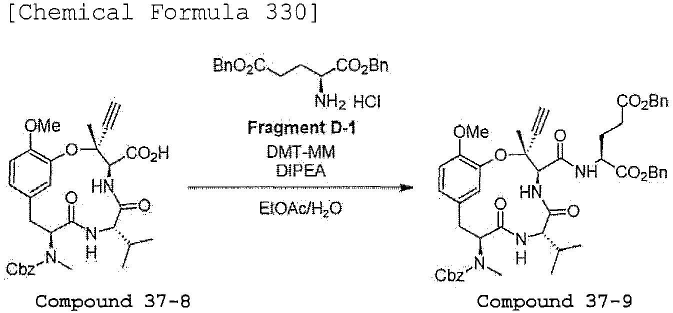

To a solution of Compound 37-8 (1.0 eq., 138 mg, calculated as 0.238 mmol) in a mixture of ethyl acetate (1.4 mL)/water (0.42 mL) was added dibenzyl L-glutamate hydrochloride (Fragment D-1, 1.5 eq., 130 mg, 0.357 mmol) at room temperature. DIPEA (2.4 eq., 97.2 µL, 0.572 mmol) was added under ice-cooling, and the mixture was stirred for 30 min, DMT-MM (1.7 eq., 113 mg, 0.408 mmol) was added, and the mixture was stirred at the same temperature for 3 hr. To the reaction solution was added ethyl acetate (20 mL), and the mixture was washed once with water (10 mL), twice with saturated aqueous ammonium chloride solution (10 mL), twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (250 mg) as a yellow oil. The crude product was purified by flash silica gel column (NH₂ silica gel 6 g, hexane/ethyl acetate = 75/25 - 50/50) to give Compound 37-9 (155 mg, yield 75%) as a white amorphous.

Synthesis of Compound 37 from Compound 37-9 was carried out as follows.

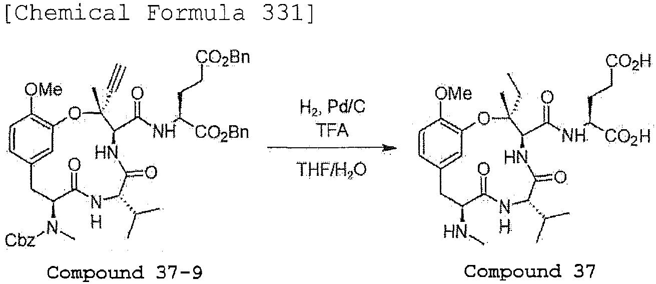

Under nitrogen atmosphere, to a solution of Compound 37-9 (1.0 eq., 154 mg, 0.176 mmol) in a mixture of THF (3 mL)/water (0.3 mL) was added TFA (5.0 eq., 67.1 µL, 0.877 mmol) at room temperature, and the mixture was stirred at the same temperature for 40 min. 10% Palladium-carbon (N.E. CHEMCAT NX type, 50% hydrous product, 15.4 mg) was added, the system was replaced with hydrogen, and the mixture was stirred at the same temperature for 16 hr. The catalyst was removed by filtration through Celite, and the filtrate was filtered through 0.45 µm filter, and concentrated under reduced pressure to give a crude product (137 mg) as a white solid. The crude product was purified by flash silica gel column (reverse-phase silica gel 10 g, water/acetonitrile = 95/5 - 81/19), and freeze-dried to give a TFA salt of Compound 37 (50.1 mg, yield 51%, purity 99.3%) as a white solid.

¹H-NMR(400MHz,DMSO-d6)δ8.30(d,J=8.2Hz,1H),8.15(d,J=7.1Hz,1H),7.58(d,J=8.9Hz,1H),6. 89-6.85(m,3H),4.71(d,J=9.6Hz,1H),4.21-4.14(m,2H),3.68(s,3H) ,2.84-2.72(m,2H),2.32-2.18(m,SH),2.09-2.00(m,1H),1.93-1.71(m,3H),1.64-1.55(m,1H),1.32(s,3H),0.92(t,J=7.2Hz,3H),0.79(d,J=6.4Hz,6H).

### (Example 29J: Synthesis of Compound 39)

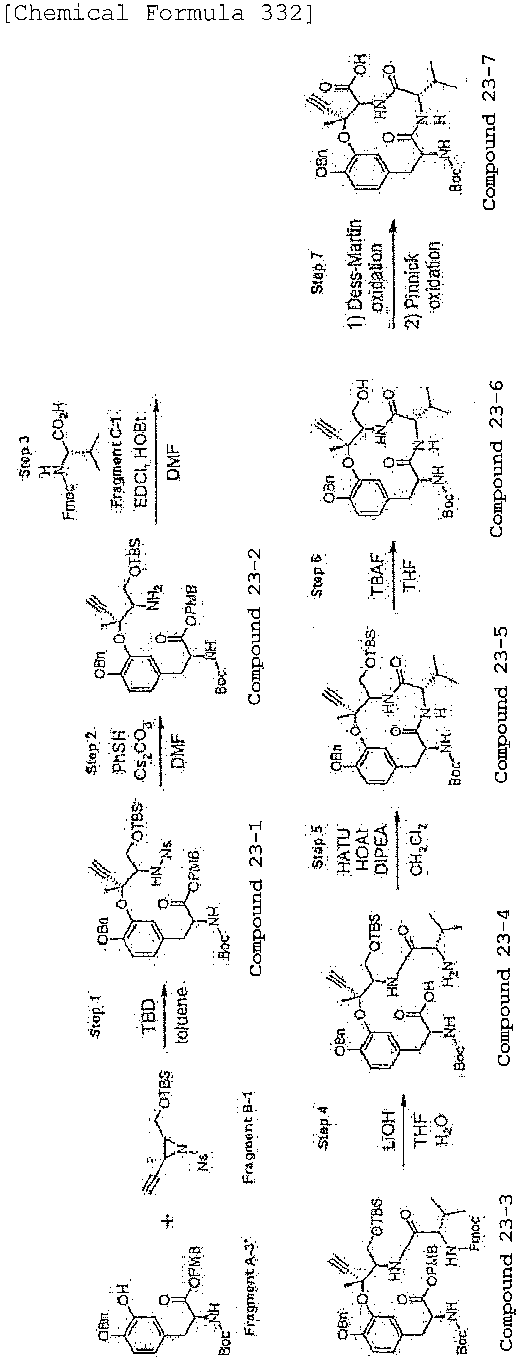

Synthesis of Compound 23-7 was carried out as described in Example 28.

Synthesis of Compound 39-1 from Compound 23-7 was carried out as follows.

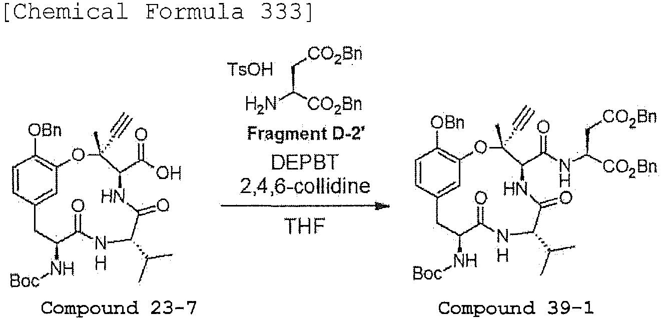

Under nitrogen atmosphere, to a solution of Compound 23-7 (1.0 eq., 201 mg, 339 µmol) and dibenzyl L-glutamate paratoluenesulfonate (Fragment D-2, 1.6 eq., 255 mg, 524 µmol) in THF (4 mL) were added DEPBT (1.5 eq., 154 mg, 516 µmol) and 2,4,6-collidine (3.1 eq., 140 µL, 1.06 mmol) under ice-cooling, and the mixture was stirred under ice-cooling for 8 hr, and then at room temperature for 20 hr. To the reaction solution was added saturated aqueous sodium hydrogencarbonate solution (10 mL), and the mixture was extracted three times with ethyl acetate (5 mL) by liquid separation. The organic layers were combined, and washed once with a mixture of saturated aqueous sodium hydrogencarbonate solution (15 mL)/saturated aqueous sodium chloride solution (15 mL) by liquid separation. The organic layer was dried over magnesium sulfate, the magnesium sulfate was removed by filtration, and the filtrate was concentrated under reduced pressure to give a crude product (524 mg). The crude product was purified twice by flash silica gel column (1st run: NH₂ silica gel 8 g, hexane/ethyl acetate = 80/20 - 10/90, 2nd run: normal phase silica gel 10 g, hexane/ethyl acetate = 2/1 - 1/2) to give Compound 39-1 (51 mg, yield 17%) as a pale-yellow amorphous.

Synthesis of Compound 39-2 from Compound 39-1 was carried out as follows.

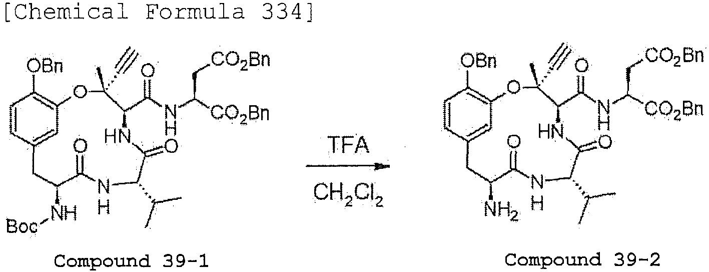

Under nitrogen atmosphere, to a solution of Compound 39-1 (1.0 eq., 51 mg, 57 µmol) in dichloromethane (1 mL) was added TFA (23.0 eq., 100 µL, 1.31 mmol) under ice-cooling, and the mixture was stirred at room temperature for 1.5 hr. To the reaction solution was added saturated aqueous sodium hydrogencarbonate solution (5 mL), and the mixture was extracted three times with ethyl acetate (5 mL) by liquid separation. The organic layers were combined, and washed once with saturated aqueous sodium hydrogencarbonate solution (10 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give Compound 39-2 (45 mg, crude yield 100%).

Synthesis of Compound 39-3 from Compound 39-2 was carried out as follows.

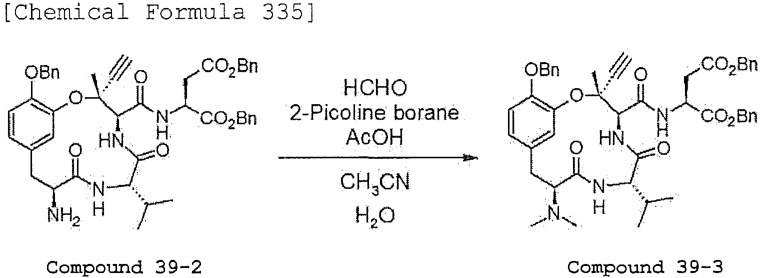

To a solution of Compound 39-2 (1.0 eq., 45 mg, calculated as 57 µmol) in a mixture of acetonitrile (400 µL)/water (200 µL) were added 37% formaldehyde aqueous solution (10.8 eq., 50 µL, 616 µmol) and acetic acid (2.2 eq., 7 µL, 122 µmol) at room temperature, and the mixture was stirred at room temperature for 30 min. Then, 2-picoline borane (2.5 eq., 15 mg, 142 µmol) was added under ice-cooling, and the mixture was stirred at room temperature for 30 min. To the reaction solution was added saturated aqueous sodium hydrogencarbonate solution (5 mL), and the mixture was extracted three times with ethyl acetate (5 mL) by liquid separation. The organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (29 mg). The crude product was purified by flash silica gel column (normal phase silica gel 7 g, chloroform/methanol = 50/1 - 30/1) to give Compound 39-3 (26 mg, two-step yield 57%) as a white amorphous.

Synthesis of Compound 39 from Compound 39-3 was carried out as follows.

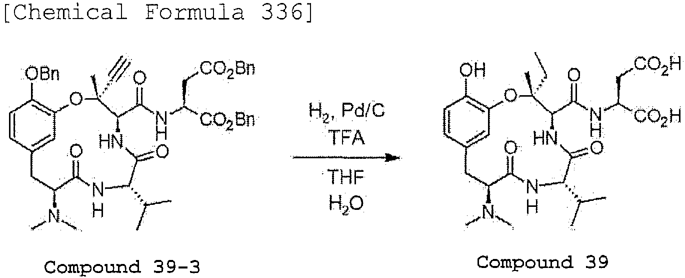

Under nitrogen atmosphere, to a solution of Compound 39-3 (1.0 eq., 26 mg, 32 µmol) in a mixture of THF (2 mL)/water (100 µL) was added TFA (4.0 eq., 10 µL, 131 µmol) at room temperature, and the mixture was stirred at the same temperature for 2 hr. Then, 10% palladium-carbon (N.E. CHEMCAT NX type, 50% hydrous product, 3 mg) was added at room temperature, the system was replaced with hydrogen, and the mixture was stirred at room temperature for 2 hr. The catalyst was removed by filtration through Celite, and the filtrate was concentrated under reduced pressure to give a crude product (24 mg). The crude product was purified by flash silica gel column (reverse-phase silica gel 12 g, 0.05% aqueous TFA solution/acetonitrile = 100/0 - 0/100), and freeze-dried to give a TFA salt of Compound 39 (8.0 mg, yield 37%, purity 93%) as a white solid.

¹H-NMR(400MHz,D₂O)δ6.81-6.76(m,3H),4.45(dd,J=7.4,5.0Hz,1H),3.93(d,J=10.4Hz,1H),3.82(dd, J=11.6,5.2Hz,1H),3.29(dd,J=12.4,5.2Hz,1H),2.86(brs,1H),2.81-2.65(m,3H),1.99-1.90(m,1H),1.76-1.66(m,1H),1.57-1.46(m,4H),0.93(t,J=7.2Hz,3H),0.69(d,J=6.8Hz,3H),0.59(d,J=6.8Hz ,3H).

### (Example 29K: Synthesis of Compound 40)

Compound 40 was synthesized.

Synthesis was carried out as follows.

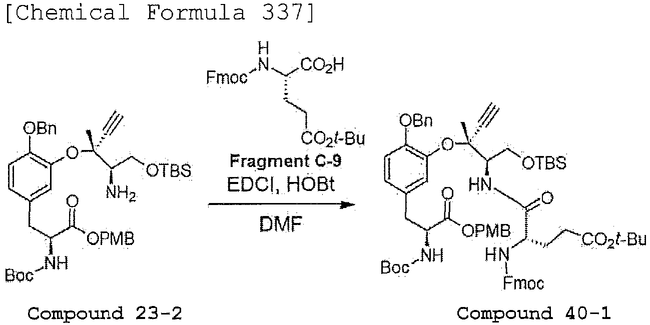

Under nitrogen atmosphere, to a solution of Compound 23-2 (1.0 eq., 1.02 g, calculated as 1.39 mmol) and 5-t-Bu-N-Fmoc-L-aspartic acid (Fragment C-9,1.2 eq., 697 mg, 1.64 mmol) in DMF (14 mL) were added HOBt•H₂O (1.2 eq., 251 mg, 1.63 mmol) and EDCI (1.2 eq., 312 mg, 1.63 mmol) at room temperature, and the mixture was stirred at the same temperature for 19 hr. A mixture of hexane (5 mL)/ethyl acetate (15 mL) was added, and the mixture was washed three times with water (10 mL) by liquid separation. The organic layer was dried over magnesium sulfate, the magnesium sulfate was removed by filtration, and the filtrate was concentrated under reduced pressure to give crude Compound 40-1 (1.59 g, crude yield 100%) as a white amorphous.

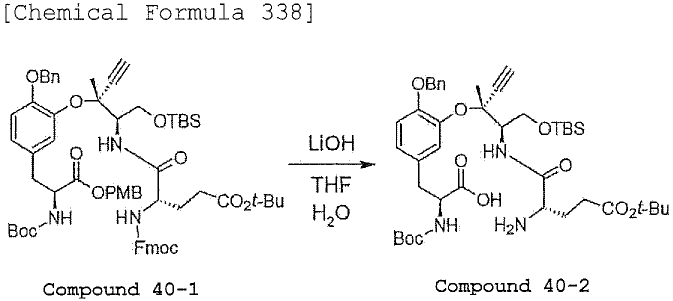

Under nitrogen atmosphere, to a solution of Compound 40-1 (1.0 eq., 1.59 g, 1.39 mmol) in a mixture of THF (10 mL)/water (5 mL) was added lithium hydroxide (3.9 eq., 130 mg, 5.44 mmol) at room temperature, and the mixture was stirred at the same temperature for 2.5 hr. 2N Aqueous hydrochloric acid solution (1.8 mL) was added under ice-cooling, and the mixture was extracted once with ethyl acetate (10 mL) and once with ethyl acetate (5 mL) by liquid separation. The organic layers were combined, and dried over magnesium sulfate, the magnesium sulfate was removed by filtration, and the filtrate was concentrated under reduced pressure to give a crude product (2.36 g). The crude product was purified by flash silica gel column (normal phase silica gel 17.8 g, ethyl acetate/methanol = 1/0 - 10/1) to give Compound 40-2 (601 mg, two-step yield 54%) as a white amorphous.

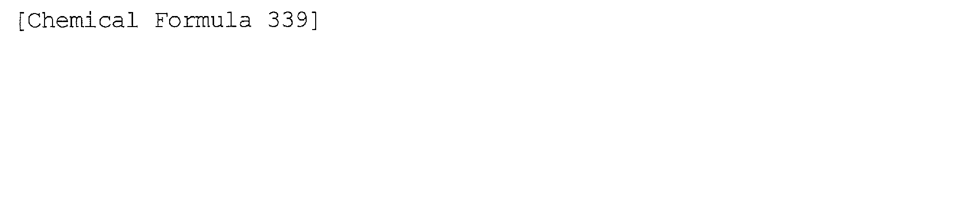

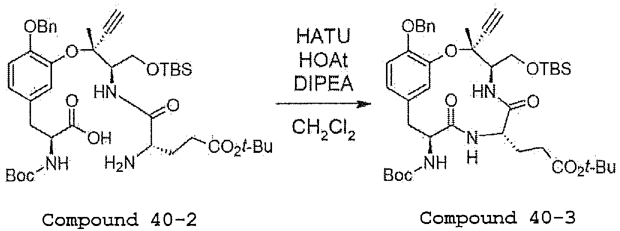

A solution of Compound 40-2 (1.0 eq., 601 mg, 754 µmol) in dichloromethane (15 mL) was added dropwise over 2.5 hr to a solution of HATU (2.0 eq., 576 mg, 1.52 mmol), HOAt (2.0 eq., 206 mg, 1.52 mmol) and DIPEA (2.0 eq., 250 µL, 1.47 mmol) in dichloromethane (380 mL) at room temperature, and the mixture was stirred at the same temperature for 2.5 hr. The reaction solution was concentrated under reduced pressure to 50 mL, saturated aqueous ammonium chloride solution (50 mL) was added, and the mixture was extracted once with ethyl acetate (75 mL) and once with ethyl acetate (30 mL) by liquid separation. The organic layers were combined, and washed once with saturated aqueous sodium hydrogencarbonate solution (30 mL), once with water (20 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give a crude product (758 mg) as an orange amorphous. The crude product was purified by flash silica gel column (normal phase silica gel 7.9 g, hexane/ethyl acetate = 3/1 - 2/1) to give Compound 40-3 (254 mg, apparent yield 43%, containing about 25% diastereomer) as a white amorphous.

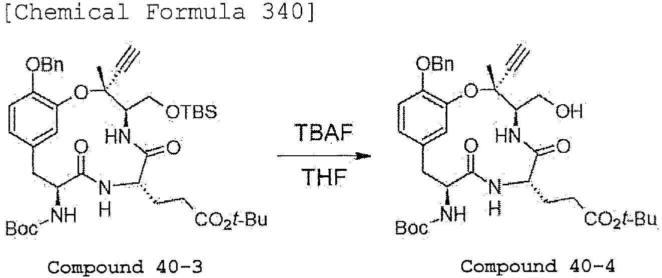

Under nitrogen atmosphere, to a solution of Compound 40-3 (1.0 eq., 254 mg, 325 umol, containing about 25% diastereomer) in THF (3 mL) was added TBAF (1M in THF, 1.5 eq., 500 µL, 500 µmol) under ice-cooling, and the mixture was stirred at room temperature for 1 hr. To the reaction solution was added saturated aqueous ammonium chloride solution (3 mL), and the mixture was extracted with ethyl acetate (15 mL) by liquid separation. The organic layer was washed three time with saturated aqueous ammonium chloride solution (3 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give a crude product (243 mg) as a white amorphous. The crude product was slurry-washed three time with hexane (2.7 mL)/ethyl acetate (0.3 mL) to give Compound 40-4 (207 mg, yield 95%, containing about 25% diastereomer) as a white solid.

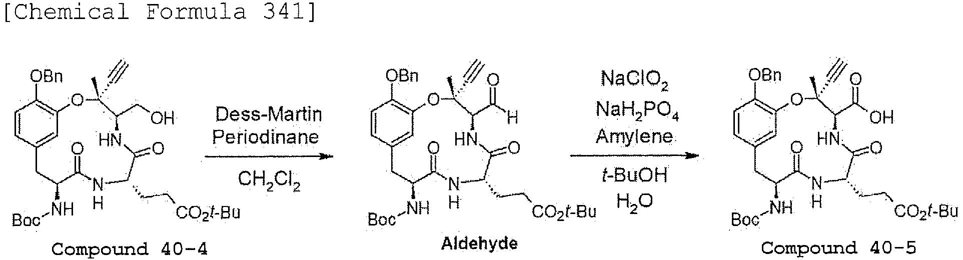

Under nitrogen atmosphere, to a solution of Compound 40-4 (1.0 eq., 207 mg, 310 µmol, containing about 25% diastereomer) in dichloromethane (3 mL) was added Dess-Martin periodinane (1.5 eq., 198 mg, 466 µmol) under ice-cooling, and the mixture was stirred at room temperature for 1.5 hr. To the reaction solution was added a mixture of 10% sodium hydrogen sulfite aqueous solution (2 mL)/saturated aqueous sodium hydrogencarbonate solution (2 mL), and the mixture was extracted with ethyl acetate (15 mL) by liquid separation. The organic layer was washed twice with saturated aqueous sodium hydrogencarbonate solution (3 mL), once with water (3 mL) and once with saturated aqueous sodium chloride solution (2 mL) by liquid separation. The organic layer was dried over magnesium sulfate, the magnesium sulfate was removed by filtration, and the filtrate was concentrated under reduced pressure to give Aldehyde (196 mg) as a white solid.

To a solution of Aldehyde (1.0 eq., 196 mg, calculated as 310 µmol) in a mixture of t-butyl alcohol (1.5 mL)/amylene (0.7 mL) was added dropwise a solution of sodium dihydrogen phosphate dihydrate (3.5 eq., 169 mg, 1.08 mmol) and 80% sodium sulfite (4.6 eq., 160 mg, 1.41 mmol) in water (0.7ml) at room temperature, and the mixture was stirred at the same temperature for 2 hr. To the reaction solution was added saturated aqueous sodium hydrogencarbonate solution (3 mL), and the mixture was extracted twice with ethyl acetate (5 mL) by liquid separation. The organic layers were combined, and dried over magnesium sulfate, the magnesium sulfate was removed by filtration, and the filtrate was concentrated under reduced pressure to give a crude product (233 mg) as a white amorphous. The crude product was purified by flash silica gel column (normal phase silica gel 4.3 g, hexane/ethyl acetate = 2/1→ethyl acetate/methanol=5/1) to give Compound 40-5 (74.8 mg, yield 35%) as a white amorphous.

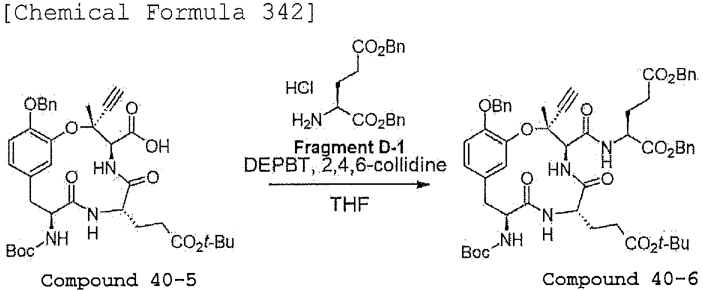

Under nitrogen atmosphere, to a solution of Compound 40-5 (1.0 eq., 116 mg, 170 µmol) and dibenzyl L-glutamate hydrochloride (Fragment D-1, 1.5 eq, 93.8 mg, 257 pmol) in THF (1.7 mL) were added 2,4,6-collidine (3.1 eq., 70 µL, 531 µmol) and DEPBT (1.5 eq., 78.0 mg, 261 µmol) under ice-cooling, and the mixture was stirred under ice-cooling for 6 hr, and then at room temperature for 18 hr. To the reaction solution was added saturated aqueous ammonium chloride solution (2 mL), and the mixture was extracted once with ethyl acetate (8 mL) and once with ethyl acetate (5 mL) by liquid separation. The organic layers were combined, and washed once with saturated aqueous sodium hydrogencarbonate solution (3 mL) and once with saturated aqueous sodium chloride solution (3 mL) by liquid separation. The organic layer was dried over magnesium sulfate, the magnesium sulfate was removed by filtration, and the filtrate was concentrated under reduced pressure to give a crude product (244 mg) as a deep-green viscous product. The crude product was purified twice by flash silica gel column (1st run: NH₂ silica gel 4.1 g, hexane/ethyl acetate = 3/1 - 1/1, 2nd run: normal phase silica gel 5.7 g, hexane/ethyl acetate = 3/1 - 1/1) to give Compound 40-6 (45.3 mg, yield 27%) as a white amorphous.

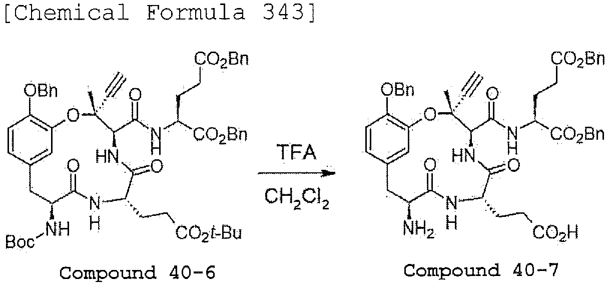

Under nitrogen atmosphere, to a solution of Compound 40-6 (1.0 eq., 45.3 mg, 45.8 µmol) in dichloromethane (400 µL) was added TFA (14 eq., 50 µL, 653 µmol) at room temperature, and the mixture was stirred at room temperature for 1.5 hr. TFA (7.1 eq., 25 µL, 327 µmol) was added under ice-cooling, and the mixture was stirred at room temperature for 2 hr. To the reaction solution was added saturated aqueous sodium hydrogencarbonate solution (1 mL), and the mixture was extracted once with ethyl acetate (4 mL) by liquid separation. The organic layer was washed once with water (1 mL) by liquid separation, and the aqueous layer was extracted again with ethyl acetate (5 mL) by liquid separation. The organic layers were combined, and dried over magnesium sulfate, the magnesium sulfate was removed by filtration, and the filtrate was concentrated under reduced pressure to give crude Compound 40-7 (44.9 mg, crude yield 118%).

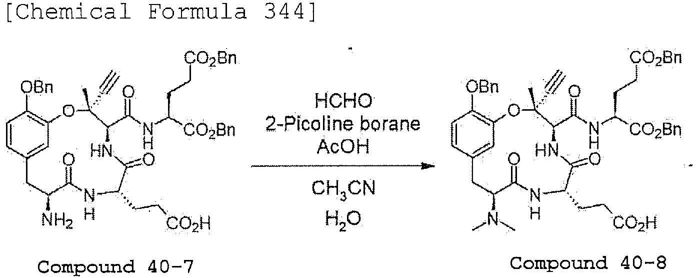

To a solution of Compound 40-7 (1.0 eq., 45.3 mg, 45.8 µmol) in a mixture of acetonitrile (300 µL)/water (150 µL) were added acetic acid (2.0 eq., 5.3 µL, 92.7 µmol) and 37% formaldehyde aqueous solution (10 eq., 34.3 µL, 465 µmol) at room temperature, and the mixture was stirred at room temperature for 30 min. Then, 2-picoline borane (2.6 eq., 12.5 mg, 117 µmol) was added under ice-cooling, and the mixture was stirred at room temperature for 1 hr. To the reaction solution was added saturated aqueous sodium hydrogencarbonate solution (500 µL), and the mixture was extracted twice with ethyl acetate (5 mL) by liquid separation. The organic layers were combined, and dried over magnesium sulfate, the magnesium sulfate was removed by filtration, and the filtrate was concentrated under reduced pressure to give a crude product (42.1 mg). The crude product was purified by flash silica gel column (normal phase silica gel 5.7 g, ethyl acetate/methanol = 1/0 - 3/1) to give Compound 40-8 (23.8 mg, two-step yield 60%) as a white amorphous.

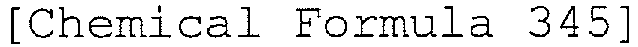

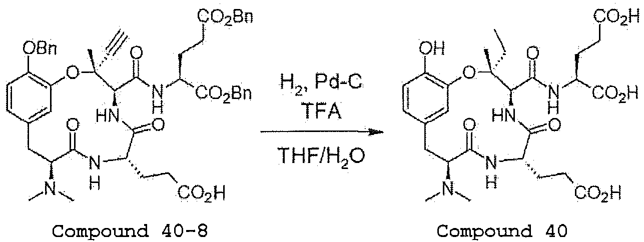

Under nitrogen atmosphere, to a solution of Compound 40-8 (1.0 eq., 36 mg, 0.042 mmol) in a mixture of THF (1440 µL)/water (72 µL) was added TFA (5.0 eq., 16 µL, 0.209 mmol) at room temperature, and the mixture was stirred at the same temperature for 1 hr. 10% Palladium-carbon (N.E. CHEMCAT NX type, 50% hydrous product, 3.6 mg) was added, the system was replaced with hydrogen, and the mixture was stirred at the same temperature for 23.5 hr. The catalyst was removed by filtration through Celite, and the filtrate was concentrated under reduced pressure to give a crude product. The crude product was purified by flash silica gel column (reverse-phase silica gel 30 g, 0.05% aqueous TFA solution/acetonitrile = 95/5 - 90/10), and freeze-dried to give a TFA salt of Compound 40 (15.3 mg, yield 52%, purity 95.0%) as a white solid.

¹H-NMR(400MHz,D₂O)57.04(s,1H)6.95(s,2H)4.77-4.74 (m,1H),4.48-4.39(m,2H),3.92(dd,J=11.2,4.8Hz,1H),3.42(dd,J=12.4,4.8Hz,1H),3. 01(brs,6H),2.88(t,J=12.0Hz,1H),2.52-2.36(m,2H),2.35-2.15(m,3H),2.13-1.87(m,3H),1.86-1.74(m,1H),1.73-1.60(m,4H),1.03(t,J=7.2Hz,3H).

### (Example 29M2: Synthesis of Compounds 41 and 42)

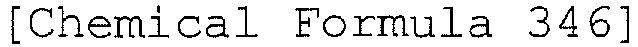

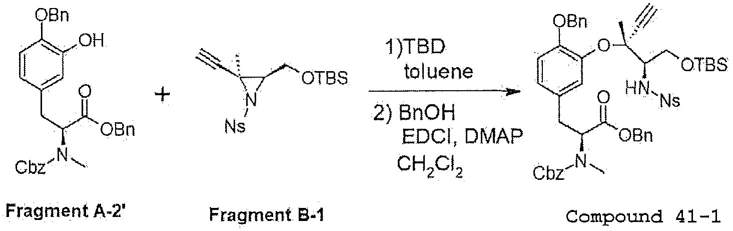

Under nitrogen atmosphere, to a solution of Fragment A-2 (1.05 eq., 4.00 g, containing 1.1wt% ethyl acetate, 7.53 mmol) and Fragment B-1 (1.0 eq., 2.94 g, 7.16 mmol) in toluene (72 mL) was added TBD (1.05 eq., 1.05 g, 7.54 mmol) at room temperature, and the mixture was stirred for 21 hr. To the reaction solution was added 5% aqueous citric acid solution (30 mL), and the mixture was washed by liquid separation, and the organic layer was washed twice with 5% aqueous sodium chloride solution (30 mL) by liquid separation, and dried over sodium sulfate. The sodium sulfate was removed by filtration, and the filtrate was concentrated under reduced pressure to give a mixture (7.28 g) of Compound 41-1 and a de-Bn product of Compound 41-1 as a yellow viscous product.

Under nitrogen atmosphere, to a solution of a mixture of Compound 41-1 and a de-Bn product of Compound 41-1 (1.0 eq., 7.28 g, calculated as 7.16 mmol) in dichloromethane (30 mL) were added benzyl alcohol (0.2 eq., 0.15 g, 1.39 mmol), DMAP (0.05 eq., 44 mg, 0.36 mmol) and EDCI (0.2 eq., 0.27 g, 1.41 mmol) at room temperature, and the mixture was stirred at the same temperature for 1 hr. The reaction solution was washed once with a mixture of 5% aqueous citric acid solution (30 mL)/5% aqueous sodium chloride solution (20 mL), once with 5% aqueous sodium hydrogencarbonate solution (30 mL) and once with 5% aqueous sodium chloride solution (30 mL) by liquid separation, and dried over sodium sulfate. The sodium sulfate was removed by filtration, the filtrate was concentrated under reduced pressure, and the obtained crude product (7.67 g) was purified twice by flash silica gel column (normal phase silica gel 40 g, hexane/ethyl acetate = 90/10 - 50/50) to give Compound 41-1 (5.56 g, containing 2.3wt% ethyl acetate, conversion yield 81%) as a pale-yellow viscous product.

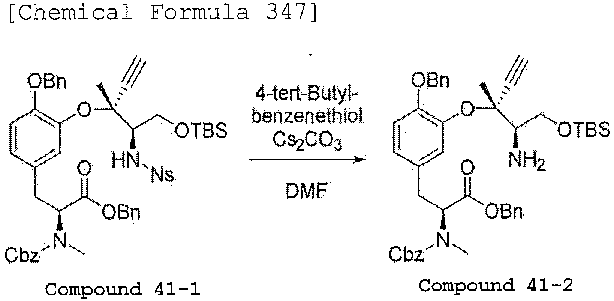

Under nitrogen atmosphere, to a solution of Compound 41-1 (1.0 eq., 1.00 g, containing 2.3wt% ethyl acetate, 1.04 mmol) in DMF (5 mL) were added 4-tert-butylbenzenethiol (3.0 eq., 0.52 g, 3.13 mmol) and cesium carbonate (3.0 eq., 1.02 g, 3.13 mmol) at room temperature, and the mixture was stirred at the same temperature for 1.5 hr. To the reaction solution was added toluene (20 mL), and the mixture was washed once with water (20 mL) and once with a mixture of 5% aqueous potassium carbonate solution (20 mL)/5% aqueous sodium chloride solution (20 mL) by liquid separation, and dried over sodium sulfate. The sodium sulfate was removed by filtration, the filtrate was concentrated under reduced pressure, and the obtained crude product (1.37 g) was purified by flash silica gel column (normal phase silica gel, 25 g, hexane/ethyl acetate = 90/10 - 0/100) to give Compound 41-2 (0.76 g, containing 3.0wt% ethyl acetate, conversion yield 94%) as a pale-yellow oil.

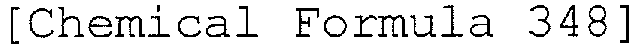

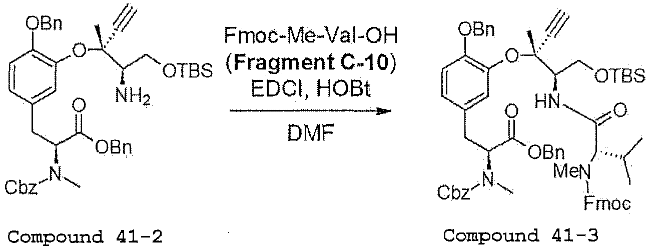

Under nitrogen atmosphere, to a solution of Compound 41-2 (1.0 eq., 660 mg, containing 3.0wt% ethyl acetate, 0.85 mmol) in DMF (6.6 mL) were added N-Fmoc-N-methyl-L-valine (Fragment C-10, 1.5 eq., 0.45 g, 1.27 mmol), EDCI (1.5 eq., 0.25 g, 1.30 mmol) and HOBt•H₂O (1.5 eq., 0.20 g, 1.31 mmol), and the mixture was stirred at room temperature for 19.5 hr. To the reaction solution was added toluene (15 mL), and the mixture was washed once with 5% aqueous sodium hydrogencarbonate solution (15 mL) and once with 5% aqueous sodium chloride solution (15 mL) by liquid separation, and dried over sodium sulfate. The sodium sulfate was removed by filtration, and the filtrate was concentrated under reduced pressure to give Compound 41-3 (0.99 g) as a pale-yellow viscous product.

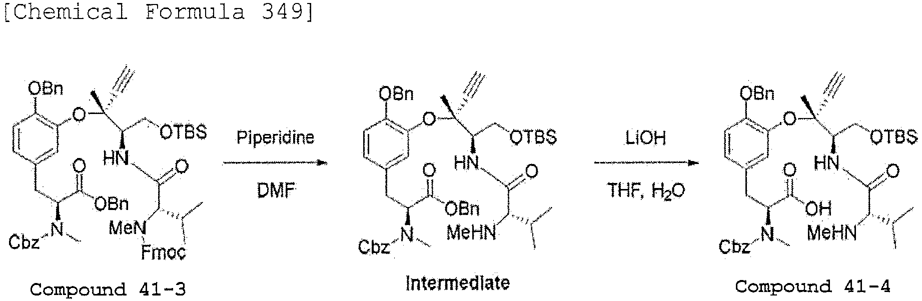

Under nitrogen atmosphere, to a solution of Compound 41-3 (1.0 eq., 0.99 g, calculated as 0.85 mmol) in DMF (10 mL) was added piperidine (0.5 mL), and the mixture was stirred at room temperature for 1 hr. To the reaction solution was added toluene (20 mL), and the mixture was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, the filtrate was concentrated under reduced pressure, and the obtained crude product (0.92 g) was purified by flash silica gel column (normal phase silica gel 10 g, hexane/ethyl acetate = 90/10 - 0/100) to give Intermediate (700 mg, containing 5.0wt% ethyl acetate, two-step conversion yield from Compound 41-2 90%) as a pale-yellow viscous product.

Under nitrogen atmosphere, to a solution of Intermediate (1.0 eq., 800 mg, containing 5.0wt% ethyl acetate, 0.88 mmol) in a mixture of THF (4 mL)/water (2 mL) was added lithium hydroxide monohydrate (2.0 eq., 74 mg, 1.76 mmol), and the mixture was stirred at room temperature for 3 hr. The reaction solution was ice-cooled, 5% aqueous citric acid solution (10 mL) was added, and the mixture was extracted once with ethyl acetate (10 mL) by liquid separation. The organic layer was washed twice with 5% aqueous sodium chloride solution (10 mL) by liquid separation, and dried over sodium sulfate. The sodium sulfate was removed by filtration, the filtrate was concentrated under reduced pressure, and the obtained crude product (780 mg) was purified by flash silica gel column (normal phase silica gel 10 g, hexane/ethyl acetate/methanol = 90/10/0 - 0/100/0 - 0/70/30) to give Compound 41-4 (688 mg, containing 3.8wt% ethyl acetate and 0.1wt% acetic acid, conversion yield 97%) as a pale-yellow amorphous.

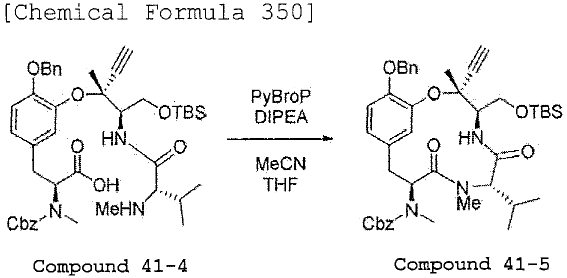

Under nitrogen atmosphere, to a solution of PyBroP (5.0 eq., 1.54 g, 3.30 mmol) in acetonitrile (648 mL) was added DIPEA (10.0 eq., 0.85 g, 6.58 mmol) at room temperature. A solution of Compound 41-4 (1.0 eq., 529 mg, containing 3.8wt% ethyl acetate and 0.1wt% acetic acid, 0.66 mmol) in a mixture of acetonitrile (6.3 mL)/THF (6.3 mL) was added over 21 hr at the external temperature of 50°C, and after the dropwise addition was completed, the mixture was stirred at the same temperature for additional 0.5 hr. The reaction solution was concentrated under reduced pressure, to the concentrated residue was added ethyl acetate (10 mL), the mixture was subjected to ultrasonic irradiation, and the insoluble substance was removed by filtration. The filtrate was washed once with 5% aqueous citric acid solution (10 mL), once with 5% aqueous sodium hydrogencarbonate solution (10 mL) and once with 5% aqueous sodium chloride solution (10 mL) by liquid separation, and dried over sodium sulfate. The sodium sulfate was removed by filtration, the filtrate was concentrated under reduced pressure, and the obtained crude product (1.65 g) was purified by flash silica gel column (normal phase silica gel 100 g, hexane/ethyl acetate = 90/10 - 0/100) to give Compound 41-5 (113 mg, containing 1.3wt% ethyl acetate, conversion yield 22%) as a pale-yellow amorphous.

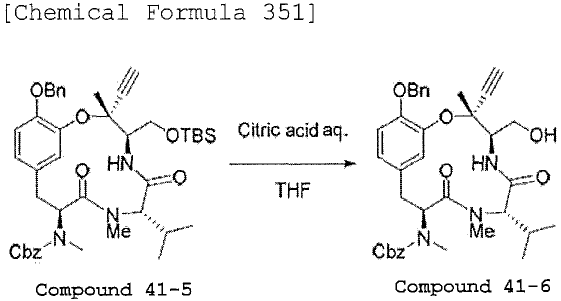

Under nitrogen atmosphere, to a solution of Compound 41-5 (1.0 eq., 0.178 mmol) in a mixture of THF (1 mL)/water (0.5 mL) was added citric acid (10.0 eq., 343 mg, 1.79 mmol), and the mixture was stirred at room temperature for 15.5 hr, and then at the external temperature of 40°C for 4.5 hr. The reaction solution was allowed to cool, ethyl acetate (3 mL) was added, and the mixture was washed once with water (3 mL), once with 5% aqueous sodium hydrogencarbonate solution (3 mL) and once with 5% aqueous sodium chloride solution (3 mL) by liquid separation, and dried over sodium sulfate. The sodium sulfate was removed by filtration, the filtrate was concentrated under reduced pressure, and the obtained crude product (136 mg) was purified by flash silica gel column (normal phase silica gel 10 g, hexane/ethyl acetate = 35/65) to give Compound 41-6 (92 mg, containing 2.1wt% ethyl acetate, conversion yield 78%) as a white amorphous.

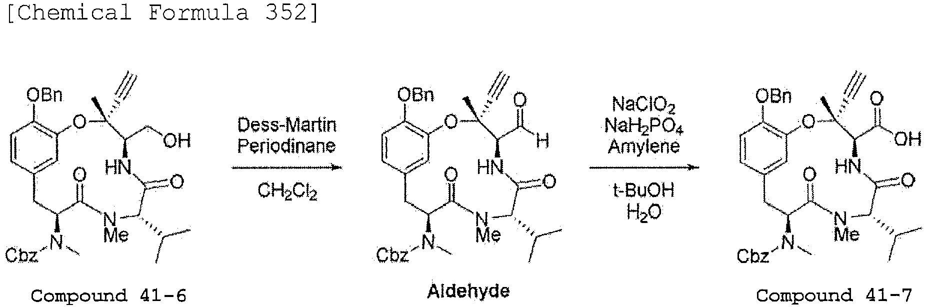

Under nitrogen atmosphere, to a solution of Compound 41-6 (1.0 eq., 92 mg, containing 2.1wt% ethyl acetate, 0.14 mmol) in dichloromethane (1 mL) was added Dess-Martin periodinane (1.5 eq., 89 mg, 0.21 mmol) under ice-cooling, and the mixture was stirred for 3 hr, and then stirred at room temperature for additional 14 hr. Dess-Martin periodinane (1.5 eq., 89 mg, 0.21 mmol) was added at room temperature, and the mixture was stirred at the same temperature for additional 2.5 hr. The reaction solution was ice-cooled, 5% aqueous sodium hydrogencarbonate solution (3 mL) and 5% aqueous sodium thiosulfate solution (3 mL) were added, and the mixture was extracted once with ethyl acetate (6 mL) by liquid separation. The organic layer was washed once with 5% aqueous sodium chloride solution (6 mL) by liquid separation, and dried over sodium sulfate. The sodium sulfate was removed by filtration, and the filtrate was concentrated under reduced pressure to give Aldehyde (102 mg) as a yellow viscous product.

Under nitrogen atmosphere, to a solution of Aldehyde (1.0 eq., 102 mg, calculated as 0.14 mmol) in a mixture of t-butyl alcohol (2 mL)/amylene (0.5 mL)/water (0.5 mL) were added sodium dihydrogen phosphate dihydrate (3.5 eq., 77 mg, 0.49 mmol) and 80% sodium chlorite (4.5 eq., 71 mg, 0.63 mmol) at room temperature, and the mixture was stirred at the same temperature for 1.5 hr. To the reaction solution was added ethyl acetate (6 mL), and the mixture was washed twice with 5% aqueous sodium chloride solution (6 mL) by liquid separation, and dried over sodium sulfate. The sodium sulfate was removed by filtration, the filtrate was concentrated under reduced pressure, and the obtained crude product (114 mg) was purified by flash silica gel column (normal phase silica gel 10 g, hexane/ethyl acetate/methanol = 90/10/0 - 0/100/0 - 0/80/20) to give Compound 41-7 (93 mg, containing 4.3wt% ethyl acetate, two-step conversion yield from Compound 41-6 97%) as a pale-yellow amorphous.

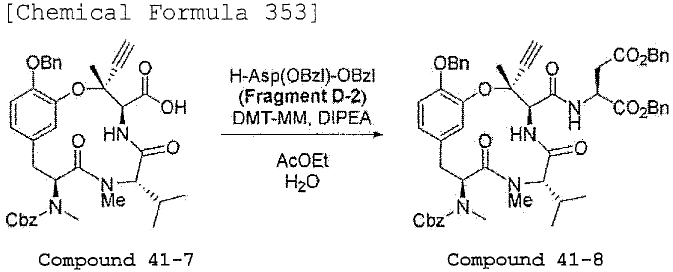

Under nitrogen atmosphere, to a solution of Compound 41-7 (1.0 eq., 20 mg, containing 4.3wt% ethyl acetate, 0.029 mmol) and dibenzyl L-aspartate hydrochloride (Fragment D-2, 1.5 eq., 15 mg, 0.043 mmol) in a mixture of ethyl acetate (200 µL)/water (60 µL) were added DIPEA (2.5 eq., 12.4 µL, 0.073 mmol) and DMT-MM (15.1% hydrous, 1.7 eq., 14 mg, 0.051 mmol) under ice-cooling, and the mixture was stirred for 3 hr, and then at room temperature for additional 14.5 hr. To the reaction solution was added ethyl acetate (1 mL), and the mixture was washed once with 5% aqueous citric acid solution (1 mL), once with 5% aqueous sodium hydrogencarbonate solution (1 mL) and once with 5% aqueous sodium chloride solution (1 mL) by liquid separation, and dried over sodium sulfate. The sodium sulfate was removed by filtration, and the filtrate was concentrated under reduced pressure to give a crude product (25 mg).

The crude product obtained from Compound 41-7 (20 mg) by the same procedure and the aforementioned crude product were combined, and purified by flash silica gel column (normal phase silica gel 10 g, hexane/ethyl acetate = 70/30 - 50/50) to give Compound 41-8 (49 mg, containing 8.9% impurities and 0.4wt% ethyl acetate, conversion yield 80%) as a pale-yellow amorphous.

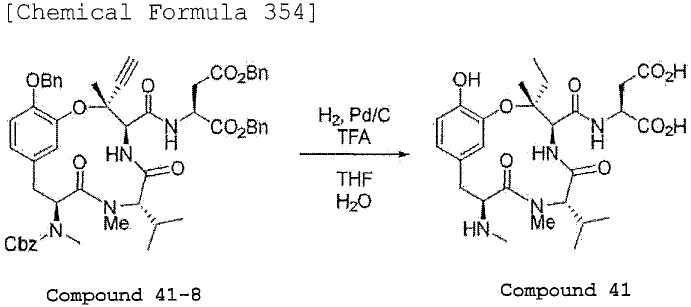

Under nitrogen atmosphere, to a solution of Compound 41-8 (1.0 eq., 26 mg, containing 8.9% impurities and 0.4wt% ethyl acetate, 0.025 mmol) in a mixture of THF (1 mL)/water (50 µL) were added TFA (2.0 eq., 3.8 µL, 0.050 mmol) and 10% palladium-carbon (N.E. CHEMCAT NX type, 50% hydrous product, 2.6 mg) at room temperature, the system was replaced with hydrogen, and the mixture was stirred at the same temperature for 26 hr. The catalyst was removed by filtration through Celite, and the filtrate was concentrated under reduced pressure to give a crude product (34 mg).

The crude product obtained from Compound 41-8 (20 mg) by the same procedure and the aforementioned crude product were combined, purified by flash silica gel column (reverse-phase silica gel 30 g, 0.05% aqueous TFA solution/acetonitrile = 95/5 - 90/10), and freeze-dried to give a TFA salt of Compound 41 (23.4 mg, yield 80%, purity 97.7%) as a white solid.

¹H-NMR(400MHz,D₂O)δ6.94-6.87(m,2H),6.58(d,J=1.6Hz,1H),4.93-4.90(m,1H),4.72(d,J=11.2Hz,1H),4.68-4.65(m,2H),3.31(dd,J=14.0,4.4Hz,1H),2.99-2.84(m,3H),2.74(s,3H),2.70(s,3H),2.22-2.12(m,1H),2.02-1.93(m,1H),1.83-1.73(m,1H),1.51(s,3H),1.07(t,J=7.2Hz,3H),0.82(t,J=7.2Hz,6H).

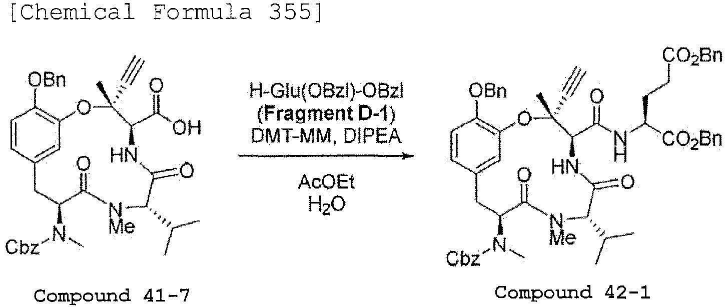

Under nitrogen atmosphere, to a solution of Compound 41-7 (1.0 eq., 51 mg, containing 4.3wt% ethyl acetate, 0.074 mmol) and dibenzyl L-glutamate hydrochloride (Fragment D-1, 1.5 eq., 41 mg, 0.113 mmol) in a mixture of ethyl acetate (500 µL)/water (150 µL) were added DIPEA (2.5 eq., 32 µL, 0.188 mmol) and DMT-MM (15.1% hydrous, 1.7 eq., 41 mg, 0.126 mmol) under ice-cooling, and the mixture was stirred for 3 hr, and then stirred at room temperature for additional 20 hr. To the reaction solution was added ethyl acetate (2 mL), and the mixture was washed once with 5% aqueous citric acid solution (2 mL), once with 5% aqueous sodium hydrogencarbonate solution (2 mL) and once with 5% aqueous sodium chloride solution (2 mL) by liquid separation, and dried over sodium sulfate. The sodium sulfate was removed by filtration, the filtrate was concentrated under reduced pressure, and the obtained crude product (94 mg) was purified by flash silica gel column (normal phase silica gel 10 g, hexane/ethyl acetate = 70/30 - 50/50) to give Compound 42-1 (76 mg, containing 13.4 % impurities, conversion yield 92%) as a pale-yellow amorphous.

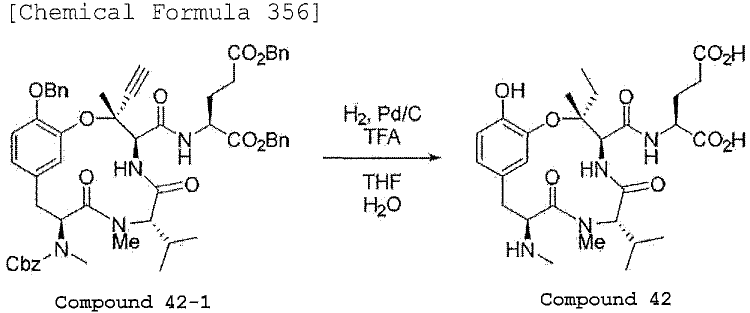

Under nitrogen atmosphere, to a solution of Compound 42-1 (1.0 eq., 64 mg, containing 13.4 % impurities, 0.057 mmol) in a mixture of THF (2 mL)/water (100 µL) were added TFA (2.0 eq., 8.8 µL, 0.115 mmol) and 10% palladium-carbon (N.E. CHEMCAT NX type, 50% hydrous product, 6.4 mg) at room temperature, the system was replaced with hydrogen, and the mixture was stirred at the same temperature for 19.5 hr. 10% Palladium-carbon (N.E. CHEMCAT NX type, 50% hydrous product, 6.4 mg) was added, and the mixture was stirred at the same temperature for additional 4.5 hr. The catalyst was removed by filtration through Celite, the filtrate was concentrated under reduced pressure, and the obtained crude product (42 mg) was purified by flash silica gel column (reverse-phase silica gel 30 g, 0.05% aqueous TFA solution/acetonitrile = 95/5 - 90/10), and freeze-dried to give a TFA salt of Compound 42 (21.4 mg, yield 55%, purity 98.6%) as a white solid.

¹H-NMR(400MHz,D₂O)δ6.94-6.87(m,2H),6.59(s,1H),4.92-4.90(m,1H),4.73(d,J=11.6Hz,1H),4.67(dd,J=8.0,4.8Hz,1H),4.40(dd, J=9.2,4.8Hz,1H),3.32(dd,J=14.0,4.8Hz,1H),2.97(dd,J=14.0,8.0Hz,1 H),2.75(s,3H),2.70(s,3H),2.52-2.37(m,2H),2.26-2.12(m,2H),2.05-1.90(m,2H)1.84-1.72(m,1H),1.51(s,3H),1.08(t,J=7.2Hz,3H),0.82(t,J=7.2Hz,6H).

### (Example 29M3: Synthesis of Compounds 43 and 44)

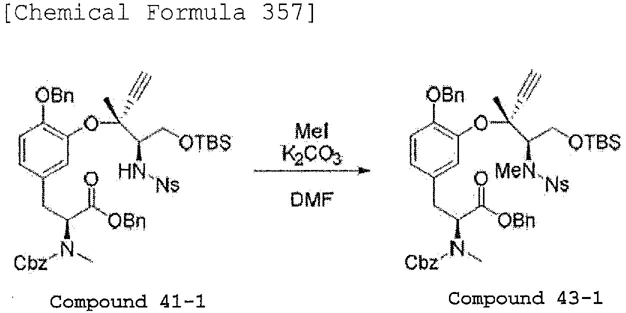

Under nitrogen atmosphere, to a solution of Compound 41-1 (1.0 eq., 2.00 g, containing 2.3wt% ethyl acetate, 2.09 mmol) in DMF (10 mL) were added potassium carbonate (2.0 eq., 0.58 g, 4.20 mmol) and methyl iodide (2.0 eq., 0.59 g, 4.16 mmol) under ice-cooling, and the mixture was stirred at the same temperature for 3 hr, and then stirred at room temperature for 14 hr. To the reaction solution was added toluene (30 mL), and the mixture was washed three times with 5% aqueous sodium chloride solution (30 mL) by liquid separation, and dried over sodium sulfate. The sodium sulfate was removed by filtration, and the filtrate was concentrated under reduced pressure to give Compound 43-1 (2.01 g, containing 3.2wt% ethyl acetate, conversion yield 98%) as a colorless oil.

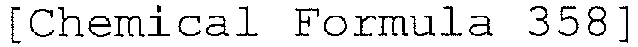

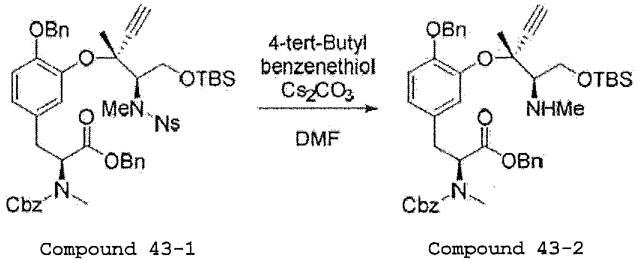

Under nitrogen atmosphere, to a solution of Compound 43-1 (1.0 eq., 2.22 g, 2.26 mmol) in DMF (11 mL) were added cesium carbonate (3.0 eq., 2.22 g, 6.81 mmol) and 4-tert-butylbenzenethiol (3.0 eq., 1.13 g, 6.80 mmol) at room temperature, and the mixture was stirred at the same temperature for 1.5 hr. To the reaction solution was added toluene (30 mL), and the mixture was washed once with water (30 mL) and once with a mixture of 5% aqueous potassium carbonate solution (30 mL)/5% aqueous sodium chloride solution (30 mL) by liquid separation, and dried over sodium sulfate. The sodium sulfate was removed by filtration, the filtrate was concentrated under reduced pressure, and the obtained crude product (3.28 g) was purified by flash silica gel column (normal phase silica gel 25 g, hexane/ethyl acetate = 90/10 - 0/100) to give Compound 43-2 (1.71 g, containing 3.5wt% ethyl acetate, conversion yield 95%) as a pale-yellow oil.

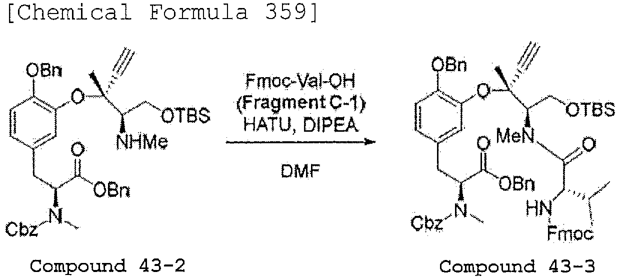

Under nitrogen atmosphere, to a solution of Compound 43-2 (1.0 eq., 0.76 g, containing 3.5wt% ethyl acetate, 0.96 mmol) in DMF (7.5 mL) were added N-Fmoc-L-valine (Fragment C-1, 1.5 eq., 0.49 g, 1.44 mmol), HATU (1.5 eq., 0.55 g, 1.45 mmol) and DIPEA (3.0 eq., 0.37 g, 2.86 mmol), and the mixture was stirred at room temperature for 16 hr. To the reaction solution was added toluene (20 mL), and the mixture was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, the filtrate was concentrated under reduced pressure, and the obtained crude product (1.29 g) was purified by flash silica gel column (normal phase silica gel 10 g, hexane/ethyl acetate = 75/25) to give Compound 43-3 (0.99 g, containing ethyl acetate and impurities, apparent yield 95%) as a pale-orange amorphous.

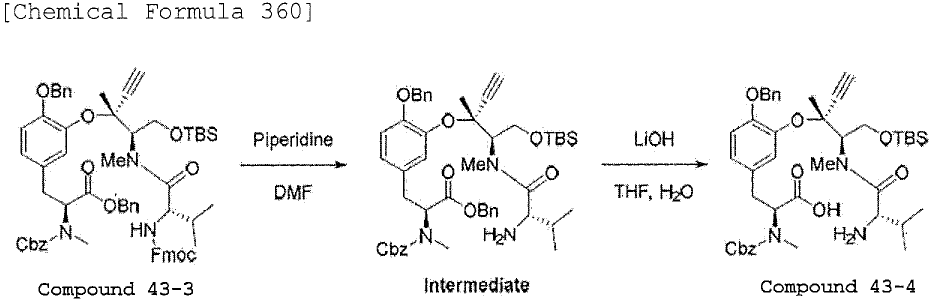

Under nitrogen atmosphere, to a solution of Compound 43-3 (1.0 eq., 0.99 g, containing ethyl acetate and impurities, calculated as 0.91 mmol) in DMF (9 mL) was added piperidine (0.45 mL), and the mixture was stirred at room temperature for 1 hr. To the reaction solution was added toluene (20 mL), and the mixture was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, the filtrate was concentrated under reduced pressure, and the obtained crude product (0.91 g) was purified by flash silica gel column (normal phase silica gel 10 g, hexane/ethyl acetate = 90/10 - 0/100) to give Intermediate (729 mg, containing 3.8wt% ethyl acetate, two-step conversion yield from Compound 43-2 85%) as a yellow viscous product.

Under nitrogen atmosphere, to a solution of Intermediate (1.0 eq., 727 mg, containing 3.8wt% ethyl acetate, 0.81 mmol) in a mixture of THF (4 mL)/water (2 mL) was added lithium hydroxide monohydrate (2.4 eq., 82 mg, 1.94 mmol), and the mixture was stirred at room temperature for 2 hr. The reaction solution was ice-cooled, 5% aqueous citric acid solution (10 mL) was added, and the mixture was extracted once with ethyl acetate (10 mL) by liquid separation. The organic layer was washed twice with 5% aqueous sodium chloride solution (10 mL) by liquid separation, and dried over sodium sulfate. The sodium sulfate was removed by filtration, the filtrate was concentrated under reduced pressure, and the obtained crude product (720 mg) was purified by flash silica gel column (normal phase silica gel 10 g, hexane/ethyl acetate/methanol = 90/10/0 - 0/100/0 - 0/50/50) to give Compound 43-4 (657 mg, containing 3.5wt% ethyl acetate, quant) as a pale-brown amorphous.

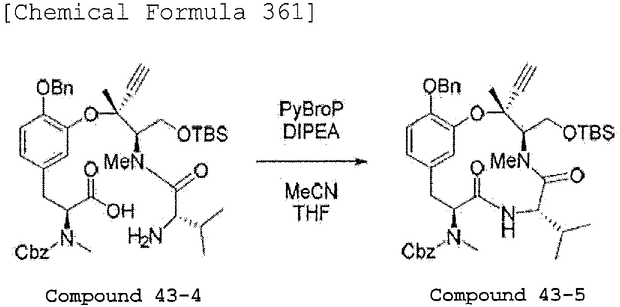

Under nitrogen atmosphere, to a solution of PyBroP (5.0 eq., 1.89 g, 4.05 mmol) in acetonitrile (797 mL) was added DIPEA (10.0 eq., 1.05 g, 8.12 mmol) at room temperature. A solution of Compound 43-4 (1.0 eq., 654 mg, containing 3.5wt% ethyl acetate, 0.81 mmol) in a mixture of acetonitrile (7.5 mL)/THF (7.5 mL) was added over 20 hr at the external temperature of 50°C, and after the dropwise addition was completed, the mixture was stirred at the same temperature for additional 0.5 hr. The reaction solution was concentrated under reduced pressure, to the concentrated residue was added ethyl acetate (10 mL), the mixture was subjected to ultrasonic irradiation, and the insoluble substance was removed by filtration. The filtrate was washed once with 5% aqueous citric acid solution (10 mL), once with 5% aqueous sodium hydrogencarbonate solution (10 mL) and once with 5% aqueous sodium chloride solution (10 mL) by liquid separation, and dried over sodium sulfate. The sodium sulfate was removed by filtration, the filtrate was concentrated under reduced pressure, and the obtained crude product (1.46 g) was purified by flash silica gel column (normal phase silica gel 100 g, hexane/ethyl acetate = 75/25) to give Compound 43-5 (201 mg, containing 1.1wt% ethyl acetate, conversion yield 32%) as a white amorphous.

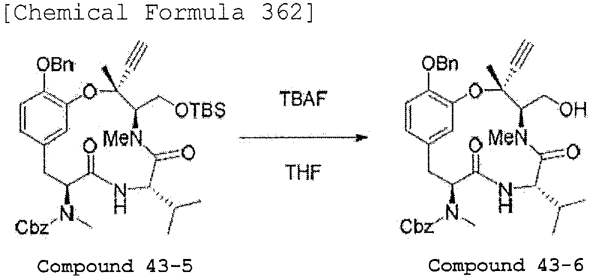

Under nitrogen atmosphere, to a solution of Compound 43-5 (1.0 eq., 189 mg, containing 1.1wt% ethyl acetate, 0.247 mmol) in THF (1.9 mL) was added TBAF (1.1M in THF, 1.1 eq., 0.247 mL, 0.272 mmol) under ice-cooling, and the mixture was stirred at the same temperature for 10 min, and then at room temperature for 1 hr. To the reaction solution was added toluene (10 mL), and the mixture was washed three time with 10% aqueous ammonium chloride solution (10 mL) and once with 5% aqueous sodium chloride solution (10 mL) by liquid separation, and dried over sodium sulfate. The sodium sulfate was removed by filtration, and the filtrate was concentrated under reduced pressure to give a crude product (164 mg).

The crude product (8 mg) obtained from Compound 43-5 (10 mg) by the same procedure and the aforementioned crude product were combined, and purified by flash silica gel column (normal phase silica gel 10 g, hexane/ethyl acetate = 90/10 - 0/100) to give Compound 43-6 (165 mg, containing 4.9wt% ethyl acetate, conversion yield 94%) as a white amorphous.

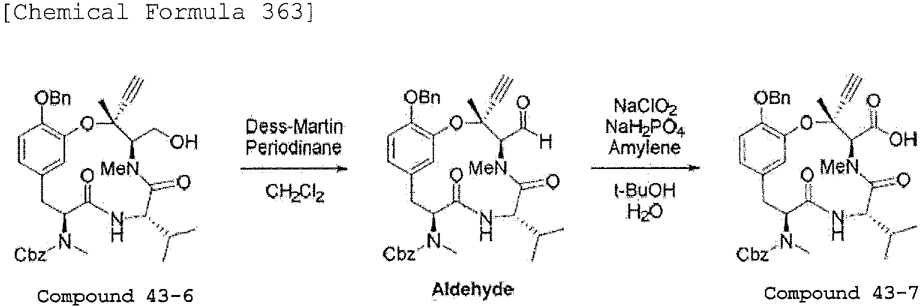

Under nitrogen atmosphere, to a solution of Compound 43-6 (1.0 eq., 71 mg, containing 4.9wt% ethyl acetate, 0.105 mmol) in dichloromethane (0.6 mL) was added Dess-Martin periodinane (1.5 eq., 67 mg, 0.158 mmol) under ice-cooling, and the mixture was stirred at the same temperature for 2.5 hr. Dess-Martin periodinane (0.5 eq., 22 mg, 0.052 mmol) was added, and the mixture was stirred at the same temperature for additional 1 hr. 5% Aqueous sodium hydrogencarbonate solution (2 mL) and 5% aqueous sodium thiosulfate solution (2 mL) were added, and the mixture was extracted once with ethyl acetate (4 mL) by liquid separation. The organic layer was washed once with 5% aqueous sodium chloride solution (4 mL) by liquid separation, and dried over sodium sulfate. The sodium sulfate was removed by filtration, and the filtrate was concentrated under reduced pressure to give Aldehyde (71 mg) as a white amorphous.

Under nitrogen atmosphere, to a solution of Aldehyde (1.0 eq., 69 mg, calculated as 0.102 mmol) in a mixture of t-butyl alcohol (1.2 mL)/amylene (0.3 mL)/water (0.3 mL) were added sodium dihydrogen phosphate dihydrate (3.5 eq., 57 mg, 0.365 mmol) and 80% sodium chlorite (4.5 eq., 53 mg, 0.469 mmol) at room temperature, and the mixture was stirred at the same temperature for 1 hr. To the reaction solution was added ethyl acetate (3 mL), and the mixture was washed twice with 5% aqueous sodium chloride solution (3 mL) by liquid separation, and dried over sodium sulfate. The sodium sulfate was removed by filtration, the filtrate was concentrated under reduced pressure, and the obtained crude product (75 mg) was purified by flash silica gel column (normal phase silica gel 10 g, hexane/ethyl acetate/methanol = 90/10/0 - 0/100/0 - 0/80/20) to give Compound 43-7 (70 mg, containing 4.1wt% ethyl acetate, two-step conversion yield from Compound 43-6 97%) as a pale-yellow amorphous.

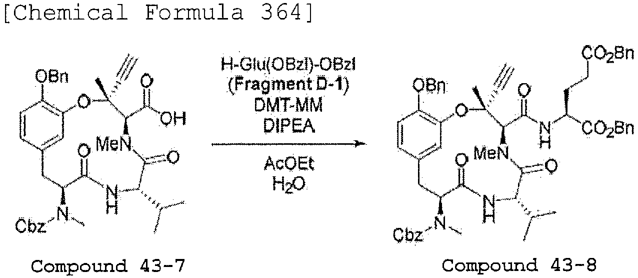

Under nitrogen atmosphere, to a solution of Compound 43-7 (1.0 eq., 63 mg, containing 5.0wt% ethyl acetate, 0.091 mmol) and dibenzyl L-glutamate hydrochloride (Fragment D-1, 1.5 eq., 50 mg, 0.137 mmol) in a mixture of ethyl acetate (630 µL)/water (190 µL) were added DIPEA (2.5 eq., 39 µL, 0.229 mmol) and DMT-MM (15.1% hydrous, 1.7 eq., 51 mg, 0.156 mmol) under ice-cooling, and the mixture was stirred at the same temperature for 1.5 hr. To the reaction solution was added ethyl acetate (2 mL), and the mixture was washed once with 5% aqueous citric acid solution (2 mL), once with 5% aqueous sodium hydrogencarbonate solution (2 mL) and once with 5% aqueous sodium chloride solution (2 mL) by liquid separation, and dried over sodium sulfate. The sodium sulfate was removed by filtration, the filtrate was concentrated under reduced pressure, and the obtained crude product (92 mg) was purified by flash silica gel column (normal phase silica gel 10 g, hexane/ethyl acetate = 70/30 - 50/50) to give Compound 43-8 (64 mg, containing 2.1% impurities and 0.2wt% ethyl acetate, conversion yield 71%) as a white amorphous.

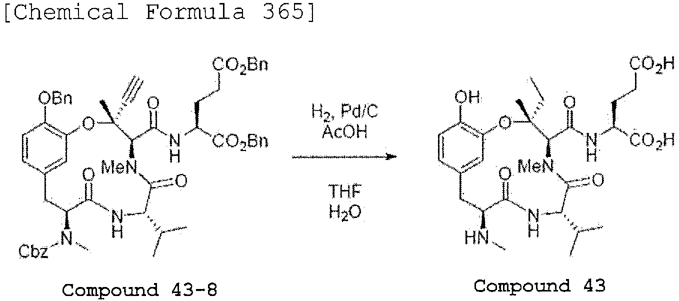

Under nitrogen atmosphere, to a solution of Compound 43-8 (1.0 eq., 56 mg, containing 2.1% impurities and 0.2wt% ethyl acetate, 0.057 mmol) in a mixture of THF (1.6 mL)/water (400 µL) were added acetic acid (5.0 eq., 16.2 µL, 0.283 mmol) and 10% palladium-carbon (N.E. CHEMCAT NX type, 50% hydrous product, 2.8 mg) at room temperature, the system was replaced with hydrogen, and the mixture was stirred at the same temperature for 16.5 hr. Additional 10% palladium-carbon (N.E. CHEMCAT NX type, 50% hydrous product, 2.9 mg) was added, and the mixture was stirred for 3.5 hr. Additional 10% palladium-carbon (N.E. CHEMCAT NX type, 50% hydrous product, 11.2 mg) was added again, and the mixture was stirred for 6 hr. The catalyst was removed by filtration through Celite, the filtrate was concentrated under reduced pressure, and the obtained crude product (27.6 mg) was purified by flash silica gel column (reverse-phase silica gel 30 g, 0.05% aqueous acetic acid solution/acetonitrile = 95/5 - 90/10), and freeze-dried to give Compound 43 (17.2 mg, containing 1.0wt% acetic acid, conversion yield 53%, purity 99.0%) as a white solid.

¹H-NMR(400MHz,D2O)δ6.89(d,J=8.0Hz,1H),6.81(dd,J=8.0,2.0Hz,1H),6.48 (d,J=2.0Hz,1H),5.50(s,1H),4.70(d,J=10.8Hz,1H),4.21(dd,J=9.2,4.8 Hz,1H),4.03(dd,J=7.2,4.0Hz,1H),3.40(s,3H),3.17(dd,J=14.0,4.4Hz, 1H),3.00(dd,J=14.0,7.2Hz,1H),2.71(s,3H),2.31(t,J=7.6Hz,3H),2.15 -1.94(m,3H)1.93-1.78(m,2H),1.61(s,3H),0.98(t,J=7.2Hz,3H),0.94(d,J=6.4Hz,3H),0.8 6(d,J=6.8Hz,3H).

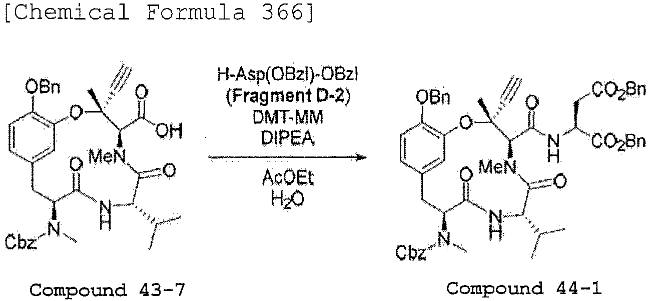

Under nitrogen atmosphere, to a solution of Compound 43-7 (1.0 eq., 67 mg, containing 4.1wt% ethyl acetate, 0.098 mmol) and dibenzyl L-aspartate hydrochloride (Fragment D-2, 1.5 eq., 51 mg, 0.146 mmol) in a mixture of ethyl acetate (670 µL)/water (200 µL) were added DIPEA (2.5 eq., 42 µL, 0.247 mmol) and DMT-MM (15.1% hydrous, 1.7 eq., 54 mg, 0.166 mmol) under ice-cooling, and the mixture was stirred under ice-cooling for 1.5 hr. To the reaction solution was added ethyl acetate (2 mL), and the mixture was washed once with 5% aqueous citric acid solution (2 mL), once with 5% aqueous sodium hydrogencarbonate solution (2 mL) and once with 5% aqueous sodium chloride solution (2 mL) by liquid separation, and dried over sodium sulfate. The sodium sulfate was removed by filtration, the filtrate was concentrated under reduced pressure, and the obtained crude product (98 mg) was purified by flash silica gel column (normal phase silica gel 10 g, hexane/ethyl acetate = 70/30 - 50/50) to give Compound 44-1 (71 mg, containing 1.4% impurities and 0.7wt% ethyl acetate, conversion yield 75%) as a white amorphous.

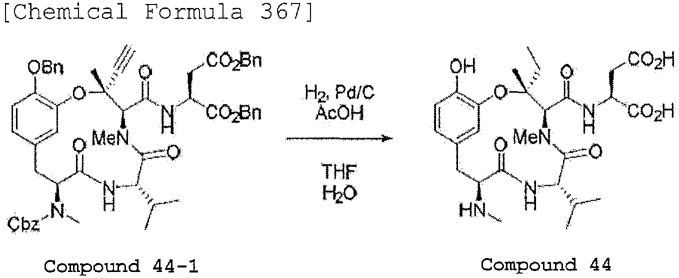

Under nitrogen atmosphere, to a solution of Compound 44-1 (1.0 eq., 68 mg, containing 1.4% impurities and 0.7wt% ethyl acetate, 0.070 mmol) in a mixture of THF (2 mL)/water (500 µL) were added acetic acid (5.0 eq., 20 µL, 0.350 mmol) and 10% palladium-carbon (N.E. CHEMCAT NX type, 50% hydrous product, 6.8 mg) at room temperature, the system was replaced with hydrogen, and the mixture was stirred at the same temperature for 16 hr. The catalyst was removed by filtration through Celite, the filtrate was concentrated under reduced pressure, and the obtained crude product (49 mg) was purified by flash silica gel column (reverse-phase silica gel 30 g, 0.05% aqueous acetic acid solution/acetonitrile = 95/5 - 90/10), and freeze-dried to give Compound 44 (32.6 mg, containing 1.2wt% acetic acid, conversion yield 83%, purity 97.4%) as a white solid.

¹H-NMR(400MHz,D₂O)δ6.88(d,J=8.0Hz,1H),6.81(dd,J=8.0,1.6Hz,1H),6.51 (d,J=1.6Hz,1H),5.52(s,1H),4.69(d,J=10.4Hz,1H),4.55(dd,J=8.4,4.4 Hz,1H),4.01(dd,J=7.6,4.4Hz,1H),3.36(s,3H),3.16(dd,J=14.0,4.4Hz, 1H),2.99(dd,J=14.0,7.6Hz,1H),2.83(dd,J=16.8,4.0Hz,1H),2.71(s,3H ),2.69(dd,J=16.8,8.4Hz,1H),2.09-1.94(m,2H),1.93-1.80(m,1H),1.60(s,3H),1.00(t,J=7.2Hz,3H),0.93(d,J=6.4Hz,3H),0.8 4(d,J=6.8Hz,3H).

### (Example 29M4: Synthesis of Compound 45)

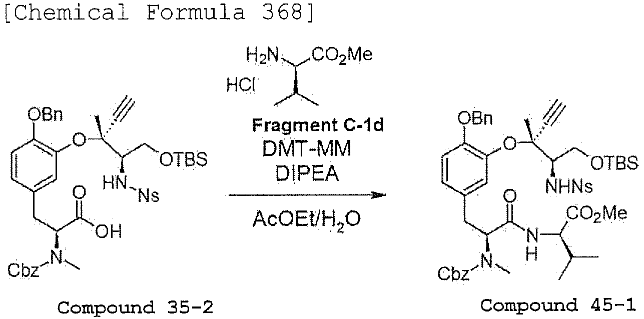

To a solution of Compound 35-2 (1.0 eq., 3.00 g, 3.55 mmol) and D-Val-OMe hydrochloride (Fragment C-1d, 1.5 eq., 0.89 g, 5.3 mmol) in a mixture of ethyl acetate (30 mL)/water (9.0 mL) was added DIPEA (1.7 eq., 1.0 mL, 5.9 mmol) at room temperature. DMT-MM (15.1% hydrous, 1.7 eq., 1.96 g, 6.01 mmol) was added at room temperature, and the mixture was stirred at the same temperature for 1 hr. The organic layer of the reaction solution was recovered, and the organic layer was washed once with 10% aqueous citric acid solution (15 mL), once with 10% aqueous sodium chloride solution (15 mL), once with 5% aqueous sodium hydrogencarbonate solution (15 mL) and once with 10% aqueous sodium chloride solution (15 mL) by liquid separation. The organic layer was dried over sodium sulfate. The sodium sulfate was removed by filtration, and the filtrate was concentrated under reduced pressure to give Compound 45-1 (3.50 g) as a yellow viscous product.

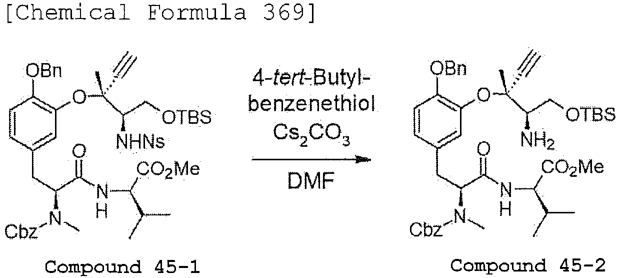

Under nitrogen atmosphere, to a solution of Compound 45-1 (1.0 eq., 3.50 g, calculated as 3.55 mmol) in DMF (8.5 mL) were added 4-tert-butyl-benzenethiol (3.0 eq., 1.77 g, 10.6 mmol) and cesium carbonate (3.0 eq., 3.47 g, 10.7 mmol) at room temperature, and the mixture was stirred at the same temperature for 1 hr. Ethyl acetate (17.0 mL) and water (25.5 mL) were added, and the mixture was extracted once by liquid separation. The organic layer was washed twice with 10% aqueous sodium chloride solution (17.0 mL) by liquid separation, and dried over sodium sulfate. The sodium sulfate was removed by filtration, and the filtrate was concentrated under reduced pressure to give a crude product. The crude product was purified by flash silica gel column (normal phase silica gel 34 g, n-heptane/ethyl acetate = 4/1 - 1/1) to give Compound 45-2 (2.68 g, two-step yield from Compound 35-2 97.7%) as a yellow liquid.

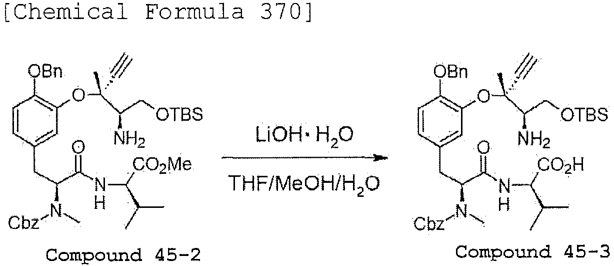

Under nitrogen atmosphere, to a solution of Compound 45-2 (1.0 eq., 2.68 g, 3.46 mmol) in a mixture of THF (10.7 mL)/methanol (5.36 mL)/water (5.36 mL) was added lithium hydroxide monohydrate (2.5 eq., 0.363 g, 8.65 mmol) at room temperature. The mixture was stirred at the same temperature for 1 hr, ethyl acetate (13.4 mL) and water (8.0 mL) were added, the mixture was extracted once by liquid separation, and the organic layer was washed twice with 10% aqueous sodium chloride solution (13.4 mL) by liquid separation. The organic layer was dried over sodium sulfate, the sodium sulfate was removed by filtration, and the filtrate was concentrated under reduced pressure to give Compound 45-3 (2.44 g, yield 92.7%) as a white solid.

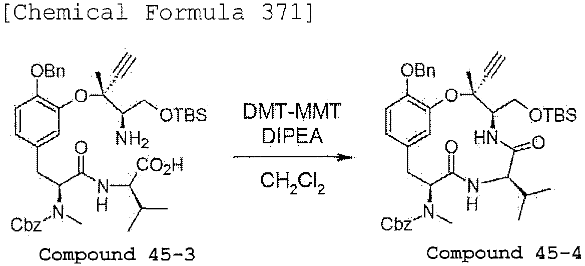

Under nitrogen atmosphere, to a solution of DMT-MMT (2.0 eq., 0.88 g, 2.67 mmol) and DIPEA (2.0 eq., 453 µL, 2.66 mmol) in dichloromethane (101.25 mL) was added dropwise a solution of Compound 45-3 (1.0 eq., 1.0125 g, 1.33 mmol) in dichloromethane (32.4 mL) over 19 hr at room temperature, and the mixture was stirred at the same temperature for 2 hr. The mixture was concentrated under reduced pressure at 35°C to 5.0wt% or less, to the concentrated residue was added ethyl acetate (10 mL), and the mixture was washed once with 10% aqueous citric acid solution (5.0 mL), once with 10% aqueous sodium chloride solution (5.0 mL), once with 5% aqueous sodium hydrogencarbonate solution (5.0 mL) and once with 10% aqueous sodium chloride solution (5.0 mL) by liquid separation. The organic layer was dried over sodium sulfate, the sodium sulfate was removed by filtration, and the filtrate was concentrated under reduced pressure to give a crude product (1.14 g). The crude product was purified twice by flash silica gel column (1st run: normal phase silica gel 41.50 g, n-heptane/ethyl acetate = 5/1 - 3/1, 2nd run: NH₂ silica gel 17.73 g, n-heptane/ethyl acetate = 2/1) to give Compound 45-4 (187.94 mg, yield 19.0%) as a colorless oil.

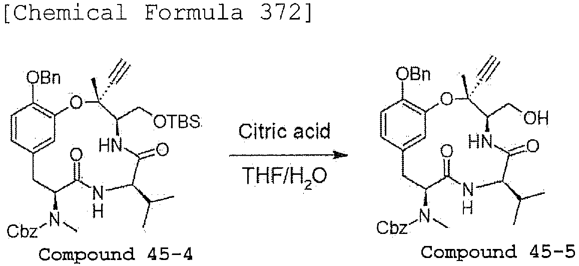

Under nitrogen atmosphere, to a solution of Compound 45-4 (1.0 eq., 187.90 mg, 0.253 mmol) in a mixture of THF (1.69 mL)/water (0.56 mL) was added citric acid (10.0 eq., 486.5 mg, 2.53 mmol) at room temperature, and the mixture was stirred at 35°C for 16 hr. To the reaction solution were added ethyl acetate (4 mL) and water (3.8 mL), and the mixture was extracted once by liquid separation. The organic layer was washed once with water (3.8 mL) by liquid separation, and then washed once with 5% aqueous sodium hydrogencarbonate solution (3.8 mL) by liquid separation until the pH of the aqueous layer reached 8 or higher. The organic layer was washed once with 10% aqueous sodium chloride solution (3.8 mL) by liquid separation, each aqueous layer was extracted again with ethyl acetate by liquid separation. The organic layers were combined, and dried over sodium sulfate, the sodium sulfate was removed by filtration, and the filtrate was concentrated under reduced pressure to give a crude product (206.2 mg). The crude product was purified by flash silica gel column (normal phase silica gel 3.8 g, n-heptane/ethyl acetate = 4/1 - 2/3) to give Compound 45-5 (174.7 mg) as a colorless liquid.

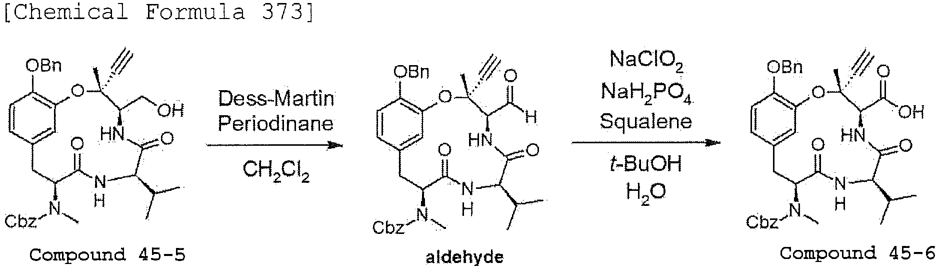

Under nitrogen atmosphere, to a solution of Compound 45-5 (1.0 eq., 109.9 mg, calculated as 0.16 mmol) in dichloromethane (2.0 mL) was added Dess-Martin periodinane (1.5 eq., 0.1013 g, 0.24 mmol) under ice-cooling, and the mixture was stirred at the same temperature for 3 hr, and then stirred at room temperature for 13 hr. 5% Aqueous sodium hydrogencarbonate solution (2.0 mL) and 10% aqueous sodium sulfite solution (2.0 mL) were added under ice-cooling, and the mixture was stirred for 30 min. The organic layer of the reaction solution was recovered, and the organic layer was washed twice with 5% aqueous sodium hydrogencarbonate solution (4 mL) and once with 10% aqueous sodium chloride solution (4 mL) by liquid separation. The organic layer was dried over sodium sulfate, the sodium sulfate was removed by filtration, and the filtrate was concentrated under reduced pressure to give Aldehyde (113.7 mg) as a brown solid.

To a solution of Aldehyde (1.0 eq., 113.7 mg, calculated as 0.16 mmol) in a mixture of t-butyl alcohol (1.495 mL)/squalene (0.099 mL)/water (0.399 mL) was added sodium dihydrogen phosphate dihydrate (3.5 eq., 87.0 mg, 0.56 mmol) at room temperature, and then 80% sodium chlorite (4.5 eq., 81.0 mg, 0.89 mmol) was added, and the mixture was stirred at the same temperature for 1 hr. To the reaction solution were added ethyl acetate (4 mL) and water (3 mL), and the mixture was extracted once by liquid separation. The organic layer was washed twice with 10% aqueous sodium chloride solution (4 mL) by liquid separation, the obtained organic layer was dried over sodium sulfate, the sodium sulfate was removed by filtration, and the filtrate was concentrated under reduced pressure to give a crude product (180.8 mg). The crude product was purified by flash silica gel column (normal phase silica gel 2 g, chloroform-methyl acetate -ethyl acetate/methanol=2/1) to give Compound 45-6 (108.4 mg) as a pale-yellow liquid.

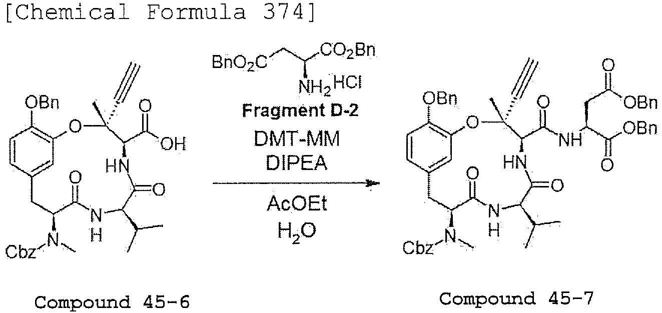

To a solution of Compound 45-6 (1.0 eq., 108.4 mg, calculated as 0.16 mmol) and dibenzyl L-aspartate hydrochloride (Fragment D-2, 1.5 eq., 83.6 mg, 0.24 mmol) in ethyl acetate (1022 µL) was added DIPEA (2.5 eq., 51.5 mg, 0.40 mmol) at room temperature, and DMT-MM (14.6% hydrous, 1.7 eq., 87.7 mg, 0.27 mmol) and water (3307 µL) were added under ice-cooling, and the mixture was stirred at the same temperature for 6 hr. The organic layer of the reaction solution was recovered, to the organic layer were added 10% aqueous sodium chloride solution (2 mL) and 10% aqueous citric acid solution (4 mL), and the mixture was washed once by liquid separation. Then, the organic layer was washed once with 10% aqueous sodium chloride solution (4 mL), once with 5% aqueous sodium hydrogencarbonate solution (4 mL) and once with 10% aqueous sodium chloride solution (4 mL) by liquid separation. The organic layer was dried over sodium sulfate, the sodium sulfate was removed by filtration, and the filtrate was concentrated under reduced pressure to give a crude product (152.2 mg). The crude product was purified by flash silica gel column (normal phase silica gel 4.0 g, n-heptane/ethyl acetate = 3/1 - 2/1) to give Compound 45-7 (132.4 mg, three-step yield from Compound 45-4 88.7%,) as a yellow liquid.

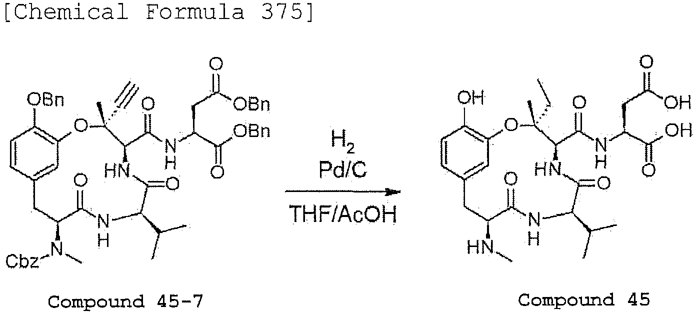

Under nitrogen atmosphere, to a solution of Compound 45-7 (1.0 eq., 186.3 mg, 0.20 mmol) in a mixture of THF (1863 µL)/acetic acid (1863 µL) was added 10% palladium-carbon (N.E. CHEMCAT NX type, 50% hydrous product, 19.82 mg) at room temperature, the system was replaced with hydrogen, and the mixture was stirred at room temperature for 22 hr. The catalyst was removed by filtration through Celite, and the filtrate was concentrated under reduced pressure to give a crude product (107.1 mg). The crude product was purified by flash silica gel column (reverse-phase silica gel 30 g, 0.05% aqueous acetic acid solution/acetonitrile = 95/5 - 90/10), and freeze-dried to give Compound 45 (43.7 mg, yield 40.4%, purity 98.9%) as a white solid.

¹H-NMR(400MHz,D₂O)δ7.00-6.89(m,3H),4.73(s,1H),4.55-4.51(m,1H),4.08(d,J=6.4Hz,1H),4.02(dd,J=11.6,4.0Hz,1H),3.20(dd, J=12.8,4.0Hz,1H),2.99(t,J=12.4Hz,1H),2.86-2.76(m,2H),2.71(s,3H),2.27-2.15(m,1H),2.06-1.95(m,1H),1.79-1.65(m,1H),1.62(s,3H),1.09(t,J=7.2Hz,3H),0.82(d,J=6.8Hz,6H).

### (Example 29L: Synthesis of Compound 47)

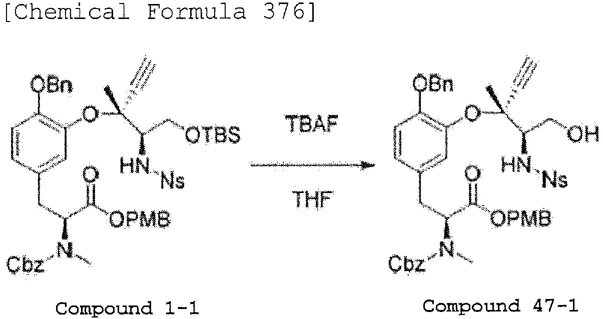

Under nitrogen atmosphere, to a solution of Compound 1-1 (1.0 eq., 1.69 g, calculated as 1.49 mmol) in THF (15 mL) was added TBAF (1.1M in THF, 1.2 eq., 1.62 mL, 1.78 mmol) under ice-cooling, and the mixture was stirred at the same temperature for 2 hr. To the reaction solution was added toluene (30 mL), and the mixture was washed three time with 10% aqueous ammonium chloride solution (30 mL) and once with 5% aqueous sodium chloride solution (30 mL) by liquid separation, and dried over sodium sulfate. The sodium sulfate was removed by filtration, the filtrate was concentrated under reduced pressure, and the obtained crude product (1.64 g) was purified by flash silica gel column (normal phase silica gel 10 g, hexane/ethyl acetate = 70/30 - 0/100) to give Compound 47-1 (1.19 g) as a white amorphous.

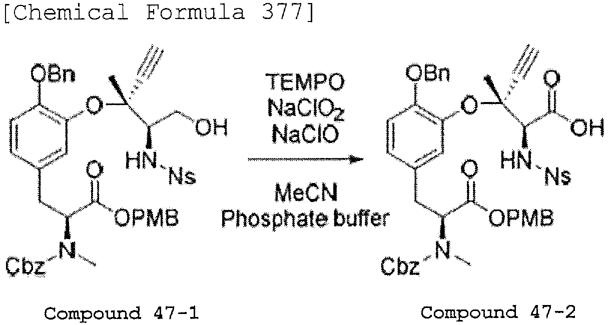

Under nitrogen atmosphere, to a solution of Compound 47-1 (1.0 eq., 1.19 g, 1.37 mmol) in a mixture of acetonitrile (8.7 mL)/0.67M phosphate buffer solution (pH6.8, 7.3 mL) were added TEMPO (0.1 eq., 22 mg, 0.14 mmol), 80% sodium chlorite (2.0 eq., 0.31 g, 2.74 mmol) and sodium hypochlorite pentahydrate (0.02 eq., 5 mg, 0.030 mmol), and the mixture was stirred at the external temperature of 30°C for 16.5 hr. To the reaction solution was added ethyl acetate (25 mL), and the mixture was washed twice with 5% aqueous sodium chloride solution (25 mL) by liquid separation, and dried over sodium sulfate. The sodium sulfate was removed by filtration, the filtrate was concentrated under reduced pressure to give a crude product (1.21 g). The crude product was purified by flash silica gel column (normal phase silica gel 25 g, hexane/ethyl acetate/methanol = 90/10/0 - 0/100/0 - 0/80/20) to give Compound 47-2 (1.18 g, conversion yield 97%) as a white amorphous.

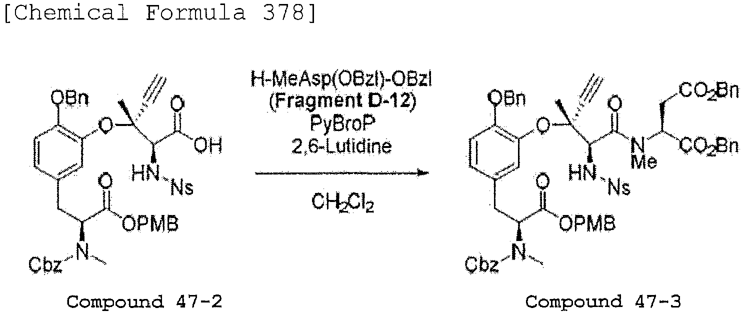

Under nitrogen atmosphere, to a solution of Compound 47-2 (1.0 eq., 1.45 mmol) and Fragment D-12 (1.5 eq., 0.71 g, 2.17 mmol) in dichloromethane (5 mL) were added PyBroP (1.5 eq., 1.01 g, 2.17 mmol) and 2,6-lutidine (3.0 eq., 0.51 mL, 4.38 mmol) under ice-cooling, and the mixture was stirred at the same temperature for 1.5 hr, and then stirred for 20 hr while warming to room temperature. To the reaction solution was added ethyl acetate (20 mL), and the mixture was washed once with 5% aqueous citric acid solution (20 mL), once with 5% aqueous sodium hydrogencarbonate solution (20 mL) and once with 5% aqueous sodium chloride solution (20 mL) by liquid separation, and dried over sodium sulfate. The sodium sulfate was removed by filtration, and the filtrate was concentrated under reduced pressure to give a crude product (2.54 g).

The crude product (84 mg) obtained from Compound 47-2 (50 mg) by the same procedure and the aforementioned crude product were combined, and purified twice by flash column silica gel (1st run: normal phase silica gel 25 g, hexane/ethyl acetate = 70/30 - 50/50, 2nd run: NH₂ silica gel 16 g, hexane/ethyl acetate = 50/50 - 0/100) to give Compound 47-3 (1.53 g, conversion yield 85%) as a yellow amorphous.

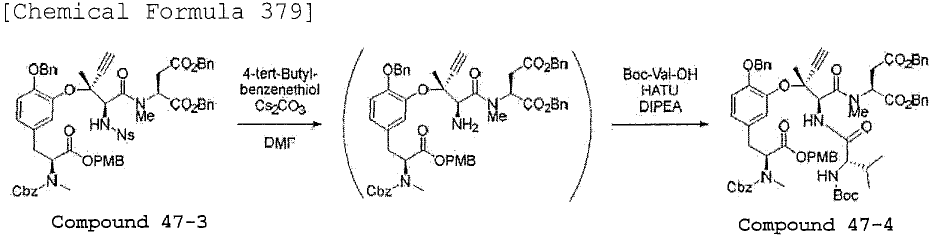

Under nitrogen atmosphere, to a solution of Compound 47-3 (1.0 eq., 500 mg, 0.42 mmol) in DMF (1.5 mL) were added cesium carbonate (1.0 eq., 138 mg, 0.42 mmol) and 4-tert-butylbenzenethiol (1.0 eq., 71 µL, 0.42 mmol) under ice-cooling, and the mixture was stirred at the same temperature for 2 hr. N-Boc-L-valine (1.5 eq., 138 mg, 0.64 mmol), HATU (1.5 eq., 241 mg, 0.63 mmol) and DIPEA (2.5 eq., 180 µL, 1.06 mmol) were added at the same temperature, and the mixture was stirred at the same temperature for 1 hr. To the reaction solution was added toluene (4 mL), and the mixture was washed once with water (4 mL), once with 5% aqueous citric acid solution (4 mL), once with 5% aqueous sodium hydrogencarbonate solution (4 mL) and once with 5% aqueous sodium chloride solution (4 mL) by liquid separation, and dried over sodium sulfate. The sodium sulfate was removed by filtration, and the filtrate was concentrated under reduced pressure to give a crude product (737 mg). The crude product (66 mg) obtained from Compound 47-3 (50 mg) by the same procedure and the aforementioned crude product were combined, and purified by flash silica gel column (NH₂ silica gel 16 g, hexane/ethyl acetate = 90/10 - 0/100) to give Compound 47-4 (273 mg, conversion yield 49%) as a pale-yellow amorphous.

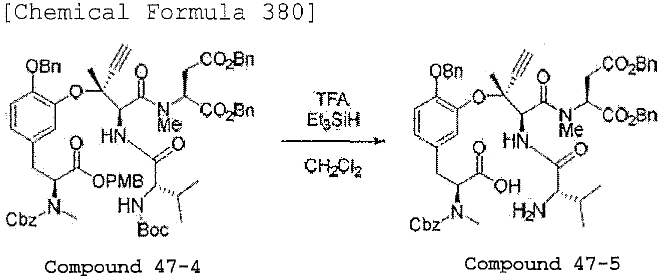

Under nitrogen atmosphere, to a solution of Compound 47-4 (1.0 eq., 249 mg, 0.21 mmol) in dichloromethane (1.75 mL) was added triethylsilane (25 eq., 0.824 mL, 5.17 mmol) at room temperature. TFA (50 eq., 0.791 mL, 10.34 mmol) was added under ice-cooling, and the mixture was stirred at the same temperature for 3.5 hr, and the reaction solution was concentrated under reduced pressure.

The concentrated residue obtained from Compound 47-4 (20 mg) by the same procedure was dissolved in dichloromethane, and the solution was combined with the aforementioned concentrated residue, and concentrated again under reduced pressure to give Compound 47-5 (369 mg) as a yellow viscous product.

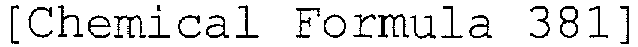

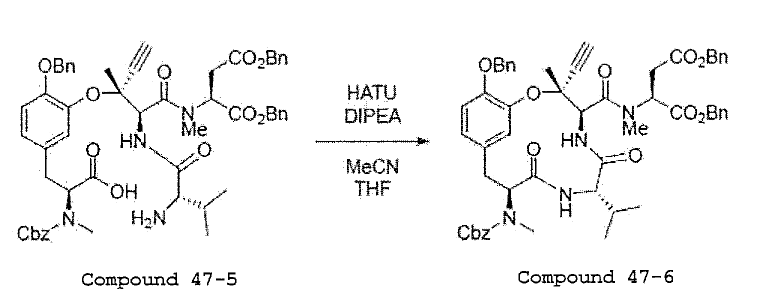

Under nitrogen atmosphere, to a solution of HATU (5.0 eq., 365 mg, 0.96 mmol) in acetonitrile (186 mL) was added DIPEA (10.0 eq., 0.327 mL, 1.92 mmol) at room temperature, and a solution of Compound 47-5 (1.0 eq., 317 mg, calculated as 0.19 mmol) in a mixture of acetonitrile (3 mL)/THF (3 mL) was added over 20 hr at the same temperature, and after the dropwise addition was completed, and the mixture was stirred at the same temperature for additional 2.5 hr. The reaction solution was concentrated under reduced pressure, to the concentrated residue was added ethyl acetate, the mixture was subjected to ultrasonic irradiation, and the insoluble substance was removed by filtration. The filtrate was washed once with 5% aqueous citric acid solution (5 mL), once with 5% aqueous sodium hydrogencarbonate solution (5 mL) and once with 5% aqueous sodium chloride solution (5 mL) by liquid separation, and dried over sodium sulfate. The sodium sulfate was removed by filtration, and the filtrate was concentrated under reduced pressure to give a crude product (378 mg). The crude product (21 mg) obtained from Compound 47-5 (37 mg) by the same procedure and the aforementioned crude product were combined, and purified twice by flash column silica gel (normal phase silica gel 10 g, 1st run: hexane/ethyl acetate = 60/40 - 50/50, 2nd run: chloroform/ethyl acetate = 85/15 - 0/100) to give Compound 47-6 (61 mg, two-step conversion yield from Compound 47-4 30%) as a white amorphous.

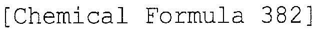

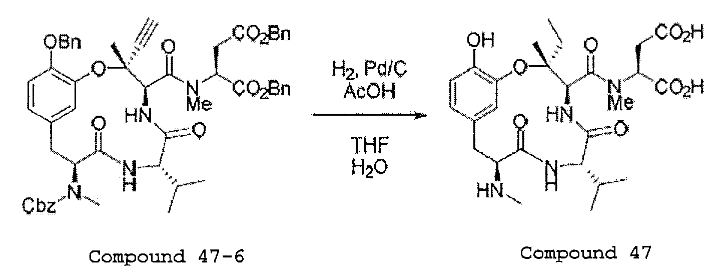

Under nitrogen atmosphere, to a solution of Compound 47-6 (1.0 eq., 56 mg, 0.058 mmol) in a mixture of THF (2 mL)/water (0.1 mL) were added acetic acid (5.0 eq., 17 µL, 0.297 mmol) and 10% palladium-carbon (N.E. CHEMCAT NX type, 50% hydrous product, 5.6 mg) at room temperature, the system was replaced with hydrogen, and the mixture was stirred at the same temperature for 19 hr. Additional 10% palladium-carbon (N.E. CHEMCAT NX type, 50% hydrous product, 5.6 mg) was added, and the mixture was stirred at the same temperature for additional 8 hr. The catalyst was removed by filtration through Celite, the filtrate was concentrated under reduced pressure, and the obtained crude product (35.5 mg) was purified by flash silica gel column (reverse-phase silica gel 30 g, 0.05% aqueous acetic acid solution/acetonitrile = 95/5 - 90/10), and freeze-dried to give Compound 47 (18.7 mg, conversion yield 57%, purity 97.1%) as a white solid.

¹H-NMR(400MHz,D₂O)56.92(d,J=8.0Hz,1H)6.88(dd,J=8.0,1.6Hz,1H)6.73 (s,1H),5.49(s,1H),4.73-4.69(m,1H),4.08(d,J=11.2Hz,1H),3.99(dd,J=10.4,5.6 Hz,1H),3.32-3.24 (m,4H),2.97 (dd, J=16.4,6.0Hz,1H),2.80-2.71(m,1H),2.70-2.62(m,4H),2.08-1.94 (m,1H),1.86-1.72(m,H),1.71-1.55(m,4H),1.06(t,J=7.2Hz,3H),0.82(d,J=6.4Hz,3H),0.70(d,J=6.8Hz ,3H).

### (Example 29M: Synthesis of Compound 48)

Compound 48 was synthesized.

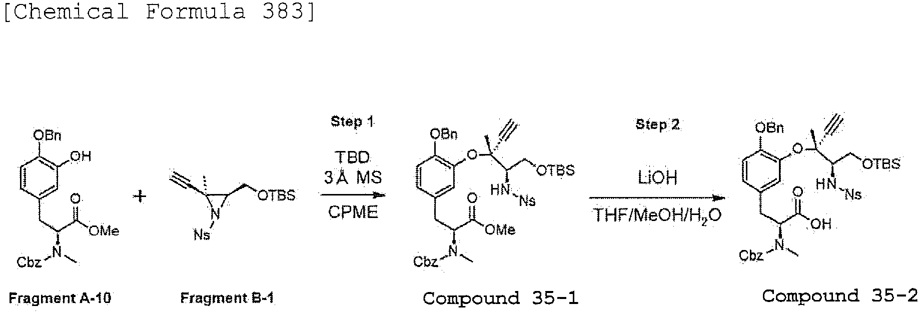

Synthesis of Compound 48-1 from Compound 35-2 was carried out as follows.

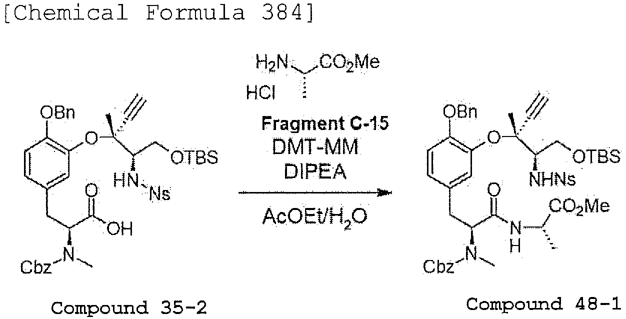

Under nitrogen atmosphere, to a solution of Compound 35-2 (1.0 eq., 1.12 g, 1.32 mmol) in a mixture of ethyl acetate (10 mL)/water (3 mL) were added L-alanine-OMe hydrochloride (1.5 eq., 278 mg, 1.99 mmol) at room temperature, and DIPEA (1.7 eq., 380 µL, 2.23 mmol) and DMT-MM (1.7 eq., 628 mg, 2.27 mmol) under ice-cooling, and the mixture was stirred at room temperature for 2 hr. The mixture was washed once with saturated aqueous sodium hydrogencarbonate solution (20 mL), once with water (20 mL), once with 1N aqueous hydrochloric acid solution (20 mL), once with water (20 mL) and once with saturated aqueous sodium chloride solution (20 mL) by liquid separation. The organic layer was dried over magnesium sulfate, the magnesium sulfate was removed by filtration, and the filtrate was concentrated under reduced pressure to give crude Compound 48-1 (1.24 g, crude yield 100%).

Synthesis of Compound 48-2 from Compound 48-1 was carried out as follows.

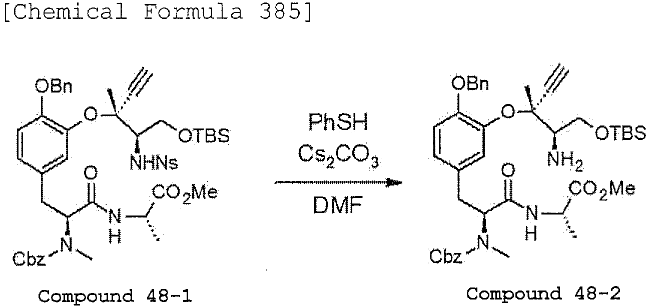

Under nitrogen atmosphere, to a solution of Compound 48-1 (1.0 eq., 1.24 g, 1.33 mmol) in DMF (13 mL) were added cesium carbonate (1.2 eq., 572 mg, 1.62 mmol) and thiophenol (1.2 eq., 160 µL, 1.57 mmol) at room temperature, and the mixture was stirred at room temperature for 3 hr. Water (15 mL) was added, and the mixture was extracted three times with a mixture of hexane (5 mL)/ethyl acetate (15 mL) by liquid separation. The organic layers were combined, and washed with saturated aqueous sodium chloride solution (50 mL) by liquid separation. The organic layer was dried over magnesium sulfate, the magnesium sulfate was removed by filtration, and the filtrate was concentrated under reduced pressure to give a crude product (1.45 g) as a yellow oil. The crude product was purified by flash silica gel column (normal phase silica gel 30 g, hexane/ethyl acetate = 75/25 - 0/100) to give Compound 48-2 (836 mg, yield 84%).

Synthesis of Compound 48-3 from Compound 48-2 was carried out as follows.

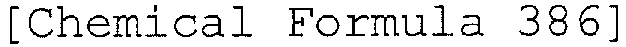

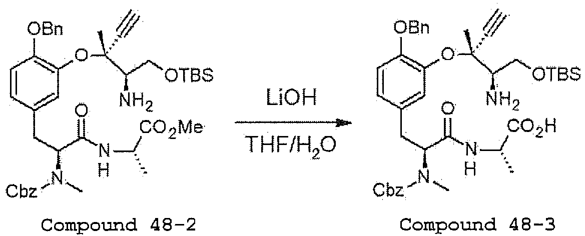

To a solution of Compound 48-2 (1.0 eq., 836 mg, 1.12 mmol) in a mixture of THF (8 mL)/water (4 mL) was added lithium hydroxide (3.0 eq., 81 mg, 3.39 mmol) at room temperature, and the mixture was stirred at room temperature for 1.5 hr. The mixture was adjusted to pH 7 by addition of 1N aqueous hydrochloric acid solution (2.5 mL), water (10 mL) was added, and the mixture was extracted three times with ethyl acetate (10 mL) by liquid separation. The organic layers were combined, and washed once with saturated aqueous sodium chloride solution (25 mL) by liquid separation. The organic layer was dried over magnesium sulfate, the magnesium sulfate was removed by filtration, and the filtrate was concentrated under reduced pressure to give crude Compound 48-3 (833 mg, crude yield 102%).

Synthesis of Compound 48-4 from Compound 48-3 was carried out as follows.

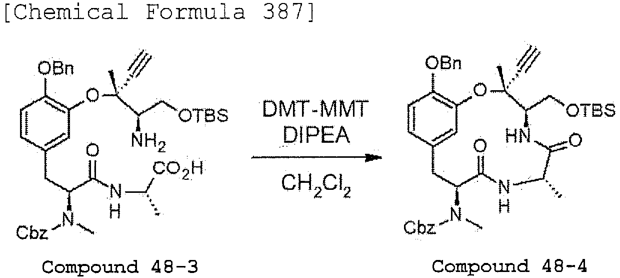

A solution of Compound 48-3 (1.0 eq., 833 mg, 1.12 mmol) in dichloromethane (50 mL) was added dropwise over 16 hr to a solution of DMT-MMT (2.0 eq., 735 mg, 2.24 mmol) and DIPEA (2.0 eq,380 µL, 2.23 mmol) in dichloromethane (83 mL) at about 50 µL/min at room temperature, and the mixture was stirred at room temperature for 5 hr. The reaction solution was concentrated under reduced pressure to evaporate the dichloromethane, ethyl acetate (30 mL) was added, and the mixture was washed once with saturated aqueous sodium hydrogencarbonate solution (30 mL), once with water (30 mL), once with 1N aqueous hydrochloric acid solution (30 mL), once with water (30 mL) and once with saturated aqueous sodium chloride solution (30 mL) by liquid separation. The organic layer was dried over magnesium sulfate, the magnesium sulfate was removed by filtration, and the filtrate was concentrated under reduced pressure to give a crude product (828 mg). The crude product was purified by flash silica gel column (normal phase silica gel 10 g, hexane/ethyl acetate = 75/25 - 65/35) to give Compound 48-4 (345 mg, containing impurities, apparent yield 43%) as a white amorphous.

Synthesis of Compound 48-5 from Compound 48-4 was carried out as follows.

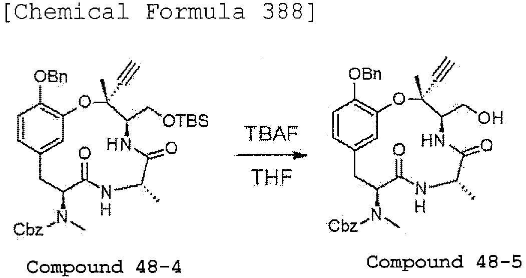

Under nitrogen atmosphere, to a solution of Compound 48-4 (1.0 eq., 345 mg, 483 µmol) in THF (5 mL) was added TBAF (1M in THF, 1.5 eq., 730 µL,730 µmol) under ice-cooling, and the mixture was stirred at room temperature for 1.5 hr. To the reaction solution was added saturated aqueous ammonium chloride solution (10 mL), and the mixture was extracted three times with ethyl acetate (5 mL) by liquid separation. The organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (333 mg). The crude product was purified by flash silica gel column (normal phase silica gel 5 g, hexane/ethyl acetate = 50/50 - 0/100) to give Compound 48-5 (110 mg, yield 38%) as a white solid.

Synthesis of Compound 48-6 from Compound 48-5 was carried out as follows.

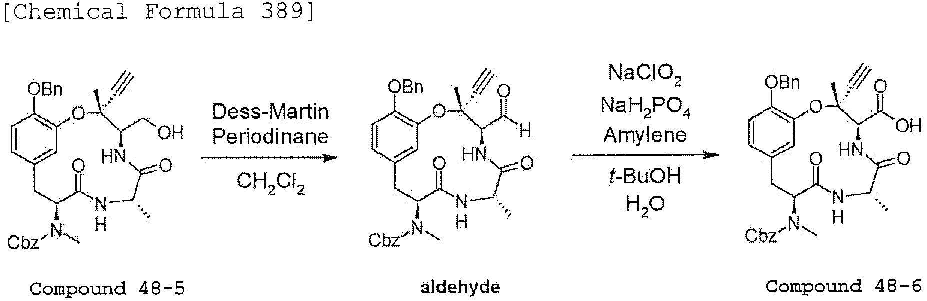

Under nitrogen atmosphere, to a solution of Compound 48-5 (1.0 eq., 110 mg, 183 µmol) in dichloromethane (2 mL) was added Dess-Martin periodinane (1.5 eq., 116 mg, 274 µmol) under ice-cooling, and the mixture was stirred at room temperature for 1 hr. Additional Dess-Martin periodinane (1.0 eq., 79 mg, 185 µmol) was added, and the mixture was stirred for 30 min. 20% Aqueous sodium sulfite solution (5 mL) and saturated aqueous sodium hydrogencarbonate solution (5 mL) were added, and the mixture was extracted three times with dichloromethane (5 mL) by liquid separation. The organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give crude Aldehyde (119 mg, crude yield 109%) as a white amorphous.

To a solution of Aldehyde (1.0 eq., 119 mg, calculated as 183 µmol) in a mixture of t-butyl alcohol (1.2 mL)/amylene (0.4 mL) was added a solution of sodium dihydrogen phosphate dihydrate (3.5 eq., 100 mg, 638 µmol) and 80% sodium chlorite (4.6 eq., 94 mg, 833 µmol) in water (0.4 mL) at room temperature, and the mixture was stirred at room temperature for 30 min. Saturated aqueous ammonium chloride solution (5 mL) was added, and the mixture was extracted three times with ethyl acetate (5 mL) by liquid separation. The organic layers were combined, and dried over magnesium sulfate, the magnesium sulfate was removed by filtration, and the filtrate was concentrated under reduced pressure to give crude Compound 48-6 (121 mg, crude yield 108%) as a white amorphous.

Synthesis of Compound 48-7 from Compound 48-6 was carried out as follows.

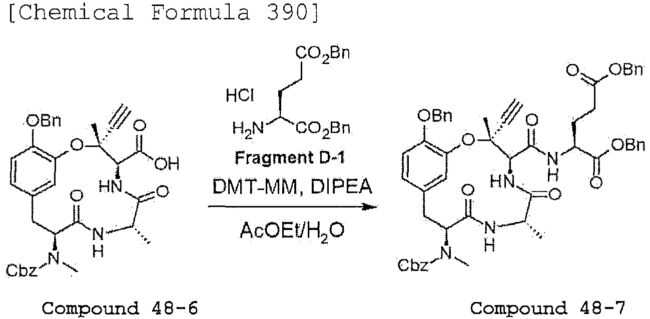

Under nitrogen atmosphere, to a solution of Compound 48-6 (1.0 eq., 121 mg, 183 µmol) in a mixture of ethyl acetate (1.4 mL)/water (0.6 mL) were added DIPEA (2.6 eq., 80 µL, 470 µmol), dibenzyl L-glutamate hydrochloride (Fragment D-1, 1.5 eq., 100 mg, 275 µmol) and DMT-MM (1.8 eq., 89 mg, 321 µmol) under ice-cooling, and the mixture was stirred under ice-cooling for 2 hr. The reaction solution was washed once with saturated aqueous sodium hydrogencarbonate solution (10 mL), once with water (10 mL), once with 1N aqueous hydrochloric acid solution (10 mL), once with water (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (157 mg) as a pale-yellow amorphous. The crude product was purified by flash silica gel column (normal phase silica gel 5 g, hexane/ethyl acetate = 50/50 - 25/75) to give Compound 48-7 (136 mg, three-step yield 80%) as a white amorphous.

Synthesis of Compound 48 from Compound 48-7 was carried out as follows.

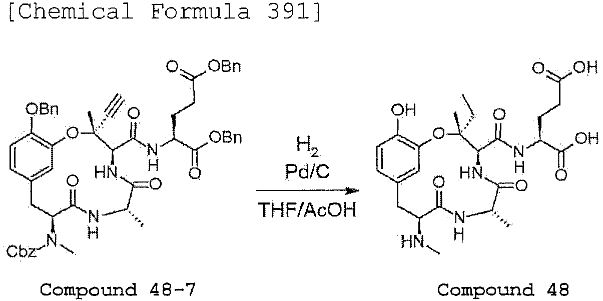

Under nitrogen atmosphere, to a solution of Compound 48-7 (1.0 eq., 136 mg, 147 µmol) in a mixture of THF (1 mL)/acetic acid (1 mL) was added 10% palladium-carbon (N.E. CHEMCAT NX type, 50% hydrous product, 7.1 mg) at room temperature, the system was replaced with hydrogen, and the mixture was stirred at room temperature for 16 hr. The catalyst was removed by filtration through Celite, and the filtrate was concentrated under reduced pressure to give a crude product (106 mg) as an orange amorphous. The crude product was purified twice by flash silica gel column (reverse-phase silica gel 6 g, 1st run: 0.05% aqueous acetic acid solution/acetonitrile = 95/5 - 20/80, 2nd run: 0.05% aqueous acetic acid solution/acetonitrile = 100/0 - 87/13), and freeze-dried to give an acetate of Compound 48 (12.8 mg, yield 17%, purity 96%) as a white solid.

¹H-NMR(400MHz,D₂0)δ7.08(s,1H),6.87-6.80(m,2H),4.56(s,1H),4.36(q,J=7.2Hz,1H),4.18(dd,J=9.0,5.0Hz,1H ),3.84(dd,J=9.7,4.2Hz,1H),3.11(dd,J=13.4,4.2Hz,1H),2.87(dd,J=13 .4,9.7Hz,1H),2.60(s,3H),2.26(t,J=7.8Hz,2H),2.09-1.98(m,1H),1.88-1.78(m,2H),1.66-1.57(m,1H),1.45(s,3H),1.13(d,J=7.2Hz,3H),0.81(t,J=7.4Hz,3H).

### (Example 29N: Synthesis of Compound 49)

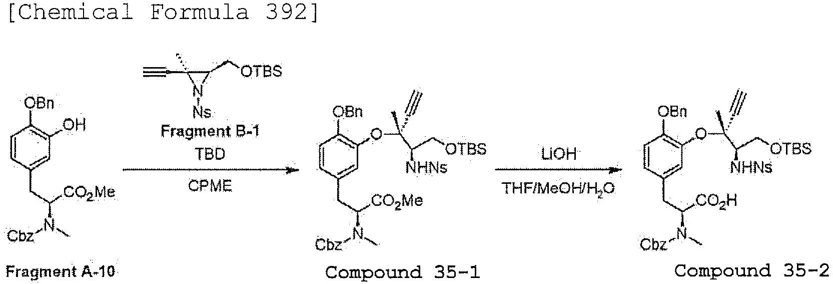

The two steps from Fragment A-10 to Compound 35-2 were carried out as described in Example 29I.

Synthesis of Compound 49-1 from Compound 35-2 was carried out as follows.

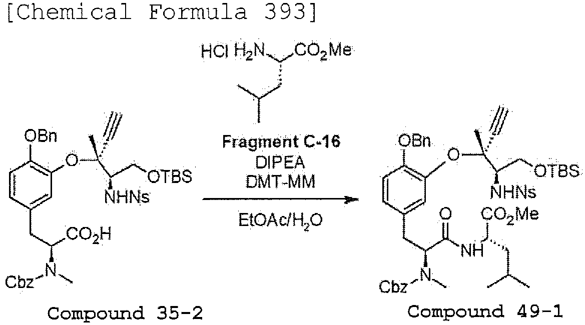

To a solution of Compound 35-2 (1.0 eq., 1.17 g, calculated as 1.39 mmol) in a mixture of ethyl acetate (14 mL)/water (5.2 mL) were added L-leucine-OMe hydrochloride (1.5 eq., 380 mg, 2.09 mmol), DIPEA (1.7 eq., 401 µL, 2.36 mmol) and DMT-MM (1.7 eq., 653 mg, 2.36 mmol) at room temperature, and the mixture was stirred at the same temperature for 3 hr. To the reaction solution was added ethyl acetate (20 mL), and the mixture was washed once with water (10 mL), twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Compound 49-1 (1.40 g, crude yield 104%) as a white amorphous.

Synthesis of Compound 49-2 from Compound 49-1 was carried out as follows.

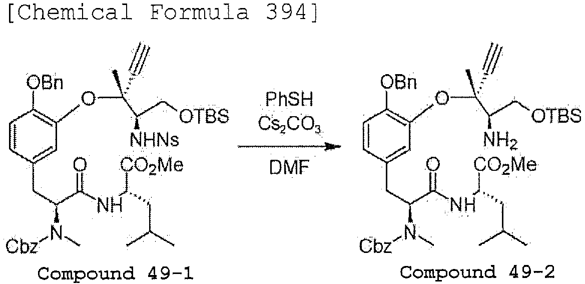

Under nitrogen atmosphere, to a solution of Compound 49-1 (1.0 eq., 1.40 g, calculated as 1.39 mmol) in DMF (14 mL) were added cesium carbonate (3.0 eq., 1.36 g, 4.16 mmol) and thiophenol (3.0 eq., 424 µL, 4.16 mmol) at room temperature, and the mixture was stirred at the same temperature for 1.5 hr. To the reaction solution was added a mixture of hexane (10 mL)/ethyl acetate (30 mL), and the mixture was washed three time with water (20 mL) and once with saturated sodium chloride (10 mL) by liquid separation. The organic layer was dried over magnesium sulfate, the magnesium sulfate was removed by filtration, and the filtrate was concentrated under reduced pressure to give a crude product. The crude product was purified by flash silica gel column (normal phase silica gel 20 g, hexane/ethyl acetate = 67/33 - 33/66) to give Compound 49-2 (953 mg, two-step yield 87%) as a pale-yellow viscous product.

Synthesis of Compound 49-3 from Compound 49-2 was carried out as follows.

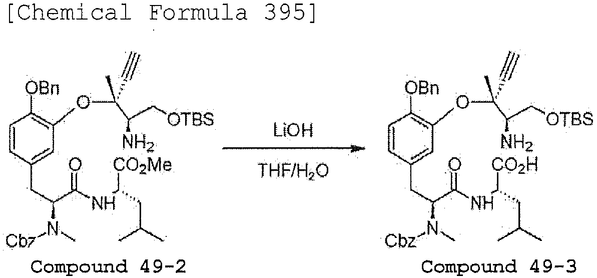

To a solution of Compound 49-2 (1.0 eq., 932 mg, 1.18 mmol) in a mixture of THF (12 mL)/water (4 mL) was added lithium hydroxide (3.0 eq., 86.0 mg, 3.59 mmol) at room temperature, and the mixture was stirred at the same temperature for 2 hr. The reaction solution was adjusted to pH 7 by addition of 1N aqueous hydrochloric acid solution (1.8 mL). To the reaction solution was added ethyl acetate (20 mL), and the mixture was washed once with water (10 mL) by liquid separation. The aqueous layer was extracted once with ethyl acetate (10 mL) by liquid separation, and the organic layers were combined, and washed once with saturated aqueous sodium chloride solution (10 mL) by liquid separation. The organic layer was dried over magnesium sulfate, the magnesium sulfate was removed by filtration, and the filtrate was concentrated under reduced pressure to give Compound 49-3 (921 mg, crude yield 101%) as a white amorphous.

Synthesis of Compound 49-4 from Compound 49-3 was carried out as follows.

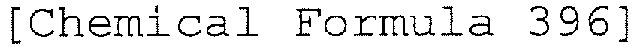

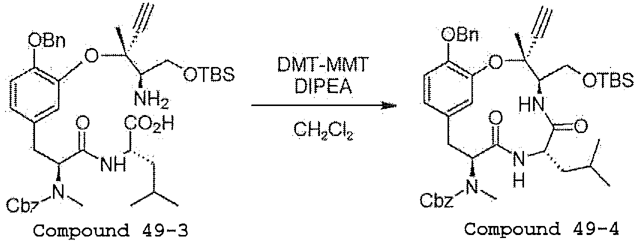

A solution of Compound 49-3 (1.0 eq., 921 mg, 1.18 mmol) in dichloromethane (55 mL) was added dropwise to a solution of DMT-MMT (1.7 eq., 655 mg, 2.00 mmol) and DIPEA (2.0 eq., 402 µL, 2.36 mmol) in dichloromethane (92 mL) at about 34 µL/min at room temperature, and the mixture was stirred at the same temperature for 19 hr. The reaction solution was concentrated under reduced pressure, to the concentrated residue was added ethyl acetate (40 mL), and the mixture was washed once with water (20 mL), twice with saturated aqueous ammonium chloride solution (20 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (987 mg) as a pale-yellow amorphous. The crude product was purified by flash silica gel column (normal phase silica gel 30 g, hexane/ethyl acetate = 80/20 - 65/35) to give Compound 49-4 (490 mg, containing impurities, apparent yield 55%) as a white amorphous.

Synthesis of Compound 49-5 from Compound 49-4 was carried out as follows.

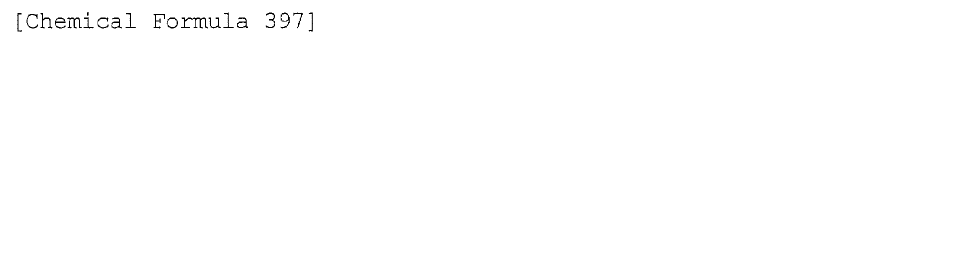

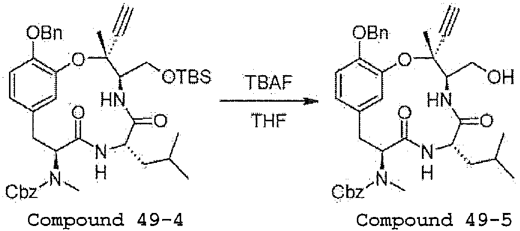

To a solution of Compound 49-4 (1.0 eq., 480 mg, calculated as 0.635 mmol) in THF (6 mL) was added TBAF (1M in THF, 2.0 eq., 1.27 mL, 1.27 mmol) at room temperature, and the mixture was stirred at the same temperature for 2 hr. To the reaction solution was added ethyl acetate (20 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (459 mg) as a white amorphous. The crude product was purified by flash silica gel column (normal phase silica gel 10 g, hexane/ethyl acetate = 60/40 - 0/100) to give Compound 49-5 (345 mg, containing impurities, yield 85%) as a white amorphous.

Synthesis of Compound 49-6 from Compound 49-5 was carried out as follows.

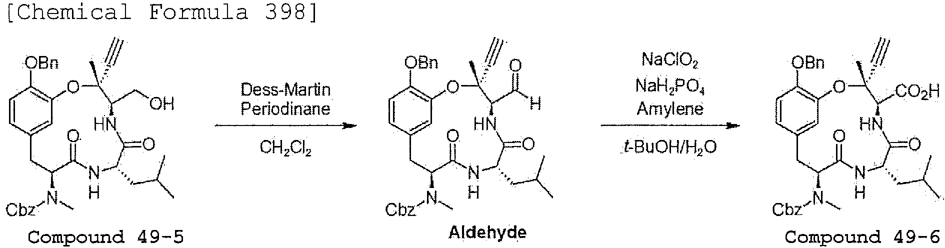

Under nitrogen atmosphere, to a solution of Compound 49-5 (1.0 eq., 338 mg, calculated as 0.526 mmol) in dichloromethane (5.3 mL) was added Dess-Martin periodinane (2.0 eq., 446 mg, 1.05 mmol) under ice-cooling, and the mixture was stirred at room temperature for 2 hr. To the reaction solution was added a mixture of 10% aqueous sodium sulfite solution (10 mL)/saturated aqueous sodium bicarbonate solution (10 mL), and the mixture was extracted once with ethyl acetate (30 mL) by liquid separation. The organic layer was washed once with a mixture of 10% aqueous sodium sulfite solution (10 mL)/saturated aqueous sodium bicarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (338 mg, crude yield 100%) as a white amorphous.

To a solution of Aldehyde (1.0 eq., 338 mg, calculated as 0.526 mmol) in a mixture of amylene (1.2 mL)/t-butyl alcohol (5 mL) was added a solution of sodium dihydrogen phosphate dihydrate (3.4 eq., 278 mg, 1.78 mmol) and 80% sodium chlorite (4.5 eq., 268 mg, 2.37 mmol) in water (1.2 mL) at room temperature, and the mixture was stirred at the same temperature for 1 hr. To the reaction solution was added ethyl acetate (20 mL), and the mixture was washed twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (367 mg) as a white amorphous. The crude product was purified by flash silica gel column (normal phase silica gel 6 g, hexane/ethyl acetate = 33/67 - 0/100) to give Compound 49-6 (335 mg, containing impurities, apparent yield 97%) as a white amorphous.

Synthesis of Compound 49-7 from Compound 49-6 was carried out as follows.

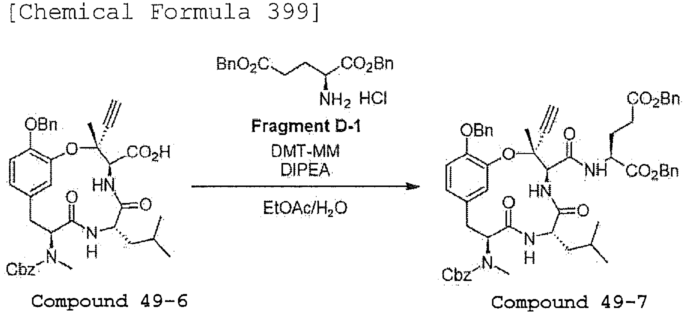

To a solution of Compound 49-6 (1.0 eq., 334 mg, calculated as 0.510 mmol) in a mixture of ethyl acetate (5 mL)/water (2.5 mL) was added dibenzyl L-glutamate hydrochloride (Fragment D-1, 1.5 eq., 279 mg, 0.768 mmol) at room temperature. DIPEA (2.5 eq., 217 µL, 1.28 mmol) was added under ice-cooling, the mixture was stirred for 10 min, DMT-MM (1.7 eq., 241 mg, 0.871 mmol) was added, and the mixture was stirred at the same temperature for 3 hr. To the reaction solution was added ethyl acetate (20 mL), and the mixture was washed once with water (10 mL), twice with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (561 mg) as a yellow amorphous. The crude product was purified twice by flash silica gel column (1st run: NH₂ silica gel 6 g, hexane/ethyl acetate = 67/33 - 50/50, 2nd run: normal phase silica gel 10 g, hexane/ethyl acetate = 75/25 - 50/50) to give Compound 49-7 (132 mg, yield 28%) as a white amorphous.

Synthesis of Compound 49 from Compound 49-7 was carried out as follows.

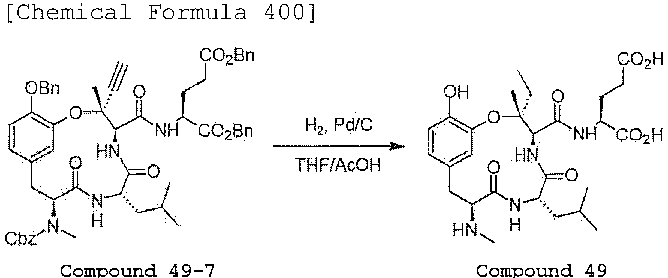

Under nitrogen atmosphere, to a solution of Compound 49-7 (1.0 eq., 131 mg, 0.140 mmol) in a mixture of THF (1.5 mL)/acetic acid (1.5 mL) was added 10% palladium-carbon (N.E. CHEMCAT NX type, 50% hydrous product, 13.1 mg) at room temperature, the system was replaced with hydrogen, and the mixture was stirred at the same temperature for 16 hr. The catalyst was removed by filtration through Celite, and the filtrate was filtered through 0.45 µm filter, and concentrated under reduced pressure to give a crude product (102 mg) as a gray solid. The crude product was purified twice by flash silica gel column (reverse-phase silica gel 30 g, 1st run: 0.05% aqueous acetic acid solution/acetonitrile = 95/5 - 69/31, 2nd run: 0.05% aqueous acetic acid solution/acetonitrile = 95/5 - 82/18), and freeze-dried to give an acetate of Compound 49 (39.0 mg, yield 49%, purity 99.0%) as a white solid.

¹H-NMR(400MHz,D₂0)δ6.87-6.78(m,3H),4.38(t,J=7.2Hz,1H),4.14(dd,J=8.8,4.7Hz,1H),3.86(dd,J =4.6,8.9Hz,1H),3.10(dd,J=4.6,13.4Hz,1H),2.83(dd,J=8.9,13.4Hz,1H ),2.57(s,3H),2.20(t,J=7.8Hz,2H),2.03-1.94(m,1H),1.88-1.75(m,2H),1.66-1.57(m,1H),1.49-1.45(m, 4H), 1.34-1.28(m,2H),0.88(t,J=7.3Hz,3H),0.72(d,J=6.2Hz,3H),0.69(d,J=6.2Hz ,3H).

### (Example 29O: Synthesis of Compound 50)

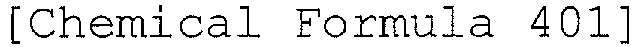

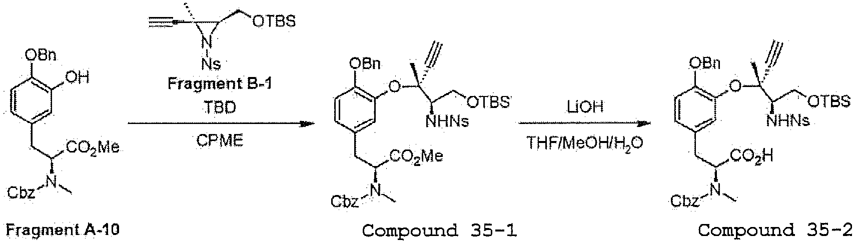

The two steps from Fragment A-10 to Compound 35-2 were carried out as described in Example 29I.

Synthesis of Compound 50-1 from Compound 35-2 was carried out as follows.

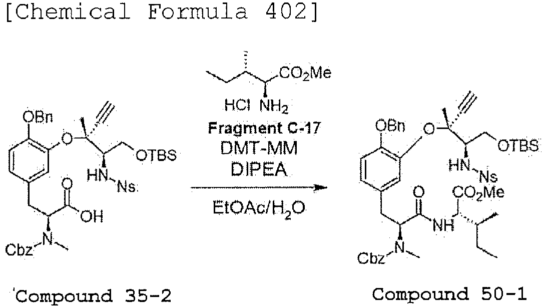

To a solution of Compound 35-2 (1.0 eq., 1.20 g, 1.42 mmol) in a mixture of ethyl acetate (11 mL)/water (3.3 mL) were added L-isoleucine-OMe hydrochloride (Fragment C-17, 1.5 eq., 388 mg, 2.14 mmol), DIPEA (1.7 eq., 400 µL, 2.35 mmol) and DMT-MM (1.7 eq., 668 mg, 2.41 mmol) at room temperature, and the mixture was stirred at the same temperature for 3 hr. To the reaction solution was added ethyl acetate (20 mL), and the mixture was washed three times with saturated aqueous ammonium chloride solution (10 mL) by liquid separation. The organic layer was dried over magnesium sulfate, the magnesium sulfate was removed by filtration, and the filtrate was concentrated under reduced pressure to give Compound 50-1 (1.54 g, crude yield 111%) as a pale-yellow amorphous.

Synthesis of Compound 50-2 from Compound 50-1 was carried out as follows.

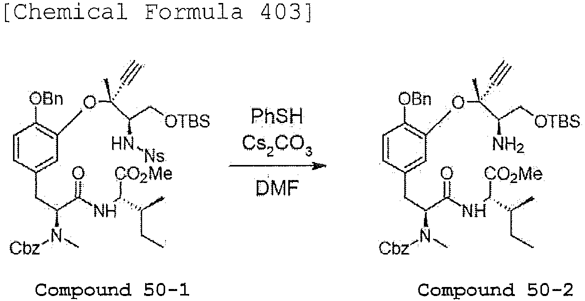

Under nitrogen atmosphere, to a solution of Compound 50-1 (1.0 eq., 1.54 g, calculated as 1.42 mmol) in DMF (14 mL) were added cesium carbonate (1.2 eq., 558 mg, 1.71 mmol) and thiophenol (1.2 eq., 175 µL, 1.71 mmol) under ice-cooling, and the mixture was stirred at the same temperature for 1.5 hr. A mixture of hexane (5 mL)/ethyl acetate (15 mL) was added, and the mixture was washed once with water (14 mL) by liquid separation. The aqueous layer was extracted three times with a mixture of hexane (3.7 mL)/ethyl acetate (1.3 mL) by liquid separation, and the organic layers were combined, and washed twice with water (10 mL) and once with saturated sodium chloride (5 mL) by liquid separation. The organic layer was dried over magnesium sulfate, the magnesium sulfate was removed by filtration, and the filtrate was concentrated under reduced pressure to give a crude product as a yellow viscous product. The crude product was purified by flash silica gel column (normal phase silica gel 16 g, hexane/ethyl acetate = 3/1 - 1/2) to give Compound 50-2 (1.21 g, yield 108%) as a pale-yellow viscous product.

Synthesis of Compound 50-3 from Compound 50-2 was carried out as follows.

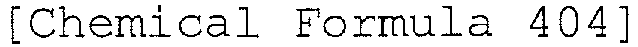

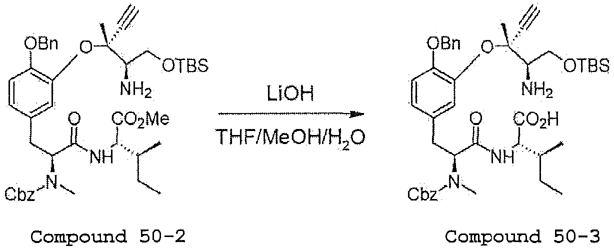

To a solution of Compound 50-2 (1.0 eq., 1.21 g, calculated as 1.42 mmol) in a mixture of THF (7 mL)/methanol (3.5 mL)/water (4 mL) was added lithium hydroxide (2.5 eq., 85.7 mg, 3.57 mmol) under ice-cooling, and the mixture was stirred at the same temperature for 3.5 hr. To the reaction solution was added 1N aqueous hydrochloric acid solution (1.1 mL), and the reaction solution was concentration under reduced pressure to approximately 10 mL. Water (10 mL) was added, and the mixture was extracted once with ethyl acetate (15 mL) and twice with ethyl acetate (3 mL) by liquid separation, and the organic layers were combined, and washed once with saturated aqueous sodium chloride solution (3 mL) by liquid separation. The organic layer was dried over magnesium sulfate, the magnesium sulfate was removed by filtration, and the filtrate was concentrated under reduced pressure to give Compound 50-3 (1.05 g, three-step yield 95%) as a white amorphous.

Synthesis of Compound 50-4 from Compound 50-3 was carried out as follows.

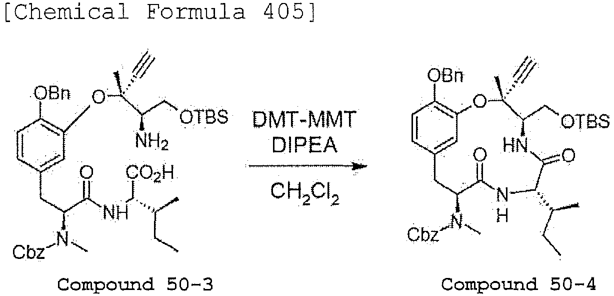

A solution of Compound 50-3 (1.0 eq., 1.05 g, 1.36 mmol) in dichloromethane (34 mL) was added dropwise over 4 hr to a solution of DMT-MMT (2.0 eq., 834 mg, 2.72 mmol) and DIPEA (2.0 eq., 460 µL, 2.70 mmol) in dichloromethane (105 mL) at room temperature, after the dropwise addition, the mixture was stirred at the same temperature for 19 hr. The reaction solution was concentrated under reduced pressure, ethyl acetate (20 mL) was added, and the mixture was washed once with saturated aqueous ammonium chloride solution (15 mL) by liquid separation. The aqueous layer was extracted once with ethyl acetate (10 mL) by liquid separation, and the organic layers were combined, and washed once with saturated aqueous ammonium chloride solution (10 mL) and once with saturated aqueous sodium chloride solution (5 mL) by liquid separation. The organic layer was dried over magnesium sulfate, the magnesium sulfate was removed by filtration, and the filtrate was concentrated under reduced pressure to give a crude product (1.30 g) as a pale-yellow amorphous. The crude product was purified by flash silica gel column (normal phase silica gel 13 g, hexane/ethyl acetate = 3/1 - 2/1) to give Compound 50-4 (267 mg, containing impurities, apparent yield 26%) as a white amorphous.

Synthesis of Compound 50-5 from Compound 50-4 was carried out as follows.

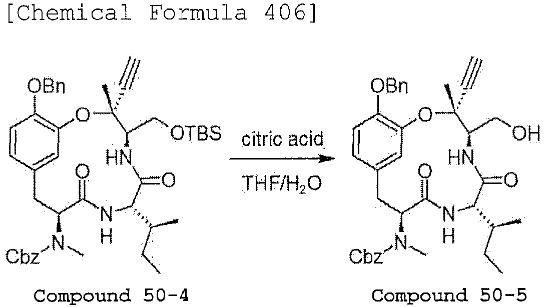

Under nitrogen atmosphere, to a solution of Compound 50-4 (1.0 eq., 264 mg, 0.349 mmol) in a mixture of THF (3.5 mL)/water (1.8 mL) was added citric acid (10 eq., 671 mg, 3.49 mmol) at room temperature, and the mixture was stirred at the external temperature of 35°C for 19 hr. To the reaction solution was added ethyl acetate (10 mL), and the mixture was washed once with water (10 mL), twice with saturated aqueous sodium hydrogencarbonate solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product. To the crude product was added ethyl acetate, and the mixture was concentrated under reduced pressure, and the precipitated white solid was collected by filtration to give Compound 50-5 (177 mg, yield 79%).

Synthesis of Compound 50-6 from Compound 50-5 was carried out as follows.

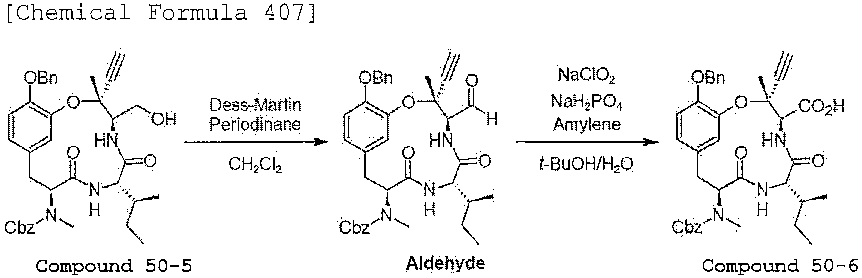

Under nitrogen atmosphere, to a solution of Compound 50-5 (1.0 eq., 177 mg, 0.275 mmol) in dichloromethane (2.8 mL) was added Dess-Martin periodinane (1.5 eq., 174 mg, 0.412 mmol) under ice-cooling, and the mixture was stirred at room temperature for 4 hr. To the reaction solution was added Dess-Martin periodinane (0.50 eq., 58.9 mg, 0.139 mmol), and the mixture was stirred at the same temperature for additional 2 hr. A mixture of 10% aqueous sodium sulfite solution (5 mL)/saturated aqueous sodium hydrogencarbonate solution (5 mL) was added under ice-cooling, and the mixture was extracted once with ethyl acetate (15 mL) by liquid separation. The organic layer was washed once with a mixture of 10% aqueous sodium sulfite solution (5 mL)/saturated aqueous sodium hydrogencarbonate solution (5 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Aldehyde (184 mg, crude yield 104%) as a white amorphous.

To a solution of Aldehyde (1.0 eq., 181 mg, calculated as 0.275 mmol) in a mixture of amylene (1 mL)/t-butyl alcohol (4 mL) was added a solution of sodium dihydrogen phosphate dihydrate (3.5 eq., 151 mg, 0.967 mmol) and 80% sodium chlorite (4.5 eq., 140 mg, 1.24 mmol) in water (1 mL) at room temperature, and the mixture was stirred at the same temperature for 1 hr. To the reaction solution was added ethyl acetate (10 mL), and the mixture was washed once with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give Compound 50-6 (192 mg, crude yield 106%) as a white amorphous.

Synthesis of Compound 50-7 from Compound 50-6 was carried out as follows.

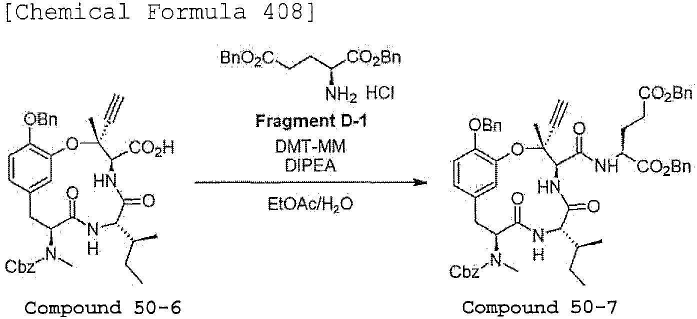

To a solution of Compound 50-6 (1.0 eq., 190 mg, calculated as 0.275 mmol) in a mixture of ethyl acetate (2.8 mL)/water (1.4 mL) was added dibenzyl L-glutamate hydrochloride (Fragment D-1, 1.5 eq., 150 mg, 0.413 mmol) at room temperature. DIPEA (2.5 eq., 117 µL, 0.688 mmol) was added under ice-cooling, the mixture was stirred for 5 min, DMT-MM (1.7 eq., 130 mg, 0.470 mmol) was added, and the mixture was stirred at the same temperature for 2 hr. To the reaction solution was added ethyl acetate (15 mL), and the mixture was washed once with water (10 mL), once with saturated aqueous ammonium chloride solution (10 mL) and once with saturated aqueous sodium chloride solution (10 mL) by liquid separation. The organic layer was dried over magnesium sulfate, the magnesium sulfate was removed by filtration, and the filtrate was concentrated under reduced pressure to give a crude product (321 mg) as a yellow amorphous. The crude product was purified twice by flash silica gel column (1st run: NH₂ silica gel 6 g, hexane/ethyl acetate = 67/33 - 50/50, 2nd run: normal phase silica gel 5 g, hexane/ethyl acetate = 60/40 - 50/50) to give Compound 50-7 (194 mg, yield 73%) as a white amorphous.

Synthesis of Compound 50 from Compound 50-7 was carried out as follows.

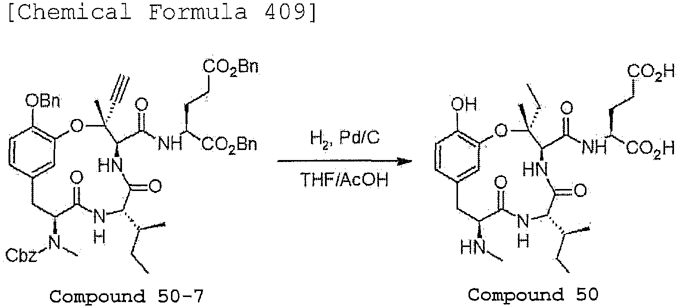

Under nitrogen atmosphere, to a solution of Compound 50-7 (1.0 eq., 193 mg, 0.200 mmol) in a mixture of THF (2 mL)/acetic acid (2 mL) was added 10% palladium-carbon (N.E. CHEMCAT NX type, 50% hydrous product, 19.5 mg) at room temperature, the system was replaced with hydrogen, and the mixture was stirred at the same temperature for 17 hr. The catalyst was removed by filtration through Celite, and the filtrate was filtered through 0.45 µm filter, and concentrated under reduced pressure to give a crude product (125 mg) as a gray solid. The crude product was purified by flash silica gel column (reverse-phase silica gel 30 g, 0.05% aqueous acetic acid solution/acetonitrile = 95/5 - 82/18), and freeze-dried to give an acetate of Compound 50 (70.7 mg, yield 63%, purity 99%) as a white solid.

¹H-NMR(400MHz,D₂O)66.82-6.75(m,3H)4.13-4.09(m,2H),3.91(dd,J=9.0,4.9Hz,1H),3.11(dd,J=13.4,4.9Hz,1H),2.7 9(dd, J=13.4,9.0Hz,1H),2.59(s,3H),2.21(t,J=7.3Hz,2H),2.01-1.85(m,2H),1.83-1.74(m,1H),1.68-1.56(m,2H),1.45(s,3H),1.30-1.22(m,1H),1.01-0.91(m,4H),0.68(t,J=7.4Hz,3H),0.62(d,J=6.9Hz,3H).

### (Example 30: Synthesis of Compound 51)

Compound 39 is produced by referring to the examples (paragraphs 0115 to 0137) of JP 2022-73598.

### (Example 31: Synthesis of Compound 52)

Compound 40 is produced by the method described in JP 2022-73598.

### (Example 32: Synthesis of Compound 53)

Compound 41 is produced by a method similar to that described in JP 2022-73598.

### (Example 33: Evaluation of activity of compound)

### (Materials)

Control substance (medium)
   Name: Otsuka physiological saline
   Manufacturing distributor: Otsuka Pharmaceutical Co., Ltd.
   Use amount: 50 µL
   Storage conditions: Room temperature
Test substance
   Name: Compound 9
   Source: Synthesized in the above Examples
   Used amount: 0.4 mL (50 mg/mL solution)
   Storage conditions: Freezing, light shielding

Method for preparing test substance (1 mg/mL): A high concentration solution prepared at 50 mg/mL was diluted 50 times with a medium to prepare a 1 mg/mL administration solution.

Storage and expiration date of preparation solution: The prepared administration solution was dispensed at 1 mL per day and stored frozen. The thawed administration solution was stored under refrigeration, the thawing date was set as the expiration date, and the remaining amount was discarded.

### Positive control substance

Name: MK-801
Distributor: SIGMA-ALDRICH
Use amount: 0.0501 g
Storage condition: refrigerated (2°C to 8°C)

Method for preparing positive control substance (10 mg/mL): 0.0501 g was weighed out and made up to a total volume of 5 mL with a control substance (physiological saline).

Storage and expiration date of preparation solution: The prepared administration solution was dispensed into 3-day portions and stored under refrigeration. The expiration date was set to one month or less for refrigeration.

### Test system

### 1. Animals used

Species and strains: Rat; Slc: SD
Number of animals on arrival and sex: 50 males
Age in weeks at arrival: 6 weeks old
Body weight range at arrival: 147.2 to 170.0 g
Source: Japan SLC, Inc.

Identification method: During the quarantine acclimation period, a symbol indicating a number was written on the tail with a red oil-based felt-tip pen, and a label with a test number, an arrival date, an age in weeks, a test item, a sex, a quarantine animal number, and a study director was attached to the front of each cage to identify an individual. After grouping, the test animal number was written on the tail with a black oil-based felt-tip pen. A label with a test number, a test date, an age in weeks, a sex, a test animal number, a test group, and a study director was attached to the front of each cage to identify an individual.

Quarantine acclimation: During the quarantine and acclimation period, general condition observations (once a day) and body weight measurements (once a week or more) were performed, and animals with good health and no abnormalities in the retina were subjected to the test.

Abnormality of the retina was determined for each individual by a fundus camera before grouping, and no abnormal individual was observed.

Handling of abnormal animals: No animal was observed to have an abnormality in general condition, increase/decrease in body weight, and the like during the test period.

### 2. Breeding conditions

Temperature: 23.0 ± 3.0°C
Humidity: 50 ± 20%
Number of times of ventilation: 10 times/hour or more
Lighting: 12 hours (turn on at 6:00 am and turn off at 6:00 pm).
Feed: Solid feed MF (Oriental Yeast Co., Ltd.) was provided ad libitum.
Drinking water: Tap water passed through a 5-µm cartridge filter was provided ad libitum.
Floor mat: Care Feeds (Hamley Co., Ltd.)
Breeding cage: polycarbonate cage (W290 × H190 × D450 mm)
Number of housed animals: 2 or 3 animals/cage

### (Experimental method)

### 1. Test group

**[Table B]**

| Test Group | Administered substance | Administration solution concentration | administration dose | Administration method | Number of cases | Test animal number |
|---|---|---|---|---|---|---|
| 1 | Saline | 0 | (5 µL x 4 times/day | Eyedrop administration | 10 | 101-110 |
| 2 | Compound 9 | 1 mg/mL | | | 10 | 201-210 |
| 3 | | | | | 10 | 301-310 |
| 4 | MK-801 | 10 mg/mL | (1 mL/kg x once) | Intravenous administration | 10 | 401-410 |

### 2. Grouping

Grouping was performed two days before the first creation of the retinal damage model induced by an anterior chamber aqueous humor pressure load. Using the daily body weight as an index, stratified random allocation was performed using SAS (Ver. 9.3, SAS Institute Japan Ltd.) and EXSUS (Ver. 8.0, CAC Croit Corporation) so that an average value of the body weights of each group was approximately uniform.

### 3. General condition observation and body weight measurement

The body weight of the animals was measured at a frequency of once a week or more, and the general condition observation was performed every day.

### 4. Administration

Administration route: Eyedrop administration, intravenous administration

Administration method (eyedrop): Eyedrop administration was performed on a treated eye (right eye) of a rat using a micropipette 4 times a day (at intervals of 1.5 hours or more).

Administration method (intravenous): The amount of administration solution, calculated from the body weight on the day of administration, was administered from the tail vein using a syringe and an injection needle about 30 minutes before ischemic load.

Time of administration: Administration was performed according to the following schedule.

**[Table C]**

| Test group administered on the following schedule | Administration date |
|---|---|
| 1 | for 13 days from the day after retinal damage model creation |
| 2 | 2 days on the day before and two days before retinal damage model creation |
| 3 | for 13 days from the day after retinal damage model creation |
| 4 | one day on the day of retinal damage model creation |

### 5. Creation of retinal damage model induced by anterior chamber aqueous humor pressure load

1. 10 mg/kg xylazine hydrochloride was intramuscularly administered on the model creation day (day 0). A rat was placed in an anesthesia box and subjected to general anesthesia with isoflurane (5%, oxygen : nitrous oxide = 1 : 3). When anesthesia was effective, the rat was taken out of the anesthesia box, an inhalation anesthetic mask was worn, and anesthesia was maintained with isoflurane (1% to 5%, oxygen : nitrous oxide = 1 : 3).
2. The body temperature was maintained at 37°C with a warming mat, and a mydrin P ophthalmic solution was instilled in the right eye (treated eye) of the rat to dilate the pupil, then the cravit ophthalmic solution 1.5% (Santen Pharmaceutical Co., Ltd.) was instilled.
3. A bottle of an IV bag containing physiological saline was previously suspended so that a water surface was 155 cm (100 mmHg or more) higher than the eye of the rat. The IV bag was connected to a pediatric infusion set (Terumo Corporation), a 27G injection needle thereof was inserted into and fixed to the anterior chamber while slightly dripping the liquid from a needle tip. The model was created in the light phase (6:00 to

18:00) in consideration of the diurnal variation of the intraocular pressure.
4. After the needle was inserted into the anterior chamber, pressure (100 mmHg or more) was loaded and maintained in the anterior chamber by associating the reflux system. Care was taken so that the needle did not hit the lens or iris when the needle was inserted.
5. After an ischemic load for 45 minutes, the injection needle and the like were removed, the inhalation anesthetic mask was removed, and the rat was sufficiently woken up under a warming mat, and then returned to the cage.
6. For 2 days after surgery, 10 mg/kg xylazine hydrochloride was intramuscularly administered as an analgesic for the purpose of alleviating the pain after the treatment.

### Eyeball enucleation, eyeball fixation, and preparation of pathological specimen

1. Fourteen days after model creation, general anesthesia was performed with isoflurane (5%, oxygen:nitrous oxide = 1:3), euthanasia was then performed by exsanguination, a suture was placed as a marker at the 12 o'clock position of the ocular conjunctiva, and eyeball enucleation was performed.
2. The eyeball was placed in an eyeball fixation solution (Superfix, Kurabo Industries Ltd.), and fixation was performed for 72 hours (room temperature).
3. Dehydration was performed with 50% alcohol for about 24 hours without washing with water.
4. Dehydration was performed with 70% alcohol for about 24 hours.
5. After dehydration with 70% alcohol for about 24 hours, the eyeball was trimmed and transferred to an embedding cassette, and then dehydrated with 80% alcohol for about 24 hours.
6. The eyeball was placed in an automatic fixing embedding device, and embedded in the following setting: 90% alcohol (2 hours) → 95% alcohol (1.5 hours) → 100% alcohols (1) to (5) each for 1 hour → xylene (1) for 1.5 hours → xylene (2) for 1.5 hours → xylene (3) for 1.5 hours → paraffins (1) to (4) each for 1 hour.
7. Three or more transversal pathological specimens (HE staining) including the optic disk were prepared.

### Photographing of pathological specimen

Three specimens each having no damage due to slicing were randomly selected from the prepared pathological specimens, and a range including the optic disk was photographed at a constant magnification using an optical microscope photographing system.

### Measurement of retinal thickness

For the retinal cross-sectional photograph of the pathological specimen, thickness of the retina (IPL, INL, and ONL) at 300, 600, and 900 µm from the optic disk was measured using Image J [National Institutes of Health (NIH)].

### Humanity endpoint

No animals were euthanized by applying humane endpoints.

### Statistical method

The obtained test results were expressed as individual values, average values, and standard errors. For the retinal layer thickness, 1-way ANOVA was performed for a control substance and a test substance, and Dunnett's test was performed in a case where the difference between groups was significant. In addition, in the comparison between the control substance and a positive control substance, Student's t-test was performed. The significance level was 5% on both sides (indication: P < 0.05 and P < 0.01).

### Results and Conclusions

The general condition and retinal thickness (inner plexiform layer, inner nuclear layer, and outer nuclear layer) when a control substance, a test substance and a positive control were administered to an anterior chamber aqueous humor pressure load rat model are shown in FIGS. 1 to 4 and Tables 1 to 7.

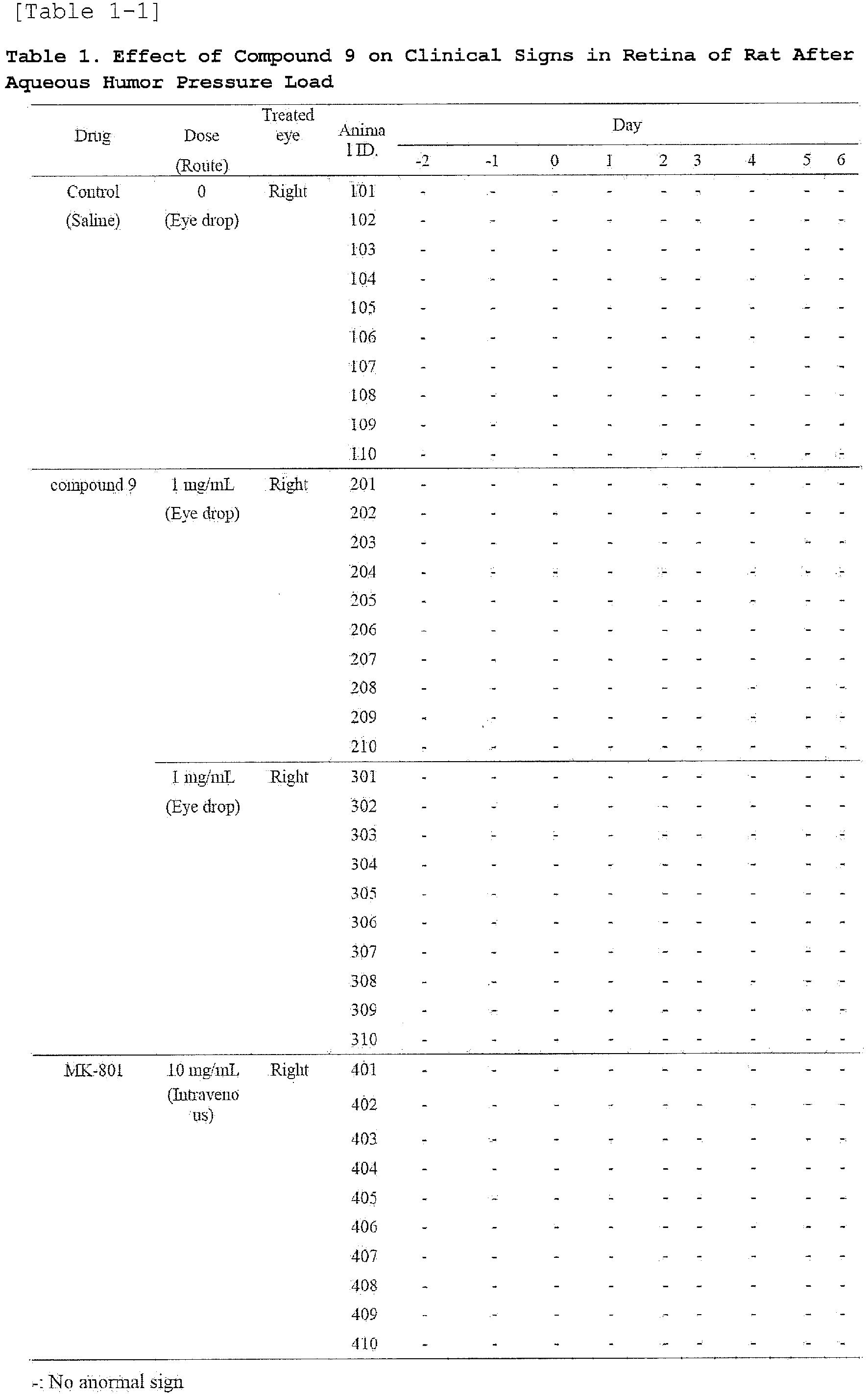

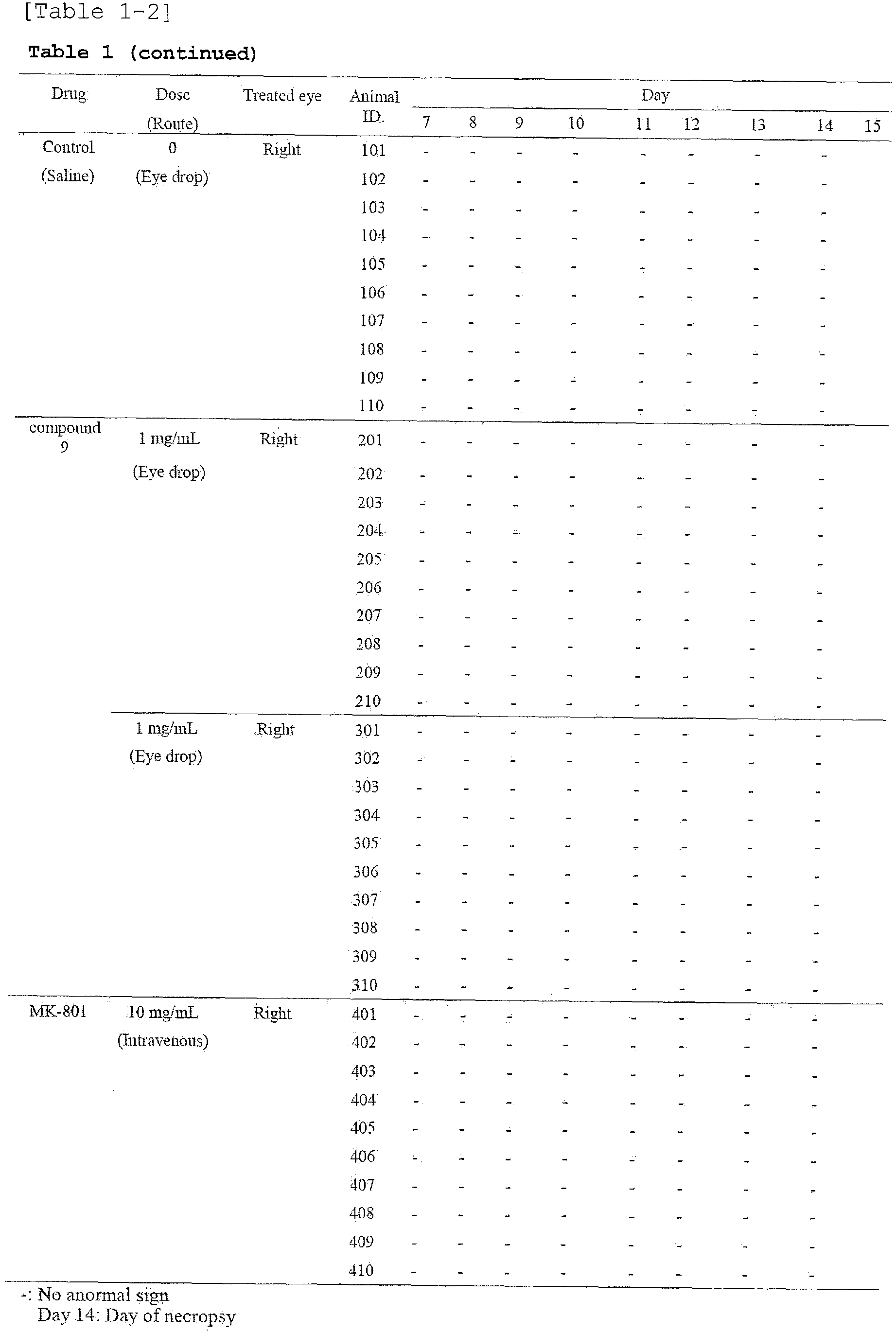

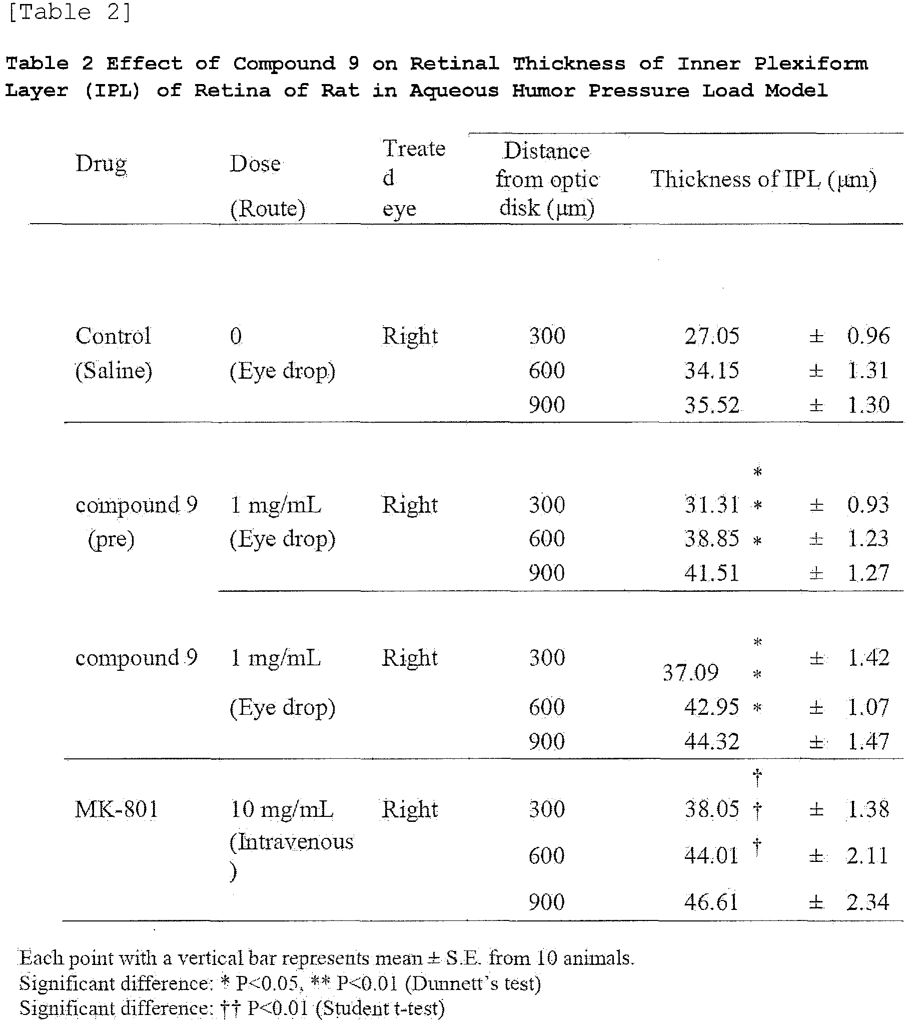

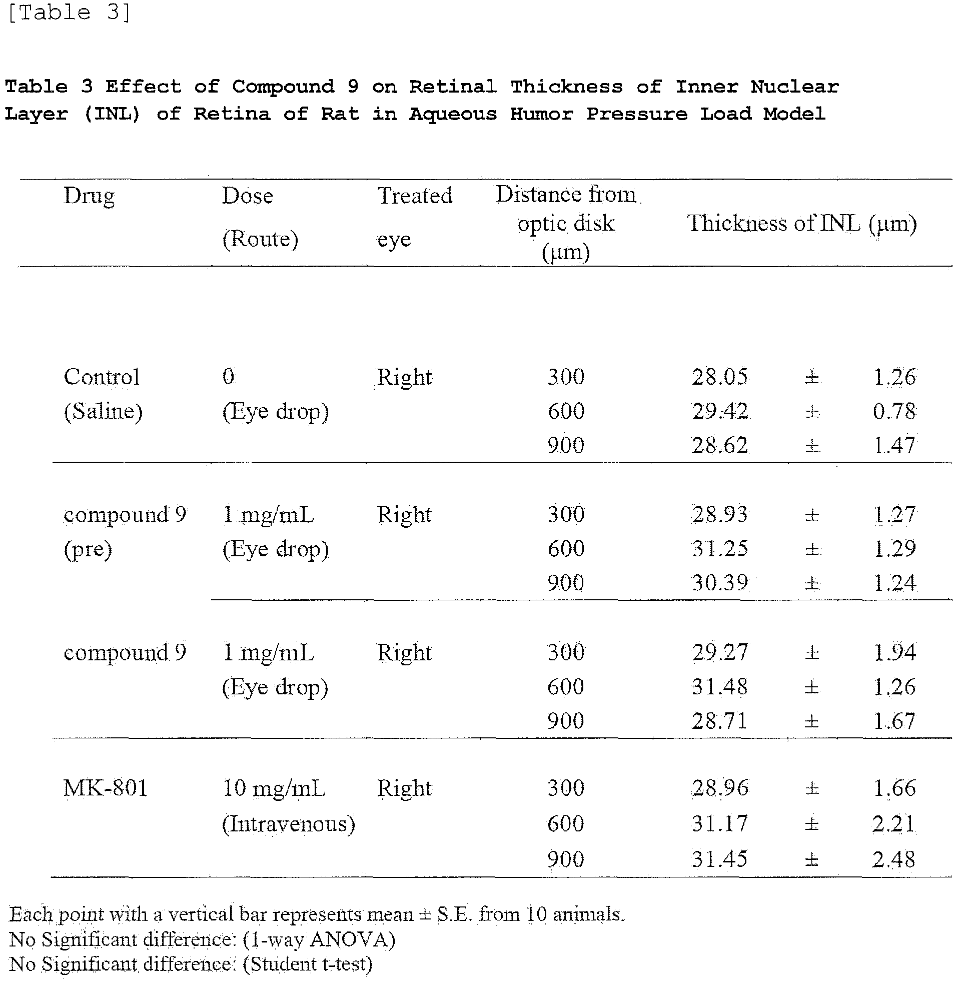

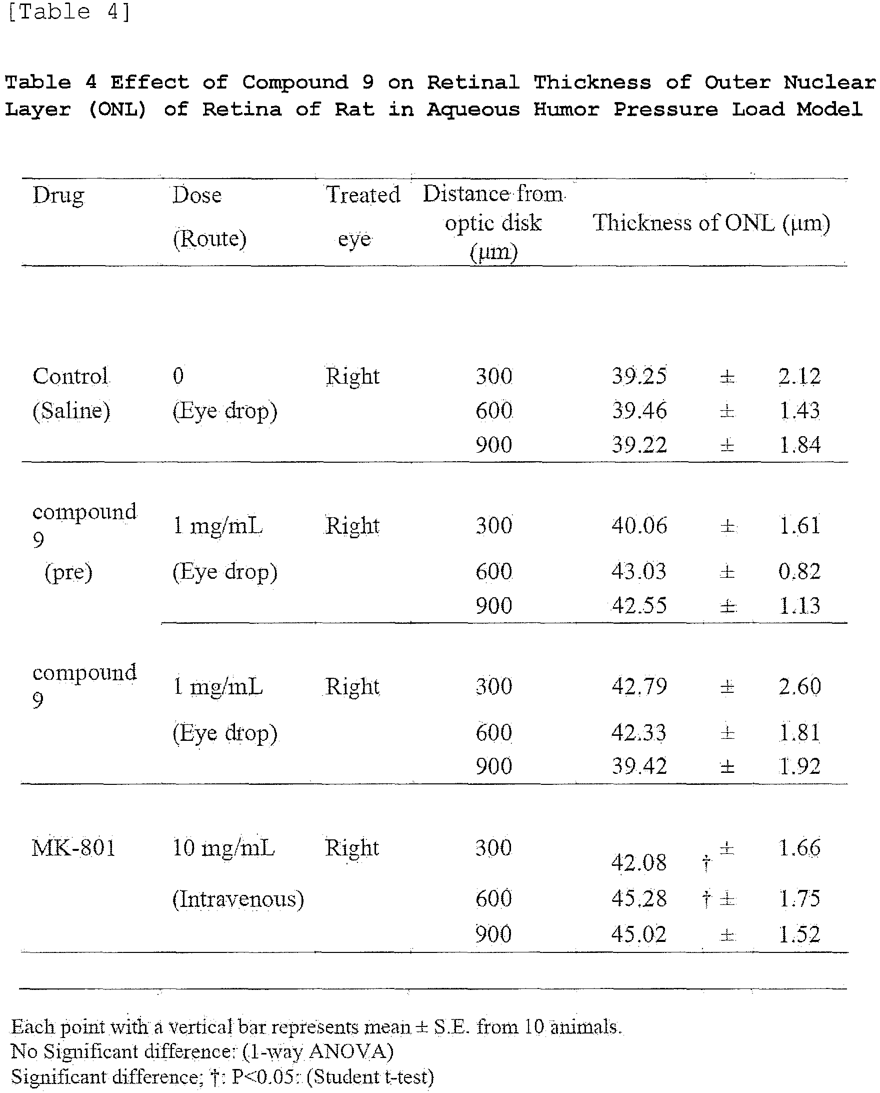

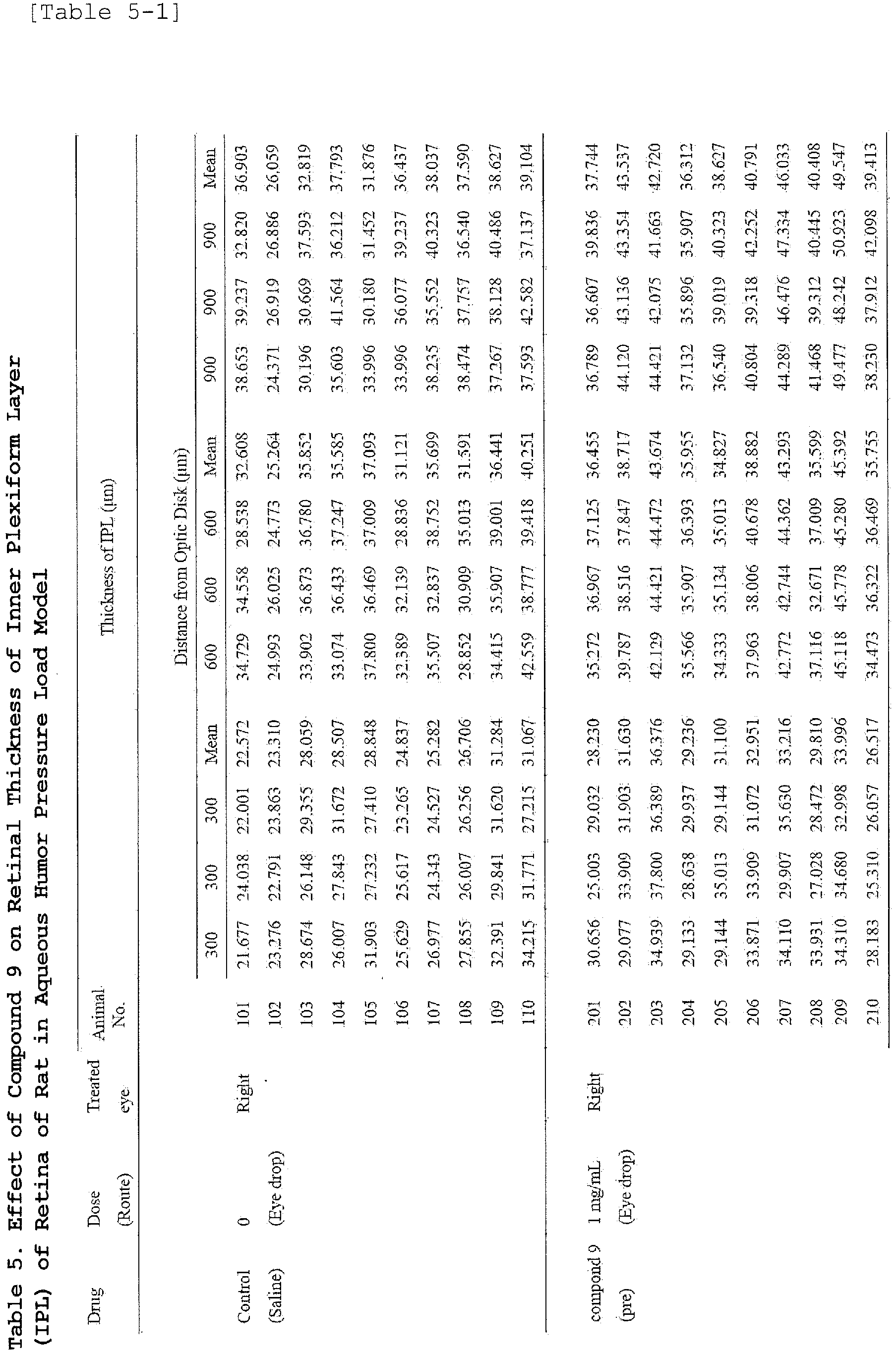

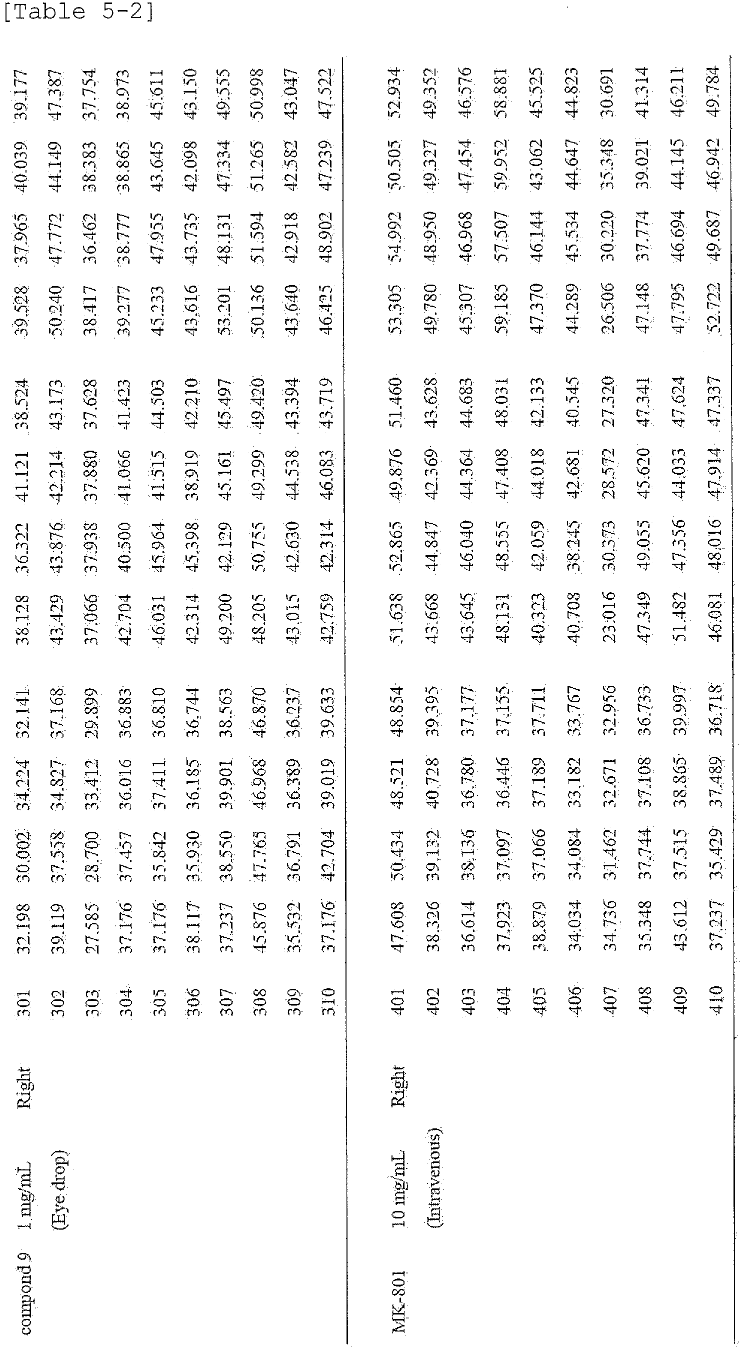

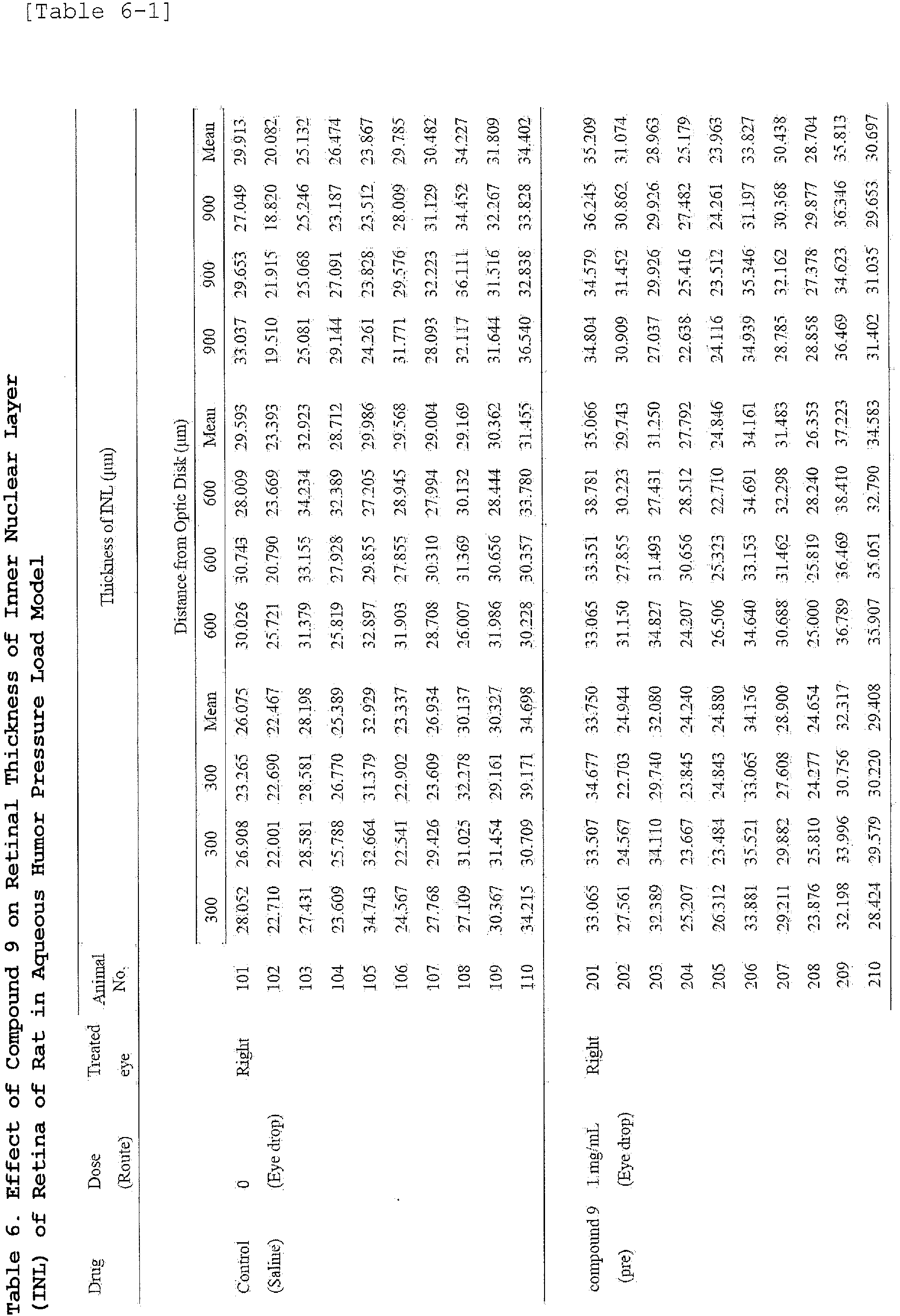

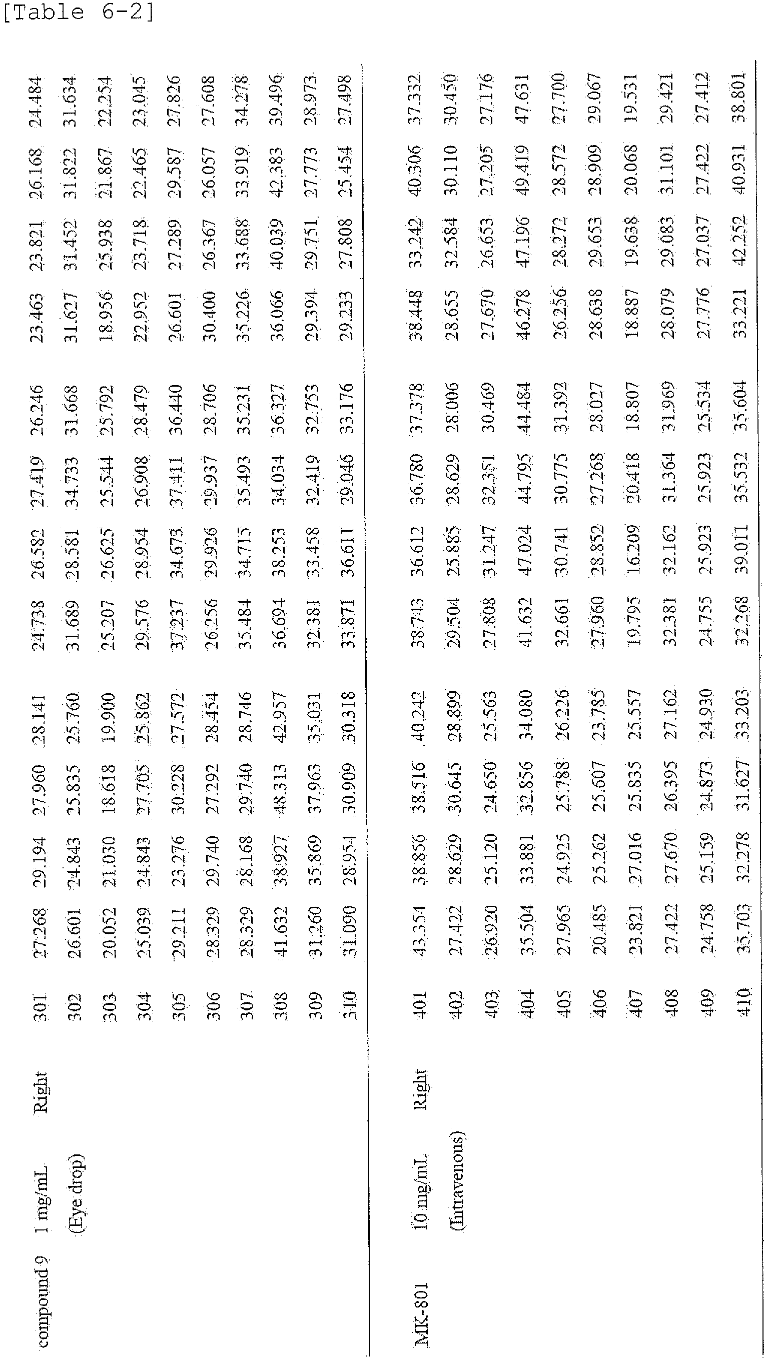

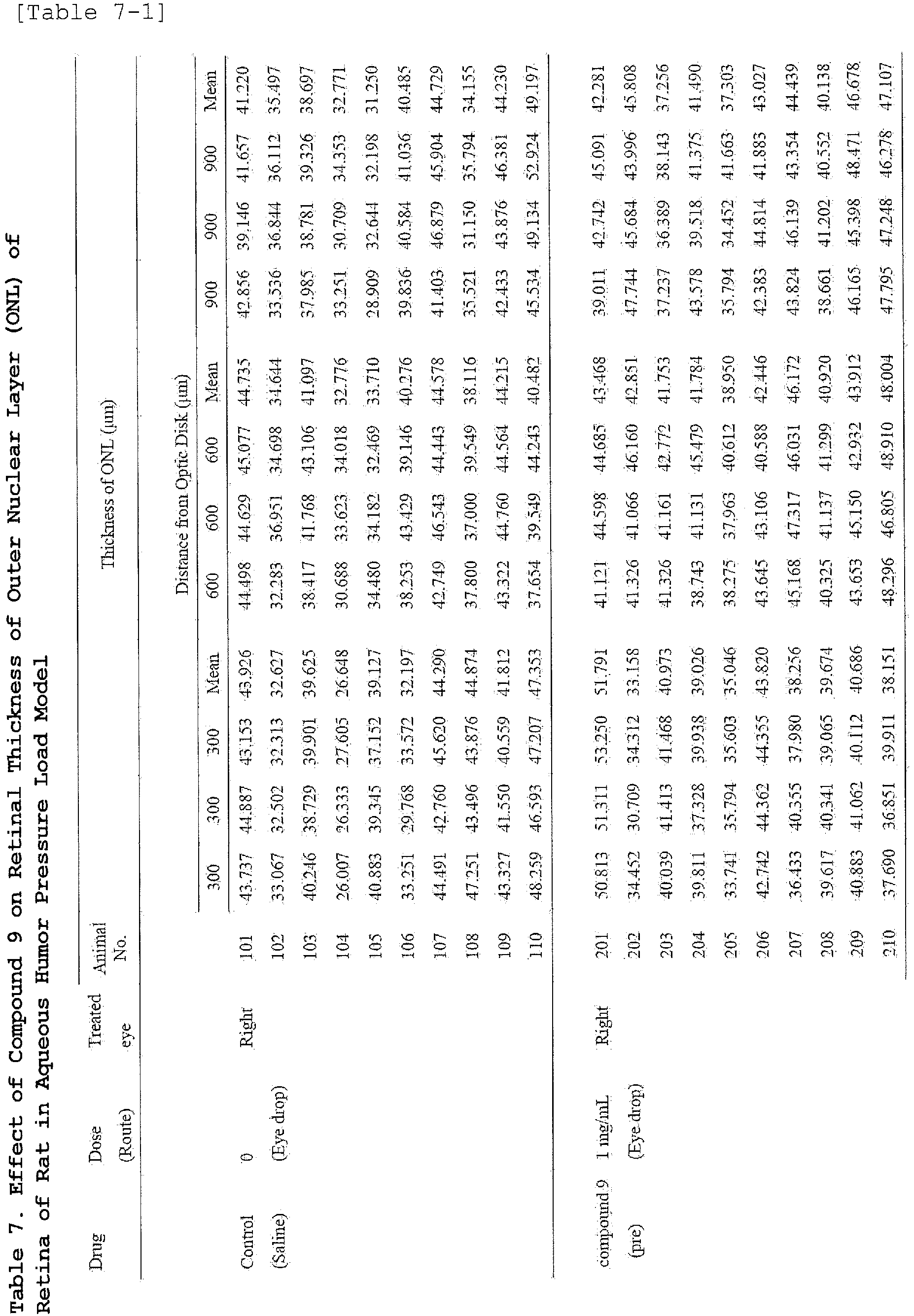

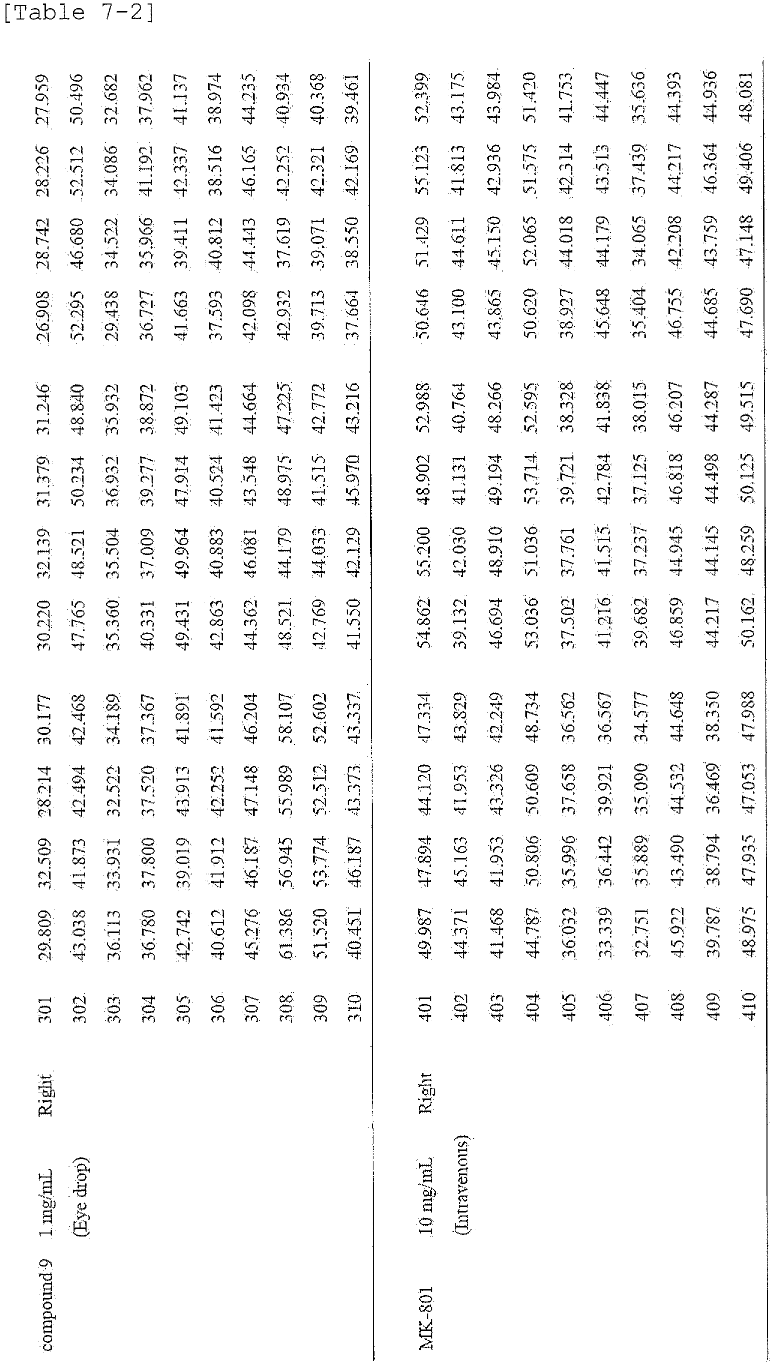

### General condition observation

No abnormality was observed in the general condition in all the test groups performed in this test from before model creation to eyeball enucleation.

### Retinal thickness

### Inner plexiform layer (IPL)

In test group 1, 27.05 ± 0.96 µm, 34.15 ± 1.31 µm, and 35.52 ± 1.30 µm were shown at 300, 600, and 900 µm from the optic disk, respectively. In test group 2, 31.31 ± 0.93 µm, 38.85 ± 1.23 µm, and 41.51 ± 1.27 µm were compared with those of the control group, and statistically significant differences were observed at all measurement sites between 300 to 900 µm from the optic disk. In test group 3, 37.09 ± 1.42 µm, 42.95 ± 1.07 µm, and 44.32 ± 1.47 µm were compared with those of the control group, and statistically significant differences were observed at all measurement sites between 300 to 900 µm from the optic disk.

On the other hand, in test group 4 used as a positive control, 38.05 ± 1.38 µm, 44.01 ± 2.11 µm, and 46.61 ± 2.34 µm were compared with those of the control group, statistically significant suppression of retinal damage was shown at all measurement sites between 300 to 900 µm from the optic disk, and it was confirmed that this test was appropriate as an experimental system for quantitatively evaluating the retinal layer thickness.

### Inner nuclear layer (INL)

In test group 1, 28.05 ± 1.26 µm, 29.42 ± 0.78 µm and 28.62 ± 1.47 µm were shown at 300, 600, and 900 µm from the optic disk, respectively. In test group 2, 28.93 ± 1.27 µm, 31.25 ± 1.29 µm, and 30.39 ± 1.24 µm were shown. In test group 3, 29.27 ± 1.94 µm, 31.48 ± 1.26 µm, and 228.71 ± 1.67 µm were shown, and no obvious change was observed in any of the test substance groups as compared with the control group. In test group 4, 28.96 ± 1.66 µm, 31.17 ± 2.21 µm, and 31.45 ± 2.48 µm were compared with those of the control group, and no obvious change was observed.

### Outer nuclear layer (ONL)

In test group 1, 39.25 ± 2.12 µm, 39.46 ± 1.43 µm, and 39.22 ± 1.84 µm were shown at 300, 600, and 900 µm from the optic disk, respectively. In test group 2, 40.06 ± 1.61 µm, 43.03 ± 0.82 µm and 42.55 ± 1.13 µm were shown. In test group 3, 42.79 ± 2.60 µm, 42.33 ± 1.81 µm, and 39.42 ± 1.92 µm were shown, and no obvious change was observed in any of the test substance groups as compared with the control group.

In test group 4, 42.08 ± 1.66 µm, 45.28 ± 1.75 µm and 45.02 ± 1.52 µm were compared with those of the control group, and statistically significant differences were observed at measurement sites between 600 to 900 µm from the optic disk.

From the above, with respect to the anterior chamber aqueous humor pressure load rat model, an inhibitory effect on retinal damage induced by ischemia of aqueous humor pressure load was observed in Compound 9, and the possibility of exhibiting a preventive effect and a therapeutic effect on retinal damage under the present test conditions was suggested.

### (Summary)

As a retinal neuroprotective effect confirmation test for a retinal damage model, the neuroprotective effect of Compound 9 was examined using an anterior chamber aqueous humor pressure load rat model of 10 animals in each group. The model using aqueous humor pressure load was created by injecting a physiological saline at a height of 155 cm (100 nnHg or more) into the anterior chamber using an infusion set and an injection needle under inhalation anesthesia, and applying pressure for 45 minutes. For the evaluation of the retinal layer thickness, the eyeball was enucleated after exsanguination under anesthesia 14 days after model creation, and three or more transected pathological specimens including the optic disk were prepared for each individual, and photographed at a constant magnification using an optical microscope photographing system. The photographed pathological specimen was measured for the thickness of inner plexiform layer (IPL), inner nuclear layer (INL), and outer nuclear layer (ONL) at 300, 600, and 900 µm from the optic disk using ImageJ. In order to confirm the neuroprotective effect of the test substance, for the preventive effect (test group 2), eyedrop administration was performed for 2 days (4 times/day) on the day before and two days before the model creation. For the therapeutic effect (test group 3), eyedrop administration was performed for 13 days (4 times/day) from the day after the retinal damage model creation. For the control substance (test group 1), eyedrop administration of physiological saline was similarly performed for 13 days (4 times/day). As a positive control substance (test group 4), MK-801 was intravenously administered about 30 minutes before the model creation day.

As a result, no obvious change was observed in the inner nuclear layer and the outer nuclear layer as compared with the control group. For the inner plexiform layer, in the test groups 2 and 3, a statistically significant effect of suppressing retinal damage by aqueous humor pressure load was observed at all measurement sites of 300 to 900 µm from the optic disk as compared with the control group, and the effect of the test group 3 was almost equivalent to that of the MK-801.

From the above, with respect to the anterior chamber aqueous humor pressure load rat model, an inhibitory effect on retinal damage induced by ischemia of aqueous humor pressure load was observed in Compound 9, and the possibility of exhibiting a preventive effect and a therapeutic effect on retinal damage under the present test conditions was suggested.

### (Example 34: Evaluation of drug efficacy of Compound 9 in laser-induced choroidal neovascularization rat model)

### 1. Materials

(1) Test substance
   Name: Compound 9
   Storage conditions: Refrigerating a powder and freezing a solution (-20°C)
   Source: Synthesized in the above Examples
(2) Positive control substance
   Name: Aflibercept
   Product name: EYLEA (registered trademark) intravitreal injection 40 mg/mL
   Storage conditions: Stored in a refrigerator in a light-shielding state.
   Manufacturer: Bayer Yakuhin, Ltd.
   Distributor: Santen Pharmaceutical Co., Ltd.
(3) Negative control substance
   Name: Physiological saline
   Storage conditions: Stored at room temperature, and after opening, stored in a refrigerator.
   Manufacturing distributor: Otsuka Pharmaceutical Co., Ltd.

### 2. Method for preparing test substance and control substance administration solution

### (1) Test substance administration solution (10 mg/mL and 1 mg/mL Compound 9 administration solution)

The test substance compound 9 was weighed, and then dissolved in physiological saline to prepare a 10 mg/mL compound 9 administration solution. A part of this solution was collected and diluted 10 times with physiological saline to prepare a 1 mg/mL compound 9 administration solution. The prepared 10 mg/mL and 1 mg/mL compound 9 administration solutions were each dispensed into 15 tubes and stored frozen at -20°C. The dispensed tubes were taken out from the frozen storage immediately before the first administration on the day of administration, naturally thawed at normal temperature, and used as a test substance administration solution. The thawed test substance administration solution was stored under refrigeration and used within 24 hours.

### (2) Positive control substance administration solution (EYLEA (registered trademark) intravitreal injection solution 40 mg/mL)

EYLEA (registered trademark) intravitreal injection solution 40 mg/mL (Bayer Yakuhin, Ltd., hereinafter referred to as "EYLEA (registered trademark)") was not prepared, and was opened at the time of administration of the first course and used as it was as an intravitreal administration solution. The vial residual liquid of EYLEA (registered trademark) opened at the time of administration of the first course was stored under refrigeration and used as an administration solution of a second course.

### (3) Negative control substance administration solution (physiological saline)

Physiological saline (Otsuka Pharmaceutical Factory, Inc.) was not prepared, and was opened and used as it was as an administration solution. The physiological saline was dispensed into 15 tubes and used within 1 month under refrigerated storage.

### 3. Administration dose, administration method, number of administrations, administration timing of test substance and control substance administration solution

(1) Test substance administration solution 1
   Administration substance name: Compound 9 (10 mg/mL solution)
   Administration method: Eyedrop administration
   Dose administered: 50 µg/5 µL/eye
   Administration eye: Right eye (laser-irradiated eye)
   Number of administrations: 56 times (for 14 days, 4 times/day at 9:00, 11:00, 13:00, and 15:00, 1 hour allowed before and after)
   Administration timing: Immediately after laser irradiation to the day before eyeball enucleation (Day 0 to 13)
(2) Test substance administration solution 2
   Administration substance name: Compound 9 (1 mg/mL solution)
   Administration method: Eyedrop administration
   Dose administered: 5 µg/5 µL/eye
   Administration eye: Right eye (laser-irradiated eye)
   Number of administrations: 56 times (for 14 days, 4 times/day at 9:00, 11:00, 13:00, and 15:00, 1 hour allowed before and after)
   Administration timing: Immediately after laser irradiation to the day before eyeball enucleation (Day 0 to 13)
(3) Positive control substance
   Administration substance name: EYLEA (registered trademark)
   Administration method: Intravitreal administration
   Dose administered: 200 µg/5 µL/eye
   Administration eye: Right eye (laser-irradiated eye)
   Number of administrations: Once (once/day)
   Administration timing: Immediately after laser irradiation (Day 0)
(4) Negative control substance
   Administration substance name: Physiological saline
   Administration method: Eyedrop administration
   Dose administered: 5 µL/eye
   Administration eye: Right eye (laser-irradiated eye)
   Number of administrations: 56 times (for 14 days, 4 times/day at 9:00, 11:00, 13:00, and 15:00, 1 hour allowed before and after)
   Administration timing: Immediately after laser irradiation to the day before eyeball enucleation (Day 0 to 13)

### 4. Preparation of reagents

(1) Ketamine hydrochloride/xylazine hydrochloride (4:1, v/v) mixed solution
   KETALAR (registered trademark) for intramuscular injection (containing ketamine at 50 mg/mL: Daiichi Sankyo Company, Limited) and Seractar (registered trademark) 2% injection (containing xylazine at 20 mg/mL: Elanco Japan K.K.) were mixed at a ratio of 4:1 to prepare a mixture, and the mixture was stored under refrigeration and used within 1 week.
(2) 4% (w/v) FITC-dextran solution
   Fluorescein isothiocyanate-dextran (average molecular weight: 20,000, SIGMA-ALDRICH) was dissolved in Dulbecco's phosphate buffer solution (FUJIFILM Wako Pure Chemical Corporation, hereinafter referred to as D-PBS) to prepare a 4% (40 mg/mL) solution, which was stored under refrigeration and used within 24 hours.
(3) 129.6 mg/mL Pentobarbital sodium solution
   Pentobarbital sodium (Tokyo Chemical Industry Co., Ltd.) and physiological saline were mixed to prepare a mixture, and the mixture was stored under refrigeration and used within one week.

### 5. Test system

### (1) Animals used

Species: Rat
System: BN/SsNSlc
Number of animals obtained: 48
Number of animals used: 40
   Age in weeks (when obtained): 6 weeks
Sex: Male
Date of acquisition: March 2, 2023
Source: Japan SLC, Inc.
Reason for selection: For animal species widely used in ophthalmological pharmacological tests, accumulated data are available, and maintenance of the system is established

### (2) Quarantine and preliminary housing

The obtained animals were preliminary housed for 5 days. During the preliminary housing period, an anterior segment of the eye was observed using a slit lamp microscope (Slit Lamp SL-15, Kowa Company, Ltd.), then Tropicamide (Mydrin (registered trademark) P ophthalmic solution, Santen Pharmaceutical Co., Ltd.) as a mydriatic agent for inspection was instilled to cause mydriasis, and the eye fundus (retina) was observed using an intermediate ocular lens and a binocular indirect ophthalmoscope (IO-α, Neitz Corporation). The general conditions (such as appearance, behavior, and state of excrement) were observed during the preliminary housing period. The body weight was measured on the arrival date and the end date of the preliminary housing period.

### (3) Individual identification of animals

The animals obtained had animal numbers written in their tails with a felt-tip pen and each cage was individually identified with an identification card. The following information were written in each identification card.
When obtaining animals: Test code number, sex, number of animals on arrival, and name of study director
After grouping: Test code number, sex, number of animals on arrival, test animal number, laser-irradiated eye (administration eye or enucleated eye), administration substance name, administration dose, administration method, administration date, eyeball enucleation date, course number, and name of study director

### (4) Environmental conditions

(4-1) Animal housing room
   First facility Animal housing room 1
(4-2) Cage
   Type: Plastic cage (made of polycarbonate)
   Size: 210 W × 320 D × 135 H mm
   Bedding: Soft chip (Sankyo Lab Service Co., Ltd.)
   Number of animals housed: 1 animal/cage
   Cage replacement frequency: Replaced twice a week.
(4-3) Temperature and humidity, ventilation, lighting, cleaning and disinfection
   Temperature: 19°C to 25°C
   Humidity: 30% to 70%
   Number of times of ventilation: 6 times or more/hour
   Lighting time: 12 hours/day (lights from 7:00 AM to 7:00 PM)
   Lighting illuminance: 300 Lux or less
   Cleaning and disinfecting method: At least three times per week, housing rooms and the housing racks were cleaned and disinfected with a solution prepared by adjusting Purelox (registered trademark) (Oyalox Co.,Ltd.) or 10% Hygieel disinfection solution 10% (Maruishi Pharmaceutical Co.,Ltd.) to 0.02% and 0.1% of tap water, respectively.
(4-4) Feed
   Name: Solid feed NMF
   Source of purchase: Oriental Yeast Co., Ltd.
   Feeding frequency: ad libitum fed.
   Nutritional composition and microorganisms: An analysis result report issued by Oriental Yeast Co., Ltd. was obtained and confirmed to comply with the acceptance criteria of the test facility.
   Contaminants such as heavy metals: An analysis result report issued by Eurofins Food and Protection Testing K.K. was obtained and confirmed to comply with the acceptance criteria of the test facility.
(4-5) Drinking water
   Source of supply of drinking water: Tap water (Chihaya akasaka village waterworks)
   Method for supplying drinking water: The water was provided ad libitum from a polycarbonate water bottle.
   Drinking water replacement frequency: The water was replaced at least 3 times per week.
   Analysis of drinking water: A water quality inspection report was obtained twice a year at KS Analysis Center Co., Ltd. It was confirmed that the analytical values were complied with the acceptance criteria of the test facility.

### 6. Test implementation method

### (1) Grouping

Individuals in which no abnormality was observed in an ophthalmic examination during the preliminary housing period were grouped according to the number of animals described in Group constitution of Item (2) so that the body weight measured at the end of the preliminary housing was uniform, and subjected to course classification. Animals excluded by grouping were transferred to an animal housing and management supervisor.

### (2) Group constitution

The groups were set as in Table 8 below. Laser irradiation was performed on the right eye in 10 animals in each group. EYLEA (registered trademark) was administered intravitreally into the right eye of individuals in Group 2 immediately after laser irradiation. For the individuals of Groups 1, 3, and 4, eyedrop administration of the administration substances of each group was performed four times a day from the day of laser irradiation to the day before eyeball enucleation.

**[Table 8]**

| Group | Administered substance (Dose administered) | Administration route (number of doses) [per day] | Number of cases (Test animal number) | |
|---|---|---|---|---|
| | | | First course | Second course |
| 1 | Saline (5 µL/eye) | Eyedrop administration (56 times) [4 times/day] | 5 (M1-1 - M1-5) | 5 (M1-6 - M1-10) |
| 2 | EYLEA^{®} (200 µg/5 µL/eye) | Intravitreal administration (once) [once a day] | 5 (M2-1- M2-5) | 5 (M2-6 - M2-10) |
| 3 | Compound 9 (1 mg/mL) (5 µg/5 µL/eye) | Eyedrop administration (56 times) [4 times/day] | 5 (M3-1 - M3-5) | 5 (M3-6 - M3-10) |
| 4 | Compound 9 (10 mg/mL) (50 µg/5 µL/eye) | Eyedrop administration (56 times) [4 times/day] | 5 (M4-1 - M4-5) | 5 (M4-6 - M4-10) |

### (3) General condition observation

The appearance, behavior, and state of excrement or the like were observed every day.

### (4) Body weight measurement

The body weight was measured on the animal arrival date, the preliminary housing end date (grouping date), the eyedrop administration start date, the laser irradiation date (Day 0), and the eyeball enucleation date (Day 14).

### (5) Preparation of laser-induced choroidal neovascularization model

Animals to be laser-irradiated were subjected to pupil dilation with Mydorin (registered trademark) P ophthalmic solution, and subjected to general anesthesia by intramuscular injection of a mixed solution of ketamine hydrochloride/xylazine hydrochloride (4:1, v/v) (1 mL/kg) into the thigh muscle. Then, fundus of the right eye was then observed using a slit lamp (SL-130, Carl Zeiss Meditec AG), and laser irradiation was performed at six locations using a green laser photocoagulation device (GYC-500, Nidek Co., Ltd.), avoiding thick capillaries of the retina. The laser conditions were a wavelength of 532 nm (green), a spot size of 50 µm, an irradiation time of 0.1 sec, and a laser irradiation intensity of 300 mW. Except for the individuals to be administered EYLEA (registered trademark), sodium hyaluronate (Hyalein (registered trademark) ophthalmic solution 0.3%, Santen Pharmaceutical Co., Ltd.) as an eye drop for treatment of corneal and conjunctival epithelial disorders was instilled into both eyes after laser irradiation.

### (6) Intravitreal administration of positive control substance (EYLEA (registered trademark))

Levofloxacin hydrate (CRAVIT (registered trademark) 1.5% ophthalmic solution, Santen Pharmaceutical Co., Ltd.) as a broad-spectrum antibacterial eye drop and oxybuprocaine hydrochloride (Benoxil (registered trademark) 0.4% ophthalmic solution, Santen Pharmaceutical Co., Ltd.) as an ophthalmic topical anesthetic were once instilled into the right eye of rats in Group 2 (EYLEA (registered trademark) administration group) after end of laser irradiation, and then EYLEA (registered trademark) was intravitreally administered at a dose of 200 µg/5 µL/eye using a 33G two-stage injection needle and a microsyringe (Ito Manufacturing Co., Ltd.). The microsyringe was washed with D-PBS before administration. Hyalein (registered trademark) ophthalmic solution 0.3% was instilled into both eyes after intravitreal administration.

### (7) Eyedrop administration of test substance and negative control substance

Eyedrop administration of the test substance and the negative control administration solution was performed in a volume of 5 µL/eye using a micropipette (Eppendorf Research Plus V, Eppendorf) at the administration period and the number of times determined for each group (see Group constitution table of Item (2)). At that time, since a test substance solution adheres to an outer side of the tip, care was taken so that the adhering test substance solution was wiped off with a Kimwipe once each time of eyedrop administration, and an excessive amount of the test substance solution was not instilled. In addition, although the tip of the pipette tip was sharp, care was taken not to damage the eyeball surface. Immediately before administration, the administration solution was lightly (about several seconds) centrifuged using a small microcentrifuge Wonder Spin, and the supernatant was used for administration.

### (8) Tail vein administration of FITC-dextran

Fourteen days after laser irradiation (Day 14), under general anesthesia by intramuscular femoral administration (1 mL/kg) of a ketamine hydrochloride/xylazine hydrochloride (4:1, v/v) mixed solution, a 4% FITC-dextran solution was administered to the tail vein at a dose of 40 mg/1 mL/animal.

### (9) Eyeball enucleation and eyeball fixation

After 1 minute from the tail vein administration of the 4% FITC-dextran solution, rats were euthanized by excessive anesthesia by administration of 129.6 mg/mL sodium pentobarbital solution and the right eye ball was enucleated. The enucleated eyeball was fixed in a 4% paraformaldehyde phosphate buffer (FUJIFILM Wako Pure Chemical Corporation, hereinafter referred to as 4% PFA) for 12 to 30 hours at normal temperature.

### (10) Preparation of choroidal flat mount specimen

The eyeball fixed with 4% PFA was placed in a petri dish containing D-PBS, and an extra tissue around the eyeball was trimmed. A hole was made in the limbus corneal using a 25G injection needle under a stereomicroscope (EZ-4, Leica Microsystems, Inc.), and starting from the hole, the entire cornea, the iris, and the lens were excised to obtain a state of the optic cup using corneal micro scissors. Retinal tissues other than retinal pigment epithelial cells were detached using a micro spatula, and the eye cup was divided into 4 to 6 parts using corneal micro scissors. In this case, the incision was made while avoiding the irradiation site. The optic nerve was excised from 4 to 6 divided eye cup using corneal micro scissors and washed with D-PBS. A 4 to 6 divided eye cup was placed on a glass slide, and surrounding moisture was sucked using filter paper.

An appropriate amount of FULLOROMOUNT (DBS) was dropped, covered with a cover glass, and sealed around the glass with a manicure so as not to mix air bubbles. The completed specimen was dried for about 24 hours under refrigeration and light shielding. The dried specimens were subsequently stored under refrigeration and light shielding.

### (11) Confocal photomicrograph of flat mount specimen

A photograph (Magnification: 20 times, number of pixels: 1024 × 1024 pixels) of the choroidal neovascularization site in a laser trace was taken using a confocal laser microscope (LSM710, Carl Zeiss).

### (12) CNV area measurement using flat mount specimen fluorescent fundus image

The area of neovascularization (CNV area, unit: pixel) by fluorescent fundus images was calculated using image processing software ImageJ (National Institutes of Health).

### (13) Discard choroidal flat mount specimens

The choroidal flat mount specimens could not be stored for a long period due to fluorescence bleaching, and thus were discarded after the test was completed.

### 7. Humanity endpoint

No animals had to be judged on the basis of the humane endpoint (indication of euthanasia in animal experiments).

### 8. Processing of data

Data was processed in an Excel file (Microsoft Excel, Microsoft).

### (1) CNV area by flat mount specimen fluorescent fundus image

Among the data of six points in one eye where laser irradiation was performed, data on unclear irradiation sites due to reasons such as the irradiation site being unidentifiable, the border being unclear, or the sample having scratches, irradiation sites where multiple irradiation marks have merged, irradiation sites where bleeding has been observed that is greater than the diameter of the irradiation mark, and the like were excluded. The CNV area values of the maximum value and the minimum value were excluded from the three or more irradiation sites, and the mean of the CNV area values (pixels) of the remaining irradiation sites was set as an individual value, and the mean value ± standard error of each group was calculated. In addition, the group value and standard error of the CNV area are expressed with one decimal place.

### 8. Statistical method

The test was performed using statistical analysis software SPSS (Version 26, IBM), and a significance level was 5% on one side.

### (1) Validation of CNV model

The validity of the CNV model was evaluated by performing the Student's t-test in the case of equal variance and the Welch's t-test in the case of unequal variance after the F-test with Group 1 as a negative control group and Group 2 as a positive control group (EYLEA (registered trademark) administration group).

### (2) Evaluation of drug efficacy of test substance

Dunnett multiple comparison test was performed between Group 1 as a negative control group and Group 3 (Compound 9 (1 mg/mL)) and Group 4 (Compound 9 (10 mg/mL)), which are test substance administration groups, to evaluate the drug efficacy of the test substances.

### 9. Results

### (1) General condition observation and body weight measurement

No individuals with a problem in general condition were identified during the test period. In addition, the body weight smoothly increased in all the individuals during the test period, and the remaining eyedrop administration group showed a slightly gradual transition in body weight increase as compared with Group 2 (EYLEA intravitreal administration group), but a large average body weight difference was not confirmed between the groups.

The body weight transition is shown in FIG. 5 and Table 9, and the individual weight is shown in Table 10.

### (2) Evaluation of drug efficacy of test substance

As a result of an F test with respect to Group 1 (physiological saline eyedrop administration group) which is a negative control group, Group 2 (EYLEA intravitreal administration group) which is a positive control group, since the variances were equal, a Student's t-test was performed and the results showed that the CNV area was significantly reduced in Group 2 (P = 0.009).

As a result of performing Dunnett's multiple comparison test on Group 3 (Compound 9 eyedrop administration group (low concentration: 1 mg/mL)) and Group 4 (Compound 9 eyedrop administration group (high concentration: 10 mg/mL)) with Group 1 (negative control group) as a control group, reduction in CNV area correlated with the administration dose was confirmed, but no significant difference in CNV area was observed in Group 3 and Group 4 with respect to Group 1.

Group values of CNV area values of each group are shown in FIG. 6 and Table 11, statistical data processing results are shown in Table 12, and individual CNV area values are shown in Table 13 and Table 14.

IVT in the drawings and the tables means intravitreal, and CNV means choroidal neovascularization.

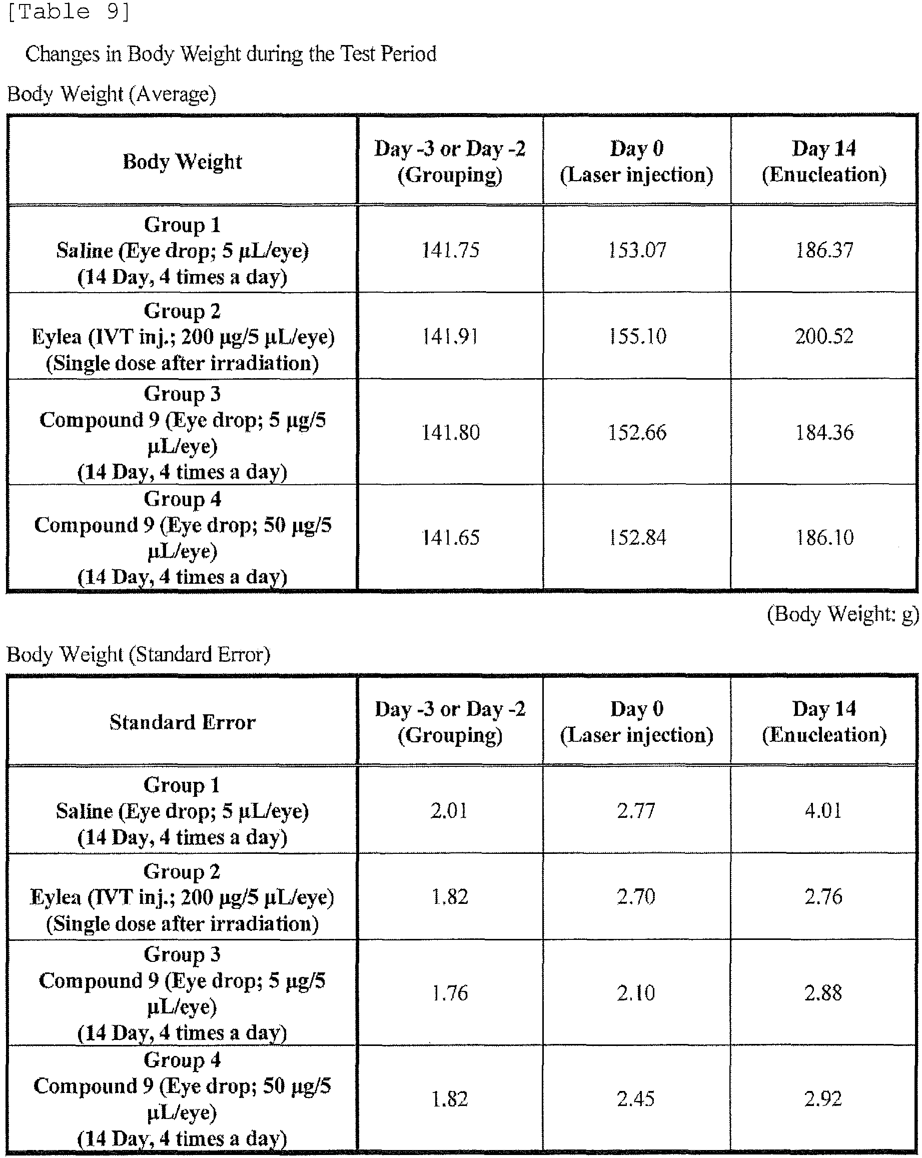

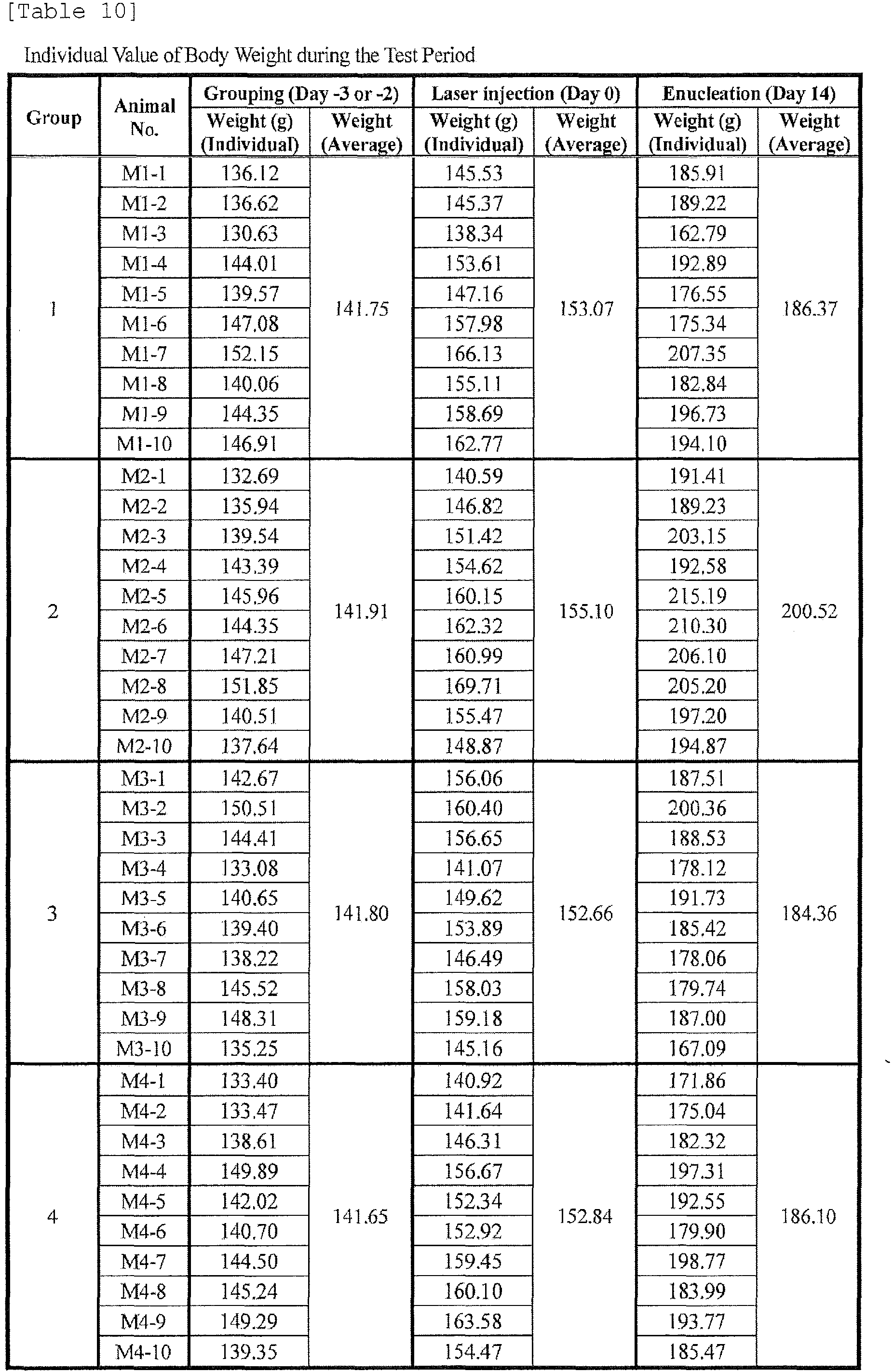

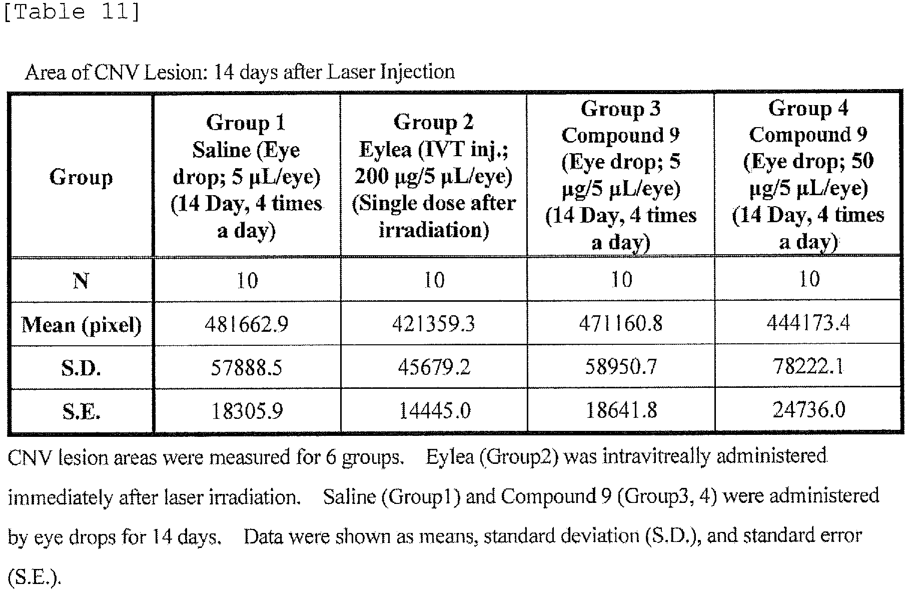

A significant difference was confirmed in the Student's t test between Group1 and Group2.

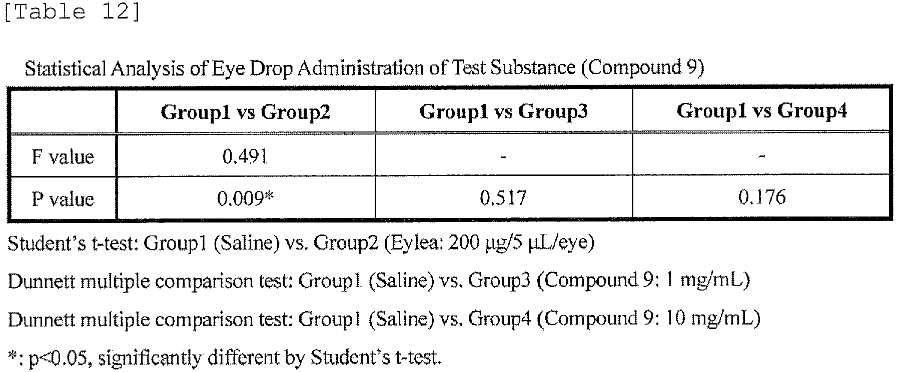

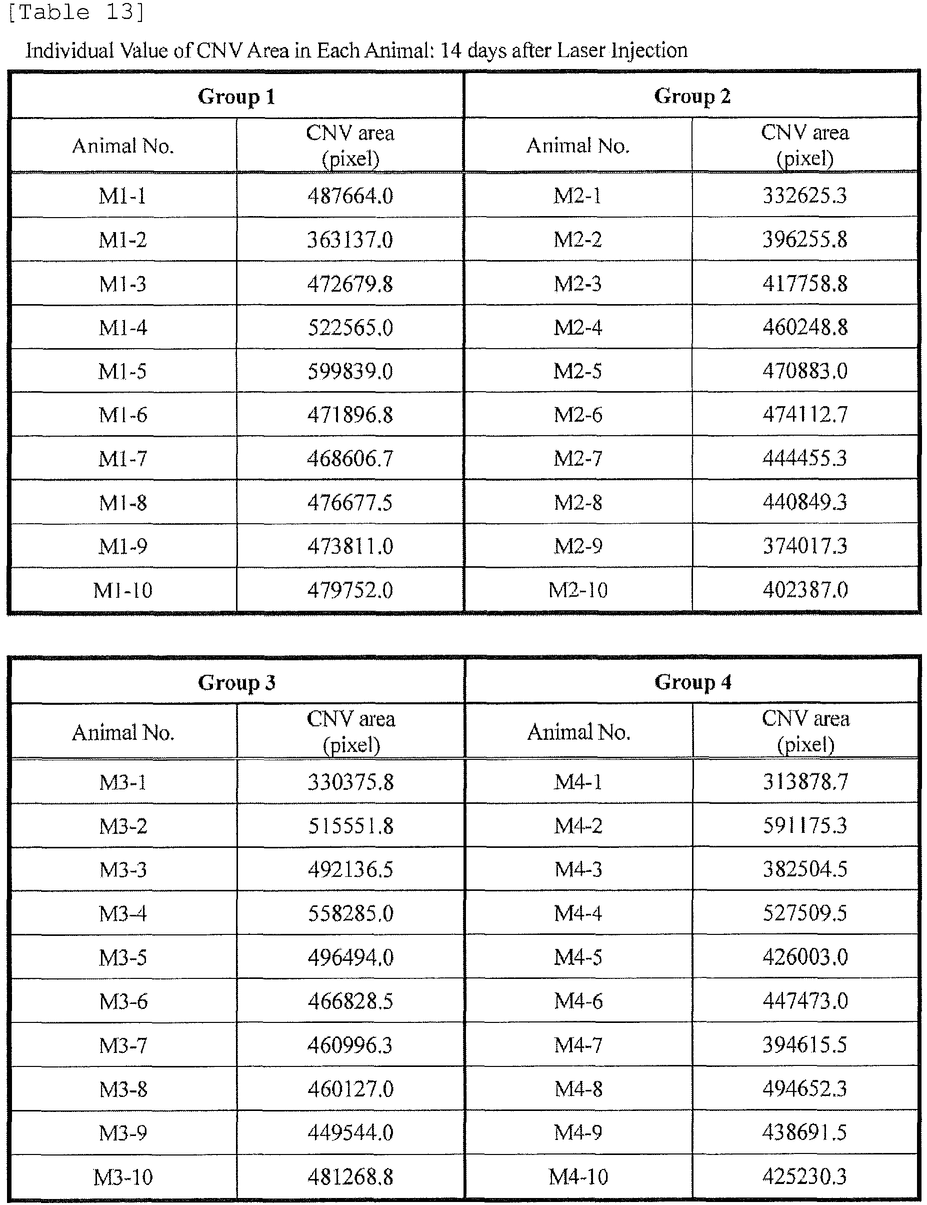

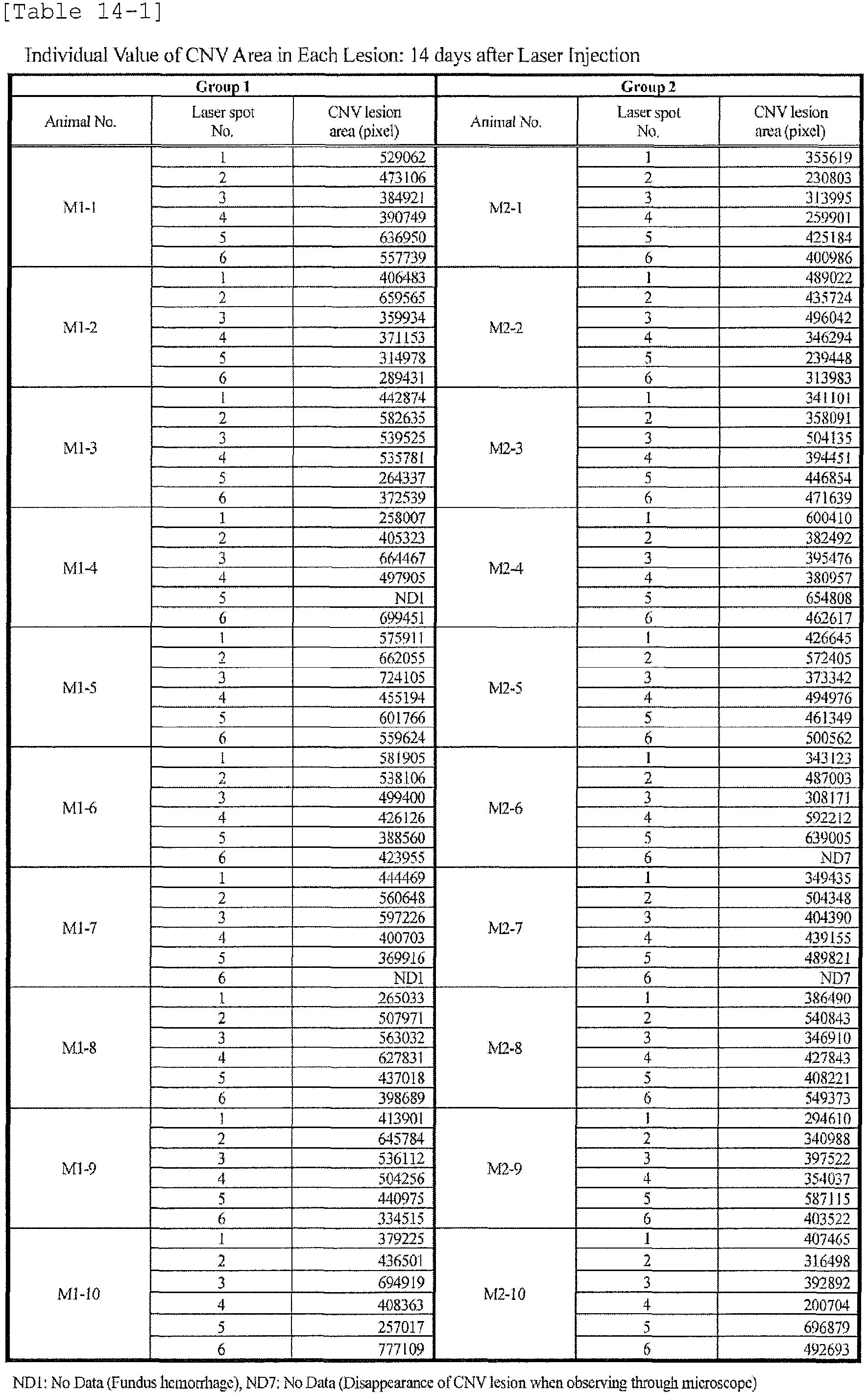

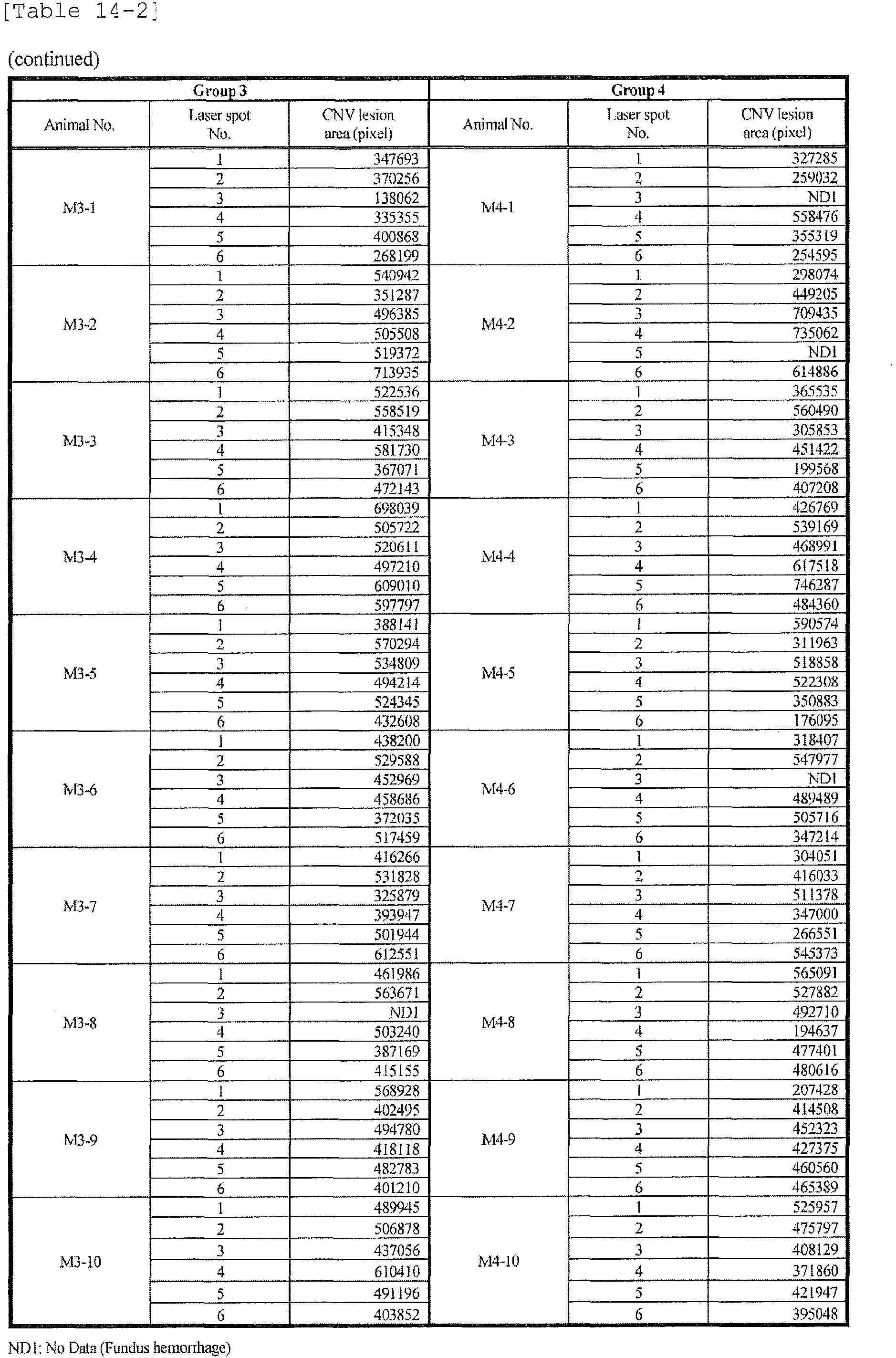

### 10. Conclusion

Eyedrop administration of the test substance compound 9 was performed on laser-induced choroidal neovascularization rat model, and the drug efficacy thereof was evaluated by comparing the area of choroidal neovascularization (CNV area).

The right eyes of rats were irradiated with a laser at an intensity of 300 mW at six locations per eye. Also, a total of four groups were set up: a physiological saline administration group as a negative control group, an EYLEA intravitreal administration group as a positive control group, and a Compound 9 eyedrop administration group (low concentration: 1 mg/mL) and a Compound 9 eyedrop administration group (high concentration: 10 mg/mL) as test substance administration groups. The eyedrop administration was performed at an administration frequency of four times a day, for 14 days from the day of laser irradiation to 13 days after irradiation at administration doses of 5 µL/eye for the physiological saline administration group, 5 µg/5 µL/eye for the Compound 9 low concentration group, and 50 µg/5 µL/eye for the Compound 9 high concentration group. Fourteen days after irradiation, FITC-dextran was administered via the tail vein, and immediately afterwards the eyeball was enucleated and fixed. The retinal layer was separated therefrom to prepare a choroidal flat mount, and the CNV area was calculated using FITC fluorescence as an index in images taken with a confocal laser microscope.

First, as a result of performing a Student's t-test on Group 2 (EYLEA intravitreal administration group) with Group 1 (negative control group) as a control group in order to confirm the validity of the CNV model, the CNV area in Group 2 was significantly reduced (P = 0.009) compared with Group 1, confirming the validity of the CNV model.

Next, as a result of performing Dunnett's multiple comparison test on Group 3 (Compound 9 eyedrop administration group (low concentration: 1 mg/mL)) and Group 4 (Compound 9 eyedrop administration group (high concentration: 10 mg/mL)) with Group 1 (negative control group) as a control group for the purpose of evaluating the drug efficacy of Compound 9, reduction in CNV area correlated with the eyedrop administration dose was confirmed, but no significant difference in CNV area was observed in either Group 3 or Group 4 compared with Group 1 (Group 1 vs Group 3: P = 0.517, Group 1 vs Group 4: P = 0.176).

From the above results, reduction in CNV area correlated with the administration dose was confirmed in the eyedrop administration of Compound 9 to the laser-induced choroidal neovascularization rat model, but no drug efficacy on CNV area was observed in either the low concentration Compound 9 administration group (1 mg/mL) or the high concentration Compound 9 administration group (10 mg/mL).

### (Note)

As described above, although the present disclosure has been illustrated using the preferable embodiments thereof, it is understood that the scope of the present disclosure should be interpreted only by the claims. It is understood that the patents, patent applications, and other documents cited herein should be incorporated by reference into this specification to the same extent as if the contents themselves were specifically set forth herein. This application claims priority to Japanese Patent Application No. 2022-159075 filed with the Japan Patent Office, and their contents are incorporated herein by reference in their entirety.

### [Industrial Applicability]

The technology provided by the present disclosure can be used for a cyclic peptide derivative and composition for treating or preventing ophthalmic diseases and its production method.

## Claims

1. A composition for treating or preventing an ophthalmic disease, comprising a compound represented by the following Formula (1) 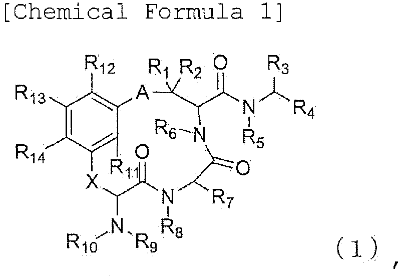 or a pharmaceutically acceptable salt, solvate or prodrug thereof:
wherein
R₁, R₂, R₅, R₆, R₇, R₈, R₉ and R₁₀ are each independently a hydrogen atom, or
an optionally substituted hydrocarbon group, or
R₇ and R₈, together with the carbon atom and nitrogen atom to which they are attached, form an optionally substituted heterocycloalkyl group,
R₃ and R₄ are each independently
a hydrogen atom, an optionally substituted hydrocarbon group, a carboxyl group,
an optionally substituted alkoxycarbonyl group, or
an optionally substituted alkoxycarbonyloxy group, R₁₁, R₁₂, R₁₃, and R₁₄ are each independently
a hydrogen atom, an optionally substituted hydrocarbon group, a hydroxy group,
an optionally substituted alkoxy group, or
an optionally substituted alkoxycarbonyloxy group,
X is CH₂ or CO, and
A is O, NH or S, wherein the NH is optionally substituted.

2. The composition of claim 1, wherein the ophthalmic disease comprises a retinal disease.

3. The composition of claim 1, wherein the ophthalmic disease comprises at least one of diabetic retinopathy, glaucoma or age-related macular degeneration.

4. The composition of any one of claims 1 to 3, wherein R₁ and R₂ are each independently a hydrogen atom, or a C₁₋₆ alkyl group.

5. The composition of any one of claims 1 to 4, wherein R₁ and R₂ are each independently a hydrogen atom, a methyl group or an ethyl group.

6. The composition of any one of claims 1 to 5, wherein R₃ and R₄ are each independently a hydrogen atom, a C₁₋₆ alkyl group substituted with a carboxyl group, or a carboxyl group.

7. The composition of any one of claims 1 to 6, wherein R₃ and R₄ are each independently a hydrogen atom, a carboxymethyl group, a carboxyethyl group, a carboxypropyl group or a carboxyl group.

8. The composition of any one of claims 1 to 7, wherein R₅ is a hydrogen atom, or a C₁₋₆ alkyl group.

9. The composition of any one of claims 1 to 8, wherein R₅ is a hydrogen atom.

10. The composition of any one of claims 1 to 9, wherein R₆ is a hydrogen atom, or a C₁₋₆ alkyl group.

11. The composition of any one of claims 1 to 10, wherein R₆ is a hydrogen atom.

12. The composition of any one of claims 1 to 11, wherein R₇ is a hydrogen atom, a C₁₋₆ alkyl group, a hydroxy C₁₋₆ alkyl group, a carbamoyl C₁₋₆ alkyl group, a C₆₋₁₀ aryl C₁₋₆ alkyl group, a hydroxy C₆₋₁₀ aryl C₁₋₆ alkyl group, a C₅₋₁₀ heteroaryl C₁₋₆ alkyl group, a carboxy C₁₋₆ alkyl group, an amino C₁₋₆ alkyl group, a thio C₁₋₆ alkyl group, a C₁₋₆ alkylthio C₁₋₆ alkyl group, or an amidinoamino C₁₋₆ alkyl group.

13. The composition of any one of claims 1 to 12, wherein R₇ is a hydrogen atom, a methyl group, an isopropyl group, an isobutyl group, a sec-butyl group, a benzyl group, a hydroxymethyl group, a 1-hydroxyethyl group, a carboxymethyl group, a carboxyethyl group, a 4-hydroxybenzyl group, a 4-aminobutyl group, a thiomethyl group, a 2-methylthioethyl group, a carbamoylmethyl group, a carbamoylethyl group, an amidinoaminopropyl group, an indolylmethyl group or a 4-imidazolemethyl group.

14. The composition of any one of claims 1 to 13, wherein R₈ is a hydrogen atom, or a C₁₋₆ alkyl group.

15. The composition of any one of claims 1 to 14, wherein R₈ is a hydrogen atom.

16. The composition of any one of claims 1 to 15, wherein R₇ and R₈, together with the carbon atom and nitrogen atom to which they are attached, form an optionally substituted heterocycloalkyl group.

17. The composition of any one of claims 1 to 16, wherein R₇ and R₈, together with the carbon atom and nitrogen atom to which they are attached, form a C₅₋₁₀ heterocycloalkyl group.

18. The composition of any one of claims 1 to 17, wherein R₉ and R₁₀ are each a hydrogen atom, or a C₁₋₆ alkyl group.

19. The composition of any one of claims 1 to 18, wherein R₉ and R₁₀ are each independently a hydrogen atom or a methyl group.

20. The composition of any one of claims 1 to 19, wherein R₁₁, R₁₂, R₁₃, and R₁₄ are each independently a hydrogen atom, an alkoxy group or a hydroxy group.

21. The composition of any one of claims 1 to 20, wherein R₁₂ is a hydrogen atom, or a hydroxy group.

22. The composition of any one of claims 1 to 21, wherein R₁₁, R₁₂, R₁₃, and R₁₄ are each independently a hydrogen atom, or a hydroxy group.

23. The composition of any one of claims 1 to 22, wherein X is CH₂ or CO.

24. The composition of any one of claims 1 to 23, wherein A is O, NH substituted with a C₁₋₆ alkyl group, NH or S.

25. The composition of any one of claims 1 to 24, wherein A is O, NH or S.
